# EUROPEAN PATENT SPECIFICATION

(11) **EP 4 426 700 B1**
(45) Date of publication and mention of the grant of the patent: **08.07.2026**
(21) Application number: 22813182.7
(22) Date of filing: 31.10.2022
(51) Int. Cl.: C07D 487/04, C07D 401/14, A61P 35/00, A61K 31/4162

(54) **HETEROBIFUNCTIONAL MOLECULES AS TEAD INHIBITORS**
HETEROBIFUNKTIONELLE MOLEKÜLE ALS TEAD-HEMMER
MOLÉCULES HÉTÉROBIFONCTIONNELS UTILISÉES EN TANT QU'INHIBITEURS DE TEAD

(30) Priority: 02.11.2021 EP 21205991
(43) Date of publication of application: 11.09.2024
(73) Proprietor: Merck Patent GmbH, 64293 Darmstadt (DE)
(72) Inventor: HEINRICH, Timo, 64293 Darmstadt (DE); GEHRTZ, Paul, 64293 Darmstadt (DE)
(74) Representative: Merck Patent Association
(86) International application number: PCT/EP2022/080318
(87) International publication number: WO 2023/078813

(56) References cited:
- WO-A1-2021/061053
- WO-A1-2021/178339
- WO-A1-2021/224291
- WO-A1-2022/018072
- SARAH A. SMITH ET AL: "Antiproliferative and Antimigratory Effects of a Novel YAP–TEAD Interaction Inhibitor Identified Using in Silico Molecular Docking", JOURNAL OF MEDICINAL CHEMISTRY, vol. 62, no. 3, 14 January 2019 (2019-01-14), US, pages 1291 - 1305, XP055623783, ISSN: 0022-2623, DOI: 10.1021/acs.jmedchem.8b01402

## Description

### Field of the invention

The present invention relates to heterocyclic derivatives. These heterocyclic derivatives are be useful for therapy and/or prophylaxis in a mammal, in particular a human. In particular, they may be useful as inhibitors and/or degraders of TEAD and thus useful for treating cancer.

### Background of the invention

In recent years the Hippo pathway has become a target of interest for the treatment of hyperproliferative disorders and diseases, in particular cancer (S. A. Smith et al., J. Med. Chem. 2019, 62, 1291-1305; K. C. Lin et al., Annu. Rev. Cancer Biol. 2018, 2: 59-79; C.-L. Kim et al., Cells (2019), 8, 468; K. F. Harvey et al., Nature Reviews Cancer, Vol. 13, 246-257 (2013)). The Hippo pathway regulates cell growth, proliferation, and migration. It is assumed that in mammals the Hippo pathway acts as a tumor suppressor, and dysfunction of Hippo signaling is frequently observed in human cancers.

Furthermore, as the Hippo pathway plays a role in several biological processes - like in self-renewal and differentiation of stem cells and progenitor cells, wound healing and tissue regeneration, interaction with other signaling pathways such as Wnt - its dysfunction may also play a role in human diseases other than cancer (C.-L. Kim et al., Cells (2019), 8, 468; Y. Xiao et al., Genes & Development (2019) 33: 1491-1505; K. F. Harvey et al., Nature Reviews Cancer, Vol. 13, 246-257 (2013)).

While several aspects of the pathway activity and regulation are still subject to further research, it is already established that in its "switched-on"-state the Hippo pathway involves a cascade of kinases (including Mst 1/2 and Lats 1/2) in the cytoplasm which results in the phosphorylation of two transcriptional co-activators, YAP (Yes-associated protein) and TAZ (Transcription co-activator with PDZ binding motif). Phosphorylation of YAP/TAZ leads to their sequestration in the cytoplasm and eventually to their degradation. In contrast, when the Hippo pathway is "switched-off" or dysfunctions, the non-phosphorylated, activated YAP/TAZ co-activators are translocated into the cell nucleus. Their major target transcription factors are the four proteins of the Transcriptional enhanced associate domain (TEAD) transcription factor family (TEAD1-4). Binding of YAP or TAZ to and activation of TEAD (or other transcription factors) have shown to induce the expression of several genes many of which mediate cell survival and proliferation. Thus, activated, non-phosphorylated YAP and TAZ may act as oncogenes, while the activated, switched-on Hippo pathway may act as a tumor suppressor by deactivating, i.e. phosphorylating YAP and TAZ.

Furthermore, the Hippo pathway may also play a role in resistance mechanisms of cancer cells to oncology and immune-oncology therapy (R. Reggiani et al., BBA - Reviews on Cancer 1873 (2020) 188341, 1-11).

Consequently, the dysfunction or aberrant regulation of the Hippo pathway as a tumor suppressor is believed to be an important event in the development of a wide variety of cancer types and diseases.

Therefore, inhibition of TEAD and YAP-TEAD or TAZ-TEAD protein-protein interaction by pharmacological intervention appears to be a reasonable and valuable strategy to prevent and/or treat cancer and other hyperproliferative disorders and diseases associated with the dysfunction of the Hippo pathway.

Small molecule degraders are increasingly utilized as tools to examine the functional roles of proteins and emerged as a novel therapeutic modality. Operating at the post-translational level, these molecules provide the potential for differentiated biological responses in comparison to classical inhibitors and expand the repertoire of methods for protein knock down beyond genetic approaches (e.g.: knock-out, siRNA).

Degrader molecules provide an example of a chemical genetic technique capable of more generally targeting the proteome. These chimeric molecules are designed to induce the degradation of their target proteins via the ubiquitin proteasome system (UPS), thereby eliminating pre-existing proteins. The UPS is the major intracellular pathway for protein degradation in which a series of enzymes known as E1s (ubiquitin activating enzymes), E2s (ubiquitin conjugating enzymes) and E3s (ubiquitin ligases) carry out covalent linkage of the 9kDa, 76 amino acid protein ubiquitin to a target protein. Subsequent enzymatic reactions result in the formation of a polyubiquitin chain, which targets the protein for degradation by the 26S proteasome.

Bifunctional degraders (sometimes also referred to as "heterobifunctional degraders") comprise an E3 ligase-binding motif that is linked to a target protein binding moiety. Consequently, these molecules hijack the cell's own degradation machinery by recruiting an E3 ligase in vicinity of the target protein. The spatial proximity enables ubiquitination of the protein and subsequent recognition and depletion by the UPS through the formation of a stable ternary complex.

Specificity for a particular target protein is associated with the E3 ligase (Li W, et al. PLoS One. 2008; 3:e1487) that facilitates the final step of ubiquitin attachment to the target protein. While the first generation degraders were successfully developed using peptides as an E3 ubiquitin ligase-recognizing motif, they were either not cell-permeable or made cell-permeable by adding a cell-permeating motif such as the TAT peptide (Sakamoto KM, et al Proc Natl Acad Sci U S A. 2001; 98:8554-8559; Zhang D, et al. Bioorg Med Chem Lett. 2004; 14:645-648; Schneekloth JS Jr. et al. J Am Chem Soc. 2004; 126:3748-3754.). The poor cell permeability of the first generation of bifunctional degraders was significantly improved by the discovery of small molecules that bind to E3 ligases such as the Von Hippel Lindau (VHL) ligand binding to VHL ligase (Buckley et al, J. Am. Chem. Soc., 2012, 134 (10), pp 4465-4468; Sosi č et al., Chem. Soc. Rev., 2022, 51, 3487-3534) or thalidomide derivatives binding to the CRBN or Cereblon E3 Ligase (Winter et al, Science 19 Jun 2015: Vol. 348, Issue 6241, pp. 1376-1381; Sosič et al., Chem. Soc. Rev., 2022, 51, 3487-3534).

Thus, one type of targeted therapy involves bifunctional degrader molecules that utilize the intracellular UPS to selectively degrade target proteins by forming a ternary complex between the target protein, the bifunctional degrader molecule, and the E3 (ubiquitin) ligase which ultimately provides for the degradation of the target protein by a proteasome.

There is a need for further and/or improved options of cancer treatment utilizing the pharmacological targeting of TEAD to provide functional alternations of the Hippo pathway. Bifunctional degrader molecules may particularly useful.

### Description of the invention

In one aspect the present invention provides a compound of formula I

Q¹-Q²-Q³ I

wherein
- Q¹: is a ubiquitin ligase ligand;
- Q²: is (i) absent; or (ii) a divalent linker formed by an unbranched alkylene chain having 2-25 C atoms, in which 1-8 non-adjacent CH₂ groups may independently from each other be replaced by O, -C(=O)-NH-, -NH-C(=O)-, -C(=O)-N(CH₃)-, -N(CH₃)-C(=O)-, -CH=CH- and/or -C=C-; in which one or two CH₂ groups may bear a methyl substituent; and in which one CH₂ group may optionally be replaced by a moiety selected from the group consisting of 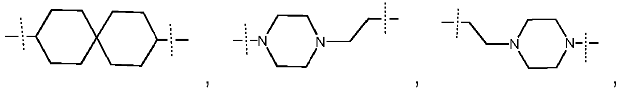 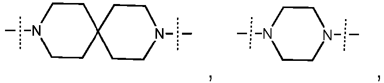 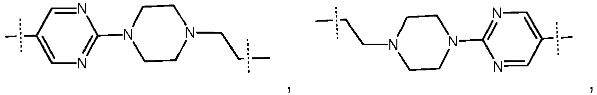 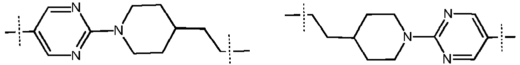 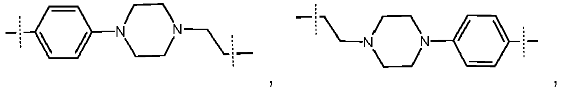 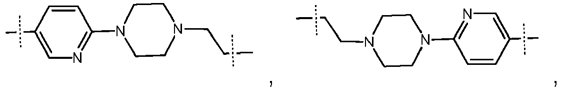 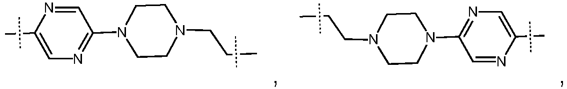 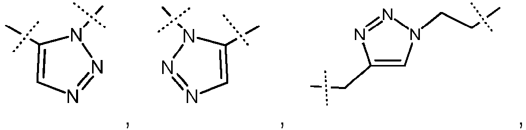 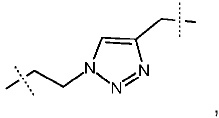 wherein the left-hand end of that moiety is directed towards the Q¹ part of the compound of formula I and the right-hand end of that moiety is directed towards the Q² part of the compound of formula I;
- Q³: denotes or 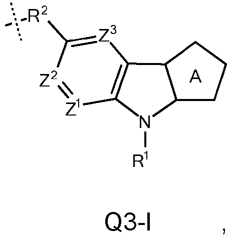
wherein
- Ring A: represents a five-membered heteroaromatic ring selected from the group consisting of the following ring moieties: 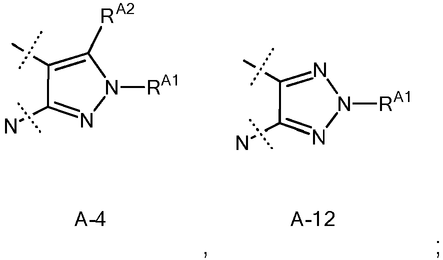
wherein
R^{A1} represents methyl;
R^{A2} represents H;
Z¹ is CH;
Z² is CH;
Z³ is CH;
R¹ represents Ar¹, Hetar¹, Cyc¹, Hetcyc¹,;
R² represents 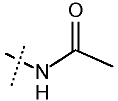 (-NH-C(=O)-);
Ar¹ is a mono- or bicyclic aryl with 6 or 10 ring carbon atoms, wherein that aryl may be unsubstituted or substituted with substituents R^{B1}, R^{B2} and/or R^{B3} which may be the same or different;
Hetar¹ is a monocyclic heteroaryl with 5 or 6 ring atoms or a bicyclic heteroaryl with 9 or 10 ring atoms wherein 1, 2, 3 or 4 of said ring atoms is/are a hetero atom(s) selected from N, O and/or S and the remaining are carbon atoms, wherein that heteroaryl may be unsubstituted or substituted with substituents R^{B1}, R^{B2} and/or R^{B3} which may be the same or different;
Cyc¹ is a saturated or partially unsaturated, mono- or bicyclic carbocycle with 3, 4, 5, 6, 7, 8, 9 or 10 ring carbon atoms, wherein that carbocycle may be unsubstituted or substituted with R^{B4}, R^{B5} and/or R^{B6} which may be the same or different;
Hetcyc¹ is a saturated or partially unsaturated, monocyclic heterocycle with 5 or 6 ring atoms wherein 1 or 2 of said ring atoms is/are a hetero atom(s) selected from N, O and/or S and the remaining are carbon atoms, wherein that heterocycle may be unsubstituted or substituted with R^{B4}, R^{B5} and/or R^{B6} which may be the same or different, wherein, if one of the heteroatoms is S, then that heterocycle may also be substituted with R^{B4}, R^{B5}, R^{B6}, R^{B7} and/or R^{B8};
R^{B1}, R^{B2}, R^{B3} represent independently from each other straight-chain or branched C₁₋₆-alkyl, which C₁₋₆-alkyl may be unsubstituted or monosubstituted with -CN or substituted with 1, 2 or 3 halogen, straight-chain or branched C₁₋₄-alkoxy, which C₁₋₄-alkoxy may be unsubstituted or substituted with 1, 2 or 3 halogen, -O-CH₂-C≡CH, straight-chain or branched -S-C₁₋₄-alkyl, which -S-C₁₋₄-alkyl may be unsubstituted or substituted with 1, 2 or 3 halogen, straight-chain or branched C₂₋₆-alkenyl, which C₂₋₆-alkenyl may be unsubstituted or monosubstituted with -CN or substituted with 1, 2 or 3 halogen, F, Cl, Br, -CN, -S(=O)-C₁₋₃-alkyl, S(=O)₂-C₁₋₃-alkyl, -N(C₁₋₃-alkyl)₂, Ar², - CH₂-Ar², Hetar², Cyc², Hetcyc²;
   or two adjacent R^{B1}, R^{B2} and/or R^{B3} form together a divalent -C₃₋₄-alkylene radical in which one of the alkylene carbon units may be replaced by a carbonyl unit (-C(=O)-), or a divalent -O-C₂₋₃-alkylene radical;
R^{B4}, R^{B5}, R^{B6} represent independently from each other F, C₁₋₄-alkyl, which C₁₋₄-alkyl may be unsubstituted or substituted with 1, 2 or 3 F, C₁₋₄-alkoxy, phenyl; or
two of R^{B4}, R^{B5} and R^{B6} are attached to the same carbon atom of said carbocycle Cyc¹ or said heterocycle Hetcyc¹ and form a divalent oxo (=O) group; or
R^{B4} and R^{B5} and R^{B7} and R^{B8} are attached to the same sulfur atom of said heterocycle and form two divalent oxo (=O) groups thereby forming an
   -S(=O)₂- moiety;
Ar² is phenyl which may be unsubstituted or substituted with one or two substituents which are independently from each other selected from OH, F, Cl, Br, C₁₋₄-alkyl and C₁₋₄-alkoxy, wherein that C₁₋₄-alkyl or C₁₋₄-alkoxy group may be substituted with 1, 2 or 3 F atoms;
Hetar² is a monocyclic heteroaryl with 5 or 6 ring atoms wherein 1, 2, 3, 4, 5 of said ring atoms is/are a hetero atom(s) selected from N, O and/or S and the remaining are carbon atoms, wherein that heteroaryl may be unsubstituted or substituted with one or two substituents which are independently from each other selected from F, Cl, Br, C₁₋₄-alkyl and C₁₋₄-alkoxy, wherein that C₁₋₄-alkyl or C₁₋₄-alkoxy group may be substituted with 1, 2 or 3 F atoms;
Cyc² is cyclopropyl, cyclobutyl, cyclopentyl or cyclohexyl, each of which may be unsubstituted or substituted with one or two substituents independently from each other selected from OH, F, Cl, Br, C₁₋₄-alkyl and C₁₋₄-alkoxy, wherein that C₁₋₄-alkyl or C₁₋₄-alkoxy group may be substituted with 1, 2 or 3 F atoms and/or 1 hydroxy group;
Hetcyc² is pyrrolidinyl, piperidinyl, each of which may unsubstituted or substituted with one or two substituents independently from each other selected from OH, F, Cl, Br, C₁₋₄-alkyl and C₁₋₄-alkoxy, wherein that C₁₋₄-alkyl or C₁₋₄-alkoxy group may be substituted with 1, 2 or 3 F atoms and/or 1 hydroxy group;
halogen is F, Cl, Br, I;
or a pharmaceutically acceptable salt, solvate, tautomer and/or stereoisomer thereof.

Any of those particular or even preferred embodiments of the present invention as specified below and in the claims do not only refer to the specified compounds of formula I but to solvates, tautomers or stereoisomers thereof as well as the pharmaceutically acceptable salts of each of the foregoing, including mixtures thereof in all ratios, too, unless indicated otherwise.

In one particular embodiment of the invention, PE1, the compound of formula I is a compound, or a pharmaceutically acceptable salt, solvate, tautomer and/or stereoisomer thereof, in which
Q¹ is a E3 ubiquitin ligase ligand;
and the remaining radicals and residues are as defined for formula I above or for any of the further particular embodiments described herein above or below.

In still a further particular embodiment, PE1a, of PE1
Q¹ is (a) a CRBN (cereblon) ligand or (b) a VHL (von Hippel-Lindau) ligand or (c) a different type of E3 uibiquitin ligase ligand (other than a CRBN or a VHL ligand).

In yet another particular embodiment. PE1aa, of PE1 or PE1a
Q¹ is a (a) CRBN (cereblon) ligand
   and
(a) the CRBN (cereblon) ligand has a structure of formula Q1-I, Q1-II, Q1-VII or Q1-VIII 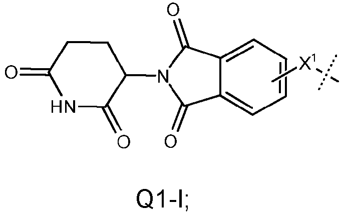 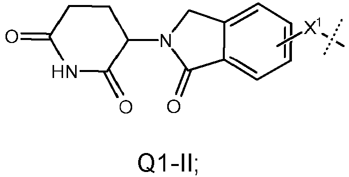 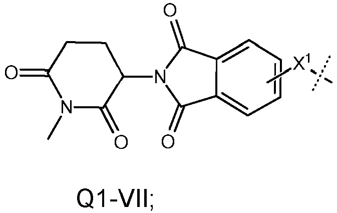 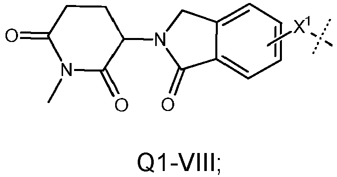
wherein
X¹ denotes a single bond, -O-, -NH-, -NCH₃-, -CH₂-, or -NH-C(=O)-.

It is a particular embodiment, PE1aaa, of PE1aa in which
Q¹ is a (a) CRBN (cereblon) ligand
   and
(a) the CRBN (cereblon) ligand has a structure selected from the structures of formulas Q1-I-1, Q1-1-2, Q1-1-3; Q1-1-4, Q1-I-5, Q1-1-6, Q1-11-1, Q1-11-2, Q1-II-3, Q1-II-4, Q1-II-5, Q1-II-6, Q1-II-7, Q1-VII-1, Q1-VII-2, and Q1-VII-3

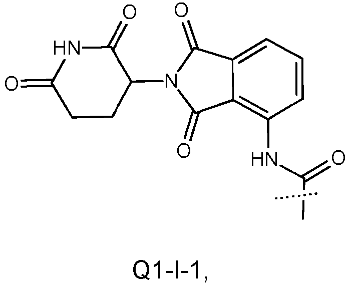

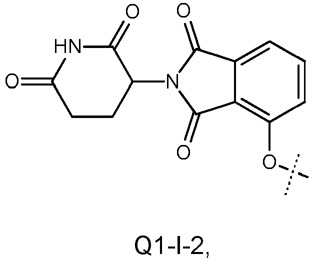

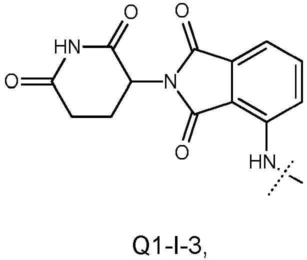

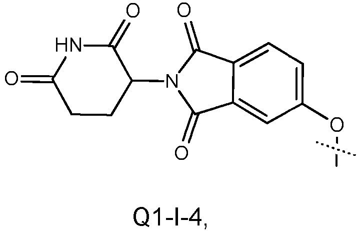

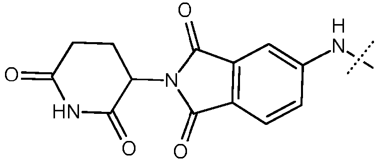

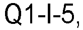

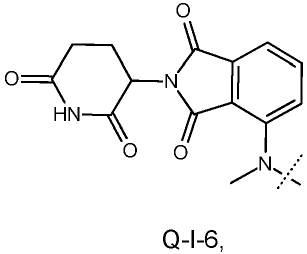

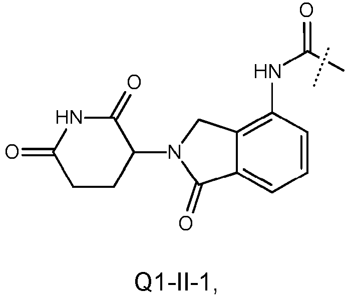

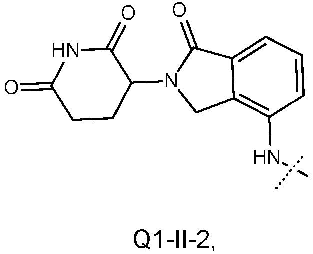

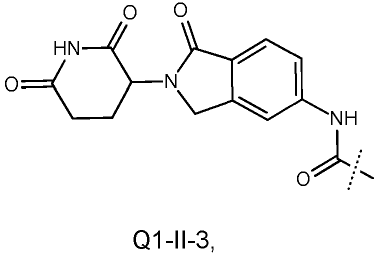

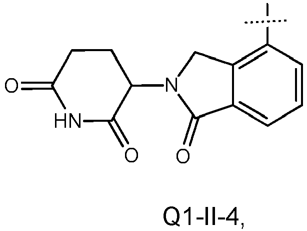

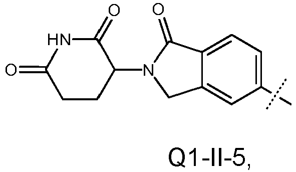

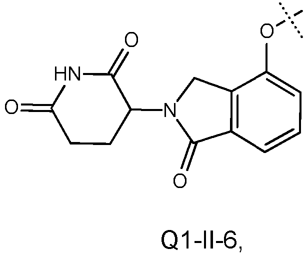

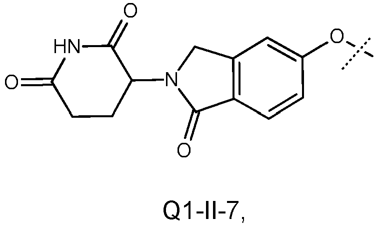

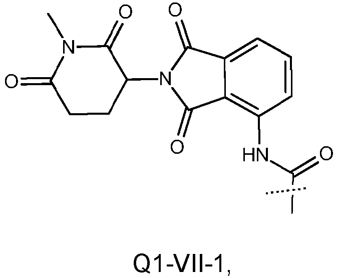

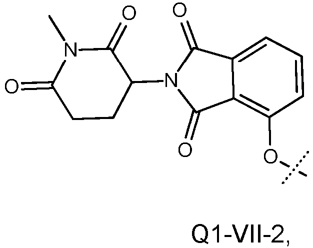

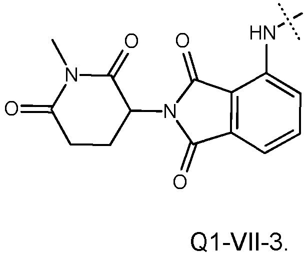

It is yet another particular embodiment, PE1aaaa, of PE1aaa in which
Q¹ is a (a) CRBN (cereblon) ligand and the CRBN (cereblon) ligand has a structure selected from the structures of formulas Q1-I-2, Q1-I-3, Q1-I-5, Q1-I-6, Q1-II-2, Q1-II-3, Q1-II-4, Q1-II-5, and Q1-VII-1.

It is still another particular embodiment, PE1aaab, of PE1aaa in which
Q¹ is a (a) CRBN (cereblon) ligand and the CRBN (cereblon) ligand has a structure selected from the structures of formulas Q1-I-1, Q1-I-2, Q1-I-3; Q1-I-4, Q1-I-5, and Q1-I-6. It is a particular embodiment, PE1aaaba, of PE1aaab in which the CRBN ligand has a structure selected from the structures of formula Q1-1-2, Q1-I-3, Q1-I-5, and Q1-I-6.

It is still another particular embodiment, PE1aaac, of PE1aaa in which
Q¹ is a (a) CRBN (cereblon) ligand and the CRBN (cereblon) ligand has a structure selected from the structures of formulas Q1-II-1, Q1-II-2, Q1-II-3, Q1-II-4, Q1-II-5, Q1-II-6, and Q1-II-7. It is a particular embodiment, PE1aaaca, of PE1aaac in which the CRBN ligand has a structure selected from the structures of formula Q1-II-2, Q1-II-3, Q1-II-4, and Q1-II-5.

It is another particular embodiment, PE1ab, of PE1a in which
Q¹ is a (b) VHL (von Hippel-Lindau) ligand; and the VHL ligand has a structure of formula Q1-III: 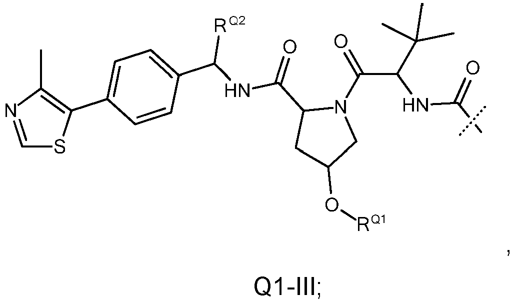
wherein
   - R^{Q1}: denotes H or -C(=O)-CH₃;
   - R^{Q2}: denotes H or CH₃;
including all possible stereoisomeric forms of formula Q1-III.

It is another particular embodiment, PE1aba, of PE1a or PE1ab in which
Q¹ is a (b) VHL (von Hippel-Lindau) ligand; and the VHL ligand has a structure selected from formulas Q1-III-1, Q1-III-2, Q1-III-3, Q1-III-4, and Q1-III-5

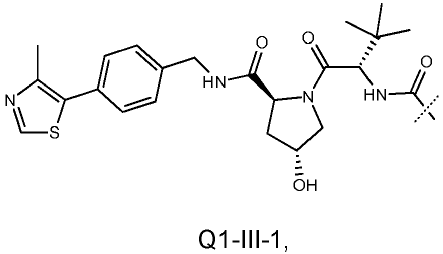

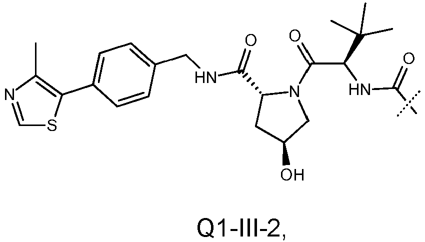

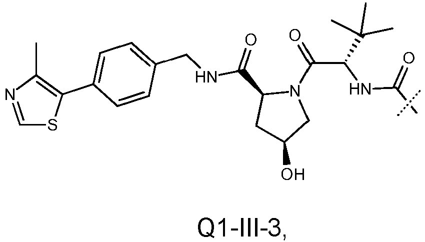

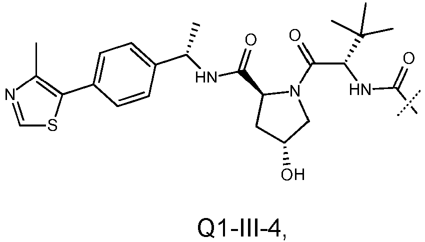

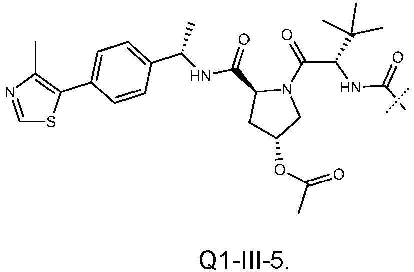

It is another particular embodiment, PE1abaa, of PE1aba in which the VHL ligand has a structure selected from the structures of formulas Q1-III-1 and Q1-III-4.

In particular Q¹ has the structure of formula Q1-III-1 (PE1abaaa):

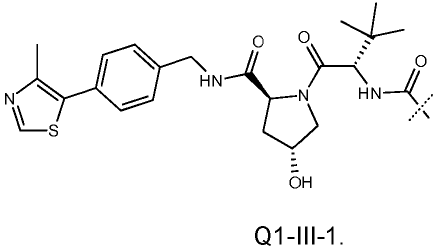

It is another particular embodiment, PE1ac, of PE1a in which
Q¹ is a (c) different type of E3 ubiquitin ligase ligand other than a CRBN or a VHL ligand; and that different type of E3 ubiquitin ligase ligand has a structure selected from the structures of formulas Q1-IV, Q1-V, Q1-VI, Q1-XII (in particular Q1-XII-1), Q1-XIII (in particular Q1-XIII-1):

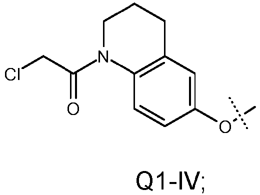

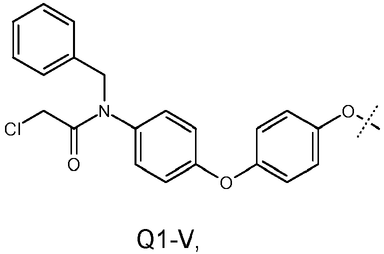

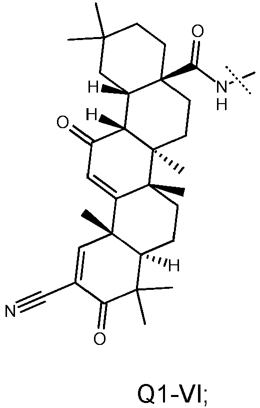

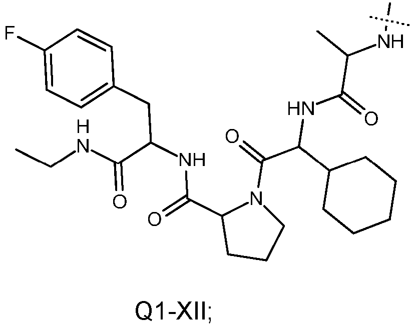

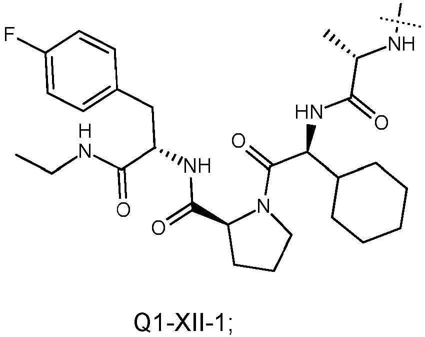

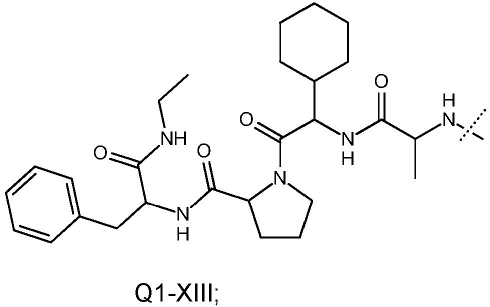

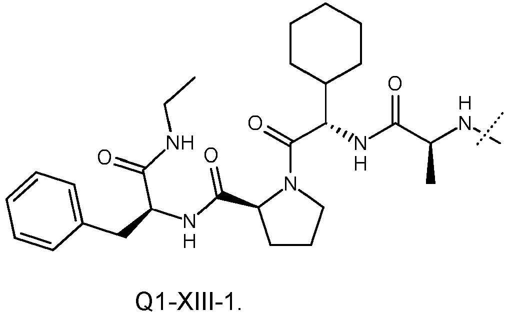

In another particular embodiment of the compounds of formula I, PE2,
Q² is a divalent linker formed by an unbranched alkylene chain having 2-25 C atoms, in which 1-8 non-adjacent CH₂ groups may independently from each other be replaced by O, -C(=O)-NH-, -NH-C(=O)-, -C(=O)-N(CH₃)-, -N(CH₃)-C(=O)-, and/or -C=C-; in which one or two CH₂ groups may bear a methyl substituent; and in which one CH₂ group may optionally be replaced by a moiety selected from the group consisting of 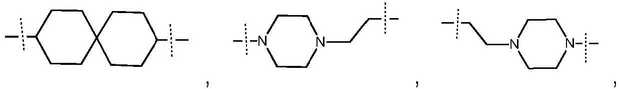 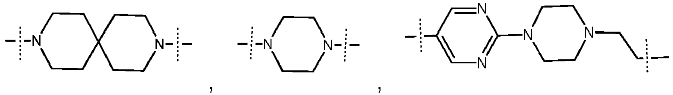 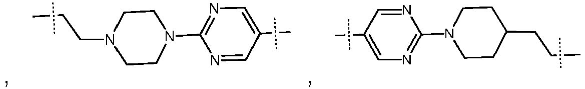 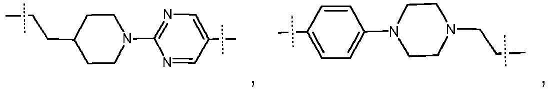 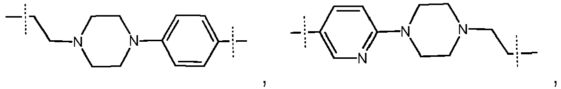 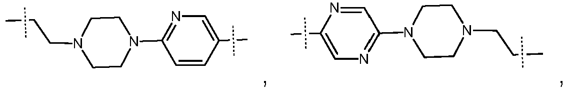 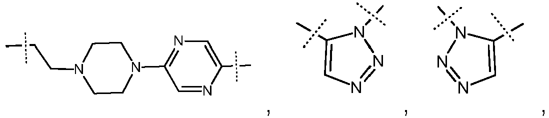 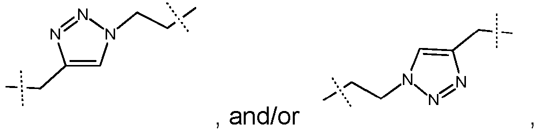 wherein the left-hand end of that moiety is directed towards the Q¹ part of the compound of formula I and the right-hand end of that moiety is directed towards the Q³ part of the compound of formula I;
and the remaining radicals and residues are as defined for formula I above or for any of the further particular embodiments described herein above or below.

In one particular embodiment, PE2a, of PE2
Q² is an unbranched alkylene chain with 2 to 10 (i.e., 2, 3, 4, 5, 6, 7, 8, 9, or 10) CH₂ (methylene) units.

In another particular embodiment, PE2b, of PE2
Q² is an unbranched alkylene chain with 4 to 25 CH₂ (methylene) units, in which 1, 2, 3, 4, 5, or 6 non-adjacent CH₂ groups are replaced by O thereby forming a polyether linker chain. If more than one CH₂ group is replaced by O, typically two oxygen atoms are sepated by at least 2 CH₂ units, or 3 or 4 CH₂ units thereby forming, for instance polyether units such as -CH₂-O-CH₂-CH₂-O-CH₂-CH₂-O-, -O-CH₂-CH₂-CH₂-O-CH₂-CH₂-CH₂-, -CH₂-CH₂-O-CH₂-CH₂-CH₂-O-CH₂-CH₂-, -CH₂-CH₂-CH₂-O-CH₂-CH₂-CH₂-CH₂-O- or -CH₂-CH₂-O-CH₂-CH₂-CH₂-CH₂-O-. Other suitable polyether linkers may be those utilized in some of the exemplary compounds of this invention.

In still particular embodiment, PE2c, of PE2
Q² is an unbranched alkylene chain with 4 to 25 CH₂ (methylene) units in which one CH₂ unit is replaced by -C(=O)-NH- or -NH-C(=O)-.

In still particular embodiment, PE2d, of PE2 elements of PE2b and PE2c are combined, i.e.
Q² is an unbranched alkylene chain with 4 to 25 CH₂ (methylene) units in which (i) 1, 2, 3, 4, 5, or 6 non-adjacent CH₂ groups are replaced by O thereby forming a polyether linker chain, and (ii) another CH₂ unit is replaced by -C(=O)-NH- or -NH-C(=O)-; whereby the oxygen atom(s) and the -C(=O)-NH- or -NH-C(=O)- moiety are separated by at least two methylene units.

In still another particular embodiment, PE2e, of PE2
Q² is an unbranched alkylene chain with 4 to 25 CH₂ (methylene) units in which a CH₂ unit is replaced by a moiety selected from the group consisting of

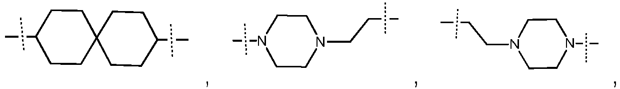

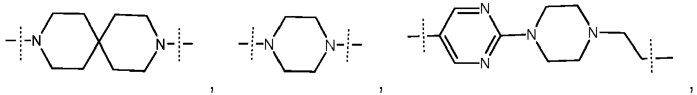

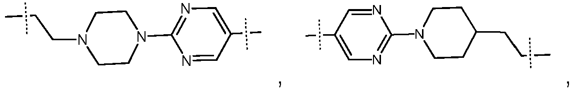

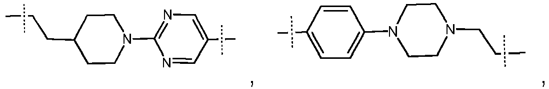

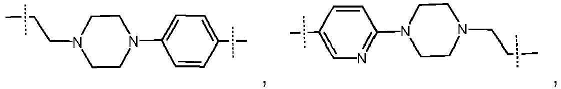

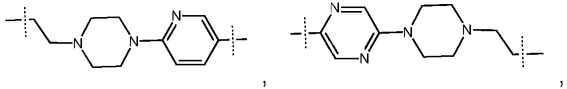

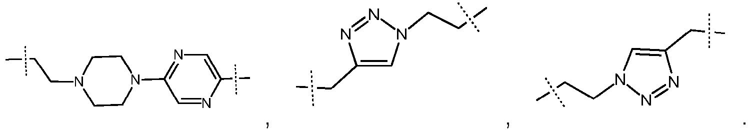

In yet another particular embodiment, PE2f, of PE2
- Q²: is selected from the group consisting of

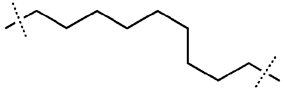

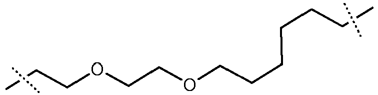

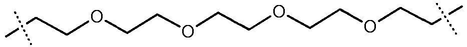

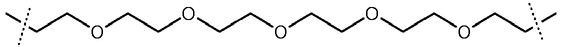

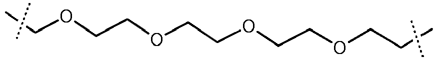

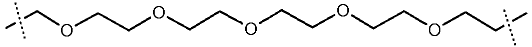

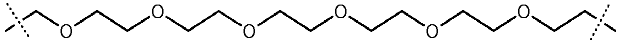

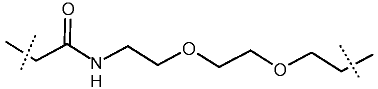

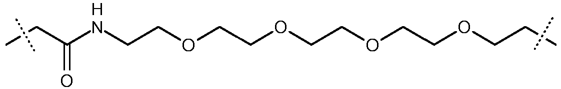

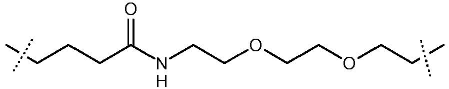

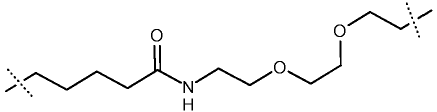

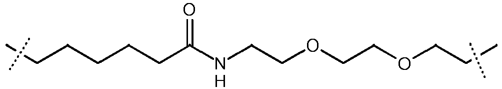

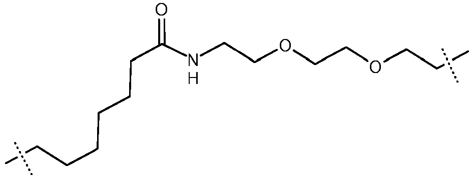

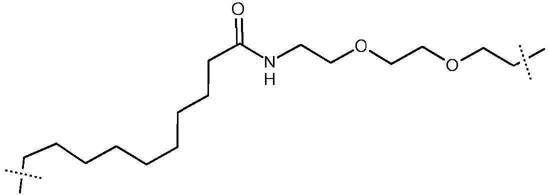

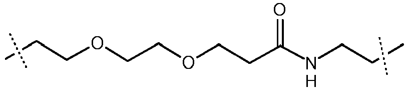

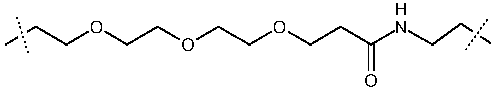

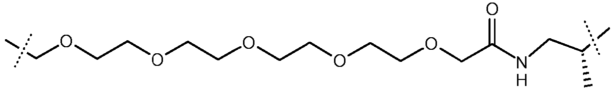

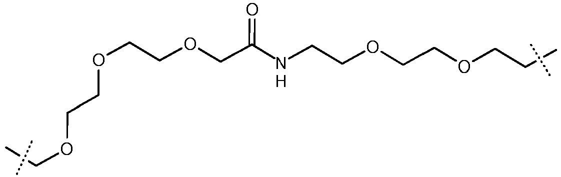

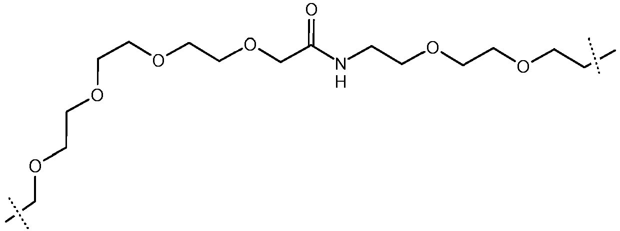

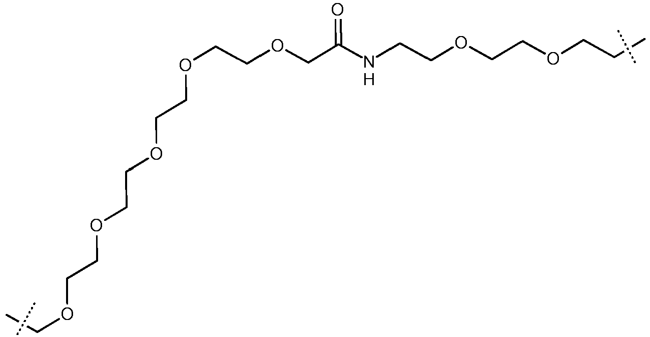

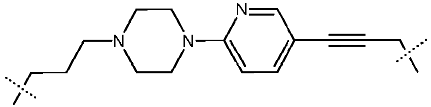

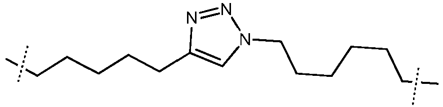

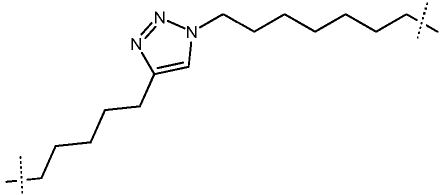

It is a particular embodiment, PE2fa, of PE2f in which
- Q²: is selected from the group consisting of

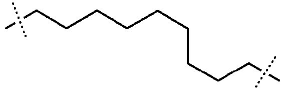

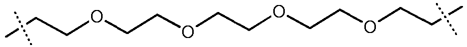

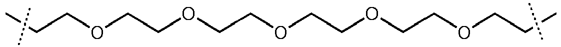

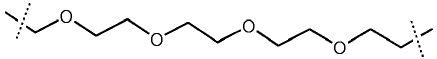

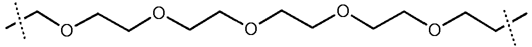

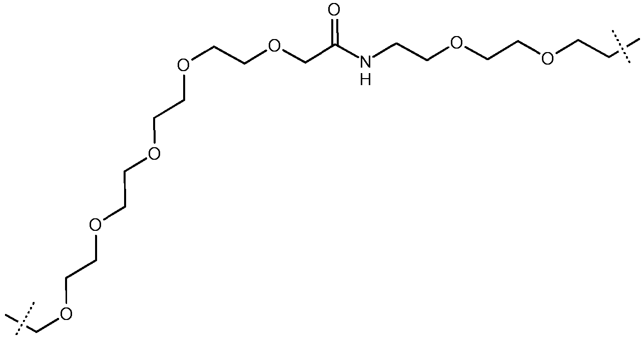

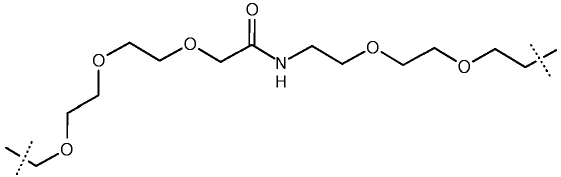

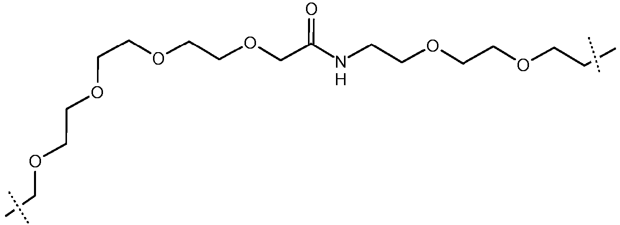

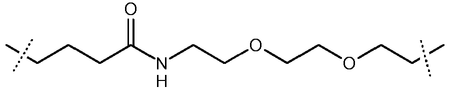

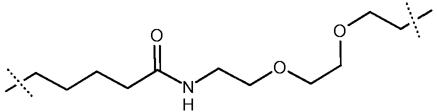

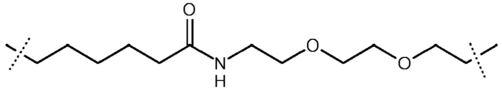

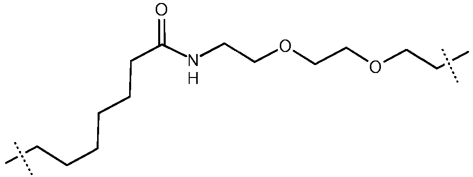

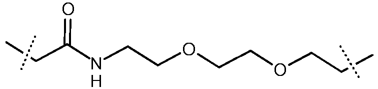

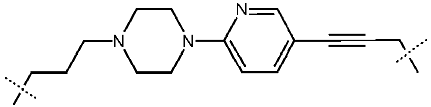

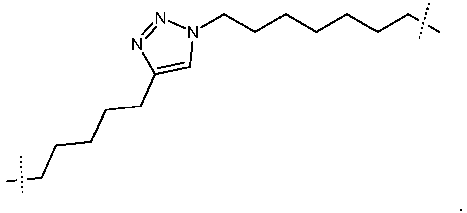

In another particular embodiment, PE2g, of PE2
Q² is a divalent linker formed by an unbranched alkylene chain having 2-25 C atoms, in which 1-8 non-adjacent CH₂ groups may independently from each other be replaced by O, -C(=O)-NH-, -NH-C(=O)-, -C(=O)-N(CH₃)-, -N(CH₃)-C(=O)-, and/or -C=C-; in which one or two CH₂ groups may optionally bear a methyl substituent; and in which one CH₂ group may independently from each other optionally be replaced by a moiety selected from the group consisting of 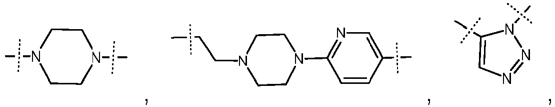 and/or

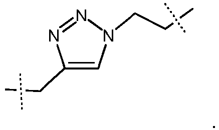

In another particular embodiment, PE2h, of PE2,
- Q²: is selected from the group consisting of

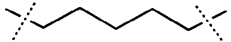

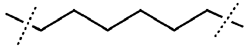

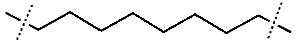

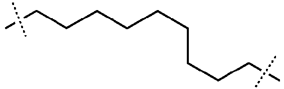

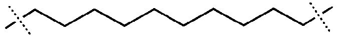

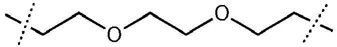

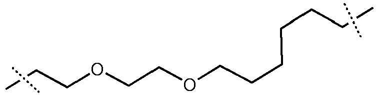

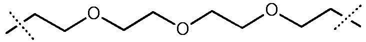

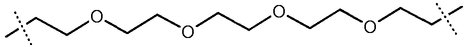

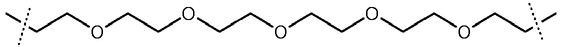

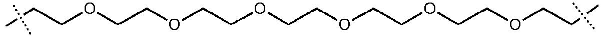

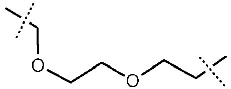

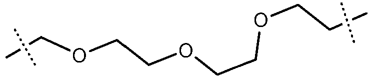

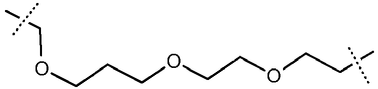

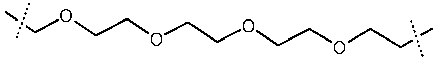

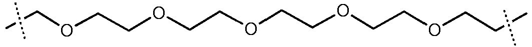

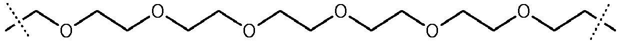

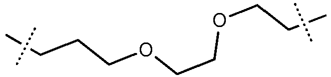

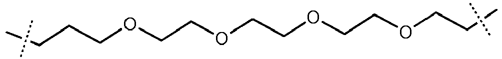

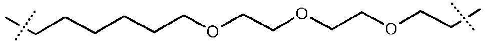

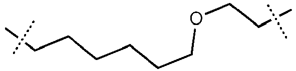

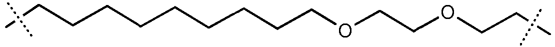

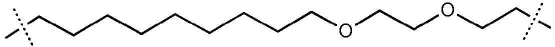

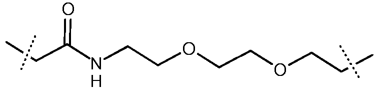

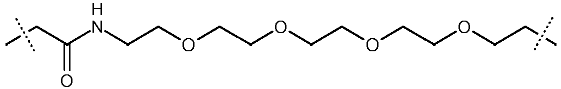

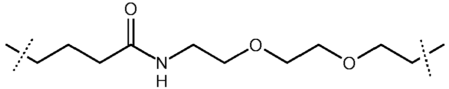

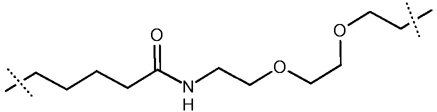

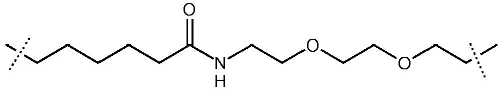

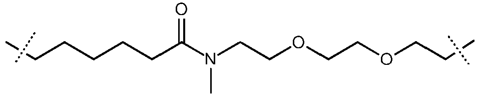

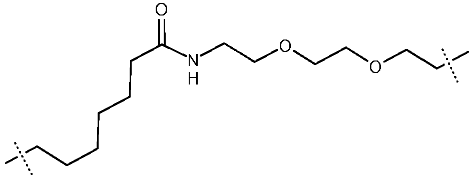

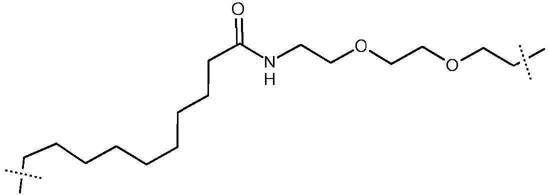

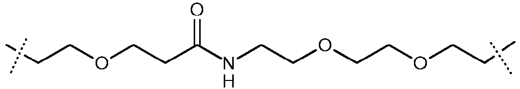

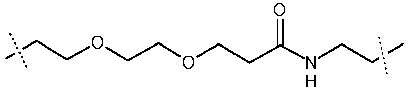

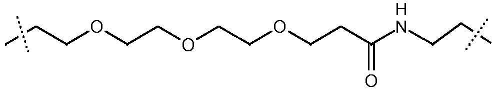

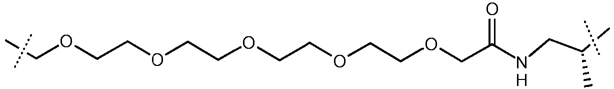

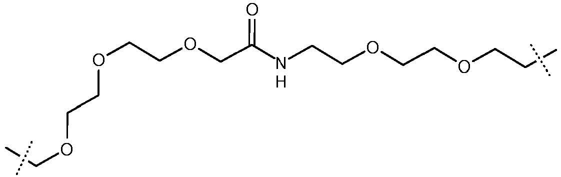

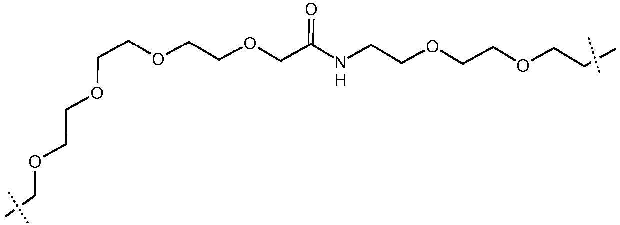

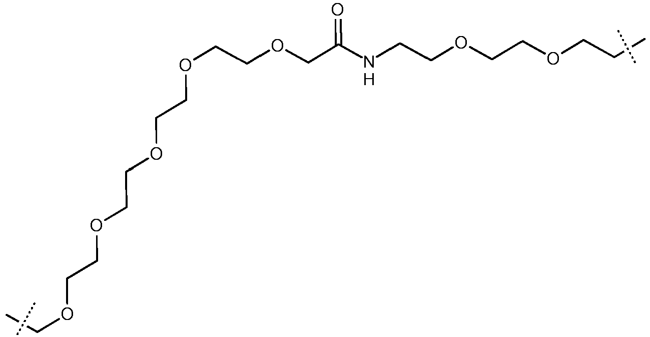

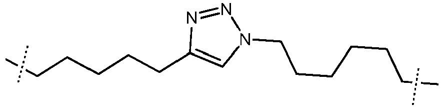

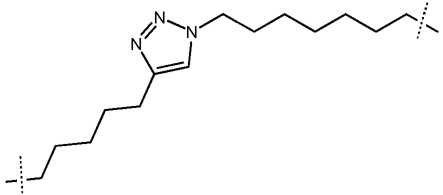

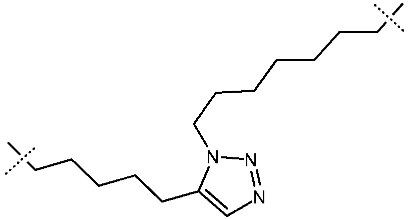

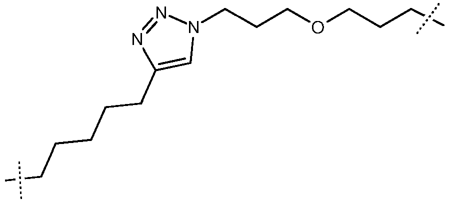

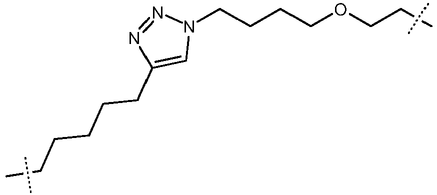

It is a particular embodiment, PE2ha, of PE2h in which
- Q²: is selected from the group consisting of

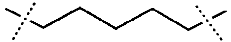

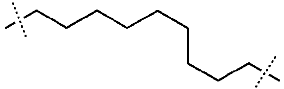

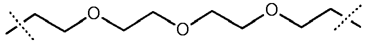

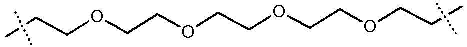

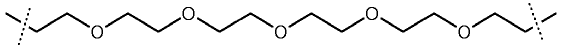

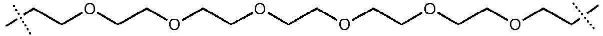

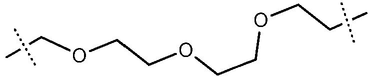

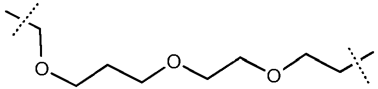

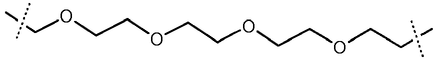

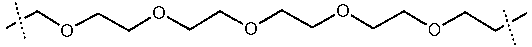

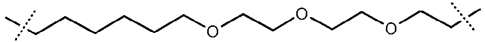

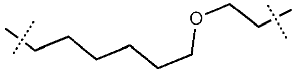

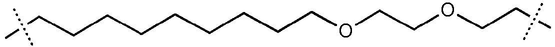

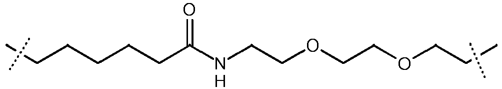

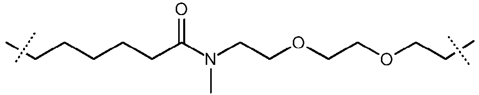

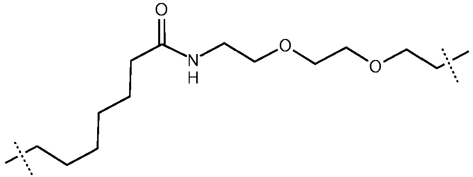

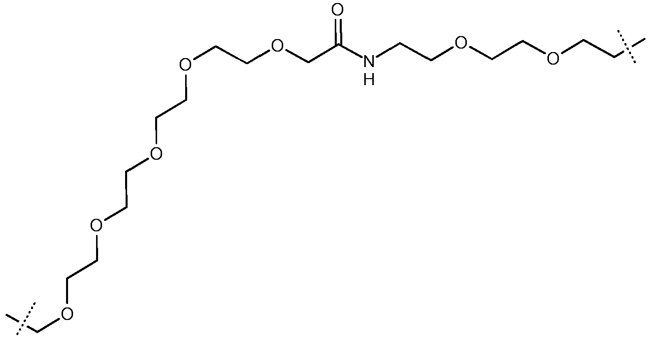

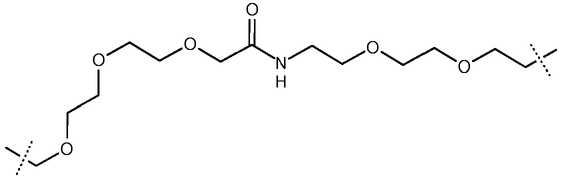

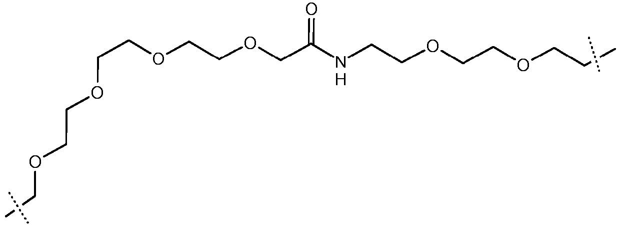

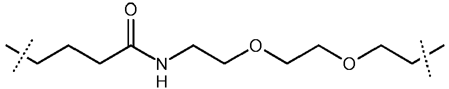

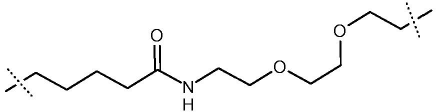

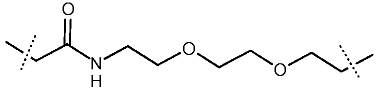

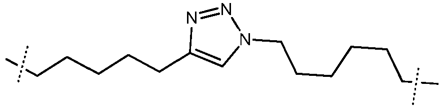

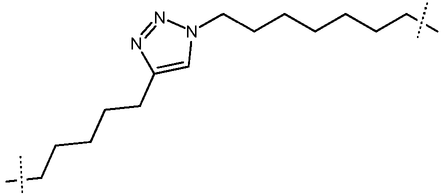

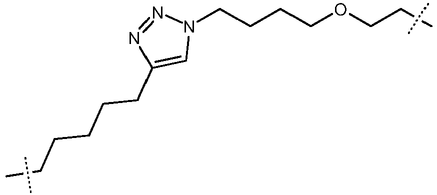

In yet another particular embodiment, PE2-0, of the invention
Q² is absent thereby forming a compound of formula I-A

Q¹-Q³ I-A,

in which the ubiquitin ligase ligand Q¹ and the TEAD-binding moiety Q² are directly linked to each other, i.e. not separated by a suitable linker Q².

In a further particular embodiment, PE4, the compound of the present invention is a compound of formula I, or a pharmaceutically acceptable salt, solvate, tautomer and/or stereoisomer thereof, wherein
- Q³: has a structure of formula Q3-I 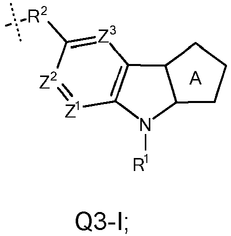
and the remaining radicals and residues are as defined for formula I above or for any of the further particular embodiments described herein above or below.

In still another particular embodiment, PE4ad, of PE4, Ring A ist selected from the group consisting of

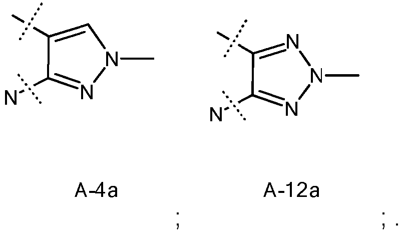

In a further particular embodiment of the invention, PE6, the compound of the present invention is a compound of formula I, or a pharmaceutically acceptable salt, solvate, tautomer and/or stereoisomer thereof, wherein
Q³ has a structure of formula Q3-I
   wherein
Ring A represents a five-membered heteroaromatic ring selected from the group consisting of the following ring moieties: 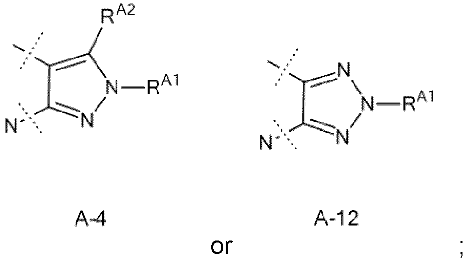 wherein
R^{A1} represents methyl ;
R^{A2} represents H;
Z¹ is CH;
Z² is CH;
Z³ is CH;
R¹ represents Ar¹, Hetar¹, Cyc¹, Hetcyc¹wherein
Ar¹ is a mono- or bicyclic aryl with 6 or 10 ring carbon atoms, wherein that aryl may be unsubstituted or substituted with substituents R^{B1}, R^{B2} and/or R^{B3} which may be the same or different;
Hetar¹ is a monocyclic heteroaryl with 5 or 6 ring atoms or a bicyclic heteroaryl with 9 or 10 ring atoms wherein 1, 2 or 3 of said ring atoms is/are a hetero atom(s) selected from N, O and/or S and the remaining are carbon atoms, wherein that heteroaryl may be unsubstituted or substituted with substituents R^{B1}, R^{B2} and/or R^{B3} which may be the same or different; preferably the heteroaryl is unsubstituted or substituted with substituents R^{B1} and/or R^{B2} which may be the same or different;
Cyc¹ is a saturated or partially unsaturated, mono- or bicyclic carbocycle with 3, 4, 5, 6, 7 or 8 ring carbon atoms, wherein that carbocycle may be unsubstituted or substituted with R^{B4}, R^{B5} and/or R^{B6} which may be the same or different;
Hetcyc¹ is a saturated or partially unsaturated, monocyclic heterocycle with 5 or 6 ring atoms wherein 1 or 2 of said ring atoms is/are a hetero atom(s) selected from N, O and/or S and the remaining are carbon atoms, wherein that heterocycle may be unsubstituted or substituted with R^{B4}, R^{B5} and/or R^{B6} which may be the same or different, wherein, if one of the heteroatoms is S, then that heterocycle may also be substituted with R^{B4}, R^{B5}, R^{B6}, R^{B7} and/or R^{B8};
R^{B1}, R^{B2}, R^{B3} represent independently from each other straight-chain or branched C₁₋₆-alkyl, which C₁₋₆-alkyl may be unsubstituted or monosubstituted with -CN or substituted with 1, 2 or 3 halogen, straight-chain or branched C₁₋₄-alkoxy, which C₁₋₄-alkoxy may be unsubstituted or substituted with 1, 2 or 3 halogen, -O-CH-C=CH, straight-chain or branched -S-C₁₋₄-alkyl, which -S-C₁₋₄-alkyl may be unsubstituted or substituted with 1, 2 or 3 halogen, F, Cl, Br, -CN, - S(=O)-C₁₋₃-alkyl, S(=O)₂-C₁₋₃-alkyl, -N(C₁₋₃-alkyl)₂, Ar², -CH₂-Ar², Hetar², Cyc², Hetcyc²; or two adjacent R^{B1}, R^{B2} and/or R^{B3} form together a divalent -C₃₋₄-alkylene radical in which one of the alkylene carbon units may be replaced by a carbonyl unit (-C(=O)-), or a divalent -O-C₂₋₃-alkylene radical;
Ar² is phenyl;
Hetar² is a monocyclic heteroaryl with 5 or 6 ring atoms wherein 1, 2, 3, 4, 5 of said ring atoms is/are a hetero atom(s) selected from N, O and/or S and the remaining are carbon atoms;
Cyc² is cyclopropyl, cyclobutyl, cyclopentyl, each of which may be unsubstituted or substituted with one or two substituents independently from each other selected from OH, F, Cl, Br, C₁₋₄-alkyl and C₁₋₄-alkoxy, wherein that C₁₋₄-alkyl or C₁₋₄-alkoxy group may be substituted with 1, 2 or 3 F atoms and/or 1 hydroxy group;
Hetcyc² is pyrrolidinyl, piperidinyl, each of which may unsubstituted or substituted with one or two substituents independently from each other selected from OH, F, Cl, Br, C₁₋₄-alkyl and C₁₋₄-alkoxy, wherein that C₁₋₄-alkyl or C₁₋₄-alkoxy group may be substituted with 1, 2 or 3 F atoms and/or 1 hydroxy group;
R^{B4}, R^{B5}, R^{B6} represent independently from each other F, C₁₋₂-alkyl, which C₁₋₂-alkyl may be unsubstituted or substituted with 1, 2 or 3 F, C₁₋₂-alkoxy,phenyl; or
two of R^{B4}, R^{B5}, R^{B6} are attached to the same carbon atom of said carbocycle Cyc¹ or said heterocycle Hetcyc¹ and form a divalent oxo (=O) group; or
R^{B4} and R^{B5} and R^{B7} and R^{B8} are attached to the same sulfur atom of said heterocycle and form two divalent oxo (=O) groups thereby forming an -S(=O)₂- moiety;
halogen is F, Cl, Br;
and the remaining radicals and residues are as defined for formula I above or for any of the further particular embodiments described herein above or below.

In another particular embodiment, PE6a, of PE6
R¹ represents Ar¹, Hetar¹, Cyc¹, Hetcyc¹; wherein
Ar¹ is phenyl or naphthalenyl, in particular phenyl, which may be unsubstituted or substituted with substituents R^{B1} and or R^{B2} which may be the same or different;
Hetar¹ is a monocyclic heteroaryl with 5 or 6 ring atoms or a bicyclic heteroaryl with 9 or 10 ring atoms wherein 1, 2 or 3 of said ring atoms is/are a hetero atom(s) selected from N, O and/or S and the remaining are carbon atoms, wherein that heteroaryl may be unsubstituted or substituted with substituents R^{B1} and/or R^{B2} which may be the same or different;
Cyc¹ is a saturated or partially unsaturated, mono- or bicyclic carbocycle with 3, 4, 5, 6, 7 or 8 ring carbon atoms, wherein that carbocycle may be unsubstituted or substituted with R^{B4} and/or R^{B5} which may be the same or different;
Hetcyc¹ is a saturated monocyclic heterocycle with 5 or 6 ring atoms wherein 1 of said ring atoms is a hetero atom selected from O and S and the remaining are carbon atoms, wherein that heterocycle may be unsubstituted or substituted with R^{B8} and/or R^{B9} which may be the same or different, wherein, if one of the heteroatoms is S, then that heterocycle may also be substituted with R^{B4}, R^{B5}, R^{B7} and R^{B8};
R^{B1}, R^{B2} represent independently from each other straight-chain or branched C₁₋₆-alkyl, which C₁₋₆-alkyl may be unsubstituted or monosubstituted with -CN or substituted with 1, 2 or 3 halogen, e.g. -CF₃, straight-chain or branched C₁₋₄-alkoxy, which C₁₋₄-alkoxy may be unsubstituted or substituted with 1, 2 or 3 halogen, e.g. -OCF₃, - O-CH-C=CH, straight-chain or branched -S-C₁₋₄-alkyl, which -S-C₁₋₄-alkyl may be unsubstituted or substituted with 1, 2 or 3 halogen, F, Cl, Br, -CN, -S(=O)-C₁₋₃-alkyl, S(=O)₂-C₁₋₃-alkyl, -N(C₁₋₃-alkyl)₂, Ar², - CH₂-Ar², Hetar², Cyc², Hetcyc²; or two adjacent R^{B1}, R^{B2} form together a divalent -C₃₋₄-alkylene radical in which one of the alkylene carbon units may be replaced by a carbonyl unit (-C(=O)-), or a divalent -O-C₂₋₃-alkylene radical;
Ar² is phenyl;
Hetar² is a monocyclic heteroaryl with 5 ring atoms wherein 1 of said ring atoms is N and the remaining are carbon atoms or 1 of said ring atoms is N and 1 of said ring atoms is S and the remaining are carbon atoms;
Cyc² is cyclopropyl, 1-trifluoromethylcyclopropyl, cyclopentyl;
Hetcyc² is pyrrolidinyl;
R^{B4}, R^{B5} represent independently from each other F, C₁₋₂-alkyl, which C₁₋₂-alkyl may be unsubstituted or substituted with 1, 2 or 3 F, C₁₋₂-alkoxy, phenyl; or
R^{B4} and R^{B5} are attached to the same carbon atom of said carbocycle Cyc¹ or said heterocycle Hetcyc¹ and form a divalent oxo (=O) group; or
R^{B4} and R^{B5} and R^{B7} and R^{B8} are attached to the same sulfur atom of said heterocycle and form two divalent oxo (=O) groups thereby forming an -S(=O)₂- moiety;
halogen is F, Cl, Br;
and the remaining radicals and residues are as defined for formula I above or for any of the further particular embodiments described herein above or below.

In still another particular embodiment, PE6aa, of PE6 or PE6a,
R¹ represents phenyl, 3-fluorophenyl, 4-fluorophenyl, 4-chlorophenyl, 4-methylphenyl, 4-ethylphenyl, 4-difluoromethylphenyl, 3-trifluoromethylphenyl, 4-trifluoromethylphenyl, 4-(1,1-difluorethyl)phenyl, 4-(2,2,2-trifluorethyl)phenyl, 4-(1-trifluoromethylcyclopropyl)-phen-1-yl, 4-cyclopentylphenyl, 4-ethoxyphenyl, 4-difluormethoxyphenyl, 4-trifluoromethoxyphenyl, 3-(trifluoromethyl)sulfanylphenyl, 4-(trifluoromethyl)sulfanylphenyl, 3-trifluoromethyl-4-methylphenyl, 2-fluoro-4-trifluoromethylphenyl, 2-fluoro-4-trifluoromethoxyphenyl, 3-fluoro-4-(n-propyl)phenyl, 2,3-dimethyl-4-methoxyphenyl, 6-fluoronaphth-2-yl; 5-trifluoromethylfuran-2-yl; 5-trifluoromethylthiophen-2-yl, 2-trifluoromethyl-1,3-thiazol-4-yl, 3-fluoropyridin-2-yl, 6-methylpyridin-3-yl, 6-methoxypyridin-3-yl, 3-ethylpyridin-2-yl, 6-ethylpyridin-3-yl, 4-difluoromethylpyridin-2-yl, 4-trifluoromethylpyridin-2-yl, 4-trifluoro-methoxypyridin-2-yl, 4-cyanopyridin-2-yl, 5-trifluoromethylpyridin-2-yl, 6-trifluoromethylpyridin-2-yl, 6-trifluoromethylpyridin-3-yl (2-trifluoromethylpyridin-5-yl), 6-trifluoromethoxypyridin-3-yl (2-trifluoromethoxypyridin-5-yl), 5-cyanopyridin-2-yl, 5-cyano-methylpyridin-2-yl, 5-methanesulfonylpyridin-2-yl, 6-methoxypyridin-2-yl, 4-methylpyrimidin-2-yl, 4-ethylpyrimidin-2-yl, 4-methylsulfanylpyrimidin-2-yl, 5-cyclopropylpyrimidin-2-yl, 5-ethyl-pyrimidin-2-yl, 5-difluoromethylpyrimidin-2-yl, 5-trifluoromethylpyrimidin-2-yl, 5-cyanopyrimidin-2-yl, 5-cyano-3-fluoropyridin-2-yl, 5-cyano-6-methylpyridin-2-yl, 3-fluoro-5-(trifluoromethyl)pyridin-2-yl, 5-oxo-5H,6H,7H-cyclopenta[b]pyridin-2-yl, 5,6,7,8-tetrahydroquinolin-2-yl, 5-oxo-5,6,7,8-tetrahydroquinolin-2-yl, 5H,6H,7H-cyclopenta[b]pyridin-2-yl, quinolin-2-yl, isoquinolin-3-yl, 6-methylquinolin-2-yl, 8-methoxyquinolin-4-yl, furo[3,2-b]pyridin-5-yl, quinazolin-2-yl, 6-fluoroquinazolin-2-yl, 1,5-naphthyridin-2-yl; 3-methylcyclobutyl, cyclopentyl, 3-methylcyclopentyl, 3,3-dimethylcyclopentyl, 3-trifluoromethyl-bicyclo[1.1.1]petan-1-yl, cyclohexyl, 4-methylcyclohexyl, 4-(trifluoromethyl)cyclohexyl, 4,4-difluorocyclohexyl, cyclohex-1-enyl, 2-oxocycloheptyl, 6,6-difluorospiro[3.3]heptan-2-yl, 1H-inden-2-yl;
and the remaining radicals and residues are as defined for formula I above or for any of the further particular embodiments described herein above or below.

In particular embodiment PE6aaa of PE6, PE6a or PE6aa R¹ is selected from the group consisting of 4-methylphenyl, 4-difluoromethylphenyl, 4-trifluormethylphenyl, 4-fluorophenyl; in particular 4-trifluormethylphenyl (PE6aaaa).

In still another particular embodiment of the invention, PE6b, of PE6 the compound of the present invention is a compound of formula I, or any solvate, tautomer or stereoisomer thereof and/or any pharmaceutically acceptable salt of each of the foregoing, including mixtures thereof in all ratios, wherein
R¹ is selected from the group consisting of 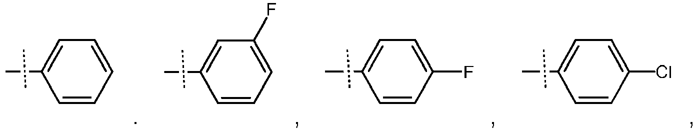 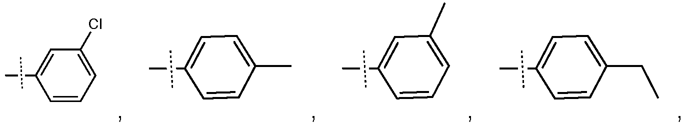 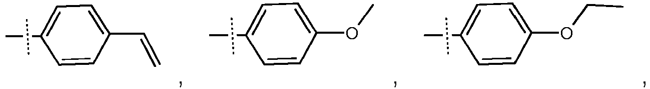 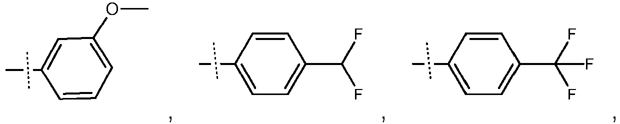 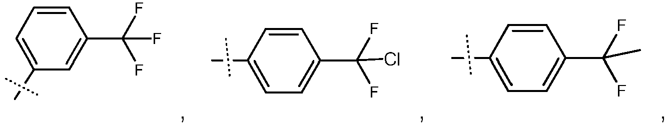 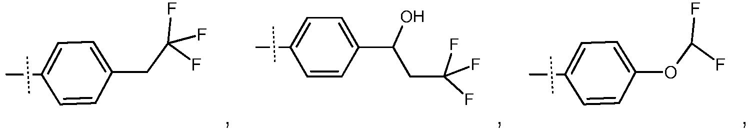 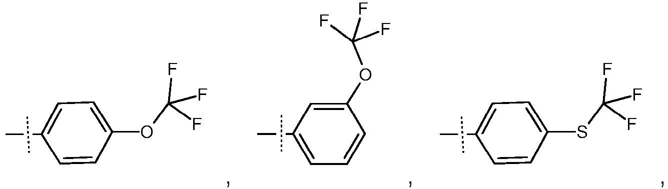 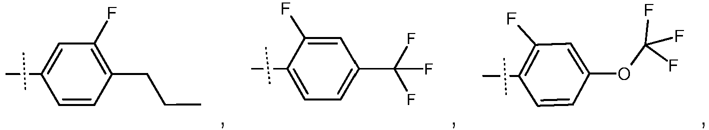 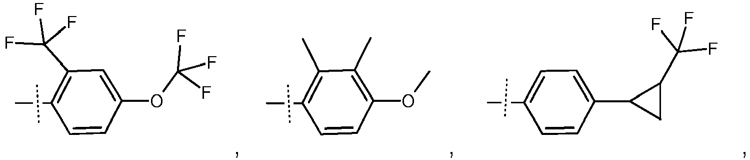 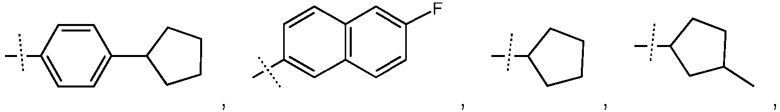 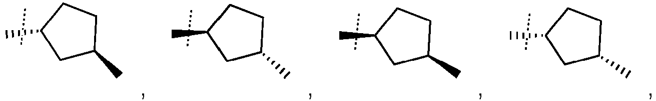 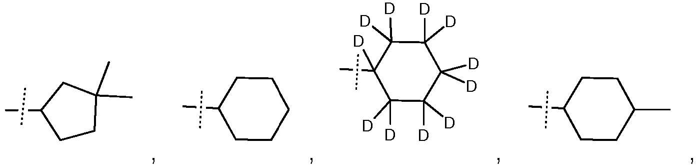 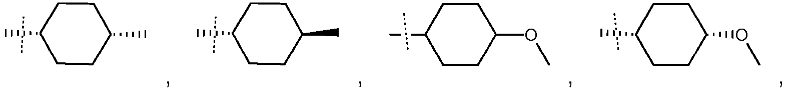 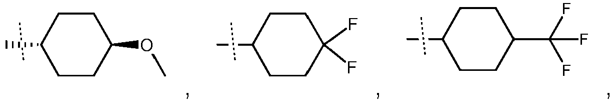 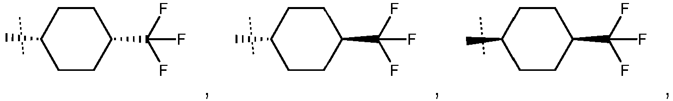 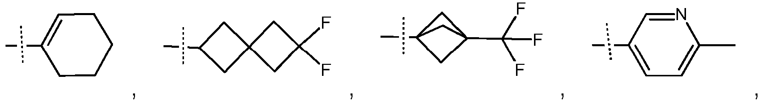 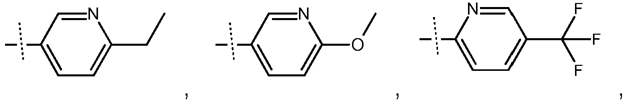 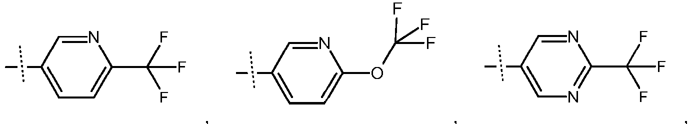 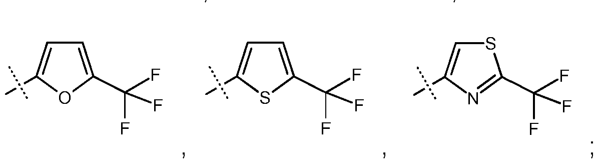 and the remaining radicals and residues are as defined for formula I above or for any of the further particular embodiments described herein above or below.

In a particular embodiment, PE6ba, of PE6b
R¹ is selected from the group consisting of 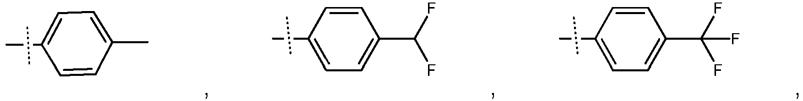 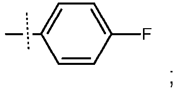 and the remaining radicals and residues are as defined for formula I above or for any of the further particular embodiments described herein above or below. Especially, R¹ is 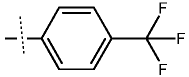 (particular embodiment PE6baa).

In still another particular embodiment, PE6baaa, of PEb, PE6ba, and/or PE6baa, additionally
Z¹, Z² and Z³ each represent CH in formula Q3-I or Q3-II.

Yet another particular embodiment, PE7, of the invention is a compound of formula I

Q¹-Q²-Q³ I

wherein
Q¹ is a
(a) CRBN (cereblon) ligand; and
the CRBN (cereblon) ligand has a structure selected from the structures of formulas Q1-I-1, Q1-I-2, Q1-I-3; Q1-I-4, Q1-I-5, Q1-I-6, Q1-II-1, Q1-II-2, Q1-II-3, Q1-II-4, Q1-II-5, and Q1-VII-1 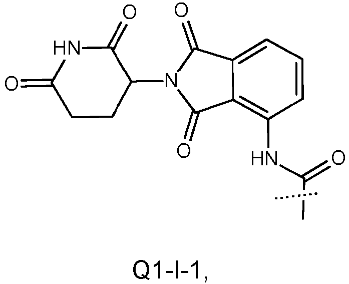 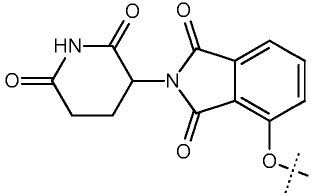 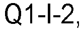 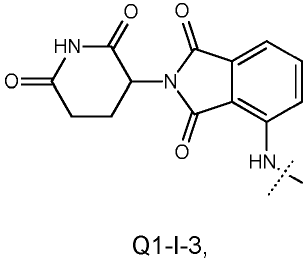 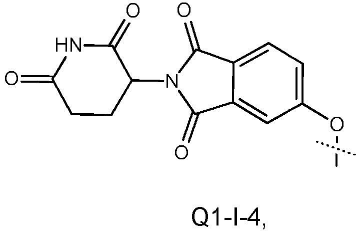 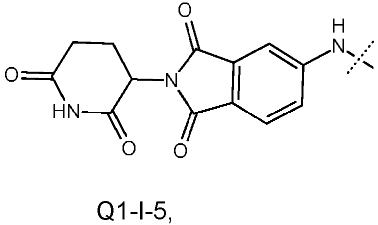 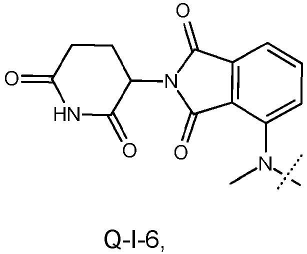 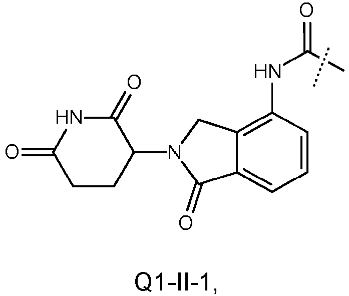 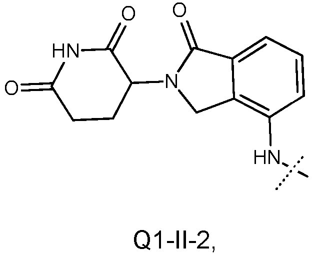 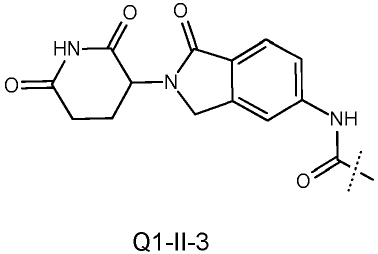 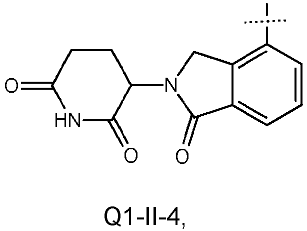 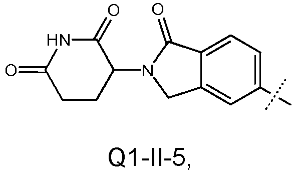 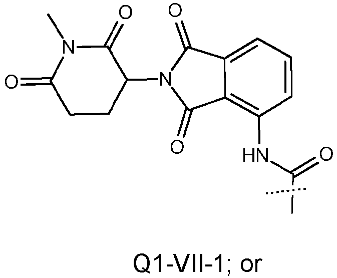
(b) a VHL (von Hippel-Lindau) ligand; and the VHL ligand has a structure selected from the structures of of formula Q1-III-1, Q1-III-2, Q1-III-4, and Q1-III-5: 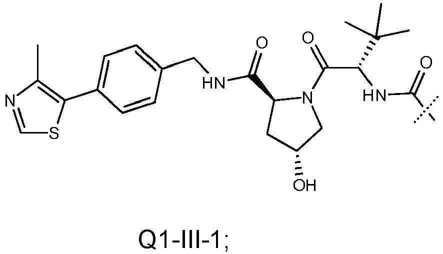 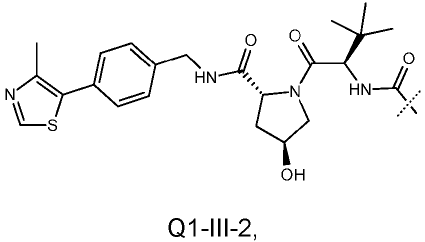 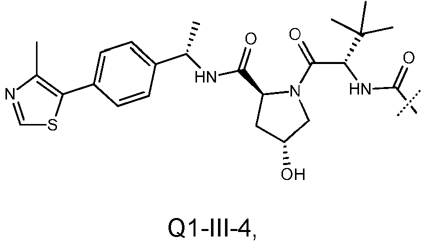 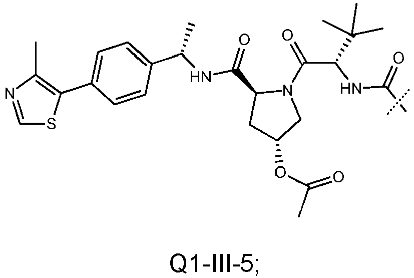
   - Q²: is selected from the group consisting of 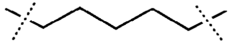 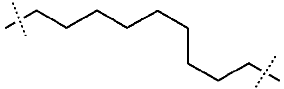 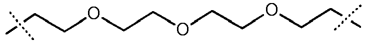 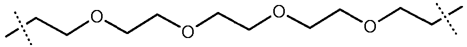 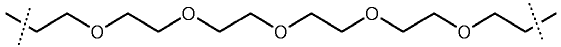 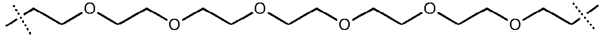 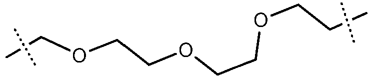 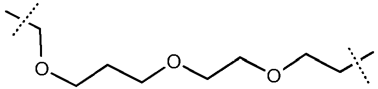 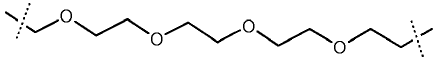 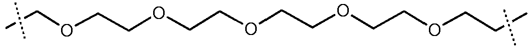 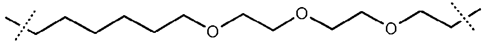 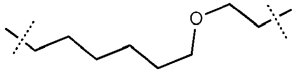 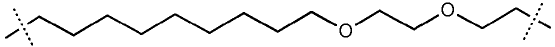 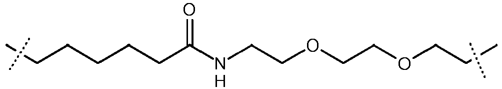 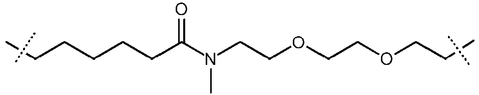 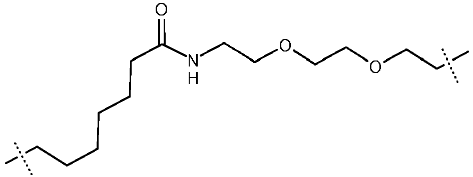 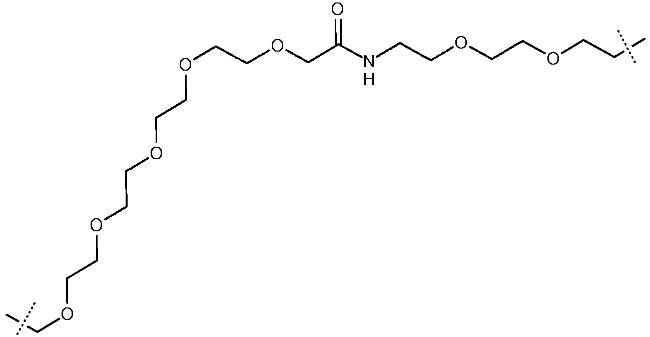 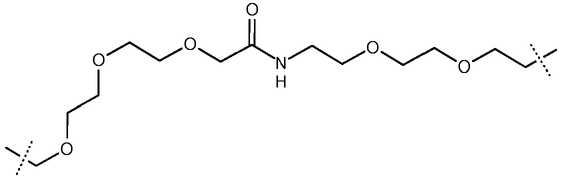 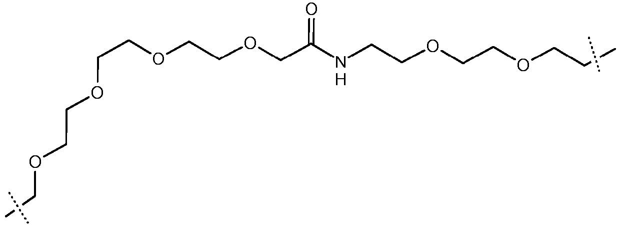 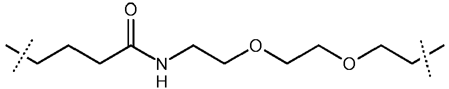 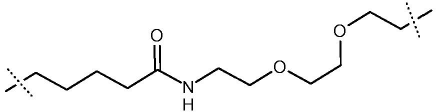 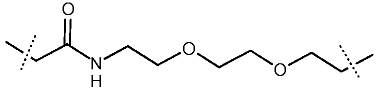 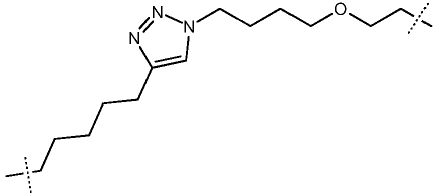 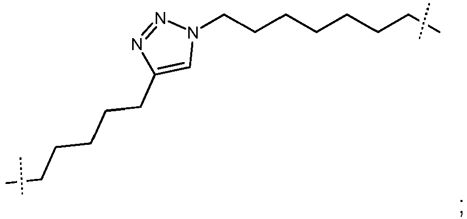
   - Q³: has a structure of formula Q3-I 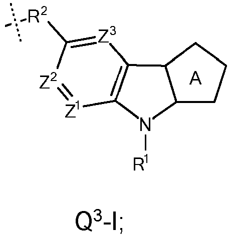
   - Ring A: is ring A-4 or ring A-12 as defined above;
   - R^{A1}: represents methyl;
   - R^{A2}: represents hydrogen;
   - R¹: denotes 4-trifluoromethylphenyl;
   - R²: represents (-NH-C(=O)-); 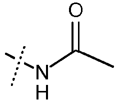
   - Z¹, Z² and Z³: each represent CH in formula Q3-I.
or a pharmaceutically acceptable salt, solvate, tautomer and/or stereoisomer thereof.

It is a particular embodiment, PE7a, of PE7 in which
Q¹ is a (a) CRBN ligand and has a structure selected from the structures of formula Q1-I-2, Q1-I-3, Q1-I-5, Q1-1-6, Q1-II-2, Q1-II-3, Q1-II-4, and Q1-II-5; or is a (b) VHL ligand and has structure selected from the structures of formula Q1-III-1, and Q1-III-4.

In still another particular embodiment, PE8, the compound of the present invention is a tricyclic heterocycle selected from the compounds shown in Table 1 below including compounds nos. 1 to 122, or a pharmaceutically acceptable salt, solvate, tautomer and/or stereoisomer thereof. In yet another particular embodiment, PE8a, of PE8, the compound is selected from Table 1 and is a compound of formula I as described hereinabove and in the claims. It is understood that each single compound depicted in Table 1 as well as any a pharmaceutically acceptable salt, solvate, tautomer and/or stereoisomer of such compound represents a particular embodiment of the present invention. In yet a further particular embodiment, PE8b, of PE8 or PE8a, the compound is selected from Table 1, is a compound of formula I as described hereinabove and in the claims, and is within Group A or Group B in the SK-HEP1 TEAD degradation assay and/or within Group A or Group B in the NCI-H226 TEAD degradation assay as provided in Table 4a below.

As used herein, the following definitions shall apply unless otherwise indicated or defined specifically elsewhere in the description and/or the claims for specific substituents, radicals, residues, groups or moieties.

The term "ubiquitin ligase ligand" refers to a structural moiety or compound capable of binding to any kind of a ubiquitin ligase. The term "E3 ubiquitin ligase ligand" refers to a structural moiety or compound capable of binding to an E3 ubiquitin ligase.

The term "aliphatic" or "aliphatic group", as used herein, means a straight-chain (i.e., unbranched) or branched, substituted or unsubstituted hydrocarbon chain that is completely saturated or that contains one or more units of unsaturation, or a monocyclic hydrocarbon or bicyclic hydrocarbon or tricyclic hydrocarbon that is completely saturated or that contains one or more units of unsaturation, such as one or more C=C double bond(s) and/or C≡C triple bond(s), but which is not aromatic (also referred to herein as "carbocycle", "cycloaliphatic" or "cycloalkyl"), that has - in general and if not defined otherwise in this specification or the accompanied claims - a single point of attachment to the rest of the molecule. Unless otherwise specified, aliphatic groups contain 1 to 10 (i.e., 1, 2, 3, 4, 5, 6, 7, 8, 9, or 10) 1 to 8 (i.e., 1, 2, 3, 4, 5, 6, 7, or 8) or 1 to 6 (i.e., 1, 2, 3, 4, 5, or 6) aliphatic carbon atoms ("C₁₋₁₀-aliphatic", "C₁₋₈-aliphatic" and "C₁₋₆-aliphatic", respectively). In some embodiments, aliphatic groups contain 1-5 (i.e., 1, 2, 3, 4, or 5) aliphatic carbon atoms ("C₁₋₅-aliphatic"). In other embodiments, aliphatic groups contain 1-4 (i.e., 1, 2, 3, or 4) aliphatic carbon atoms ("C₁₋₄-aliphatic"). In still other embodiments, aliphatic groups contain 1-3 (i.e., 1, 2, or 3) aliphatic carbon atoms ("C₁₋₃-aliphatic"), and in yet other embodiments, aliphatic groups contain 1-2 aliphatic carbon atoms ("C₁₋₂-aliphatic"). In some embodiments, "cycloaliphatic" ("cycloalkyl") refers to a monocyclic C₃-C₇ hydrocarbon (i.e., a monocyclic hydrocarbon with 3, 4, 5, 6, or 7 ring carbon atoms) or to a bicyclic C₅₋₈ hydrocarbon (i.e. a bicyclic hydrocarbon with 5, 6, 7, or 8 ring carbon atoms) that is completely saturated or that contains one or more units of unsaturation, but which is not aromatic, that has a single point of attachment to the rest of the molecule. In another embodiment the term "cycloaliphatic" or "carbocycle" refers to a monocyclic or bicyclic cycloaliphatic ring system which is fused to an aromatic, heteroaromatic or heterocyclic ring or ring system via 2 adjacent ring atoms of that aromatic, heteroaromatic or heterocyclic ring or ring system; in other words, such carbocycle shares two ring atoms with the ring or ring system to which it is fused thereby having two points of attachment to the rest of the molecule. In another embodiment the term "carbocycle" refers to bicyclic spiro-cycles in which two monocyclic carbocycles are fused to each other via the same single carbon atom. In general, the term "aliphatic" encompasses, to the extent chemically possible, straight-chain, i.e. unbranched, as well as branched hydrocarbon chains, if not defined differently in a particular instance. Also, in general this term encompasses, to the extent chemically possible, unsubstituted and substituted hydrocarbon moieties, if not defined differently in a particular instance. Typical substituents of an aliphatic group include, but are not limited to halogen, in particular F, cyano, hydroxy, alkoxy, unsubstituted or mono- or di-substituted amino, aryl, in particular unsubstituted or substituted phenyl, heteroaryl, in particular unsubstituted or substituted pyridyl or pyrimidinyl, heterocyclyl, in particular unsubstituted or substituted pyrrolidinyl, piperidinyl, piperazinyl or morpholinyl. Suitable aliphatic groups include, but are not limited to, linear or branched, substituted or unsubstituted alkyl, alkenyl, alkynyl, cycloalkyl, cycloalkenyl groups and hybrids thereof as (cycloalkyl)alkyl, (cycloalkenyl)alkyl or (cycloalkyl)alkenyl.

The term "alkyl" usually refers to a saturated aliphatic and acyclic moiety, while the term "alkenyl" usually refers to an unsaturated aliphatic and acyclic moiety with one or more C=C double bonds and the term "alkynyl" usually refers to an aliphatic and acyclic moiety with one or more C≡C triple bonds. It is understood that the term "alkenyl" comprises all forms of isomers, i.e. E-isomers, Z-isomers as well as mixtures thereof (E/Z-isomers). Exemplary aliphatic groups are linear or branched, substituted or unsubstituted C₁₋₁₀-alkyl, C₁₋₈-alkyl, C₁₋₆-alkyl, C₁₋₄-alkyl, C₁₋₃-alkyl, C₁₋₂-alkyl, C₂₋₈-alkenyl, C₂₋₆-alkenyl, C₂₋₈-alkynyl, C₂₋₆-alkynyl groups and hybrids thereof such as (cycloalkyl)alkyl, (cycloalkenyl)alkyl or (cycloalkyl)alkenyl.

In particular, the term "C₁₋₃-alkyl" refers to alkyl groups, i.e. saturated acyclic aliphatic groups, having 1, 2 or 3 carbon atoms. Exemplary C₁₋₃-alkyl groups are methyl, ethyl, propyl and isopropyl. The term "C₁₋₄-alkyl" refers to alkyl groups having 1, 2, 3 or 4 carbon atoms. Exemplary C₁₋₄-alkyl groups are methyl, ethyl, propyl, isopropyl, butyl, isobutyl, and tert-butyl. The term "C₁₋₆-alkyl" refers to alkyl groups having 1, 2, 3, 4, 5 or 6 carbon atoms. Exemplary C₁₋₆-alkyl groups are methyl, ethyl, propyl, isopropyl, butyl, isobutyl, tert-butyl, n-pentyl, 2-pentyl, n-hexyl, and 2-hexyl. The term "C₁₋₈-alkyl" refers to alkyl groups having 1, 2, 3, 4, 5, 6, 7, or 8 carbon atoms. Exemplary C₁₋₈-alkyl groups are methyl, ethyl, propyl, isopropyl, butyl, isobutyl, tert-butyl, n-pentyl, 2-pentyl, n-hexyl, 2-hexyl n-heptyl, 2-heptyl, n-octyl, 2-octyl, and 2,2,4-trimethylpentyl. The term "C₁₋₁₀-alkyl" refers to alkyl groups having 1, 2, 3, 4, 5, 6, 7, 8, 9, or 10 carbon atoms. Exemplary C₁₋₁₀-alkyl groups are methyl, ethyl, propyl, isopropyl, butyl, isobutyl, tert-butyl, n-pentyl, 2-pentyl, n-hexyl, 2-hexyl n-heptyl, 2-heptyl, n-octyl, 2-octyl, 2,2,4-trimethylpentyl, and n-decyl, Each of these alkyl groups may be straight-chain or - except for C₁-alkyl and C₂-alkyl - branched and may be unsubstituted or substituted with 1, 2 or 3 substituents that may be the same or different and may be, if not specified differently elsewhere in this specification and/or the accompanying claims, selected from the group comprising halogen, in particular F, hydroxy, alkoxy, unsubstituted or mono- or di-substituted amino, aryl, in particular unsubstituted or substituted phenyl, heteroaryl, in particular unsubstituted or substituted pyridyl or pyrimidinyl, heterocyclyl, in particular unsubstituted or substituted pyrrolidinyl, piperidinyl, piperazinyl or morpholinyl. Exemplary substituted alkyl groups are difluoromethyl, trifluoromethyl, 2,2,2-trifluoroethyl, hydroxymethyl, 2-hydroxyethyl.

In some instances the C₁₋₃-alkyl, C₁₋₄-alkyl, C₁₋₆-alkyl, C₁₋₈-alkyl, C₁₋₁₀-alkyl groups - both unbranched and branched - may also comprise those residues in which 1 or 2 of non-terminal and non-adjacent -CH₂-(methylene) groups are replaced by -O-, -S- and/or 1 or 2 non-terminal and non-adjacent -CH₂- or -CH- groups are replaced by -NH- or -N-. These replacements yield, for instance, (modified) alkyl groups like -CH₂-CH₂-O-CH₃, -CH₂-CH₂-CH₂-S-CH₃, CH₂-CH₂-NH-CH₂-CH₃, CH₂-CH₂-O-CH₂-CH₂-O-CH₃, CH₂-CH₂-O-CH₂-CH₂-O-CH₂-CH₃, CH₂-CH₂-N(CH₃)-CH₂-CH₃, and the like. Further and/or different replacements of -CH- and -CH₂- groups may be defined for specific alkyl substituents or radicals elsewhere in the description and/or the claims. As described for "unmodified" alkyl groups hereinabove these "modified" alkyl groups may optionally be substituted with 1, 2 or 3 substituents that may be the same or different and may be, if not specified differently elsewhere in this specification and/or the accompanying claims, selected from the group comprising halogen, in particular F, hydroxy, alkoxy, unsubstituted or mono- or di-substituted amino, aryl, in particular unsubstituted or substituted phenyl, heteroaryl, in particular unsubstituted or substituted pyridyl or pyrimidinyl, heterocyclyl, in particular unsubstituted or substituted pyrrolidinyl, piperidinyl, piperazinyl or morpholinyl. Examplary modified alkyl groups are CH₂-CH₂-O-CH₂-CH₂-O-CH₂-CH₂-NH₂, CH₂-CH₂-O-CH₂-CH₂-O-CH₂-CH₂-NH-C(=O)-CH₃, CH₂-CH₂-CH₂-CH₂-CH₂-O-CH₂-CH₂-NH₂, CH₂-CH₂-CH₂-CH₂-CH₂-O-CH₂-CH₂-NH-C(=O)-CH₃, CH₂-CH(OH)-CH₂-CH₂-O-CH₂-CH₂-O-CH₂-CH₂-NH₂, CHR-CH(OH)-CH₂-CH₂-O-CH₂-CH₂-O-CH₂-CH₂-NH₂ wherein "R" denotes another substituent.

The term "C₃₋₇-cycloalkyl" refers to a cycloaliphatic hydrocarbon, as defined above, with 3, 4, 5, 6 or 7 ring carbon atoms. Likewise, the term "C₃₋₆-cycloalkyl" refers to a cycloaliphatic hydrocarbon with 3, 4, 5, or 6 ring carbon atoms. The terms "cycloalkyl", "C₃₋₇-cycloalkyl" and "C₃₋₆-cycloalkyl" as used herein comprise cyclic hydrocarbons which are saturated or contain one or more units of unsaturation, such as a C=C double bond; such cyclic hydrocarbons having at least one unit of unsaturation may also referred to as "cycloalkenyl" group. C₃₋₇-cycloalkyl groups may be unsubstituted or substituted with - unless specified differently elsewhere in this specification - 1, 2 or 3 substituents that may be the same of different and are - unless specified differently elsewhere in this specification - selected from the group comprising C₁₋₆-alkyl, O-C₁₋₆-alkyl (alkoxy), halogen, hydroxy, unsubstituted or mono- or di-substituted amino, aryl, in particular unsubstituted or substituted phenyl. If substituted, C₃₋₇-cycloalkyl comprises all possible stereoisomers. Exemplary C₃₋₇-cycloalkyl groups are cyclopropyl, 2-methyl-cyclopropyl, cyclopropenyl, cyclobutyl, cyclobutenyl, cyclopentyl, cyclopentenyl, cyclohexyl, cyclohexenyl, cycloheptyl, cycloheptenyl. The term "bicyclic C₅₋₈-cycloalkyl" refers to a bicyclic cycloaliphatic hydrocarbon, as defined above, with 5, 6, 7, or 8 ring carbon atoms; it includes spirocyclic ring systems, i.e. ring systems in which the two carbocycles of the bicyclic C₅₋₈-cycloalkyl are attached to each other via the same carbon atom. Bicylic C₅₋₈-cycloalkyl groups may be unsubstituted or substituted with - unless specified differently elsewhere in this specification - 1, 2 or 3 substituents that may be the same of different and are - unless specified differently elsewhere in this specification - selected from the group comprising C₁₋₆-alkyl, O-C₁₋₆-alkyl (alkoxy), halogen, hydroxy, unsubstituted or mono- or di-substituted amino. If substituted, bicyclic C₅₋₈-cycloalkyl comprises all possible stereoisomers. Exemplary bicyclic C₅₋₈-cycloalkyl are spiro[3.3]heptanyl, bicyclo[2.2.1]heptan-2-yl, bicyclo[2.2.2]octan-2-yl, bicyclo[2.2.1]hept-5-en-2-ylmethyl, bicyclo[3.1.1]hept-2-en-2-yl.

The term "aliphatoxy" refers to saturated or unsaturated aliphatic groups or substituents as defined above that are connected to another structural moiety via an oxygen atom (-O-). The term "C₁₋₆-aliphatoxy" refers to an aliphatoxy radical with 1, 2, 3, 4, 5, or 6 carbon atoms within the aliphatic group. The term "alkoxy" refers to a particular subgroup of saturated aliphatoxy, i.e. to alkyl substituents and residues that are connected to another structural moiety via an oxygen atom (-O-). Sometimes, it is also referred to as "O-alkyl" and more specifically as "O-C₁₋₂-alkyl", "O-C₁₋₃-alkyl", "O-C₁₋₄-alkyl", "O-C₁₋₆-alkyl", "O-C₁₋₈-alkyl". Like the similar alkyl groups, it may be straight-chain or - except for -O-C₁-alkyl and -O-C₂-alkyl - branched and may be unsubstituted or substituted with 1, 2 or 3 substituents that may be the same or different and are, if not specified differently elsewhere in this specification, selected from the group comprising halogen, unsubstituted or mono- or di-substituted amino. Exemplary alkoxy groups are methoxy, fluoromethoxy, difluoromethoxy, trifluoromethoxy, ethoxy, 2,2,2-trifluoroethoxy, n-propoxy, iso-propoxy, n-butoxy, sec-butoxy, tert-butoxy, n-pentoxy.

The term "alkylene" refers to a divalent aliphatic group and in particular a divalent alkyl group. An "alkylene chain" is a polymethylene group, i.e., -(CH₂)ⱼ-, wherein j is a positive integer, preferably 1, 2, 3, 4, 5 or 6. In the context of the present invention "C₁₋₃-alkylene" refers to an alkylene moiety with 1, 2 and 3, respectively, -CH₂- groups; the term "alkylene", however, not only comprises linear alkylene groups, i.e. "alkylene chains", but branched alkylene groups as well. The term "C₁₋₆-alkylene" refers to an alkylene moiety that is either linear, i.e. an alkylene chain, or branched and has 1, 2, 3, 4, 5 or 6 carbon atoms. The term "C₂₋₆-alkylene" refers to an alkylene moiety with 2, 3, 4, 5, or 6 carbon atoms, while a "C₃₋₄-alkylene" refers to an alkylene moiety with 3 or 4 carbon atoms and "C₂₋₃-alkylene" refers to an alkylene moiety with 2 or 3 carbon atoms. A substituted alkylene is a group in which one or more methylene hydrogen atoms are replaced by (or with) a substituent. Suitable substituents include those described herein for a substituted alkyl group. In some instances 1 or 2 methylene groups of the alkylene chain may be replaced by, for instance, O, S and/or NH or N-C₁₋₄-alkyl. Exemplary alkylene groups are -CH₂-, - CH₂-CH₂-, -CH₂-CH₂-CH₂-CH₂-, -O-CH₂-CH₂-, -O-CH₂-CH₂-CH₂-, - CH₂-O-CH₂-CH₂-, -O-CH₂-O-, -O-CH₂-CH₂-O-, -O-CH₂-CH₂-CH₂-O-,-CH₂-NH-CH₂-CH₂-, -CH₂-N(CH₃)-CH₂-CH₂-.

The term "alkenylene" refers to a divalent alkenyl group. A substituted alkenylene chain is a polymethylene group containing at least one double bond in which one or more hydrogen atoms are replaced with a substituent. Suitable substituents include those described herein for a substituted aliphatic group. The term "alkenylene" not only refers to straight-chain divalent alkenylene radicals, i.e. an alkenylene chain, but to branched alkenylene groups as well. The term "C₂₋₆-alkenylene" refers to an alkenylene radical having 2, 3, 4, 5, or 6 carbon atoms.

The term "alkynylene" refers to a divalent alkynyl group. A substituted alkynylene chain is a polymethylene group containing at least one triple bond in which one or more hydrogen atoms are replaced with a substituent. Suitable substituents include those described herein for a substituted aliphatic group.

The term "halogen" means F, Cl, Br, or I.

The term "heteroatom" means one or more of oxygen (O), sulfur (S), or nitrogen (N), including, any oxidized form of nitrogen or sulfur, e.g. N-oxides, sulfoxides and sulfones; the quaternized form of any basic nitrogen or a substitutable nitrogen of a heterocyclic or heteroaromatic ring, for example N (as in 3,4-dihydro-2H-pyrrolyl), NH (as in pyrrolidinyl) or N-SUB with SUB being a suitable substituent (as in N-substituted pyrrolidinyl).

The term "aryl" used alone or as part of a larger moiety as in "aralkyl", "aralkoxy", or "aryloxyalkyl", refers to monocyclic, bicyclic and tricyclic ring systems having a total of five to fourteen ring members, that ring members being carbon atoms, wherein at least one ring in the system is aromatic, i.e., it has (4n+2) π (pi) electrons (with n being an integer selected from 0, 1, 2, 3), which electrons are delocalized over the system, and wherein each ring in the system contains three to seven ring members. Preferably, all rings in the aryl system or the entire ring system are aromatic. The term "aryl" is used interchangeably with the term "aryl ring". In certain embodiments of the present invention, "aryl" refers to an "aromatic ring system". More specifically, those aromatic ring systems may be mono-, bi- or tricyclic with 5, 6, 7, 8, 9, 10, 11, 12, 13, 14 ring carbon atoms. Even more specifically, those aromatic ring systems may be mono- or bicyclic with 6, 7, 8, 9, 10 ring carbon atoms. Exemplary aryl groups are phenyl, biphenyl, naphthyl, anthracyl and the like, which may be unsubstituted or substituted with one or more identical or different substituents. Also included within the scope of the terms "aryl" or "aromatic ring system", as they are used herein, is a group in which an aromatic ring is fused to one or more non-aromatic rings, such as indanyl, phthalimidyl, naphthimidyl, phenanthridinyl, or tetrahydronaphthyl, and the like. In the latter case the "aryl" group or substituent is attached to its pendant group via the aromatic part of the ring system.

The term "benzo" refers to a six-membered aromatic ring (with carbon ring atoms) that is fused via two adjacent carbon atoms to another ring, being it a cycloaliphatic, aromatic, heteroaromatic or heterocyclic (heteroaliphatic) ring; as a result a ring system with at least two rings is formed in which the benzo ring shares two common carbon atoms with the other ring to which it is fused. For example, if a benzo ring is fused to a phenyl ring, a napthaline ring system is formed, while fusing a benzo ring to a pyridine provides for either a quinoline or an isoquinoline; fusing a benzo ring to a cyclopentene ring provides an indene ring.

The terms "heteroaryl" and "heteroar-", used alone or as part of a larger moiety, e.g., "heteroaralkyl", or "heteroaralkoxy", refer to groups having 3, 4, 5, 6, 7, 8, 9, 10, 11, 12, 13, 14 ring atoms (which atoms are carbon and hetero atoms), preferably 5, 6, 9 or 10 ring atoms; having 6, 10, or 14 π (pi) electrons shared in a cyclic array; and having, in addition to carbon atoms, 1, 2, 3, 4 or 5 heteroatoms. The term "heteroatom" refers to nitrogen, oxygen, or sulfur, and includes any oxidized form of nitrogen or sulfur, and any quaternized form of a basic nitrogen. In other words, a "heteroaryl" ring or ring system may also be described as an aromatic heterocycle. Heteroaryl groups include, without limitation, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, furazanyl, pyridyl (pyridinyl), pyridazinyl, pyrimidinyl, pyrazinyl, indolizinyl, purinyl, naphthyridinyl, pteridinyl, and pyrrolopyridinyl, in particular pyrrolo[2,3-b]pyridinyl. The terms "heteroaryl" and "heteroar-", as used herein, also include groups in which a heteroaromatic ring is fused to one or more aryl, cycloaliphatic, or heterocyclyl rings, where the radical or point of attachment is preferably on the heteroaromatic or, if present, the aryl ring.

Nonlimiting examples include indolyl, isoindolyl, benzothienyl (benzothiophenyl), benzofuranyl, dibenzofuranyl, indazolyl, benzimidazolyl, benzothiazolyl, quinolyl, isoquinolyl, cinnolinyl, phthalazinyl, quinazolinyl, quinoxalinyl, 4H-quinolizinyl, carbazolyl, acridinyl, phenazinyl, phenothiazinyl, phenoxazinyl, tetrahydroquinolinyl, tetrahydroisoquinolinyl, 9H-carbazolyl, dibenzofuranyl and pyrido[2,3-b]-1,4-oxazin-3(4H)-one. For example, an indolyl ring may be attached via one of the ring atoms of the six-membered aryl ring or via one of the ring atoms of the five-membered heteroaryl ring. A heteroaryl group is optionally mono-, bi- or tricyclic. The term "heteroaryl" is used interchangeably with the terms "heteroaryl ring", "heteroaryl group", or "heteroaromatic", any of which terms include rings that are unsubstituted or substituted with one or more identical or different substituents. The term "heteroaralkyl" refers to an alkyl group substituted by a heteroaryl, wherein the alkyl and heteroaryl portions independently are optionally substituted.

A heteroaryl ring can be attached to its pendant group at any of its hetero or carbon ring atoms which attachment results in a stable structure or molecule: any of the ring atoms may be unsubstituted or substituted.

The structures of typical examples of "heteroaryl" substituents as used in the present invention are depicted below:

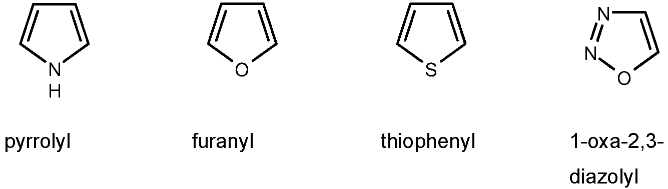

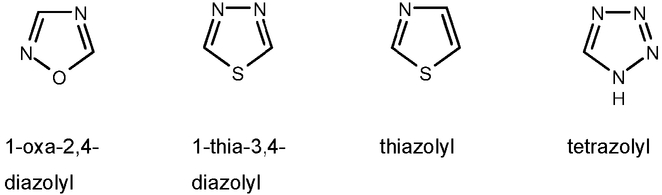

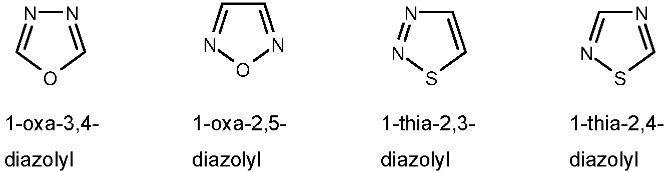

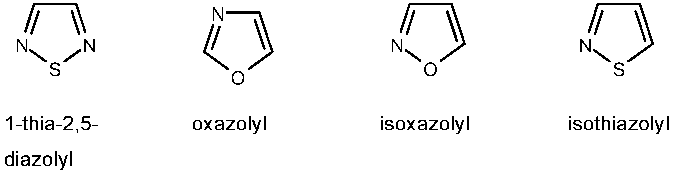

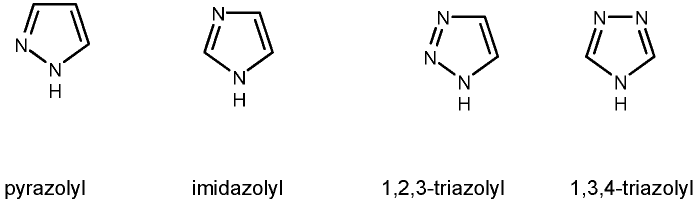

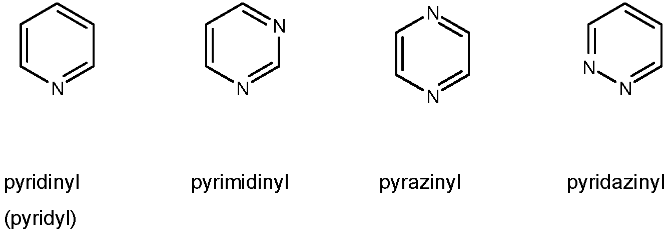

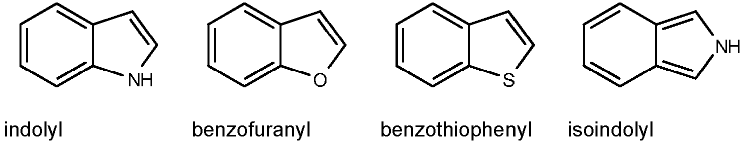

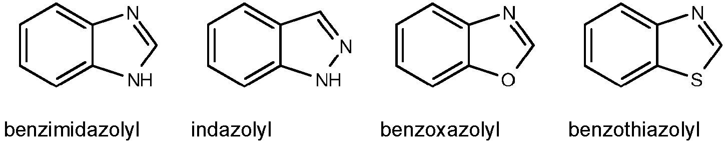

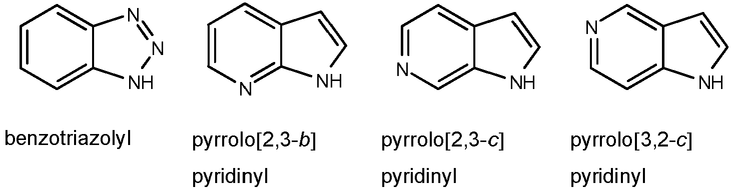

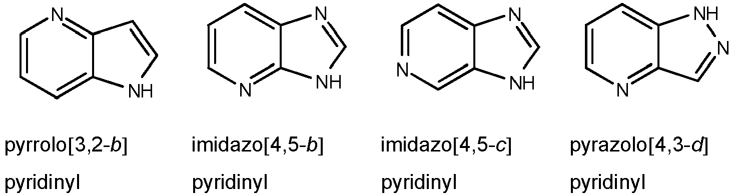

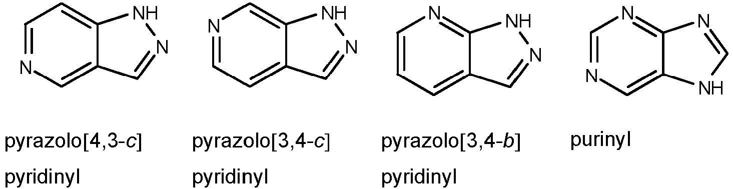

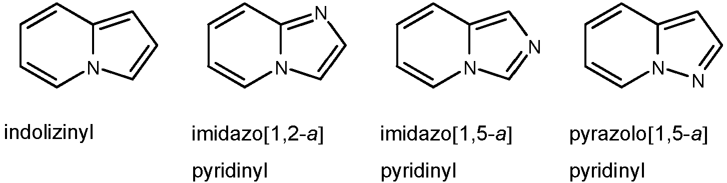

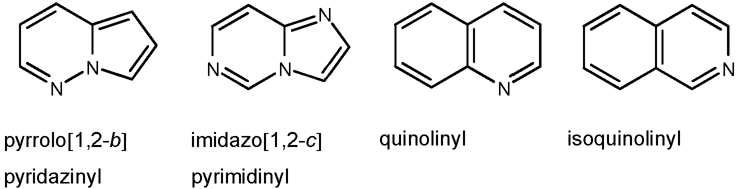

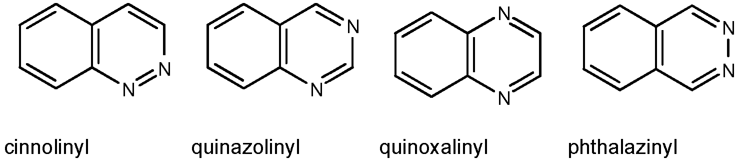

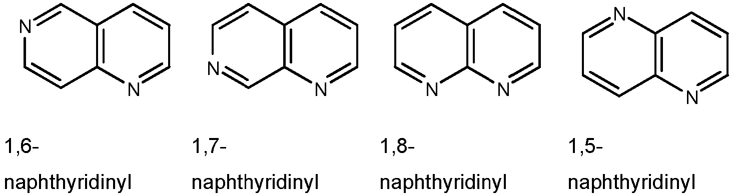

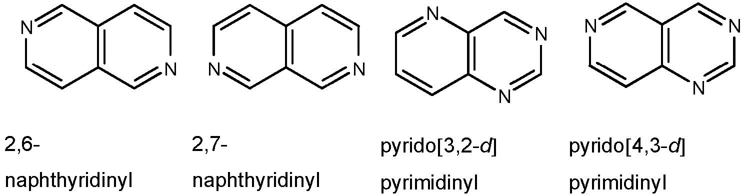

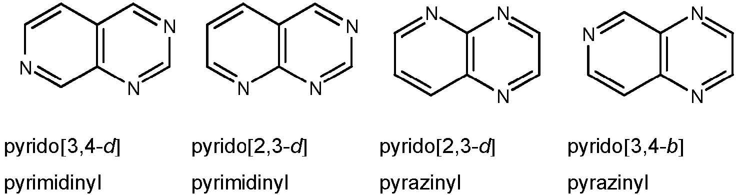

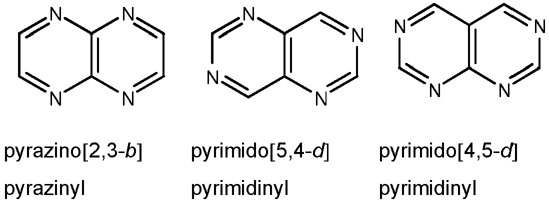

Those heteroaryl substituents can be attached to any pendant group via any of its ring atoms suitable for such an attachment.

As used herein, the terms "heterocycle", "heterocyclyl", "heterocyclic radical", and "heterocyclic ring" are used interchangeably and refer to a stable mono- bi- or tricyclic heterocyclic moiety with 5, 6, 7, 8, 9, 10, 11, 12, 13, 14 ring atoms wherein 1, 2, 3, 4, 5 of said ring atoms are hetero atoms and wherein that heterocyclic moiety is either saturated or partially unsaturated; heterocyclic moieties that are aromatic rings or ring systems are usually referred to as "heteroaryl" moieties as described hereinabove.

Preferably, the heterocycle is a stable saturated or partially unsaturated 3-, 4-, 5-, 6-, or 7-membered monocyclic or 7-, 8-, 9-, 10-, or 11-membered bicyclic or 11-, 12-, 13-, or 14-membered tricyclic heterocyclic moiety.

When used in reference to a ring atom of a heterocycle, the term "nitrogen" includes a substituted nitrogen. As an example, in a saturated or partially unsaturated ring having 1-3 heteroatoms selected from oxygen, sulfur or nitrogen, the nitrogen is N (as in 3,4-dihydro-2H-pyrrolyl), NH (as in pyrrolidinyl), or N-SUB with SUB being a suitable substituent (as in N-substituted pyrrolidinyl).

In the context of the term "heterocycle" the term "saturated" refers to a completely saturated heterocyclic system, like pyrrolidinyl, piperidinyl, morpholinyl, piperidinonyl, tetrahydrofuranyl, thianyl, and dioxothianyl. With regard to the term "heterocycle" the term "partially unsaturated" refers to heterocyclic systems (i) that contain one or more units of unsaturation, e.g. a C=C or a C=Heteroatom bond, but that are not aromatic, for instance, tetrahydropyridinyl; or (ii) in which a (saturated or unsaturated but non-aromatic) heterocyclic ring is fused with an aromatic or heteroaromatic ring system, wherein, however, the "partially unsaturated heterocycle" is attached to the rest of the molecule (its pendant group) via one of the ring atoms of the "heterocyclic" part of the system and not via the aromatic or heteroaromatic part. This first class (i) of "partially unsaturated" heterocycles may also be referred to as "non-aromatic partially unsaturated" heterocycles. This second class (ii) of "partially unsaturated" heterocycles may also be referred to as (bicyclic or tricyclic) "partially aromatic" heterocycles indicating that at least one of the rings of that heterocycle is a saturated or unsaturated but non-aromatic heterocycle that is fused with at least one aromatic or heteroaromatic ring system. Typical examples of these "partially aromatic" heterocycles are 1,2,3,4-tetrahydroquinolinyl and 1,2,3,4-tetrahydroisoquinolinyl.

A heterocyclic ring can be attached to its pendant group at any heteroatom or carbon atom that results in a stable structure and any of the ring atoms may be unsubstituted or substituted. Examples of such saturated or partially unsaturated heterocyclic radicals include, without limitation, tetrahydrofuranyl, tetrahydropyranyl, thianyl, dioxothianyl, tetrahydrothiophenyl, pyrrolidinyl, piperidinyl, pyrrolinyl, morpholinyl, tetrahydroquinolinyl, tetrahydroisoquinolinyl, decahydroquinolinyl, oxazolidinyl, piperazinyl, dioxanyl, dioxolanyl, diazepinyl, oxazepinyl, thiazepinyl, morpholinyl, and quinuclidinyl. The terms "heterocycle", "heterocyclyl", "heterocyclyl ring", "heterocyclic group", "heterocyclic moiety", and "heterocyclic radical", are used interchangeably herein, and also include groups in which a heterocyclyl ring is fused to one or more aryl, heteroaryl, or cycloaliphatic rings, such as indolinyl, 3H-indolyl, chromanyl, phenanthridinyl, or tetrahydroquinolinyl, where the radical or point of attachment is on the heterocyclyl ring. A heterocyclyl group is optionally mono-, bi- or tricyclic. The term "heterocyclylalkyl" refers to an alkyl group substituted by a heterocyclyl, wherein the alkyl and heterocyclyl portions independently are unsubstituted or substituted.

The term "unsaturated", as used herein, means that a moiety or group or substituent has one or more units of unsaturation.

As used herein with reference to any rings, ring systems, ring moieties, and the like, the term "partially unsaturated" refers to a ring moiety that includes at least one double or triple bond. The term "partially unsaturated" is intended to encompass rings having multiple sites of unsaturation. In particular, it encompasses (i) non-saturated (mono-, bi- or tricyclic) ring systems without any aromatic or heteroaromatic moiety or part; and (ii) bi- or tricyclic ring systems in which one of the rings of that system is an aromatic or heteroaromatic ring which is fused with another ring that is neither an aromatic nor a heteroaromatic ring, e.g. tetrahydronaphthyl or tetrahydroquinolinyl. The first class (i) of "partially unsaturated" rings, ring systems, ring moieties may also be referred to as "non-aromatic partially unsaturated" rings, ring systems, ring moieties, while the second class (ii) may be referred to as "partially aromatic" rings, ring systems, ring moieties.

As used herein, the term "bicyclic", "bicyclic ring" or "bicyclic ring system" refers to any bicyclic ring system, i.e. carbocyclic or heterocyclic, saturated or having one or more units of unsaturation, i.e. being partially unsaturated or aromatic, having one or more atoms in common between the two rings of the ring system. Thus, the term includes any permissible ring fusion, such as ortho-fused or spirocyclic. As used herein, the term "heterobicyclic" is a subset of "bicyclic" that requires that one or more heteroatoms are present in one or both rings of the bicycle. Such heteroatoms may be present at ring junctions and are optionally substituted, and may be selected from nitrogen (including N-oxides), oxygen, sulfur (including oxidized forms such as sulfones and sulfonates), phosphorus (including oxidized forms such as phosphates), boron, etc. In some embodiments, a bicyclic group has 7-12 ring members and 0-4 heteroatoms independently selected from nitrogen, oxygen, or sulfur. Likewise, the term "tricyclic", "tricyclic ring" or "tricyclic ring system" refers to any tricyclic ring system, i.e. carbocyclic or heterocyclic, saturated or having one or more units of unsaturation, i.e. being partially unsaturated or aromatic, in which a bicyclic ring system (as defined above) is fused with another, third ring. Thus, the term includes any permissible ring fusion. As used herein, the term "heterotricyclic" is a subset of "tricyclic" that requires that one or more heteroatoms are present in one or both rings of the tricycle. Such heteroatoms may be present at ring junctions and are optionally substituted, and may be selected from nitrogen (including N-oxides), oxygen, sulfur (including oxidized forms such as sulfones and sulfonates), phosphorus (including oxidized forms such as phosphates), boron, etc. In some embodiments, a tricyclic group has 10-14 ring members and 0-5 heteroatoms independently selected from nitrogen, oxygen, or sulfur.

As described herein, certain compounds of the invention contain "substituted" or "optionally substituted" moieties. In general, the term "substituted", whether preceded by the term "optionally" or not, means that one or more hydrogens of the designated moiety are replaced with a suitable substituent. "Substituted" applies to one or more hydrogens that are either explicit or implicit from the structure. Unless otherwise indicated, a "substituted" or "optionally substituted" group has a suitable substituent at each substitutable position of the group, and when more than one position in any given structure is substituted with more than one substituent selected from a specified group, the substituent is either the same or different at every position. If a certain group, substituent, moiety or radical is "mono-substituted", it bears one (1) substituent. If it is "di-substituted", it bears two (2) substituents, being either the same or different; if it is "trisubstituted", it bears three (3) substituents, wherein all three are the same or two are the same and the third is different or all three are different from each other. Combinations of substituents envisioned by this invention are preferably those that result in the formation of stable or chemically feasible compounds. The term "stable", as used herein, refers to compounds that are not substantially altered when subjected to conditions to allow for their production, detection, and, in certain embodiments, their recovery, purification, and use for one or more of the purposes disclosed herein.

If not specified otherwise elsewhere in the specification or the accompanying claims it is understood that each optional substituent on a substitutable carbon is a monovalent substituent independently selected from halogen; -(CH₂)₀₋₄R°; -(CH₂)₀₋₄OR°; -O(CH₂)₀₋₄R^{o}, -O-(CH₂)₀₋₄C(O)OR°; -(CH₂)₀₋₄CH(OR°)₂; -(CH₂)₀₋₄SR°; -(CH₂)₀₋₄Ph, which may be substituted with one or more R°; -(CH₂)₀₋₄O(CH₂)₀₋₁Ph which may be substituted with one or more R°; -CH=CHPh, which may be substituted with one or more R°; -(CH₂)₀₋₄O(CH₂)₀₋₁-pyridyl which may be substituted with one or more R°; -NO₂; -CN; -N₃; -(CH₂)₀₋₄N(R°)₂; -(CH₂)₀₋₄N(R°)C(O)R°; -N(R°)C(S)R°; -(CH₂)₀₋₄N(R°)C(O)NR°₂; -N(R°)C(S)NR°2; -(CH₂)₀₋₄N(R°)C(O)OR°; - N(R°)N(R°)C(O)R°; -N(R°)N(R°)C(O)NR°2; -N(R°)N(R°)C(O)OR°; - (CH₂)₀₋₄C(O)R°; -C(S)R°; -(CH²)₀₋₄C(O)OR°; -(CH₂)₀₋₄C(O)SR°; -(CH₂)₀₋₄C(O)OSiR°₃; -(CH₂)₀₋₄OC(O)R°; -OC(O)(CH₂)₀₋₄SR-, SC(S)SR°; - (CH₂)₀₋₄SC(O)R°; -(CH₂)₀₋₄C(O)NR°₂; -C(S)NR°₂; -C(S)SR°; - SC(S)SR°, -(CH₂)₀₋₄OC(O)NR°₂; -C(O)N(OR°)R°; -C(O)C(O)R°; - C(O)CH₂C(O)R°; -C(NOR°)R°; -(CH₂)₀₋₄SSR°; -(CH₂)₀₋₄S(O)₂R°; - (CH₂)₀₋₄S(O)₂OR°; -(CH₂)₀₋₄OS(O)₂R°; -S(O)₂NR°₂; -S(O)(NR°)R°; - S(O)₂N=C(NR°₂)₂; -(CH₂)₀₋₄S(O)R°; -N(R°)S(O)₂NR°₂; -N(R°)S(O)₂R°; - N(OR°)R°; -C(NH)NR°₂; -P(O)₂R°; -P(O)R°₂; -OP(O)R°₂; -OP(O)(OR°)₂; SiR°₃; -(C₁₋₄ straight or branched alkylene)O-N(R°)₂; or -(C₁₋₄ straight or branched alkylene)C(O)O-N(R°)₂. It is understood that "Ph" means phenyl; and that "-(CH₂)₀₋₄" means that there is either no alkylene group if the subscript is "0" (zero) or an alkylene group with 1, 2, 3 or 4 CH₂ units.

Each R° is independently hydrogen, halogen, C₁₋₆ aliphatic, -CH₂Ph, - O(CH₂)₀₋₁ Ph, -CH₂-(5-6 membered heteroaryl ring), or a 5-6-membered saturated, partially unsaturated, or aryl ring having 0-4 heteroatoms independently selected from nitrogen, oxygen, or sulfur, or, notwithstanding the definition above, two independent occurrences of R°, taken together with their intervening atom(s), form a 3-12-membered saturated, partially unsaturated, or aryl mono- or bicyclic ring having 0-4 heteroatoms independently selected from nitrogen, oxygen, or sulfur, which may be substituted by a divalent substituent on a saturated carbon atom of R° selected from =O and =S; or each R° is optionally substituted with a monovalent substituent independently selected from halogen, - (CH₂)₀₋₂R^{•}, -(haloR^{•}), -(CH₂)₀₋₂OH, -(CH₂)₀₋₂OR^{•}, -(CH₂)₀₋₂CH(OR^{•})₂; O(haloR^{•}), -CN, -N₃, -(CH₂)₀₋₂C(O)R^{•}, -(CH₂)₀₋₂C(O)OH, -(CH₂)₀₋₂C(O)OR^{•}, -(CH₂)₀₋₂SR^{•}, -(CH₂)₀₋₂SH, -(CH₂)₀₋₂NH₂, -(CH₂)₀₋₂NHR^{•}, - (CH₂)₀₋₂NR^{•}₂, -NO₂, -SiR^{•}₃, -OSiR^{•}₃, C(O)SR^{•}, -(C₁₋₄ straight or branched alkylene)C(O)OR^{•}, or -SSR^{•}. It is understood that "Ph" means phenyl; "halo" means halogen; and "-(CH₂)₀₋₂" means that there is either no alkylene group if the subscript is "0" (zero) or an alkylene group with 1 or 2 CH₂ units.

Each R^{•} is independently selected from C₁₋₄ aliphatic, -CH₂Ph, -O(CH₂)₀₋₁Ph, or a 5-6-membered saturated, partially unsaturated, or aryl ring having 0-4 heteroatoms independently selected from nitrogen, oxygen, or sulfur, and wherein each R^{•} is unsubstituted or where preceded by halo is substituted only with one or more halogens; or wherein an optional substituent on a saturated carbon is a divalent substituent independently selected from =O, =S, =NNR*₂, =NNHC(O)R*, =NNHC(O)OR*, =NNHS(O)₂R*, =NR*, =NOR*, -O(C(R*₂))₂₋₃O-, or -S(C(R*₂))₂₋₃S-, or a divalent substituent bound to vicinal substitutable carbons of an "optionally substituted" group is -O(CR*₂)₂₋₃O-, wherein each independent occurrence of R* is selected from hydrogen, C₁₋₆ aliphatic or an unsubstituted 5-6-membered saturated, partially unsaturated, or aryl ring having 0-4 heteroatoms independently selected from nitrogen, oxygen, or sulfur.

When R* is C₁₋₆ aliphatic, R* is optionally substituted with halogen, -R^{•}, (haloR^{•}), OH, -OR^{•}, -O(haloR^{•}), -CN, -C(O)OH, -C(O)OR^{•}, -NH₂, - NHR^{•}, -NR^{•}₂, or -NO₂, wherein each R^{•} is independently selected from C₁₋₄ aliphatic, -CH₂Ph, -O(CH₂)₀₋₁Ph, or a 5-6-membered saturated, partially unsaturated, or aryl ring having 0-4 heteroatoms independently selected from nitrogen, oxygen, or sulfur, and wherein each R^{•} is unsubstituted or where preceded by halo is substituted only with one or more halogens.

An optional substituent on a substitutable nitrogen is independently -R^{†}, - NR^{†}₂, -C(O)R^{†}, -C(O)OR^{†}, -C(O)C(O)R^{†}, - C(O)CH₂C(O)R^{†}, -S(O)₂R^{†}, -S(O)₂NR^{†}₂, -C(S)NR^{†}₂, -C(NH)NR^{†}₂, or - N(R^{†})S(O)₂R^{†}; wherein each R^{†} is independently hydrogen, C₁₋₆ aliphatic, unsubstituted -OPh, or an unsubstituted 5-6-membered saturated, partially unsaturated, or aryl ring having 0-4 heteroatoms independently selected from nitrogen, oxygen, or sulfur, or, two independent occurrences of R^{†}, taken together with their intervening atom(s) form an unsubstituted 3-12-membered saturated, partially unsaturated, or aryl mono- or bicyclic ring having 0-4 heteroatoms independently selected from nitrogen, oxygen, or sulfur; wherein when R^{†} is C₁₋₆ aliphatic, R^{†} is optionally substituted with halogen, -R^{•}, -(haloR^{•}), -OH, -OR^{•}, -O(haloR*), -CN, - C(O)OH, -C(O)OR^{•}, -NH₂, -NHR^{•}, -NR^{•}₂, or -NO₂, wherein each R^{•} is independently selected from C₁₋₄ aliphatic, -CH₂Ph, -O(CH₂)₀₋₁Ph, or a 5-6-membered saturated, partially unsaturated, or aryl ring having 0-4 heteroatoms independently selected from nitrogen, oxygen, or sulfur, and wherein each R^{•} is unsubstituted or where preceded by halo is substituted only with one or more halogens. It is understood that "Ph" means phenyl; and "halo" means halogen.

The term "solvates" means addition forms of the compounds of the present invention with solvents, preferably pharmaceutically acceptable solvents that contain either stoichiometric or non-stoichiometric amounts of solvent. Some compounds have a tendency to trap a fixed molar ratio of solvent molecules in the crystalline solid state, thus forming a solvate. If the solvent is water the solvate formed is a hydrate, e.g. a hemi-, mono- or dihydrate. If the solvent is alcohol, the solvate formed is an alcoholate, e.g., a methanolate or ethanolate. If the solvent is an ether, the solvate formed is an etherate, e.g., diethyl etherate.

The compounds of formula I may - also depending on the nature of substituents they may bear - have one or more centers of chirality. They may accordingly occur in various enantiomeric and diastereomeric forms, as the case may be, and be in racemic or optically active form. The invention, therefore, also relates to the optically active forms, enantiomers, racemates, diastereomers, mixtures thereof in all ratios, collectively: "stereoisomers" for the purpose of the present invention, of these compounds. Since the pharmaceutical activity of the racemates or stereoisomers of the compounds according to the invention may differ, it may be desirable to use a specific stereoisomer, e.g. one specific enantiomer or diastereomer. In these cases, a compound according to the present invention obtained as a racemate or even intermediates thereof - may be separated into the stereoisomeric (enantiomeric, diastereoisomeric) compounds by chemical or physical measures known to the person skilled in the art. Another approach that may be applied to obtain one or more specific stereoisomers of a compound of the present invention in an enriched or pure form makes use of stereoselective synthetic procedures, e.g. applying starting material in a stereoisomerically enriched or pure form (for instance using the pure or enriched (R)- or (S)-enantiomer of a particular starting material bearing a chiral center) or utilizing chiral reagents or catalysts, in particular enzymes. In the context of the present invention the term "pure enantiomer" usually refers to a relative purity of one enantiomer over the other (its antipode) of equal to or greater than 95%, preferably ≥ 98 %, more preferably ≥ 98.5%, still more preferably ≥ 99%.

Thus, for example, the compounds of the invention which have one or more centers of chirality and which occur as racemates or as mixtures of enantiomers or diastereoisomers can be fractionated or resolved by methods known per se into their optically pure or enriched isomers, i.e. enantiomers or diastereomers. The separation of the compounds of the invention can take place by chromatographic methods, e.g. column separation on chiral or nonchiral phases, or by recrystallization from an optionally optically active solvent or by use of an optically active acid or base or by derivatization with an optically active reagent such as, for example, an optically active alcohol, and subsequent elimination of the radical.

In the context of the present invention the term "tautomer" refers to compounds of the present invention that may exist in tautomeric forms and show tautomerism; for instance, carbonyl compounds may be present in their keto and/or their enol form and show keto-enol tautomerism. Those tautomers may occur in their individual forms, e.g., the keto or the enol form, or as mixtures thereof and are claimed separately and together as mixtures in any ratio. The same applies for cis/trans isomers, E/Z isomers, conformers and the like.

In one embodiment the compounds of the present invention are in the form of free base or acid - as the case may be -, i.e. in their non-salt (or salt-free) form. In another embodiment the compounds of the present invention are in the form of a pharmaceutically acceptable salt, a pharmaceutically acceptable solvate, or a pharmaceutically acceptable solvate of a pharmaceutically acceptable salt.

The term "pharmaceutically acceptable salts" refers to salts prepared from pharmaceutically acceptable bases or acids, including inorganic bases or acids and organic bases or acids. In cases where the compounds of the present invention contain one or more acidic or basic groups, the invention also comprises their corresponding pharmaceutically acceptable salts. Thus, the compounds of the present invention which contain acidic groups, such as carboxyl groups, can be present in salt form, and can be used according to the invention, for example, as alkali metal salts, alkaline earth metal salts, aluminium salts or as ammonium salts. More precise examples of such salts include lithium salts, sodium salts, potassium salts, calcium salts, magnesium salts, barium salts or salts with ammonia or organic amines such as, for example, ethylamine, ethanolamine, diethanolamine, triethanolamine, piperdine, N-methylglutamine or amino acids. These salts are readily available, for instance, by reacting the compound having an acidic group with a suitable base, e.g. lithium hydroxide, sodium hydroxide, sodium propoxide, potassium hydroxide, potassium ethoxide, magnesium hydroxide, calcium hydroxide or barium hydroxide. Other base salts of compounds of the present invention include but are not limited to copper(I), copper(II), iron(II), iron (III), manganese(II) and zinc salts. Compounds of the present invention which contain one or more basic groups, e.g. groups which can be protonated, can be present in salt form, and can be used according to the invention in the form of their addition salts with inorganic or organic acids. Examples of suitable acids include hydrogen chloride, hydrogen bromide, hydrogen iodide, phosphoric acid, sulfuric acid, nitric acid, methanesulfonic acid, p-toluenesulfonic acid, naphthalenedisulfonic acid, sulfoacetic acid, trifluoroacetic acid, oxalic acid, acetic acid, tartaric acid, lactic acid, salicylic acid, benzoic acid, carbonic acid, formic acid, propionic acid, pivalic acid, diethylacetic acid, malonic acid, succinic acid, pimelic acid, fumaric acid, malonic acid, maleic acid, malic acid, embonic acid, mandelic acid, sulfaminic acid, phenylpropionic acid, gluconic acid, ascorbic acid, isonicotinic acid, citric acid, adipic acid, taurocholic acid, glutaric acid, stearic acid, glutamic acid or aspartic acid, and other acids known to the person skilled in the art. The salts which are formed are, inter alia, hydrochlorides, chlorides, hydrobromides, bromides, iodides, sulfates, phosphates, methanesulfonates (mesylates), tosylates, carbonates, bicarbonates, formates, acetates, sulfoacetates, triflates, oxalates, malonates, maleates, succinates, tartrates, malates, embonates, mandelates, fumarates, lactates, citrates, glutarates, stearates, aspartates and glutamates. The stoichiometry of the salts formed from the compounds of the invention may moreover be an integral or non-integral multiple of one.

Compounds of the present invention which contain basic nitrogen-containing groups can be quaternized using agents such as (C₁-C₄)alkyl halides, for example methyl, ethyl, isopropyl and tert-butyl chloride, bromide and iodide; di(C₁-C₄)alkyl sulfates, for example dimethyl, diethyl and diamyl sulfate; (C₁₀-C₁₈)alkyl halides, for example decyl, dodecyl, lauryl, myristyl and stearyl chloride, bromide and iodide; and aryl(C₁-C₄)alkyl halides, for example benzyl chloride and phenethyl bromide. Both water- and oil-soluble compounds according to the invention can be prepared using such salts.

If the compounds of the present invention simultaneously contain acidic and basic groups in the molecule, the invention also includes, in addition to the salt forms mentioned, inner salts or betaines (zwitterions). The respective salts can be obtained by customary methods which are known to a person skilled in the art, for example by contacting these with an organic or inorganic acid or base in a solvent or dispersant, or by anion exchange or cation exchange with other salts. The present invention also includes all salts of the compounds of the present invention which, owing to low physiological compatibility, are not directly suitable for use in pharmaceuticals but which can be used, for example, as intermediates for chemical reactions or for the preparation of pharmaceutically acceptable salts.

Therefore, the following items are also in accordance with the invention:
(a) all stereoisomers or tautomers of the compounds, including mixtures thereof in all ratios;
(b) pharmaceutically acceptable salts of the compounds and of the items mentioned under (a);
(c) pharmaceutically acceptable solvates of the compounds and of the items mentioned under (a) and (b).

It should be understood that all references to compounds above and below are meant to include these items, in particular pharmaceutically acceptable solvates of the compounds, or pharmaceutically acceptable solvates of their pharmaceutically acceptable salts.

There is furthermore intended that a compound of the present invention includes isotope-labelled forms thereof. An isotope-labelled form of a compound of the formula I is identical to this compound apart from the fact that one or more atoms of the compound have been replaced by an atom or atoms having an atomic mass or mass number which differs from the atomic mass or mass number of the atom which usually occurs naturally. Examples of isotopes which are readily commercially available and which can be incorporated into a compound of the present invention by well-known methods include isotopes of hydrogen, carbon, nitrogen, oxygen, phosphorus, sulfur, fluorine and chlorine, for example ²H (D), ³H, ¹³C, ¹⁴C, ¹⁵N, ¹⁸O, ¹⁷O, ³¹P, ³²P, ³³S, ³⁴S, ³⁵S, ³⁶S, ¹⁸F and ³⁶Cl, respectively. A compound of formula I or a pharmaceutically acceptable salt thereof which contains one or more of the above-mentioned isotopes and/or other isotopes of other atoms is intended to be part of the present invention. An isotope-labelled compound of formula I can be used in a number of beneficial ways. For example, an isotope-labelled compound of the present invention into which, for example, a radioisotope, such as ³H or ¹⁴C, has been incorporated is suitable for medicament and/or substrate tissue distribution assays. These radioisotopes, i.e. tritium (³H) and carbon-14 (¹⁴C), are particularly preferred owing to simple preparation and excellent detectability. Incorporation of heavier isotopes, for example deuterium (²H), into a compound of formula I has therapeutic advantages owing to the higher metabolic stability of this isotope-labelled compound. Higher metabolic stability translates directly into an increased in vivo half-life or lower dosages, which under most circumstances would represent a preferred embodiment of the present invention. An isotope-labelled compound of formula I can usually be prepared by carrying out the procedures disclosed in the synthesis schemes and the related description, in the example part and in the preparation part in the present text, replacing a non-isotope-labelled reactant by a readily available isotope-labelled reactant.

Deuterium (²H; D) can also be incorporated into a compound of formula I for the purpose of manipulating the oxidative metabolism of the compound by way of the primary kinetic isotope effect. The primary kinetic isotope effect is a change of the rate for a chemical reaction that results from exchange of isotopic nuclei, which in turn is caused by the change in ground state energies necessary for covalent bond formation after this isotopic exchange. Exchange of a heavier isotope usually results in a lowering of the ground state energy for a chemical bond and thus cause a reduction in the rate in rate-limiting bond breakage. If the bond breakage occurs in or in the vicinity of a saddle-point region along the coordinate of a multi-product reaction, the product distribution ratios can be altered substantially. For explanation: if deuterium is bonded to a carbon atom at a non-exchangeable position, rate differences of k_{M}/k_{D} = 2-7 are typical. If this rate difference is successfully applied to a compound of the formula I that is susceptible to oxidation, the profile of this compound in vivo can be drastically modified and result in improved pharmacokinetic properties.

When discovering and developing therapeutic agents, the person skilled in the art attempts to optimize pharmacokinetic parameters while retaining desirable in vitro properties. It is reasonable to assume that many compounds with poor pharmacokinetic profiles are susceptible to oxidative metabolism. In vitro liver microsomal assays currently available provide valuable information on the course of oxidative metabolism of this type, which in turn permits the rational design of deuterated compounds of the formula I with improved stability through resistance to such oxidative meta-bolism. Significant improvements in the pharmacokinetic profiles of compounds of the formula I are thereby obtained, and can be expressed quantitatively in terms of increases in the in vivo half-life (t1/2), concentration at maximum therapeutic effect (Cₘₐₓ), area under the dose response curve (AUC), and F; and in terms of reduced clearance, dose and materials costs.

The following is intended to illustrate the above: a compound of formula I which has multiple potential sites of attack for oxidative metabolism, for example benzylic hydrogen atoms and hydrogen atoms bonded to a nitrogen atom, is prepared as a series of analogues in which various combinations of hydrogen atoms are replaced by deuterium atoms, so that some, most or all of these hydrogen atoms have been replaced by deuterium atoms. Half-life determinations enable favourable and accurate determination of the extent of the extent to which the improvement in resistance to oxidative metabolism has improved. In this way, it is deter-mined that the half-life of the parent compound can be extended by up to 100% as the result of deuterium-hydrogen exchange of this type.

Deuterium-hydrogen exchange in a compound of the present invention can also be used to achieve a favourable modification of the metabolite spectrum of the starting compound in order to diminish or eliminate undesired toxic metabolites. For example, if a toxic metabolite arises through oxidative carbon-hydrogen (C-H) bond cleavage, it can reasonably be assumed that the deuterated analogue will greatly diminish or eliminate production of the unwanted metabolite, even if the particular oxidation is not a rate-determining step. Further information on the state of the art with respect to deuterium-hydrogen exchange may be found, for example in Hanzlik et al., J. Org. Chem. 55, 3992-3997, 1990, Reider et al., J. Org. Chem. 52, 3326-3334, 1987, Foster, Adv. Drug Res. 14, 1-40, 1985, Gillette et al, Biochemistry 33(10) 2927-2937, 1994, and Jarman et al. Carcinogenesis 16(4), 683-688, 1995.

Furthermore, the present invention relates to pharmaceutical compositions comprising at least one compound of formula I, or a pharmaceutically acceptable salt, solvate, tautomer and/or stereoisomer thereof, as active ingredient, together with a pharmaceutically acceptable carrier.

For the purpose of the present invention the term "pharmaceutical composition" (or "pharmaceutical formulation") refers to a composition or product comprising one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing at least one compound of the present invention and a pharmaceutically acceptable carrier. It may further comprise physiologically acceptable excipients, auxiliaries, adjuvants, diluents and/or additional pharmaceutically active substance other than the compounds of the invention.

The pharmaceutical compositions include compositions and pharmaceutical formulations suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

A pharmaceutical composition of the present invention may additionally comprise one or more other compounds as active ingredients (drugs), such as one or more additional compounds of the present invention. In a particular embodiment the pharmaceutical composition further comprises a second active ingredient or its derivatives, prodrugs, solvates, tautomers or stereoisomers thereof as well as the pharmaceutically acceptable salts of each of the foregoing, including mixtures thereof in all ratios, wherein that second active ingredient is other than a compound of formula I; preferably, that second active ingredient is a compound that is useful in the treatment, prevention, suppression and/or amelioration of medicinal conditions or pathologies for which the compounds of the present invention are useful as well and which are listed elsewhere hereinbefore or hereinafter. Such combination of two or more active ingredients or drugs may be safer or more effective than either drug or active ingredient alone, or the combination is safer or more effective than it would be expected based on the additive properties of the individual drugs. Such other drug(s) may be administered, by a route and in an amount commonly used contemporaneously or sequentially with a compound of the invention. When a compound of the invention is used contemporaneously with one or more other drugs or active ingredients, a combination product containing such other drug(s) and the compound of the invention - also referred to as "fixed dose combination" - is preferred. However, combination therapy also includes therapies in which the compound of the present invention and one or more other drugs are administered on different overlapping schedules. It is contemplated that when used in combination with other active ingredients, the compound of the present invention or the other active ingredient or both may be used effectively in lower doses than when each is used alone. Accordingly, the pharmaceutical compositions of the present invention include those that contain one or more other active ingredients, in addition to a compound of the invention.

The compounds of the present invention - or any pharmaceutically acceptable salt, solvate, tautomer and/or stereoisomer thereof - can be used as medicaments. Without wishing to be bound by theory, it is assumed that the compounds of formula I are bifunctional degraders wherein structural moiety Q¹ is functioning as a ubiquitin ligase ligand and structural moiety Q³ is a TEAD binding functional unit (or TEAD ligand) while Q² is a suitable linker which, if present, covalently links moieties Q¹ and Q³. Compounds of formula I have been found to exhibit pharmacological activity by binding to TEAD and/or inhibiting TEAD - which may include inhibiting YAP-TEAD and/or TAZ-TEAD protein-protein interaction without being limited thereto - and/or degrading TEAD. A compound of formula I may form a ternary complex comprising (i) a compound of formula I; (ii) a TEAD protein; and (iii) a ubiquitin ligase; thereby mediating the pharmacological activity desired. It is assumed that by this activity the compounds of the present invention may reverse or counteract dysfunction of the Hippo pathway. Apart from reversing or counteracting dysfunction of the Hippo pathway and independent of upstream Hippo regulation, the pharmacological activity of the compounds of the present invention may also be useful in other pathophysiological scenarios where inhibition or disruption or degradation of TEAD binding (and/or aberrant YAP-TEAD and/or aberrant TAZ-TEAD signaling) would be beneficial.

Thus, the compounds of the present invention being TEAD binders and/or inhibitors and/or degraders are useful in particular in the treatment, prevention, suppression and/or amelioration of hyperproliferative disorders and cancer, in particular tumors including solid tumors, of breast cancer, lung cancer, mesothelioma, epithelioid hemangioendothelioma, uveal melanoma, liver cancer, ovarian cancer, squamous cancer, renal cancer, gastric cancer, medulloblastoma, colon cancer, pancreatic cancer, schwannoma, meningioma, glioma, basal cell carcinoma. Without wishing to commit to any specific theory or explanation it may be assumed that the compounds might be able to achieve this by direct effects on the cancer cells and/or indirectly by modulating the response of the immune system against the tumor. Furthermore, the compounds of the present invention may also be useful in the treatment, prevention, suppression and/or amelioration of non-cancerous disorders and diseases, e.g. cardiovascular diseases and fibrosis (like liver fibrosis).

**In** a particular embodiment the compounds of the present invention are for use in the prevention and/or treatment, especially in the treatment of any of the disorders or diseases listed above, preferably of cancer, in particular tumors including solid tumors, of the specific types of cancer disclosed in the previous paragraph; or of any of the non-cancerous disorders or diseases disclosed in the previous paragraph.

Another particular embodiment of the present invention is a method for preventing and/or treating, preferably treating a disorder or disease selected from the group consisting of hyperproliferative disorders and cancer, in particular tumors including solid tumors, of the specific types of cancer disclosed in the previous paragraphs; or of any of the non-cancerous disorders or diseases disclosed in the previous paragraphs.

Still another particular embodiment of the invention is the use of a compound of the present invention - or solvates, tautomers or stereoisomers thereof and/or the pharmaceutically acceptable salts of each of the foregoing, including mixtures thereof in all ratios - for the manufacturing of a medicament, in particular for preventing and/or treating, preferably treating a disorder or disease selected from the group consisting of hyperproliferative disorders and cancer, in particular tumors including solid tumors, of the specific types of cancer disclosed in the previous paragraphs; or of any of the non-cancerous disorders or diseases disclosed in the previous paragraphs.

Preferably, the present invention relates to a compound of the present invention for use in the prevention and/or treatment of a disease - or, alternatively, a method for preventing and/or treating a disease by administering an effective amount of a compound of the present invention ; or, in another alternative, a use of a compound of the present invention for the manufacturing of a medicament for the prevention and/or treatment of a disease - wherein that disease is a cancer, in particular tumors including solid tumors, of the specific types of cancer disclosed in the previous paragraphs; and more preferably, wherein administration of the compound is simultaneous, sequential or in alternation with administration of at least one other active drug agent.

The disclosed compounds of the present invention and in particular of formula I can be administered in combination with other known therapeutic agents, including anticancer agents. As used here, the term "anticancer agent" relates to any agent which is administered to a patient with cancer for the purposes of treating the cancer. The anti-cancer treatment defined above may be applied as a monotherapy or may involve, in addition to the herein disclosed compounds of the present invention, conventional surgery or radiotherapy or medicinal therapy. Such medicinal therapy, e.g. a chemotherapy or a targeted therapy, may include one or more, but preferably one, of the following anti-tumor agents:
Alkylating agents
   such as altretamine, bendamustine, busulfan, carmustine, chlorambucil, chlormethine, cyclophosphamide, dacarbazine, ifosfamide, improsulfan, tosilate, lomustine, melphalan, mitobronitol, mitolactol, nimustine, ranimustine, temozolomide, thiotepa, treosulfan, mechloretamine, carboquone; apaziquone, fotemustine, glufosfamide, palifosfamide, pipobroman, trofosfamide, uramustine, evofosfamide, VAL-083^{[4]};
Platinum Compounds
   such as carboplatin, cisplatin, eptaplatin, miriplatine hydrate, oxaliplatin, lobaplatin, nedaplatin, picoplatin, satraplatin;
DNA altering agents
   such as amrubicin, bisantrene, decitabine, mitoxantrone, procarbazine, trabectedin, clofarabine;
   amsacrine, brostallicin, pixantrone, laromustine^{[1],[3]};
Topoisomerase Inhibitors
   such as etoposide, irinotecan, razoxane, sobuzoxane, teniposide, topotecan;
   amonafide, belotecan, elliptinium acetate, voreloxin;
Microtubule modifiers
   such as cabazitaxel, docetaxel, eribulin, ixabepilone, paclitaxel, vinblastine, vincristine, vinorelbine, vindesine, vinflunine; fosbretabulin, tesetaxel;
Antimetabolites
   such as asparaginase^{[3]}, azacitidine, calcium levofolinate, capecitabine, cladribine, cytarabine, enocitabine, floxuridine, fludarabine, fluorouracil, gemcitabine, mercaptopurine, methotrexate, nelarabine, pemetrexed, pralatrexate, azathioprine, thioguanine, carmofur; doxifluridine, elacytarabine, raltitrexed, sapacitabine, tegafur^{[2],[3]}, trimetrexate;
Anticancer antibiotics
   such as bleomycin, dactinomycin, doxorubicin, epirubicin, idarubicin, levamisole, miltefosine, mitomycin C, romidepsin, streptozocin, valrubicin, zinostatin, zorubicin, daunurobicin, plicamycin; aclarubicin, peplomycin, pirarubicin;
Hormones/Antagonists
   such as abarelix, abiraterone, bicalutamide, buserelin, calusterone, chlorotrianisene, degarelix, dexamethasone, estradiol, fluocortolone, fluoxymesterone, flutamide, fulvestrant, goserelin, histrelin, leuprorelin, megestrol, mitotane, nafarelin, nandrolone, nilutamide, octreotide, prednisolone, raloxifene, tamoxifen, thyrotropin alfa, toremifene, trilostane, triptorelin, diethylstilbestrol; acolbifene, danazol, deslorelin, epitiostanol, orteronel, enzalutamide ^{[1],[3]};
Aromatase inhibitors
   such as aminoglutethimide, anastrozole, exemestane, fadrozole, letrozole, testolactone; formestane;
Small molecule kinase inhibitors
   such as crizotinib, dasatinib, erlotinib, imatinib, lapatinib, nilotinib, pazopanib, regorafenib, ruxolitinib, sorafenib, sunitinib, vandetanib, vemurafenib, bosutinib, gefitinib, axitinib; afatinib, alisertib, dabrafenib, dacomitinib, dinaciclib, dovitinib, enzastaurin, nintedanib, lenvatinib, linifanib, linsitinib, masitinib, midostaurin, motesanib, neratinib, orantinib, perifosine, ponatinib, radotinib, rigosertib, tepotinib, tipifarnib, tivantinib, tivozanib, trametinib, pimasertib, brivanib alaninate, cediranib, apatinib^{[4]}, cabozantinib S-malate^{[1],[3]}, ibrutinib^{[1],[3]}, icotinib^{[4]}, buparlisib^{[2]}, cipatinib^{[4]}, cobimetinib^{[1],[3]}, idelalisib^{[1],[3]}, fedratinib^{[1]},tesevatinib;
Photosensitizers
   such as methoxsalen^{[3]}; porfimer sodium, talaporfin, temoporfin;
Antibodies
   such as alemtuzumab, besilesomab, brentuximab vedotin, cetuximab, denosumab, ipilimumab, ofatumumab, panitumumab, rituximab, tositumomab, trastuzumab, bevacizumab, pertuzumab^{[2],[3]}; catumaxomab, elotuzumab, epratuzumab, farletuzumab, mogamulizumab, necitumumab, nimotuzumab, obinutuzumab, ocaratuzumab, oregovomab, ramucirumab, rilotumumab, siltuximab, tocilizumab, zalutumumab, zanolimumab, matuzumab, dalotuzumab^{[1],[2],[3]}, onartuzumab^{[1],[3]}, racotumomab^{[1]}, tabalumab^{[1],[3]}, EMD-525797^{[4],} atezolizumab, durvalumab, pembrolizumab, nivolumab^{[1],[3]};
Cytokines
   such as aldesleukin, interferon alfa2, interferon alfa2a^{[3]}, interferon alfa2b^{[2],[3]};
   celmoleukin, tasonermin, teceleukin, oprelvekin^{[1],[3]}, recombinant interferon beta-1a^{[4]};
Drug Conjugates
   such as denileukin diftitox, ibritumomab tiuxetan, iobenguane I 123, prednimustine, trastuzumab emtansine, estramustine, gemtuzumab, ozogamicin, aflibercept; cintredekin besudotox, edotreotide, inotuzumab ozogamicin, naptumomab estafenatox, oportuzumab monatox, technetium (99mTc) arcitumomab^{[1],[3]}, vintafolide^{[1],[3]};
Vaccines
   such as sipuleucel^{[3]}; vitespen^{[3]}, emepepimut-S^{[3]}, oncoVAX^{[4]}, rindopepimut^{[3]}, troVax^{[4]}, MGN-1601^{[4]}, MGN-1703^{[4]};
Miscellaneous
   alitretinoin, bexarotene, bortezomib, everolimus, ibandronic acid, imiquimod, lenalidomide, lentinan, metirosine, mifamurtide, pamidronic acid, pegaspargase, pentostatin, sipuleucel^{[3]}, sizofiran, tamibarotene, temsirolimus, thalidomide, tretinoin, vismodegib, zoledronic acid, vorinostat; celecoxib, cilengitide, entinostat, etanidazole, ganetespib, idronoxil, iniparib, ixazomib, lonidamine, nimorazole, panobinostat, peretinoin, plitidepsin, pomalidomide, procodazol, ridaforolimus, tasquinimod, telotristat, thymalfasin, tirapazamine, tosedostat, trabedersen, ubenimex, valspodar, gendicine^{[4]}, picibanil^{[4]}, reolysin^{[4]}, retaspimycin hydrochloride^{[1],[3]}, trebananib^{[2],[3]}, virulizin^{[4]}, carfilzomib^{[1],[3]}, endostatin^{[4]}, immucothel^{[4]}, belinostat^{[3]};
PARP inhibitors
   Olaparib, Veliparib.
MCT1 inhibitors
   AZD3965^{[4]}, BAY-8002^{[4]}.
   ^{[1]} **Prop. INN** (**P**roposed **I**nternational **N**onproprietary **N**ame)
   **^{[2]} Rec. INN** (**R**ecommended **I**nternational **N**onproprietary **N**ames)
   **^{[3]} USAN** (**U**nited **S**tates **A**dopted **N**ame)
   **^{[4]} no INN.**

In another aspect of the invention, a set or kit is provided comprising a therapeutically effective amount of at least one compound of the invention and/or at least one pharmaceutical composition as described herein and a therapeutically effective amount of at least one further pharmacologically active substance other than the compounds of the invention. It is preferred that this set or kit comprises separate packs of
a) an effective amount of a compound of formula I, or a pharmaceutically acceptable salt, solvate, tautomer and/or stereoisomer thereof; and
b) an effective amount of a further active ingredient that further active ingredient not being a compound of formula I.

A further embodiment of the present invention is a process for the manufacture of the pharmaceutical compositions of the present invention, characterized in that one or more compounds according to the invention and one or more compounds selected from the group consisting of solid, liquid or semiliquid excipients, auxiliaries, adjuvants, diluents, carriers and pharmaceutically active agents other than the compounds according to the invention, are converted in a suitable dosage form.

The pharmaceutical compositions (formulations) of the present invention may be administered by any means that achieve their intended purpose. For example, administration may be via oral, parenteral, topical, enteral, intravenous, intramuscular, inhalant, nasal, intraarticular, intraspinal, transtracheal, transocular, subcutaneous, intraperitoneal, transdermal, or buccal routes. Alternatively, or concurrently, administration may be via the oral route. The dosage administered will be dependent upon the age, health, and weight of the recipient, kind of concurrent treatment, if any, frequency of treatment, and the nature of the effect desired. Parenteral administration is preferred. Oral administration is especially preferred.

Suitable dosage forms include, but are not limited to capsules, tablets, pellets, dragees, semi-solids, powders, granules, suppositories, ointments, creams, lotions, inhalants, injections, cataplasms, gels, tapes, eye drops, solution, syrups, aerosols, suspension, emulsion, which can be produced according to methods known in the art, for example as described below:
Tablets: mixing of active ingredient/s and auxiliaries, compression of said mixture into tablets (direct compression), optionally granulation of part of mixture before compression.
Capsules: mixing of active ingredient/s and auxiliaries to obtain a flowable powder, optionally granulating powder, filling powders/granulate into opened capsules, capping of capsules.
Semi-solids (ointments, gels, creams): dissolving/dispersing active ingredient/s in an aqueous or fatty carrier; subsequent mixing of aqueous/fatty phase with complementary fatty/ aqueous phase, homogenization (creams only).
Suppositories (rectal and vaginal): dissolving/dispersing active ingredient/s in carrier material liquified by heat (rectal: carrier material normally a wax; vaginal: carrier normally a heated solution of a gelling agent), casting said mixture into suppository forms, annealing and withdrawal suppositories from the forms.
Aerosols: dispersing/dissolving active agent/s in a propellant, bottling said mixture into an atomizer.

**In** general, non-chemical routes for the production of pharmaceutical compositions and/or pharmaceutical preparations comprise processing steps on suitable mechanical means known in the art that transfer one or more compounds of the invention into a dosage form suitable for administration to a patient in need of such a treatment. Usually, the transfer of one or more compounds of the invention into such a dosage form comprises the addition of one or more compounds, selected from the group consisting of carriers, excipients, auxiliaries and pharmaceutical active ingredients other than the compounds of the invention. Suitable processing steps include, but are not limited to combining, milling, mixing, granulating, dissolving, dispersing, homogenizing, casting and/or compressing the respective active and nonactive ingredients. Mechanical means for performing said processing steps are known in the art, for example from Ullmann's Encyclopedia of Industrial Chemistry, 5th Edition. In this respect, active ingredients are preferably at least one compound of the invention and optionally one or more additional compounds other than the compounds of the invention, which show valuable pharmaceutical properties, preferably those pharmaceutical active agents other than the compounds of the invention, which are disclosed herein.

Particularly suitable for oral use are tablets, pills, coated tablets, capsules, powders, granules, syrups, juices or drops, suitable for rectal use are suppositories, suitable for parenteral use are solutions, preferably oilbased or aqueous solutions, furthermore suspensions, emulsions or implants, and suitable for topical use are ointments, creams or powders. The compounds of the invention may also be lyophilized and the resultant lyophilizates used, for example, for the preparation of injection preparations. The preparations indicated may be sterilized and/or comprise assistants, such as lubricants, preservatives, stabilizers and/or wetting agents, emulsifiers, salts for modifying the osmotic pressure, buffer substances, dyes, flavors and/or a plurality of further active ingredients, for example one or more vitamins.

Suitable excipients are organic or inorganic substances, which are suitable for enteral (for example oral), parenteral or topical administration and do not react with the compounds of the invention, for example water, vegetable oils, benzyl alcohols, alkylene glycols, polyethylene glycols, glycerol triacetate, gelatin, carbohydrates, such as lactose, sucrose, mannitol, sorbitol or starch (maize starch, wheat starch, rice starch, potato starch), cellulose preparations and/or calcium phosphates, for example tricalcium phosphate or calcium hydrogen phosphate, magnesium stearate, talc, gelatin, tragacanth, methyl cellulose, hydroxypropylmethylcellulose, sodium carboxymethylcellulose, polyvinyl pyrrolidone and/or vaseline.

If desired, disintegrating agents may be added such as the above-mentioned starches and also carboxymethyl-starch, cross-linked polyvinyl pyrrolidone, agar, or alginic acid or a salt thereof, such as sodium alginate. Auxiliaries include, without limitation, flow-regulating agents and lubricants, for example, silica, talc, stearic acid or salts thereof, such as magnesium stearate or calcium stearate, and/or polyethylene glycol. Dragee cores are provided with suitable coatings, which, if desired, are resistant to gastric juices. For this purpose, concentrated saccharide solutions may be used, which may optionally contain gum arabic, talc, polyvinyl pyrrolidone, polyethylene glycol and/or titanium dioxide, lacquer solutions and suitable organic solvents or solvent mixtures. In order to produce coatings resistant to gastric juices or to provide a dosage form affording the advantage of prolonged action, the tablet, dragee or pill can comprise an inner dosage and an outer dosage component the latter being in the form of an envelope over the former. The two components can be separated by an enteric layer, which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, acetyl alcohol, solutions of suitable cellulose preparations such as acetyl-cellulose phthalate, cellulose acetate or hydroxypropylmethylcellulose phthalate, are used. Dye stuffs or pigments may be added to the tablets or dragee coatings, for example, for identification or in order to characterize combinations of active compound doses.

Suitable carrier substances are organic or inorganic substances which are suitable for enteral (e.g. oral) or parenteral administration or topical application and do not react with the novel compounds, for example water, vegetable oils, benzyl alcohols, polyethylene glycols, gelatin, carbohydrates such as lactose or starch, magnesium stearate, talc and petroleum jelly. In particular, tablets, coated tablets, capsules, syrups, suspensions, drops or suppositories are used for enteral administration, solutions, preferably oily or aqueous solutions, furthermore suspensions, emulsions or implants, are used for parenteral administration, and ointments, creams or powders are used for topical application. The compounds of the invention can also be lyophilized and the lyophilizates obtained can be used, for example, for the production of injection preparations.

Other pharmaceutical preparations, which can be used orally include push-fit capsules made of gelatin, as well as soft, sealed capsules made of gelatin and a plasticizer such as glycerol or sorbitol. The push-fit capsules can contain the active compounds in the form of granules, which may be mixed with fillers such as lactose, binders such as starches, and/or lubricants such as talc or magnesium stearate and, optionally, stabilizers. In soft capsules, the active compounds are preferably dissolved or suspended in suitable liquids, such as fatty oils, or liquid paraffin. In addition, stabilizers may be added.

The liquid forms in which the novel compositions of the present invention may be incorporated for administration orally include aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinylpyrrolidone or gelatin.

Suitable formulations for parenteral administration include aqueous solutions of the active compounds in water-soluble form, for example, water-soluble salts and alkaline solutions. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides or polyethylene glycol-400 (the compounds are soluble in PEG-400).

Aqueous injection suspensions may contain substances, which increase the viscosity of the suspension, including, for example, sodium carboxymethyl cellulose, sorbitol, and/or dextran, optionally, the suspension may also contain stabilizers.

For administration as an inhalation spray, it is possible to use sprays in which the active ingredient is either dissolved or suspended in a propellant gas or propellant gas mixture (for example CO₂ or chlorofluorocarbons). The active ingredient is advantageously used here in micronized form, in which case one or more additional physiologically acceptable solvents may be present, for example ethanol. Inhalation solutions can be administered with the aid of conventional inhalers.

Possible pharmaceutical preparations, which can be used rectally include, for example, suppositories, which consist of a combination of one or more of the active compounds with a suppository base. Suitable suppository bases are, for example, natural or synthetic triglycerides, or paraffin hydrocarbons. In addition, it is also possible to use gelatin rectal capsules, which consist of a combination of the active compounds with a base. Possible base materials include, for example, liquid triglycerides, polyethylene glycols, or paraffin hydrocarbons.

The pharmaceutical preparations can be employed as medicaments in human and veterinary medicine. As used herein, the term "effective amount" means that amount of a drug or pharmaceutical agent that will elicit the biological or medical response of a tissue, system, animal or human that is being sought, for instance, by a researcher or clinician. Furthermore, the term also includes within its scope a "therapeutically effective amount" which means any amount which, as compared to a corresponding subject who has not received such amount, results in improved treatment, healing, prevention, or amelioration of a disease, disorder, or side effect, or a decrease in the rate of advancement of a disease or disorder, or of symptoms associated with such disease or disorder; it may also refer to preventing or providing prophylaxis for the disease or disorder in a subject having or at risk for developing a disease disclosed herein. The term also includes within its scope amounts effective to enhance normal physiological function. Said therapeutic effective amount of one or more of the compounds of the invention is known to the skilled artisan or can be easily determined by standard methods known in the art.

"Treating" or "treatment" as used herein, means an alleviation, in whole or in part, of symptoms associated with a disorder or disease, or slowing, or halting of further progression or worsening of those symptoms, or prevention or prophylaxis of the disease or disorder in a subject at risk for developing the disease or disorder.

The compounds of the present invention and the optional additional active substances are generally administered analogously to commercial preparations. Usually, suitable doses that are therapeutically effective lie in the range between 0.0005 mg and 1000 mg, preferably between 0.005 mg and 500 mg and especially between 0.5 mg and 100 mg per dose unit. The daily dose is preferably between about 0.001 mg/kg and 10 mg/kg of body weight.

Those of skill will readily appreciate that dose levels can vary as a function of the specific compound, the severity of the symptoms and the susceptibility of the subject to side effects. Some of the specific compounds are more potent than others. Preferred dosages for a given compound are readily determinable by those of skill in the art by a variety of means. A preferred means is to measure the physiological potency of a given compound.

The specific dose for the individual patient, in particular for the individual human patient, depends, however, on the multitude of factors, for example on the efficacy of the specific compounds employed, on the age, body weight, general state of health, the sex, the kind of diet, on the time and route of administration, on the excretion rate, the kind of administration and the dosage form to be administered, the pharmaceutical combination and severity of the particular disorder to which the therapy relates. The specific therapeutic effective dose for the individual patient can readily be determined by routine experimentation, for example by the doctor or physician, which advises or attends the therapeutic treatment.

The compounds of the present invention can be prepared according to the procedures of the following Schemes, Experimental Part and Examples, using appropriate materials, and as further exemplified by the following specific examples. They may also be prepared by methods known per se, as described in the literature (for example in standard works, such as Houben-Weyl, Methoden der Organischen Chemie [Methods of Organic Chemistry], Georg Thieme Verlag, Stuttgart; Organic Reactions, John Wiley & Sons, Inc., New York), to be precise under reaction conditions which are known and suitable for the said reactions. Use can also be made of variants which are known per se, but are not mentioned here in greater detail.

Likewise, the starting materials for the preparation of compounds of the present invention can be prepared by methods as described in the examples or by methods known per se, as described in the literature of synthetic organic chemistry and known to the skilled person, or can be obtained commercially. The starting materials for the processes claimed and/or utilized may, if desired, also be formed in situ by not isolating them from the reaction mixture, but instead immediately converting them further into the compounds of the invention or intermediate compounds. On the other hand, in general it is possible to carry out the reaction stepwise.

Preferably, the reaction of the compounds is carried out in the presence of a suitable solvent, which is preferably inert under the respective reaction conditions. Examples of suitable solvents comprise but are not limited to hydrocarbons, such as hexane, petroleum ether, benzene, toluene or xylene; chlorinated hydrocarbons, such as trichlorethylene, 1,2-dichloroethane, tetrachloromethane, chloroform or dichloromethane; alcohols, such as methanol, ethanol, isopropanol, n-propanol, n-butanol or tert-butanol; ethers, such as diethyl ether, diisopropyl ether, tetrahydrofuran (THF) or dioxane; glycol ethers, such as ethylene glycol monomethyl or monoethyl ether or ethylene glycol dimethyl ether (diglyme); ketones, such as acetone or butanone; amides, such as acetamide, dimethylacetamide, dimethylformamide (DMF) or N-methyl pyrrolidinone (NMP); nitriles, such as acetonitrile; sulfoxides, such as dimethyl sulfoxide (DMSO); nitro compounds, such as nitromethane or nitrobenzene; esters, such as ethyl acetate, or mixtures of the said solvents or mixtures with water.

The reaction temperature is between about -100°C and 300°C, depending on the reaction step and the conditions used.

Reaction times are generally in the range between a fraction of a minute and several days, depending on the reactivity of the respective compounds and the respective reaction conditions. Suitable reaction times are readily determinable by methods known in the art, for example reaction monitoring. Based on the reaction temperatures given above, suitable reaction times generally lie in the range between 10 minutes and 48 hours.

Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds.

As will be understood by the person skilled in the art of organic synthesis compounds of the present invention, in particular compounds of formula I, are readily accessible by various synthetic routes, some of which are exemplified in the accompanying Experimental Part. The skilled artisan will easily recognize which kind of reagents and reactions conditions are to be used and how they are to be applied and adapted in any particular instance - wherever necessary or useful - in order to obtain the compounds of the present invention. Furthermore, some of the compounds of the present invention can readily be synthesized by reacting other compounds of the present invention under suitable conditions, for instance, by converting one particular functional group being present in a compound of the present invention, or a suitable precursor molecule thereof, into another one by applying standard synthetic methods, like reduction, oxidation, addition or substitution reactions; those methods are well known to the skilled person. Likewise, the skilled artisan will apply - whenever necessary or useful - synthetic protecting (or protective) groups; suitable protecting groups as well as methods for introducing and removing them are well-known to the person skilled in the art of chemical synthesis and are described, in more detail, in, e.g., P.G.M. Wuts, T.W. Greene, "Greene's Protective Groups in Organic Synthesis", 4th edition (2006) (John Wiley & Sons).

It will be understood that several compounds or precursors of compounds forming the Q¹ part of the compounds of formula I, i.e. the ubiquitin ligase ligand, are commercially available or readibly available by well-known synthetic methods, including those described in the Experimental Part for exemplary compounds. Those compounds or precursors include, but are not limited to, thalidomide and lenalodimide and derivatives therof.

It will be further understood that several compounds or prescursors of compounds forming the Q² part of the compounds of formula I, i.e. the divalent linker, are commercially available or readibly available by well-known synthetic methods, including those described in the Experimental Part for exemplary compounds.

It will be further understood that several compounds or prescursors of compounds forming the Q³ part of the compounds of formula I, i.e. the TEAD ligand, are readibly available by well-known synthetic methods, including those described in the Experimental Part for exemplary compounds.

In the following general synthetic routes that may be utilized to prepare compounds or prescursors of compounds forming the Q³ part of the compounds of formula I are described in more detail in Schemes A, B, C, and D. 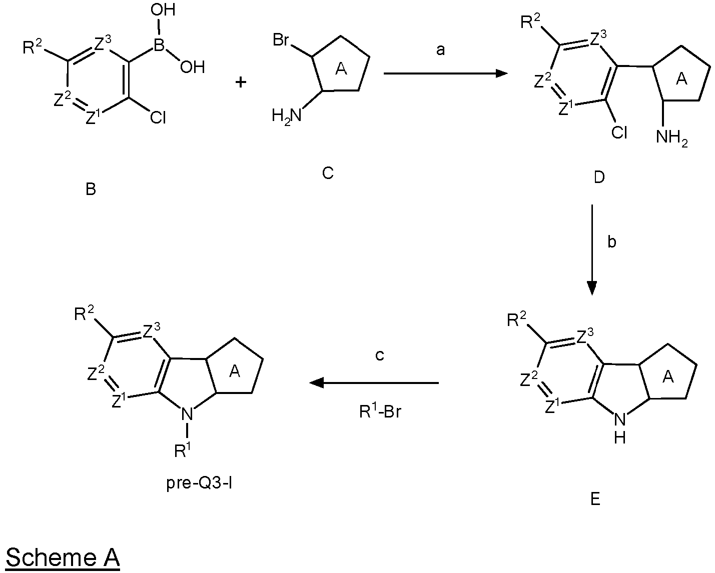 (Z¹, Z², Z³, R¹ and ring A are as defined for formula Q3-I above and in the claims; R² may be a suitable carboxylic acid derivative such as the free acid, a suitable salt thereof, an ester or an amide)

Scheme A above depicts a general synthesis route for preparing tricyclic hetereocycles of formula pre-Q3-I. In reaction step a the boronic acid B - which is readily available, for instance, by first reacting the respective bromo-substituted aryl or heteroaryl with a suitable organometallic base like n-butyl lithium and subsequent reaction with a suitable boron acid ester like B(OCH₃)₃ - is reacted with the 1-amino-2-bromo-substituted heterocycle C under typical C-C cross coupling conditions, e.g., under conditions typical for Suzuki cross coupling reactions (for instance, reacting a solution of B and C in a suitable solvent like 1,4-dioxane with cesium carbonate in the presence of a Palladium catalyst like Pd(dppf)₂Cl₂ (1,1'-Bis(diphenylphosphino)ferrocene]palladium(II) dichloride)) to yield compound D. It is understood that ring A in that 1-amino-2-bromo-substituted heterocycle C has the same meaning as "ring A" for structure Q3-I, i.e. is selected from the five-membered heteroaromatic rings A-1 to A-24 as defined above and in the claims. For instance, if ring A is selected to be ring A-1, then the respective compound C would have the following formula C-1:

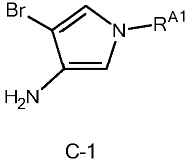

Compound D may then be subjected to an intra-molecular C-N cross-coupling reaction (step b), for instance, under conditions typical for a Hartwig-Buchwald reaction (e.g., reaction with cesium carbonate in a suitable solvent like 1,4-dioxane in the presence of a suitable palladium catalyst like di-tert-butyl[2',4',6'-tris(propan-2-yl)-[1,1'-biphenyl]-2-yl]phosphane {2'-amino-[1,1'-biphenyl]-2-yl}palladiumylium methanesulfonate) to yield the tricyclic heterocycle E. This heterocycle E may then in turn be reacted with the bromide R¹-Br in another C-N coupling reaction (step c) under similar conditions, for instance with cesium carbonate in the presence of a suitable palladium catalyst (e.g., Chloro(2-dicyclohexylphosphino-2',4',6'-triisopropyl-1,1'-biphenyl)[2-(2'-amino-1,1'-biphenyl)]palladium(II), X-Phos aminobiphenyl palladium chloride, XPhosPd G2) to provide the compound of the present invention of formula I. Depending on the nature of the various substituent R¹, R² and on ring A, this compound of formula pre-Q3-1 may optionally converted into further compounds of formula pre-Q3-I. For instance, if R² is a carboxylic ester (-C(=O)-OR²³), then this ester may be subjected to a saponification reaction using suitable acids or bases thereby providing either the respective carboxylic acid (R² = -C(=O)-OH) or a salt thereof (e.g., R² = -C(=O)-OCat with Cat being Li, Na, K or NH₄) or an amidation reaction with a suitable amine derivative to provide the respective amide.

In some instances compound D as shown in Scheme A above - instead of being subjected to the subsequent reaction steps b and c, i.e. two consecutive C-N coupling reactions - may be reacted with a suitable compound R¹-Br under C-N coupling reaction conditions (with as suitable base like cesium carbonate or sodium hydride in the presence of a suitable palladium catalyst) to directly provide the respective compound of formula pre-Q3-I.

Furthermore, it is well understood that starting from compound E compounds of formular pre-Q3-I may be synthesized by utilizing suitable reaction partners other than the bromo-substituted compound R¹-Br under suitable reaction conditions. For instance, if R¹ is chosen to be L¹-Ar or L¹-Hetar¹ with L¹ being -S(=O)₂-, then compound E may be reacted with the respective thionyl chloride under suitable reaction conditions to yield the respective sulfonyl derivative of pre-Q3-I. 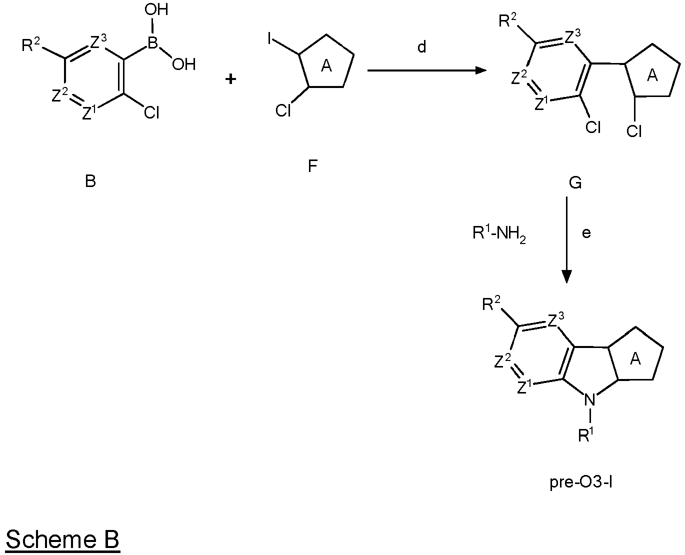 (Z¹, Z², Z³, R¹ and ring A are as defined for formula I-A above and in the claims; R² may be a suitable carboxylic acid derivative such as the free acid, a suitable salt thereof, an ester or an amide.)

Scheme B above depicts another synthetic route for making compounds of formula pre-Q3-I. Here the boronic acid B (or a suitable boronic acid ester) is reacted in a C-C cross-coupling reaction under similar conditions described for step a in Scheme A with the 1-chloro-2-iodo-substituted heterocycle F (step d) which reaction yields the dichloro-substituted compound G. Compound G may then be converted in a C-N coupling reaction with the primary amine R¹-NH₂ (step e) in the presence of a suitable base like cesium carbonate and a suitable palladium catalyst (as described for Scheme A) into the desired compound of pre-Q3-I. 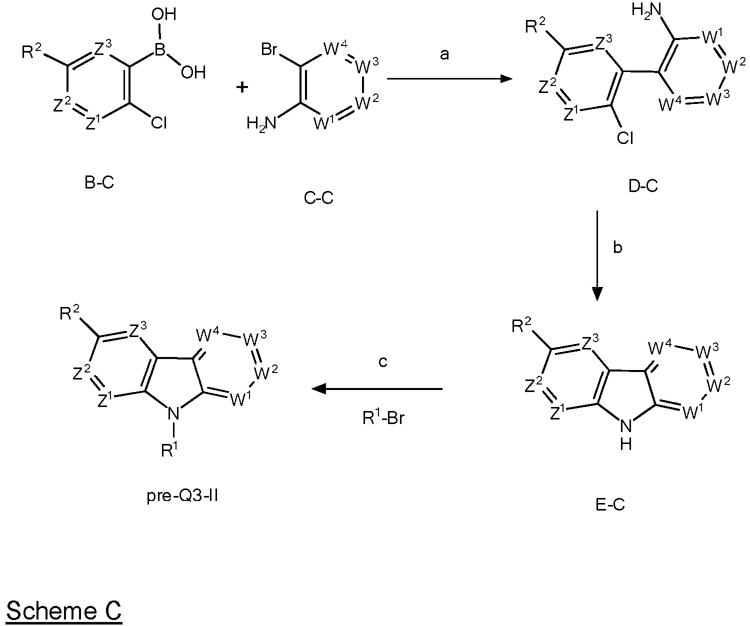 (Z¹, Z², Z³, R¹, W¹, W², W³ and W⁴ are as defined for formula Q3-II above and in the claims; R² may be a suitable carboxylic acid derivative such as the free acid, a suitable salt thereof, an ester or an amide.)

Scheme C above depicts a general synthesis route for preparing compounds of formula pre-Q3-II. In reaction step a the boronic acid B-C - which is readily available, for instance, by first reacting the respective bromo-substituted aryl or heteroaryl with a suitable organometallic base like n-butyl lithium and subsequent reaction with a suitable boron acid ester like B(OCH₃)₃ - is reacted with the 1-amino-2-bromo-substituted phenyl or heterocycle C-C under typical C-C cross coupling conditions, e.g., under conditions typical for Suzuki cross coupling reactions (for instance, reacting a solution of BC and C-C in a suitable solvent like 1,4-dioxane with cesium carbonate in the presence of a Palladium catalyst like Pd(dppf)₂Cl₂ (1,1'-Bis(diphenylphosphino)ferrocene]palladium(II) dichloride)) to yield compound D-C. Compound D-C may then be subjected to an intra-molecular C-N cross-coupling reaction (step b), for instance, under conditions typical for a Hartwig-Buchwald reaction (e.g., reaction with cesium carbonate in a suitable solvent like 1,4-dioxane in the presence of a suitable palladium catalyst like di-tert-butyl[2',4',6'-tris(propan-2-yl)-[1,1'-biphenyl]-2-yl]phosphane {2'-amino-[1,1'-biphenyl]-2-yl}palladiumylium methanesulfonate) to yield the tricyclic heterocycle E-C. This heterocycle E-C may then in turn be reacted with the bromide R¹-Br in another C-N coupling reaction (step c) under similar conditions, for instance with cesium carbonate in the presence of a suitable palladium catalyst (e.g., Chloro(2-dicyclohexylphosphino-2',4',6'-triisopropyl-1,1'-biphenyl)[2-(2'-amino-1,1'-biphenyl)]palladium(II), X-Phos aminobiphenyl palladium chloride, XPhosPd G2) to provide the compound of formula pre-Q3-II. Depending on the nature of the various substituents R¹, R² and of W¹, W²*,* W³ and W⁴, this compound of formula pre-Q3-II may optionally converted into further compounds of formula I. For instance, if R² is a carboxylic ester (-C(=O)-OR^{2a}), then this ester may be subjected to a saponification reaction using suitable acids or bases thereby providing either the respective carboxylic acid (R² = -C(=O)-OH) or a salt thereof (e.g., R² = -C(=O)-OCat with Cat being Li, Na, K or NH₄) or an amidation reaction with a suitable amine derivative to provide the respective amide.

In some instances compound D-C as shown in Scheme C above - instead of being subjected to the subsequent reaction steps b and c, i.e. two consecutive C-N coupling reactions - may be reacted with a suitable compound R¹-Br under C-N reaction conditions (with as suitable base like cesium carbonate or sodium hydride in the presence of a suitable palladium catalyst) to directly provide the respective compound of formula pre-Q3-II.

In some further instances compound D-C - before it is either converted into compound E-C or into compound pre-O3-II - may be modified by introducing suitable substituents at W¹, W²*,* W³ or W⁴. For instance, if in compound D-C W³ represents C-R^{W1} with R^{W1} being Br, then this bromo-substituted compound may be subjected to a suitable C-C coupling reaction to introduce another substituent R^{W1}, e.g. -CH₂-Ar^{W} to provide the respective compound D-C.

Furthermore, it is well understood that starting from compound E-C compounds of formula pre-Q3-II may be synthesized by utilizing suitable reaction partners other than the bromo-substituted compound R¹-Br under suitable reaction conditions. For instance, if R¹ is chosen to be L¹-Ar or L¹-Hetar¹ with L¹ being -S(=O)₂-, then compound E-C may be reacted with the respective thionyl chloride under suitable reaction conditions to yield the respective sulfonyl derivative of formula pre-Q3-II. 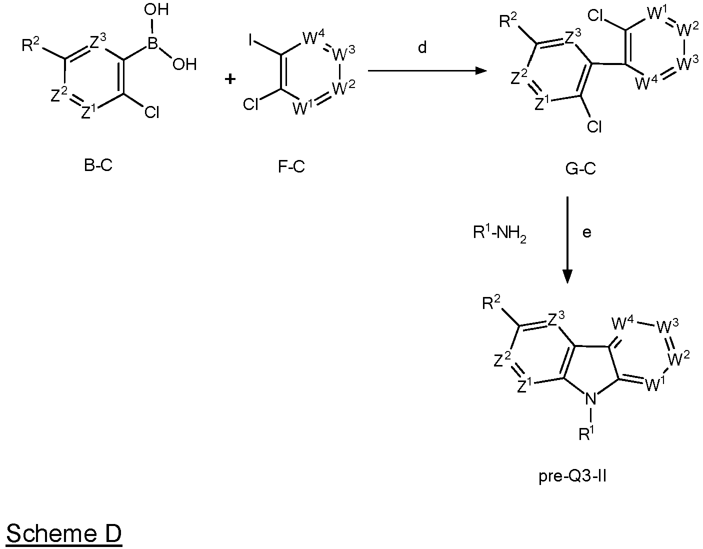 (Z¹, Z², R¹, W¹, W²*,* W³ and W⁴ are as defined for formula Q3-II above and in the claims; R² may be a suitable carboxylic acid derivative such as the free acid, a suitable salt thereof, an ester or an amide.)

Scheme D above depicts another synthetic route for making compounds of the formula pre-Q3-II. Here the boronic acid B-C (or a suitable boronic acid ester) is reacted in a C-C cross-coupling reaction under similar conditions described for step a in Scheme C with the 1-chloro-2-iodo-substituted heterocycle F-C (step d) which reaction yields the dichloro-substituted compound G-C. Compound G-C may then be converted in a C-N coupling reaction with the primary amine R¹-NH₂ (step e) in the presence of a suitable base like cesium carbonate and a suitable palladium catalyst (as described for Scheme A) into the desired compound of formula pre-Q3-II.

Further synthetic routes and procedures for preparing compounds or precursors of compounds forming the Q³ part of the compounds of formula I are disclosed in WO 2021/224291 A1 (Q3-I) and WO 2022/018072 A1.

It will also be understood that the methods for preparation of compounds of formula I by linking or connecting the compounds or prescursors of compounds forming the parts Q¹, Q² and Q³ are in general well known to those skilled in the art. These methods may become apparent from the procedures described in detail in the Experimental Part for exemplary compounds and include ether-forming reactions, esterification reactions, and amidation reactions. With regard to synthetic methods for making bifunctional degraders, like the compounds of formula I, and prescursors thereof it is also referred to Sosič et al., Chem. Soc. Rev., 2022, 51, 3487-3534, and the references cited therein.

Another particular embodiment of the invention, PE9, comprises some of the compounds which serve as intermediates in the synthesis of the compounds of formula I. Thus, PE9 comprises a compound selected from the group consisting of the intermediates depicted in Table **INT** below, or a pharmaceutically acceptable salt, solvate, tautomer and/or stereoisomer thereof. The syntheses of those intermediates are described in the Experimental Part.

**Table INT**

| **Intermediate** | **Structure** |
|---|---|
| 1 | 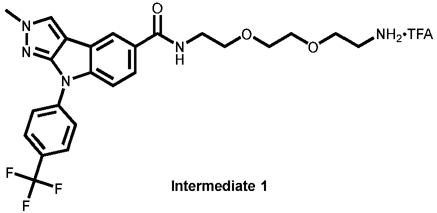 |
| 8 | 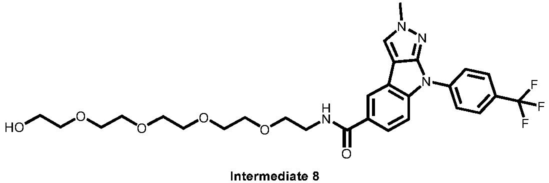 |
| 9 | 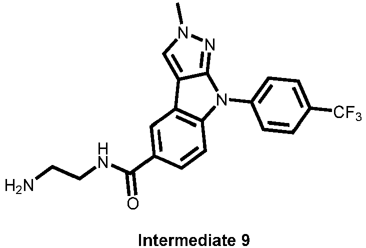 |
| 10 | 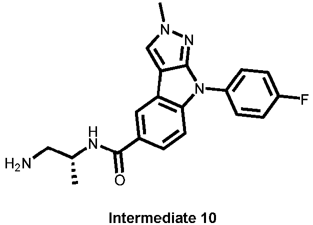 |
| 11 | 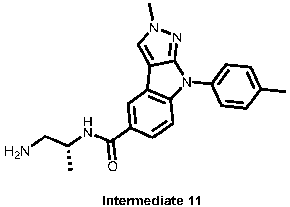 |
| 20 | 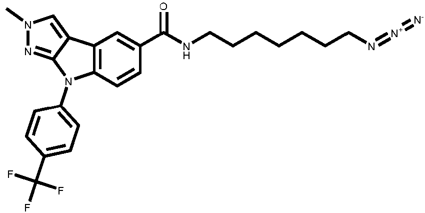 |
| 29 | 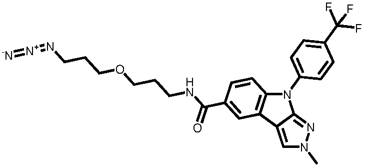 |
| 33 | 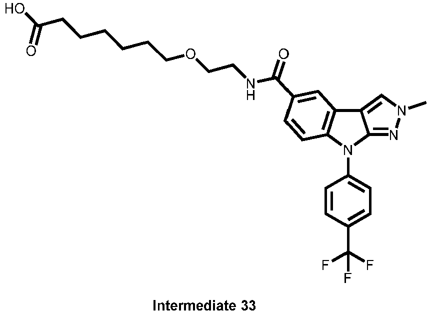 |
| 34 | 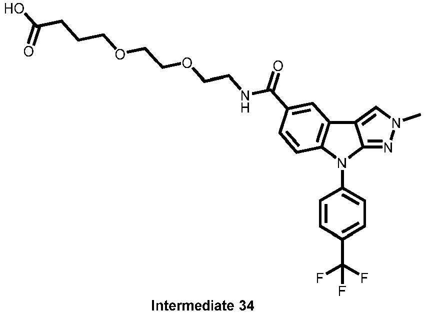 |
| 37 | 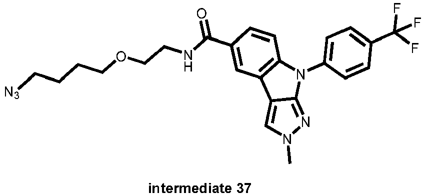 |
| 38 | 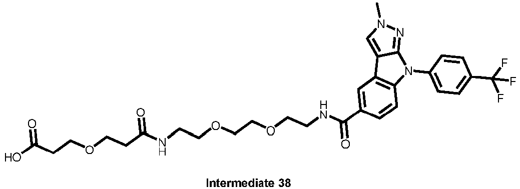 |
| 39 | 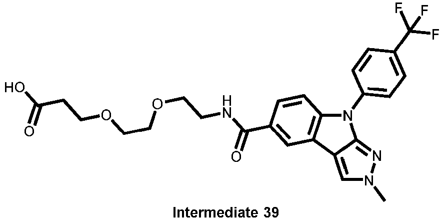 |
| 40 | 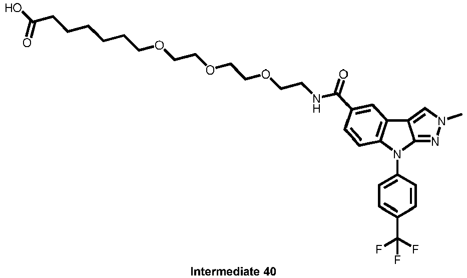 |
| 41 | 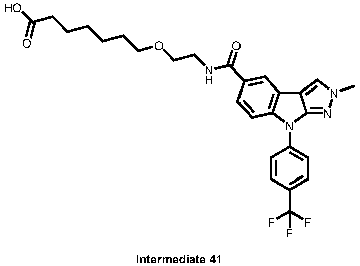 |
| 43 | 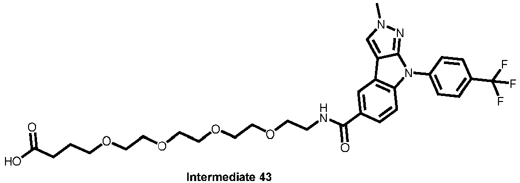 |
| 45 | 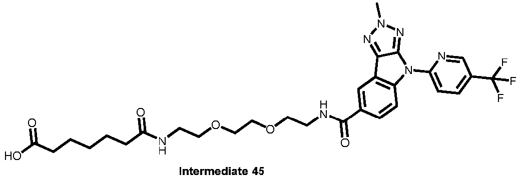 |
| 46 | 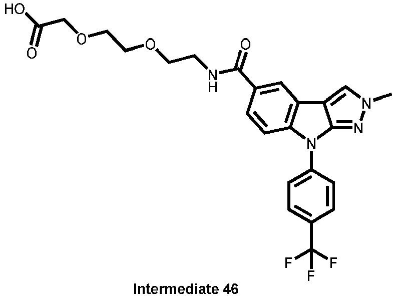 |
| 47 | 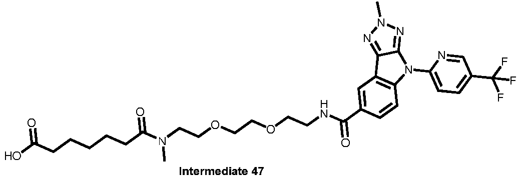 |
| 68 | 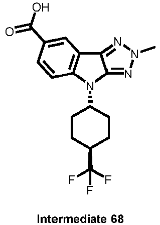 |
| 71 | 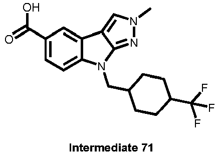 |

It is to be noted that - except for instances where it is specifically stated or the context provides for a different meaning - in general the number of a term, i.e. its singular and plural form, is used and can be read interchangeably. For example, the term "compound" in its singular form may also comprise or refer to a plurality of compounds, while the term "compounds" in its plural form may also comprise or refer to a singular compound.

### Examples and Experimental Part

The compounds of the present invention can be prepared according to the procedures of the following Schemes and Examples, using appropriate materials and are further exemplified by the following specific examples. The compounds are shown in Table 1. Analytical data of compounds made according to the following examples are shown with the examples.

The invention will be illustrated, but not limited, by reference to the specific embodiments described in the following examples. Unless otherwise indicated in the schemes, the variables have the same meaning as described above and in the claims.

Unless otherwise specified, all starting materials are obtained from commercial suppliers and used without further purifications. Unless otherwise specified, all temperatures are expressed in °C and all reactions are conducted at room temperature (RT). Compounds are purified by either silica chromatography or preparative HPLC.

### ¹H NMR:

¹H-NMR data is provided in in the experimental part below for individual compounds. ¹H NMR spectra were usually acquired on a Bruker Avance DRX 500, Bruker Avance 400, a Bruker DPX 300 NMR or a a Bruker Avance III 700 MHz spectrometer under standard conditions using TMS (tetramethylsilane) as internal reference and DMSO-d6 as standard solvents, if not reported otherwise. NS (Number of Scans): 32. TE (Temperature): 297 K. Chemical shifts (δ) are reported in ppm relative to the TMS signal. ¹H NMR data are reported as follows: chemical shift (multiplicity, coupling constants and number of hydrogens). Multiplicity is abbreviated as follows: s (singlet), d (doublet), t (triplet), q (quartet), m (multiplet), dd (doublet of doublets), tt (triplet of triplets), td (triplet of doublets) br (broad) and coupling constants (J) are reported in Hz.

### LC-MS:

LC-MS data provided in the experimental part below for individual compounds are given with mass in m/z. The results can be obtained by one of the methods described below.

### Syntheses

The following abbreviations have the meaning given in parantheses behind the specific abbreviation:
aq (aqueous), h (hour), g (gram), L (liter), mg (milligram), MHz (Megahertz), min. (minute), mm (millimeter), mmol (millimole), mM (millimolar), m.p. (melting point), eq (equivalent), mL (milliliter), ACN (acetonitrile), AcOH (acetic acid), CDCl₃ (deuterated chloroform), CD₃OD (deuterated methanol), CH₃CN (acetonitrile), c-hex (cyclohexane), DCC (dicyclohexyl carbodiimide), DCM (dichloromethane), DIC (diisopropyl carbodiimide), DIEA (diisopropylethylamine), DMF (dimethylformamide), DMSO (dimethylsulfoxide), DMSO-d₆ (deuterated dimethylsulfoxide), DMT-Si (Dimercaptotriazine-functionalized silica gel), EDC (1-(3-dimethyl-amino-propyl)-3-ethylcarbodiimide), ESI (Electro-spray ionization), EtOAc (ethyl acetate), Et₂O (diethyl ether), EtOH (ethanol), HATU (dimethylamino-([1,2,3]triazolo[4,5-b]pyridin-3-yloxy)-methylene]-dimethyl-ammonium hexafluorophosphate), G-II catalyst (1,3-Bis(2,4,6-trimethylphenyl)-2-imidazolidinylidene)dichloro(phenylmethylene)-(tricyclohexylphosphine)ruthenium), HPLC (High Performance Liquid Chromatography), i-PrOH (2-propanol), K₂CO₃ (potassium carbonate), LC (Liquid Chromatography), MeOH (methanol), MgSO₄ (magnesium sulfate), MS (mass spectrometry), MTBE (Methyl tert-butyl ether), NaHCO₃ (sodium bicarbonate), NaBH₄ (sodium borohydride), NMM (N-methyl morpholine), NMR (Nuclear Magnetic Resonance), oNsCl (2-Nitrobenzenesulfonyl chloride), Pd(dppf)Cl₂ ([1,1'-bis(diphenylphosphino)ferrocene]dichloro-palladium(II)), Pd(dtbpf)Cl₂ (1,1'-Bis-(di-tert.-butylphosphino-)ferrocen-palladiumdichlorid), PyBOP (benzotriazole-1-yl-oxy-tris-pyrrolidino-phosphonium hexafluorophosphate), RBF (round-bottom flask), RT (room temperature), Rt (retention time), Si-DMT (Dimercaptotriazine-functionalized Silica gel), SPE (solid phase extraction), TBTU (2-(1-H-benzotriazole-1-yl)-1,1,3,3-tetramethyluromium tetrafluoro borate), TEA (triethylamine), TFA (trifluoroacetic acid), THF (tetrahydrofuran), TLC (Thin Layer Chromatography), UV (Ultraviolet), XPhos Pd G2 (chloro(2-dicyclohexylphosphino-2',4',6'-triisopropyl-1,1'-biphenyl)[2-(2'-amino-1,1'-biphenyl)]palladium(II)), XPhos Pd G4 ((2-Dicyclohexylphosphino-2',4',6'-triisopropyl-1,1'-biphenyl)[2-(2'-dimethylamino-1,1'-biphenyl)]palladium(II) methanesulfonate)

Above and below, all temperatures are indicated in °C. In the following examples, "conventional work-up" means: water is added if necessary, the pH is adjusted, if necessary, to values between 2 and 10, depending on the constitution of the end product, the mixture is extracted with ethyl acetate or dichloromethane, the phases are separated, the organic phase is dried over sodium sulfate and evaporated, and the residue is purified by chromatography on silica gel and/or by crystallisation. Rf values on silica gel; eluent: ethyl acetate/methanol 9:1.

All synthetic procedures were carried out under air unless otherwise noted, using analytical grade solvents. The use of "water" refers to deionized water. Reaction progress was checked by LC-MS or TLC analysis (using known visualization methods). The following method abbreviations are used in the synthetic procedures to describe conditions for analytical LC-MS. Analytical LC-MS method "Sunfire": Solvent A: H2O + 0.05% HCOOH, B: MeCN + 0.04% HCOOH + 1% H2O, T: 40 °C, Flow: 1,4 ml/min, MS: 61-1000 amu positive (ESI+), Column: SunFire C18 5,0 µm 100-3 mm, 1% - > 99% B: 0 -> 2,0 min then 99% B: 2,0 -> 2,7 min. Analytical LC-MS method "Chromolith": Solvent A: H2O + 0,05% HCOOH | B: MeCN + 0,04% HCOOH / 4% -> 100% B: 0 -> 2,8 min | 100% B: 2,8 -> 3,3 min, T: 40 °C, Flow: 3,3 ml/min, MS: 100-2000 amu positive, Column: Chromolith RP-18e 50-4,6 mm. Method 1: LC-MS Agilent 1200 Series Chromolith RP-18e 50-4,6mm; 3.3 ml/min solvent A: Water + 0.05% HCOOH solvent B: Acetonitrile + 0.04% HCOOH 220 nm 0 to 2.0 min:1%B to 99%B 2.0 to 2.5 min: 100%B. Method 2: Column:HALO,3.0*30mm,2um;Column Oven:40C;Mobile phase A:Water/0.1% FA;Mobile phase B:Acetonitrile/0.1% FA. Method 3: LC-MS Agilent 1200 Series Sunfire C18 100-3 mm; 1,4 ml/min solvent A: Warer+0.05% HCOOH solvent B: Acetonitrile+0.04% HCOOH 220 nm 0 to 2.0 min:1%B to 99%B 2.0 to 2.7 min: 99%B. Method 4: Column:Halo C18,100 mm,4.6 mm; Column Oven: 40 C;Mobile Phase A:6.5mM NH4HCO3+ NH4OH (pH010),Mobile Phase B:Acetonitrile. Method 5: LC-MS Agilent 1200 Series Cortec T3 30-4,6 mm; 0.9 ml/min solvent A: Water + 0.05% HCOOH solvent B: Acetonitrile + 0.04% HCOOH 220 nm 0 to 0.8 min:1%B to 99%B 0.8 to 1.05 min: 99%B. Method 6: UPLC Cortecs T3 30-2,1 mm; 0.9 ml/min solvent A: Water + 0.05% HCOOH solvent B: Acetonitrile + 0.04% HCOOH 220 nm 0 to 0.6 min:2%B to 99%B 0.6 to 0.8 min: 99%B. Method 7: Column:HALO,3.0*30mm,2um;Mobile phase A:Water/0.05% TFA;Mobile phase B:Acetonitrile/0.05% TFA. Method 8: UPLC Kinetex EVO-C18 50-2,1 mm; solvent A: Water + 0.05% HCOOH; solvent B: Acetonitrile + 0,04% HCOOH; flow 0,9ml; 1% -> 99% B: 0 -> 1,0 min | 99% B: 1,0 -> 1,3 min. Method 9: Column: Agilent HPH, 3.0 x 50 mm, 2.7 µm;Mobile Phase A:6.5mM NH4HCO3+NH4OH(pH=10)Mobile Phase B: AcetonitrileFlow rate: 1.2mL/min; Gradient:10%B to 95%B in 1.9 min, hold 0.8 min; 254nm. Method 10: LC-MS Agilent 1200 Series Chromolith RP-18e 50-4,6mm; 3.3 ml/min solvent A: Water + 0.1% TFA solvent B: Acetonitrile + 0.1% TFA 220 nm 0 to 2.0 min:1%B to 99%B 2.0 to 2.5 min: 99%B. Method Himass: A: H2O + 0,05% HCOOH, B: MeCN + 0,04% HCOOH, T: 45 °C, Flow: 3,3 ml/min, MS: 100-2000 amu positive, Column: Chromolith HR RP-18e 50-4,6 mm. Gradient: 1% -> 99% B: 0 -> 2,0 min 99% B: 2,0 -> 2,5 min. Preparative RP-HPLC separations were carried out on a AcquPrep machine (Teledyne Isco) using Sunfire or Chromolith columns (0.1% formic acid or TFA as modifiers with ACN/H₂O). Method A: Column: Waters XBridge C18 3.5um, 50*4.6mm; SolventA:water+0.1% TFA; Solvent: ACN; Flow:1.5ml/min; Time:6.5min; Gradient :0.15 min: 10 % B, 4.5 min£°80 % B£¬4.6 min: 95 % B, 6.0 min: 95 % B, 6.1 min: 5% B, 6.5 min: 5% B.

### Buildling block 1

### Ethyl 4-chloro-3-(4,4,5,5-tetramethyl-1,3,2-dioxaborolan-2-yl)benzoate

### (Building block 1)

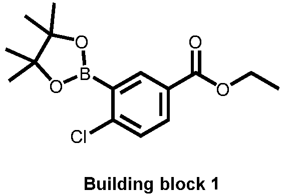

Into a 50-L 4-necked round-bottom flask purged and maintained with an inert atmosphere of nitrogen, was placed a solution of 3-bromo-4-chlorobenzoic acid (3000.00 g, 12741.01 mmol, 1.00 equiv) in EtOH (30 L). This was followed by the addition of H₂SO₄ (1250 g, 12741 mmol) dropwise at 0°C. The resulting solution was stirred for 12 h at 70°C. The reaction mixture was cooled to 25°C, then concentrated. The mixture was then quenched by the addition of 5 L of water/ice. The resulting solution was diluted with 10 L of MTBE, extracted water phase with 2x5 L of MTBE. The combined organic phase was washed with 1x5 L of NaHCO₃ (10%), then washed with 1x5 L of Brine, concentrated to 2 L, slurried with PE (9 L). This resulted in 3023 g (90%) of ethyl 3-bromo-4-chlorobenzoate as a white solid.

Into a 50-L 4-necked round-bottom flask purged and maintained with an inert atmosphere of nitrogen, was placed a solution of ethyl 3-bromo-4-chlorobenzoate (3023 g, 11472 mmol), bis(pinacolato)diboron (5826 g, 22943 mmol), KOAc (2252 g, 22943 mmol), Pd(dppf)Cl₂ (504 g, 688 mmol) in Dioxane (30 L), then purged with N₂ for 3 times. The resulting solution was stirred for 12 h at 100°C. The reaction was cooled to 25°C, quenched by the addition of 3 L of water/ice. The resulting solution was diluted with 3 L of EtOAc. The resulting solution was extracted with 2x4 L of ethyl acetate and the organic layer was washed with 1x3 L of Brine. The mixture was dried over anhydrous sodium sulfate and concentrated. The residue was applied onto a silica gel column with ethyl acetate/petroleum ether (1:1). This resulted in 2300 g (65%) of ethyl 4-chloro-3-(4,4,5,5-tetramethyl-1,3,2-dioxaborolan-2-yl)benzoate **(building block 1)** as a yellow solid.

### Building block 2

### 4-bromo-1-methylpyrazol-3-amine (Building block 2)

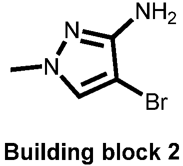

Into a 20-L 4-necked round-bottom flask purged and maintained with an inert atmosphere of nitrogen, was placed a solution of 1-methylpyrazol-3-amine (900 g, 9267 mmol) in DCM (9 L). This was followed by the addition of Br₂ (1407 g, 8803 mmol) dropwise with stirring at 15°C in 2 h. The resulting solution was stirred for 1 h at 15°C, then solids were collected by filtration. The resulting solids were dissolved with 2 L of H₂O. The pH value of the solution was adjusted to 9 with NaOH (1 mol/L). The mixture was extracted with 3x3 L of ethyl acetate. The organic layers were combined, washed with 1x3 L of brine, dried with Na₂SO₄. The resulting mixture was concentrated to 2 L, the solids were collected by filtration. This resulted in 1100 g (67%) of 4-bromo-1-methylpyrazol-3-amine **(building block 2)** as a light brown solid. LC-MS: (ES, m/z): 176 [M+H]⁺ 1H-NMR (300 MHz, DMSO-d6, ppm): δ 7.53 (s, 1H), 4.66 (s, 2H), 3.58 (s, 3H).

### Building block 3

### Ethyl 3-(3-amino-1-methylpyrazol-4-yl)-4-chlorobenzoate (Building block 3)

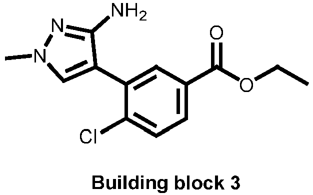

Into a 20-L 4-necked round-bottom flask purged and maintained with an inert atmosphere of nitrogen, was placed a solution of **building block 2** (1000 g, 5681 mmol) and **building block 1** (2294 g, 7386 mmol) in Dioxane (10 L). Then a solution of K₂CO₃ (1570 g, 11363 mmol) in H₂O (1 L) and Pd(dppf)Cl₂ (457 g, 625 mmol) was added and purged with N₂ for 3 times. The resulting mixture was stirred for 8 h at 100°C and then cooled to 25°C, followed by the addition of 4 L of water/ice. The resulting solution was diluted with 4 L of EtOAc and extracted with 2x3 L of ethyl acetate. The organic phase was washed with 1x3 L of Brine. The mixture was dried over anhydrous sodium sulfate and concentrated. The residue was applied onto a silica gel column with ethyl acetate/petroleum ether (1:1). This resulted in 730 g (46%) of ethyl 3-(3-amino-1-methylpyrazol-4-yl)-4-chlorobenzoate **(building block 3)** as a brown solid.

### Building block 4

### Ethyl 2-methyl-8-[4-(trifluoromethyl)phenyl]-2H,8H-pyrazolo[3,4-b]indole-5-carboxylate (Building block 4)

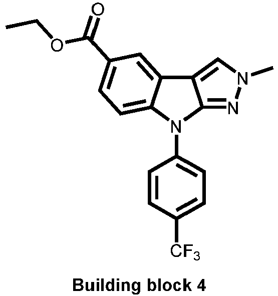

Into a 20-L 4-necked round-bottom flask purged and maintained with an inert atmosphere of nitrogen, was placed a mixture of **building block 3** (730 g, 2610 mmol), 1-bromo-4-(trifluoromethyl)benzene (881 g, 3915 mmol), Cs₂CO₃ (1701 g, 5220 mmol) and XPhos-Pd-G4 (233 g, 261 mmol) in Dioxane (7.3 L), which was then purged with N₂ for 3 times. The resulting mixture was stirred for 8 h at 100°C and then cooled to 25°C. The reaction was then quenched by the addition of 3 L of water/ice. The resulting solution was diluted with 3 L of EtOAc. The resulting solution was extracted with 2x4 L of ethyl acetate and the organic layer was washed with 1x3 L of Brine. The mixture was dried over anhydrous sodium sulfate and concentrated. The residue was applied onto a silica gel column with ethyl acetate/petroleum ether (1:1). The crude product was slurrieded with petroleum ether (3 L). This resulted in 720 g (71 %) of ethyl 2-methyl-8-[4-(trifluoromethyl)phenyl]pyrazolo[3,4-b]indole-5-carboxylate as a light brown solid. The solid was dissolved with 3L THF, then Si-DMT (1000 g) was added, the mixture was stirred at 25°C overnight, the filtrate was collected and evaporated to get 715 g product **(building block 4).** LC-MS: (ESI, m/z): 388 [M+H]⁺ 1H-NMR: (300 MHz, DMSO-d6, ppm): δ 8.44 (d, J = 1.7 Hz, 1H), 8.22 (s, 1H), 8.07 (d, J = 8.5 Hz, 2H), 8.00-7.90 (m, 3H), 7.78 (d, J = 8.7 Hz, 1H), 4.35 (q, J = 7.1 Hz, 2H), 4.03 (s, 3H), 1.36 (t, J = 7.1 Hz, 3H).

### Building block 5

### 8-(4-fluorophenyl)-2-methyl-2H,8H-pyrazolo[3,4-b]indole-5-carboxylic acid (building block 5)

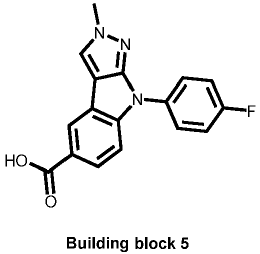

A mixture of **building block** 3 (200 mg; 0.64 mmol), 1-bromo-4-fluorobenzene (135 mg; 0.77 mmol), XPhosPd G2 (56 mg; 0.07 mmol) and Cs₂CO₃ (629 mg; 1.93 mmol) in 1,4-Dioxane (10 mL). The mixture was stirred under N₂ at 120°C for 16 h and then filtered. The organic phase was concentrated and purified by chromatography (SiO₂, petroleum ether:EtOAc 10:1) to obtain ethyl 8-(4-fluorophenyl)-2-methyl-2H,8H-pyrazolo[3,4-b]indole-5-carboxylate (186 mg; 0.52 mmol; 81 %) as a light-yellow solid. To a solution of this ester (180 mg; 0.51 mmol) in EtOH (5 mL) was added 1M sodium hydroxide aqueous solution (1.70 mL). The mixture was stirred under N₂ at 60 °C for 16h and then concentrated. To the residue was added H₂O (15 mL) and adjusted by 1N hydrochloric acid to pH = 1. After filtration, the filter cake was washed by H₂O (10 mL * 3). To the filter cake was added EtOAc (3 mL) and N-hexane (3 mL), then stirred for 30 min. The suspension was filtered off, the filter cake was dried by vacuum to provide **building block 5** (100 mg; 0.31 mmol; 62 %) as a white solid. LC-MS: (ESI+, Method A,, m/z): 309 [M+H]⁺. ¹H NMR (400 MHz, DMSO) 12.65 (s, 1H), 8.41 (d, J = 1.4 Hz, 1H), 8.18 (s, 1H), 7.90 (dd, J = 8.7, 1.6 Hz, 1H), 7.83 ¨C 7.77 (m, 2H), 7.54 (d, J = 8.7 Hz, 1H), 7.49 ¨C 7.42 (m, 2H), 4.01 (s, 3H).

### Building block 6

### 2-methyl-8-(4-methylphenyl)-2H,8H-pyrazolo[3,4-b]indole-5-carboxylic acid (building block 6)

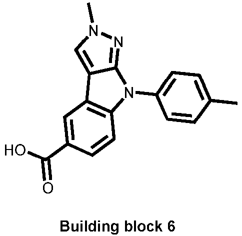

A mixture of **building block 3** (200 mg; 0.64 mmol), 1-bromo-4-methylbenzene (132 mg; 0.77 mmol), XPhosPd G2 (56 mg; 0.07 mmol) and Cs₂CO₃ (629 mg; 1.93 mmol) in Dioxane-1,4 (10 mL) was stirred under N₂ at 120 °C for 16 h. The mixture was filtered, the organic phase was concentrated and purified by chromatography (SiO₂, PE:EA 10:1) to obtain ethyl 2-methyl-8-(4-methylphenyl)-2H,8H-pyrazolo[3,4-b]indole-5-carboxylate (207 mg; 0.58 mmol; 91%) as a light yellow solid. To a solution of this ester (200 mg; 0.57 mmol) in EtOH (6 mL) was added 1M sodium hydroxide aqueous solution (2 mL). The mixture was stirred under N₂ at 60 °C for 2 h and then concentrated. To the residue was added H₂O (10 mL) and the pH was adjusted by 1N hydrochloric acid to pH = 1. The suspension was filtered, the filter cake was washed by H₂O (15 mL * 3). The filter cake was dried in vacuum to provide building block 6 (160 mg; 0.50 mmol; 87%) as an off-white solid. LC-MS: (ESI+, Method A, m/z): 305 [M+H]⁺. ¹H NMR (400 MHz, DMSO) 12.60 (s, 1H), 8.40 (d, J = 1.5 Hz, 1H), 8.17 (s, 1H), 7.89 (dt, J = 5.2, 3.3 Hz, 1H), 7.63 (d, J = 8.3 Hz, 2H), 7.54 (d, J = 8.7 Hz, 1H), 7.41 (d, J = 8.2 Hz, 2H), 4.00 (s, 3H), 2.40 (s, 3H).

### Building block 7

### 8-{6,6-difluorospiro[3.3]heptan-2-yl}-2-methyl-2H,8H-pyrazolo[3,4-b]indole-5-carboxylic acid (building block 7)

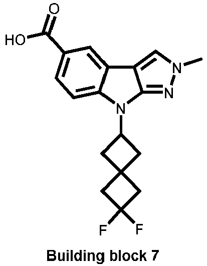

Step 1. **Building block 3** (450.0 mg; 1.419 mmol; 1.00 eq.) and 6,6-difluorospiro[3.3]heptan-2-one (311 mg; 2.128 mmol; 1.50 eq.) were filled in the vial and dissolved in anhydrous DCM (5.625 ml). To the brown solution glacial acetic acid (227 µl; 3.972 mmol; 2.80 eq.) was added. The reaction mixture was stirred under argon atmosphere at 40°C (heating block temperature) for 1h. Then the vial was cooled down to room temperature, Sodium triacetoxyborohydride (633 mg; 2.837 mmol; 2.00 eq.) was added causing gas evolution. Stirring was continued at room temparature for 2 h under pressure equalization. As the reaction was not complete, further 6,6-difluorospiro[3.3]heptan-2-one (311 mg; 2.128 mmol; 1.50 eq.), glacial acetic acid (227 µl; 3.972 mmol; 2.80 eq.) and Sodium triacetoxyborohydride (633 mg; 2.837 mmol; 2.00 eq.) were added and stirring was continued at room temperature for 16.5 h. The reaction mixture was neutralized with 5 mL saturated ammonium chloride solution, diluted with 10 mL water and extracted three times with ethyl acetate (10 mL each). The colourless aqueous phase (pH = 5) did not contain any product and was discarded. The combined organic phases (brown) were dried over sodium sulfate, filtered with suction and evaporated in vacuo. The crude product (1.2491g brown oil) was fused onto Isolute HM-N^{®} and purified by Isco CombiFlash Rf^{®} (flash chromatography, 12g SiO2, 0-60%EE). The fractions containing the desired product were combined and evaporated in vacuo. The crude product (971.7mg yellow-brown oil) was fused onto Isolute HM-N^{®} and purified by Isco CombiFlash Rf^{®} (flash chromatography, 24g SiO2, 0-20%EE). The pure fractions containing the desired product were combined and evaporated in vacuo to give ethyl 4-chloro-3-[3-({6,6-difluorospiro[3.3]heptan-2-yl}amino)-1-methyl-1H-pyrazol-4-yl]benzoate (449.4 mg; 1.073 mmol; 68%) as a yellow oil, which crytallized over night to give a yellow solid. LC-MS (ESI+, Method 10): tR = 1.70 min, m/z 410.1 [M+H]+.

Step 2. The product from the previous step (399.4 mg; 0.954 mmol; 1.00 eq.) and Cesium carbonate (622 mg; 1.907 mmol; 2.00 eq.) were filled into a vial and suspended in anhydrous1,4-Dioxane (6 mL). The pale yellow suspension was placed under argon atmosphere by three cycles of evacuation - degassing in an ultrasonic bath - inlet of argon. Then XPhos Pd G4 95% (129.6 mg; 0.143 mmol; 0.15 eq.) was added. The pale yellow suspension was stirred under argon atmosphere at 110°C (heating block temperature) for 17 h. The reaction mixture was filtered through 0.6 g Celite. The residue was washed once with 10mL ethyl acetate. Then the black residue was discarded. The black filtrate was diluted with 10 mL deionized water and 5 mL saturated sodium chloride solution (NaCl) and the phases were separated. The aqueous phase was extracted twice with ethyl acetate (10 mL each). The colourless aqueous phase (pH = 6) did not contain any product and was discarded. The combined organic phases (brown) were dried over sodium sulfate (Na₂SO₄), filtered with suction and evaporated in vacuo. The crude product (554.4mg dark brown oil) was fused onto Isolute HM-N^{®} and purified by Isco CombiFlash Rf^{®} (flash chromatography, 12g SiO2; 0-20%EE). The pure fractions containing the desired product were combined and evaporated in vacuo to give ethyl 8-{6,6-difluorospiro[3.3]heptan-2-yl}-2-methyl-2H,8H-pyrazolo[3,4-b]indole-5-carboxylate (369,400 mg; 0,915 mmol; 85,28%) as a brown oil. LC-MS (ESI+, Method 10): tR = 1.83 min, m/z 374.1 [M+H]+.

Step 3. The product from the previous step (369.400 mg; 0.915 mmol; 1.00 eq.) was filled into a vial and dissolved in Tetrahydrofuran (3.7 ml). To the yellow-brown solution water (1.85 ml) was added, which caused a two-phase system to form. Then aq. Lithium hydroxide solution (0.035 ml; 3.660 mmol; 4.00 eq.) was added. The yellow-brown suspension was stirred at 65°C (heating block temperature) for 16 h, followed by another portion of LiOH solution (0.035 ml; 3.660 mmol; 4.00 eq.) and stirring for 47 h at 65 °C. The cooled reaction mixture was diluted with 50 mL deionized water and extracted three times with tert-butyl methyl ether (10 mL each). The combined organic phases were washed three times with a mixture of water (10 mL each) and sodium hydroxide solution (NaOH, c=1mol/L, 1mL each). The colourless organic phase did not contain any desired product and was discarded. The off-white aqueous phase (suspension) was acidified with 9 mL hydrochloric acid (HCl, c=2mol/L), which caused the suspension to thicken. Then it was extracted three times with ethyl acetate (20 mL each). The colourless aqueous phase (pH=1) did not contain any product and was discarded. The combined organic phases (pale yellow) were dried over sodium sulfate (Na₂SO₄), filtered with suction and evaporated in vacuo to give 8-{6,6-difluorospiro[3.3]heptan-2-yl}-2-methyl-2H,8H-pyrazolo[3,4-b]indole-5-carboxylic acid (261,300 mg; max. 0,712 mmol; max. 77,83 %) as the crude product in insufficient purity. A portion (61.3 mg) of this crude material was purified by RP-HPLC, followed by extraction of the resultant target fractions by EtOAc (3 x 10 mL). The combined organic phases were dried over sodium sulfate (Na₂SO₄), filtered with suction and evaporated in vacuo. The beige residue was suspended in 3mL methanol, centrifugated under vacuum at 55 °C for 45 min and dried in a vacuum drying oven at 60°C and 10-20mbar for 19h to give 8-{6,6-difluorospiro[3.3]heptan-2-yl}-2-methyl-2H,8H-pyrazolo[3,4-b]indole-5-carboxylic acid **building block 7** (29.100 mg; 0.084 mmol; 9.21%) as an off-white solid. LC-MS (ESI+, Method 10): tR = 1.50 min, m/z 346.1 [M+H]+.

### TEAD ligand 1 - sodium carboxylate and free acid form

### Sodium 2-methyl-8-[4-(trifluoromethyl)phenyl]pyrazolo[3,4-b]indole-5-carboxylate (TEAD ligand 1) and 2-methyl-8-[4-(trifluoromethyl)phenyl]-pyrazolo[3,4-b]indole-5-carboxylic acid (TEAD ligand 1 free acid)

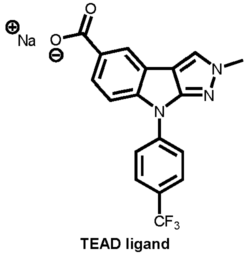

Into a 25-mL 3-necked round-bottom flask purged and maintained with an inert atmosphere of nitrogen, was placed a solution of ethyl 2-methyl-8-[4-(trifluoromethyl)phenyl]pyrazolo[3,4-b]indole-5-carboxylate **(Building block 4)** (1 g, 2.58 mmol) in EtOH/H₂O = 4:1 (10 mL) and NaOH (0.11 g, 2.75 mmol). The reaction mixture was stirred for 3 h at 40°C. After concentration of the reaction mixture, it was diluted with 20 mL of H₂O. The pH value of the solution was adjusted to 3 with HCl (1 N) and then extracted with 2 x 50 mL of ethyl acetate. After evaporation, the crude was re-dissolved in 20 mL acetone, and Na₂CO₃ (0.27 g, 2.54 mmol) was added. The resulting solution was stirred for 2 h at 40°C, the excess Na₂CO₃ was filtered out and the filtrate was concentrated. This resulted in 1 g (99%) of sodium 2-methyl-8-[4-(trifluoromethyl)phenyl]pyrazolo[3,4-b]indole-5-carboxylate **(TEAD ligand 1)** as a light brown solid. LC-MS: (ES, m/z): 360 [M+1]⁺ 1H-NMR (300 MHz, DMSO-d6, ppm): δ 8.32 (d, J = 1.5 Hz, 1H), 8.19-8.04 (m, 3H).

The free acid **(TEAD ligand 1 free acid)** can be accessed in the following manner: To the residue was added H₂O (15 mL per 200 mg of carboxylate) and adjusted by 1 M aq. hydrochloric acid to pH = 1. After filtration, the filter cake was washed by H₂O (10 mL * 3, per 200 mg of carboxylate). To the filter cake was added EtOAc (3 mL per 200 mg of carboxylate) and N-hexane (3 mL per 200 mg of carboxylate), then stirred for 30 min. The suspension was filtered off, the filter cake was dried by vacuum to provide the free acid in quantitative yield from the carboxylate form.

### TEAD ligand 2

### 2-methyl-4-[4-(trifluoromethyl)phenyl]-2H,4H-[1,2,3]triazolo[4,5-b]indole-7-carboxylic acid (TEAD Ligand 2)

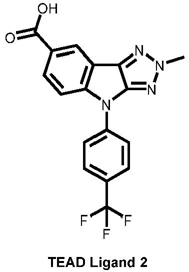

Step 1. To a solution of 2-methyl-2H-1,2,3-triazol-4-amine (commercially available from Enamine, 9.3 g; 90.00 mmol; 1.20 eq.) in anhydrous DMSO (100.0 ml) was added unter argon Potassium tert-butylate for synthesis (25.5 g; 224.99 mmol; 3.00 eq.). The reaction was stirred for 0.5 h at rt. To the brown supension was added 3-Bromo-4-fluorobenzonitrile (commercially available from ABCR GmbH, 15.0 g; 75.00 mmol; 1.00 eq.) in anhydrous DMSO (100,0 ml) dropwise at an addition rate which kept the internal temperature under 25 °C with the aid of an ice bath. After complete addition, the reaction was stirred for 3 h at 50 °C. The brown suspension was poured in approx. 2L water and the resulting white precipitate was vacuum-filtered, washed with approx. 2x 300 ml water and dried in vacuum (50 °C, 3 mbar), which gave 3-bromo-4-[(2-methyl-2H-1,2,3-triazol-4-yl)amino]benzonitrile (15.0 g, 45.96 mmol, 85%).

Step 2. To 3-bromo-4-[(2-methyl-2H-1,2,3-triazol-4-yl)amino]benzonitrile (15.0 g; 45.95 mmol; 1.00 eq.) in anhydrous NMP (200,0 ml) was added Tetrabutylammonium acetate (52.3 g; 170.03 mmol; 3.70 eq.). The reaction was purged with argon and Pd(PPh₃)₂Cl₂ (3,3 g; 4,60 mmol; 0,10 eq.) was added. The reaction was stirred for 16 hrs at 120°C. HPLC-MS showed the formation of the required product as maincompound. The brown suspension was vacuum-filtered over celite and the mother liquor was poured in 1,5 L water. The resulting precipitate was vacuum-filtered and the filter cake washed with water. The aqueous filtrate was discarded. The brown solid was suspended in MTBE/MeCN 1:1 v/v (approx. 200 ml) and the unsoluble solid was vacuum filtered, washed with MTBE and dried at 50°C in vacuum to give 2-methyl-2H,4H-[1,2,3]triazolo[4,5-b]indole-7-carbonitrile (4,7 g; 23,83 mmol; 52 %).The organic filtrate was evaporated to dryness and the residue was triturated with MTBE/MeCN 1:1 v/v (approx. 100 mL) and the unsoluble solid was vacuum filtered, washed with MTBE and dried in vacuum at 50 °C for 1 h to give 2-methyl-2H,4H-[1,2,3]triazolo[4,5-b]indole-7-carbonitrile (1,6 g; 7,84 mmol; 17 %)

Step 3. To a brown supension of 2-methyl-2H,4H-[1,2,3]triazolo[4,5-b]indole-7-carbonitrile (4.7 g; 23.83 mmol; 0.75 eq.) in anhydrous 1,4-dioxane (150.0 ml) was added unter argon 4-lodobenzotrifluoride (5,6 ml; 38,01 mmol; 1,20 eq.), N,N'-DIMETHYLETHYLENEDIAMINE, 99% 99% (3.4 ml; 31.7 mmol; 1.0 eq.), Cs2CO3 (20.8 g; 63.34 mmol; 2.00 eq.) and Cul (3.0 g; 15.84 mmol; 0.50 eq.). The reaction was stirred for 16 hrs at 100°C. The reaction was filtered over celite and washed with 200 mL EtOAc. The mother liquor was washed 3x with 150 ml water, dried over Na₂SO₄ and evaporated to dryness. The resulting solid was triturated with EtOAc/MTBE (approx. 150 ml) and the solid was vacuum-filtered, washed with hexanes, MTBE and dried in vacuum to give 2-methyl-4-[4-(trifluoromethyl)phenyl]-2H,4H-[1,2,3]triazolo[4,5-b]indole-7-carbonitrile (9.0 g, 26.13 mmol, 83%).

Step 4. To a supsension of 2-methyl-4-[4-(trifluoromethyl)phenyl]-2H,4H-[1,2,3]triazolo[4,5-b]indole-7-carbonitrile (9.0 g; 26.24 mmol; 1.00 eq.) in Ethanol (140.0 ml) / water (140.0 ml) was added powedered Sodium hydroxide, (10.8 g; 262.39 mmol; 10.00 eq.) and stirred for 16 hrs at 80°C.

Ethanol was distilled off. The residue was acidified with aq. HCl 37%, diluted with 400 ml EtOAc, washed 3x with 150 ml water, dried over Na₂SO₄ and evaporated to dryness. The residue was triturated with MTBE/heptane and the solid was vacuum-filtered, the filter cake washed with heptane and dried in vacuum to provide 2-methyl-4-[4-(trifluoromethyl)phenyl]-2H,4H-[1,2,3]triazolo[4,5-b]indole-7-carboxylic acid **TEAD ligand 2** (8.6 g, 23.73 mmol, 90%). LC-MS (ESI+, Method 1): t_{R} = 1.76 min, m/z 361.0 [M+H]+. ¹H NMR (500 MHz, DMSO) d 12.97 (s, 1H), 8.53 (d, J = 1.7 Hz, 1H), 8.11 - 8.04 (m, 4H), 8.02 (d, J = 8.6 Hz, 2H), 7.93 (d, J = 8.8 Hz, 1H), 4.38 (s, 3H).

### VHL-Lig

### (2S,4R)-1-[(2S)-2-amino-3,3-dimethylbutanoyl]-4-hydroxy-N-{[4-(4-methyl-1,3-thiazol-5-yl)phenyl]methyl}pyrrolidine-2-carboxamide (VHL-Lig)

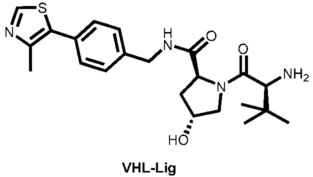

Step 1. To a stirred mixture of (2S,4R)-1-[(2S)-2-{[(tert-butoxy)carbonyl]-amino}-3,3-dimethylbutanoyl]-4-hydroxypyrrolidine-2-carboxylic acid (commercially available from Pharmaron, 5.00 g; 13.79 mmol; 1.00 eq.), 1-[4-(4-methyl-1,3-thiazol-5-yl)phenyl]methanamine hydrochloride (commercially available from Pharmaron, 5.24 g; 20.69 mmol; 1.50 eq.) in DCM (20.00 ml) was addedEDCI (5.57 g; 27.58 mmol; 2.00 eq.), Et3N (6.05 ml; 41.38 mmol; 3.00 eq.) at room temperature. The resulting mixture was stirred for 16 h at 25 degrees C.

The reaction was quenched with water at room temperature. The resulting mixture was extracted with EtOAc (3 x 150 mL). The combined organic layers were washed with brine (1x100 mL), dried over anhydrous Na2SO4. After filtration, the filtrate was concentrated under reduced pressure. The residue was purified by silica gel column chromatography, eluted with PE/EtOAc (1:1) to afford tert-butyl N-[(2S)-1-[(2S,4R)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]carbamate (6.95 g; 11.90 mmol; 86.3 %) as a yellow oil.

Step 2. To a stirred mixture of tert-butyl N-[(2S)-1-[(2S,4R)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]carbamate (5.90 g; 10.10 mmol; 1.00 eq.) in DCM (30.00 ml) was added TFA (10.00 ml)at room temperature. The resulting mixture was stirred for 1 h at room temperature. The reaction was quenched with H₂O at room temperature. The mixture was brought to pH 7 with NaHCO₃ (aq.). The resulting mixture was extracted with DCM (3 x 50 mL). The combined organic layers were washed with brine (1x10 mL), dried over anhydrous Na₂SO₄. After filtration, the filtrate was concentrated under reduced pressure.(2S,4R)-1-[(2S)-2-amino-3,3-dimethylbutanoyl]-4-hydroxy-N-{[4-(4-methyl-1,3-thiazol-5-yl)phenyl]methyl}pyrrolidine-2-carboxamide **(VHL Lig)** (4.16 g; 9.29 mmol; 92.0 %) as a yellow solid. (ESI+, Method 7): tR = 0.96 min, m/z 431.0 [M+H]+.

### Intermediate 1

### N-{2-[2-(2-aminoethoxy)ethoxy]ethyl}-2-methyl-8-[4-(trifluoromethyl)phenyl]-2H,8H-pyrazolo[3,4-b]indole-5-carboxamide TFA salt (Intermediate 1)

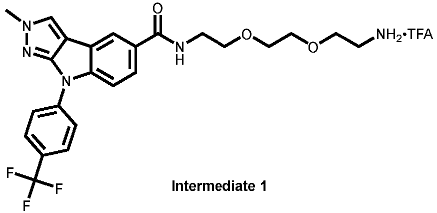

An 8 mL vial was charged with **TEAD ligand 1** (50 mg; 0.14 mmol) which was dissolved in dry N,N-dimethylformamide (3 mL) under Ar. This was followed by addition of 1-(t-Butyloxycarbonyl-amino)-3,6-dioxa-8-octaneamine (0.07 ml; 0.28 mmol ), 4-methylmorpholine (61 µl; 0.56 mmol) and finally HATU (63 mg; 0.17 mmol). The vial was capped and stirred under Ar for 18 h at RT. The reaction mixture was partitioned between MTBE and Water, the phases were separated and the aq. phase was extracted with further MTBE (2 x 3 mL). The organic phase was dried over Na₂SO₄, filtered, and concentrated by rotary evaporation. The resultant residue was purified by flash chromatography (4 g SiO₂, 0 - 10% MeOH in DCM) to give the product as a colourless oil (79 mg, 0.13 mmol, 96 %). 59 mg of this produce were dissolved in DCM (1 mL), followed by addition of TFA (1 mL) under stirring. After 30 min the solvent was evaporated to deliver **Intermediate 1** (49 mg, 0.08 mmol, 81 %) which slowly solidified at RT. LC-MS (ESI+, Method Sunfire): *t*_{R} = 1,75 min, m/z 489.9 [M+H]⁺.

### Intermediate 2

### 5-{[2-(2,6-dioxopiperidin-3-yl)-1-oxo-2,3-dihydro-1H-isoindol-4-yl]amino}pentanoic acid (Intermediate 2)

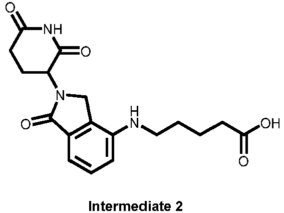

Lenalidomide (commercially available) (300 mg; 1.10 mmol) was dissolved in 1-Methyl-2-pyrrolidone (NMP) (2 mL) in a 5 mL microwave vial, followed by addition of 5-Bromo-pentanoic acid tert-butyl ester (0.26 ml; 1.32 mmol) and finally DIPEA (0.59 ml; 3.30 mmol). The vial was capped and the reaction mixture stirred at 170 °C in a microwave reactor for 1 h and 15 min. The reaction mixture was diluted with water (2 mL). The crude mixture was injected into an MS-coupled prep. RP-HPLC system for purification. The target fractions were lyophilized to give tert-butyl 5-{[2-(2,6-dioxopiperidin-3-yl)-1-oxo-2,3-dihydro-1H-isoindol-4-yl]amino}pentanoate (167 mg; 0.40 mmol) as a white powder, which was deprotected by dissolution in DCM (3 mL), followed by addition of TFA (3 mL). The reaction mixture was stirred for 3 h at RT, the volatiles were evaporated which furnished **Intermediate 2** as a colorless oil (50 mg, 0.14 mmol) which was directly used in the next step. LC-MS (ESI+, Method Sunfire): *t*_{R} = 1,87 min, m/z 359,9 [M+H]⁺.

### Intermediate 3

### 4-{[2-(2,6-dioxopiperidin-3-yl)-1-oxo-2,3-dihydro-1H-isoindol-4-yl]amino}butanoic acid (intermediate 3)

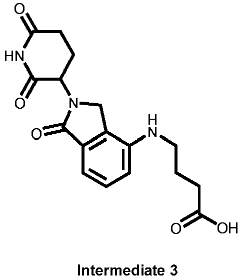

Lenalidomide (300 mg; 1.10 mmol) was dissolved in 1-Methyl-2-pyrrolidon (NMP) (5 mL), followed by addition of tert-Butyl 4-bromobutanoate (0.25 ml; 1.32 mmol) and N-Ethyldiisopropylamin (*i*Pr₂NEt) (0.56 ml; 3.30 mmol). The vial was capped and heated for 12 h in a microwave reactor at 110 °C. The reaction mixture was purified by prep HPLC, and the target fractions were lyophilised to give tert-butyl 4-{[2-(2,6-dioxopiperidin-3-yl)-1-oxo-2,3-dihydro-1H-isoindol-4-yl]amino}butanoate (88 mg; 0.22 mmol, 20 %) as a white lyophilisate. This intermediate was deprotected in 33% TFA in DCM (6 mL) for 1 h at RT to provide **intermediate 3** (quant.) after triple rotary evaporation from DCM solutions. LC-MS (ESI+, Method Sunfire): *t*_{R} = 1,81 min, m/z 345,9 [M+H]⁺.

### Intermediate 4

### 7-{[2-(2,6-dioxopiperidin-3-yl)-1-oxo-2,3-dihydro-1H-isoindol-4-yl]amino}heptanoic acid (intermediate 4)

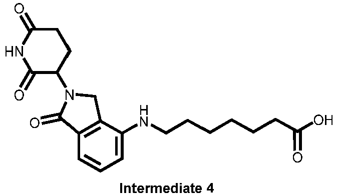

A 5 mL microwave reaction vessel was charged with Lenalidomide (300 mg; 1.10 mmol), dissolved in 1-Methyl-2-pyrrolidone (NMP) (5.00 ml) followed by addition of N-Ethyldiisopropylamin (*i*Pr₂NEt) (0.56 ml; 3.30 mmol) and tert-Butyl 7-bromoheptanoate (368 mg; 1.32 mmol). The vial was capped and heated at 110°C for 12 h in a microwave reactor. The cooled reaction mixture was used for purification by prep RP-HPLC-MS in two sequential runs (2.5 mL injection) to give tert-butyl 7-{[2-(2,6-dioxopiperidin-3-yl)-1-oxo-2,3-dihydro-1H-isoindol-4-yl]amino}heptanoate (167 mg, 0.38 mmol, 34 %). This was deprotected by treatment with TFA (10 mL) in DCM (15 mL) for 18 h at RT. The volatiles were removed by rotary evaporation to provide **intermediate 4** (146 mg, 0.38 mmol, quant.). LC-MS (ESI+, Method Sunfire): *t*_{R} = 2.04 min, m/z 387.9 [M+H]⁺.

### Intermediate 5

### 6-{[(2S)-1-[(2S,4S)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]carbamoyl}hexanoic acid (Intermediate 5)

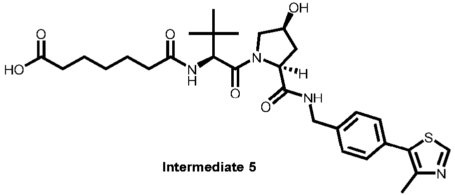

In a 50 mL RBF with stir bar was placed pimelic acid (186 mg; 1.16 mmol) and (2S,4S)-1-[(2S)-2-amino-3,3-dimethylbutanoyl]-4-hydroxy-N-{[4-(4-methyl-1,3-thiazol-5-yl)phenyl]methyl}pyrrolidine-2-carboxamide **(MeVHL-Lig,** commercially available from Sigma-Aldrich or Ambeed) (100 mg; 0.23 mmol). These were suspended in Dichloromethane (DCM) (5 ml) and Tetrahydrofuran (THF) (5 ml) followed by addition of triethylamine (0.11 ml; 0.82 mmol). The resulting solution was cooled to 0°C, and HATU (106 mg; 0.28 mmol) was added. The reaction mixture was stirred for 2 h at 0 °C, and then stirred at RT overnight. The reaction was quenched with water and the mixture was concentrated by rotary evaporation. The remaining residue was dissolved in DMSO and purified by prep RP-HPLC-MS to give **intermediate 5** (70 mg; 0.12 mmol; 53 %) after lyophilization. LC-MS (ESI+, Method Sunfire): *t*_{R} = 2,08 min, m/z 572,8 [M+H]⁺.

### Intermediate 6

### 6-{[(2S)-1-[(2S,4R)-4-hydroxy-2-{[(1S)-1-[4-(4-methyl-1,3-thiazol-5-yl)phenyl]ethyl]carbamoyl}pyrrolidin-1-yl]-3,3-dimethyl-1-oxobutan-2-yl]carbamoyl}hexanoic acid (Intermediate 6)

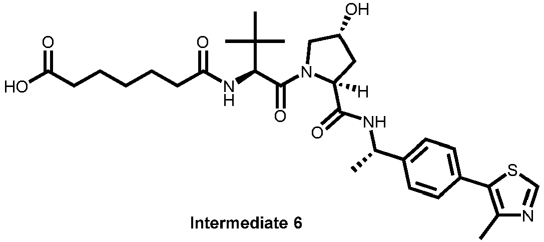

Pimelic acid (166 mg; 1.04 mmol) and (2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxamide hydrochloride (commercially available from Sigma-Aldrich or Ambeed) (100 mg; 0.21 mmol) were combined in a 50 mL RBF with stir bar and dissolved in Dichlormethane (DCM) (5 ml) and Tetrahydrofuran (THF) (5 ml), followed by addition of triethylamine (0.10 ml; 0.74 mmol). The mixture was cooled to 0°C with an ice bath and HATU (95 mg; 0.25 mmol) was added under stirring. The reaction mixture was warmed to RT overnight. The volatiles were removed by rotary evaporation and the remaining residue partially re-dissolved in DMSO (2 mL). The cloudy solution was filtered through a 2 µm filter and the filtrate used directly for injection into a prep RP-HPLC-MS system. The target fractions were lyophilized to give **intermediate 6** (77 mg; 0.13 mmol, 63 %) as a white lyophilisate. LC-MS (ESI+, Method Sunfire): *t*_{R} = 2,10 min, m/z 586,6 [M+H]⁺.

### Intermediate 7

### tert-butyl 3-(4-hydroxy-1,3-dioxo-2,3-dihydro-1H-isoindol-2-yl)-2,6-dioxo-piperidine-1-carboxylate (Intermediate 7)

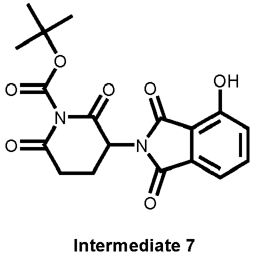

4-Hydroxythalidomide (1.2 g; 4.38 mmol) was combined with DMAP (535 mg; 4.38 mmol) in 1,4-Dioxane (40 ml), followed by additon of Boc anhydride (1.9 g; 8.75 mmol). The reacation mixture was stirred for 3 h at RT. Piperidine (0.43 ml; 4.38 mmol) was added and the reaction mixture stirred for 1 h. The reaction mixture was partitioned between water and EtOAc, the organic phase was filterd over Na₂SO₄, followed by purification by flash chromatography to give **intermediate 7** (1.22 g; 3.26 mmol) as an orange solid. LC-MS (ESI+, Method Sunfire): *t*_{R} = 2,28 min, m/z 274,9 [M-Boc+H]⁺.

### Intermediate 8

### N-(14-hydroxy-3,6,9,12-tetraoxatetradecan-1-yl)-2-methyl-8-[4-(trifluoromethyl)phenyl]-2H,8H-pyrazolo[3,4-b]indole-5-carboxamide

### (Intermediate 8)

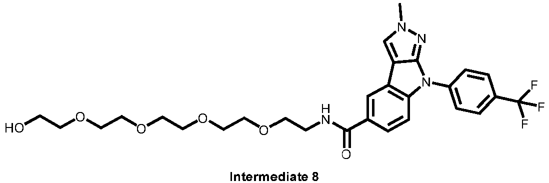

**TEAD ligand 1** (112 mg; 0.31 mmol) and 14-amino-3,6,9,12-tetraoxatetradecan-1-ol (148 mg; 0.63 mmol) were dissolved in N,N-Dimethylformamid (1 ml) in a 8 mL vial with stir bar, followed by 4-Methylmorpholine (138 µl; 1.25 mmol) and finally HATU (143 mg; 0.38 mmol). The reaction mixture was stirred for 18 h at RT, and then directly purified by prep RP-HPLC-MS to give intermediate 8 (235 mg; 0.39 mmol quant.) after lyophilization. LC-MS (ESI+, Method Sunfire): *t*_{R} = 2,31 min, m/z 578,8 [M+H]⁺.

### Intermediate 9

### N-(2-aminoethyl)-2-methyl-8-[4-(trifluoromethyl)phenyl]-2H,8H-pyrazolo[3,4-b]indole-5-carboxamide (Intermediate 9)

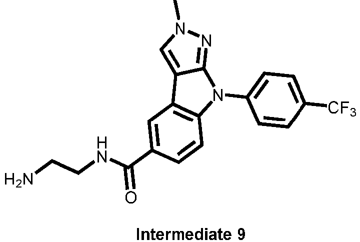

**TEAD ligand 1** (500 mg; 1.39 mmol), N-Boc-ethylenediamine (446 mg; 2.78 mmol), HATU (635 mg; 1.67 mmol) and 4-Methylmorpholine (612 µL; 5.57 mmol) were dissolved in N,N-Dimethylformamid (25 ml) and stirred overnight at room temperature. Water was added and the resulting precipitate was vacuum filtered. The filter cake was dried under vacuum to provide **Boc-intermediate 9** (571, mg; 1.14 mmol, 82 %). LC-MS (ESI+, Method Chromolith): *t*_{R} = 1,81 min, m/z 502,00 [M+H]⁺. This material (571 mg; 1.14 mmol) was deprotected in DCM (20 mL) using 4 M HCl (7.12 mL of a solution in 1,4-dioxane) for 2 d at RT. The resulting precipitate was filtered under vacuum, the filter cake was washed with MTBE and then dried under vacuum to provide **intermediate 9** (429 mg; 1.07 mmol, 94 %). LC-MS (ESI+, Method Chromolith): *t*_{R} = 1,32 min, m/z 402 [M+H]⁺.

### Intermediate 10

### N-[(2R)-1-aminopropan-2-yl]-8-(4-fluorophenyl)-2-methyl-2H,8H-pyrazolo[3,4-b]indole-5-carboxamide (Intermediate 10)

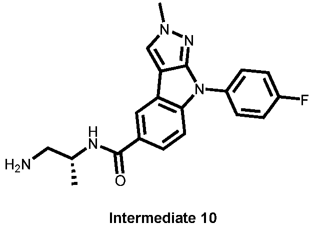

To a solution of **building block 5** (50 mg; 0.16 mmol) in DMF (3 ml) was added tert-butyl N-[(2R)-2-aminopropyl]carbamate (commercially available from Enamine Ltd., 59 mg; 0.32 mmol), EDC hydrochloride (62 mg; 0.32 mmol), HOBt hydrate (25 mg; 0.16 mmol) and 4-Methylmorpholine (0.1 mL). The reaction was stirred overnight at RT and then evaporated to dryness to give crude **Boc-intermediate 10** (185 mg; 0.18 mmol, quant.). All of this material was deprotected in DCM (3 mL) using 4 M HCl (1.58 mL of a solution in 1,4-dioxane) at RT. The resultant precipitate was filtered under vacuum, the filter cake washed with MTBE and then dried under vacuum. The crude residue was purified by flash chromatography to provide **intermediate 10** (78 mg; 0.21 mmol, 85 %). LC-MS (ESI+, Method Chromolith): *t*_{R} = 1,20 min, m/z 366,0 [M+H]⁺.

### Intermediate 11

### N-[(2R)-1-aminopropan-2-yl]-2-methyl-8-(4-methylphenyl)-2H,8H-pyrazolo[3,4-b]indole-5-carboxamide (Intermediate 11)

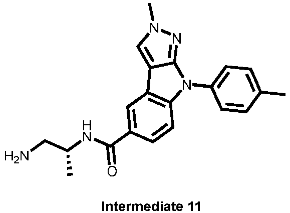

To a solution of **building block 6** (50 mg; 0.16 mmol) in DMF (3 ml) was added tert-butyl N-[(2R)-2-aminopropyl]carbamate (commercially available from Enamine Ltd., 60 mg; 0.33 mmol), N-(3-Dimethylaminopropyl)-N'-ethylcarbodiimide hydro chloride (63 mg; 0.33 mmol), 1-Hydroxybenzotriazole hydrate (25 mg; 0.16 mmol) and 4-Methylmorpholine (0.1 mL). The reaction was stirred overnight at RT and then evaporated to dryness. All of this material was dissolved in Dichloromethane (3 ml) and added to HCl (4.0 M in dioxane ;1.80 mL) overnight at RT. The resulting precipitate was filtered off, the filter cake was washed with MTBE and dried in vacuum. The crude residue was purified by flash column chromatography to give **intermediate 11** (65 mg; 0.18 mmol, 62 %).

### Intermediate 12

### 1-{[2-(2,6-dioxopiperidin-3-yl)-1 ,3-dioxo-2,3-dihydro-1H-isoindol-4-yl]carbamoyl}-2,5,8,11,14-pentaoxahexadecan-16-oic acid (intermediate 12)

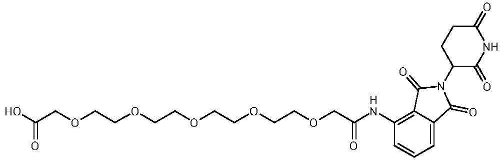

To a solution of NaH (9.06 g, 226.0 mmol, 60.0% purity, 2.20 eq) in DMF (200 mL) was added tetraethylene glycol (20.0 g, 103.0 mmol, 17.7 mL, 1.00 eq) stirred at 15 °C for 2 hrs, and then the mixture was added drop wise to a solution of tert-butyl 2-bromoacetate (80.3 g, 411.0 mmol, 60.9 mL, 4.00 eq) in DMF (80 mL) at 0°C and stirred for 30 min, then warmed to 15 °C for 2 hrs. The reaction was added sat. NH₄Cl (500 mL) and extracted with ethyl acetate (100 mL × 2), and the organic layer was washed with sat. NaCl (50 mL × 3), and then the organic layer dried over with Na₂SO₄ and concentrated to give a residue. The residue was purified by column chromatography (SiO₂, petroleum ether: ethyl acetate = 1: 0 to 3: 1) to give 3,6,9,12,15-pentaoxaheptadecanedioic acid di-tert-butyl ester (20.0 g, 47.3 mmol, 46.0% yield) as a yellow oil.

A solution of said ester (20.0 g, 47.3 mmol, 1.00 eq) in HCl/dioxane (4 M, 50 mL, 4.23 eq) was stirred at 15 °C for 10 hrs. The reaction mixture was concentrated to give 3,6,9,12,15-pentaoxaheptadecanedioic acid (12.0 g, 38.7 mmol, 81.7% yield) as a black brown oil. To a solution of 3,6,9,12,15-pentaoxaheptadecanedioic acid (6.00 g, 19.3 mmol, 1.00 eq) in DCM (30 mL) was added (COCl)₂ (9.82 g, 77.4 mmol, 6.77 mL, 4.00 eq) and stirred at 40°C for 1 hr, then concentrated and dissolved in THF (30 mL), then 4-aminothalidomide (3.70 g, 13.5 mmol, 0.700 eq) in THF (30 mL) was added to the reaction and stirred at 70°C for 3 hrs. The reaction mixture was filtered and the cake was washed with MeOH (5 mL × 2), then the filtrate was concentrated to give a residue. The residue was purified by prep-HPLC to give **intermediate 12** (2.05 g, 3.44 mmol, 17.8% yield, 95.3% purity) as a yellow solid. **LCMS:** m/z (M+H⁺) = 566.4

### Intermediate 13

### 2-{2-[2-({[2-(2,6-dioxopiperidin-3-yl)-1,3-dioxo-2,3-dihydro-1H-isoindol-4-yl]carbamoyl}methoxy)ethoxy]ethoxy}acetic acid (Intermediate 13)

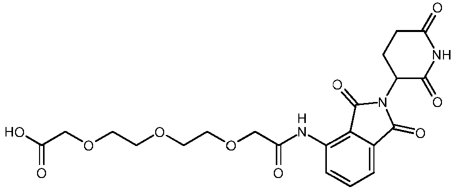

To a solution of NaH (16.6 g, 414.0 mmol, 60.0% purity, 2.20 eq) in DMF (200 mL) was added diethylene glycol (20.0 g, 188.0 mmol, 17.9 mL, 1.00 eq) stirred at 15°C for 2 hrs, and then the mixture was added drop wise to a solution of tert-butyl 2-bromoacetate (147.0 g, 753.0 mmol, 111.0 mL, 4.00 eq) in DMF (80 mL) at 0°C and stirred for 30 min, then warmed to 15°C for 2 hrs. The reaction was added sat.NH₄Cl (500 mL) and extracted with ethyl acetate (100 mL×2), and the organic layer was washed with sat. NaCl (50 mL × 3), and then the organic layer dried over with Na₂SO₄ and concentrated to give a residue. The residue was purified by column chromatography (SiO₂, petroleum ether: ethyl acetate = 1: 0 to 3: 1) to give tert-butyl 2-(2-{2-[2-(tert-butoxy)-2-oxoethoxy]ethoxy}ethoxy)acetate (25.0 g, 74.8 mmol, 39.7% yield) as yellow oil. A solution of tert-butyl 2-(2-{2-[2-(tert-butoxy)-2-oxoethoxy]ethoxy}ethoxy)acetate (25.0 g, 74.8 mmol, 1.00 eq) in HCl (6 M, 50 mL, 4.01 eq) was stirred at 15 °C for 3 hrs. The reaction mixture was concentrated to give 2-{2-[2-(carboxymethoxy)ethoxy]ethoxy}acetic acid (13.0 g, 58.5 mmol, 78.3% yield) as a brown oil. To a solution of 2-{2-[2-(carboxy-methoxy)ethoxy]ethoxy}acetic acid (6.00 g, 27.0 mmol, 1.00 eq) in DCM (20 mL) was added (COCl)₂ (13.7 g, 108.0 mmol, 9.46 mL, 4.00 eq) and stirred at 40°C for 1 hr, then concentrated and dissolved in THF (20 mL), then 4-aminothalidomide (5.17 g, 18.9 mmol, 0.700 eq) in THF (20 mL) was added to the reaction and stirred at 70°C for 3 hrs. LCMS showed the desired MS was detected. The reaction mixture was concentrated to give a residue. The reaction mixture was purified by prep-HPLC to give intermediate 13 (1.94 g, 3.97 mmol, 14.7% yield, 97.6% purity) as a off-white solid. LC-MS: m/z (M+H⁺) = 478.1

### Intermediate 14

### 1-{[(2S)-1-[(2S,4R)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]carbamoyl}-2,5,8,11-tetraoxatridecan-13-oic acid (Intermediate 14)

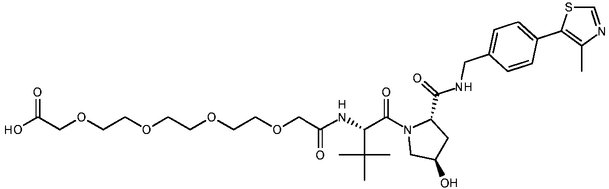

To a solution of NaH (11.7 g, 293.0 mmol, 60.0% purity, 2.20 eq) in DMF (200 mL) was added triethylene glycol (20.0 g, 133.0 mmol, 17.9 mL, 1.00 eq) stirred at 15 °C for 2 hrs, and then the mixture was added drop wise to a solution of tert-butyl 2-bromoacetate (103.0 g, 532.0 mmol, 78.7 mL, 4.00 eq) in DMF (80 mL) at 0°C and stirred for 30 min, then warmed to 15 °C for 2 hrs. To the reaction was added sat.NH₄Cl (500 mL) and extracted with ethyl acetate (100 mL × 2), and the organic layer was washed with sat.NaCl (50 mL × 3), and then the organic layer dried over with Na₂SO₄ and concentrated to give a residue. The residue was purified by column chromatography (SiO₂, pertroleum ether: ethyl acetate = 1: 0 to 3: 1) to give tert-butyl 2-(2-{2-[2-(tert-butoxy)-2-oxoethoxy]ethoxy}ethoxy)acetate (25.0 g, 66.1 mmol, 49.6% yield) as yellow oil. A solution of tert-butyl 2-(2-{2-[2-(tert-butoxy)-2-oxoethoxy]ethoxy}ethoxy)acetate (25.0 g, 66.1 mmol, *1.00* eq) in HCl (6 M, 50 mL, 4.54 eq) was stirred at 15°C for 3 hrs. The reaction mixture was concentrated to give 2-{2-[2-(carboxymethoxy)ethoxy]ethoxy}acetic acid (15.0 g, 56.3 mmol, 85.3% yield) as a brown oil.

To a mixture of 2-{2-[2-(carboxymethoxy)ethoxy]ethoxy}acetic acid (5.70 g, 21.4 mmol, 2.50 eq) in DMF (10 mL) was added HATU (4.88 g, 12.9 mmol, 1.50 eq) and DIEA (3.32 g, 25.7 mmol, 4.48 mL, 3.00 eq) and stirred at 15°C for 0.5 hr, then (2S,4R)-1-[(S)-2-Amino-3,3-dimethylbutanoyl]-4-hydroxy-N-[4-(4-methylthiazol-5-yl)benzyl]pyrrolidine-2-carboxamide Hydrochloride (4.00 g, 8.56 mmol, 1.00 eq, HCl) in DMF (10 mL) was added and stirred for 0.5 hr at 15 °C. The reaction mixture was filtered, and the filtrate was collected to give a residue. The residue was purified by prep-HPLC to give intermediate 14 (2.31 g, 3.21 mmol, 37.5% yield, 94.3% purity) as a yellow gum.

### Intermediate 15

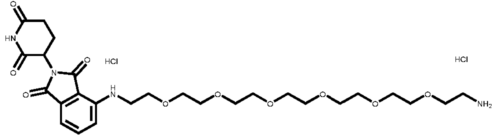

Step 1. In a reaction vial with stir bar 2-(2,6-Dioxo-piperidin-3-yl)-4-fluoro-isoindoline-1,3-dione (45.0 mg; 0.163 mmol; 1.00 eq.), tert-butyl 20-amino-3,6,9,12,15,18-hexaoxaicos-1-ylcarbamate (100.0 mg; 0.236 mmol; 1.45 eq.) and N-Ethyldiisopropylamine (73.0 µl; 0,410 mmol; 2.52 eq.) were dissolved in 1-Methyl-2-pyrrolidone (1.00 ml; 10.390 mmol; 63.78 eq.) and stirred for 1 h at 160°C in a Biotage^{®} microwave reactor. The reaction mixture was concentrated under reduced pressure, dissolved in DMSO and purified by prep. HPLC-MS and the product fractions were unified and concentrated under reduced pressure to provide tert-butyl N-(20-{[2-(2,6-dioxopiperidin-3-yl)-1,3-dioxo-2,3-dihydro-1H-isoindol-4-yl]amino}-3,6,9,12,15,18-hexaoxaicosan-1-yl)carbamate (83.7 mg; 0.109 mmol) as an amorphous residue. LC-MS (ESI+, Method 8): t_{R} = 0.49 min, m/z 681.2 [M+H]+.

Step 2. In a 8 mL reaction vial with stir bar tert-butyl N-(20-{[2-(2,6-dioxopiperidin-3-yl)-1,3-dioxo-2,3-dihydro-1H-isoindol-4-yl]amino}-3,6,9,12,15,18-hexaoxaicosan-1-yl)carbamate (83.70 mg; 0.116 mmol; 1.00 eq.) was suspended in 4 M solution of hydrogen chloride in dioxane (500.00 µl; 2.000 mmol; 17.19 eq.) and stirred at room temperature for 18 h. The mixture was absorbed on Isolute sorbent, purified by flash chromatography and the product fractions were concentrated under reduced pressure to provide 4-(21-amino-4,7,10,13,16,19-hexaoxa-1-azahenicosan-1-yl)-2-(2,6-dioxopiperidin-3-yl)-2,3-dihydro-1H-isoindole-1,3-dione dihydrochloride (122.00 mg; 0.186 mmol) as a clear oily residue. LC-MS (ESI+, Method 8): tR = 0.34 min, m/z 581.4 [M+H]+.

### Intermediate 16

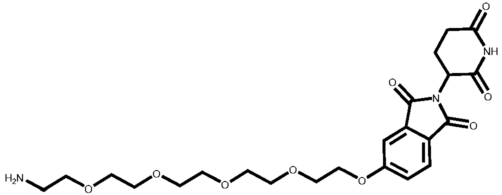

In a microwave reaction vial tert-butyl 3-(5-hydroxy-1,3-dioxo-2,3-dihydro-1H-isoindol-2-yl)-2,6-dioxopiperidine-1-carboxylate **(Intermediate 17)** (100.00 mg; 0.251 mmol; 1.00 eq.), tert-butyl-N-(14-hydroxy-3,6,9,12-Tetraoxatetradecan-1-yl)carbamate (commercially available from Activate Scientific GmbH, 110.00 mg; 0.310 mmol; 1.23 eq.) and triphenylphosphine, polymer-bound (392.00 mg; 0.627 mmol; 2.50 eq.) were dissolved in tetrahydrofuran (4.00 ml) at 0°C, then diisopropyl azodicarboxylate (123.00 µl; 0.627 mmol; 2.50 eq.) was added. It was stirred at room temperature for 3 days. The reaction mixture was filtered off and absorbed on Isolute sorbent, purified by flash chromatography and the product fractions were unified and concentrated under reduced pressure to provide tert-butyl 3-{5-[(14-{[(tert-butoxy)carbonyl]amino}-3,6,9,12-tetraoxatetradecan-1-yl)oxy]-1,3-dioxo-2,3-dihydro-1H-isoindol-2-yl}-2,6-dioxopiperidine-1-carboxylate (42.0 mg; 0.0076 mmol) as a yellow residue. LC-MS (ESI+, Method 8): t_{R} = 0.31 min, m/z 494.3 [M+H]+.

### Intermediate 17

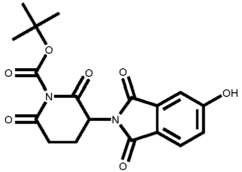

In a 250 mL round bottom flask with stir bar and drying tube 2-(2,6-dioxopiperidin-3-yl)-5-hydroxyisoindoline-1,3-dione (3.00 g; 10.94 mmol; 1.00 eq.) and 4-(dimethylamino)pyridine (1.34 g; 10.94 mmol; 1.00 eq.) were dissolved in DMF (97.50 ml) followed by additon of di-tert-butyl dicarbonate (4.68 ml; 21.880 mmol; 2.00 eq.). The reaction mixture was stirred for 1 hour at room temperature. Piperidine (1.08 ml; 10.940 mmol; 1.00 eq.) was added and the reaction mixture stirred for 1,5 hours at room temperature. The reaction mixture was extracted with deionizied water and ethyl acetate. The organic phase was filtered over Na₂SO₄, absorbed on Isolute sorbent, purified by flash chromatography and the product fractions were unified and concentrated under reduced pressure to provide tert-butyl 3-(5-hydroxy-1,3-dioxo-2,3-dihydro-1H-isoindol-2-yl)-2,6-dioxo-piperidine-1-carboxylate (2.77 g; 6.948 mmol) as a yellow solid. LC-MS (ESI+, Method 8): t_{R} = 0.47 min, m/z 375.2 [M+H]+.

### Intermediate 20

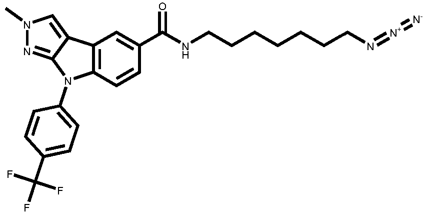

In a 8 mL vial with stir bar was placed **TEAD ligand 1 free acid** (106.70 mg; 0.30 mmol; 1.00 eq.), dissolved in DMF (1.00 ml), to which 7-azido-1-heptanamine (46.39 mg; 0.30 mmol; 1.00 eq.), 4-methylmorpholine (0.13 ml; 1.19 mmol; .,00 eq.) and HATU (135.50 mg; 0.36 mmol; 1.20 eq.) was added. The reaction mixture was stirred at RT. The crude mixture was purified by prep. RP-HPLC-MS. The target fractions lyophilized to provide N-(7-azidoheptyl)-2-methyl-8-[4-(trifluoromethyl)phenyl]-2H,8H-pyrazolo[3,4-b]indole-5-carboxamide (69.00 mg; 0.14 mmol) as a white solid. LC-MS (ESI+, Method 3): t_{R} = 2.81 min, m/z 497.7 [M+H]+.

### Intermediate 21

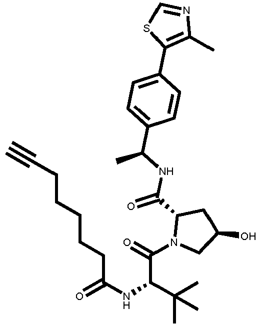

In a reaction vial with stir bar oct-7-ynoic acid (15.77 mg; 0.11 mmol; 1.00 eq.), (2S,4R)-1-[(2S)-2-amino-3,3-dimethylbutanoyl]-4-hydroxy-N-[(1S)-1-[4-(4-methyl-1,3-thiazol-5-yl)phenyl]ethyl]pyrrolidine-2-carboxamide hydrochloride (**MeVHL-Lig**, commercially available from Ambeed (50.00 mg; 0.11 mmol; 1.00 eq.) and HATU (51.31 mg; 0.13 mmol; 1.20 eq.) were dissolved in DMF (1.10 ml) at room temperature. 4-Methylmorpholine (50.00 µl; 0.45 mmol; 4.00 eq.) was added and the reaction mixture was stirred for 16 h at room temperature. The reaction mixture was purified by prep. HPLC to provide (2S,4R)-1-[(2S)-3,3-dimethyl-2-(oct-7-ynamido)-butanoyl]-4-hydroxy-N-[(1S)-1-[4-(4-methyl-1,3-thiazol-5-yl)phenyl]ethyl]-pyrrolidine-2-carboxamide (53.1 mg; 0.094 mmol) as a colorless solid. LC-MS (ESI+, Method 3): t_{R} = 2.33 min, m/z 567.8 [M+H]+.

### Intermediate 22

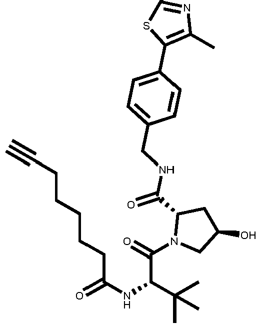

Prepared and purified as described for Intermediate 21 but using intermediate **VHL-Lig** instead of intermediate **MeVHL-Lig** which gave (2S,4R)-1-[(2S)-3,3-dimethyl-2-(oct-7-ynamido)butanoyl]-4-hydroxy-N-{[4-(4-methyl-1,3-thiazol-5-yl)phenyl]methyl}pyrrolidine-2-carboxamide (22.1 mg; 0,04 mol) as a colorless solid. LC-MS (ESI+, Method 3): t_{R} = 2.27 min, m/z 552.8 [M+H]+.

### Intermediate 29

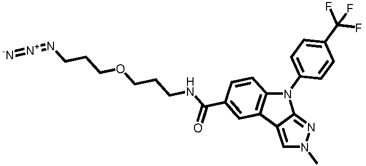

Step 1. To a stirred mixture of 2-methyl-8-[4-(trifluoromethyl)phenyl]-2H,8H-pyrazolo[3,4-b]indole-5-carboxylic acid (360.00 mg; 0.95 mmol; 1.00 eq.),3-aminopropan-1-ol (90.00 mg; 1.14 mmol; 1.20 eq.), Et₃N (0.26 ml; 1.90 mmol; 2.00 eq.) in DCM (5.00 ml) was added HATU (762.00 mg; 1.90 mmol; 2.00 eq.) at room temperature. The resulting mixture was stirred for 16 h at 25 degrees C. The reaction was quenched with water at room temperature. The resulting mixture was extracted with DCM(3 x 150 mL). The combined organic layers were washed with brine (1x100 mL), dried over anhydrous Na2SO4. After filtration, the filtrate was concentrated under reduced pressure. The residue was purified by silica gel column chromatography, eluted with PE/EtOAc (1:1) to afford N-(3-hydroxypropyl)-2-methyl-8-[4-(trifluoromethyl)phenyl]-2H,8H-pyrazolo[3,4-b]indole-5-carboxamide (381.00 mg; 0.90 mmol; 94%) as a yellow oil. LC-MS (ESI+, Method 2): t_{R} = 1.02 min, m/z 417.0 [M+H]+.

Step 2. To a solution of NaH (60.00 mg; 1.50 mmol; 2.12 eq.) in DMF (5.00 ml) was added N-(3-hydroxypropyl)-2-methyl-8-[4-(trifluoromethyl)phenyl]-2H,8H-pyrazolo[3,4-b]indole-5-carboxamide (300.00 mg; 0.71 mmol; 1.00 eq.) at 0 degrees C. The mixture was stirred for 0.5 h. 1-[(3-azidopropoxy)sulfonyl]-4-methylbenzene (228.00 mg; 0.85 mmol; 1.20 eq.) was added and the mixture was allowed to warm to room temperature and stirred overnight. The reaction was quenched with water at room temperature. The resulting mixture was extracted with EtOAc (3 x 150 mL). The combined organic layers were washed with brine (1x100 mL), dried over anhydrous Na₂SO₄. After filtration, the filtrate was concentrated under reduced pressure, to afford N-[3-(3-azidopropoxy)propyl]-2-methyl-8-[4-(trifluoromethyl)phenyl]-2H,8H-pyrazolo[3,4-b]indole-5-carboxamide (220.00 mg; 0.06 mmol; 8.6 %) as a colorless oil. LC-MS (ESI+, Method 2): t_{R} = 1.09 min, m/z 500.0 [M+H]+.

### Intermediate 30

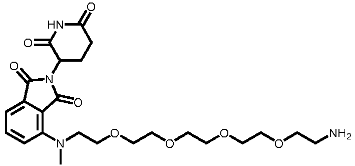

Step 1. In a 5 ml microwave vial, to a solution of tert-butyl N-(14-amino-3,6,9,12-tetraoxatetradecan-1-yl)carbamate (commercially available from Combi-Blocks, 100.00 mg; 0.30 mmol; 1.00 eq.) in dichlormethan (DCM) (2.00 ml; 31.3 mmol; 105.4 eq.) was added triethylamin (82 µl; 0.59 mmol; 2.00 eq.) at RT. Then, *o*-NsCl (65.9 mg; 0,30 mmol; 1,00 eq.) was added and the reaction mixture was stirred for 30 min. After complete conversion of the starting material, the solvent was removed under reduced pressure and the residue was taken up in DMF (2.00 ml; 25.8 mmol; 86.5 eq.). Then, K₂CO₃ (206.2 mg; 1.49 mmol; 5.00 eq.) was added followed by Mel (21 µl; 0.33 mmol; 1.10 eq.). The reaction mixture was heated to 60 °C. After 20 min, Mel (21 µl; 0.33 mmol; 1.10 eq.) is added again and the reaction mixture is stirred for 24 h.The mixture was diluted with water (10 mL) and EtOAc (20 mL) and the layers were separated. The aqueous phase was extracted with EtOAc (3 x 20 ml). The combined organic extracts were washed with brine (30 ml) and dried with Na₂SO₄. The solvent was removed under reduced pressure and the crude product was purified by HPLC (see attached file).

Step 2. In a 2 ml microwave vial, tert-butyl N-[14-(N-methyl-2-nitrobenzenesulfonamido)-3,6,9,12-tetraoxatetradecan-1-yl]carbamate (step 1) (62.7 mg; 0.12 mmol; 1.00 eq.) was dissolved in anhydrous MeCN (1.0 ml) under an argon atmosphere. Cesium carbonate (209.8 mg; 0.64 mmol; 5.50 eq.) and PhSH (16 µl; 0.15 mmol; 1.30 eq.) were added successively and the reaction mixture was heated to 50 °C for 16 h The reaction mixture was diluted with water (15 ml) and EtOAc (20 ml). The layers were seperated and the aqueous phase was extracted with EtOAc (2x 20 ml). The combined organic extracts were dried with Na₂SO₄ and the solvent was removed under reduced pressure yielding the crude product. The crude product was immediatly used in the next step without further purification.

Step 3. In a 2 ml microwave vial, 2-(2,6-dioxopiperidin-3-yl)-4-fluoroisoindoline-1,3-dione (commercially available from Ambeed Inc., 32.3 mg; 0.12 mmol; 1.00 eq.) and tert-butyl N-(5,8,11,14-tetraoxa-2-azahexadecan-16-yl)carbamate (step 2) (41.0 mg; 0.12 mmol; 1.00 eq.) were dissolved in anhydrous NMP (1.2 ml). DIPEA (41 µl; 0.23 mmol; 2.00 eq.) was added and the reaction mixture was heated in a microwave reactor at 130 °C for 1 h. The crude product (as a solution in NMP) was purified by HPLC.

Step 4. In a 2 ml microwave vial, tert-butyl N-{2-[2-(2,6-dioxopiperidin-3-yl)-1,3-dioxo-2,3-dihydro-1H-isoindol-4-yl]-5,8,11,14-tetraoxa-2-azahexadecan-16-yl}carbamate (step 3) (18,7 mg; 0,03 mmol; 1,00 eq.) was dissolved in DCM (1,0 ml) and TFA (0,5 ml) was added at RT. After 30 min the volatiles were removed under reduced pressure. The crude product was immediately used in the next reaction without further purification.

### Intermediate 33

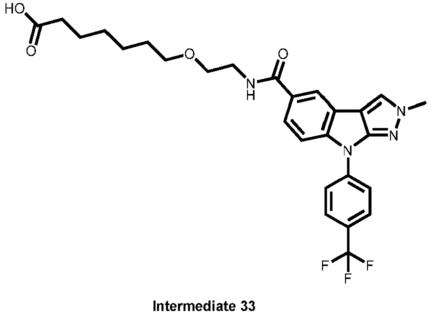

Step 1. To a solution of NaH (0.36 g; 9.00 mmol; 1.53 eq.) in DMF (10.00 ml) was added tert-butyl N-(2-hydroxyethyl)carbamate (1.00 g; 5.89 mmol; 1.00 eq.) at 0 degrees C. The mixture was stirred for 0.5 h. methyl 7-bromoheptanoate (1.60 g; 6.81 mmol; 1.16 eq.) was added and the mixture was allowed to warm to room temperature and stirred for 2 h. The reaction was quenched with water at room temperature. The resulting mixture was extracted with EtOAc (3 x 150 mL). The combined organic layers were washed with brine (1x100 mL), dried over anhydrous Na2SO4. After filtration, the filtrate was concentrated under reduced pressure to afford crude methyl 7-(2-{[(tert-butoxy)carbonyl]amino}ethoxy)heptanoate (1.62 g; 4.54 mmol; 77.0 %) as a colorless oil.

Step 2. A mixture of methyl 7-(2-{[(tert-butoxy)carbonyl]amino}ethoxy)-heptanoate (1.62 g; 4.54 mmol; 1.00 eq.) and 4 M HCl in 1,4-dioxane (10.00 ml) was stirred for 1 h at room temperature. The reaction was concentrated under reduced pressure, to afford crude methyl 7-(2-aminoethoxy)heptanoate (1.05 g; 4.39 mmol; 96.7 %) as a yellow oil.

Step 3. To a stirred mixture of methyl 7-(2-aminoethoxy)heptanoate (300.00 mg; 1.25 mmol; 1.00 eq.), 2-methyl-8-[4-(trifluoromethyl)phenyl]-2H,8H-pyrazolo[3,4-b]indole-5-carboxylic acid (**TEAD ligand 1 free acid)** (569.00 mg; 1.50 mmol; 1.20 eq.) in DCM (5.00 ml)was added Et₃N (0.55 ml; 3.76 mmol; 3.00 eq.) HATU (1004.00 mg; 2.51 mmol; 2.00 eq.)at room temperature. The resulting mixture was stirred for 16 h at room temperature. The reaction was quenched with water at room temperature. The resulting mixture was extracted with DCM (3 x 150 mL). The combined organic layers were washed with brine (1x100 mL), dried over anhydrous Na2SO4. After filtration, the filtrate was concentrated under reduced pressure. The residue was purified by silica gel column chromatography, eluted with PE/EtOAc (1:2) to afford methyl 7-[2-({2-methyl-8-[4-(trifluoromethyl)phenyl]-2H,8H-pyrazolo[3,4-b]indol-5-yl}formamido)-ethoxy]heptanoate (430.00 mg; 0.68 mmol; 54%)

Step 4. To a stirred mixture of methyl 7-[2-({2-methyl-8-[4-(trifluoromethyl)phenyl]-2H,8H-pyrazolo[3,4-b]indol-5-yl}formamido)-ethoxy]heptanoate (430.00 mg; 0.68 mmol; 1.00 eq.) in MeOH (10.00 ml), H2O (2.00 ml) was added NaOH (143.00 mg; 3.40 mmol; 5.00 eq.) at room temperature. The resulting mixture was stirred for 16 h at 60 °C. The reaction was quenched with H2O at room temperature. The mixture was acidified to pH 2 with HCl (aq.). The resulting mixture was extracted with EtOAc (3 x 50 mL). The combined organic layers were washed with brine (1x10 mL), dried over anhydrous Na₂SO₄. After filtration, the filtrate was concentrated under reduced pressure to give 7-[2-({2-methyl-8-[4-(trifluoromethyl)phenyl]-2H,8H-pyrazolo[3,4-b]indol-5-yl}formamido)-ethoxy]heptanoic acid (315.00 mg; 0.54 mmol; 80%) as a white solid. LC-MS (ESI+, Method 7): tR = 0.93 min, m/z 531.2 [M+H]+.

### Intermediate 34

### 4-{2-[2-({2-methyl-8-[4-(trifluoromethyl)phenyl]-2H,8H-pyrazolo[3,4-b]indol-5-yl}formamido)ethoxy]ethoxy}butanoic acid (intermediate 34)

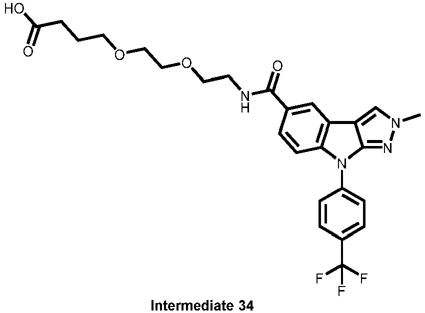

Step 1. To a stirred mixture of tert-butyl 4-[2-(2-aminoethoxy)-ethoxy]butanoate (commercially available from Pharmaron, 200.00 mg; 0.77 mmol; 1.00 eq.) in DCM (10.00 ml) was added 2-methyl-8-[4-(trifluoromethyl)phenyl]-2H,8H-pyrazolo[3,4-b]indole-5-carboxylic acid **TEAD ligand 1 free acid** (349.00 mg; 0.92 mmol; 1.20 eq.), HATU (614.00 mg; 1.53 mmol; 2.00 eq.), Et₃N (0.28 ml; 1.92 mmol; 2.50 eq.) at room temperature. The resulting mixture was stirred for 16 h at room temperature. The reaction was quenched with water at room temperature. The resulting mixture was extracted with DCM (3 x 150 mL). The combined organic layers were washed with brine (1x100 mL), dried over anhydrous Na2SO4. After filtration, the filtrate was concentrated under reduced pressure. The residue was purified by silica gel column chromatography, eluted with PE/EtOAc (1:1) to afford tert-butyl 4-{2-[2-({2-methyl-8-[4-(trifluoromethyl)phenyl]-2H,8H-pyrazolo[3,4-b]indol-5-yl}formamido)-ethoxy]ethoxy}butanoate (341.00 mg; 0.56 mmol; 73%) as a colorless oil.

Step 2. To a stirred mixture of tert-butyl 4-{2-[2-({2-methyl-8-[4-(trifluoromethyl)phenyl]-2H,8H-pyrazolo[3,4-b]indol-5-yl}formamido)ethoxy]-ethoxy}butanoate (300.00 mg; 0.49 mmol; 1.00 eq.) in HCl(gas) in 1,4-dioxane (5.00 ml; 20.00 mmol; 40.57 eq.) at room temperature. The resulting mixture was stirred for 1 h at room temperature. The reaction was quenched with NaHOC3 at room temperature. The resulting mixture was extracted with EtOAc (3 x 150 mL). The combined organic layers were washed with brine (1x100 mL), dried over anhydrous Na2SO4. After filtration, the filtrate was concentrated under reduced pressure. To afford 4-{2-[2-({2-methyl-8-[4-(trifluoromethyl)phenyl]-2H,8H-pyrazolo[3,4-b]-indol-5-yl}formamido)ethoxy]ethoxy}butanoic acid (247.00 mg; 0.44 mmol; 88%) as a white solid in the crude state. LC-MS (ESI+, Method 7): t_{R} = 0.82 min, m/z 533.0 [M+H]+.

### Intermediate 35

### (2S,4R)-1-[(2S)-2-(2-{3-[2-(2-aminoethoxy)ethoxy]propoxy}acetamido)-3,3-dimethylbutanoyl]-4-hydroxy-N-{[4-(4-methyl-1,3-thiazol-5-yl)phenyl]methyl}pyrrolidine-2-carboxamide TFA salt (intermediate 35)

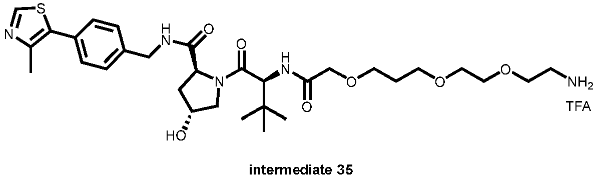

Step 1. To a stirred solution of methyl 2-(3-hydroxypropoxy)acetate (1.00 g; 6.55 mmol; 1.00 eq.) and I₂ (2.56 g; 9.58 mmol; 1.46 eq.) in THF (40.00 ml) were added PPh₃ (2.12 g; 7.68 mmol; 1.17 eq.) and 1H-imidazole (0.56 g; 7.81 mmol; 1.19 eq.) at room temperature. The mixture was stirred for 1.5 h at room temperature under a nitrogen atmosphere. The resulting mixture was extracted with HCl and NaHCO₃ (3x50 mL). The combined organic layers were washed with H₂O (3x50 mL), dried over anhydrous Na₂SO₄. After filtration, the filtrate was concentrated under reduced pressure. The residue was purified by silica gel column chromatography, eluted with EtOAc/Hexanes(1:10) to afford methyl 2-(3-iodopropoxy)-acetate (1.400 g; 5.425 mmol; 83%) as a yellow liquid.

Step 2. To a stirred solution of tert-butyl N-[2-(2-hydroxyethoxy)ethyl]-carbamate (1.000 g; 4.726 mmol; 1.0 eq.) in DMF (30.000 ml) were added NaH (0.560 g; 14.000 mmol; 3.0 eq.) and dropwise methyl 2-(3-iodopropoxy)acetate (1.400 g; 5.425 mmol; 1.1 eq.) at 0 °C. The mixture was stirred for 3 h at RT. The reaction was quenched by the addition of H₂O (50 mL) at room temperature.The resulting mixture was extracted with EtOAc (3x50 mL). After filtration, the filtrate was concentrated under reduced pressure. The crude product was purified by reverse phase flash chromratography to afford 2-{3-[2-(2-{[(tert-butoxy)carbonyl]amino}-ethoxy)ethoxy]propoxy}acetic acid (240.000 mg; 0.747 mmol; 15.8 %)

Step 3. To a stirred solution of 2-{3-[2-(2-{[(tert-butoxy)carbonyl]amino}-ethoxy)ethoxy]propoxy}acetic acid (210.000 mg; 0.653 mmol; 4.0 eq.) and (2S,4R)-1-[(2S)-2-amino-3,3-dimethylbutanoyl]-4-hydroxy-N-{[4-(4-methyl-1,3-thiazol-5-yl)phenyl]methyl}pyrrolidine-2-carboxamide **VHL-Lig** (70.000 mg; 0.163 mmol; 1.0 eq.) in DMF (10.000 ml) was added EDCI (35.000 mg; 0.183 mmol; 1.1 eq.), HOBT(25.000 mg; 0.176 mmol; 1.1 eq.) and DIEA (90.000 µl; 0.491 mmol; 3.0 eq.) at room temperature under nitrogen atmosphere. The resulting mixture was stirred for 18 h at room temperature under nitrogen atmosphere. The resulting mixture was extracted with EA (3x50 mL). The combined organic layers were washed with H2O (3x50 mL), dried over anhydrous Na₂SO₄. After filtration, the filtrate was concentrated under reduced pressure. The residue was purified by silica gel column chromatography, eluted with DCM/ MeOH(9:1) to afford tert-butyl N-(2-{2-[3-({[(2S)-1-[(2S,4R)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]carbamoyl}methoxy)propoxy]ethoxy}ethyl)carbamate (75.000 mg; 0.102 mmol; 63%) as a white solid.

Step 4. A mixture of tert-butyl N-(2-{2-[3-({[(2S)-1-[(2S,4R)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]carbamoyl}methoxy)propoxy]ethoxy}ethyl)-carbamate (75.000 mg; 0.102 mmol; 1.0 eq.) and TFA (1.000 ml) in DCM (5.000 ml) was stirred at room temperature. The resulting mixture was stirred for 4h at room temperature. The resulting mixture was concentrated under reduced pressure. The result was (2S,4R)-1-[(2S)-2-(2-{3-[2-(2-aminoethoxy)ethoxy]propoxy}acetamido)-3,3-dimethylbutanoyl]-4-hydroxy-N-{[4-(4-methyl-1,3-thiazol-5-yl)phenyl]methyl}pyrrolidine-2-carboxamide TFA salt (85.000 mg; 0.095 mmol; 93%) as a yellow crude liquid product. LC-MS (ESI+, Method 3): t_{R} = 0.63 min, m/z 634.3 [M+H]+.

### Intermediate 37

### N-[2-(4-azidobutoxy)ethyl]-2-methyl-8-[4-(trifluoromethyl)phenyl]-2H,8H-pyrazolo[3,4-b]indole-5-carboxamide (intermediate 37)

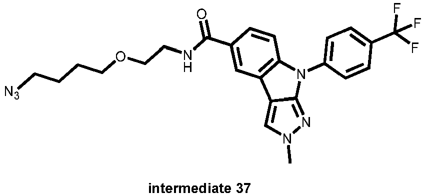

Step 1. To a stirred mixture of 2-methyl-8-[4-(trifluoromethyl)phenyl]-2H,8H-pyrazolo[3,4-b]indole-5-carboxylic acid **TEAD ligand 1 free acid** (250.00 mg; 0.66 mmol; 1.00 eq.), 2-aminoethan-1-ol (51.00 mg; 0.79 mmol; 1.20 eq.), ET3N (0.18 ml; 1.32 mmol; 2.00 eq.) in DCM (3.00 ml) was added HATU (529.00 mg; 1.32 mmol; 2.00 eq.) at room temperature. The resulting mixture was stirred for 16 h at room temperature. The reaction was quenched with water at room temperature. The resulting mixture was extracted with EtOAc (3 x 150 mL). The combined organic layers were washed with brine (1x100 mL), dried over anhydrous Na₂SO₄. After filtration, the filtrate was concentrated under reduced pressure. The residue was purified by silica gel column chromatography, eluted with PE/EtOAc (1:1) to afford N-(2-hydroxyethyl)-2-methyl-8-[4-(trifluoromethyl)phenyl]-2H,8H-pyrazolo[3,4-b]indole-5-carboxamide (252.00 mg; 0.59 mmol; 89%) as a yellow oil.

Step 2. To a solution of NaH (37.00 mg; 0.93 mmol; 1.97 eq.) in DMF (3.00 ml) was added N-(2-hydroxyethyl)-2-methyl-8-[4-(trifluoromethyl)phenyl]-2H,8H-pyrazolo[3,4-b]indole-5-carboxamide (200.00 mg; 0.47 mmol; 1.00 eq.) at 0 °C. The mixture was stirred for 0.5 h. 1-[(4-azidobutoxy)sulfonyl]-4-methylbenzene (160.00 mg; 0.56 mmol; 1.20 eq.) was added and the mixture was allowed to warm to room temperature and stirred for 1 h. The reaction was quenched with water at room temperature. The resulting mixture was extracted with EtOAc (3 x 150 mL). The combined organic layers were washed with brine (1x100 mL), dried over anhydrous Na2SO4. After filtration, the filtrate was concentrated under reduced pressure to afford N-[2-(4-azidobutoxy)ethyl]-2-methyl-8-[4-(trifluoromethyl)phenyl]-2H,8H-pyrazolo[3,4-b]indole-5-carboxamide (231.00 mg; 0.25 mmol; 53 %) as a yellow solid. LC-MS (ESI+, Method 7): t_{R} = 0.61 min, m/z 403.0 [M+H]+.

### Intermediate 38

### 3-{2-[(2-{2-[2-({2-methyl-8-[4-(trifluoromethyl)phenyl]-2H,8H-pyrazolo[3,4-b]indol-5-yl}formam ido)ethoxy]ethoxy}ethyl)carbamoyl]ethoxy}propanoic acid (intermediate 38)

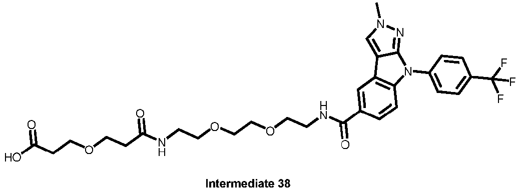

Step 1. A solution of 3-[3-(tert-butoxy)-3-oxopropoxy]propanoic acid (100.00 mg; 0.46 mmol; 1.00 eq.), benzyl N-{2-[2-(2-aminoethoxy)ethoxy]-ethyl}carbamate (129.00 mg; 0.46 mmol; 1.00 eq.) and HATU (183.00 mg; 0.46 mmol; 1.00 eq.) in DIEA (177.50 mg; 1.31 mmol; 2.85 eq.) was stirred for 2 hours at degrees overnight under DCM (8.00 ml) atmosphere. The crude product was purified by Prep-HPLC to afford tert-butyl 3-[2-({2-[2-(2-{[(benzyloxy)carbonyl]amino}ethoxy)ethoxy]ethyl}carbamoyl)ethoxy]prop anoate (yellow oil).

Step 2. A solution of tert-butyl 3-[2-({2-[2-(2-{[(benzyloxy)carbonyl]-amino}ethoxy)ethoxy]ethyl}carbamoyl)ethoxy]propanoate (50.00 mg; 0.08 mmol; 1.00 eq.) and Pd/C (20.00 mg; 0.02 mmol; 0.23 eq.) in MeOH (5.00 ml) was stirred for 2 hours at degrees room temperature.

Step 3. A solution of tert-butyl 3-[2-({2-[2-(2-aminoethoxy)ethoxy]ethyl}-carbamoyl)ethoxy]propanoate (100.00 mg; 0.29 mmol; 1.00 eq.),2-methyl-8-[4-(trifluoromethyl)phenyl]-2H,8H-pyrazolo[3,4-b]indole-5-carboxylic acid **TEAD ligand 1 free acid** (103.00 mg; 0.29 mmol; 1.00 eq.) and HATU (115.00 mg; 0.29 mmol; 1.00 eq.) in DIEA (111.00 mg; 0.82 mmol; 2.84 eq.) was stirred for 2 hours at degrees room temperature under DCM (10.00 ml) atmosphere.The resulting mixture was extracted with EtOAc. The combined organic layers were washed with HCl(aq),NaHCO3(aq) NaCl(aq.), dried over anhydrous Na2SO4. After filtration, the filtrate was concentrated under reduced pressure. The residue was purified by silica gel column chromatography, eluted with PE/EtOAc (5:1) to afford tert-butyl 3-{2-[(2-{2-[2-({2-methyl-8-[4-(trifluoromethyl)phenyl]-2H,8H-pyrazolo[3,4-b]indol-5-yl}formam ido)ethoxy]ethoxy}ethyl)carbamoyl]ethoxy}propanoate (170.00 mg; 0.17 mmol; 59.0 %; yellow brown solid).

Step 4. The resulting mixture tert-butyl 3-{2-[(2-{2-[2-({2-methyl-8-[4-(trifluoromethyl)phenyl]-2H,8H-pyrazolo[3,4-b]indol-5-yl}formamido)-ethoxy]ethoxy}ethyl)carbamoyl]ethoxy}propanoate (300.00 mg; 0.30 mmol; 1.00 eq.)and HCl(gas) in 1,4-dioxane (10.00 ml; 40.00 mmol; 133.93 eq.).The mixture was stirred for 1h at room temperature. The resulting solid was dried under by lyophilization/under infrared light oven under reduced pressure.3-{2-[(2-{2-[2-({2-methyl-8-[4-(trifluoromethyl)-phenyl]-2H,8H-pyrazolo[3,4-b]indol-5-yl}formamido)ethoxy]ethoxy}ethyl)-carbamoyl]ethoxy}propanoic acid (200.00 mg; 0.24 mmol; 81.9 %; yellow solid). LC-MS (ESI+, Column: XBridge C8, 3.5 µm, 4.6 x 50 mm;Solvent A: water + 0.1 % TFA; Solvent B: ACN + 0.1 % TFA; Flow: 2 ml/min; Gradient: 0 min: 5 % B, 8 min: 100 % B, 8.1 min: 100 % B, 8.5 min: 5% B, 10 min 5% B.): t_{R} = 0.61 min, m/z 403.0 [M+H]+.

### Intermediate 39

### 3-{2-[2-({2-methyl-8-[4-(trifluoromethyl)phenyl]-2H,8H-pyrazolo[3,4-b]indol-5-yl}formamido)ethoxy]ethoxy}propanoic acid (intermediate 39)

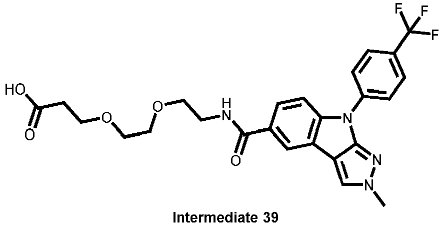

Step 1. To a stirred mixture of 2-methyl-8-[4-(trifluoromethyl)phenyl]-2H,8H-pyrazolo[3,4-b]indole-5-carboxylic acid (300.00 mg; 0.79 mmol; 1.00 eq.), tert-butyl 3-[2-(2-aminoethoxy)ethoxy]propanoate (233.76 mg; 0.95 mmol; 1.20 eq.) in DCM (10.00 ml) was added EDCl (320.12 mg; 1.59 mmol; 2.00 eq.), Et₃N (0.22 ml; 1.59 mmol; 2.00 eq.)at room temperature. The resulting mixture was stirred for 16 h at room temperature. The reaction was quenched with water at room temperature. The resulting mixture was extracted with DCM (3 x 150 mL). The combined organic layers were washed with brine (1x100 mL), dried over anhydrous Na2SO4. After filtration, the filtrate was concentrated under reduced pressure. The residue was purified by silica gel column chromatography, eluted with PE/EtOAc (4:1) to afford tert-butyl 3-{2-[2-({2-methyl-8-[4-(trifluoromethyl)phenyl]-2H,8H-pyrazolo[3,4-b]indol-5-yl}formamido)-ethoxy]ethoxy}propanoate (395.00 mg; 0.68 mmol; 86.0 %; colorless oil).

Step 2. To a stirred mixture oftert-butyl 3-{2-[2-({2-methyl-8-[4-(trifluoromethyl)phenyl]-2H,8H-pyrazolo[3,4-b]indol-5-yl}formamido)-ethoxy]ethoxy}propanoate (355.00 mg; 0.61 mmol; 1.00 eq.) in 4 M HCl(gas) in 1,4-dioxane (3.00 ml; 12.00 mmol; 19.57 eq.) at room temperature. The resulting mixture was stirred for 1 h at room temperature and then concentrated under reduced pressure to afford 3-{2-[2-({2-methyl-8-[4-(trifluoromethyl)phenyl]-2H,8H-pyrazolo[3,4-b]indol-5-yl}formamido)ethoxy]ethoxy}propanoic acid (275.00 mg; 0.49 mmol; 79.9 %; yellow oil). LC-MS (ESI+, Method 7): t_{R} = 0.97 min, m/z 519.0 [M+H]+.

### Intermediate 40

### 7-(2-{2-[2-({2-methyl-8-[4-(trifluoromethyl)phenyl]-2H,8H-pyrazolo[3,4-b]-indol-5-yl}formamido)ethoxy]ethoxy}ethoxy)heptanoic acid (intermediate 40)

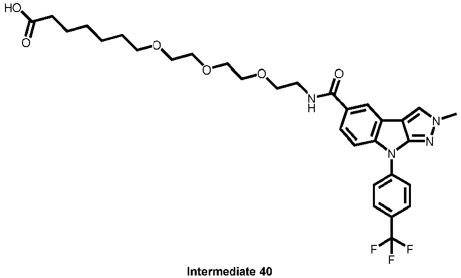

Step 1. To a stirred solution of tert-butyl N-{2-[2-(2-hydroxyethoxy)-ethoxy]ethyl}carbamate (1.20 g; 4.67 mmol; 1.07 eq.) in THF (20.00 ml) were added NaH (0.60 g; 15.00 mmol; 3.45 eq.) and dropwise methyl 7-bromoheptanoate (1.00 g; 4.35 mmol; 1.00 eq.) at 0 °C. The mixture was stirred for 3 h at RT under a nitrogen atmosphere. The reaction was quenched by the addition of H₂O at room temperature. The resulting mixture was extracted with EtOAc (3x50 mL). The combined organic layers were washed with H2O (3x50 mL]), dried over anhydrous Na₂SO₄. After filtration, the filtrate was concentrated under reduced pressure. The result was methyl 7-{2-[2-(2-{[(tert-butoxy)carbonyl]amino}ethoxy)ethoxy]-ethoxy}heptanoate (1.40 g; 2.86 mmol; 65.8 %; yellow liquid; Crude Product).

Step 2. To a stirred solution of the product (1.4 g) from the last step in DCM (20.000 ml) was added TFA (5.000 ml).The mixture was stirred for 3 h at RT. The resulting mixture was concentrated under reduced pressure. The resulting mixture was extracted with EtOAc (3x50 mL). The combined organic layers were washed with H2O (3x50 mL), dried over anhydrous Na2SO4. After filtration, the filtrate was concentrated under reduced pressure. The result was methyl 7-{2-[2-(2-aminoethoxy)-ethoxy]ethoxy}heptanoate (brown liquid; Crude Product), assumed quantitative yield.

Step 3. To a stirred solution of methyl 7-{2-[2-(2-aminoethoxy)ethoxy]-ethoxy}heptanoate (1.300 g; 0.892 mmol; 1.6 eq.) and 2-methyl-8-[4-(trifluoromethyl)phenyl]-2H,8H-pyrazolo[3,4-b]indole-5-carboxylic acid **TEAD ligand 1 free acid** (0.200 g; 0.557 mmol; 1.0 eq.) in DCM (20.000 ml) was added HATU (233.000 mg; 0.582 mmol; 1.0 eq.) and DIEA (276.000 µl; 1.505 mmol; 2.7 eq.) . The mixture was stirred for 2h at 25 degree under a nitrogen atmosphere. The resulting mixture was extracted with DCM/H2O (3x50 mL). The combined organic layers were washed with H2O (3x50 mL), dried over anhydrous Na2SO4. After filtration, the filtrate was concentrated under reduced pressure. The residue was purified by silica gel column chromatography, eluted with EtOAc/Hexanes to afford methyl 7-(2-{2-[2-({2-methyl-8-[4-(trifluoromethyl)phenyl]-2H,8H-pyrazolo[3,4-b]indol-5-yl}formamido)ethoxy]ethoxy}ethoxy)heptanoate (170.000 mg; 0.212 mmol; 38.2 %; yellow solid; Purified Product) .

Step 4. To a stirred solution of methyl 7-(2-{2-[2-({2-methyl-8-[4-(trifluoromethyl)phenyl]-2H,8H-pyrazolo[3,4-b]indol-5-yl}formamido)ethoxy]-ethoxy}ethoxy)heptanoate (170.000 mg; 0.212 mmol; 1.0 eq.) in MeOH (20.000 ml) was added NaOH (50.000 mg; 1.188 mmol; 5.6 eq.) in H2O (2.000 ml) at room temperature under nitrogen atmosphere. The resulting mixture was stirred for 4 h at 60 °C under nitrogen atmosphere. The resulting mixture was concentrated under reduced pressure. The mixture was acidified to pH 6 with conc. HCl.The precipitated solids were collected by filtration and washed with H2O. The result was 7-(2-{2-[2-({2-methyl-8-[4-(trifluoromethyl)phenyl]-2H,8H-pyrazolo[3,4-b]indol-5-yl}formamido)-ethoxy]ethoxy}ethoxy)heptanoic acid (40.000 mg; 0.058 mmol; 27.3 %; yellow liquid). LC-MS (ESI+, Method 3): tR = 0.92 min, m/z 619.0 [M+H]+.

### Intermediate 41

### 7-[2-({2-methyl-8-[4-(trifluoromethyl)phenyl]-2H,8H-pyrazolo[3,4-b]indol-5-yl}formamido)ethoxy]heptanoic acid (intermediate 41)

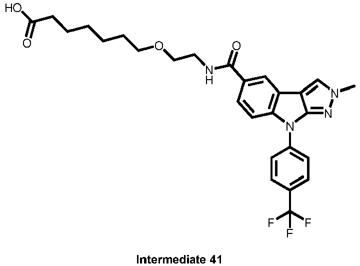

Step 1. To a solution of NaH (0.71 g; 17.68 mmol; 1.50 eq.) in DMF (20.00 ml) was added tert-butyl N-(2-hydroxyethyl)carbamate (2.00 g; 11.79 mmol; 1.00 eq.) at 0 °C. The mixture was stirred for 0.5 h. methyl 7-bromoheptanoate (3.32 g; 14.14 mmol; 1.20 eq.) was added and the mixture was allowed to warm to room temperature and stirred for 2 h. The reaction was quenched with water at room temperature. The resulting mixture was extracted with EtOAc (3 x 150 mL). The combined organic layers were washed with brine (1x100 mL), dried over anhydrous Na2SO4. After filtration, the filtrate was concentrated under reduced pressure to afford methyl 7-(2-{[(tert-butoxy)carbonyl]amino}ethoxy)heptanoate (3.58 g; 10.03 mmol; 85.1 %; colorless oil).

Step 2. To a stirred mixture of methyl 7-(2-{[(tert-butoxy)carbonyl]-amino}ethoxy)heptanoate (3.54 g; 9.92 mmol; 1.00 eq.) in 4 M HCl(gas) in 1,4-dioxane (20.00 ml) at room temperature. The resulting mixture was stirred for 1 h at room temperature. The reaction was concentrated under reduced pressure to afford methyl 7-(2-aminoethoxy)heptanoate (2.17 g; 9.07 mmol; 91.5 %; yellow oil).

Step 3. To a stirred mixture of methyl 7-(2-aminoethoxy)heptanoate (200.00 mg; 0.84 mmol; 1.00 eq.) 2-methyl-8-[4-(trifluoromethyl)phenyl]-2H,8H-pyrazolo[3,4-b]indole-5-carboxylic acid (380.00 mg; 1.00 mmol; 1.20 eq.) in DCM (10.00 ml) was added Et₃N (260.00 mg; 2.54 mmol; 3.04 eq.), HATU (670.00 mg; 1.67 mmol; 2.00 eq.)at room temperature. The resulting mixture was stirred for 16 h at room temperature. The reaction was quenched with water at room temperature. The resulting mixture was extracted with DCM (3 x 150 mL). The combined organic layers were washed with brine (1x100 mL), dried over anhydrous Na₂SO₄. After filtration, the filtrate was concentrated under reduced pressure. The residue was purified by silica gel column chromatography, eluted with PE/EtOAc (1:2) to afford methyl 7-[2-({2-methyl-8-[4-(trifluoromethyl)-phenyl]-2H,8H-pyrazolo[3,4-b]indol-5-yl}formamido)ethoxy]heptanoate (382.00 mg; 0.67 mmol; 79.8 %; white solid). LC-MS (ESI+, Method 7): tR = 0.97 min, m/z 545.0 [M+H]+.

Step 4. To a stirred mixture of methyl 7-[2-({2-methyl-8-[4-(trifluoromethyl)-phenyl]-2H,8H-pyrazolo[3,4-b]indol-5-yl}formamido)ethoxy]heptanoate (340.00 mg; 0.59 mmol; 1.00 eq.) in MeOH (10.00 ml), H2O (2.00 ml)was added NaOH (125.00 mg; 2.97 mmol; 5.00 eq.) at room temperature. The resulting mixture was stirred for 16 h at 60 degrees C. The reaction was quenched with H₂O at room temperature. The mixture was acidified to pH 2 with HCl (aq.). The resulting mixture was extracted with EtOAc (3 x 50 mL). The combined organic layers were washed with brine (1x10 mL), dried over anhydrous Na₂SO₄. After filtration, the filtrate was concentrated under reduced pressure to give 7-[2-({2-methyl-8-[4-(trifluoromethyl)-phenyl]-2H,8H-pyrazolo[3,4-b]indol-5-yl}formamido)ethoxy]heptanoic acid (297.00 mg; 0.54 mmol; 91.2 %; white solid). LC-MS (ESI+, Method 7): tR = 0.92 min, m/z 531.0[M+H]+.

### Intermediate 43

### 1-({2-methyl-8-[4-(trifluoromethyl)phenyl]-2H,8H-pyrazolo[3,4-b]indol-5-yl}formamido)-3,6,9,12-tetraoxahexadecan-16-oic acid (intermediate 43)

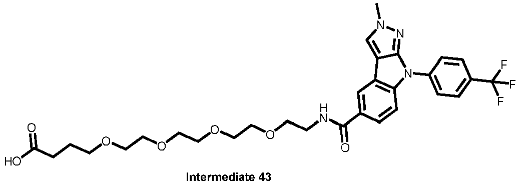

Step 1. A solution of tert-butyl 1-amino-3,6,9,12-tetraoxahexadecan-16-oate (commercially available from Pharmaron, 200.00 mg; 0.57 mmol; 1.00 eq.), 2-methyl-8-[4-(trifluoromethyl)phenyl]-2H,8H-pyrazolo[3,4-b]-indole-5-carboxylic acid **TEAD ligand 1 free acid** (280.00 mg; 0.62 mmol; 1.10 eq.) and HATU (222.00 mg; 0.55 mmol; 0.98 eq.) in DIEA (216.00 mg; 1.59 mmol; 2.80 eq.DCM (20.00 ml).The mixture was stirred for 2 hours at room temperature under atmosphere. The resulting mixture was extracted with EtOAc. The combined organic layers were washed with HCl(aq),NaHCO3(aq) NaCl(aq.), dried over anhydrous Na2SO4. After filtration, the filtrate was concentrated under reduced pressure. The residue was purified by silica gel column chromatography, eluted with PE/EtOAc (5:1) to afford tert-butyl 1-({2-methyl-8-[4-(trifluoromethyl)phenyl]-2H,8H-pyrazolo[3,4-b]indol-5-yl}formamido)-3,6,9,12-tetraoxahexadecan-16-oate (300.00 mg; 0.42 mmol; 74.2 %; yellow brown solid).

Step 2. To a stirred mixture of tert-butyl 1-({2-methyl-8-[4-(trifluoromethyl)-phenyl]-2H,8H-pyrazolo[3,4-b]indol-5-yl}formamido)-3,6,9,12-tetraoxahexadecan-16-oate (300.00 mg; 0.37 mmol; 1.00 eq.) and 4 M HCl(gas) in 1,4-dioxane (10.00 ml; 40.00 mmol; 107.42 eq.) The mixture was at degrees room temperature for 2h. The resulting solid was dried under vacuum lyophilization/under infrared in an oven under reduced pressure. The crude product was purified by Prep-HPLC with the following conditions (2#SHIMADZU (HPLC-01)): Column, XBridge Shield RP18 OBD Column, 19*250mm,10um; mobile phase, Water(0.1%FA) and MeOH- (20% PhaseB up to 40% in 8 min); Detector, uv 254 nm to afford 1-({2-methyl-8-[4-(trifluoromethyl)phenyl]-2H,8H-pyrazolo[3,4-b]indol-5-yl}formamido)-3,6,9,12-tetraoxahexadecan-16-oic acid (200.00 mg; 0.18 mmol; 47.6 %; yellow solid). LC-MS (ESI+, Column: XBridge C8, 3.5 µm, 4.6 x 50 mm;Solvent A: water + 0.1 % TFA; Solvent B: ACN + 0.1 % TFA; Flow: 2 ml/min; Gradient: 0 min: 5 % B, 8 min: 100 % B, 8.1 min: 100 % B, 8.5 min: 5% B, 10 min 5% B.): t_{R} = 0.85 min, m/z 621.0 [M+H]+.

### Intermediate 45

### 7-[methyl(2-{2-[2-({2-methyl-8-[4-(trifluoromethyl)pyridin-2-yl]-2H,8H-pyrazolo[3,4-b]indol-5-yl}formamido)ethoxy]ethoxy}ethyl)amino]heptanoic acid (intermediate 45)

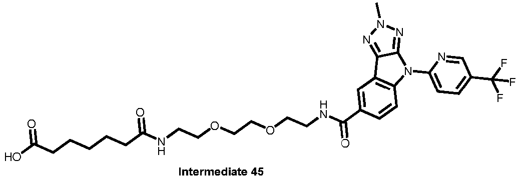

Step 1. To a stirred solution of 7-ethoxy-7-oxoheptanoic acid (2.00 g; 10.41 mmol; 1.00 eq.) and tert-butyl N-{2-[2-(2-aminoethoxy)ethoxy]ethyl}-carbamate (2.60 g; 10.26 mmol; 0.99 eq.) in DCM (50.00 ml) was added HATU (4.50 g; 11.24 mmol; 1.08 eq.) and DIEA (6738.54 µl; 36.75 mmol; 3.53 eq.) The mixture was stirred for 2h at 25 degree. The resulting mixture was extracted with HCl. The combined organic layers were washed with NaHCO₃ and NaCl, dried over anhydrous Na₂SO₄. After filtration, the filtrate was concentrated under reduced pressure. The result was ethyl 6-({2-[2-(2-{[(tert-butoxy)carbonyl]amino}ethoxy)ethoxy]ethyl}-carbamoyl)hexanoate (7.00 g; 8.36 mmol; 80.3 %; yellow liquid)

Step 2. To a stirred solution of ethyl 6-({2-[2-(2-{[(tert-butoxy)carbonyl]-amino}ethoxy)ethoxy]ethyl}carbamoyl)hexanoate (6.95 g; 8.30 mmol; 1.00 eq.) in DCM (120.00 ml; 1879.15 mmol; 226.32 eq.) was added TFA (8.00 ml). The mixture was stirred for 4 h at 25 °C. The resulting mixture was concentrated under reduced pressure. The resulting mixture was extracted with NaHCO3. After filtration, the filtrate was concentrated under reduced pressure. The result was ethyl 6-({2-[2-(2-aminoethoxy)ethoxy]ethyl}-carbamoyl)hexanoate (2.30 g; 7.01 mmol; 84.5 %; yellow liquid).

Step 3. To a stirred solution of 2-methyl-4-[5-(trifluoromethyl)pyridin-2-yl]-2H,4H-[1 2,3]triazolo[4,5-b]indole-7-carboxylic acid (prepared analogously to **TEAD ligand 2,** 300.00 mg; 0.78 mmol; 1.00 eq.) and ethyl 6-({2-[2-(2-aminoethoxy)ethoxy]ethyl}carbamoyl)hexanoate (660.00 mg; 2.01 mmol; 2.58 eq.) in DCM (50.00 ml) was added HATU (380.00 mg; 0.95 mmol; 1.22 eq.) and DIEA (0.50 ml; 2.73 mmol; 3.49 eq.) .The mixture was stirred for 2 h at 25 °C.The resulting mixture was extracted with NaHCO3 (3x50 mL). The combined organic layers were washed with NaCl (3x50 mL), dried over anhydrous Na2SO4. After filtration, the filtrate was concentrated under reduced pressure. The result was ethyl 6-[(2-{2-[2-({2-methyl-4-[5-(trifluoromethyl)pyridin-2-yl]-2H,4H-[1,2,3]triazolo[4,5-b]indol-7-yl}formamido)ethoxy]ethoxy}ethyl)carbamoyl]hexanoate (500.00 mg; 0.67 mmol; 85.4 %; yellow liquid).

Step 4. To a stirred solution of ethyl 6-[(2-{2-[2-({2-methyl-4-[5-(trifluoromethyl)pyridin-2-yl]-2H,4H-[1,2,3]triazolo[4,5-b]indol-7-yl}-formamido)ethoxy]ethoxy}ethyl)carbamoyl]hexanoate (500.00 mg; 0.67 mmol; 1.00 eq.) in EtOH (30.00 ml) and H20 (2.00 ml) was added NaOH (200.00 mg; 4.75 mmol; 7.13 eq.) The mixture was stirred for 6h at 60 °C. The resulting mixture was concentrated under reduced pressure. The mixture was acidified to pH 6 with conc. HCl.The precipitated solids were collected by filtration and washed with H2O.The result was 6-[(2-{2-[2-({2-methyl-4-[5-(trifluoromethyl)pyridin-2-yl]-2H,4H-[1,2,3]triazolo[4,5-b]indol-7-yl}formamido)ethoxy]ethoxy}ethyl)carbamoyl]hexanoic acid (400.00 mg; 0.63 mmol; 94.7 %; yellow liquid). LC-MS (ESI+, Method 3): tR = 0.92 min, m/z 531.0 [M+H]+.

### Intermediate 46

### 7-[methyl(2-{2-[2-({2-methyl-8-[4-(trifluoromethyl)phenyl]-2H,8H-pyrazolo[3,4-b]indol-5-yl}formamido)ethoxy]ethoxy}ethyl)amino]heptanoic acid (intermediate 46)

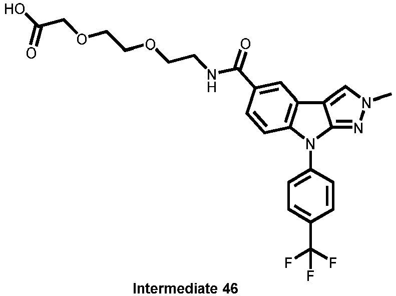

Step 1. A solution of tert-butyl 2-[2-(2-aminoethoxy)ethoxy]acetate (225.00 mg; 0.82 mmol; 1.00 eq.),2-methyl-8-[4-(trifluoromethyl)phenyl]-2H,8H-pyrazolo[3,4-b]indole-5-carboxylic acid **TEAD ligand 1 free acid** (246.00 mg; 0.65 mmol; 0.79 eq.) and HATU (273.00 mg; 0.68 mmol; 0.83 eq.) in DIEA (265.00 mg; 1.95 mmol; 2.37 eq.) was stirred for 2 hours at degrees room temperature under DCM (10.00 ml) atmosphere. The crude product was purified by Prep-HPLC with the following conditions (2#SHIMADZU (HPLC-01)): Column, XBridge Shield RP18 OBD Column, 19*250mm,10um; mobile phase, Water(0.1%FA) and MeOH- (20% PhaseB up to 40% in 8 min); Detector, uv 254 nm to afford tert-butyl 2-{2-[2-({2-methyl-8-[4-(trifluoromethyl)phenyl]-2H,8H-pyrazolo[3,4-b]indol-5-yl}formamido)ethoxy]ethoxy}acetate (200.00 mg; 0.36 mmol; 43.5 %; white solid).

Step 2. To a stirred mixture of tert-butyl 2-{2-[2-({2-methyl-8-[4-(trifluoromethyl)phenyl]-2H,8H-pyrazolo[3,4-b]indol-5-yl}formamido)-ethoxy]ethoxy}acetate (160.00 mg; 0.23 mmol; 1.00 eq.) and HCl-dioxane (6.00 ml; 24.00 mmol; 105.11 eq.),The mixture was stirred 2 hours at degrees room temperature.The resulting solid was dried under vacuum lyophilization/under infrared in an oven under reduced pressure. The crude product was purified by Prep-HPLC to afford 2-{2-[2-({2-methyl-8-[4-(trifluoromethyl)phenyl]-2H,8H-pyrazolo[3,4-b]indol-5-yl}formamido)-ethoxy]ethoxy}acetic acid (100.00 mg; 0.20 mmol; 86.8 %; white solid). LC-MS (ESI+, Column: XBridge C8, 3.5 µm, 4.6 x 50 mm;Solvent A: water + 0.1 % TFA; Solvent B: ACN + 0.1 % TFA; Flow: 2 ml/min; Gradient: 0 min: 5 % B, 8 min: 100 % B, 8.1 min: 100 % B, 8.5 min: 5% B, 10 min 5% B.): tR = 0.83 min, m/z 505.0 [M+H]+.

### Intermediate 47

### 7-[methyl(2-{2-[2-({2-methyl-8-[4-(trifluoromethyl)phenyl]-2H,8H-pyrazolo[3,4-b]indol-5-yl}formamido)ethoxy]ethoxy}ethyl)amino]heptanoic acid (intermediate 47)

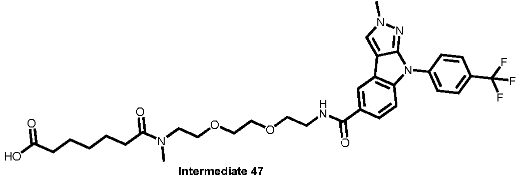

Prepared in the same manner as **intermediate** 45 but using tert-butyl N-(2-{2-[2-(methylamino)ethoxy]ethoxy}ethyl)carbamate (sec. amine) in step 1 instead of the free primary amine and TEAD **ligand** 1 instead in step 3 instead.

### Intermediate 60

### (2S,4R)-1-[(2S)-2-{10-[2-(2-aminoethoxy)ethoxy]decanamido}-3,3-dimethylbutanoyl]-4-hydroxy-N-{[4-(4-methyl-1,3-thiazol-5-yl)phenyl]methyl}pyrrolidine-2-carboxamide (intermediate 60)

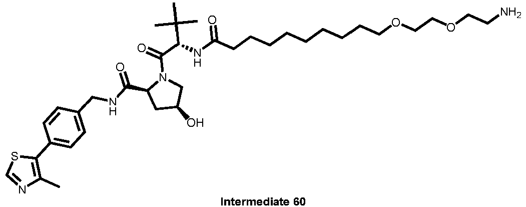

Step 1. Phthalic anhydride (1,479 g; 9,987 mmol; 105,00 mol%) and 2-(2-aminoethoxy)ethan-1-ol (commercially available from Acros Organics, 1,000 g; 9,512 mmol; 100,00 mol%) were premixed in the microwave vial and irradiated for 2 hrs at 150°C in a microwave reactor. The reaction mixture was dissolved with water and extracted 3 times with DCM. The combined organic layers were combined, dried over Na2SO4 and filtered. The concentrated crude product was purified by flash chromatography to give 2-(2-hydroxyethoxy)ethyl]-2,3-dihydro-1H-isoindole-1,3-dione (1,040 g; 4,266 mmol). LC-MS (ESI+, Method 1): tR = 1.10 min, m/z 236.1 [M+H]+.

Step 2. 2-[2-(2-hydroxyethoxy)ethyl]-2,3-dihydro-1H-isoindole-1,3-dione (500,000 mg; 2,075 mmol; 100,00 mol%) was dissolved in DMF (5,000 ml; 64,301 mmol; 3.099,56 mol%) under N2 protection. The mixture was cooled with ice-water. At 0°C sodium hydride (165,947 mg; 6,224 mmol; 300,00 mol%) was added to the mixture (colorless suspension). Afterwards tert-butyl 10-bromodecanoate (637,429 mg; 2,075 mmol; 100,00 mol%) was added dropwise. The mixture (off-white suspension) was warmed up to r.t. and stirred overnight at r.t.. More tert-butyl 10-bromodecanoate (318,715 mg; 1,037 mmol; 50,00 mol%) was addded. The reaction mixture was stirred overnight at r.t. The reaction mixture was stirred for 4 more hours at r.t. The reaction mixture was carefully quenched with water and 1N HCl. Then the mixture was extracted 3 times with EE. The combined organic layers were dried over Na₂SO₄, filtered and evaporated to give 10-{2-[2-(1,3-dioxo-2,3-dihydro-1H-isoindol-2-yl)ethoxy]ethoxy}decanoic acid (865.9 mg, 0.61 mmol, 29%). LC-MS (ESI+, Method 1): tR = 1.88 min, m/z 406 [M+H]+. Tert-butylated side product was deprotected with TFA/DCM (quant. Yield assumed) and used in the next step after evaporation of the volatiles and purification by prep HPLC.

Step 3. 10-{2-[2-(1,3-dioxo-2,3-dihydro-1H-isoindol-2-yl)ethoxy]ethoxy}-decanoic acid (95,992 mg; 0,232 mmol; 100,00 mol%) was dissolved in DMF (5,000 ml; 64,301 mmol; 27.715,85 mol%) at r.t. Then HATU, 97% (176,430 mg; 0,464 mmol; 200,00 mol%) and N,N-diisopropylethylamine (200,000 µl; 1,547 mmol; 667,01 mol%) were added. The reaction mixture was stirred for 10 min at room temperature. After that was added a solution of N,N-diisopropylethylamine (200,000 µl) in DMF (5,000 ml; 64,301 mmol; 27.715,85 mol%) and (2S,4S)-1-((S)-2-amino-3,3-dimethyl-butyryl)-4-hydroxy-pyrrolidine-2-carboxylic acid 4-(4-methyl-thiazol-5-yl)-benzyl-amide (commercially available from WuXi AppTec). The reaction mixture was stirred at room temperature overnight. The reaction mixture was filtered and purified by prep. HPLC-MS. to give (2S,4R)-1-[(2S)-2-(10-{2-[2-(1,3-dioxo-2,3-dihydro-1H-isoindol-2-yl)ethoxy]ethoxy}decanamido)-3,3-dimethylbutanoyl]-4-hydroxy-N-{[4-(4-methyl-1,3-thiazol-5-yl)phenyl]-methyl}pyrrolidine-2-carboxamide (160 mg, 0.18 mmol, 78%) as a colorless gum. LC-MS (ESI+, Method 1): tR = 1.79 min, m/z 818.0 [M+H]+.

Step 4. In a microwave vial Hydrazinium hydroxide (0,070 ml; 1,438 mmol; 800,00 mol%) was added to a solution of in Ethanol (2,000 ml). The mixture was irradiated in a microwave reactor for 30 min at 50°C. After cooling to r.t. and standing overnight - a precipitate was formed. The precipitated Phthalhydrazide was filtered and the filtrate was evaporated in vacuo to give (2S,4S)-1-[(2S)-2-{10-[2-(2-aminoethoxy)ethoxy]decanamido}-3,3-dimethylbutanoyl]-4-hydroxy-N-{[4-(4-methyl-1,3-thiazol-5-yl)-phenyl]methyl}pyrrolidine-2-carboxamide (159.4 mg, 0.16 mmol, 89%) as a colorless oil. LC-MS (ESI+, Method 1): tR = 1.34 min, m/z 347.7 [M+2H]2+.

### Intermediate 68

### 2-methyl-4-[(1r,4r)-4-(trifluoromethyl)cyclohexyl]-2H,4H-[1,2,3]triazolo[4,5-b]indole-7-carboxylic acid (intermediate 68)

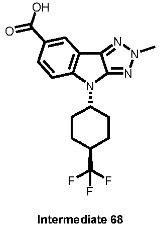

Step 1. To a stirred mixture of 4-(trifluoromethyl)cyclohexan-1-ol (303.00 mg; 1.80 mmol; 1.50 eq.),Cyanomethylene tributylphosphorane (645.00 mg; 2.41 mmol; 2.00 eq.),2-methyl-2H,4H-[1,2,3]triazolo[4,5-b]indole-7-carbonitrile (see synthesis of **TEAD ligand 2,** step 2) 300.00 mg; 1.20 mmol; 1.00 eq.) in Toluene (30.00 ml) at room temperature. The resulting mixture was stirred for 16 h at 90 °C under nitrogen atmosphere. The reaction was quenched with water at room temperature. The resulting mixture was extracted with EtOAc (3 x 50 mL). The combined organic layers were washed with H2O (3x100 mL), dried over anhydrous Na2SO4. After filtration, the filtrate was concentrated under reduced pressure. The residue was purified by silica gel column chromatography, eluted with PE/EtOAc (1:1) to afford 2-methyl-4-[4-(trifluoromethyl)cyclohexyl]-2H,4H-[1,2,3]triazolo[4,5-b]indole-7-carbonitrile (180.00 mg; 0.52 mmol; 43.1 %; yellow solid; Purified Product). LC-MS (ESI+, Method 7): tR = 0.73 min, m/z 348.0 [M+H]+.

Step 2. To a stirred mixture of 2-methyl-4-[4-(trifluoromethyl)cyclohexyl]-2H,4H-[1,2,3]triazolo[4,5-b]indole-7-carbonitrile (170.00 mg; 0.49 mmol; 1.00 eq.) in Water (10.00 ml) and EtOH (10.00 ml) was added NaOH (8488.32 mg; 201.65 mmol; 412.00 eq.) at room temperature. The resulting mixture was stirred for overnight at 80 degrees C . The reaction was quenched with H2O at room temperature. The mixture was acidified to pH 2 with Cond. HCl aq. The resulting mixture was extracted with EtOAc (3 x 50 mL). The combined organic layers were washed with brine (1x10 mL), dried over anhydrous Na2SO4. After filtration, the filtrate was concentrated under reduced pressure. The residue was purified by silica gel column chromatography, eluted with DCM/MeOH (5:1) to afford 2-methyl-4-[4-(trifluoromethyl)cyclohexyl]-2H,4H-[1,2,3]triazolo[4,5-b]indole-7-carboxylic acid (160.00 mg; 0.44 mmol; 89.2 %; white solid; Purified Product).

Step 3. The product was separated by Column: Xselect CSH OBD Column 30*150mm 5um, n; Mobile Phase A:Water (10MMOL/L NH4HCO3+0.1%NH3.H2O), Mobile Phase B:ACN; Flow rate:60 mL/min; Gradient:35 B to 55 B in 8 min). LC-MS (ESI+, Method 7): tR = 0.92 min, m/z 367.0 [M+H]+. ¹H NMR (300 MHz, DMSO-d6) 8.45 (d, J = 1.7 Hz, 1H), 8.08 - 7.99 (m, 1H), 7.72 (d, J = 8.9 Hz, 1H), 4.65 (s, 1H), 4.33 (s, 3H), 2.64 (s, 1H), 2.35 - 2.21 (m, 2H), 2.09 (d, J = 12.8 Hz, 2H), 1.93 (s, 4H).

### Intermediate 71

### 2-methyl-8-{[4-(trifluoromethyl)cyclohexyl]methyl}-2H,8H-pyrazolo[3,4-b]indole-5-carboxylic acid (intermediate 71)

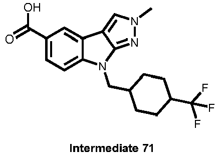

Step 1. To a stirred solution of ethyl 3-(3-amino-1-methylpyrazol-4-yl)-4-chlorobenzoate(10.00 g, 33.105 mmol, 1.00 equiv, 92.6%) and Cs₂CO₃ (23.28 g, 67.864 mmol, 2.05 equiv, 95%) in dioxane (300 mL) were added t-BuXPhos(2.96 g, 6.621 mmol, 0.20 equiv, 95%) and tBuXPhos Pd G3(2.77 g, 3.310 mmol, 0.10 equiv, 95%) at room temperature under nitrogen atmosphere. The resulting mixture was stirred for overnight at 120 degrees C under nitrogen atmosphere. The reaction was quenched with Water at room temperature. The resulting mixture was extracted with EtOAc (3 x200 mL). The combined organic layers were washed with brine (2x500 mL), dried over anhydrous Na2SO4. After filtration, the filtrate was concentrated under reduced pressure. The residue was purified by silica gel column chromatography, eluted with PE/EtOAc (2:1) to afford ethyl 2-methyl-8H-pyrazolo[3,4-b]indole-5-carboxylate(4.0523 g, 48%) as a white solid. LC-MS (ESI+, Method 3): tR = 0.77 min, m/z 244.1 [M+H]+.

Step 2. To a solution of ethyl 2-methyl-2H,8H-pyrazolo[3,4-b]indole-5-carboxylate (60,0 mg; 0,23 mmol; 1,00 äq.) in dry DMF (3,0 ml) was added Sodium hydride suspension (60% suspension in paraffin oil) (28,1 mg; 0,70 mmol; 3,00 äq.) and stirred for 0,5 hrs at rt. Then 1-(bromomethyl)-4-(trifluoromethyl)cyclohexane (69,0 mg; 0,28 mmol; 1,20 äq.) was added and stirred for 16 hrs at rt. More 1-(bromomethyl)-4-(trifluoromethyl)-cyclohexane (69,0 mg; 0,28 mmol; 1,20 äq.) was added and stirred for 16 hrs at rt. To the reaction was added water and purified direct by prep. HPLC which gave the desired 2-methyl-8-{[4-(trifluoromethyl)cyclohexyl]methyl}-2H,8H-pyrazolo[3,4-b]indole-5-carboxylic acid (30 mg, 0.08 mmol, 32%) alongside with the corresponding ethyl ester (discarded).

### Intermediate 78

### 9-{[4-(trifluoromethyl)phenyl]methyl}-9H-pyrido[3,4-b]indole-3-carboxylic acid (intermediate 78)

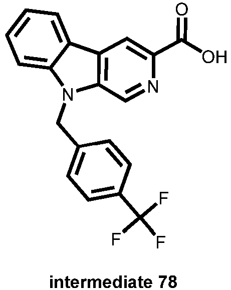

Step 1. To 9H-β-Carboline-3-carboxylic acid (Merck KGaA, 500.0 mg; 2.36 mmol; 1.00 Eq.) in Ethanol (200 ml) was added Thionyl chloride (1709.3 µl; 23.56 mmol; 10,00 Eq.) dropwise and stirred for 2 d and 16 hrs at 80°C. To the reaction was added some water and evaporate to dryness. The residue was basified with NaOH 2N and evaporated with EtOAc (2x). The combined organic layers were washed 2x with water, dried over Na₂SO₄ and evaporated to dryness. LC-MS (ESI+, Method 1): tR = 1.34 min, m/z 241.00 [M+H]+.

Step 2. To a solution of ethyl 9H-pyrido[3,4-b]indole-3-carboxylate (60.0 mg; 0.25 mmol; 1.00 Eq.) in DMF (3.0 ml) was added NaH (60% suspension in paraffin oil, 29,8 mg; 0,74 mmol; 3,00 äq.) and stirred for 0.5 h at RT. Then (4-trifluoromethyl)benzylbromide (71,1 mg; 0,30 mmol; 1,20 äq.) was added and stirred for 2 h at rt. To the reaction was added water and purified direct by prep. HPLC to give the desired product after lyophilization. LC-MS (ESI+, Method 1): t_{R} = 1.51 min, m/z 371.00 [M+H]+. ¹H NMR (700 MHz, DMSO-d6) 9.26 (s, 1H), 9.09 (s, 1H), 8.55 (d, J = 7.8 Hz, 1H), 7.81 (d, J = 8.4 Hz, 1H), 7.72 - 7.69 (m, 1H), 7.69 - 7.66 (m, 2H), 7.44 - 7.41 (m, 1H), 7.40 - 7.38 (m, 2H), 6.04 (s, 2H).

### Compound 1

### N'-[2-(2,6-dioxopiperidin-3-yl)-1,3-dioxo-2,3-dihydro-1H-isoindol-4-yl]-N-(2-{2-[2-({2-methyl-8-[4-(trifluoromethyl)phenyl]-2H,8H-pyrazolo[3,4-b]indol-5-yl}formamido)ethoxy]ethoxy}ethyl)-3,6,9,12,15-pentaoxaheptadecanediamide (Compound 1)

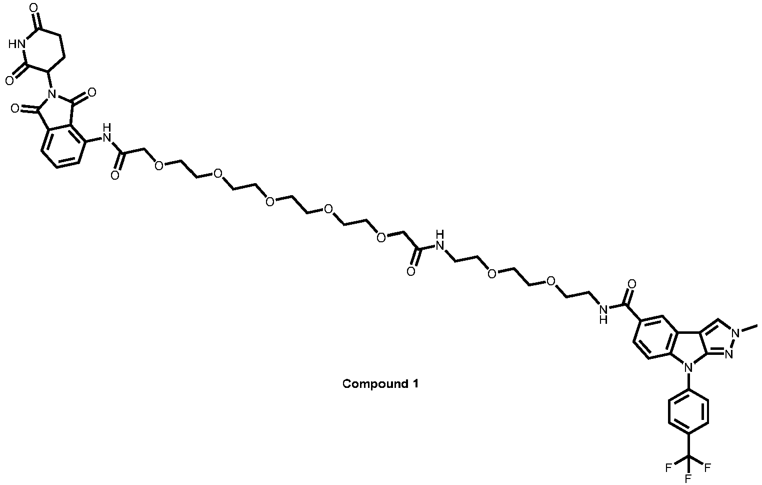

**Intermediate 1** (25 mg; 0.05 mmol) was dissolved in dry N,N-dimethylformamide (1 ml) in a 8 mL vial, followed by addition of **intermediate 12** (29 mg; 0.05 mmol), 4-methylmorpholine (0.06 ml) and HATU (23 mg; 0.06 mmol). The reaction was stirred for 18 h at RT. The crude reaction mixture was partitioned between 10% MeOH in DCM (1 mL) and water (1 mL). The aq. phase was extracted with further 10% MeOH in DCM (2 x 1 mL). The combined organic phases were filtered over a plug of Na₂SO₄ and concentrated by rotary evaporation. The crude material was dissolved in DCM (800 µL) and purified by flash chromatography (solution injection, 4 g SiO₂, 0 - 10% MeOH in DCM, 13 mL/min). After evaporation of volatiles, the compound was obtained as an oil, which was re-dissolved in ACN/H₂O and lyophilised to give **compound 1** (18 mg; 0.02 mmol, 33 %) as a white lyophilisate. LC-MS (ESI+, Method Himass): *t*_{R} = 1,65 min, m/z 1036,5 [M+H]⁺. ¹H NMR (700 MHz, DMSO-d6) δ 11.15 (s, 1H), 10.33 (s, 1H), 8.73 - 8.68 (m, 1H), 8.52 (t, J = 5.6 Hz, 1H), 8.33 (d, J = 1.9 Hz, 1H), 8.19 (s, 1H), 8.08 (d, J = 8.3 Hz, 2H), 7.96 (d, J = 8.5 Hz, 2H), 7.85 (ddd, J = 8.4, 4.5, 2.6 Hz, 2H), 7.77 (d, J = 8.6 Hz, 1H), 7.65 - 7.60 (m, 2H), 5.16 (dd, J = 13.0, 5.4 Hz, 1H), 4.18 (s, 2H), 4.03 (s, 3H), 3.85 (s, 2H), 3.78 - 3.72 (m, 2H), 3.69 - 3.63 (m, 2H), 3.59 - 3.41 (m, 21H), 3.26 (q, J = 5.9 Hz, 2H), 2.89 (ddd, J = 17.3, 14.0, 5.5 Hz, 1H), 2.61 (ddd, J = 17.4, 4.6, 2.5 Hz, 1H), 2.57 - 2.50 (m, 1H), 2.07 (tdd, J = 8.6, 5.5, 2.8 Hz, 1H).

### Compound 2

### 2-{2-[2-({[2-(2,6-dioxopiperidin-3-yl)-1,3-dioxo-2,3-dihydro-1H-isoindol-4-yl]carbamoyl}methoxy)ethoxy]ethoxy}-N-(2-{2-[2-({2-methyl-8-[4-(trifluoromethyl)phenyl]-2H,8H-pyrazolo[3,4-b]indol-5-yl}formamido)ethoxy]ethoxy}ethyl)acetamide (Compound 2)

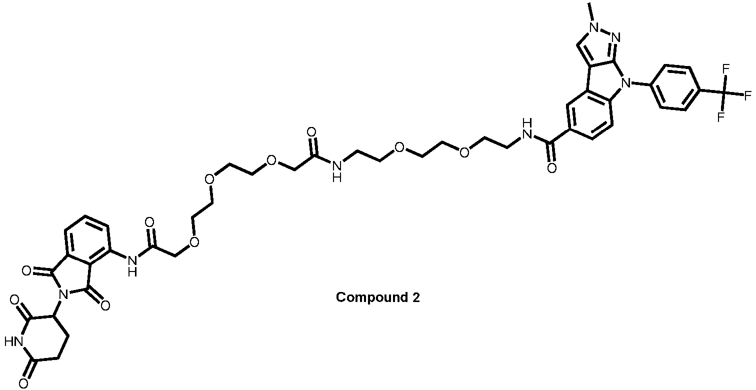

**Intermediate 1** (50 mg; 0.08 mmol) and **intermediate 13** 40 mg; 0.08 mmol) were dissolved in N,N-dimethylformamide (1mL) in a 8 mL vial with stir bar, followed by addition of 4-methylmorpholine (0.1 mL) and HATU (41 mg; 0.11 mmol). The reaction mixture was stirred for 8 h at RT and then adsorbed on celite to be purified by flash chromatography (4 g SiO₂, 0-10% MeOH in DCM, 13 ml/min) to give the product as an oil. The product was re-dissolved in ACN/H₂O and lyophilized to provide **compound 2** (44 mg; 0.04 mmol, 54 %) as an off-white lyophilisate. LC-MS (ESI+, Method Himass): *t*_{R} = 1.65 min, m/z 948.5 [M+H]⁺. ¹H NMR (700 MHz, DMSO-d6) δ 11.15 (s, 1H), 10.33 (s, 1H), 9.58 (s, 2H), 8.69 (d, J = 8.4 Hz, 1H), 8.51 (t, J = 5.6 Hz, 1H), 8.33 (d, J = 1.8 Hz, 1H), 8.19 (s, 1H), 8.08 (d, J = 8.3 Hz, 2H), 7.96 (d, J = 8.5 Hz, 2H), 7.87 - 7.81 (m, 2H), 7.76 (d, J = 8.6 Hz, 1H), 7.64 - 7.58 (m, 2H), 5.15 (dd, J = 13.0, 5.5 Hz, 1H), 4.18 (s, 2H), 4.03 (s, 3H), 3.99 - 3.96 (m, 4H), 3.84 (s, 2H), 3.76 (dd, J = 5.8, 3.6 Hz, 2H), 3.68 (dd, J = 5.8, 3.6 Hz, 2H), 3.61 - 3.49 (m, 13H), 3.44 (dt, J = 18.6, 5.9 Hz, 4H), 3.25 (q, J = 5.9 Hz, 2H), 3.06 (s, 4H), 2.93 - 2.86 (m, 1H), 2.81 (s, 6H), 2.64 - 2.58 (m, 1H), 2.57 - 2.51 (m, 1H), 2.07 (ddq, J = 10.6, 5.5, 2.8 Hz, 1H).

### Compound 3

### N-[(2S)-1-[(2S,4R)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]-N'-(2-{2-[2-({2-methyl-8-[4-(trifluoromethyl)phenyl]-2H,8H-pyrazolo[3,4-b]indol-5-yl}formamido)ethoxy]ethoxy}ethyl)-3,6,9,12-tetraoxatetradecanediamide (Compound 3)

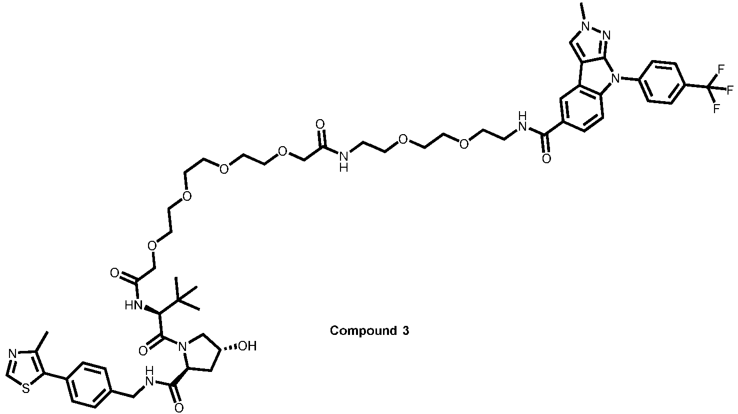

**Intermediate 1** (50 mg; 0.08 mmol) and **intermediate 14** were dissolved in N,N-dimethylformamide (1 ml) in a 8 mL vial with stir bar, followed by addition of 4-methylmorpholine (0.1 mL) and HATU (41 mg; 0.11 mmol). The reaction mixture was stirred for 18 h at RT and then adsorbed on celite for purification by flash chromatography (4 g SiO₂, 0-10% MeOH in DCM, 13 ml/min) to give the product as an oil. The product was lyophilized and still obtained as an oil. This oil was purified by prep. RP-HPLC to provide **compound 3** as a white lyophilisate (33 mg; 0.03 mmol, 35 %). LC-MS (ESI+, Method Sunfire): *t*_{R} = 2,37 min, m/z 575,4 [M+2H]²⁺. ¹ H NMR (700 MHz, DMSO-d6) δ 8.97 (s, 1H), 8.59 (t, J = 6.1 Hz, 1H), 8.53 (t, J = 5.6 Hz, 1H), 8.34 (d, J = 1.7 Hz, 1H), 8.20 (s, 1H), 8.08 (d, J = 8.4 Hz, 2H), 7.97 (d, J = 8.4 Hz, 2H), 7.85 (dd, J = 8.7, 1.9 Hz, 1H), 7.77 (d, J = 8.6 Hz, 1H), 7.64 (t, J = 5.9 Hz, 1H), 7.46 - 7.36 (m, 5H), 5.15 (s, 1H), 4.56 (d, J = 9.6 Hz, 1H), 4.44 (t, J = 8.2 Hz, 1H), 4.39 (dd, J = 15.7, 6.4 Hz, 1H), 4.35 (s, 1H), 4.30 - 4.23 (m, 1H), 4.03 (s, 3H), 3.96 (s, 2H), 3.85 (s, 2H), 3.67 (dd, J = 10.6, 4.0 Hz, 1H), 3.63 - 3.57 (m, 3H), 3.59 - 3.56 (m, 1H), 3.56 (s, 2H), 3.56 (q, J = 2.0 Hz, 3H), 3.55 - 3.49 (m, 10H), 3.45 (dt, J = 18.9, 5.9 Hz, 4H), 3.26 (q, J = 6.0 Hz, 2H), 2.43 (s, 3H), 2.09 - 2.03 (m, 1H), 1.90 (ddd, J = 13.0, 8.8, 4.5 Hz, 1H), 0.94 (s, 9H).

### Compound 4

### N-[(2S)-1-[(2S,4R)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]-N'-(2-{2-[2-({2-methyl-8-[4-(trifluoromethyl)phenyl]-2H,8H-pyrazolo[3,4-b]indol-5-yl}formamido)ethoxy]ethoxy}ethyl)heptanediamide (Compound 4)

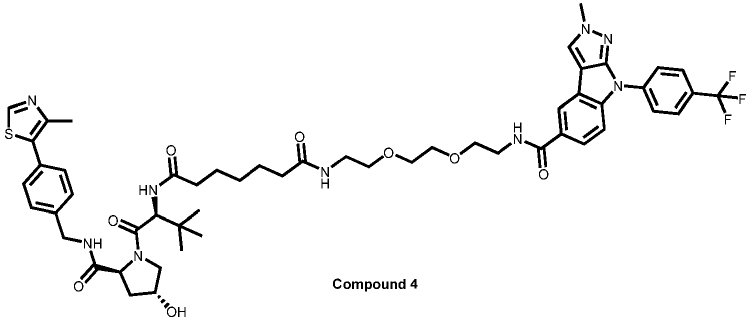

**Intermediate 1** (50 mg; 0.08 mmol) and 6-{[(2S)-1-[(2S,4R)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]carbamoyl}hexanoic acid (accessible analogously to **intermediate 5,** 47,45 mg; 0.08 mmol) were placed in a 8 mL vial with stir bar, followed by addition of 4-methylmorpholine (0.1 mL) and HATU (41 mg; 0.11 mmol). The reaction mixture was stirred for 18 h at RT and then adsorbed on celite to be purified by flash chromatography. The obtained crude oil was purified by prep. RP-HPLC, followed by lyophilization to give **compound 4** (19 mg; 0.02 mmol, 22 %) as a white lyophilisate. LC-MS (ESI+, Method Sunfire): *t*_{R} = 2,39 min, m/z 522,4 [M+2H]²⁺. ¹H NMR (700 MHz, DMSO-d6) δ 8.98 (s, 1H), 8.55 (dt, J = 11.6, 5.8 Hz, 2H), 8.35 (d, J = 1.8 Hz, 1H), 8.21 (s, 1H), 8.09 (d, J = 8.4 Hz, 2H), 7.97 (d, J = 8.4 Hz, 2H), 7.87 - 7.82 (m, 2H), 7.80 (t, J = 5.7 Hz, 1H), 7.77 (d, J = 8.6 Hz, 1H), 7.42 (d, J = 8.2 Hz, 2H), 7.38 (d, J = 8.1 Hz, 2H), 5.13 (s, 1H), 4.53 (d, J = 9.4 Hz, 1H), 4.46 - 4.40 (m, 2H), 4.35 (tt, J = 4.4, 2.4 Hz, 1H), 4.22 (dd, J = 15.8, 5.5 Hz, 1H), 4.03 (s, 3H), 3.69 - 3.62 (m, 2H), 3.59 - 3.53 (m, 4H), 3.53 (dd, J = 5.9, 3.4 Hz, 2H), 3.46 (q, J = 6.0 Hz, 2H), 3.40 (t, J = 6.0 Hz, 2H), 3.18 (q, J = 5.9 Hz, 2H), 2.44 (s, 3H), 2.23 (ddd, J = 14.9, 8.4, 6.9 Hz, 1H), 2.10 (ddd, J = 14.7, 8.6, 6.3 Hz, 1H), 2.03 (t, J = 7.5 Hz, 3H), 1.90 (ddd, J = 12.9, 8.6, 4.7 Hz, 1H), 1.53 - 1.39 (m, 4H), 1.20 (q, J = 7.9 Hz, 2H), 0.93 (s, 9H).

### Compound 4-N

### N-[(2S)-1-[(2S,4S)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]-N'-(2-{2-[2-({2-methyl-8-[4-(trifluoromethyl)phenyl]-2H,8H-pyrazolo[3,4-b]indol-5-yl}formamido)ethoxy]ethoxy}ethyl)heptanediamide (Compound 4-N)

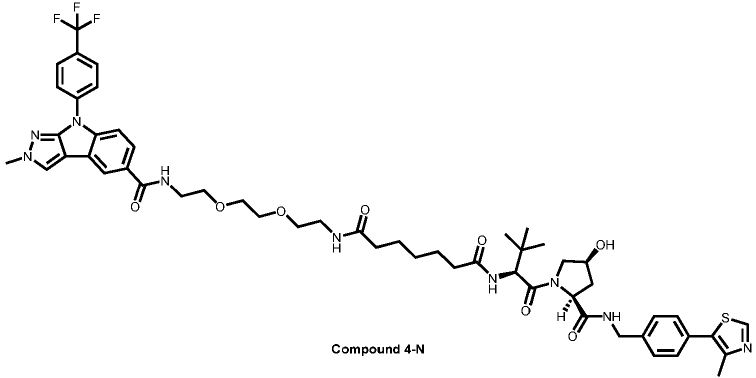

The reaction and purification was carried out analogously to the preparation of compound 4, but using the (S,S,S)-configured acid building block **(Intermediate 5)** and 1 h reaction time instead. After lyophilization, **Compound 4-N** (50 mg, 0.05 mmol, 39 %) was obtained as a clear film which was scratched to provide a white solid. LC-MS (ESI+, Method Chromolith): *t*_{R} = 1,70 min, m/z 1043,6 [M+H]⁺. ¹H NMR (500 MHz, DMSO-d6) δ 8.98 (s, 1H), 8.62 (t, J = 6.1 Hz, 1H), 8.53 (t, J = 5.6 Hz, 1H), 8.35 (d, J = 1.8 Hz, 1H), 8.20 (s, 1H), 8.09 (d, J = 8.3 Hz, 2H), 7.96 (d, J = 8.4 Hz, 2H), 7.89 - 7.74 (m, 4H), 7.40 (q, J = 8.2 Hz, 4H), 5.43 (s, 1H), 4.45 (dd, J = 14.6, 7.6 Hz, 2H), 4.37 (dd, J = 8.6, 6.1 Hz, 1H), 4.25 (ddt, J = 22.3, 11.3, 5.6 Hz, 2H), 4.04 (s, 3H), 3.94 (dd, J = 10.1, 5.6 Hz, 1H), 3.61 - 3.55 (m, 4H), 3.53 (dd, J = 6.1, 3.6 Hz, 2H), 3.51 - 3.38 (m, 5H), 3.19 (q, J = 5.9 Hz, 2H), 2.44 (s, 3H), 2.38 - 2.29 (m, 1H), 2.22 (dt, J = 14.8, 7.6 Hz, 1H), 2.14 - 2.05 (m, 1H), 2.04 (t, J = 7.5 Hz, 2H), 1.76 (dt, J = 12.3, 5.9 Hz, 1H), 1.48 (d, J = 7.8 Hz, 1H), 1.48 - 1.38 (m, 3H), 1.19 (p, J = 7.3 Hz, 2H), 0.95 (s, 9H).

### Compound 5

### 2-{[2-(2,6-dioxopiperidin-3-yl)-1,3-dioxo-2,3-dihydro-1H-isoindol-4-yl]oxy}-N-(2-{2-[2-({2-methyl-8-[4-(trifluoromethyl)phenyl]-2H,8H-pyrazolo[3,4-b]indol-5-yl}formamido)ethoxy]ethoxy}ethyl)acetamide (Compound 5)

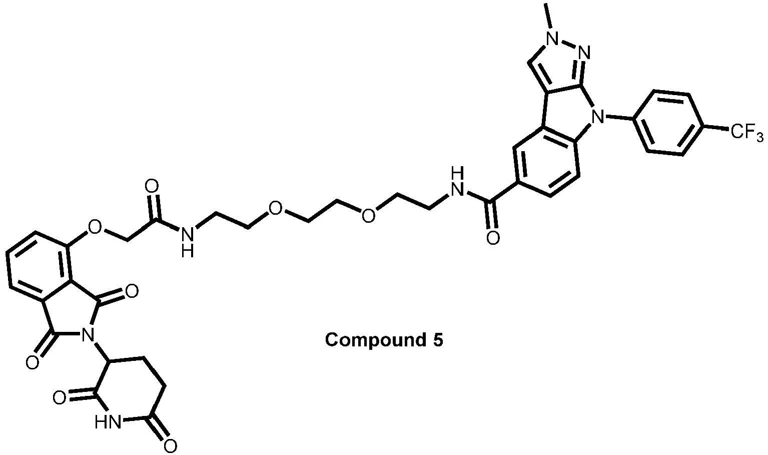

**Intermediate 1** (50 mg; 0.08 mmol) and 2-((2-(2,6-Dioxopiperidin-3-yl)-1,3-dioxoisoindolin-4-yl)oxy)acetic acid (accessible as described in Chem. Commun., 2020, 56, 2881-2884, 28 mg; 0.08 mmol) were dissolved in N,N-dimethylformamide (1 mL) in a 8 mL vial with stir bar, followed by addition of 4-methylmorpholine (0.1 mL) and then HATU (41 mg; 0.11 mmol). The reaction mixture was stirred for 18 h at RT, diluted with DMSO (1 mL) and purified by prep RP-HPLC. The target fractions were lyophilized to provide **compound 5** (26 mg, 0.03 mmol, 39 %) as a white lyophilisate. LC-MS (ESI+, Method Sunfire): *t*_{R} = 2,33 min, m/z 803,6 [M+H]⁺. ¹H NMR (700 MHz, DMSO-*d*₆) δ 11.12 (s, 1H), 8.51 (t, *J* = 5.6 Hz, 1H), 8.33 (d, *J =* 1.8 Hz, 1H), 8.19 (s, 1H), 8.08 (d, *J* = 8.4 Hz, 2H), 8.00 (t, *J* = 5.7 Hz, 1H), 7.96 (d, *J* = 8.5 Hz, 2H), 7.84 (dd, *J* = 8.6, 1.9 Hz, 1H), 7.80 - 7.74 (m, 2H), 7.46 (d, *J* = 7.2 Hz, 1H), 7.37 (d, *J* = 8.6 Hz, 1H), 5.76 (s, 1H), 5.11 (dd, J = 13.0, 5.4 Hz, 1H), 4.77 (s, 2H), 4.03 (s, 3H), 3.59 - 3.53 (m, 6H), 3.47 (dt, *J* = 11.8, 5.8 Hz, 4H), 3.31 (q, *J* = 5.6 Hz, 2H), 2.89 (ddd, *J* = 17.1, 14.0, 5.4 Hz, 1H), 2.59 (dt, *J* = 17.0, 3.4 Hz, 1H), 2.57 - 2.50 (m, 1H), 2.03 (dtd, *J* = 12.9, 5.4, 2.4 Hz, 1H).

### Compound 6

### 7-{[2-(2,6-dioxopiperidin-3-yl)-1,3-dioxo-2,3-dihydro-1H-isoindol-4-yl]amino}-N-(2-{2-[2-({2-methyl-8-[4-(trifluoromethyl)phenyl]-2H,8H-pyrazolo[3,4-b]indol-5-yl}formamido)ethoxy]ethoxy}ethyl)heptanamide (Compound 6)

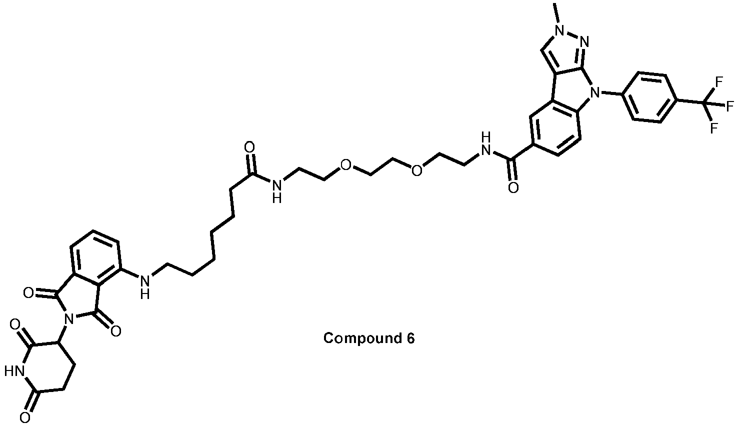

**Intermediate 1** (50 mg; 0.08 mmol) and Pomalidomide-C6-CO2H (commercially available from Sigma-Aldrich, 33 mg; 0.08 mmol) were dissolved in N,N-dimethylformamide (1 mL) in a 8 mL vial with stir bar, followed by addition of 4-methylmorpholine (01 ml) and then HATU (41 mg; 0.11 mmol). The reaction mixture was stirred for 18 h at RT. After dilution with DMSO (1 mL) the mixture was injected for purification by prep. RP-HPLC. The target fractions were lyophilized to provide **compound 6** (34 mg; 0.04 mmol, 47 %) as a bright yellow lyophilisate. LC-MS (ESI+, Method Sunfire): *t*_{R} = 2,47 min, m/z 872,6 [M+H]⁺. ¹H NMR (700 MHz, DMSO-*d*₆) δ 11.09 (s, 1H), 8.53 (q, *J* = 5.8, 5.1 Hz, 1H), 8.34 (d, *J* = 1.8 Hz, 1H), 8.20 (s, 1H), 8.08 (d, *J* = 8.4 Hz, 2H), 7.96 (d, *J* = 8.5 Hz, 2H), 7.85 (dd, *J* = 8.7, 1.8 Hz, 1H), 7.81 (t, *J* = 5.7 Hz, 1H), 7.77 (d, *J* = 8.6 Hz, 1H), 7.55 (dd, *J* = 8.5, 7.1 Hz, 1H), 7.05 (d, *J* = 8.6 Hz, 1H), 7.00 (d, *J =* 7.0 Hz, 1H), 6.49 (d, *J* = 6.2 Hz, 1H), 5.05 (dd, J = 12.8, 5.5 Hz, 1H), 4.03 (s, 2H), 3.59 - 3.50 (m, 5H), 3.46 (q, *J* = 6.0 Hz, 2H), 3.40 (t, *J* = 5.9 Hz, 2H), 3.24 (q, *J* = 6.6 Hz, 2H), 3.18 (q, *J* = 5.8 Hz, 2H), 2.88 (ddd, *J* = 17.1, 13.9, 5.4 Hz, 1H), 2.62 - 2.53 (m, 1H), 2.09 - 1.99 (m, 3H), 1.50 (dp, *J* = 40.8, 7.3 Hz, 4H), 1.36 - 1.21 (m, 4H).

### Compound 7

### 2-{[2-(2,6-dioxopiperidin-3-yl)-1,3-dioxo-2,3-dihydro-1H-isoindol-5-yl]oxy}-N-(2-{2-[2-({2-methyl-8-[4-(trifluoromethyl)phenyl]-2H,8H-pyrazolo[3,4-b]indol-5-yl}formamido)ethoxy]ethoxy}ethyl)acetamide (Compound 7)

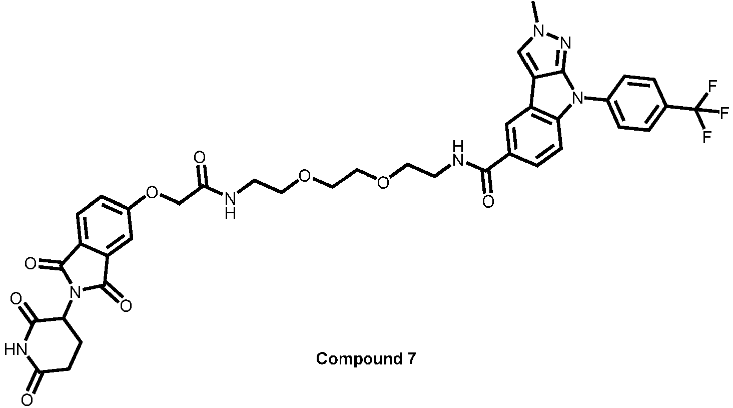

2-(2,6-dioxopiperidin-3-yl)-5-hydroxy-2,3-dihydro-1H-isoindole-1,3-dione (1 g; 3.68 mmol) was dissolved in N,N-Dimethylformamide (7.5 mL) in a 50 mL RBF with stir bar. Sodium hydrogen carbonate (766 mg, 9.12 mmol) and tert-Butyl bromoacetate (0.7 ml; 4.56 mmol) were added. The reaction mixture was stirred for 18 hours at 60°C. The reaction mixture was cooled to RT and poured into 100 mL water. It was extracted twice with each 100 mL EtOAc. The combined organic layers were dried with Na₂SO₄, filtered and evaporated under reduced pressure. The residue (1,49 g brown oil) was chromatographed over a 40 g silica gel column with dichloromethane / methanol gradient. The evaporated target fractions were suspended in MTBE, the suspension filtered off and the filter cake dried under vacuum. The filter cake was washed with 10 ml diethyl ether and dried under vacuum at RT. The compound was deprotected in DCM (10 mL) by addition of TFA (5 mL). The reaction mixture was stirred for 4 h at RT, the volatiles were evaporated and the residue was suspended in MTBE, the suspension filtered off and the filter cake was dried under vacuum. The filter cake was washed with 10 mL MTBE and dried under vacuum at 50°C for 2 h to give 2-{[2-(2,6-dioxopiperidin-3-yl)-1,3-dioxo-2,3-dihydro-1H-isoindol-5-yl]oxy}acetic acid (1.03 g; 2.94 mmol; 75 % over two steps). LC-MS (ESI+, Method Chromolith): *t*_{R} = 1,45 min, m/z 389 [M+H]⁺. **Intermediate 1** (50 mg; 0.08 mmol) and 2-{[2-(2,6-dioxopiperidin-3-yl)-1,3-dioxo-2,3-dihydro-1H-isoindol-5-yl]oxy}acetic acid (28 mg; 0.08 mmol) were dissolved in N,N-dimethylformamide (1 mL) in a 8 mL vial with stir bar, followed by addition of 4-methylmorpholine (01 mL) and HATU (41 mg; 0.11 mmol) to start the reaction, which was stirred for 3 d at RT. The crude mixture was diluted with DMSO (1 mL), followed by purification with prep. RP-HPLC. The target fractions were lyophilized to provide **compound 7** (29 mg; 0.04 mmol; 44 %) as a white lyophilisate. LC-MS (ESI+, Method Sunfire): *t*_{R} = 2,33 min, m/z 803,6 [M+H]⁺. ¹H NMR (700 MHz, DMSO-*d*₆) δ 11.12 (s, 1H), 8.51 (t, *J* = 5.6 Hz, 1H), 8.33 (d, *J* = 1.8 Hz, 1H), 8.19 (s, 1H), 8.08 (d, *J* = 8.4 Hz, 2H), 8.00 (t, *J* = 5.7 Hz, 1H), 7.96 (d, *J* = 8.5 Hz, 2H), 7.84 (dd, *J* = 8.6, 1.9 Hz, 1H), 7.80 - 7.74 (m, 2H), 7.46 (d, *J* = 7.2 Hz, 1H), 7.37 (d, *J* = 8.6 Hz, 1H), 5.76 (s, 1H), 5.11 (dd, *J =* 13.0, 5.4 Hz, 1H), 4.77 (s, 2H), 4.03 (s, 3H), 3.59 - 3.53 (m, 6H), 3.47 (dt, *J* = 11.8, 5.8 Hz, 4H), 3.31 (q, *J* = 5.6 Hz, 2H), 2.89 (ddd, *J* = 17.1, 14.0, 5.4 Hz, 1H), 2.59 (dt, *J* = 17.0, 3.4 Hz, 1H), 2.57 - 2.50 (m, 1H), 2.03 (dtd, *J* = 12.9, 5.4, 2.4 Hz, 1H).

### Compound 8

### N-[2-(2,6-dioxopiperidin-3-yl)-1-oxo-2,3-dihydro-1H-isoindol-4-yl]-N'-(2-{2-[2-({2-methyl-8-[4-(trifluoromethyl)phenyl]-2H,8H-pyrazolo[3,4-b]indol-5-yl}formamido)ethoxy]ethoxy}ethyl)pentanediamide (Compound 8)

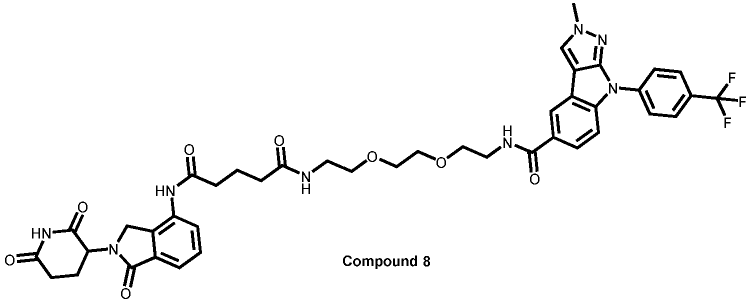

To a solution of lenalidomide (1.20 g; 4.63 mmol) in toluene (10 ml) was added glutaric anhydride (634 mg; 5.55 mmol) and the reaction was stirred at 100°C for 4 h. After cooling to 0°C, the solid was filtered off and washed with cold toluene to furnish 4-{[2-(2,6-dioxopiperidin-3-yl)-1-oxo-2,3-dihydro-1H-isoindol-4-yl]carbamoyl}butanoic acid as a pale yellow solid (1.21 g; 3.03 mmol, 66 % based on 94 % purity by LCMS analysis). LC-MS (ESI+, Method Sunfire): *t*_{R} = 1,67 min, m/z 373,9 [M+H]⁺. **Intermediate 1** (50 mg; 0.08 mmol) was dissolved in N,N-dimethylformamide (1 mL) in a 8 mL vial with stir bar, followed by addition of 4-{[2-(2,6-dioxopiperidin-3-yl)-1-oxo-2,3-dihydro-1H-isoindol-4-yl]carbamoyl}butanoic acid (31 mg; 0.08 mmol) and 4-methylmorpholine (0.1 ml). The reaction was started by addition of HATU (41 mg; 0.11 mmol). The reaction mixture was stirred at RT for 18 h., diluted with DMSO (1 mL) and purified by prep. RP-HPLC. The target fractions were lyophilized to provide **compound 8** (31 mg; 0.04 mmol; 44 %) as a white lyophilisate. LC-MS (ESI+, Method Sunfire): *t*_{R} = 2,20 min, m/z 844,6 [M+H]⁺. ¹H NMR (400 MHz, DMSO-*d*₆) δ 10.98 (s, 1H), 9.73 (s, 1H), 8.49 (t, *J* = 5.6 Hz, 1H), 8.33 (d, *J* = 1.8 Hz, 1H), 8.19 (s, 1H), 8.08 (d, *J* = 8.4 Hz, 2H), 7.96 (d, *J* = 8.7 Hz, 2H), 7.88 - 7.78 (m, 3H), 7.76 (d, *J* = 8.6 Hz, 1H), 7.53 - 7.42 (m, 2H), 5.13 (dd, *J* = 13.3, 5.1 Hz, 1H), 4.44 - 4.29 (m, 2H), 4.03 (s, 3H), 3.61 - 3.50 (m, 6H), 3.44 (dt, *J =* 18.6, 5.8 Hz, 4H), 3.20 (q, *J* = 5.9 Hz, 2H), 2.91 (ddd, *J* = 17.2, 13.5, 5.4 Hz, 1H), 2.65 - 2.56 (m, 1H), 2.42 - 2.27 (m, 3H), 2.15 (t, *J* = 7.3 Hz, 2H), 2.09 - 1.96 (m, 1H), 1.82 (p, *J* = 7.5 Hz, 2H).

### Compound 9

### 5-{[2-(2,6-dioxopiperidin-3-yl)-1-oxo-2,3-dihydro-1H-isoindol-4-yl]amino}-N-(2-{2-[2-({2-methyl-8-[4-(trifluoromethyl)phenyl]-2H,8H-pyrazolo[3,4-b]indol-5-yl}formamido)ethoxy]ethoxy}ethyl)pentanamide (Compound 9)

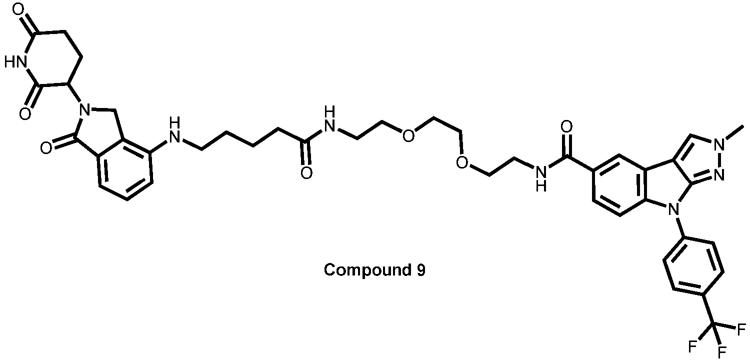

**Intermediate 1** (72 mg; 0.12 mmol) and **Intermediate 2** (43 mg; 0.12 mmol) were combined in a 8 mL vial with stir bar, dissolved in dimethylformamide (DMF) (1 mL), followed by addition of 4-methylmorpholine (0.13 ml) and then HATU (59 mg; 0.16 mmol). The reaction mixture was stirred at RT for 1 d. Then the mixture was diluted with H₂O (1 mL) and injected into an MS coupled prep HPLC system. The target fractions were lyophilized to provide the product as a white lyophilisate (37 mg, 0.04 mmol, 37 %). LC-MS (ESI+, Method Sunfire): *t*_{R} = 2,29 min, m/z 830,6 [M+H]⁺. ¹H NMR (500 MHz, DMSO-*d*₆) δ 10.99 (s, 1H), 8.53 (t, *J* = 5.6 Hz, 1H), 8.34 (d, *J* = 1.8 Hz, 1H), 8.20 (s, 1H), 8.08 (d, *J* = 8.5 Hz, 2H), 7.96 (d, *J* = 8.5 Hz, 2H), 7.88 - 7.81 (m, 2H), 7.77 (d, *J =* 8.7 Hz, 1H), 7.26 (t, *J* = 7.7 Hz, 1H), 6.92 (d, *J* = 7.2 Hz, 1H), 6.71 (d, *J =* 8.0 Hz, 1H), 5.55 (s, 1H), 5.11 (dd, *J* = 13.3, 5.1 Hz, 1H), 4.22 (d, *J =* 17.1 Hz, 1H), 4.12 (d, *J* = 17.1 Hz, 1H), 4.03 (s, 3H), 3.59 - 3.53 (m, 4H), 3.52 (dd, *J* = 5.6, 3.0 Hz, 2H), 3.46 (q, *J* = 6.0 Hz, 2H), 3.40 (t, *J* = 5.9 Hz, 2H), 3.19 (q, *J =* 5.9 Hz, 2H), 3.09 (t, *J* = 6.7 Hz, 2H), 2.92 (ddd, *J =* 17.3, 13.6, 5.4 Hz, 1H), 2.65 - 2.57 (m, 1H), 2.29 (qd, *J* = 13.2, 4.5 Hz, 1H), 2.10 (q, *J* = 8.5, 7.7 Hz, 2H), 2.03 (ddq, *J* = 10.3, 5.3, 2.6 Hz, 1H), 1.63 - 1.48 (m, 3H).

### Compound 10

### N-[2-(2,6-dioxopiperidin-3-yl)-1,3-dioxo-2,3-dihydro-1H-isoindol-4-yl]-17-({2-methyl-8-[4-(trifluoromethyl)phenyl]-2H,8H-pyrazolo[3,4-b]indol-5-yl}formamido)-3,6,9,12,15-pentaoxaheptadecanamide (Compound 10)

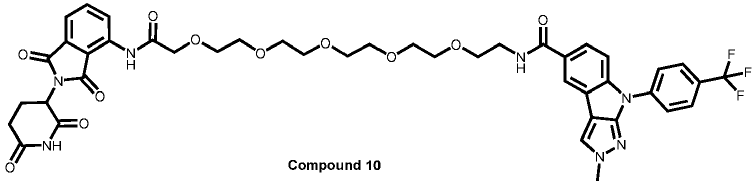

To a mixture of 4-amino-2-(2,6-dioxopiperidin-3-yl)-2,3-dihydro-1H-isoindole-1,3-dione (Pomalidomide, commercially available from Sigma Aldrich) (300 mg; 1.08 mmol) in DMF (4 mL) was added 17-{[(tert-butoxy)carbonyl]amino}-3,6,9,12,15-pentaoxaheptadecanoic acid (commercially from Sigma-Aldrich; 780 mg; 1.93 mmol) at 0 °C. Then Pyridine (0.9 mL) and T3P (50% in EtOAc solution (4 mL; 6.73 mmol) were added at 0°C. After the mixture was stirred at 80°C for 30 min. The resulting mixture was cooled to room temperature, then diluted with water (100 mL) and extracted with EtOAc (30 mL x 5). The combined organic layers were concentrated and the residue was purified by reverse phase chromatography (Column: C18 silica gel; Mobile Phase A: water (0.1 % FA) and B: MeCN; Gradient: 5% to 100% in 40 min; Detector: 254/220 nm) to afford tert-butyl N-(1-{[2-(2,6-dioxopiperidin-3-yl)-1,3-dioxo-2,3-dihydro-1H-isoindol-4-yl]carbamoyl}-2,5,8,11,14-pentaoxahexadecan-16-yl)carbamate (450 mg; 0.68 mmol; 63 %; green solid). To a solution of tert-butyl N-(1-{[2-(2,6-dioxopiperidin-3-yl)-1,3-dioxo-2,3-dihydro-1H-isoindol-4-yl]carbamoyl}-2,5,8,11,14-pentaoxahexadecan-16-yl)carbamate (300 mg; 0.45 mmol) in DCM (4 mL) was added TFA (1.20 mL) at 0°C. The solution was stirred at RT for 30 min and then concentrated under reduced pressure to afford 17-amino-N-[2-(2,6-dioxopiperidin-3-yl)-1,3-dioxo-2,3-dihydro-1H-isoindol-4-yl]-3,6,9,12,15-pentaoxaheptadecanamide (220 mg; 0.38 mmol; 85 %) as a green solid. **TEAD ligand 1** (20 mg; 0.06 mmol), 17-amino-N-[2-(2,6-dioxopiperidin-3-yl)-1,3-dioxo-2,3-dihydro-1H-isoindol-4-yl]-3,6,9,12,15-pentaoxaheptadecanamide (61 mg; 0.11 mmol), HATU (25 mg; 0.07 mmol) and 4-methylmorpholine (24 µL; 0.22 mmol) were dissolved in DMF (5 ml) and stirred overnight at RT. The reaction mixture was directly purified by prep. RP-HPLC and the target fractions freed from solvent *in vacuo,* giving **compound 10** (26 mg; 0.03 mmol, 52 %). LC-MS (ESI+, Method Chromolith): *t*_{R} = 1,68 min, m/z 892,00 [M+H]⁺. ¹H NMR (700 MHz, DMSO-*d*₆) δ 11.14 (s, 1H), 10.32 (s, 1H), 8.70 (d, *J =* 8.4 Hz, 1H), 8.51 (t, *J =* 5.6 Hz, 1H), 8.33 (d, *J* = 1.9 Hz, 1H), 8.19 (s, 1H), 8.08 (d, *J* = 8.4 Hz, 2H), 7.96 (d, *J* = 8.5 Hz, 2H), 7.87 - 7.82 (m, 2H), 7.76 (d, *J* = 8.6 Hz, 1H), 7.61 (d, *J* = 7.3 Hz, 1H), 5.15 (dd, *J* = 13.0, 5.4 Hz, 1H), 4.17 (s, 2H), 4.03 (s, 3H), 3.75 - 3.71 (m, 2H), 3.65 - 3.61 (m, 2H), 3.58 - 3.42 (m, 17H), 2.89 (s, 1H), 2.93 - 2.85 (m, 1H), 2.73 (s, 0H), 2.62 (dd, *J* = 4.4, 2.5 Hz, 1H), 2.06 (dtt, *J* = 13.0, 5.5, 2.7 Hz, 1H), 1.99 (s, 1H), 1.17 (t, *J* = 7.1 Hz, 2H).

### Compound 11

### (2R,4S)-1-[(2R)-3,3-dimethyl-2-[17-({2-methyl-8-[4-(trifluoromethyl)phenyl]-2H,8H-pyrazolo[3,4-b]indol-5-yl}formamido)-3,6,9,12,15-pentaoxaheptadecanamido]butanoyl]-4-hydroxy-N-{[4-(4-methyl-1,3-thiazol-5-yl)phenyl]methyl}pyrrolidine-2-carboxamide (Compound 11)

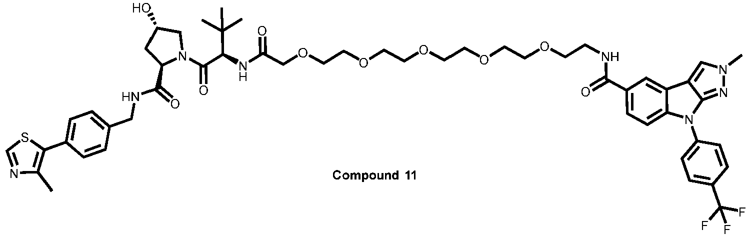

17-{[(tert-butoxy)carbonyl]amino}-3,6,9,12,15-pentaoxaheptadecanoic acid (commercially available from Sigma-Aldrich; 898 mg; 2.27 mmol) was diluted in N,N-Dimethylformamide (15 ml) and cooled down to 0°C.Then HATU (802 mg; 3.41 mmol) was added and the mixture was stirred for 5 minutes. Finally, (2S,4R)-1-((S)-2-Amino-3,3-dimethyl-butyryl)-4-hydroxy-pyrrolidine-2-carboxylic acid 4-(4-methyl-thiazol-5-yl)-benzylamide (815 mg; 1.89 mmol) and N-Ethyldiisopropylamine (772 µL; 4.54 mmol) were added and the reaction mixture was stirred at RT overnight. HATU (89 mg; 0.38 mmol) and (2S,4R)-1-((S)-2-Amino-3,3-dimethyl-butyryl)-4-hydroxy-pyrrolidine-2-carboxylic acid 4-(4-methyl-thiazol-5-yl)-benzylamide (163 mg; 0.38 mmol) were added and the mixture was stirred overnight again. The reaction mixture was evaporated under reduced pressure to dryness. The crude product was dissolved in MeOH / DMSO and purified by prep. flash chromatography (Teledyne Isco). The product fractions were combined and evaporated under reduced pressure to dryness. The two main fractions showed an impurity via LC-MS measurement. Both fractions were combined and purified by prep. flash chromatography again. The obtained yellow residue was dissolved in dichloromethane (15 mL) and Trifluoroacetic acid (7 mL). The mixture was stirred overnight at room temperature. LC-MS showed complete consumption. The mixture was evaporated under reduced pressure and coevaporated 3 times with toluene (15 mL each). Finally, (2S,4R)-1-[(2S)-2-(17-amino-3,6,9,12,15-pentaoxaheptadecanamido)-3,3-dimethylbutanoyl]-4-hydroxy-N-{[4-(4-methyl-1,3-thiazol-5-yl)phenyl]methyl}pyrrolidine-2-carboxamide (1224,70 mg; 1,73 mmol) was obtained as a colorless solid. LCMS (ESI+, Method Chromolith): *t*_{R} = 1,17 min, m/z 708,4 [M+H]⁺. **TEAD ligand 1** (30 mg; 0.08 mmol), (2R,4S)-1-[(2R)-2-(17-amino-3,6,9,12,15-pentaoxaheptadecan-amido)-3,3-dimethylbutanoyl]-4-hydroxy-N-{[4-(4-methyl-1,3-thiazol-5-yl)phenyl]methyl}pyrrolidine-2-carboxamide (59 mg; 0.08 mmol), HATU (38 mg; 0.10 mmol) and 4-Methylmorpholine (37 µL; 0.33 mmol) were dissolved in N,N-Dimethylformamid (5 ml) and stirred overnight at room temperature. The reaction mixture was directly purified by prep. RP-HPLC. column chromatography and the target fractions freed from solvent in vacuo, giving **compound 11** (23 mg; 0.02 mmol, 25 % based on 95 % purity by LCMS). LC-MS (ESI+, Method Chromolith): *t*_{R} = 1,68 min, m/z 525,0 [M+2H]²⁺. ¹H NMR (700 MHz, DMSO-*d*₆) δ 8.97 (s, 1H), 8.59 (t, *J =* 6.1 Hz, 1H), 8.53 (t, *J* = 5.6 Hz, 1H), 8.34 (d, *J* = 1.8 Hz, 1H), 8.20 (s, 1H), 8.08 (d, *J* = 8.6 Hz, 2H), 7.96 (d, *J* = 8.3 Hz, 2H), 7.85 (dd, *J* = 8.7, 1.9 Hz, 1H), 7.77 (d, *J* = 8.7 Hz, 1H), 7.45 - 7.36 (m, 4H), 5.14 (d, *J* = 3.6 Hz, 1H), 4.56 (d, *J* = 9.5 Hz, 1H), 4.44 (t, *J* = 8.2 Hz, 1H), 4.39 (dd, *J* = 15.7, 6.4 Hz, 1H), 4.35 (s, 1H), 4.25 (td, *J* = 15.7, 14.4, 5.6 Hz, 1H), 4.03 (s, 3H), 3.95 (s, 2H), 3.66 (dd, *J* = 10.7, 4.0 Hz, 1H), 3.62 - 3.55 (m, 3H), 3.57 - 3.52 (m, 5H), 3.54 - 3.48 (m, 7H), 3.48 (d, *J* = 2.6 Hz, 1H), 3.45 (ddd, *J =* 11.8, 7.4, 4.3 Hz, 4H), 2.43 (s, 2H), 2.05 (dd, *J* = 12.8, 7.7 Hz, 1H), 1.90 (ddd, *J* = 13.0, 8.8, 4.6 Hz, 1H), 0.93 (s, 9H).

### Compound 12

### (2S,4R)-1-{(2S)-3,3-dimethyl-2-[14-({2-methyl-8-[4-(trifluoromethyl)phenyl]-2H,8H-pyrazolo[3,4-b]indol-5-yl}formamido)-3,6,9,12-tetraoxatetradecanamido]butanoyl}-4-hydroxy-N-{[4-(4-methyl-1,3-thiazol-5-yl)phenyl]methyl}pyrrolidine-2-carboxamide (Compound 12)

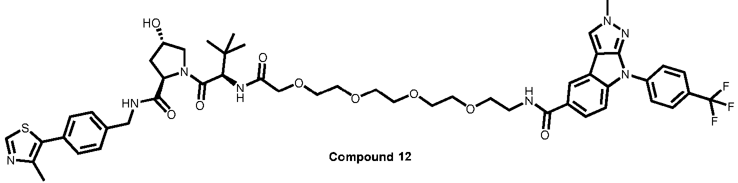

To a solution of (2S,4R)-1-[(2S)-2-amino-3,3-dimethylbutanoyl]-4-hydroxy-N-{[4-(4-methyl-1,3-thiazol-5-yl)phenyl]methyl}pyrrolidine-2-carboxamide hydrochloride (commercially from Sigma-Aldrich; 500 mg; 1.06 mmol) and 14-{[(tert-butoxy)carbonyl]amino}-3,6,9,12-tetraoxatetradecanoic acid (380 mg; 1.07 mmol) in ACN (5 ml) was added *N,N,N',N'*-tetramethylchloroformamidinium hexafluorophosphate (360 mg; 1.22 mmol) and N-methylimidazole (0.35 ml; 4.17 mmol). The resulting mixture was stirred for 1 h at RT. The mixture was purified directly via reverse phase chromatography (Column: C18 silica gel; Mobile phase, A: water (containing 0.1% FA) and B: CH3CN (hold 5% in 5 min, and then 5% to 100% in 30 min); Detector: 220/254 nm) to afford tert-butyl N-(1-{[(2S)-1-[(2S,4R)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]carbamoyl}-2,5,8,11-tetraoxatridecan-13-yl)carbamate (640 mg; 0.84 mmol; 79 %) as a colorless oil. **TEAD ligand 1** (30 mg; 0.08 mmol), (2S,4R)-1-[(2S)-2-(14-amino-3,6,9,12-tetraoxatetradecanamido)-3,3-dimethylbutanoyl]-4-hydroxy-N-{[4-(4-methyl-1,3-thiazol-5-yl)phenyl]-methyl}pyrrolidine-2-carboxamide (55 mg; 0.08 mmol), HATU (38 mg; 0.10 mmol) and 4-Methylmorpholine (37 µL; 0.33 mmol) were dissolved in N,N-Dimethylformamid (5 ml) and stirred overnight at RT. Water and ethyl acetate were added. The layers were separated, the water layer was extracted twice with ethyl acetate and the combined organic layers were washed with water and brine, dried over sodium sulfate, filtered, and evaporated to dryness. The crude residue was purified by prep. RP-HPLC. Target fractions were dried under vacuum to give **compound 12** (37 mg; 0.03 mmol; 39 % based on 87 % purity by LCMS). LC-MS (ESI+, Method Chromolith): *t*_{R} = 1,69 min, m/z 503,0 [M+2H]²⁺. ¹H NMR (700 MHz, DMSO-*d*₆) δ 8.97 (s, 1H), 8.59 (t, *J* = 6.2 Hz, 1H), 8.53 (t, *J* = 5.6 Hz, 1H), 8.34 (d, *J* = 1.9 Hz, 1H), 8.20 (s, 1H), 8.11 - 8.06 (m, 2H), 7.98 - 7.94 (m, 3H), 7.85 (dd, *J* = 8.7, 1.9 Hz, 1H), 7.76 (d, *J* = 8.6 Hz, 1H), 7.42 (dd, *J =* 15.3, 8.8 Hz, 1H), 7.41 - 7.36 (m, 4H), 5.14 (d, *J* = 3.6 Hz, 1H), 4.56 (d, *J* = 9.6 Hz, 1H), 4.44 (t, *J* = 8.2 Hz, 1H), 4.38 (dd, *J* = 15.7, 6.4 Hz, 1H), 4.35 (s, 1H), 4.25 (dd, *J* = 15.7, 5.7 Hz, 1H), 4.03 (s, 4H), 4.03 (q, *J* = 7.1 Hz, 1H), 3.94 (s, 2H), 3.66 (dd, *J* = 10.6, 4.0 Hz, 1H), 3.62 - 3.49 (m, 14H), 3.45 (q, *J* = 6.0 Hz, 2H), 3.02 (s, 1H), 2.43 (s, 3H), 2.08 - 2.03 (m, 1H), 1.99 (s, 2H), 1.90 (ddd, *J* = 12.9, 8.8, 4.5 Hz, 1H), 1.17 (t, *J* = 7.1 Hz, 2H), 0.93 (s, 9H).

### Compound 13

### N-[2-(2,6-dioxopiperidin-3-yl)-1,3-dioxo-2,3-dihydro-1H-isoindol-5-yl]-14-({2-methyl-8-[4-(trifluoromethyl)phenyl]-2H,8H-pyrazolo[3,4-b]indol-5-yl}formamido)-3,6,9,12-tetraoxatetradecanamide (Compound 13)

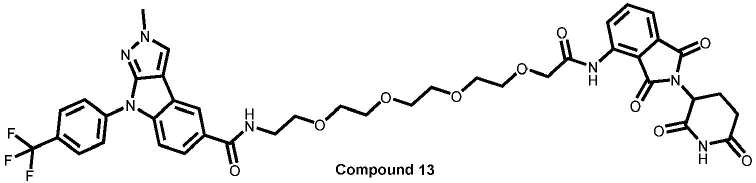

**TEAD ligand 1** (40 mg; 0.11 mmol), 14-amino-N-[2-(2,6-dioxopiperidin-3-yl)-1,3-dioxo-2,3-dihydro-1H-isoindol-5-yl]-3,6,9,12-tetraoxatetradecanamide (prepared analogously as described in WO 2020/152067 A1; 56 mg; 0.11 mmol), HATU (51 mg; 0.13 mmol) and 4-Methylmorpholine (49 mL; 0,.45 mmol) were dissolved in N,N-Dimethylformamid (5 ml) and stirred overnight at RT. The reaction mixture was directly purified by prep. RP-HPLC. Fractions with product were combined and dried under vacuum to provide **compound 13** (14 mg; 0.02 mmol; 14 % based on 94% purity by LCMS). LC-MS (ESI+, Method Chromolith): *t*_{R} = 1,62 min, m/z 848,0 [M+H]⁺. 1H NMR (700 MHz, DMSO-d6) δ 11.11 (s, 1H), 10.29 (s, 1H), 8.51 (t, J = 5.6 Hz, 1H), 8.33 (d, J = 1.9 Hz, 1H), 8.27 (d, J = 1.9 Hz, 1H), 8.19 (s, 1H), 8.11 - 8.06 (m, 2H), 8.03 - 7.94 (m, 3H), 7.94 - 7.82 (m, 2H), 7.80 - 7.74 (m, 1H), 5.12 (dd, J = 12.9, 5.5 Hz, 1H), 4.15 (s, 2H), 4.06 - 4.00 (m, 4H), 3.68 - 3.64 (m, 2H), 3.61 - 3.57 (m, 2H), 3.59 - 3.52 (m, 10H), 3.45 (q, J = 6.0 Hz, 2H), 2.88 (ddd, J = 17.1, 13.9, 5.5 Hz, 1H), 2.59 (d, J = 21.3 Hz, 1H), 2.04 (dtd, J = 11.1, 6.0, 5.5, 2.8 Hz, 1H), 1.99 (s, 1H), 1.17 (t, J = 7.1 Hz, 1H).

### Compound 14

### 4-{[2-(2,6-dioxopiperidin-3-yl)-1-oxo-2,3-dihydro-1H-isoindol-4-yl]amino}-N-(2-{2-[2-({2-methyl-8-[4-(trifluoromethyl)phenyl]-2H,8H-pyrazolo[3,4-b]indol-5-yl}formamido)ethoxy]ethoxy}ethyl)butanamide (Compound 14)

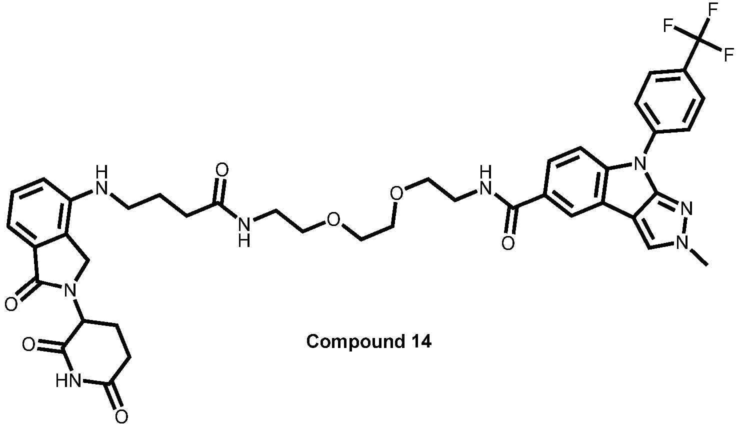

An 8 mL vial with stir bar was charged with **intermediate 1** (60 mg; 0.10 mmol) and **intermediate 3** (34 mg; 0.10 mmol), followed by dissolution in Dimethylformamid (DMF) (1 mL) and addition of 4-methylmorpholine (0.11 ml; 0.99 mmol) and finally HATU (49 mg; 0.13 mmol). The reaction mixture was stirred for 18 h at RT, then diluted with water (2 mL), followed by purification with MS-coupled prep. RP-HPLC. The target fractions were lyophilized to give **compound 14** (23 mg; 0.03 mmol, 28 %) as a white lyophilisate. LC-MS (ESI+, Method Sunfire): *t*_{R} = 2,27 min, m/z 816,6 [M+H]⁺. ¹H NMR (500 MHz, DMSO-*d*₆) δ 10.99 (s, 1H), 8.53 (t, *J* = 5.6 Hz, 1H), 8.34 (d, *J* = 1.8 Hz, 1H), 8.20 (s, 1H), 8.08 (d, *J* = 8.4 Hz, 2H), 7.96 (d, *J* = 8.5 Hz, 2H), 7.90 - 7.82 (m, 2H), 7.77 (d, *J* = 8.7 Hz, 1H), 7.26 (t, *J* = 7.7 Hz, 1H), 6.92 (d, *J* = 7.4 Hz, 1H), 6.72 (d, *J* = 8.1 Hz, 1H), 5.61 (s, 1H), 5.11 (dd, *J =* 13.3, 5.1 Hz, 1H), 4.22 (d, *J* = 17.1 Hz, 1H), 4.12 (d, *J* = 17.1 Hz, 1H), 4.03 (s, 3H), 3.59 - 3.53 (m, 4H), 3.52 (dd, *J* = 5.5, 3.0 Hz, 2H), 3.46 (q, *J* = 5.9 Hz, 2H), 3.41 (t, *J* = 5.9 Hz, 2H), 3.21 (q, *J* = 5.8 Hz, 2H), 3.09 (t, *J* = 7.1 Hz, 2H), 2.92 (ddd, *J* = 17.3, 13.6, 5.4 Hz, 1H), 2.61 (dt, *J* = 17.4, 4.1 Hz, 1H), 2.34 - 2.23 (m, 1H), 2.19 (t, *J =* 7.3 Hz, 2H), 2.03 (ddq, *J* = 10.5, 5.5, 3.1, 2.7 Hz, 1H), 1.79 (p, *J* = 7.2 Hz, 2H).

### Compound 15

### 7-{[2-(2,6-dioxopiperidin-3-yl)-1-oxo-2,3-dihydro-1H-isoindol-4-yl]amino}-N-(2-{2-[2-({2-methyl-8-[4-(trifluoromethyl)phenyl]-2H,8H-pyrazolo[3,4-b]indol-5-yl}formamido)ethoxy]ethoxy}ethyl)heptanamide (Compound 15)

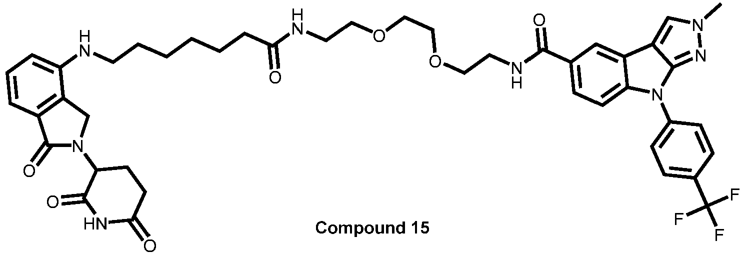

**Intermediate 4** (73 mg; 0.19 mmol) and **intermediate 1** (114 mg; 0.19 mmol) were dissolved in DMF (2 mL) in a 8 mL vial with stir bar followed by addition of 4-methylmorpholine (0.1 mL; 0.94 mmol) and finally HATU (86 mg; 0.23 mmol). The reaction mixture was stirred for 24 h at RT and then directly injected for purification by prep RP-HPLC-MS. The target fractions were lyophilized to give **compound 15** (65 mg; 0.08 mmol, 47 %) as a white solid. LC-MS (ESI+, Method Sunfire): *t*_{R} = 2,47 min, m/z 872,6 [M+H]⁺. ¹H NMR (700 MHz, DMSO-*d*₆) δ 11.09 (s, 1H), 8.53 (q, *J* = 5.8, 5.1 Hz, 1H), 8.34 (d, *J* = 1.8 Hz, 1H), 8.20 (s, 1H), 8.08 (d, *J* = 8.4 Hz, 2H), 7.96 (d, *J* = 8.5 Hz, 2H), 7.85 (dd, *J* = 8.7, 1.8 Hz, 1H), 7.81 (t, *J =* 5.7 Hz, 1H), 7.77 (d, *J* = 8.6 Hz, 1H), 7.55 (dd, *J* = 8.5, 7.1 Hz, 1H), 7.05 (d, *J =* 8.6 Hz, 1H), 7.00 (d, *J =* 7.0 Hz, 1H), 6.49 (d, *J* = 6.2 Hz, 1H), 5.05 (dd, *J* = 12.8, 5.5 Hz, 1H), 4.03 (s, 2H), 3.59 - 3.50 (m, 5H), 3.46 (q, *J* = 6.0 Hz, 2H), 3.40 (t, *J* = 5.9 Hz, 2H), 3.24 (q, *J* = 6.6 Hz, 2H), 3.18 (q, *J* = 5.8 Hz, 2H), 2.88 (ddd, *J* = 17.1, 13.9, 5.4 Hz, 1H), 2.62 - 2.53 (m, 1H), 2.09 - 1.99 (m, 3H), 1.50 (dp, *J* = 40.8, 7.3 Hz, 4H), 1.36 - 1.21 (m, 4H).

### Compound 16

### N-(14-{[2-(2,6-dioxopiperidin-3-yl)-1,3-dioxo-2,3-dihydro-1H-isoindol-4-yl]oxy}-3,6,9,12-tetraoxatetradecan-1-yl)-2-methyl-8-[4-(trifluoromethyl)phenyl]-2H,8H-pyrazolo[3,4-b]indole-5-carboxamide (Compound 16)

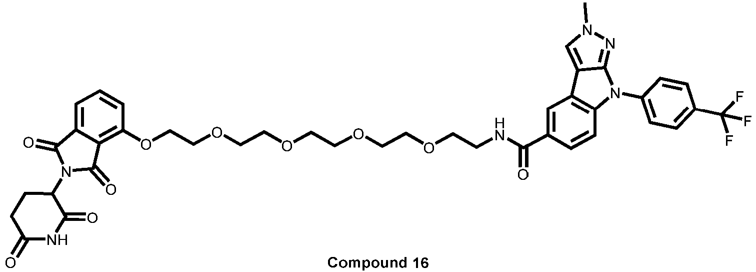

Triphenylphosphine (56 mg; 0.22 mmol) was placed in a heat gun dried 10 mL Schlenk tube with stir bar under N₂, dissolved in dry THF (1 mL) and cooled to 0°C, followed by addition of Diisopropyl azodicarboxylate (44 mg; 0.22 mmol). The mixture was stirred at 0°C until formation of a milky precipitate occurred (around 20 min). Meanwhile, a solution of **intermediate 8** (62 mg; 0.11 mmol) and **intermediate 7** (81 mg; 0.22 mmol) in dry THF (3 mL) was prepared. This solution was added to the milky precipitate by syringe at 0 °C. The reaction mixture was stirred for 1 h at 0°C, the ice bath was removed and the reaction mixture was allowed to stir at RT for 2 h. The volatiles were removed by rotary evaporation and the residue re-dissolved in DMF (2 mL). The resulting solution was used for purifcation by prep RP-HPLC-MS. The target fractions (last peak) were lyophilized to provide **Boc-16** (49 mg, 0.05 mmol, 49 %) as an amber oil. This intermediate was dissolved in DCM (5 mL) followed by addition of TFA (5 mL) at RT. The reaction mixture was stirred for 45 min at RT. The volatiles were removed by rotary evaporation and the crude mixture re-dissolved in DMF (2 mL). The resultant solution was used for injection into a prep. RP-HPLC-MS system. The target fractions were lyophilized to provide **compound 16** (32 mg, 0.04 mmol, 73 %) as a white solid. LC-MS (ESI+, Method Sunfire): *t*_{R} = 2,40 min, m/z 834,6 [M+H]⁺. ¹H NMR (700 MHz, DMSO-*d*₆) δ 11.10 (s, 1H), 8.52 (t, *J* = 5.6 Hz, 1H), 8.34 (d, *J* = 1.9 Hz, 1H), 8.20 (s, 1H), 8.08 (d, *J* = 8.5 Hz, 2H), 7.96 (d, *J* = 8.6 Hz, 2H), 7.85 (dd, *J* = 8.6, 1.9 Hz, 1H), 7.80 - 7.75 (m, 2H), 7.49 (d, *J* = 8.6 Hz, 1H), 7.44 (d, *J* = 7.1 Hz, 1H), 5.08 (dd, *J* = 12.9, 5.4 Hz, 1H), 4.32 - 4.26 (m, 2H), 4.03 (s, 3H), 3.79 - 3.74 (m, 2H), 3.61 (dd, *J* = 5.9, 3.8 Hz, 2H), 3.58 - 3.48 (m, 12H), 3.45 (q, *J* = 5.9 Hz, 2H), 2.88 (ddd, *J* = 17.1, 14.0, 5.4 Hz, 1H), 2.58 (dt, *J* = 17.2, 3.4 Hz, 1H), 2.01 (dtd, *J* = 13.1, 5.4, 2.3 Hz, 1H).

### Compound 17

### 3-[2-(2-{[2-(2,6-dioxopiperidin-3-yl)-1,3-dioxo-2,3-dihydro-1H-isoindol-4-yl]carbamoyl}ethoxy)ethoxy]-N-[2-({2-methyl-8-[4-(trifluoromethyl)phenyl]-2H,8H-pyrazolo[3,4-b]indol-5-yl}formamido)ethyl]propenamide (Compound 17)

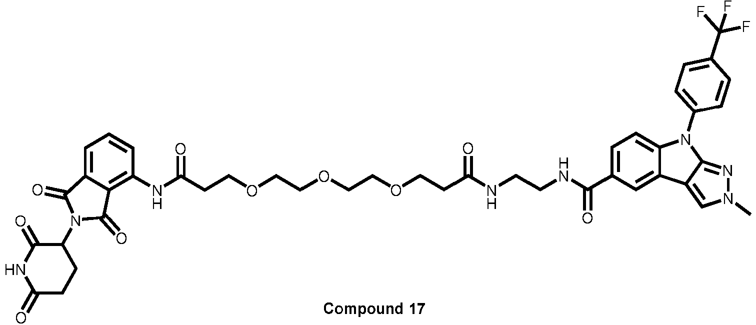

**Intermediate 9** (40 mg; 0.10 mmol) was dissolved in DMF. The reaction was set up with a SynpleChem machine, using cartridge P043 (available from SynpleChem AG; see also T. Jiang, et al., *Chem.* Sci., 2021, 12, 6977-6982). The reaction mixture was evaporated to dryness and the crude residue purified by flash column chromatography (4 g SiO2 cartridge, 0 - 80% MeOH in DCM). The target fractions were freed from solvent to give **compound 17** (29 mg; 0.03 mmol; 32 % based on 99 % purity by LCMS). LC-MS (ESI+, Method Chromolith): *t*_{R} = 1,60 min, m/z 889,00 [M+H]⁺. ¹H NMR (500 MHz, DMSO-*d*₆) δ 11.13 (s, 1H), 9.84 (s, 1H), 8.54 - 8.46 (m, 2H), 8.32 (d, *J* = 1.8 Hz, 1H), 8.19 (s, 1H), 8.08 (d, *J* = 8.4 Hz, 2H), 7.96 (d, *J* = 8.8 Hz, 3H), 7.86 - 7.74 (m, 3H), 7.58 (dd, *J =* 7.3, 0.8 Hz, 1H), 5.13 (dd, *J* = 12.9, 5.4 Hz, 1H), 4.03 (q, *J* = 7.1 Hz, 1H), 4.03 (s, 3H), 3.71 (t, *J* = 6.0 Hz, 2H), 3.61 - 3.47 (m, 6H), 3.47 - 3.41 (m, 3H), 3.31 - 3.23 (m, 3H), 2.89 (ddd, *J* = 17.1, 13.8, 5.4 Hz, 1H), 2.68 (t, *J* = 6.0 Hz, 2H), 2.64 - 2.52 (m, 1H), 2.31 (t, *J* = 6.5 Hz, 2H), 1.99 (s, 1H), 1.18 (t, *J* = 7.1 Hz, 1H).

### Compound 18

### 3-[2-(2-{[2-(2,6-dioxopiperidin-3-yl)-1,3-dioxo-2,3-dihydro-1H-isoindol-4-yl]carbamoyl}ethoxy)ethoxy]-N-[2-({2-methyl-8-[4-(trifluoromethyl)phenyl]-2H,8H-pyrazolo[3,4-b]indol-5-yl}formamido)ethyl]propanamide (Compound 18)

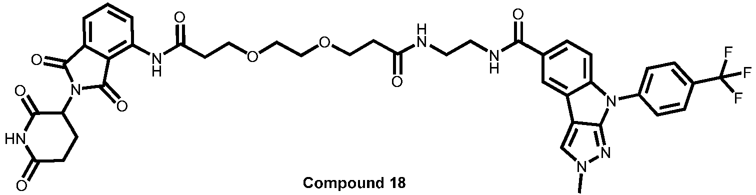

**Intermediate 9** (40 mg; 0.10 mmol) was dissolved in DMF. The reaction was set up with a Synple Chem machine, using cartridge P042 (available from SynpleChem AG; see also T. Jiang, et al., Chem. Sci., 2021, 12, 6977-6982). The reaction mixture was evaporated to dryness. The crude residue was purified by flash column chromatography (4 g SiO₂ cartridge, 0 - 70% MeOH in DCM). Fractions with product were combined and evaporated to dryness to give **compound 18** (40 mg; 0.04 mmol; 44 % based on 94 % purity by LCMS). LC-MS (ESI+, Method Chromolith): *t*_{R} = 1,59 min, m/z 845,00 [M+H]⁺. ¹H NMR (700 MHz, DMSO-*d*₆) δ 11.14 (s, 1H), 9.84 (s, 1H), 8.52 (d, *J* = 8.3 Hz, 1H), 8.49 (t, *J* = 5.6 Hz, 1H), 8.32 (d, *J* = 1.9 Hz, 1H), 8.19 (s, 1H), 8.08 (d, *J* = 8.3 Hz, 2H), 7.97 (t, *J* = 8.2 Hz, 3H), 7.83 (dd, *J* = 8.7, 1.9 Hz, 1H), 7.80 (dd, *J* = 8.4, 7.3 Hz, 1H), 7.76 (d, *J = 8.6* Hz, 1H), 7.58 (dd, *J* = 7.4, 0.7 Hz, 1H), 5.14 (dd, *J* = 13.0, 5.5 Hz, 1H), 4.03 (s, 3H), 3.70 (t, *J* = 6.0 Hz, 2H), 3.60 (t, *J* = 6.5 Hz, 2H), 3.54 (dd, *J* = 6.3, 3.9 Hz, 2H), 3.52 - 3.48 (m, 2H), 3.47 - 3.41 (m, 1H), 3.34 (s, 1H), 3.30 (s, 1H), 3.25 (q, *J* = 6.4 Hz, 2H), 2.89 (ddd, *J* = 17.3, 13.8, 5.5 Hz, 1H), 2.67 (t, *J* = 6.0 Hz, 2H), 2.64 - 2.58 (m, 1H), 2.54 (dd, *J* = 13.1, 4.5 Hz, 1H), 2.31 (t, *J* = 6.5 Hz, 2H), 2.06 (dtd, *J* = 12.9, 5.3, 2.3 Hz, 1H), 1.06 (t, *J* = 7.0 Hz, 1H).

### Compound 19

### N-(3-{6-[4-(3-{[2-(2,6-dioxopiperidin-3-yl)-1-oxo-2,3-dihydro-1H-isoindol-5-yl]carbamoyl}propyl)piperazin-1-yl]pyridin-3-yl}prop-2-yn-1-yl)-2-methyl-8-[4-(trifluoromethyl)phenyl]-2H,8H-pyrazolo[3,4-b]indole-5-carboxamide, formate salt (Compound 19)

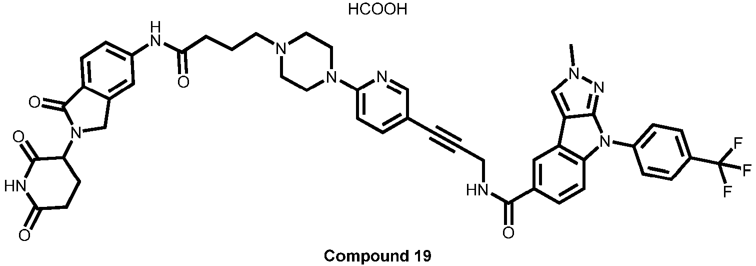

14-{4-[5-(3-aminoprop-1-yn-1-yl)pyridin-2-yl]piperazin-1-yl}-N-[2-(2,6-dioxopiperidin-3-yl)-1-oxo-2,3-dihydro-1H-isoindol-5-yl]butanamide hydrochloride (commercially available from Sigma-Aldrich, 53 mg; 0.09 mmol) and **TEAD ligand 1** (33 mg; 0.09 mmol) were suspended in Dimethylformamid (DMF) (1 ml), followed by addition of 4-methylmorpholine (0.05 ml). The mixture was vortexed until all components were dissolved. HATU (42 mg; 0.11 mmol) was added and the reaction mixture was stirred for 18 h at RT. The mixture was directly injected into a prep RP-HPLC-MS system and the target fractions lyophilised to give **compound 19** (40 mg; 0.04 mmol, 45 %) as an off white solid. LC-MS (ESI+, Method Sunfire): *t*_{R} = 1,87 min, m/z 443,00 [M+2H]²⁺. ¹H NMR (500 MHz, DMSO-*d*₆) δ 10.97 (s, 1H), 10.22 (s, 1H), 9.01 (t, *J =* 5.5 Hz, 1H), 8.40 (d, *J* = 1.8 Hz, 1H), 8.23 - 8.16 (m, 2H), 8.14 (s, 1H), 8.09 (d, *J* = 8.4 Hz, 2H), 8.01 - 7.94 (m, 3H), 7.90 (dd, *J* = 8.8, 1.9 Hz, 1H), 7.79 (d, *J* = 8.7 Hz, 1H), 7.65 (d, *J* = 8.3 Hz, 1H), 7.59 (dd, *J* = 8.4, 1.7 Hz, 1H), 7.55 (dd, *J* = 8.9, 2.4 Hz, 1H), 6.80 (d, *J* = 9.0 Hz, 1H), 5.08 (dd, *J* = 13.3, 5.1 Hz, 1H), 4.41 (d, *J* = 17.2 Hz, 1H), 4.36 (d, *J* = 5.4 Hz, 2H), 4.28 (d, *J* = 17.2 Hz, 1H), 4.04 (s, 3H), 3.52 (t, *J* = 5.1 Hz, 4H), 2.91 (ddd, *J* = 17.3, 13.6, 5.4 Hz, 1H), 2.60 (dt, *J* = 15.1, 2.8 Hz, 1H), 2.48 (d, *J* = 5.1 Hz, 4H), 2.43 (d, *J* = 7.3 Hz, 2H), 2.42 - 2.31 (m, 3H), 1.99 (dtd, *J =* 12.7, 5.3, 2.3 Hz, 1H), 1.81 (p, *J* = 7.2 Hz, 2H).

### Compound 20

### (2S,4R)-1-[(2S)-3,3-dimethyl-2-[10-({2-methyl-8-[4-(trifluoromethyl)phenyl]-2H,8H-pyrazolo[3,4-b]indol-5-yl}formamido)decanamido]butanoyl]-4-hydroxy-N-{[4-(4-methyl-1,3-thiazol-5-yl)phenyl]methyl}pyrrolidine-2-carboxamide (Compound 20)

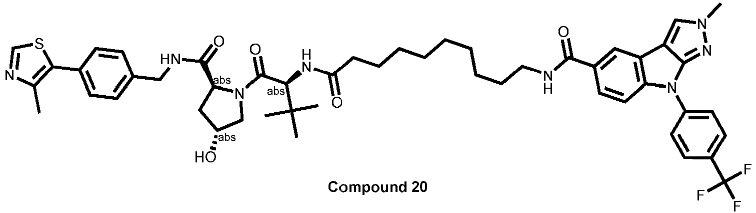

((2S,4R)-1-((S)-2-(10-aminodecanamido)-3,3-dimethylbutanoyl)-4-hydroxy-N-(4-(4-methylthiazol-5-yl)benzyl)pyrrolidine-2-carboxam ide hydrochloride (commercially available from Sigma-Aldrich, 48 mg; 0.08 mmol) and **TEAD ligand 1** (27 mg; 0.08 mmol) were dissolved in Dimethylformamid (DMF) (1 mL), followed by addition of 4-methylmorpholine (0.04 ml) and finally HATU (34 mg; 0.09 mmol). The reaction mixture was stirred at RT overnight. The mixture was directly purified with prep RP-HPLC-MS, and the target fraction lyophilised to provide **compound 20** (44 mg; 0.05 mmol, 62 %) as a microcrystalline powder. LC-MS (ESI+, Method Sunfire): *t*_{R} = 2,68 min, m/z 471,22 [M+2H]²⁺. ¹H NMR (500 MHz, DMSO-*d*₆) δ 8.98 (s, 1H), 8.55 (t, *J* = 6.1 Hz, 1H), 8.45 (t, *J* = 5.7 Hz, 1H), 8.33 (d, *J* = 1.8 Hz, 1H), 8.20 (s, 1H), 8.08 (d, *J* = 8.4 Hz, 2H), 7.96 (d, *J* = 8.5 Hz, 2H), 7.87 - 7.80 (m, 2H), 7.76 (d, *J =* 8.7 Hz, 1H), 7.45 - 7.35 (m, 4H), 4.54 (d, J = 9.4 Hz, 1H), 4.43 (ddd, *J =* 9.9, 6.7, 3.2 Hz, 2H), 4.35 (tt, *J* = 4.3, 2.5 Hz, 1H), 4.22 (dd, *J* = 15.8, 5.5 Hz, 1H), 4.03 (s, 3H), 3.71 - 3.61 (m, 2H), 3.28 (q, *J* = 6.7 Hz, 2H), 2.44 (s, 3H), 2.26 (dt, *J* = 14.8, 7.6 Hz, 1H), 2.11 (ddd, *J* = 14.2, 8.1, 6.3 Hz, 1H), 2.07 - 1.99 (m, 1H), 1.91 (ddd, *J* = 12.9, 8.5, 4.6 Hz, 1H), 1.50 (ddt, *J* = 38.5, 13.4, 6.9 Hz, 4H), 1.33 (t, *J* = 5.0 Hz, 1H), 1.27 (h, *J* = 9.2, 7.0 Hz, 10H), 0.93 (s, 9H).

### Compound 21

### 10-{[2-(2,6-dioxopiperidin-3-yl)-1,3-dioxo-2,3-dihydro-1H-isoindol-4-yl]amino}-N-(2-{2-[2-({2-methyl-8-[4-(trifluoromethyl)phenyl]-2H,8H-pyrazolo[3,4-b]indol-5-yl}formamido)ethoxy]ethoxy}ethyl)decanamide (Compound 21)

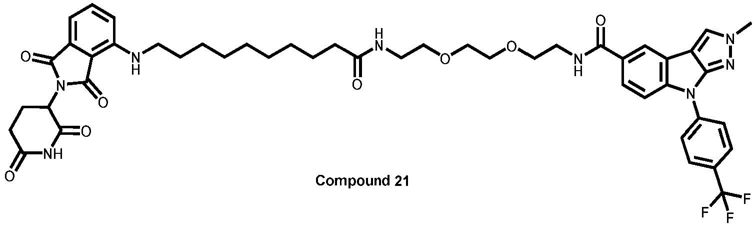

In a 8 mL vial with stir bar was placed **intermediate 1** (50 mg; 0.08 mmol) and 10-((2-(2,6-Dioxopiperidin-3-yl)-1,3-dioxoisoindolin-4-yl)amino)decanoic (commercially available from Sigma-Aldrich, 37 mg; 0.08 mmol), dissolved in N,N-dimethylformamide (1 ml), followed by addition of 4-methylmorpholine (01 mL) and finally HATU (41 mg; 0.11 mmol). The reaction mixture was stirred for 18 h at RT. The mixture was used for purification by prep. RP-HPLC to **compound 21** (33 mg; 0.04 mmol, 44 %) as a yellow residue after lyophilization of the target fractions. LC-MS (ESI+, Method Sunfire): *t*_{R} = 2,63 min, m/z 914,6 [M+H]⁺. ¹H NMR (500 MHz, DMSO-*d*₆) δ 11.08 (s, 1H), 8.52 (t, *J* = 5.6 Hz, 1H), 8.34 (d, *J =* 1.8 Hz, 1H), 8.19 (s, 1H), 8.08 (d, *J* = 8.3 Hz, 2H), 7.96 (d, *J* = 8.4 Hz, 2H), 7.85 (dd, *J* = 8.7, 1.8 Hz, 1H), 7.77 (d, *J* = 8.6 Hz, 2H), 7.56 (dd, *J* = 8.6, 7.1 Hz, 1H), 7.05 (d, *J* = 8.6 Hz, 1H), 7.00 (d, *J* = 7.0 Hz, 1H), 6.48 (t, *J =* 5.9 Hz, 1H), 5.04 (dd, *J* = 12.8, 5.4 Hz, 1H), 4.03 (s, 3H), 3.60 - 3.54 (m, 4H), 3.52 (dd, *J* = 6.1, 3.5 Hz, 2H), 3.46 (q, *J* = 6.0 Hz, 2H), 3.40 (t, *J* = 5.9 Hz, 2H), 3.24 (q, *J* = 6.6 Hz, 2H), 3.18 (q, *J* = 5.8 Hz, 2H), 2.88 (ddd, *J =* 17.0, 13.8, 5.4 Hz, 1H), 2.63 - 2.52 (m, 2H), 2.02 (t, *J* = 7.5 Hz, 3H), 1.58 - 1.45 (m, 2H), 1.43 (q, *J* = 7.2 Hz, 2H), 1.34 - 1.21 (m, 2H), 1.20 (s, 5H).

### Compound 22

### N-[2-(2,6-dioxopiperidin-3-yl)-1 ,3-dioxo-2,3-dihydro-1H-isoindol-4-yl]-N'-[(2R)-2-{[8-(4-fluorophenyl)-2-methyl-2H,8H-pyrazolo[3,4-b]indol-5-yl]formamido}propyl]-3,6,9,12,15-pentaoxaheptadecanediamide (Compound 22)

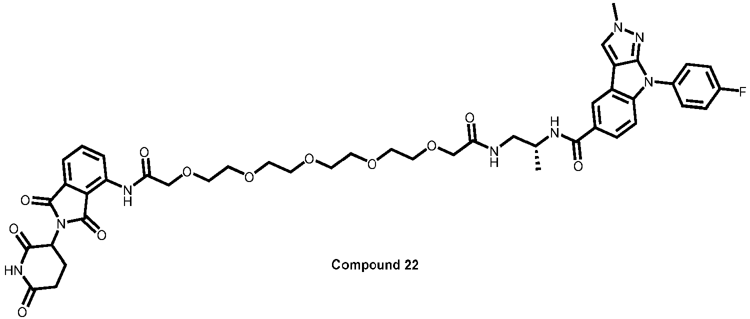

To **intermediate 10** (40 mg; 0.11 mmol) in DMF (3 ml) was added 1-{[2-(2,6-dioxopiperidin-3-yl)-1,3-dioxo-2,3-dihydro-1H-isoindol-4-yl]carbamoyl}-2,5,8,11,14-pentaoxahexadecan-16-oic acid (accessible analogously to compound 24 in ACS Chem. Biol. 2017, 12, 2570-2578 (Supporting Information); 62 mg; 0.11 mmol), EDC hydrochloride (42 mg; 0.22 mmol), HOBT hydrate (17 mg; 0.11 mmol) and 4-Methylmorpholine (0.06 mL; 0.55 mmol). The reaction was stirred overnight at RT and then evaporated to dryness. The crude residue was purified by prep. RP-HPLC. Fractions with product were combined and dried under vacuum to give **compound 22** (15 mg; 0.02 mmol; 15 %). LC-MS (ESI+, Method Chromolith): *t*_{R} = 1,52 min, m/z 457,00 [M+2H]²⁺. ¹H NMR (500 MHz, DMSO-*d*₆) δ 11.13 (s, 1H), 10.32 (s, 1H), 8.70 (d, *J* = 8.4 Hz, 1H), 8.29 (d, *J* = 1.8 Hz, 1H), 8.19 (d, *J* = 7.9 Hz, 1H), 8.15 (s, 1H), 7.84 (dd, *J* = 8.5, 7.3 Hz, 1H), 7.84 - 7.75 (m, 4H), 7.64 - 7.59 (m, 1H), 7.52 (d, *J* = 8.6 Hz, 1H), 7.48 - 7.39 (m, 2H), 5.15 (dd, *J* = 12.9, 5.4 Hz, 1H), 4.22 - 4.12 (m, 3H), 4.00 (s, 3H), 3.87 (s, 2H), 3.73 (dd, *J* = 5.8, 3.5 Hz, 2H), 3.68 - 3.61 (m, 2H), 3.56 - 3.43 (m, 7H), 3.43 (s, 4H), 3.28 (s, 1H), 3.23 (dd, *J* = 13.4, 6.7 Hz, 1H), 2.89 (ddd, J = 17.0, 13.9, 5.4 Hz, 1H), 2.65 - 2.52 (m, 2H), 2.13 - 2.03 (m, 1H), 1.15 (d, *J* = 6.7 Hz, 3H).

### Compound 23

### N-[2-(2,6-dioxopiperidin-3-yl)-1,3-dioxo-2,3-dihydro-1H-isoindol-4-yl]-N'-[(2R)-2-{[2-methyl-8-(4-methylphenyl)-2H,8H-pyrazolo[3,4-b]indol-5-yl]formamido}propyl]-3,6,9,12,15-pentaoxaheptadecanediamide (Compound 23)

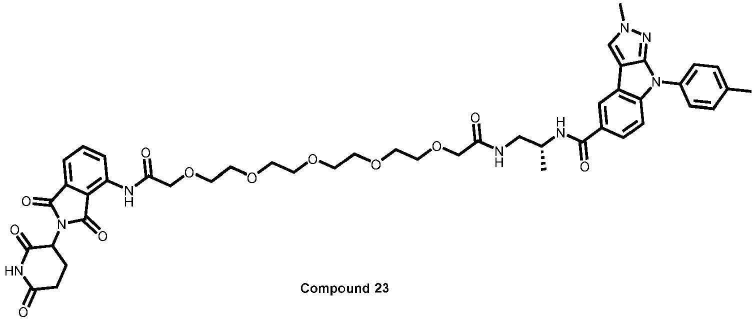

To **intermediate 11** (40 mg; 0.11 mmol) in DMF (3 ml) was added 1-{[2-(2,6-dioxopiperidin-3-yl)-1,3-dioxo-2,3-dihydro-1H-isoindol-4-yl]carbamoyl}-2,5,8,11,14-pentaoxahexadecan-16-oic acid (accessible analogously to compound 24 in ACS Chem. Biol. 2017, 12, 2570-2578 (Supporting Information); 63 mg; 0.11 mmol), EDC hydrochloride (42 mg; 0,22 mmol), HOBt hydrate (17 mg; 0.11 mmol)) and 4-Methylmorpholine (0.06 mL; 0.55 mmol). The reaction was stirred overnight at RT. The mixture was evaporated to dryness. The crude residue was purified by prep. RP-HPLC. Fractions with product were combined and dried under vacuum to give **compound 23** (25 mg; 0.03 mmol; 25 %). LC-MS (ESI+, Method Chromolith): *t*_{R} = 1,52 min, m/z 455,00 [M+2H]²⁺. ¹H NMR (500 MHz, DMSO-*d*₆) δ 11.13 (s, 1H), 10.32 (s, 1H), 8.70 (d, *J* = 8.4 Hz, 1H), 8.28 (d, *J* = 1.8 Hz, 1H), 8.18 (d, *J* = 7.9 Hz, 1H), 8.13 (s, 1H), 7.88 - 7.75 (m, 3H), 7.62 (td, *J* = 6.9, 1.4 Hz, 3H), 7.52 (d, *J* = 8.6 Hz, 1H), 7.43 - 7.37 (m, 2H), 5.15 (dd, *J* = 12.9, 5.4 Hz, 1H), 4.21 - 4.12 (m, 3H), 4.00 (s, 3H), 3.87 (s, 2H), 3.72 (dd, *J* = 5.8, 3.5 Hz, 2H), 3.63 (dd, *J* = 5.8, 3.6 Hz, 2H), 3.56 - 3.42 (m, 6H), 3.43 (s, 3H), 3.22 (dd, *J* = 13.4, 6.7 Hz, 1H), 2.89 (ddd, *J* = 17.0, 13.8, 5.4 Hz, 1H), 2.62 (d, *J* = 3.2 Hz, 1H), 2.39 (s, 3H), 1.14 (d, *J* = 6.6 Hz, 3H).

### Compound 24

### N'-[(2S)-1-[(2S,4R)-4-hydroxy-2-{[(1S)-1-[4-(4-methyl-1,3-thiazol-5-yl)phenyl]ethyl]carbamoyl}pyrrolidin-1-yl]-3,3-dimethyl-1-oxobutan-2-yl]-N-(2-{2-[2-({2-methyl-8-[4-(trifluoromethyl)phenyl]-2H,8H-pyrazolo[3,4-b]indol-5-yl}formamido)ethoxy]ethoxy}ethyl)heptanediamide (Compound 24)

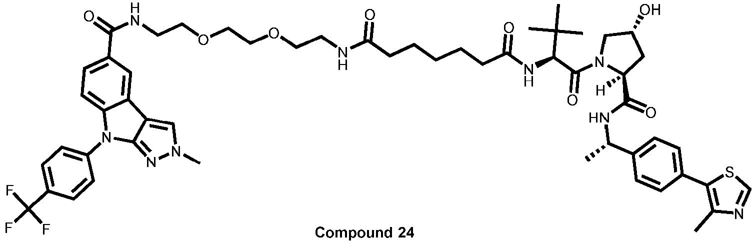

**Intermediate 6** (39 mg; 0.06 mmol), **intermediate 1** (40 mg; 0.06 mmol) and 4-methylmorpholine (0.03 mL) were dissolved in Dimethylformamid (DMF) (1 mL), followed by addition of HATU (30 mg; 0.08 mmol) in a 100 mL RBF with stir bar. The reaction mixture was stirred at RT for 1 h, upon which the crude mixture was injected into a prep. RP-HPLC-MS system to give **compound 24** (32 mg, 0.03 mmol, 46 %). LC-MS (ESI+, Method Sunfire): *t*_{R} = 2,43 min, m/z 529,40 [M+2H]²⁺. ¹H NMR (500 MHz, DMSO-*d*₆) δ 8.99 (s, 1H), 8.60 (t, *J* = 5.6 Hz, 1H), 8.43 (d, *J* = 7.8 Hz, 1H), 8.35 (d, *J* = 1.7 Hz, 1H), 8.22 (s, 1H), 8.09 (d, *J* = 8.4 Hz, 2H), 7.98 (d, *J* = 8.7 Hz, 2H), 7.90 - 7.81 (m, 3H), 7.78 (d, *J* = 8.7 Hz, 1H), 7.46 - 7.40 (m, 2H), 7.40 - 7.30 (m, 2H), 5.15 (s, 1H), 4.91 (p, *J* = 7.1 Hz, 1H), 4.51 (d, *J* = 9.4 Hz, 1H), 4.41 (t, *J* = 8.1 Hz, 1H), 4.28 (q, *J =* 3.3 Hz, 1H), 4.04 (s, 3H), 3.62 - 3.50 (m, 8H), 3.17 (q, *J* = 5.9 Hz, 2H), 2.45 (s, 3H), 2.22 (dt, *J* = 14.8, 7.7 Hz, 1H), 2.14 - 2.05 (m, 1H), 2.02 (q, *J* = 8.3, 7.8 Hz, 3H), 1.77 (ddd, *J* = 13.0, 8.7, 4.6 Hz, 1H), 1.51 - 1.38 (m, *J =* 6.7, 5.9 Hz, 4H), 1.36 (d, *J* = 7.0 Hz, 3H), 1.19 (dq, *J* = 14.5, 8.7, 7.0 Hz, 2H), 0.92 (s, 9H).

### Compound 25

### (3R,5S)-1-[(2S)-3,3-dimethyl-2-{6-[(2-{2-[2-({2-methyl-8-[4-(trifluoromethyl)phenyl]-2H,8H-pyrazolo[3,4-b]indol-5-yl}formam ido)ethoxy]ethoxy}ethyl)carbamoyl]hexanam ido}butanoyl]-5-{[(1S)-1-[4-(4-methyl-1,3-thiazol-5-yl)phenyl]ethyl]carbamoyl}pyrrolidin-3-yl acetate (Compound 25)

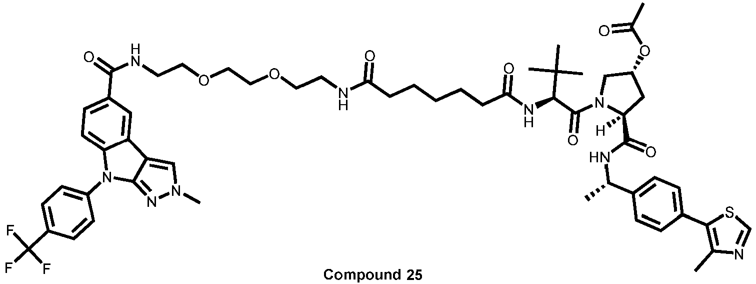

**Intermediate 6** (39 mg; 0.06 mmol), **intermediate 1** (40 mg; 0.06 mmol), 4-methylmorpholine (0.03 mL; 0.26 mmol) were dissolved in Dimethylformamid (DMF) (1 mL), followed by addition of HATU (30 mg; 0.08 mmol) in a 100 mL RBF with stir bar. The reaction mixture was stirred at RT for 1 h, followed by addition of acetic anhydride (12 µL; 0.13 mmol) at RT for 15 min. No reaction was observed by LC-MS analysis. Next, DMAP (7 mg, 0.06 mmol) was added, followed by further AcCl (10 µL, 0.14 mmol). The reaction mixture was stirred for 6 h, and subsequent LC-MS analysis indicated product formation. The reaction mixture was diluted with water and DMSO (1 mL each) and directly injected into a prep. RP-HPLC-MS system to give **compound 25** (12 mg, 0.01 mmol, 17 %) as a white film after lyophilization. LC-MS (ESI+, Method Chromolith): *t*_{R} = 1,78 min, m/z 1100,50 [M+H]⁺. ¹H NMR (500 MHz, DMSO-*d*₆) δ 8.99 (s, 1H), 8.60 (t, *J = 5.6* Hz, 1H), 8.46 (d, *J* = 7.6 Hz, 1H), 8.35 (d, *J* = 1.8 Hz, 1H), 8.22 (s, 1H), 8.09 (d, *J* = 8.4 Hz, 2H), 7.98 (d, *J* = 8.4 Hz, 2H), 7.90 - 7.83 (m, 3H), 7.78 (d, *J* = 8.7 Hz, 1H), 7.46 - 7.40 (m, 2H), 7.39 - 7.31 (m, 2H), 5.17 (s, 1H), 4.90 (p, *J* = 7.2 Hz, 1H), 4.44 (dd, *J* = 9.1, 7.7 Hz, 1H), 4.33 (d, *J =* 8.7 Hz, 1H), 4.03 (s, 3H), 3.95 (d, *J* = 11.7 Hz, 1H), 3.72 (dd, *J* = 11.8, 4.1 Hz, 1H), 3.59 - 3.50 (m, 6H), 3.46 (s, 1H), 3.17 (q, *J* = 5.9 Hz, 2H), 2.45 (s, 3H), 2.21 (tt, *J* = 14.8, 7.5 Hz, 2H), 2.14 - 1.92 (m, 6H), 1.44 (h, *J* = 8.0 Hz, 4H), 1.36 (d, *J* = 7.1 Hz, 3H), 1.25 - 1.12 (m, 3H), 0.94 (s, 9H).

### Compound 26

### (2S,4R)-1-[(2S)-3,3-dimethyl-2-[4-(4-{5-[3-({2-methyl-8-[4-(trifluoromethyl)phenyl]-2H,8H-pyrazolo[3,4-b]indol-5-yl}formamido)prop-1-yn-1-yl]pyridin-2-yl}piperazin-1-yl)butanamido]butanoyl]-4-hydroxy-N-{[4-(4-methyl-1,3-thiazol-5-yl)phenyl]methyl}pyrrolidine-2-carboxamide, bis-TFA salt (Compound 26)

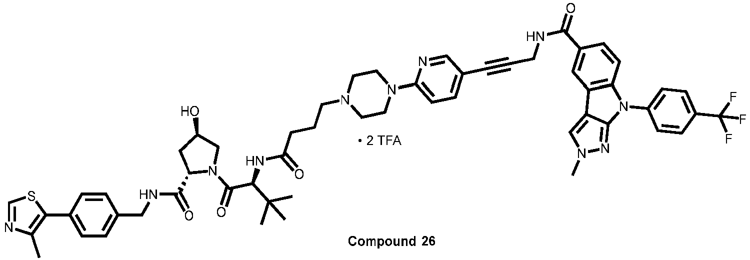

In a 8 mL vial with stir bar was placed (2S,4R)-1-[(2S)-2-(4-{4-[5-(3-aminoprop-1-yn-1-yl)pyridin-2-yl]piperazin-1-yl}butanamido)-3,3-dimethylbutanoyl]-4-hydroxy-N-{[4-(4-methyl-1,3-thiazol-5-yl)phenyl]-methyl}pyrrolidine-2-carboxamide hydrochloride (commercially available from Sigma-Aldrich, 52 mg; 0.07 mmol) and **TEAD ligand 1** (25 mg; 0.07 mmol), dissolved in Dimethylformamid (DMF) (1 mL), followed by addition of 4-methylmorpholine (0.03 mL; 0.28 mmol) and then HATU (32 mg; 0.08 mmol) to start the reaction. The reaction mixture was stirred for 18 h at RT, filtered through a 0.2 µm membrane filter, and the filtrate was directly injected into a prep. RP-HPLC-MS system using a TFA modifier. The target fractions were lyophilized to **compound 26** (43 mg; 0.03 mmol, 48 % based on 99 % purity by LCMS analysis) as a yellowish solid. LC-MS (ESI+, Method Chromolith): *t*_{R} = 1,56 min, m/z 1055,50 [M+H]⁺. ¹H NMR (500 MHz, DMSO-*d*₆) δ 9.84 (s, 1H), 9.10 (t, *J* = 5.5 Hz, 1H), 9.02 (s, 1H), 8.64 (t, *J* = 6.1 Hz, 1H), 8.41 (d, *J* = 1.8 Hz, 1H), 8.28 - 8.22 (m, 2H), 8.17 (d, *J* = 9.3 Hz, 1H), 8.10 (d, *J* = 8.4 Hz, 2H), 7.98 (d, *J* = 8.6 Hz, 2H), 7.91 (dd, *J* = 8.8, 1.9 Hz, 1H), 7.81 (d, *J* = 8.7 Hz, 1H), 7.68 (dd, *J* = 8.9, 2.4 Hz, 1H), 7.43 (d, *J =* 8.2 Hz, 2H), 7.39 (d, *J =* 8.2 Hz, 2H), 6.96 (d, *J =* 8.9 Hz, 1H), 4.57 (d, *J* = 9.3 Hz, 1H), 4.45 (ddd, J = 18.6, 9.5, 6.0 Hz, 4H), 4.37 (s, 1H), 4.22 (dd, *J* = 15.9, 5.4 Hz, 1H), 4.05 (s, 3H), 3.68 (dd, *J =* 10.6, 3.9 Hz, 1H), 3.64 (d, *J =* 10.7 Hz, 1H), 3.59 (d, *J =* 11.8 Hz, 2H), 3.18 (d, *J =* 12.5 Hz, 1H), 3.12 (t, *J* = 9.3 Hz, 3H), 3.07 (s, 2H), 3.06 - 3.00 (m, 1H), 2.45 (s, 3H), 2.38 (dt, *J =* 14.9, 7.3 Hz, 1H), 2.29 (dt, *J =* 14.9, 7.1 Hz, 1H), 2.06 (dd, *J* = 12.9, 7.8 Hz, 1H), 1.91 (ddt, *J* = 14.6, 9.5, 5.1 Hz, 3H), 0.96 (s, 9H).

### Compound 27

### N-{6-[2-(2-{4-[4-(N-benzyl-2-chloroacetamido)phenoxy]phenoxy}ethoxy)ethoxy]hexyl}-2-methyl-8-[4-(trifluoromethyl)phenyl]-2H,8H-pyrazolo[3,4-b]indole-5-carboxamide (Compound 27)

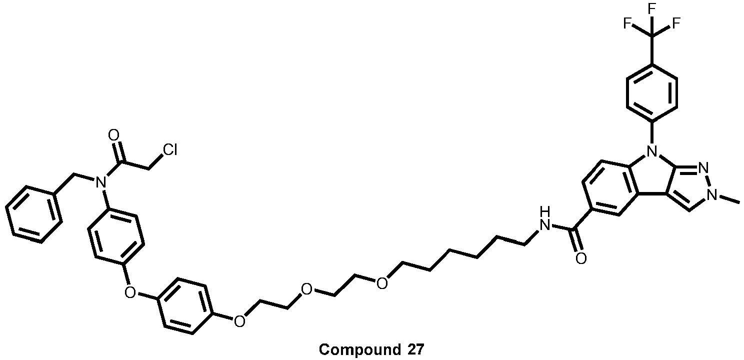

N-{4-[4-(2-{2-[(6-aminohexyl)oxy]ethoxy}ethoxy)phenoxy]phenyl}-N-benzyl-2-chloroacetamide; trifluoroacetic acid (51 mg; 0.08 mmol; freshly prepared by Boc-deprotection of tert-Butyl (6-(2-(2-(4-(4-(N-benzyl-2-chloroacetamido)phenoxy)phenoxy)ethoxy)ethoxy)hexyl)carbamate (commercially available from Sigma-Aldrich) in 50% TFA in DCM for 30 min at 0 °C followed by volatiles evaporation,) was placed in a 10 mL RBF with stir bar, dissolved in DCM (1) and cooled to 0°C, followed by addition of DIPEA (0.05 ml; 0.30 mmol) and then **TEAD ligand 1** (27 mg; 0.08 mmol). The reaction was started by additon of HATU (35 mg; 0.09 mmol). The ice bath was removed, and the reaction mixture was stirred overnight at RT. The reaction mixture was freed from DCM by rotary evaporation at 40°C. The brownish residue was immediately re-dissolved in DMSO (1.5 mL), followed by injection into a prep RP-HPLC-MS system. The target fractions were combined and bulk solvents removed by rotary evaporation. Residual water was removed by triple rotary evaporation of the remaining residue from toluene. **Compound 27** (27 mg; 0.03 mmol, 40 %) was obtained as a colorless oil. LC-MS (ESI+, Method Sunfire): *t*_{R} = 2,95 min, m/z 895,5 [M+H]⁺. ¹H NMR (500 MHz, Chloroform-d) δ 8.25 (d, *J* = 1.8 Hz, 1H), 7.93 (d, *J* = 8.3 Hz, 2H), 7.83 (d, *J* = 8.4 Hz, 2H), 7.74 - 7.67 (m, 2H), 7.62 (d, *J* = 8.6 Hz, 1H), 7.28 (pd, J = 4.8, 1.7 Hz, 3H), 7.21 (dd, *J* = 7.3, 2.1 Hz, 2H), 7.02 - 6.95 (m, 2H), 6.98 - 6.88 (m, 4H), 6.90 - 6.82 (m, 2H), 6.36 (t, *J* = 5.7 Hz, 1H), 4.87 (s, 2H), 4.14 (dd, *J* = 5.6, 3.9 Hz, 2H), 4.09 (s, 3H), 3.88 (s, 3H), 3.88 (d, *J* = 9.6 Hz, 1H), 3.79 - 3.72 (m, 2H), 3.67 - 3.62 (m, 2H), 3.51 (q, *J* = 6.5 Hz, 4H), 2.29 (s, 3H), 1.66 (dp, *J* = 16.4, 6.4 Hz, 2H), 1.45 (p, *J* = 3.2 Hz, 4H).

### Compound 28

### N-(14-{[2-(2,6-dioxopiperidin-3-yl)-1,3-dioxo-2,3-dihydro-1H-isoindol-4-yl]amino}-3,6,9,12-tetraoxatetradecan-1-yl)-2-methyl-8-[4-(trifluoromethyl)phenyl]-2H,8H-pyrazolo[3,4-b]indole-5-carboxamide (Compound 28)

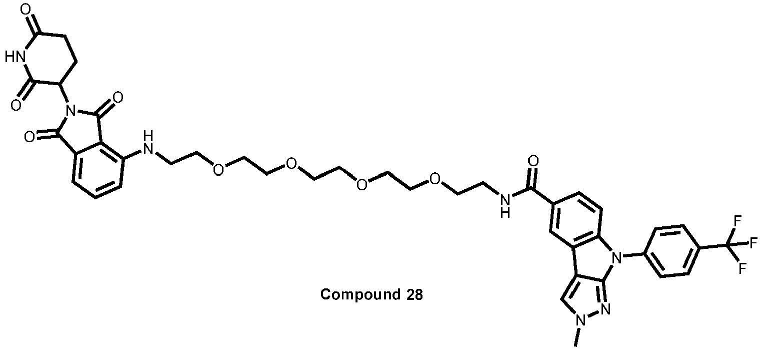

To **TEAD ligand 1** (60 mg; 0.17 mmol) in DMF (3 ml) were added 4-(15-amino-4,7,10,13-tetraoxa-1-azapentadecan-1-yl)-2-(2,6-dioxopiperidin-3-yl)-2,3-dihydro-1H-isoindole-1,3-dione (accessible as described in *ACS Med. Chem. Lett.* 2019, 10, 5, 767-772 (Supporting Information); 82 mg; 0.17 mmol), EDC hydrochloride (64 mg; 0,.33 mmol), HOBT hydrate (26 mg; 0.17 mmol) and 4-Methylmorpholine (0.1 mL; 0.83 mmol). The reaction was stirred overnight at RTand then evaporated to dryness. The crude residue was purified by prep. RP-HPLC. Fractions with product were combined and dried under vacuum to give **compound 28** (14 mg; 0.02 mmol; 10 % based on 94 % purity by LCMS analysis). LC-MS (ESI+, Method Chromolith): *t*_{R} = 1,72 min, m/z 834,10 [M+H]⁺. ¹H NMR (500 MHz, DMSO-d6) δ 11.08 (s, 1H), 8.50 (t, J = 5.6 Hz, 1H), 8.34 (d, J = 1.8 Hz, 1H), 8.19 (s, 1H), 8.10 - 8.06 (m, 2H), 7.98 - 7.94 (m, 2H), 7.85 (dd, J = 8.7, 1.8 Hz, 1H), 7.76 (d, J = 8.7 Hz, 1H), 7.55 (dd, J = 8.5, 7.1 Hz, 1H), 7.10 (d, J = 8.6 Hz, 1H), 7.02 (d, J = 7.0 Hz, 1H), 6.57 (t, J = 5.8 Hz, 1H), 5.04 (dd, J = 12.8, 5.4 Hz, 1H), 4.03 (s, 3H), 3.61 - 3.48 (m, 16H), 3.48 - 3.40 (m, 4H), 2.92 - 2.83 (m, 1H), 2.61 - 2.54 (m, 1H), 2.54 - 2.46 (m, 1H), 2.04 - 1.98 (m, 1H).

### Compound 29

### (2S,4R)-1-[(2S)-3,3-dimethyl-2-(6-{1-[7-({2-methyl-8-[4-(trifluoromethyl)phenyl]-2H,8H-pyrazolo[3,4-b]indol-5-yl}formamido)heptyl]-1H-1,2,3-triazol-4-yl}hexanamido)butanoyl]-4-hydroxy-N-[(1S)-1-[4-(4-methyl-1,3-thiazol-5-yl)phenyl]ethyl]pyrrolidine-2-carboxamide (Compound 29)

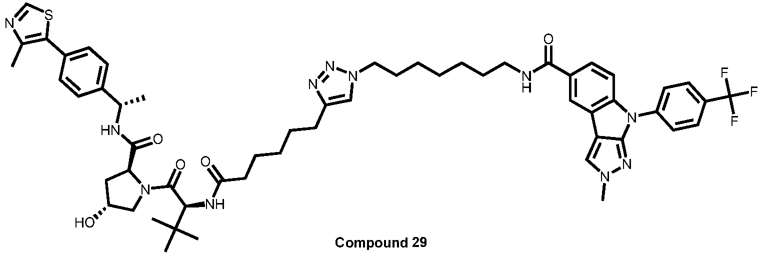

(2S,4R)-1-((S)-2-amino-3,3-dimethylbutanoyl)-4-hydroxy-N-((S)-1-(4-(4-methylthiazol-5-yl)phenyl)ethyl)pyrrolidine-2-carboxamide hydrochloride (commercially available from Sigma-Aldrich; 40 mg; 0.08 mmol), oct-7-ynoic acid (12 mg; 0.08 mmol) were dissolved in N,N-Dimethylformamide (0.5 mL), followed by addition of 4-Methylmorpholine, (purified by redistillation; 34 mg; 0.33 mmol) and then HATU (38 mg; 0.10 mmol) to start the reaction. The reaction mixture was stirred for 1 h at RT and then directly used for the next step as a crude solution. In a separate 8 mL vessel with stir bar were placed **TEAD ligand 1** (30 mg; 0.08 mmol), 7-azidoheptan-1-amine (13 mg; 0.08 mmol) and dissolved in DMF (0.5 mL), followed by addition of 4-Methylmorpholine (37 µL; 0.33 mmol) and then HATU (38 mg; 0.10 mmol): The reaction mixture was stirred for 1 h at RT and then directly used for the next step as a crude solution. A solution of Copper(II) sulfate pentahydrate (4 mg; 0.02 mmol) was prepared in DI water (250 µL) in a 4 mL vial. The aqueous copper sulfate solution was transferred to the vial containing the crude terminal alkyne by microliter pipette to give a deep green solution. The solution containing the crude azide was transferred to the mixture of copper sulfate and the terminal alkyne. To start the reaction, a freshly (< 5 min) made solution of sodium ascorbate (32 mg; 0.16 mmol) in DI water (500 µL) was added. The reaction mixture was briefly vortexed, then additional 500 µL of DMF were added. The reaction was vortexed until a yellow homogeneous solution was obtained. The reaction mixture was stirred overnight (18 h) at RT, and analysed by LCMS, indicating complete conversion to the desired product. The reaction mixture was filtered through a 0.2 µm membrane filter and the filtrate directly injected into a prep RP-HPLC-MS system. The target fractions were lyophilized to give **compound 29** (28 mg; 0.03 mmol, 33 % over two steps) as a white lyophilisate. LC-MS (ESI+, Method Chromolith): *t*_{R} = 1,87 min, m/z 1064,6 [M+H]⁺. ¹H NMR (500 MHz, DMSO-*d*₆) δ 8.97 (s, 1H), 8.45 (t, *J =* 5.6 Hz, 1H), 8.38 - 8.31 (m, 2H), 8.19 (s, 1H), 8.08 (d, *J* = 8.5 Hz, 2H), 7.96 (d, *J* = 8.5 Hz, 2H), 7.84 (dd, *J* = 8.7, 1.8 Hz, 1H), 7.80 (s, 1H), 7.76 (dd, *J* = 8.9, 1.7 Hz, 2H), 7.46 - 7.30 (m, 4H), 5.09 (d, *J* = 3.6 Hz, 1H), 4.92 (p, *J* = 7.0 Hz, 1H), 4.52 (d, *J* = 9.3 Hz, 1H), 4.43 (t, *J* = 8.0 Hz, 1H), 4.28 (t, *J* = 7.1 Hz, 3H), 4.03 (s, 3H), 3.65 - 3.57 (m, 2H), 3.28 (t, *J* = 6.6 Hz, 2H), 2.57 (t, *J* = 7.5 Hz, 2H), 2.45 (s, 3H), 2.29 - 2.19 (m, 1H), 2.11 (ddd, *J* = 14.4, 8.2, 6.4 Hz, 1H), 2.01 (ddd, *J =* 12.4, 7.6, 2.8 Hz, 1H), 1.80 (dddd, *J* = 13.1, 9.3, 5.9, 3.1 Hz, 3H), 1.53 (s, 2H), 1.63 - 1.42 (m, 5H), 1.40 - 1.20 (m, 12H), 0.93 (s, 9H).

### Compound 30

### N-(17-{4-[4-(N-benzyl-2-chloroacetamido)phenoxy]phenoxy}-3,6,9,12,15-pentaoxaheptadecan-1-yl)-2-methyl-8-[4-(trifluoromethyl)phenyl]-2H,8H-pyrazolo[3,4-b]indole-5-carboxamide (Compound 30)

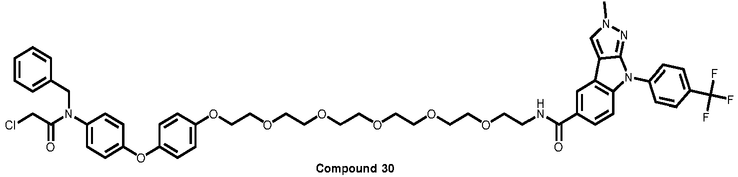

In a RBF with stir bar was placed tert-butyl N-(17-{4-[4-(N-benzyl-2-chloroacetamido)phenoxy]phenoxy}-3,6,9,12,15-pentaoxaheptadecan-1-yl)carbamate (commercially available from Sigma-Aldrich, 51 mg; 0.08 mmol) and dissolved in DCM (1 mL), to which TFA (1 mL) was added. The reaction mixture was stirred for 1 h at RT, analysed by LCMS, indicating complete conversion to the amine TFA salt. The reaction solvent was thoroughly removed by repeated evaporation from DCM. The remaining residue was re-dissolved in N,N-Dimethylformamide (1 mL), followed by addition of **TEAD ligand 1** (25 mg; 0.07 mmol), 4-Methylmorpholine (purified by redistillation) (38 µL; 0.34 mmol) and finally HATU (31 mg; 0.08 mmol). The reaction mixture was stirred at RT for 1 h, and then directly injected into a prep RP-HPLC-MS system and the target fractions lyophilized to provide **compound 30** (30 mg; 0.03 mmol, 43 % based on 95 % purity by LCMS analysis) as white microcrystals. LC-MS (ESI+, Method Sunfire): *t*_{R} = 2,79 min, m/z 971,6 [M+H]⁺. ¹H NMR (500 MHz, DMSO-ds) δ 12.73 (s, 1H), 8.77 (d, *J* = 4.5 Hz, 1H), 8.56 - 8.48 (m, 2H), 8.43 (d, *J* = 1.7 Hz, 1H), 8.35 (d, *J* = 1.8 Hz, 1H), 8.20 (d, *J* = 9.0 Hz, 2H), 8.08 (d, *J* = 8.2 Hz, 4H), 7.98 (s, 1H), 7.96 (d, *J* = 8.1 Hz, 3H), 7.93 (dd, *J* = 8.7, 1.8 Hz, 1H), 7.86 (dd, *J* = 8.7, 1.8 Hz, 1H), 7.77 (t, *J* = 8.4 Hz, 2H), 7.52 (dd, *J* = 8.4, 4.4 Hz, 1H), 7.33 - 7.15 (m, 8H), 6.97 (q, *J* = 9.1 Hz, 5H), 6.87 (d, *J* = 8.6 Hz, 2H), 4.84 (s, 2H), 4.08 (s, 2H), 4.04 (dd, *J* = 8.7, 3.3 Hz, 8H), 3.71 (t, *J* = 4.6 Hz, 2H), 3.60 - 3.43 (m, 20H).

### Compound 31

### 2-{[1-(2-chloroacetyl)-1,2,3,4-tetrahydroquinolin-6-yl]oxy}-N-[14-({2-methyl-8-[4-(trifluoromethyl)phenyl]-2H,8H-pyrazolo[3,4-b]indol-5-yl}formamido)-3,6,9,12-tetraoxatetradecan-1-yl]acetamide (Compound 31)

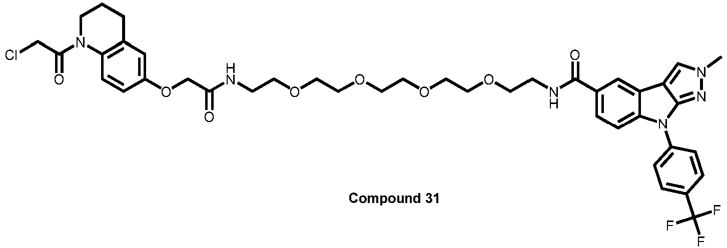

**TEAD ligand 1** (100 mg; 0.28 mmol) and tert-Butyl (14-amino-3,6,9,12-tetraoxatetradecyl)carbamate (commercially available from Sigma-Aldrich; 98 mg; 0.28 mmol) were dissolved in N,N-Dimethylformamide (2 ml), followed by addition of 4-Methylmorpholine (purified by redistillation) (0.12 mL; 1.11 mmol) and finally HATU (127 mg; 0.33 mmol) to start the reaction. The reaction mixture was stirred for 1 h at RT and then filtered through a 0.2 µm membrane filter.The filtrate was directly injected into a prep RP-HPLC-MS system. The target fractions were lyophilized to provide tert-butyl N-[14-({2-methyl-8-[4-(trifluoromethyl)phenyl]-2H,8H-pyrazolo[3,4-b]indol-5-yl}formamido)-3,6,9,12-tetraoxatetradecan-1-yl]carbamate (117 mg; 0.17 mmol, 62 %) as a white solid. This intermediate was deprotected in 50% TFA in DCM (4 mL) for 30 min at RT. The volatiles were thoroughly removed by triple rotary evaporation from DCM solutions. Freshly prepared (< 1 d old, as described in Nat. Chem. Biol. 2019, 15, 737-746, Supplementary Note 1. Synthetic Procedures) 2-{[1-(2-chloroacetyl)-1,2,3,4-tetrahydroquinolin-6-yl]oxy}acetic acid was used to effect amide coupling with the amine TFA salt, using 4-methylmorpholine (90 µL, 0.82 mmol) and HATU (93 mg, 0.25 mmol) at RT for 1 h. The crude reaction mixture was directly injected into a prep. RP-HPLC-MS system to give **compound 31** (43 mg; 0.05 mmol; 25 %) after lyophilization of the target fractions. LC-MS (ESI+, Method Sunfire): *t*_{R} = 2,46 min, m/z 842,6 [M+H]⁺. ¹H NMR (500 MHz, DMSO-d6) 8.51 (t, J = 5.6 Hz, 1H), 8.35 (d, J = 1.8 Hz, 1H), 8.20 (s, 1H), 8.08 (d, J = 8.4 Hz, 2H), 8.03 (q, J = 5.7 Hz, 1H), 7.96 (d, J = 8.6 Hz, 2H), 7.86 (dd, J = 8.7, 1.9 Hz, 1H), 7.77 (d, J = 8.7 Hz, 1H), 6.83 - 6.73 (m, 2H), 4.49 - 4.42 (m, 3H), 4.03 (s, 3H), 3.65 (t, J = 6.3 Hz, 2H), 3.60 - 3.40 (m, 20H), 3.28 (q, J = 5.9 Hz, 2H), 2.67 (t, J = 6.6 Hz, 2H), 1.91 - 1.79 (m, 2H). ¹³C NMR (126 MHz, DMSO-d6) δ 168.14, 166.80, 165.64, 156.39, 143.60, 141.40, 132.07, 127.88, 127.35 (q, J = 3.7 Hz), 125.37, 124.20, 123.47, 123.19, 120.76, 119.98, 114.79, 112.79, 110.85, 108.78, 70.26, 70.25, 70.22, 70.17, 70.09, 70.00, 69.48, 69.25, 67.57, 38.71, 26.74, 23.68.

### Compound 32

### (2S,4R)-1-[(2S)-3,3-dimethyl-2-[20-({2-methyl-8-[4-(trifluoromethyl)phenyl]-2H,8H-pyrazolo[3,4-b]indol-5-yl}formamido)-3,6,9,12,15,18-hexaoxaicosanamido]butanoyl]-4-hydroxy-N-{[4-(4-methyl-1,3-thiazol-5-yl)phenyl]methyl}pyrrolidine-2-carboxamide (Compound 32)

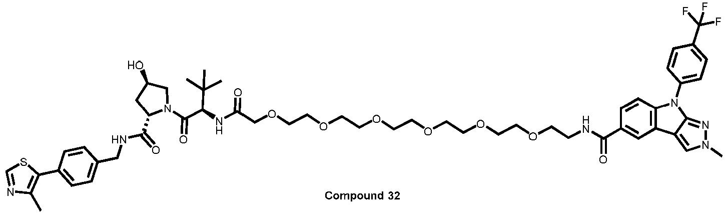

In a 8 mL vial with stir bar was added (2S,4R)-1-((S)-23-Amino-2-(tert-butyl)-4-oxo-6,9,12,15,18,21-hexaoxa-3-azatricosanoyl)-4-hydroxy-N-(4-(4-methylthiazol-5-yl)benzyl)pyrrolidine-2-carboxamide hydrochloride (commercially available from Sigma-Aldrich, 50 mg; 0.06 mmol), dissolved in N,N-Dimethylformamide, (1 mL), followed by addition of **TEAD ligand 1** (22 mg; 0.06 mmol), 4-Methylmorpholine (purified by redistillation) (33 µL; 0.30 mmol) and finally HATU (27 mg; 0.07 mmol). The reaction mixture was stirred at RT for 1 h, and then directly injected into a prep RP-HPLC-MS system and lyophilized to provide **compound 32** (31,00 mg; 0,03 mmol, 47%) as a colorless oil. LC-MS (ESI+, Method Sunfire): *t*_{R} = 2,45 min, m/z 546,9 [M+2H]²⁺. ¹H NMR (500 MHz, DMSO-d6) δ 8.97 (s, 1H), 8.57 (t, J = 6.1 Hz, 1H), 8.51 (t, J = 5.6 Hz, 1H), 8.34 (d, J = 1.8 Hz, 1H), 8.20 (s, 1H), 8.08 (d, J = 8.4 Hz, 2H), 7.96 (d, J = 8.5 Hz, 2H), 7.85 (dd, J = 8.7, 1.8 Hz, 1H), 7.77 (d, J = 8.7 Hz, 1H), 7.46 - 7.35 (m, 5H), 5.13 (d, J = 3.6 Hz, 1H), 4.56 (d, J = 9.6 Hz, 1H), 4.48 - 4.35 (m, 2H), 4.35 (s, 1H), 4.25 (dd, J = 15.8, 5.7 Hz, 1H), 4.03 (s, 3H), 3.96 (s, 2H), 3.70 - 3.42 (m, 26H), 2.43 (s, 3H), 2.05 (dd, J = 12.9, 7.9 Hz, 1H), 1.90 (ddd, J = 13.0, 8.7, 4.5 Hz, 1H), 0.94 (s, 9H). ¹³C NMR (126 MHz, DMSO-d6) δ 172.23, 169.61, 169.05, 151.89, 143.60, 131.60, 129.15, 127.93, 127.91, 127.38, 127.35, 124.20, 123.48, 123.20, 120.77, 110.87, 108.80, 70.91, 70.29, 70.25, 70.22, 70.19, 70.10, 70.07, 70.05, 69.49, 69.35, 59.21, 57.03, 56.17, 42.16, 38.38, 36.17, 26.64, 16.37.

### Compound 33

### (4aS,6aR,6bS,8aR,12aS,14aR,14bS)-11-cyano-2,2,6a,6b,9,9,12a-heptamethyl-N-[14-({2-methyl-8-[4-(trifluoromethyl)phenyl]-2H,8H-pyrazolo[3,4-b]indol-5-yl}formamido)-3,6,9,12-tetraoxatetradecan-1-yl]-10,14-dioxo-1,2,3,4,4a,5,6,6a,6b,7,8,8a,9,10,12a,14,14a,14b-octadecahydropicene-4a-carboxamide (compound 33)

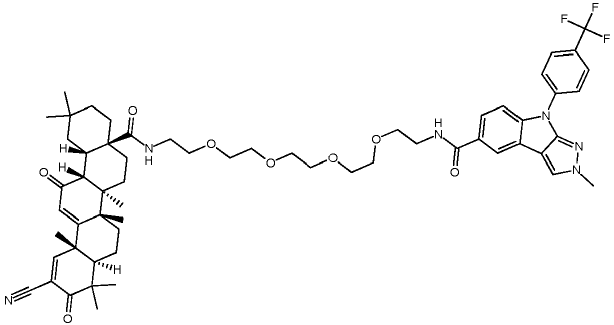

TEAD ligand 1 (100.00 mg; 0.28 mmol), tert-Butyl (14-amino-3,6,9,12-tetraoxatetradecyl)carbamate (commercially available from CombiBlocks, 97,53 mg; 0,28 mmol) were dissolved in N,N-Dimethylformamide (2.00 ml), followed by addition of 4-Methylmorpholine (0.12 ml; 1.11 mmol) and finally HATU (126.99 mg; 0,33 mmol) to start the reaction. The reaction mixture was stirred for 1 h at RT. The reaction mixture was filtered through a 0.2 µm membrane filter, and the filtrate directly injected into a prep RP-HPLC-MS system. The target fractions were lyophilized to provide tert-butyl N-[14-({2-methyl-8-[4-(trifluoromethyl)phenyl]-2H,8H-pyrazolo[3,4-b]indol-5-yl}formamido)-3,6,9,12-tetraoxatetradecan-1-yl]carbamate (117.00 mg; 0.17 mmol) as a white solid. This compound was deprotected in DCM/TFA 50% at RT for 1 h, the reaction mixture evaporated to dryness and the crude amine TFA salt used directly in the next step. Bardoxolone (commercially available from Advanced Chem Blocks, , 102.49 mg; 0.20 mmol) was placed in a 8 mL vial with stir bar, followed by addition of N-(14-amino-3,6,9,12-tetraoxatetradecan-1-yl)-2-methyl-8-[4-(trifluoromethyl)phenyl]-2H,8H-pyrazolo[3,4-b]indole-5-carboxamide (118.00 mg; 0.20 mmol), 4-methylmorpholine (0.09 ml; 0.82 mmol) and finally HATU (93.22 mg; 0.25 mmol). The reaction mixture was stirred for 1 h at RT. The reaction was filtered and directly injected into prep. RP-HPLC-MS system. The target fractions were lyophilized to provide (4aS,6aR,6bS,8aR,12aS,14aR,14bS)-11-cyano-2,2,6a,6b,9,9,12a-heptamethyl-N-[14-({2-methyl-8-[4-(trifluoromethyl)phenyl]-2H,8H-pyrazolo[3,4-b]indol-5-yl}formamido)-3,6,9,12-tetraoxatetradecan-1-yl]-10,14-dioxo-1,2,3,4,4a,5,6,6a,6b,7,8,8a,9,10,12a,14,14a,14b-octadecahydropicene-4a-carboxamide (32.00 mg; 0.03 mmol) as a white powder. LC-MS (ESI+, Method Chromolith): *t*_{R} = 2.06 min, m/z 1050.60 [M+H]⁺

### Compound 36

### N-[2-(1-methyl-2,6-dioxopiperidin-3-yl)-1,3-dioxo-2,3-dihydro-1H-isoindol-4-yl]-14-({2-methyl-8-[4-(trifluoromethyl)phenyl]-2H,8H-pyrazolo[3,4-b]indol-5-yl}formamido)-3,6,9,12-tetraoxatetradecanamide (Compound 36)

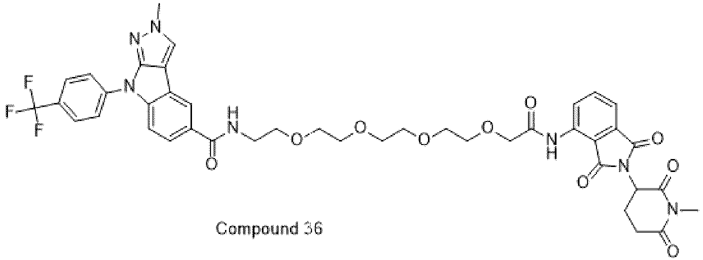

Diisopropylazodicarboxylat (37 mg; 0.18 mmol) was added to Triphenylphosphine (47 mg; 0.18 mmol) in dry THF at 0°C. The mixture was stirred under nitrogen until the formation of a milky precipitate was observed. Methanol (7.35 µL; 0.18 mmol) and N-[2-(2,6-dioxopiperidin-3-yl)-1,3-dioxo-2,3-dihydro-1H-isoindol-4-yl]-14-({2-methyl-8-[4-(trifluoromethyl)phenyl]-2H,8H-pyrazolo[3,4-b]indol-5-yl}formamido)-3,6,9,12-tetraoxatetradecanamide (77 mg; 0.09 mmol) were added in dry THF (1 mL) at 0°C. The solution was warmed to RT after stirring at 0°C for 30 min, and stirred at RT overnight. Because of very little conversion Cesium carbonate (89 mg; 0.27 mmol) was added to the reaction mixture, producing a suspension, to which lodomethane (220 µl) was added and the reaction mixture was stirred until full conversion (4 h). The reaction mixture was diluted with water, and the resulting mixture extracted with 20 % iPrOH in CHCI3 (2 x 2 mL). The extracts were freed from organic solvent by rotary evaporation. The remaining residue was partially dissolved in DMSO (1 mL) and filtered. The clear DMSO filtrate was employed for direct purification by HPLC. The target fractions were lyophilised to provide the product as a white lyophilizate (52 mg; 66%). ¹H NMR (400 MHz, DMSO-*d*₆) δ 10.27 (s, 1H), 8.48 (t, *J* = 5.6 Hz, 1H), 8.33 (d, *J* = 1.7 Hz, 1H), 8.27 (d, *J* = 1.8 Hz, 1H), 8.18 (s, 1H), 8.11 - 7.92 (m, 5H), 7.90 - 7.81 (m, 2H), 7.76 (d, *J* = 8.7 Hz, 1H), 5.19 (dd, *J* = 13.0, 5.4 Hz, 1H), 4.15 (s, 2H), 4.03 (s, 3H), 3.67 - 3.53 (m, 12H), 3.46 (m, 2H), 3.02 (s, 3H), 2.98 - 2.88 (m, 1H), 2.76 (m, 1H), 2.63 - 2.47 (m, 3H), 2.12 - 2.01 (m, 1H).

### Compound 37

### (2S,4R)-1-[(2S)-3,3-dimethyl-2-(6-{1-[7-({2-methyl-8-[4-(trifluoromethyl)-phenyl]-2H,8H-pyrazolo[3,4-b]indol-5-yl}formamido)heptyl]-1H-1,2,3-triazol-5-yl}hexanamido)butanoyl]-4-hydroxy-N-{[4-(4-methyl-1,3-thiazol-5-yl)phenyl]methyl}pyrrolidine-2-carboxamide (Compound 37)

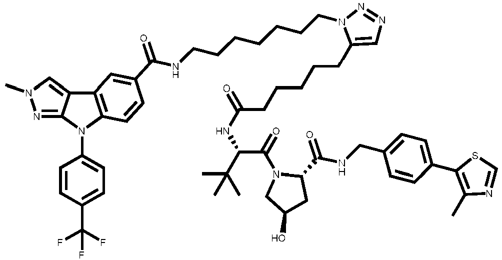

Prepared and purified as described for **Compound 38,** but using **intermediate 22** instead of intermediate **intermediate 21** which gave (2S,4R)-1-[(2S)-3,3-dimethyl-2-(6-{1-[7-({2-methyl-8-[4-(trifluoromethyl)-phenyl]-2H,8H-pyrazolo[3,4-b]indol-5-yl}formamido)heptyl]-1H-1,2,3-triazol-5-yl}hexanamido)butanoyl]-4-hydroxy-N-{[4-(4-methyl-1,3-thiazol-5-yl)phenyl]methyl}pyrrolidine-2-carboxamide (28.2 mg; 0.0026 mmol) as a colorless oil. LC-MS (ESI+, Method 5): tR = 0.97 min, m/z 525.4 [M+2H]2+.¹ H NMR (700 MHz, DMSO-*d*₆) δ 9.0 (s, 1H), 8.6 (t, *J* = 6.1 Hz, 1H), 8.5 (t, *J* = 5.6 Hz, 1H), 8.3 (d, *J* = 1.8 Hz, 1H), 8.2 (s, 1H), 8.1 (d, *J =* 8.4 Hz, 2H), 8.0 (d, *J* = 8.4 Hz, 2H), 7.9 - 7.8 (m, 2H), 7.8 (d, *J* = 8.6 Hz, 1H), 7.5 (s, 1H), 7.4 (d, *J* = 8.0 Hz, 2H), 7.4 - 7.4 (m, 2H), 5.0 - 4.6 (m, 0H), 4.6 - 4.2 (m, 10H), 4.0 (s, 3H), 3.7 (qd, *J* = 9.8, 9.0, 3.0 Hz, 2H), 3.3 (q, *J* = 6.7 Hz, 2H), 2.6 (t, *J* = 7.7 Hz, 2H), 2.4 (s, 3H), 2.3 - 2.2 (m, 1H), 2.2 - 2.1 (m, 1H), 2.1 - 2.0 (m, 1H), 1.9 - 1.9 (m, 1H), 1.8 - 1.7 (m, 2H), 1.6 - 1.4 (m, 5H), 1.4 - 1.2 (m, 7H), 0.9 (s, 9H).

### Compound 38

### (2S,4R)-1-[(2S)-3,3-dimethyl-2-(6-{1-[7-({2-methyl-8-[4-(trifluoromethyl)-phenyl]-2H,8H-pyrazolo[3,4-b]indol-5-yl}formamido)heptyl]-1H-1,2,3-triazol-5-yl}hexanamido)butanoyl]-4-hydroxy-N-[(1S)-1-[4-(4-methyl-1,3-thiazol-5-yl)phenyl]ethyl]pyrrolidine-2-carboxamide (Compound 38)

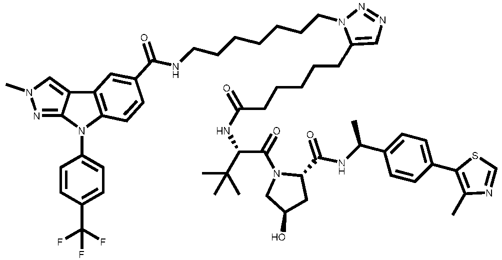

A 10 ml Schlenk tube was charged with pentamethylcyclopentadienyl-bis-(triphenylphosphin)-ruthenium(II)-chloride (1.5 mg; 0.002 mmol; 0.05 eq.) under nitrogen. N-(7-azidoheptyl)-2-methyl-8-[4-(trifluoromethyl)phenyl]-2H,8H-pyrazolo[3,4-b]indole-5-carboxamide **(intermediate 20)** (21.2 mg; 0.04 mmol; 1.20 eq.) and (2S,4R)-1-[(2S)-3,3-dimethyl-2-(oct-7-ynamido)butanoyl]-4-hydroxy-N-[(1S)-1-[4-(4-methyl-1,3-thiazol-5-yl)phenyl]ethyl]pyrrolidine-2-carboxamide **(intermediate 21)** (20.1 mg; 0.04 mmol; 1.00 eq.) were dissolved in dry dimethylformamide (1.0 ml) and added to the catalyst. The reaction mixture was irridiated at 60°C for 3h in the microwave. The resulting orange solution was filtered over a short pad of silica gel and then purified by prep. RP-HPLC to provide (2S,4R)-1-[(2S)-3,3-dimethyl-2-(6-{1-[7-({2-methyl-8-[4-(trifluoromethyl)phenyl]-2H,8H-pyrazolo[3,4-b]indol-5-yl}formamido)heptyl]-1H-1,2,3-triazol-5-yl}-hexanamido)butanoyl]-4-hydroxy-N-[(1S)-1-[4-(4-methyl-1,3-thiazol-5-yl)phenyl]ethyl]pyrrolidine-2-carboxamide (27.9mg; 0.0026 mmol) as a pale yellow solid. LC-MS (ESI+, Method 3): tR = 2.60 min, m/z 532.5 [M+2H]2+. ¹H NMR (500 MHz, DMSO-*d*₆) δ 9.0 (s, 1H), 8.4 (t, *J* = 5.7 Hz, 1H), 8.4 - 8.3 (m, 2H), 8.2 (s, 1H), 8.1 (d, *J* = 8.3 Hz, 2H), 8.0 (d, *J* = 8.4 Hz, 2H), 7.9 - 7.7 (m, 3H), 7.5 - 7.3 (m, 4H), 5.1 (d, *J* = 3.6 Hz, 1H), 4.9 (p, *J* = 7.1 Hz, 1H), 4.5 (dd, *J* = 9.3, 1.8 Hz, 1H), 4.4 (td, *J* = 8.1, 2.1 Hz, 1H), 4.3 - 4.2 (m, 3H), 4.0 (s, 3H), 3.7 - 3.6 (m, 2H), 3.3 - 3.2 (m, 2H), 2.6 - 2.5 (m, 2H), 2.5 (s, 3H), 2.3 - 2.2 (m, 1H), 2.2 - 2.1 (m, 1H), 2.1 - 2.0 (m, 1H), 1.8 - 1.7 (m, 3H), 1.6 - 1.5 (m, 7H), 1.5 - 1.4 (m, 1H), 1.4 - 1.2 (m, 9H), 1.1 (s, 1H), 0.9 (s, 9H).

### Compound 84

### (2S,4R)-1-[(2S)-3,3-dimethyl-2-[6-(1-{3-[3-({2-methyl-8-[4-(trifluoromethyl)phenyl]-2H,8H-pyrazolo[3,4-b]indol-5-yl}formamido)propoxy]-propyl}-1H-1,2,3-triazol-4-yl)hexanamido]butanoyl]-4-hydroxy-N-[(1S)-1-[4-(4-methyl-1,3-thiazol-5-yl)phenyl]ethyl]pyrrolidine-2-carboxamide (Compound 84)

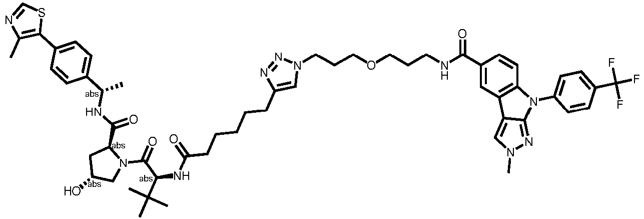

To a stirred mixture of N-[3-(3-azidopropoxy)propyl]-2-methyl-8-[4-(trifluoromethyl)phenyl]-2H,8H-pyrazolo[3,4-b]indole-5-carboxamide **(intermediate 29)** (210.0 mg; 0.08 mmol; 1.00 eq.), (2S,4R)-1-[(2S)-3,3-dimethyl-2-(oct-7-ynamido)butanoyl]-4-hydroxy-N-[(1S)-1-[4-(4-methyl-1,3-thiazol-5-yl)phenyl]ethyl]pyrrolidine-2-carboxamide **(intermediate 21)** (52.0 mg; 0.09 mmol; 1.21 eq.) in DMF (10.0 ml), H2O (2.5 ml) was added CuSO₄•5H₂O (2.0 mg; 0.01 mmol; 0.10 eq.), sodium ascorbate (2.0 mg; 0.01 mmol; 0.13 eq.) at room temperature. The resulting mixture was stirred for 1 d at room temperature. The reaction was quenched with water at room temperature. The resulting mixture was extracted with EtOAc (3 x 150 mL). The combined organic layers were washed with brine (1x100 mL), dried over anhydrous Na₂SO₄. After filtration, the filtrate was concentrated under reduced pressure and purified by preparative RP-HPLC to afford (2S,4R)-1-[(2S)-3,3-dimethyl-2-[6-(1-{3-[3-({2-methyl-8-[4-(trifluoromethyl)phenyl]-2H,8H-pyrazolo[3,4-b]indol-5-yl}formamido)-propoxy]propyl}-1H-1,2,3-triazol-4-yl)hexanamido]butanoyl]-4-hydroxy-N-[(1S)-1-[4-(4-methyl-1,3-thiazol-5-yl)phenyl]ethyl]pyrrolidine-2-carboxamide as a white solid (2.1 mg; 2.6 %). LC-MS (ESI+, Method 7): t_{R} = 0.93 min, m/z 1066.5 [M+H]+. 1H-NMR (300 MHz, DMSO-d6) 8.98 (s, 1H), 8.47 (s, 1H), 8.34 (s, 2H), 8.20 (s, 1H), 8.09 (d, J = 8.3 Hz, 2H), 7.97 (d, J = 8.2 Hz, 2H), 7.80 (q, J = 11.6, 8.5 Hz, 4H), 7.47 - 7.35 (m, 4H), 5.09 (s, 1H), 4.91 (s, 1H), 4.51 (d, J = 8.8 Hz, 1H), 4.40 (d, J = 7.6 Hz, 2H), 4.27 (s, 1H), 4.03 (s, 3H), 3.60 (s, 2H), 3.45 (s, 5H), 3.05 (s, 1H), 2.85 (s, 8H),1.80 (s, 3H), 1.52 (s, 4H),1.37 (d, J = 6.9 Hz, 3H), 1.24 (s, 2H), 0.92 (s, 9H).

### Compound 81

### 8-{6,6-difluorospiro[3.3]heptan-2-yl}-N-(14-{[2-(2,6-dioxopiperidin-3-yl)-1,3-dioxo-2,3-dihydro-1H-isoindol-4-yl]amino}-3,6,9,12-tetraoxatetradecan-1-yl)-2-methyl-2H,8H-pyrazolo[3,4-b]indole-5-carboxamide (Compound 81)

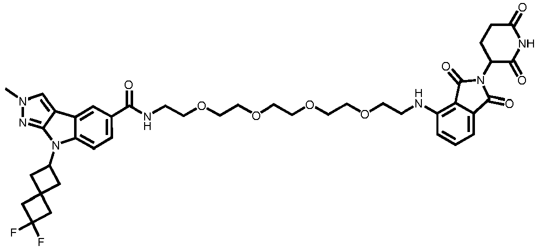

To **building block 7** (0.05 mmol; 1.00 eq.; 16.8 mg) in anhydrous DMF (3.00 ml) was added 4-(15-amino-4,7,10,13-tetraoxa-1-azapentadecan-1-yl)-2-(2,6-dioxopiperidin-3-yl)-2,3-dihydro-1H-isoindole-1,3-dione (commercially available from Acros Organics, 1.05 mmol; 1.00 eq.; 24.0 mg) EDC•HCl (0.10 mmol; 2.00 eq.; 18.7 mg), HOBt • H₂O (0.05 mmol; 1.00 eq.; 7.5 mg) and 4-methylmorpholine for synthesis (0.24 mmol; 5.00 eq.; 0.03 ml). The reaction was stirred overnight at rt. The reaction mixture was evaporated to dryness. The crude residue was purified by prep. RP-HPLC Fractions with product were combined and the solvent removed under vacuum. 8-{6,6-difluorospiro[3.3]heptan-2-yl}-N-(14-{[2-(2,6-dioxo-piperidin-3-yl)-1,3-dioxo-2,3-dihydro-1H-isoindol-4-yl]amino}-3,6,9,12-tetraoxatetradecan-1-yl)-2-methyl-2H,8H-pyrazolo[3,4-b]indole-5-carboxamide (14.80 mg; 0.02 mmol, 37%). LC-MS (ESI+, Method 7): t_{R} = 0.93 min, m/z 1066.5 [M+H]+. 1H NMR (700 MHz, DMSO-d6) δ 11.1 (s, 1H), 8.4 (t, J = 5.6 Hz, 1H), 8.2 (d, J = 1.9 Hz, 1H), 8.1 (s, 1H), 7.8 (dd, J = 8.6, 1.8 Hz, 1H), 7.6 (dd, J = 8.6, 7.1 Hz, 1H), 7.4 (d, J = 8.7 Hz, 1H), 7.1 (d, J = 8.6 Hz, 1H), 7.0 (d, J = 7.1 Hz, 1H), 6.6 (t, J = 5.9 Hz, 1H), 5.0 (dd, J = 12.9, 5.5 Hz, 1H), 5.0 (tt, J = 9.0, 7.7 Hz, 1H), 4.0 (s, 3H), 3.6 (t, J = 5.4 Hz, 2H), 3.6 - 3.5 (m, 16H), 3.5 - 3.4 (m, 4H), 3.1 - 3.0 (m, 2H), 2.9 - 2.8 (m, 1H), 2.8 (td, J = 12.5, 2.7 Hz, 2H), 2.7 - 2.7 (m, 2H), 2.6 - 2.5 (m, 2H), 2.0 - 2.0 (m, 1H).

### Compound 80

### N-{2-[2-(2,6-dioxopiperidin-3-yl)-1,3-dioxo-2,3-dihydro-1H-isoindol-4-yl]-5,8,11,14-tetraoxa-2-azahexadecan-16-yl}-2-methyl-8-[4-(trifluoromethyl)phenyl]-2H,8H-pyrazolo[3,4-b]indole-5-carboxamide (Compound 80)

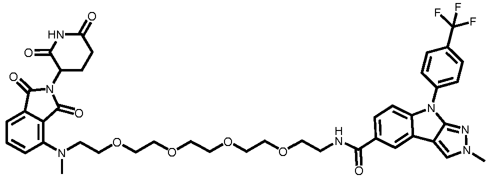

In a 4 ml glass vial, 4-(16-amino-5,8,11,14-tetraoxa-2-azahexadecan-2-yl)-2-(2,6-dioxopiperidin-3-yl)-2,3-dihydro-1H-isoindole-1,3-dione **(intermediate 30)** (15.6 mg; 0.03 mmol; 1.00 eq.) and **TEAD 1 ligand free acid** (11.1 mg; 0.03 mmol; 1.00 eq.) were dissolved in anhydrous DMF (1.0 ml). HATU (14.1 mg; 0.04 mmol; 1.20 eq.) and 4-methylmorpholine (27.0 µl; 0.25 mmol; 8.00 eq.) were added and the reaction mixture was stirred for 2 h at rt. The crude product (as solution in DMF) was purified by reversed phase prep. HPLC to give the desired product (21.3 mg, 82%). LC-MS (ESI+, Method 3): t_{R} = 2.38 min, m/z 848.6 [M+H]+. 1H NMR (500 MHz, DMSO-d6) δ 11.1 (s, 1H), 8.5 (t, J = 5.6 Hz, 1H), 8.3 (d, J = 1.8 Hz, 1H), 8.2 (s, 1H), 8.1 (d, J = 8.5 Hz, 2H), 8.0 (d, J = 8.5 Hz, 2H), 7.8 (dd, J = 8.7, 1.9 Hz, 1H), 7.8 (d, J = 8.7 Hz, 1H), 7.6 (dd, J = 8.6, 7.0 Hz, 1H), 7.3 (d, J = 8.6 Hz, 1H), 7.2 (d, J = 6.9 Hz, 1H), 5.1 (dd, J = 12.7, 5.4 Hz, 1H), 4.0 (s, 3H), 3.7 - 3.4 (m, 20H), 3.0 (s, 3H), 2.9 (ddd, J = 16.6, 13.7, 5.4 Hz, 1H), 2.6 - 2.5 (m, 2H), 2.0 (ddt, J = 12.8, 5.6, 3.0 Hz, 1H).

### Compound 77

### (2S,4R)-1-[(2S)-3,3-dimethyl-2-{7-[2-({2-methyl-8-[4-(trifluoromethyl)-phenyl]-2H,8H-pyrazolo[3,4-b]indol-5-yl}formamido)ethoxy]heptanamido}-butanoyl]-4-hydroxy-N-{[4-(4-methyl-1,3-thiazol-5-yl)phenyl]methyl}-pyrrolidine-2-carboxamide (Compound 77)

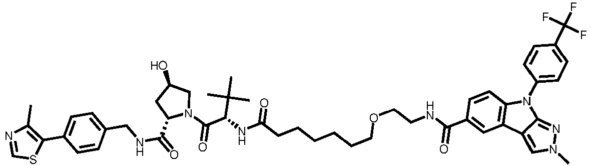

To a stirred mixture of **intermediate 33** (275.00 mg; 0.50 mmol; 1.00 eq.), (2S,4R)-1-[(2S)-2-amino-3,3-dimethylbutanoyl]-4-hydroxy-N-{[4-(4-methyl-1,3-thiazol-5-yl)phenyl]methyl}pyrrolidine-2-carboxamide **VHL-Lig** (273.00 mg; 0.60 mmol; 1.20 eq.) in DCM (15.00 ml) was added HATU (401.00 mg; 1.00 mmol; 2.00 eq.), Et₃N (0.14 ml; 1.00 mmol; 2.00 eq.) at room temperature. The resulting mixture was stirred for 16 h at room temperature. The reaction was quenched with water at room temperature. The resulting mixture was extracted with DCM (3 x 150 mL). The combined organic layers were washed with brine (1x100 mL), dried over anhydrous Na₂SO₄. After filtration, the filtrate was concentrated under reduced pressure. The crude reaction product was purified by prep RP-HPLC to afford (2S,4R)-1-[(2S)-3,3-dimethyl-2-{7-[2-({2-methyl-8-[4-(trifluoromethyl)phenyl]-2H,8H-pyrazolo[3,4-b]indol-5-yl}formamido)ethoxy]-heptanamido}butanoyl]-4-hydroxy-N-{[4-(4-methyl-1,3-thiazol-5-yl)-phenyl]methyl}pyrrolidine-2-carboxamide (27.70 mg; 0.03 mmol; 5.5 %) as a white solid. LC-MS (ESI+, Method 4): t_{R} = 1.42 min, m/z 941.4 [M+H]+ 1H NMR (300 MHz, DMSO) δ 8.99 (s, 1H), 8.60 - 8.47 (m, 2H), 8.34 (s, 1H), 8.21 (s, 1H), 8.09 (d, J = 8.5 Hz, 2H), 7.97 (d, J = 8.6 Hz, 2H), 7.85 (d, J = 8.7 Hz, 2H), 7.77 (d, J = 8.8 Hz, 1H), 7.44 - 7.36 (m, 4H), 4.54 (d, J = 9.3 Hz, 1H), 4.47 - 4.38 (m, 2H), 4.34 (s, 1H), 4.21 (dd, J = 15.8, 5.4 Hz, 2H), 3.65 (s, 2H), 3.52 - 3.37 (m, 6H), 2.44 (s, 3H), 2.25 (dt, J = 14.6, 7.5 Hz, 1H), 2.16 - 1.98 (m, 2H), 1.95 - 1.82 (m, 1H), 1.48 (s, 4H), 1.27 (s, 4H), 0.92 (s, 9H).

### Compound 75

### (2S,4R)-1-[(2S)-3,3-dimethyl-2-(4-{2-[2-({2-methyl-8-[4-(trifluoromethyl)-phenyl]-2H,8H-pyrazolo[3,4-b]indol-5-yl}formamido)ethoxy]ethoxy}-butanamido)butanoyl]-4-hydroxy-N-{[4-(4-methyl-1,3-thiazol-5-yl)phenyl]-methyl}pyrrolidine-2-carboxamide (Compound 75)

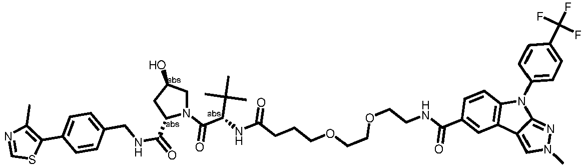

To a stirred mixture of 4-{2-[2-({2-methyl-8-[4-(trifluoromethyl)phenyl]-2H,8H-pyrazolo[3,4-b]indol-5-yl}formamido)ethoxy]ethoxy}butanoic acid **intermediate 34** (220.00 mg; 0.39 mmol; 1.00 eq.), (2S,4R)-1-[(2S)-2-amino-3,3-dimethylbutanoyl]-4-hydroxy-N-{[4-(4-methyl-1,3-thiazol-5-yl)-phenyl]methyl}pyrrolidine-2-carboxamide **VHL-Lig** 211.00 mg; 0.47 mmol; 1.20 eq.), Et₃N (87.00 mg; 0.77 mmol; 2.00 eq.)in DCM (10.00 ml) was added HATU (311.00 mg; 0.78 mmol; 2.00 eq.) at room temperature. The resulting mixture was stirred for 16 h at room temperature. The reaction was quenched with water at room temperature. The resulting mixture was extracted with DCM (3 x 150 mL). The combined organic layers were washed with brine (1x100 mL), dried over anhydrous Na₂SO₄. After filtration, the filtrate was concentrated under reduced pressure and then purified by prep RP-HPLC to afford (2S,4R)-1-[(2S)-3,3-dimethyl-2-(4-{2-[2-({2-methyl-8-[4-(trifluoromethyl)phenyl]-2H,8H-pyrazolo[3,4-b]indol-5-yl}formamido)ethoxy]ethoxy}butanamido)butanoyl]-4-hydroxy-N-{[4-(4-methyl-1,3-thiazol-5-yl)phenyl]methyl}pyrrolidine-2-carboxamide (41.90 mg; 0.04 mmol; 11.3 %) as a white solid. LC-MS (ESI+, Method 7): t_{R} = 0.99 min, m/z 941.4 [M+H]+. 1H NMR (300 MHz, DMSO-d6) 8.99 (s, 1H), 8.56 (d, J = 5.5 Hz, 2H), 8.35 (s, 1H), 8.21 (s, 1H), 8.08 (d, J = 8.5 Hz, 2H), 7.96 (d, J = 8.6 Hz, 2H), 7.92 - 7.83 (m, 2H), 7.77 (d, J = 8.7 Hz, 1H), 7.48 - 7.33 (m, 4H), 4.55 - 4.16 (m, 6H), 4.03 (s, 3H), 3.67 - 3.33 (m, 12H), 2.43 (s, 3H), 2.23 (dh, J = 21.9, 7.6 Hz, 2H), 2.03 (t, J = 10.6 Hz, 1H), 1.95 - 1.81 (m, 1H), 1.70 (q, J = 5.1, 3.1 Hz, 2H), 0.92 (s, 9H).

### Compound 74

### (2S,4R)-1-[(2S)-3,3-dimethyl-2-[2-(3-{2-[2-({2-methyl-8-[4-(trifluoromethyl)phenyl]-2H,8H-pyrazolo[3,4-b]indol-5-yl}formamido)ethoxy]-ethoxy}propoxy)acetamido]butanoyl]-4-hydroxy-N-{[4-(4-methyl-1,3-thiazol-5-yl)phenyl]methyl}pyrrolidine-2-carboxamide (Compound 74)

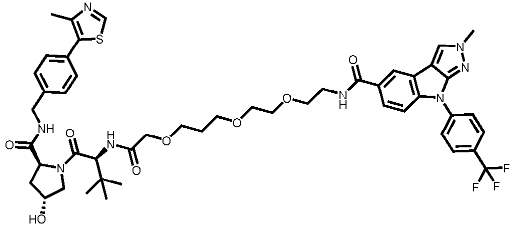

To a stirred solution of (2S,4R)-1-[(2S)-2-(2-{3-[2-(2-aminoethoxy)ethoxy]-propoxy}acetamido)-3,3-dimethylbutanoyl]-4-hydroxy-N-{[4-(4-methyl-1,3-thiazol-5-yl)phenyl]methyl}pyrrolidine-2-carboxamide TFA salt **intermediate 35** (67.000 mg; 0.075 mmol; 0.5 eq.) and **TEAD 1 ligand free acid** (50.000 mg; 0.139 mmol; 1.0 eq.) in DMF (5.000 ml) was added HATU (55.000 mg; 0.137 mmol; 1.0 eq.) and DIEA (0.100 ml; 0.545 mmol; 3.9 eq.) at room temperature. The resulting mixture was stirred for 4 h at room temperature under nitrogen atmosphere. The resulting mixture was extracted with EA (3x50 mL). The combined organic layers were washed with H₂O (3x50 mL) and dried over anhydrous Na₂SO₄. After filtration, the filtrate was concentrated under reduced pressure. The crude product was purified by reverse phase flash chromatography to afford (2S,4R)-1-[(2S)-3,3-dimethyl-2-[2-(3-{2-[2-({2-methyl-8-[4-(trifluoromethyl)phenyl]-2H,8H-pyrazolo[3,4-b]indol-5-yl}formamido)ethoxy]ethoxy}propoxy)acetamido]butanoyl]-4-hydroxy-N-{[4-(4-methyl-1,3-thiazol-5-yl)phenyl]-methyl}pyrrolidine-2-carboxamide (7.800 mg; 0.008 mmol; 5.6 %) as a white solid. LC-MS (ESI+, Method 2): t_{R} = 0.96 min, m/z 975.4 [M+H]+. 1H NMR (400 MHz, DMSO-d6) 8.98 (s, 1H), 8.61 (t, J = 6.0 Hz, 1H), 8.55 (t, J = 5.5 Hz, 1H), 8.34 (d, J = 1.8 Hz, 1H), 8.20 (s, 1H), 8.08 (d, J = 8.4 Hz, 2H), 7.96 (d, J = 8.6 Hz, 2H), 7.85 (dd, J = 8.7, 1.8 Hz, 1H), 7.77 (d, J = 8.7 Hz, 1H), 7.39 (s, 5H), 5.16 (s, 1H), 4.55 (d, J = 9.5 Hz, 1H), 4.44 (t, J = 8.2 Hz, 1H), 4.41- 4.36 (m, 1H), 4.35 (s, 1H), 4.25 (dd, J = 15.7, 5.6 Hz, 2H), 4.03 (s, 3H), 3.90 (d, J = 1.1 Hz, 2H), 3.65 (dd, J = 10.7, 3.8 Hz, 1H), 3.60 (d, J = 10.7 Hz, 1H), 3.57- 3.48 (m, 8H), 3.46 (dt, J = 7.8, 4.8 Hz, 4H), 2.43 (s, 3H), 2.09-2.03 (m, 1H), 1.91 (dt, J = 12.8, 3.9 Hz, 1H), 1.75 (q, J = 6.4 Hz, 2H), 0.93 (s, 9H).

### Compound 73

### (2S)-2-{[(2S)-1-[(2S)-2-cyclohexyl-2-[(2S)-2-{[6-({2-methyl-8-[4-(trifluoromethyl)phenyl]-2H,8H-pyrazolo[3,4-b]indol-5-yl}formamido)hexyl]amino}-propanamido]acetyl]pyrrolidin-2-yl]formamido}-N-ethyl-3-phenylpropan-amide (Compound 73)

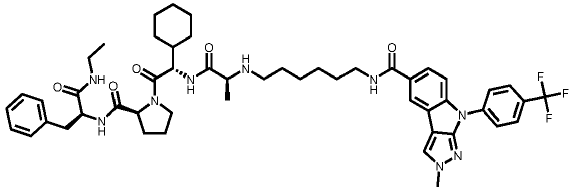

In a 8 mL vial with stir bar were placed **TEAD ligand 1 free acid** (30.00 mg; 0.08 mmol; 1.00 eq.), (2S)-2-[(6-aminohexyl)amino]-N-[(1S)-1-cyclohexyl-2-[(2S)-2-{[(1S)-1-(ethylcarbamoyl)-2-phenylethyl]carbamoyl}-pyrrolidin-1-yl]-2-oxoethyl]propanamide (commercially available from Sigma-Aldrich, 50.00 mg; 0.08 mmol; 1.00 eq.), DMF (2.00 ml), 4-methylmorpholine (37.0 µl; 0.33 mmol; 4.00 eq.). The reaction was started by addition of HATU (38.10 mg; 0.10 mmol; 1.20 eq.). The reaction mixture was stirred for 24 h at RT. The reaction mixture was filtered and purified by prep RP-HPLC-MS. The target fractions were lyophilized to provide (2S)-2-{[(2S)-1-[(2S)-2-cyclohexyl-2-[(2S)-2-{[6-({2-methyl-8-[4-(trifluoromethyl)phenyl]-2H,8H-pyrazolo[3,4-b]indol-5-yl}formamido)hexyl]amino}-propanamido]acetyl]pyrrolidin-2-yl]formamido}-N-ethyl-3-phenylpropan-amide (44.00 mg; 0.05 mmol). LC-MS (ESI+, Method 1): t_{R} = 1.63 min, m/z 940.8 [M+H]+. 1H NMR (500 MHz, DMSO-d6) δ 8.5 (t, J = 5.6 Hz, 1H), 8.3 (d, J = 1.8 Hz, 1H), 8.2 (s, 1H), 8.1 - 8.0 (m, 3H), 8.0 (d, J = 8.5 Hz, 2H), 7.9 (d, J = 7.9 Hz, 1H), 7.8 (dd, J = 8.7, 1.8 Hz, 1H), 7.8 = 7.7 (m, 2H), 7.3 - 7.2 (m, 2H), 7.2 - 7.1 (m, 3H), 4.4 (dt, J = 16.4, 8.0 Hz, 1H), 4.3 - 4.2 (m, 2H), 4.0 (s, 3H), 3.7 - 3.6 (m, 1H), 3.6 - 3.4 (m, 5H), 3.3 (q, J = 6.7 Hz, 2H), 3.3 - 3.2 (m, 1H), 3.1 - 3.0 (m, 1H), 3.0 - 2.9 (m, 3H), 2.5 (t, J = 7.1 Hz, 2H), 1.9 (ddd, J = 11.4, 8.0, 5.5 Hz, 1H), 1.9 - 1.7 (m, 2H), 1.7 - 1.5 (m, 6H), 1.5 - 1.4 (m, 2H), 1.4 - 1.3 (m, 4H), 1.2 - 1.0 (m, 6H), 1.0 - 0.9 (m, 5H).

### Compound 69

### (13S)-N-[(1S)-1-cyclohexyl-2-[(2S)-2-{[(1S)-1-(ethylcarbamoyl)-2-(4-fluorophenyl)ethyl]carbamoyl}pyrrolidin-1-yl]-2-oxoethyl]-13-methyl-1-({2-methyl-8-[4-(trifluoromethyl)phenyl]-2H,8H-pyrazolo[3,4-b]indol-5-yl}form-amido)-3,6,9-trioxa-12-azatetradecan-14-amide; formic acid (Compound 69)

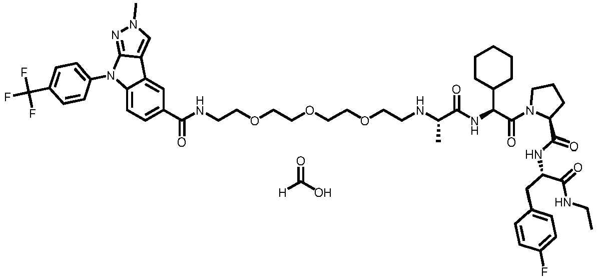

In a 4 mL vial with stir bar were combined **TEAD 1 ligand free acid** (25.9 mg; 0.07 mmol; 1.00 eq.), (13S)-1-amino-N-[(1S)-1-cyclohexyl-2-[(2S)-2-{[(1S)-1-(ethylcarbamoyl)-2-(4-fluorophenyl)ethyl]carbamoyl}pyrrolidin-1-yl]-2-oxoethyl]-13-methyl-3,6,9-trioxa-12-azatetradecan-14-amide (commercially available from Sigma-Aldrich) (50.00 mg; 0,07 mmol; 1.00 eq.), DMF (1.00 ml), 4-methylmorpholine (32 µl; 0.29 mmol; 4.00 eq.) and the reaction was started by addition of HATU (33 mg; 0.09 mmol; 1.20 eq.). The reaction mixture was stirred at RT for 1 week. The reaction mixture was diluted with 0.5 mL water and then 0.5 mL DMSO. The solution was filtered through a 0.2 µm membrane filter and directly injected for purification by prep RP-HPLC-MS. (13S)-N-[(1S)-1-cyclohexyl-2-[(2S)-2-{[(1S)-1-(ethylcarbamoyl)-2-(4-fluorophenyl)ethyl]carbamoyl}pyrrolidin-1-yl]-2-oxoethyl]-13-methyl-1-({2-methyl-8-[4-(trifluoromethyl)phenyl]-2H,8H-pyrazolo[3,4-b]indol-5-yl}formamido)-3,6,9-trioxa-12-azatetra-decan-14-amide; formic acid (60.00 mg; 0.06 mmol) was isolated as white lyophilisate. LC-MS (ESI+, Method 1): tR = 1.58 min, m/z 1034.3 [M+H]+. ¹H NMR (500 MHz, DMSO-*d*₆) δ 8.5 (t, *J* = 5.6 Hz, 1H), 8.3 (d, *J* = 1.8 Hz, 1H), 8.2 (d, *J* = 5.9 Hz, 1H), 8.1 (d, *J* = 8.4 Hz, 2H), 8.0 - 7.9 (m, 4H), 7.9 (dd, *J* = 8.7, 1.9 Hz, 1H), 7.8 (dd, *J* = 9.9, 4.5 Hz, 2H), 7.3 - 7.2 (m, 2H), 7.1 - 7.0 (m, 2H), 4.4 - 4.3 (m, 1H), 4.3 (ddd, *J* = 15.8, 8.0, 5.4 Hz, 2H), 4.0 (s, 3H), 3.8 - 3.3 (m, 24H), 3.1 (q, *J* = 6.8 Hz, 1H), 3.1 = 3.0 (m, 1H), 3.0 (tdd, *J =* 12.7, 8.9, 5.6 Hz, 2H), 2.9 (dd, *J* = 13.7, 8.3 Hz, 1H), 2.6 - 2.5 (m, 1H), 2.0 - 1.5 (m, 6H), 1.2 - 1.0 (m, 5H), 1.0 - 0.8 (m, 4H).

### Compound 82

### (2S,4R)-1-[(2S)-3,3-dimethyl-2-[6-(1-{4-[2-({2-methyl-8-[4-(trifluoromethyl)phenyl]-2H,8H-pyrazolo[3,4-b]indol-5-yl}formamido)ethoxy]butyl}-1H-1,2,3-triazol-4-yl)hexanamido]butanoyl]-4-hydroxy-N-[(1S)-1-[4-(4-methyl-1,3-thiazol-5-yl)phenyl]ethyl]pyrrolidine-2-carboxamide (Compound 82)

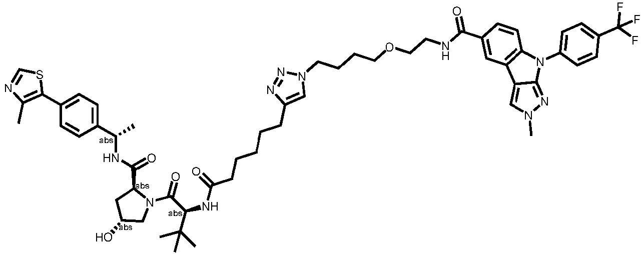

To a stirred mixture of (2S,4R)-1-[(2S)-3,3-dimethyl-2-(oct-7-ynamido)-butanoyl]-4-hydroxy-N-[(1S)-1-[4-(4-methyl-1,3-thiazol-5-yl)phenyl]ethyl]-pyrrolidine-2-carboxamide **(intermediate 21)** (200.00 mg; 0.35 mmol; 1.42 eq.), CuSO₄ 5H₂O (11.00 mg; 0.04 mmol; 0.17 eq.), sodium ascorbate (23.00 mg; 0.11 mmol; 0.44 eq.) in DMF (10.00 ml),H2O (2.50 ml) was added N-[2-(4-azidobutoxy)ethyl]-2-methyl-8-[4-(trifluoromethyl)phenyl]-2H,8H-pyrazolo[3,4-b]indole-5-carboxamide **intermediate 37** (231.00 mg; 0.25 mmol; 1.00 eq.) at room temperature. The resulting mixture was stirred for 16 h at 25 °C. The reaction was quenched with water at room temperature. The resulting mixture was extracted with EtOAc (3 x 150 mL). The combined organic layers were washed with brine (1x100 mL), dried over anhydrous Na₂SO₄. After filtration, the filtrate was concentrated under reduced pressure and then purified by preparative RP-HPLC to afford (2S,4R)-1-[(2S)-3,3-dimethyl-2-[6-(1-{4-[2-({2-methyl-8-[4-(trifluoromethyl)phenyl]-2H,8H-pyrazolo[3,4-b]indol-5-yl}formamido)ethoxy]butyl}-1H-1,2,3-triazol-4-yl)hexanamido]butanoyl]-4-hydroxy-N-[(1S)-1-[4-(4-methyl-1,3-thiazol-5-yl)phenyl]ethyl]pyrrolidine-2-carboxamide (10.60 mg; 0.01 mmol; 4.0 %) as a white solid. LC-MS (ESI+, Method 7): t_{R} = 0.93 min, m/z 1066.4 [M+H]+. 1H NMR (300 MHz, DMSO-d6) 8.98 (s, 1H), 8.52 (s, 1H), 8.34 (s, 2H), 8.20 (s, 1H), 8.08-7.97 (m, 5H), 7.83-7.75 (s, 5H), 7.41-7.32 (m, 5H), 5.09 (s, 1H), 4.90 (s, 1H), 4.50-4.41 (m, 3H), 4.28-4.21 (m, 4H), 4.03 (s, 4H), 3.60-3.45 (m, 10H), 2.50 (s, 3H), 2.00-1.82 (m, 4H), 1.49-1.23 (m, 14H).

### Compound 67

### N-(14-{[2-(2,6-dioxopiperidin-3-yl)-1,3-dioxo-2,3-dihydro-1H-isoindol-4-yl]amino}-3,6,9,12-tetraoxatetradecan-1-yl)-3-methyl-4-[4-(trifluoromethyl)phenyl]-3H,4H-[1,2,3]triazolo[4,5-b]indole-7-carboxamide (Compound 67)

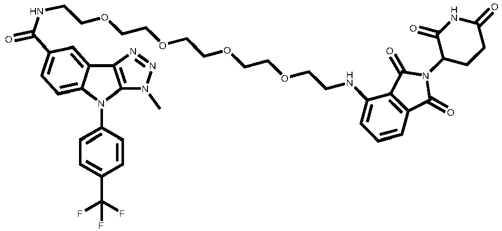

Prepared analogously to **compound** 50, but with 4-(15-amino-4,7,10,13-tetraoxa-1-azapentadecan-1-yl)-2-(2,6-dioxopiperidin-3-yl)-2,3-dihydro-1H-isoindole-1,3-dione (analogously prepared to **intermediate 15,** but using tert-Butyl (14-amino-3,6,9,12-tetraoxatetradecyl)carbamate (commercially available from CombiBlocks Inc. as the amine component instead) and 3-methyl-4-[4-(trifluoromethyl)phenyl]-3H,4H-[1 ,2,3]tri-azolo[4,5-b]indole-7-carboxylic acid (analogously prepared to TEAD ligand 2, but using 1-methyl-1H-1,2,3-triazol-4-amine instead as the amine component in step 1) which gave N-(14-{[2-(2,6-dioxopiperidin-3-yl)-1,3-dioxo-2,3-dihydro-1H-isoindol-4-yl]amino}-3,6,9,12-tetraoxatetra-decan-1-yl)-3-methyl-4-[4-(trifluoromethyl)phenyl]-3H,4H-[1,2, 3]tri-azolo[4,5-b]indole-7-carboxamide (38.2mg; 0.045mmol) as a yellow solid. LC-MS (ESI+, Method 3): t_{R} = 2.27 min, m/z 834.6 [M+H]+. ¹H NMR (500 MHz, DMSO-ds) δ 11.1 (s, 1H), 8.4 (t, *J* = 5.6 Hz, 1H), 8.3 (dd, J = 1.7, 0.7 Hz, 1H), 8.2 - 8.2 (m, 2H), 8.1 (d, *J* = 8.2 Hz, 2H), 7.9 (dd, *J* = 9.0, 1.8 Hz, 1H), 7.6 (dd, J = 8.9, 0.7 Hz, 1H), 7.6 (dd, J = 8.5, 7.1 Hz, 1H), 7.1 (d, J *=* 8.6 Hz, 1H), 7.0 (d, J = 7.0 Hz, 1H), 6.6 (t, J = 5.8 Hz, 1H), 5.0 (dd, J *=* 12.7, 5.4 Hz, 1H), 4.3 (s, 3H), 3.6 (t, J = 5.4 Hz, 2H), 3.5 - 3.5 (m, 14H), 3.5 - 3.4 (m, 4H), 2.9 - 2.8 (m, 1H), 2.6 - 2.5 (m, 2H), 2.1 - 2.0 (m, 1H).

### Compound 63

### 6-{[2-(2,6-dioxopiperidin-3-yl)-1-oxo-2,3-dihydro-1H-isoindol-4-yl]amino}-N-(2-{2-[2-({2-methyl-8-[4-(trifluoromethyl)phenyl]-2H,8H-pyrazolo[3,4-b]indol-5-yl}formamido)ethoxy]ethoxy}ethyl)hexanamide (Compound 63)

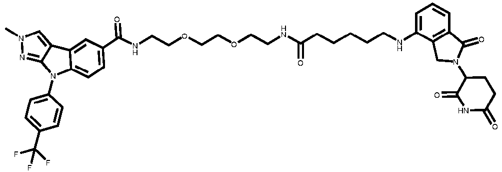

In a 8 ml glass vial, N-{2-[2-(2-aminoethoxy)ethoxy]ethyl}-2-methyl-8-[4-(trifluoromethyl)phenyl]-2H,8H-pyrazolo[3,4-b]indole-5-carboxamide **intermediate 1** (78,5 mg; 0,16 mmol; 1,26 eq.) and 6-{[2-(2,6-dioxopiperidin-3-yl)-1-oxo-2,3-dihydro-1H-isoindol-4-yl]amino}hexanoic acid (prepared analogously to **intermediate 4** using a different alkyl chain length, 47,4 mg; 0,13 mmol; 1,00 eq.) were dissolved in N,N-dimethylformamide (1.5 ml). Then, 4-methylmorpholine (113 µl; 1.02 mmol; 10.00 eq.) and HATU (62.7 mg; 0.17 mmol; 1.3 eq.) were added and the reaction mixture was stirred at rt for 3 h. The crude product (as solution in DMF) was purified by HPLC which gave 6-{[2-(2,6-dioxopiperidin-3-yl)-1-oxo-2,3-dihydro-1H-isoindol-4-yl]amino}-N-(2-{2-[2-({2-methyl-8-[4-(trifluoromethyl)phenyl]-2H,8H-pyrazolo[3,4-b]indol-5-yl}formamido)ethoxy]ethoxy}ethyl)hexanamide (62.5 mg; 0.074mmol) as a colorless solid. LC-MS (ESI+, Method 3): t_{R} = 2.28 min, m/z 844.6 [M+H]+. ¹H NMR (500 MHz, DMSO-*d*₆) δ 11.0 (s, 1H), 8.5 (t, *J* = 5.6 Hz, 1H), 8.3 (d, *J = 1.8* Hz, 1H), 8.2 (s, 1H), 8.1 (d, *J* = 8.9 Hz, 2H), 8.0 - 7.9 (m, 2H), 7.8 (dd, *J* = 8.7, 1.8 Hz, 1H), 7.8 (dd, *J* = 14.6, 7.2 Hz, 2H), 7.3 (t, *J = 7.7* Hz, 1H), 6.9 (dd, *J* = 7.5, 0.8 Hz, 1H), 6.7 (d, *J* = 8.0 Hz, 1H), 5.5 (t, *J* = 5.5 Hz, 1H), 5.1 (dd, *J* = 13.3, 5.1 Hz, 1H), 4.2 (d, *J = 17.1* Hz, 1H), 4.1 (d, *J* = 17.1 Hz, 1H), 4.0 (s, 3H), 3.6 - 3.5 (m, 4H), 3.5 - 3.5 (m, 2H), 3.5 (q, *J =* 6.0 Hz, 2H), 3.4 (t, *J* = 5.9 Hz, 2H), 3.2 (q, *J = 5.8* Hz, 2H), 3.1 (q, *J = 6.6* Hz, 2H), 3.0 - 2.9 (m, 1H), 2.7 - 2.6 (m, 1H), 2.3 (qd, *J* = 13.3, 4.4 Hz, 1H), 2.1 - 2.0 (m, 3H), 1.6 - 1.5 (m, 4H), 1.4 - 1.3 (m, 2H).

### Compound 61

### (2S,4R)-1-[(2S)-3,3-dimethyl-2-(3-{2-[(2-{2-[2-({2-methyl-8-[4-(trifluoromethyl)phenyl]-2H,8H-pyrazolo[3,4-b]indol-5-yl}formamido)-ethoxy]ethoxy}ethyl)carbamoyl]ethoxy}propanam ido)butanoyl]-4-hydroxy-N-{[4-(4-methyl-1,3-thiazol-5-yl)phenyl]methyl}pyrrolidine-2-carboxamide (Compound 61)

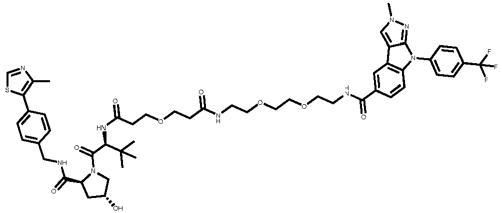

A solution of 3-{2-[(2-{2-[2-({2-methyl-8-[4-(trifluoromethyl)phenyl]-2H,8H-pyrazolo[3,4-b]indol-5-yl}formamido)ethoxy]ethoxy}ethyl)carbamoyl]-ethoxy}propanoic acid **intermediate 38** (180.00 mg; 0.26 mmol; 1.00 eq.), (2S,4R)-1-[(2S)-2-amino-3,3-dimethylbutanoyl]-4-hydroxy-N-{[4-(4-methyl-1,3-thiazol-5-yl)phenyl]methyl}pyrrolidine-2-carboxamide **VHL-Lig** (7.00 mg; 0.01 mmol; 0.05 eq.) and HATU (6.00 mg; 0.01 mmol; 0.06 eq.) in DIEA (6.00 mg; 0.04 mmol; 0.17 eq.) was prepared. The mixture was stirred for 2 hours at room temperature.The residue was purified by HPLC which gave (2S,4R)-1-[(2S)-3,3-dimethyl-2-(3-{2-[(2-{2-[2-({2-methyl-8-[4-(trifluoromethyl)phenyl]-2H,8H-pyrazolo[3,4-b]indol-5-yl}formamido)-ethoxy]ethoxy}ethyl)carbamoyl]ethoxy}propanamido)butanoyl]-4-hydroxy-N-{[4-(4-methyl-1,3-thiazol-5-yl)phenyl]methyl}pyrrolidine-2-carboxamide (59.9 mg; 0.06 mmol) as a white solid. LC-MS (ESI+, Method 2): tR = 0.89 min, m/z 1047.0 [M+H]+. 1H NMR (400 MHz, DMSO-d6) 8.98 (s, 1H), 8.57 (dt, J = 8.9, 5.7 Hz, 2H), 8.34 (d, J = 1.8 Hz, 1H), 8.21 (s, 1H), 8.09 (d, J = 8.4 Hz, 2H), 8.01 C 7.81 (m, 5H), 7.77 (d, J = 8.7 Hz, 1H), 7.39 (q, J = 8.4 Hz, 4H), 5.15 (d, J = 3.5 Hz, 1H), 4.54 (d, J = 9.4 Hz, 1H), 4.47 C 4.38 (m, 2H), 4.34 (s, 1H), 4.21 (dd, J = 15.9, 5.4 Hz, 1H), 4.03 (s, 3H), 3.70 C 3.59 (m, 2H), 3.59 ^{..}C 3.48 (m, 10H), 3.45 (d, J = 5.9 Hz, 2H), 3.40 (t, J = 6.0 Hz, 2H), 3.31 (s, 2H), 2.44 (s, 3H), 2.39 C 2.24 (m, 4H), 2.03 (t, J = 10.2 Hz, 1H), 1.89 (ddd, J = 12.9, 8.6, 4.6 Hz, 1H), 0.92 (s, 9H).

### Compound 51

### (2S,4R)-1-[(2S)-3,3-dimethyl-2-(3-{2-[2-({2-methyl-8-[4-(trifluoromethyl)-phenyl]-2H,8H-pyrazolo[3,4-b]indol-5-yl}formamido)ethoxy]ethoxy}-propanamido)butanoyl]-4-hydroxy-N-{[4-(4-methyl-1,3-thiazol-5-yl)phenyl]methyl}pyrrolidine-2-carboxamide (Compound 51)

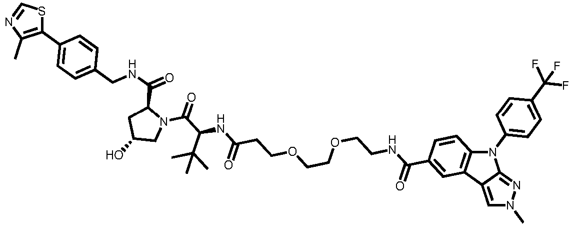

To a stirred mixture of 3-{2-[2-({2-methyl-8-[4-(trifluoromethyl)phenyl]-2H,8H-pyrazolo[3,4-b]indol-5-yl}formamido)ethoxy]ethoxy}propanoic acid **intermediate 39** (275.00 mg; 0.49 mmol; 1.00 eq.), (2S,4R)-1-[(2S)-2-amino-3,3-dimethylbutanoyl]-4-hydroxy-N-{[4-(4-methyl-1,3-thiazol-5-yl)phenyl]methyl}pyrrolidine-2-carboxamide **VHL-Lig** (96%, 264 mg, 0.59 mmol, 1.2 eqv.) and TEA (131 µL, 0.98 mmol, 2.0 eqv.) in DCM (5.00 ml) was added HATU (393.00 mg; 0.98 mmol; 2.00 eq.) at room temperature. The resulting mixture was stirred for 16 h at room temperature. The reaction was quenched with water at room temperature. The resulting mixture was extracted with DCM (3 x 150 mL). The combined organic layers were washed with brine (1x100 mL), dried over anhydrous Na2SO4. After filtration, the filtrate was concentrated under reduced pressure and purified by prep RP-HPLC to afford (2S,4R)-1-[(2S)-3,3-dimethyl-2-(3-{2-[2-({2-methyl-8-[4-(trifluoromethyl)phenyl]-2H,8H-pyrazolo[3,4-b]indol-5-yl}formamido)ethoxy]ethoxy}propanamido)butanoyl]-4-hydroxy-N-{[4-(4-methyl-1,3-thiazol-5-yl)phenyl]methyl}pyrrolidine-2-carboxamide (33.60 mg; 0.04 mmol; 7.4 %) as a white solid. LC-MS (ESI+, Method 7): t_{R} = 0.89 min, m/z 931.3 [M+H]+. 1H NMR (300 MHz, DMSO-d6) 8.97 (s, 1H), 8.56 (dt, J = 9.7, 5.7 Hz, 2H), 8.34 (s, 1H), 8.21 (s, 1H), 8.08 (d, J = 8.4 Hz, 2H), 7.99 - 7.90 (m, 3H), 7.85 (d, J = 8.8 Hz, 1H), 7.77 (d, J = 8.7 Hz, 1H), 7.44 - 7.34 (m, 4H), 5.13 (d, J = 3.5 Hz, 1H), 4.55 (d, J = 9.4 Hz, 1H), 4.48 - 4.31 (m, 3H), 4.21 (dd, J = 15.9, 5.3 Hz, 1H), 4.03 (s, 3H), 3.69 - 3.41 (m, 13H), 2.43 (s, 3H), 2.41 - 2.28 (m, 1H), 2.04 (t, J = 10.9 Hz, 1H), 1.95 - 1.83 (m, 1H), 0.92 (s, 9H).

### Compound 60

### (2S,4R)-1-[(2S)-3,3-dimethyl-2-[7-(2-{2-[2-({2-methyl-8-[4-(trifluoromethyl)phenyl]-2H,8H-pyrazolo[3,4-b]indol-5-yl}formamido)ethoxy]-ethoxy}ethoxy)heptanamido]butanoyl]-4-hydroxy-N-{[4-(4-methyl-1,3-thiazol-5-yl)phenyl]methyl}pyrrolidine-2-carboxamide (Compound 60)

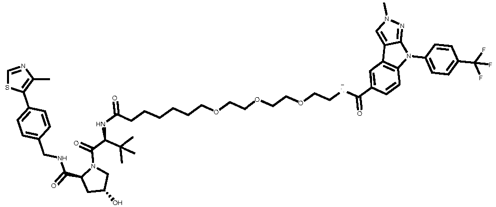

To a stirred solution of 7-(2-{2-[2-({2-methyl-8-[4-(trifluoromethyl)phenyl]-2H,8H-pyrazolo[3,4-b]indol-5-yl}formamido)ethoxy]ethoxy}ethoxy)-heptanoic acid **intermediate 40** (40.000 mg; 0.058 mmol; 1.0 eq.) and (2S,4R)-1-[(2S)-2-amino-3,3-dimethylbutanoyl]-4-hydroxy-N-{[4-(4-methyl-1,3-thiazol-5-yl)phenyl]methyl}pyrrolidine-2-carboxamide **VHL-Lig** (28.000 mg; 0.065 mmol; 1.1 eq.) in DCM (5.000 ml) was added HATU (26.000 mg; 0.065 mmol; 1.1 eq.) and DIEA (40.000 µl; 0.218 mmol; 3.8 eq.) at room temperature under nitrogen atmosphere. The resulting mixture was stirred for 2 h at room temperature under nitrogen atmosphere. The resulting mixture was concentrated under reduced pressure. The resulting mixture was extracted with EtOAc (3 x 50 mL). The combined organic layers were washed with water (3 x 50 mL) dried over anhydrous Na₂SO₄. After filtration, the filtrate was concentrated under reduced pressure. The crude product was purified by by HPLC which gave (2S,4R)-1-[(2S)-3,3-dimethyl-2-[7 -(2-{2-[2-({2-methyl-8-[4-(trifluoromethyl)phenyl]-2H,8H-pyrazolo[3,4-b]indol-5-yl}formamido)ethoxy]-ethoxy}ethoxy)heptanamido]butanoyl]-4-hydroxy-N-{[4-(4-methyl-1,3-thiazol-5-yl)phenyl]methyl}pyrrolidine-2-carboxamide (27.90 mg; 0.027 mmol) as a white solid. LC-MS (ESI+, Method 2): t_{R} = 1.0 min, m/z 1031.5 [M+H]+. 1H NMR (400 MHz, DMSO-d6) 8.98 (s, 1H), 8.54 (dt, J = 11.2, 5.7 Hz, 2H), 8.35 (d, J = 1.8 Hz, 1H), 8.20 (s, 1H), 8.08 (d, J = 8.4 Hz, 2H), 7.97 (d, J = 8.5 Hz, 2H), 7.89- 7.80 (m, 2H), 7.77 (d, J = 8.7 Hz, 1H), 7.40 (q, J = 8.2 Hz, 4H), 5.12 (d, J = 3.5 Hz, 1H), 4.54 (d, J = 9.3 Hz, 1H), 4.48-4.38 (m, 2H), 4.34 (s, 1H), 4.21 (dd, J = 15.8, 5.4 Hz, 1H), 4.03 (s, 3H), 3.71 -3.59 (m, 2H), 3.59- 3.53 (m, 5H), 3.53 - 3.44 (m, 5H), 3.42 (dd, J = 5.9, 3.7 Hz, 2H), 3.30 (s, 2H), 2.44 (s, 3H), 2.23 (dt, J = 14.7, 7.6 Hz, 1H), 2.14 - 1.98 (m, 2H), 1.95- 1.84 (m, 1H), 1.54 - 1.38 (m, 4H), 1.21 (s, 4H), 0.92 (s, 9H).

### Compound 59

### (2R,4S)-1-[(2R)-3,3-dimethyl-2-{7-[2-({2-methyl-8-[4-(trifluoromethyl)-phenyl]-2H,8H-pyrazolo[3,4-b]indol-5-yl}formamido)ethoxy]heptanamido}-butanoyl]-4-hydroxy-N-{[4-(4-methyl-1,3-thiazol-5-yl)phenyl]methyl}-pyrrolidine-2-carboxamide (Compound 59)

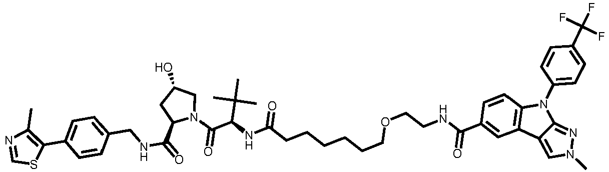

To a stirred mixture of 7-[2-({2-methyl-8-[4-(trifluoromethyl)phenyl]-2H,8H-pyrazolo[3,4-b]indol-5-yl}formamido)ethoxy]heptanoic acid **intermediate 41** (257.00 mg; 0.47 mmol; 1.00 eq.), (2R,4S)-1-[(2R)-2-amino-3,3-dimethylbutanoyl]-4-hydroxy-N-{[4-(4-methyl-1,3-thiazol-5-yl)phenyl]-methyl}pyrrolidine-2-carboxamide **VHL-Lig** (272.00 mg; 0.56 mmol; 1.20 eq.)in DCM (10.00 ml) was added HATU (375.00 mg; 0.94 mmol; 2.00 eq.), Et₃N (0.13 ml; 0.94 mmol; 2.00 eq.) at room temperature. The resulting mixture was stirred for 16 h at room temperature. The reaction mixture was purified by prep RP-HPLC to afford (2R,4S)-1-[(2R)-3,3-dimethyl-2-{7-[2-({2-methyl-8-[4-(trifluoromethyl)phenyl]-2H,8H-pyrazolo[3,4-b]indol-5-yl}formamido)ethoxy]heptanamido}butanoyl]-4-hydroxy-N-{[4-(4-methyl-1,3-thiazol-5-yl)phenyl]methyl}pyrrolidine-2-carboxamide (55.50 mg; 0.06 mmol; 12.3 %) as a white solid. LC-MS (ESI+, Method 7): tR = 0.95 min, m/z 943.4 [M+H]+. 1H NMR (300 MHz, DMSO-d6) 8.99 (s, 1H), 8.60 - 8.47 (m, 2H), 8.34 (s, 1H), 8.21 (s, 1H), 8.09 (d, J = 8.5 Hz, 2H), 7.97 (d, J = 8.6 Hz, 2H), 7.85 (d, J = 8.7 Hz, 2H), 7.77 (d, J = 8.8 Hz, 1H), 7.44 - 7.36 (m, 4H), 4.54 (d, J = 9.3 Hz, 1H), 4.47 - 4.38 (m, 2H), 4.34 (s, 1H), 4.21 (dd, J = 15.8, 5.4 Hz, 2H), 3.65 (s, 2H), 3.52 - 3.37 (m, 6H), 2.44 (s, 3H), 2.25 (dt, J = 14.6, 7.5 Hz, 1H), 2.16 - 1.98 (m, 2H), 1.95 - 1.82 (m, 1H), 1.48 (s, 4H), 1.27 (s, 4H), 0.92 (s, 9H).

### Compound 57

### [(2S)-3,3-dimethyl-2-[1-({2-methyl-8-[4-(trifluoromethyl)phenyl]-2H,8H-pyrazolo[3,4-b]indol-5-yl}formamido)-3,6,9,12-tetraoxahexadecan-16-amido]butanoyl]-4-hydroxy-N-{[4-(4-methyl-1,3-thiazol-5-yl)phenyl]-methyl}pyrrolidine-2-carboxamide (Compound 57)

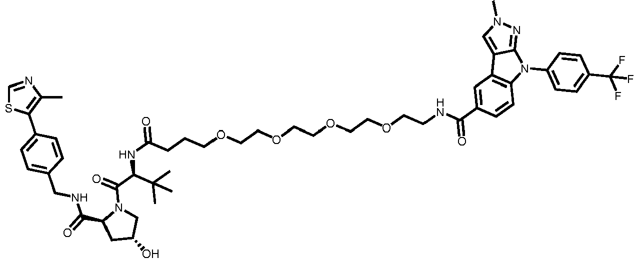

Prepared and purified as described for **compound 59,** but using intermediate **intermediate 43** instead of **intermediate 41** and DCM instead of DMF which gave (2S,4R)-1-[(2S)-3,3-dimethyl-2-[1-({2-methyl-8-[4-(trifluoromethyl)phenyl]-2H,8H-pyrazolo[3,4-b]indol-5-yl}formamido)-3,6,9,12-tetraoxahexadecan-16-amido]butanoyl]-4-hydroxy-N-{[4-(4-methyl-1,3-thiazol-5-yl)phenyl]methyl}pyrrolidine-2-carboxamide (84.80 mg; 0.08 mmol). As a white solid. LC-MS (ESI+, Method 2): t_{R} = 0.8 min, m/z 1044.5 [M+H]+. 1H NMR (400 MHz, DMSO-d6),8.98 (s, 1H), 8.57 (dt, J = 9.2, 5.8 Hz, 2H), 8.35 (d, J = 1.8 Hz, 1H), 8.21 (s, 1H), 8.09 (d, J = 8.5 Hz, 2H), 7.97 (d, J = 8.5 Hz, 2H), 7.92 C 7.83 (m, 2H), 7.77 (d, J = 8.7 Hz, 1H), 7.40 (q, J = 8.4 Hz, 4H), 5.14 (s, 1H), 4.53 (d, J = 9.3 Hz, 1H), 4.48 C 4.39 (m, 2H), 4.34 (s, 1H), 4.21 (dd, J = 15.9, 5.4 Hz, 1H), 4.03 (s, 3H), 3.65 (d, J = 4.5 Hz, 2H), 3.57 C 3.41 (m, 16H), 3.33 (d, J = 6.6 Hz, 2H), 2.44 (s, 3H), 2.31 C 2.22 (m, 1H), 2.16 (ddd, J = 14.7, 8.1, 6.6 Hz, 1H), 2.09 C 1.97 (m, 1H), 1.89 (ddd, J = 12.9, 8.7, 4.6 Hz, 1H), 1.68 (ddt, J = 13.5, 8.5, 6.9 Hz, 2H), 0.93 (s, 9H).

### Compound 56

### 2S,4R)-1-[(2S)-3,3-dimethyl-2-(10-{2-[2-({2-methyl-8-[4-(trifluoromethyl)-phenyl]-2H,8H-pyrazolo[3,4-b]indol-5-yl}formamido)ethoxy]ethoxy}decan-amido)butanoyl]-4-hydroxy-N-{[4-(4-methyl-1,3-thiazol-5-yl)phenyl]-methyl}pyrrolidine-2-carboxamide (Compound 56)

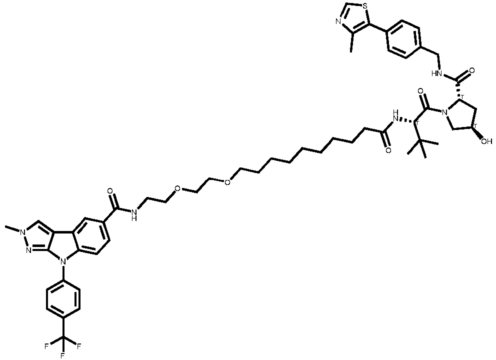

In a 4 ml glass vial sodium 2-methyl-8-[4-(trifluoromethyl)phenyl]-2H,8H-pyrazolo[3,4-b]indole-5-carboxylate **TEAD ligand 1** (4.9 mg; 0.01 mmol; 1.00 eq.), (2S,4R)-1-[(2S)-2-{10-[2-(2-aminoethoxy)ethoxy]decanamido}-3,3-dimethylbutanoyl]-4-hydroxy-N-{[4-(4-methyl-1,3-thiazol-5-yl)phenyl]-methyl}pyrrolidine-2-carboxamide (prepared in analogy to **intermediate 60,** but with (2S,4R)-1-((S)-2-amino-3,3-dimethyl-butyryl)-4-hydroxy-pyrrolidine-2-carboxylic acid 4-(4-methyl-thiazol-5-yl)-benzylamide instead of (2S,4S)-1-((S)-2-amino-3,3-dimethyl-butyryl)-4-hydroxy-pyrrolidine-2-carboxylic acid 4-(4-methyl-thiazol-5-yl)-benzylamide in step 3 instead, 8.9 mg; 0.01 mmol; 1.00 eq.) and HATU (5.9 mg; 0.02 mmol; 1.20 eq.) were dissolved in DMF (400.0 µl). NMM (17.3 µl; 0.16 mmol; 12.00 eq.) was added and the reaction mixture was stirred for 2 h at rt. Then, the product (in solution in DMF) was purified by HPLC. The target fractions were pooled together and lyophilized which gave (2S,4R)-1-[(2S)-3,3-dimethyl-2-(10-{2-[2-({2-methyl-8-[4-(trifluoromethyl)phenyl]-2H,8H-pyrazolo[3,4-b]indol-5-yl}formamido)ethoxy]ethoxy}decanamido)butanoyl]-4-hydroxy-N-{[4-(4-methyl-1,3-thiazol-5-yl)phenyl]methyl}pyrrolidine-2-carboxamide (7,2 mg; 0.007mmol) as a colorless solid. LC-MS (ESI+, Method 3): t_{R} = 2.67 min, m/z 515.0 [M+2H]2+. 1H NMR (400 MHz, DMSO-d6)8.98 (s, 1H), 8.56-8.50(m, 2H), 8.34 (d, J = 1.8 Hz, 1H), 8.20 (s, 1H), 8.08 (d, J = 8.4 Hz, 2H), 7.96 (d, J = 8.4 Hz, 2H), 7.90-7.74 (m, 3H), 7.39 (q, J = 8.2 Hz, 4H), 5.11 (d, J = 3.6 Hz, 1H), 4.53 (d, J = 9.4 Hz, 1H), 4.47-4.39 (m, 2H), 4.34 (s, 1H), 4.24-4.18 (m, 1H), 4.03 (s, 3H), 3.65 (d, J = 5.9 Hz, 2H), 3.59-3.52 (m, 4H), 3.50-3.41 (m, 4H), 3.35 (d, J = 6.6 Hz, 2H), 2.44 (s, 3H), 2.27-2.19 (m, 1H), 2.11-2.00 (m, 2H), 1.95-1.84 (m, 1H), 1.42 (d, J = 8.1 Hz, 4H), 1.17 (s, 10H), 0.92 (s, 9H).

### Compound 55

### N-[(2S)-1-[(2S,4R)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]-N'-(2-{2-[2-({2-methyl-4-[5-(trifluoromethyl)pyridin-2-yl]-2H,4H-[1,2,3]triazolo[4,5-b]indol-7-yl}formamido)ethoxy]ethoxy}ethyl)-heptanediamide (Compound 55)

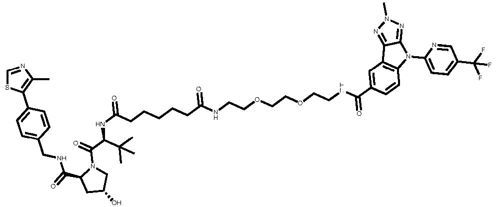

To a stirred solution of 6-[(2-{2-[2-({2-methyl-4-[5-(trifluoromethyl)pyridin-2-yl]-2H,4H-[1,2,3]triazolo[4,5-b]indol-7-yl}formamido)ethoxy]ethoxy}-ethyl)carbamoyl]hexanoic acid **intermediate 45** (200.0 mg; 0.32 mmol; 1.00 eq.) and (2S,4R)-1-[(2S)-2-amino-3,3-dimethylbutanoyl]-4-hydroxy-N-{[4-(4-methyl-1,3-thiazol-5-yl)phenyl]methyl}pyrrolidine-2-carboxamide **VHL-Lig** (136.00 mg; 0.32 mmol; 1.00 eq.) in dichloromethane (20.00 ml) was added HATU (126.00 mg; 0.31 mmol; 1.00 eq.) and diisopropylethylamine (180.00 µl; 0.98 mmol; 3.11 eq.) . The mixture was stirred for 2h at 25 °C. The resulting mixture was extracted. After filtration, the filtrate was concentrated under reduced pressure. The crude product was purified by prep. RP-HPLC to provide N-[(2S)-1-[(2S,4R)-4-hydroxy-2-({[4-(4-methyl-1,3-thiazol-5-yl)phenyl]methyl}carbamoyl)pyrrolidin-1-yl]-3,3-dimethyl-1-oxobutan-2-yl]-N'-(2-{2-[2-({2-methyl-4-[5-(trifluoromethyl)-pyridin-2-yl]-2H,4H-[1,2,3]triazolo[4,5-b]indol-7-yl}formamido)ethoxy]-ethoxy}ethyl)heptanediamide (15.40 mg; 0.01 mmol) as a white solid. LC-MS (ESI+, Method 1): t_{R} = 0.96 min, m/z 1046.4 [M+H]+.1H NMR (300 MHz, DMSO-d6) 9.08 (d, J = 8.9 Hz, 1H), 9.00 (d, J = 12.4 Hz, 2H), 8.71 (d, J = 5.7 Hz, 1H), 8.61- 8.46 (m, 3H), 8.39 (d, J = 8.8 Hz, 1H), 8.08 (dd, J = 8.9, 1.9 Hz, 1H), 7.89-7.77 (m, 2H), 7.46- 7.33 (m, 4H), 5.13 (d, J = 3.5 Hz, 1H), 4.53 (d, J = 9.4 Hz, 1H), 4.43 (d, J = 5.2 Hz, 5H), 4.34 (s, 1H), 4.21 (dd, J = 15.7, 5.4 Hz, 1H), 3.65 (s, 2H), 3.62- 3.44 (m, 8H), 3.39 (t, J = 6.0 Hz, 2H), 3.18 (t, J = 5.8 Hz, 2H), 2.43 (s, 3H), 2.26-2.16 (m, 1H), 2.07 (dt, J = 27.2, 7.0 Hz, 4H), 1.89 (t, J = 12.8 Hz, 1H), 1.45 (s, 4H), 1.26- 1.13 (m, 2H), 0.92 (s, 9H).

### Compound 54

### (2S,4R)-1-[(2S)-3,3-dimethyl-2-(2-{2-[2-({2-methyl-8-[4-(trifluoromethyl)-phenyl]-2H,8H-pyrazolo[3,4-b]indol-5-yl}formamido)ethoxy]ethoxy}-acetamido)butanoyl]-4-hydroxy-N-{[4-(4-methyl-1,3-thiazol-5-yl)phenyl]-methyl}pyrrolidine-2-carboxamide (Compound 54)

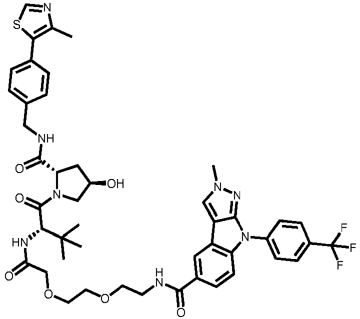

A solution of 2-{2-[2-({2-methyl-8-[4-(trifluoromethyl)phenyl]-2H,8H-pyrazolo[3,4-b]indol-5-yl}formamido)ethoxy]ethoxy}acetic acid **intermediate 46** (250.00 mg; 0.50 mmol; 1.00 eq.), (2S,4R)-1-[(2S)-2-amino-3,3-dimethylbutanoyl]-4-hydroxy-N-{[4-(4-methyl-1,3-thiazol-5-yl)phenyl]-methyl}pyrrolidine-2-carboxamide **VHL-Lig** (213.00 mg; 0.49 mmol; 1.00 eq.), EDCI (105.00 mg; 0.55 mmol; 1.11 eq.) and DIEA (264.00 µL; 1.41 mmol; 2.85 eq.) in HOBt (175.00 mg; 1.23 mmol; 2.48 eq.) was stirred for overnights at degrees room temperature under DCM (10.00 ml) atmosphere. The crude product was purified by prep RP-HPLC to afford (2S,4R)-1-[(2S)-3,3-dimethyl-2-(2-{2-[2-({2-methyl-8-[4-(trifluoromethyl)-phenyl]-2H,8H-pyrazolo[3,4-b]indol-5-yl}formamido)ethoxy]ethoxy}acetamido)butanoyl]-4-hydroxy-N-{[4-(4-methyl-1,3-thiazol-5-yl)phenyl]-methyl}pyrrolidine-2-carboxamide (52.10 mg; 0.06 mmol; 11.2 %) as a white solid. LC-MS (ESI+, Method 2): t_{R} = 0.95 min, m/z 918.0 [M+H]+. 1H NMR (400 MHz, DMSO-d6) 8.95 (s, 1H), 8.57 (dt, J = 11.7, 5.7 Hz, 2H), 8.33 (d, J = 1.8 Hz, 1H), 8.20 (s, 1H), 8.08 (d, J = 8.4 Hz, 2H), 7.97 (d, J = 8.6 Hz, 2H), 7.84 (dd, J = 8.8, 1.8 Hz, 1H), 7.76 (d, J = 8.7 Hz, 1H), 7.47 (d, J = 9.5 Hz, 1H), 7.38 (s, 4H), 5.18 (d, J = 3.5 Hz, 1H), 4.58 (d, J = 9.6 Hz, 1H), 4.45 (t, J = 8.1 Hz, 1H), 4.39 4.34 (m, 2H), 4.26 (d, J = 5.6 Hz, 1H), 4.01 (d, J = 19.6 Hz, 4H), 3.66 3.58 (m, 8H), 3.49 (t, J = 5.7 Hz, 2H), 2.42 (s, 3H), 2.06 (s, 1H), 1.93 1.88 (m, 1H), 0.94 (s, 9H).

### Compound 53

### N-[(2S)-1-[(2S,4R)-4-hydroxy-2-({[4-(4-methyl-1,3-thiazol-5-yl)phenyl]-methyl}carbamoyl)pyrrolidin-1-yl]-3,3-dimethyl-1-oxobutan-2-yl]-N'-methyl-N'-(2-{2-[2-({2-methyl-8-[4-(trifluoromethyl)phenyl]-2H,8H-pyrazolo[3,4-b]indol-5-yl}formamido)ethoxy]ethoxy}ethyl)heptanediamide (Compound 53)

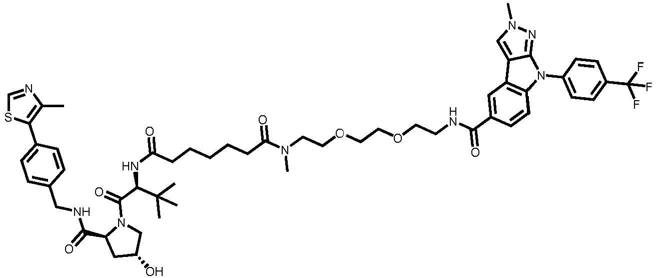

To a stirred solution of 6-[methyl(2-{2-[2-({2-methyl-8-[4-(trifluoromethyl)-phenyl]-2H,8H-pyrazolo[3,4-b]indol-5-yl}formamido)ethoxy]ethoxy}ethyl)-carbamoyl]hexanoic acid **intermediate 47** (prepared analogously to **intermediate 45,** 200.00 mg; 0.28 mmol; 1.00 eq.) and (2S,4R)-1-[(2S)-2-amino-3,3-dimethylbutanoyl]-4-hydroxy-N-{[4-(4-methyl-1,3-thiazol-5-yl)-phenyl]methyl}pyrrolidine-2-carboxamide (commercially available from Sigma-Aldrich) (134.00 mg; 0.31 mmol; 1.10 eq.) in DCM (20.00 ml) was added HATU (124.00 mg; 0.31 mmol; 1.09 eq.) and DIEA (160.00 µl; 0.87 mmol; 3.08 eq.). The mixture was stirred for 2h at 25 degree. The resulting mixture was extracted. After filtration, the filtrate was concentrated under reduced pressure. The crude product was purified by Prep RP-HPLC to afford N-[(2S)-1-[(2S,4R)-4-hydroxy-2-({[4-(4-methyl-1,3-thiazol-5-yl)-phenyl]methyl}carbamoyl)pyrrolidin-1-yl]-3,3-dimethyl-1-oxobutan-2-yl]-N'-methyl-N'-(2-{2-[2-({2-methyl-8-[4-(trifluoromethyl)phenyl]-2H,8H-pyrazolo[3,4-b]indol-5-yl}formamido)ethoxy]ethoxy}ethyl)heptanediamide (67.10 mg; 0.06 mmol; 22.1 %) as a white solid. LC-MS (ESI+, Method 2): t_{R} = 0.94 min, m/z 1058.4 [M+H]+. 1H NMR (300 MHz, DMSO-d6) 8.98 (d, J = 0.9 Hz, 1H), 8.61- 8.51 (m, 2H), 8.35 (s, 1H), 8.21 (s, 1H), 8.09 (d, J = 8.4 Hz, 2H), 7.97 (d, J = 8.6 Hz, 2H), 7.90- 7.81 (m, 2H), 7.77 (d, J = 8.2 Hz, 1H), 7.46 -7.33 (m, 4H), 5.12 (d, J = 3.6 Hz, 1H), 4.53 (d, J = 9.3 Hz, 1H), 4.49- 4.36 (m, 2H), 4.34 (s, 1H), 4.21 (dd, J = 16.0, 5.5 Hz, 1H), 4.03 (s, 3H), 3.65 (s, 2H), 3.54 (q, J = 5.3 Hz, 7H), 3.45 (q, J = 5.4, 4.8 Hz, 4H), 3.40 (d, J = 5.6 Hz, 2H), 2.93 (s, 1H), 2.78 (s, 1H), 2.44 (d, J = 1.4 Hz, 3H), 2.32 - 1.96 (m, 6H), 1.91 (dd, J = 8.5, 4.5 Hz, 1H), 1.50-1.39 (m, 4H), 1.25-1.15 (m, 2H), 0.92 (s, 9H).

### Compound 52

### 2S,4R)-1-[(2S)-3,3-dimethyl-2-[2-(2-{2-[2-({2-methyl-8-[4-(trifluoromethyl)-phenyl]-2H,8H-pyrazolo[3,4-b]indol-5-yl}formamido)ethoxy]-ethoxy}ethoxy)acetamido]butanoyl]-4-hydroxy-N-{[4-(4-methyl-1,3-thiazol-5-yl)phenyl]methyl}pyrrolidine-2-carboxamide (Compound 52)

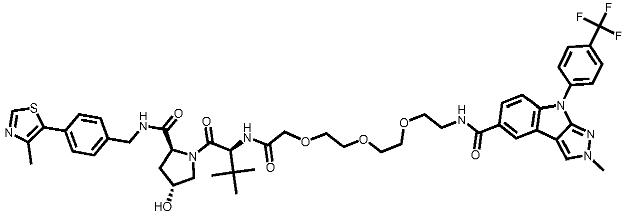

A solution of 2-(2-{2-[2-({2-methyl-8-[4-(trifluoromethyl)phenyl]-2H,8H-pyrazolo[3,4-b]indol-5-yl}formamido)ethoxy]ethoxy}ethoxy)acetic acid (prepared analogously to **intermediate 46,** but using tert-butyl 2-{2-[2-(2-aminoethoxy)ethoxy]ethoxy}acetate as the amine coupling partner in step 1 instead, 190.00 mg; 0.28 mmol; 1.00 eq.),(2S,4R)-1-[(2S)-2-amino-3,3-dimethylbutanoyl]-4-hydroxy-N-{[4-(4-methyl-1,3-thiazol-5-yl)phenyl]-methyl}pyrrolidine-2-carboxamide **VHL-Lig** (150.00 mg; 0.33 mmol; 1.19 eq.), EDCI (73.10 mg; 0.38 mmol; 1.38 eq.) and DIEA (134.00 mg; 0.99 mmol; 3.56 eq.) in HOBT (52.00 mg; 0.37 mmol; 1.32 eq.) DCM (10.00 ml) was stirred for 18 h at room temperature. The crude product was purified by Prep RP-HPLC to afford (2S,4R)-1-[(2S)-3,3-dimethyl-2-[2-(2-{2-[2-({2-methyl-8-[4-(trifluoromethyl)phenyl]-2H,8H-pyrazolo[3,4-b]indol-5-yl}formamido)ethoxy]ethoxy}ethoxy)acetamido]butanoyl]-4-hydroxy-N-{[4-(4-methyl-1,3-thiazol-5-yl)phenyl]methyl}pyrrolidine-2-carboxamide (83.00 mg; 0.09 mmol; 30.7 %) as a white solid. LC-MS (ESI+, Method 2): t_{R} = 0.95 min, m/z 962.0 [M+H]+. 1H NMR (400 MHz, DMSO-d6) 8.97 (s, 1H), 8.61 (t, J = 6.0 Hz, 1H), 8.54 (t, J = 5.5 Hz, 1H), 8.34 (d, J = 1.8 Hz, 1H), 8.20 (s, 1H), 8.08 (d, J = 8.5 Hz, 2H), 7.97 (d, J = 8.6 Hz, 2H), 7.85 (dd, J = 8.8, 1.9 Hz, 1H), 7.76 (d, J = 8.6 Hz, 1H), 7.38 (s, 5H), 5.16 (s, 1H), 4.56 (d, J = 9.6 Hz, 1H), 4.49 ^{..}C 4.31 (m, 3H), 4.25 (dd, J = 15.8, 5.7 Hz, 1H), 4.03 (s, 3H), 3.95 (s, 2H), 3.63 , 3.52 (m, 12H), 3.44 (q, J = 6.0 Hz, 2H), 2.43 (s, 3H), 2.05 (t, J = 9.4 Hz, 1H), 1.90 (ddd, J = 13.0, 8.9, 4.5 Hz, 1H), 0.93 (s, 9H).

### Compound 50

### N-(2-{2-[2-(2-{[2-(2,6-dioxopiperidin-3-yl)-1,3-dioxo-2,3-dihydro-1H-isoindol-4-yl]amino}ethoxy)ethoxy]ethoxy}ethyl)-2-methyl-8-[4-(trifluoromethyl)phenyl]-2H,8H-pyrazolo[3,4-b]indole-5-carboxamide (Compound 50)

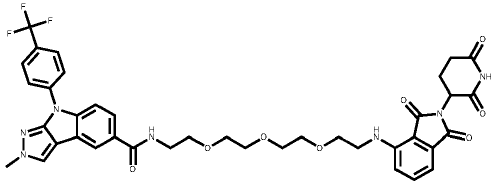

In a 8 ml vial with stir bar were placed 2-methyl-8-[4-(trifluoromethyl)phenyl]-2H,8H-pyrazolo[3,4-b]indole-5-carboxylic acid **TEAD ligand 1 free acid** (40.06 mg; 0.11 mmol; 1.00 eq.), 4-(12-amino-4,7,10-trioxa-1-azadodecan-1-yl)-2-(2,6-dioxopiperidin-3-yl)-2,3-dihydro-1H-iso-indole-1,3-dione (prepared analogously to **intermediate 15,** 50.00 mg; 0.11 mmol; 1.00 eq.)and 4-Methylmorpholine (49.03 µl; 0.45 mmol; 4.00 eq.)). The reactants were dissolved in N,N-Dimethylformamide, anhydrous, 99.8% (1.00 ml)), followed by addition HATU (50.87 mg; 0.13 mmol; 1.20 eq.) The reaction mixture was stirred at RT for 18 h. The reaction mixture was filtered and directly injected into a prep RP-HPLC-MS system.The target fractions were lyophilized which gave N-(2-{2-[2-(2-{[2-(2,6-dioxopiperidin-3-yl)-1,3-dioxo-2,3-dihydro-1H-isoindol-4-yl]-amino}ethoxy)ethoxy]ethoxy}ethyl)-2-methyl-8-[4-(trifluoromethyl)phenyl]-2H,8H-pyrazolo[3,4-b]indole-5-carboxamide (17.0 mg; 0.02 mmol) as a solid. LC-MS (ESI+, Method 3): t_{R} = 2.44 min, m/z 789.6 [M+H]+. ¹H NMR (500 MHz, DMSO-*d*₆) δ 11.1 (s, 1H), 8.5 (t, J = 5.5 Hz, 1H), 8.3 (d, *J* = 1.9 Hz, 1H), 8.2 (s, 1H), 8.1 (d, J = 8.2 Hz, 2H), 8.0 (d, J = 8.4 Hz, 2H), 7.9 (dd, J = 8.7, 1.9 Hz, 1H), 7.8 (d, J = 8.7 Hz, 1H), 7.6 = 7.5 (m, 1H), 7.1 (d, *J* = 8.6 Hz, 1H), 7.0 (d, *J* = 7.1 Hz, 1H), 6.6 -6.5 (m, 1H), 5.0 (dd, *J* = 12.8, 5.4 Hz, 1H), 4.0 (s, 3H), 3.6 - 3.5 (m, 12H), 3.5 (t, J = 5.9 Hz, 2H), 3.4 (d, *J* = 5.0 Hz, 2H), 2.9 (ddd, J = 16.7, 13.7, 5.3 Hz, 1H), 2.6 - 2.5 (m, 2H), 2.1 - 2.0 (m, 1H).

### Compound 49

### N-{2-[2-(2-{[2-(2,6-dioxopiperidin-3-yl)-1,3-dioxo-2,3-dihydro-1H-isoindol-4-yl]amino}ethoxy)ethoxy]ethyl}-2-methyl-8-[4-(trifluoromethyl)phenyl]-2H,8H-pyrazolo[3,4-b]indole-5-carboxamide (Compound 49)

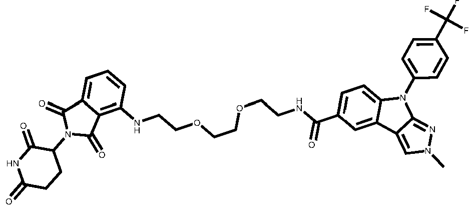

Prepared analogously to **compound 50** with a different amine coupling partner (4-({2-[2-(2-aminoethoxy)ethoxy]ethyl}amino)-2-(2,6-dioxopiperi-din-3-yl)-2,3-dihydro-1H-isoindole-1,3-dione, prepared analogously to **intermediate 15)** which gave N-{2-[2-(2-{[2-(2,6-dioxopiperidin-3-yl)-1,3-dioxo-2,3-dihydro-1H-isoindol-4-yl]amino}ethoxy)ethoxy]ethyl}-2-methyl-8-[4-(trifluoromethyl)phenyl]-2H,8H-pyrazolo[3,4-b]indole-5-carboxamide (59.0 mg; 0.08 mmol) as a solid. LC-MS (ESI+, Method 3): t_{R} = 2.45 min, m/z 745.6 [M+H]+. ¹H NMR (500 MHz, DMSO-*d*₆) δ 11.1 (s, 1H), 8.5 (t, J = 5.6 Hz, 1H), 8.3 (d, J = 1.8 Hz, 1H), 8.2 (s, 1H), 8.1 (d, J = 8.4 Hz, 2H), 8.0 (d, J = 8.6 Hz, 2H), 7.8 (dd, J = 8.7, 1.9 Hz, 1H), 7.7 (d, J = 8.7 Hz, 1H), 7.5 (dd, J = 8.6, 7.1 Hz, 1H), 7.0 (d, J = 8.6 Hz, 1H), 7.0 (d, *J = 7.0* Hz, 1H), 6.6 (t, J = 5.8 Hz, 1H), 5.0 (dd, J = 12.7, 5.5 Hz, 1H), 4.0 (s, 3H), 3.7 - 3.6 (m, 4H), 3.6 (s, 4H), 3.5 (q, J = 5.9 Hz, 2H), 3.4 (q, J = 5.6 Hz, 2H), 2.9 - 2.8 (m, 1H), 2.6 - 2.5 (m, 2H), 2.1 - 2.0 (m, 1H).

### Compound 48

### 2-methyl-N-(14-{[2-(1-methyl-2,6-dioxopiperidin-3-yl)-1,3-dioxo-2,3-dihydro-1H-isoindol-4-yl]amino}-3,6,9,12-tetraoxatetradecan-1-yl)-8-[4-(trifluoromethyl)phenyl]-2H,8H-pyrazolo[3,4-b]indole-5-carboxamide (Compound 48)

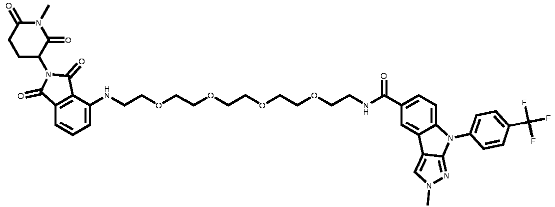

Prepared analogously to **compound 50** with a different amine coupling partner (4-(15-amino-4,7,10,13-tetraoxa-1-azapentadecan-1-yl)-2-(1-methyl-2,6-dioxopiperidin-3-yl)-2,3-dihydro-1H-isoindole-1,3-dione; trifluoroacetic acid, prepared analogously to **intermediate** 15) which gave 2-methyl-N-(14-{[2-(1-methyl-2,6-dioxopiperidin-3-yl)-1,3-dioxo-2,3-di-hydro-1H-isoindol-4-yl]amino}-3,6,9,12-tetraoxatetradecan-1-yl)-8-[4-(trifluoromethyl)phenyl]-2H,8H-pyrazolo[3,4-b]indole-5-carboxamide (52.0 mg; 0.06 mmol) as a yellow solid. LC-MS (ESI+, Method 3): t_{R} = 2.51 min, m/z 847.6 [M+H]+. ¹H NMR (500 MHz, DMSO-ds) δ 8.5 (t, J = 5.6 Hz, 1H), 8.3 (d, J = 1.8 Hz, 1H), 8.2 (s, 1H), 8.1 (d, J = 8.4 Hz, 2H), 8.0 (d, J = 8.5 Hz, 2H), 7.9 (dd, J = 8.7, 1.8 Hz, 1H), 7.8 (d, J = 8.7 Hz, 1H), 7.6 (dd, J *=* 8.6, 7.1 Hz, 1H), 7.1 (d, J = 8.6 Hz, 1H), 7.0 (d, J = 7.0 Hz, 1H), 6.6 (t, J *=* 5.8 Hz, 1H), 5.1 (dd, J = 13.0, 5.4 Hz, 1H), 4.0 (s, 3H), 3.6 - 3.4 (m, 19H), 3.0 (s, 3H), 3.0 - 2.9 (m, 1H), 2.7 (ddd, J = 17.2, 4.5, 2.6 Hz, 1H), 2.6 (dd, *J* = 13.0, 4.5 Hz, 2H), 2.0 (dtd, J = 13.1, 5.4, 2.5 Hz, 1H).

### Compound 47

### N-(17-{[2-(2,6-dioxopiperidin-3-yl)-1,3-dioxo-2,3-dihydro-1H-isoindol-4-yl]amino}-3,6,9,12,15-pentaoxaheptadecan-1-yl)-2-methyl-8-[4-(trifluoromethyl)phenyl]-2H,8H-pyrazolo[3,4-b]indole-5-carboxamide (Compound 47)

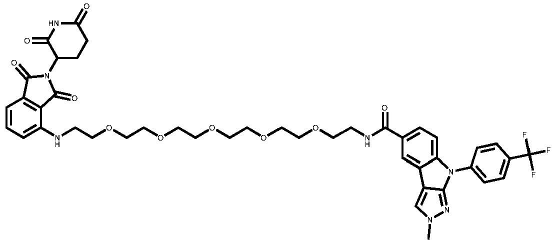

Prepared analogously to **compound 50** with a different amine coupling partner (4-(18-amino-4,7,10,13,16-pentaoxa-1-azaoctadecan-1-yl)-2-(2,6-dioxopiperidin-3-yl)-2,3-dihydro-1H-isoindole-1,3-dione, prepared analogously to **intermediate 15)** which gave N-(17-{[2-(2,6-dioxopiperidin-3-yl)-1,3-dioxo-2,3-dihydro-1H-isoindol-4-yl]amino}-3,6,9,12,15-pentaoxa-heptadecan-1-yl)-2-methyl-8-[4-(trifluoromethyl)phenyl]-2H,8H-pyrazolo[3,4-b]indole-5-carboxamide (13.0 mg; 0.01 mmol) as a yellow lyophilizate. LC-MS (ESI+, Method 3): t_{R} = 2.46 min, m/z 877.9 [M+H]+. 1H NMR (700 MHz, DMSO) d 11.08 (s, 1H), 8.52 (t, J = 5.6 Hz, 1H), 8.34 (d, J = 1.8 Hz, 1H), 8.20 (s, 1H), 8.08 (d, J = 8.3 Hz, 2H), 7.96 (d, J = 8.7 Hz, 2H), 7.85 (dd, J = 8.7, 1.9 Hz, 1H), 7.77 (d, J = 8.6 Hz, 1H), 7.56 (dd, J = 8.5, 7.1 Hz, 1H), 7.11 (d, J = 8.6 Hz, 1H), 7.02 (d, J = 7.0 Hz, 1H), 6.58 (t, J = 5.9 Hz, 1H), 5.05 (dd, J = 12.9, 5.4 Hz, 1H), 4.03 (s, 3H), 3.61 - 3.41 (m, 26H), 2.87 (ddd, J = 17.2, 14.0, 5.5 Hz, 1H), 2.60 - 2.51 (m, 3H), 2.01 (dtd, J = 13.0, 5.4, 2.4 Hz, 1H).

### Compound 45

### N-(14-{[2-(2,6-dioxopiperidin-3-yl)-1,3-dioxo-2,3-dihydro-1H-isoindol-4-yl]amino}-3,6,9,12-tetraoxatetradecan-1-yl)-2-methyl-4-[4-(trifluoromethyl)phenyl]-2H,4H-[1,2,3]triazolo[4,5-b]indole-7-carboxamide (Compound 45)

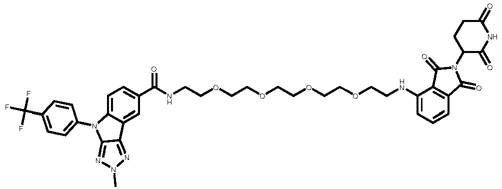

Prepared analogously to **compound 50** with a different acid coupling partner **(TEAD ligand** 2) and a different amine coupling partner (4-(15-amino-4,7,10,13-tetraoxa-1-azapentadecan-1-yl)-2-(2,6-dioxopiperidin-3-yl)-2,3-dihydro-1H-isoindole-1,3-dione; formic acid, prepared analogously to **intermediate 15)** which gave N-(14-{[2-(2,6-dioxopiperidin-3-yl)-1,3-dioxo-2,3-dihydro-1H-isoindol-4-yl]amino}-3,6,9,12-tetraoxatetradecan-1-yl)-2-methyl-4-[4-(trifluoromethyl)phenyl]-2H,4H-[1,2,3]triazolo[4,5-b]-indole-7-carboxamide (21.0 mg; 0.03 mmol) as a yellow lyophilizate. LC-MS (ESI+, Method 3): t_{R} = 2.46 min, m/z 835.2 [M+H]+. ¹H NMR (500 MHz, DMSO-ds) δ 11.1 (s, 1H), 8.6 (t, *J* = 5.5 Hz, 1H), 8.6 (d, *J =* 1.8 Hz, 1H), 8.1 - 8.0 (m, 5H), 7.9 (d, *J =* 8.8 Hz, 1H), 7.5 (dd, *J =* 8.5, 7.1 Hz, 1H), 7.1 (d, *J* = 8.6 Hz, 1H), 7.0 (d, *J* = 7.0 Hz, 1H), 6.6 (t, *J =* 5.8 Hz, 1H), 5.0 (dd, *J* = 12.7, 5.4 Hz, 1H), 4.4 (s, 3H), 3.6 - 3.5 (m, 4H), 3.6 - 3.4 (m, 13H), 3.4 (q, *J* = 5.6 Hz, 2H), 2.9 - 2.8 (m, 1H), 2.6 - 2.5 (m, 1H), 2.6 - 2.5 (m, 2H), 2.1 - 2.0 (m, 1H).

### Compound 43

### N-(14-{[2-(2,6-dioxopiperidin-3-yl)-1-oxo-2,3-dihydro-1H-isoindol-4-yl]-amino}-3,6,9,12-tetraoxatetradecan-1-yl)-2-methyl-8-[4-(trifluoromethyl)-phenyl]-2H,8H-pyrazolo[3,4-b]indole-5-carboxamide (Compound 43)

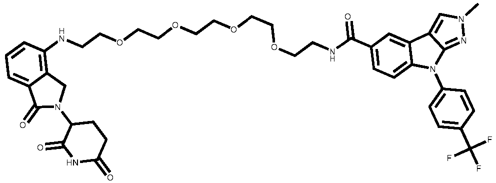

Prepared analogously to **compound 50** with a different amine coupling partner (3-[4-(15-amino-4,7,10,13-tetraoxa-1-azapentadecan-1-yl)-1-oxo-2,3-dihydro-1H-isoindol-2-yl]piperidine-2,6-dione; trifluoroacetic acid, prepared analogously to **intermediate 4)** instead which gave N-(14-{[2-(2,6-dioxopiperidin-3-yl)-1-oxo-2,3-dihydro-1H-isoindol-4-yl]amino}-3,6,9,12-tetraoxatetradecan-1-yl)-2-methyl-8-[4-(trifluoromethyl)phenyl]-2H,8H-pyrazolo[3,4-b]indole-5-carboxamide (32.0 mg; 0.04 mmol) as a colorless solid. LC-MS (ESI+, Method 3): tR = 2.36 min, m/z 819.6 [M+H]+. ¹H NMR (500 MHz, DMSO-*d*₆) δ 11.0 (s, 1H), 8.5 (t, *J* = 5.6 Hz, 1H), 8.3 (d, *J* = 1.8 Hz, 1H), 8.2 (s, 1H), 8.1 (d, *J =* 8.3 Hz, 2H), 8.0 (d, *J* = 8.4 Hz, 2H), 7.9 (dd, *J* = 8.6, 1.9 Hz, 1H), 7.8 (d, *J* = 8.7 Hz, 1H), 7.3 (t, *J* = 7.7 Hz, 1H), 7.0 (d, *J* = 7.4 Hz, 1H), 6.8 (d, *J* = 8.0 Hz, 1H), 5.1 (dd, *J* = 13.2, 5.1 Hz, 1H), 4.2 (d, *J = 17.1* Hz, 1H), 4.1 (d, *J = 17.1* Hz, 1H), 4.0 (s, 3H), 3.6 - 3.4 (m, 18H), 3.3 (t, *J =* 5.9 Hz, 2H), 3.0 - 2.9 (m, 1H), 2.7 - 2.6 (m, 1H), 2.5 (s, 1H), 2.4 - 2.2 (m, 1H), 2.1 - 2.0 (m, 1H).

### Compound 42

### N-(14-{[2-(2,6-dioxopiperidin-3-yl)-1,3-dioxo-2,3-dihydro-1H-isoindol-5-yl]amino}-3,6,9,12-tetraoxatetradecan-1-yl)-2-methyl-8-[4-(trifluoromethyl)phenyl]-2H,8H-pyrazolo[3,4-b]indole-5-carboxamide (Compound 42)

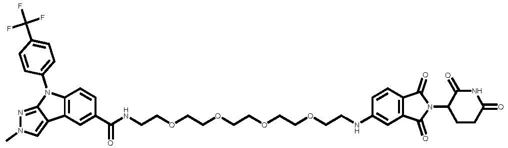

Prepared analogously to **compound 50** with a different amine coupling partner (5-(15-amino-4,7,10,13-tetraoxa-1-azapentadecan-1-yl)-2-(2,6-dioxopiperidin-3-yl)-2,3-dihydro-1H-isoindole-1,3-dione; trifluoroacetic acid, prepared analogously to **intermediate 15)** instead which gave N-(14-{[2-(2,6-dioxopiperidin-3-yl)-1,3-dioxo-2,3-dihydro-1H-isoindol-5-yl]-amino}-3,6,9,12-tetraoxatetradecan-1-yl)-2-methyl-8-[4-(trifluoromethyl)-phenyl]-2H,8H-pyrazolo[3,4-b]indole-5-carboxamide (114.0 mg; 0.14 mmol) as a bright yellow solid. LC-MS (ESI+, Method 3): tR = 2.39 min, m/z 833.6 [M+H]+. ¹H NMR (500 MHz, DMSO-*d*₆) δ 11.1 (s, 1H), 8.5 (t, J = 5.6 Hz, 1H), 8.3 (d, *J* = 1.8 Hz, 1H), 8.2 (s, 1H), 8.1 (d, *J* = 8.3 Hz, 2H), 7.9 (d, *J* = 8.4 Hz, 2H), 7.9 (dd, *J* = 8.7, 1.9 Hz, 1H), 7.8 (d, *J* = 8.7 Hz, 1H), 7.5 (d, *J* = 8.3 Hz, 1H), 7.0 (d, *J = 2.2* Hz, 1H), 6.9 (dd, *J = 8.4,* 2.1 Hz, 1H), 5.0 (dd, *J* = 12.8, 5.4 Hz, 1H), 4.0 (s, 3H), 3.6 - 3.5 (m, 7H), 3.5 - 3.4 (m, 12H), 3.3 (t, J = 5.5 Hz, 2H), 2.9 - 2.8 (m, 1H), 2.6 - 2.5 (m, 2H), 2.0 - 2.0 (m, 1H).

### Compound 87

### N-(20-{[2-(2,6-dioxopiperidin-3-yl)-1,3-dioxo-2,3-dihydro-1H-isoindol-4-yl]amino}-3,6,9,12,15,18-hexaoxaicosan-1-yl)-2-methyl-8-[4-(trifluoromethyl)phenyl]-2H,8H-pyrazolo[3,4-b]indole-5-carboxamide (Compound 87)

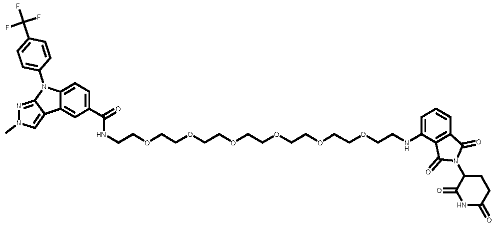

In a 8 ml reaction vial with magnetic stir bar sodium 2-methyl-8-[4-(trifluoromethyl)phenyl]-2H,8H-pyrazolo[3,4-b]indole-5-carboxylate **(TEAD ligand 1)** (40.0 mg; 0.105 mmol; 1.00 eq.), N-(3-dimethylaminopropyl)-N'-ethyl carbodiimide hydrochloride (40.22 mg; 0.210 mmol; 2.00 eq.), 1-hydroxybenzotriazole hydrate (24.1 mg; 0.157 mmol; 1.50 eq.) were dissolved in anhydrous DMF (2.00 ml; 257.20 mmol) and 4-(21-amino-4,7,10,13,16,19-hexaoxa-1-azahenicosan-1-yl)-2-(2,6-dioxo-piperidin-3-yl)-2,3-dihydro-1H-isoindole-1,3-dione dihydrochloride **(intermediate 15)** (122.00 mg; 0.116 mmol; 1.11 eq.) was added, followed by 4-methylmorpholine (57.6 µl; 0.525 mmol; 5.00 eq.). The reaction mixture was stirred overnight at room temperature. The reaction mixture was concentrated under reduced pressure, dissolved in DMSO and purified by prep. RP-HPLC. The product fractions were concentrated under reduced pressure to provide N-(20-{[2-(2,6-dioxopiperidin-3-yl)-1,3-dioxo-2,3-dihydro-1H-isoindol-4-yl]amino}-3,6,9,12,15,18-hexaoxaicosan-1-yl)-2-methyl-8-[4-(trifluoromethyl)phenyl]-2H,8H-pyrazolo[3,4-b]indole-5-carboxamide (85.8 mg; 0.088 mmol) as a solid. LC-MS (ESI+, Method 6): tR = 0.55 min, m/z 922.6 [M+H]+. 1H NMR (700 MHz, DMSO) d 11.09 (s, 1H), 8.52 (t, J = 5.6 Hz, 1H), 8.34 (d, J = 1.8 Hz, 1H), 8.20 (s, 1H), 8.08 (d, J = 8.3 Hz, 2H), 7.96 (d, J = 8.5 Hz, 2H), 7.85 (dd, J = 8.6, 1.9 Hz, 1H), 7.77 (d, J = 8.7 Hz, 1H), 7.56 (dd, J = 8.5, 7.0 Hz, 1H), 7.12 (d, J = 8.6 Hz, 1H), 7.03 (d, J = 7.0 Hz, 1H), 6.58 (t, J = 5.8 Hz, 1H), 5.05 (dd, J = 12.9, 5.5 Hz, 1H), 4.03 (s, 3H), 3.65 - 3.40 (m, 30H), 2.88 (ddd, J = 17.1, 13.9, 5.5 Hz, 1H), 2.61 - 2.51 (m, 2H), 2.01 (dtd, J = 13.0, 5.4, 2.4 Hz, 1H).

### Compound 88

### N-(14-{[2-(2,6-dioxopiperidin-3-yl)-1,3-dioxo-2,3-dihydro-1H-isoindol-5-yl]oxy}-3,6,9,12-tetraoxatetradecan-1-yl)-2-methyl-8-[4-(trifluoromethyl)-phenyl]-2H,8H-pyrazolo[3,4-b]indole-5-carboxamide (Compound 88)

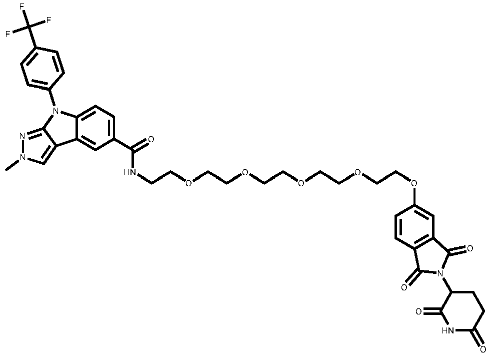

Prepared and purified as described for compound 87, but using intermediate 16 instead of intermediate 15 which gave N-(14-{[2-(2,6-dioxopiperidin-3-yl)-1,3-dioxo-2,3-dihydro-1H-isoindol-5-yl]oxy}-3,6,9,12-tetraoxatetradecan-1-yl)-2-methyl-8-[4-(trifluoromethyl)phenyl]-2H,8H-pyrazolo[3,4-b]indole-5-carboxamide (41.50 mg; 0.049 mmol) as a solid. LC-MS (ESI+, Method 6): tR = 0.53 min, m/z 835.4 [M+H]+. 1H NMR (700 MHz, DMSO) d 11.10 (s, 1H), 8.51 (t, J = 5.6 Hz, 1H), 8.34 (d, J = 1.8 Hz, 1H), 8.20 (s, 1H), 8.08 (d, J = 8.3 Hz, 2H), 7.96 (d, J = 8.6 Hz, 2H), 7.85 (dd, J = 8.7, 1.8 Hz, 1H), 7.80 (d, J = 8.3 Hz, 1H), 7.77 (d, J = 8.7 Hz, 1H), 7.42 (d, J = 2.3 Hz, 1H), 7.33 (dd, J = 8.3, 2.3 Hz, 1H), 5.11 (dd, J = 12.9, 5.5 Hz, 1H), 4.29 - 4.24 (m, 2H), 4.03 (s, 3H), 3.76 - 3.72 (m, 2H), 3.63 - 3.47 (m, 14H), 3.45 (q, J = 6.0 Hz, 2H), 2.88 (ddd, J = 17.1, 14.0, 5.5 Hz, 1H), 2.60 - 2.51 (m, 1H), 2.03 (dtd, J = 13.1, 5.5, 2.4 Hz, 1H).

### Compound 122

### N-(14-{[2-(2,6-dioxopiperidin-3-yl)-1,3-dioxo-2,3-dihydro-1H-isoindol-4-yl]amino}-3,6,9,12-tetraoxatetradecan-1-yl)-9-{[4-(trifluoromethyl)phenyl]-methyl}-9H-pyrido[3,4-b]indole-3-carboxamide (Compound 122)

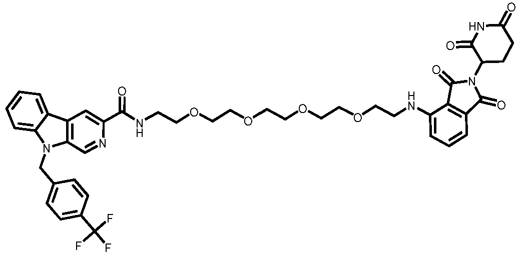

In a 4 mL glass vial with stir bar were placed 4-(15-amino-4,7,10,13-tetraoxa-1-azapentadecan-1-yl)-2-(2,6-dioxopiperidin-3-yl)-2,3-dihydro-1H-isoindole-1,3-dione (prepared analogously to **intermediate 15,** 31,82 mg; 0,054 mmol; 105,00 mol%) which was dissolved in anhydrous DMF (500,00 µl). 4-methylmorpholine (23 µl; 0.207 mmol; 400.0 mol%), 9-{[4-(trifluoromethyl)phenyl]methyl}-9H-pyrido[3,4-b]indole-3-carboxylic acid **intermediate 78** (20.00 mg; 0.052 mmol; 100.00 mol%) and HATU (23.6 mg; 0.062 mmol; 120.00 mol%) were added. The yellow reaction solution was stirred at RT for 18 h. The reaction mixture was purified by prep RP-HPLC-MS and the target fractions lyophilized to give N-(14-{[2-(2,6-dioxopiperidin-3-yl)-1,3-dioxo-2,3-dihydro-1H-isoindol-4-yl]amino}-3,6,9,12-tetraoxatetradecan-1-yl)-9-{[4-(trifluoromethyl)phenyl]methyl}-9H-pyrido[3,4-b]indole-3-carboxamide (14.5 mg). LC-MS (ESI+, Method 1): t_{R} = 1.84 min, m/z 845.2 [M+H]+. 1H NMR (500 MHz, DMSO) 11.08 (s, 1H), 9.08 (d, J = 1.0 Hz, 1H), 8.90 (d, J = 1.0 Hz, 1H), 8.63 (t, J = 5.8 Hz, 1H), 8.47 (dd, J = 7.9, 1.2 Hz, 1H), 7.76 (d, J = 8.3 Hz, 1H), 7.68 - 7.61 (m, 3H), 7.55 (dd, J = 8.6, 7.1 Hz, 1H), 7.39 - 7.33 (m, 3H), 7.09 (d, J = 8.6 Hz, 1H), 7.01 (d, J = 7.0 Hz, 1H), 6.57 (t, J = 5.8 Hz, 1H), 5.97 (s, 2H), 5.04 (dd, J = 12.7, 5.4 Hz, 1H), 3.62 - 3.46 (m, 18H), 3.42 (q, J = 5.6 Hz, 2H), 2.87 (ddd, J = 16.8, 13.7, 5.4 Hz, 1H), 2.61 - 2.52 (m, 2H), 2.01 (ddd, J = 13.0, 5.7, 3.4 Hz, 1H).

### Compound 117

### (2S,4S)-1-[(2S)-3,3-dimethyl-2-(10-{2-[2-({2-methyl-8-[4-(trifluoromethyl)-phenyl]-2H,8H-pyrazolo[3,4-b]indol-5-yl}formamido)ethoxy]ethoxy}decan-amido)butanoyl]-4-hydroxy-N-{[4-(4-methyl-1,3-thiazol-5-yl)phenyl]-methyl}pyrrolidine-2-carboxamide (Compound 117)

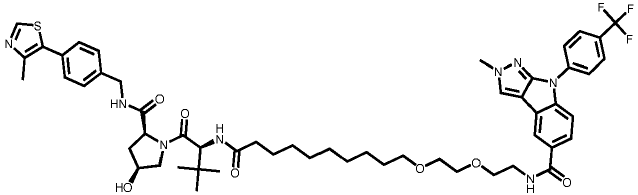

In a 20 mL vial with stir bar (2S,4R)-1-[(2S)-2-{10-[2-(2-aminoethoxy)ethoxy]decanamido}-3,3-dimethylbutanoyl]-4-hydroxy-N-{[4-(4-methyl-1,3-thiazol-5-yl)phenyl]methyl}pyrrolidine-2-carboxamide **intermediate 60** (129.4 mg; 0.13 mmol; 1 equiv.) and **TEAD ligand 1 free acid** were dissolved in DMF (3.0 ml; 38.58 mmol) at RT. Then HATU (99 mg; 0.259 mmol; 2 equiv.) and DIPEA (67 µL; 0.389 mmol; 3 equiv.) were added. The reaction mixture was stirred for 10 min at room temperature. After that was added a solution of N,N-diisopropylethylamine, purified by redistillation, 99.5% (25,125 mg; 0,194 mmol; 150,00 mol%) in N,N-dimethylformamide GR for analysis ACS,ISO,Reag. Ph Eur (3,000 ml; 38,580 mmol; 29.768,75 mol%), followed by N,N-diisopropylethylamine, purified by redistillation, 99.5% (25,125 mg; 0,194 mmol; 150,00 mol%). The reaction mixture was stirred at room temperature overnight. The reaction mixture was filtered and purified by prep. RP-HPLC-MS, the target fractions were combined and concentrated under reduced pressure to give the desired product (15.2 mg, 0.02 mmol, 9%). LC-MS (ESI+, Method 1): tR = 1.89 min, m/z 515.3 [M+2H]2+. 1H NMR (700 MHz, DMSO-d6) δ 9.0 (s, 1H), 8.6 (d, J = 6.0 Hz, 1H), 8.1 (s, 1H), 8.0 (d, J = 20.8 Hz, 1H), 7.8 (d, J = 9.2 Hz, 1H), 7.4 - 7.4 (m, 5H), 5.4 (d, J = 7.1 Hz, 1H), 4.4 (dd, J = 19.7, 7.5 Hz, 2H), 4.4 - 4.3 (m, 1H), 4.3 - 4.2 (m, 2H), 4.0 (s, 1H), 3.9 (d, J = 9.4 Hz, 1H), 3.6 (s, 4H), 2.9 (s, 1H), 2.7 (s, 1H), 2.6 (s, 2H), 2.6 - 2.6 (m, 5H), 2.4 (d, J = 2.3 Hz, 7H), 2.4 (d, J = 3.1 Hz, 6H), 2.3 (s, 4H), 2.2 (dt, J = 14.7, 7.5 Hz, 2H), 2.2 (s, 2H), 2.1 - 2.1 (m, 1H), 1.8 - 1.7 (m, 2H), 1.5 (d, J = 34.7 Hz, 3H), 0.9 (s, 9H).

### Compound 95

### N-(14-{[2-(2,6-dioxopiperidin-3-yl)-1,3-dioxo-2,3-dihydro-1H-isoindol-4-yl]amino}-3,6,9,12-tetraoxatetradecan-1-yl)-2-methyl-4-[(1r,4r)-4-(trifluoromethyl)cyclohexyl]-2H,4H-[1,2,3]triazolo[4,5-b]indole-7-carboxamide (Compound 95)

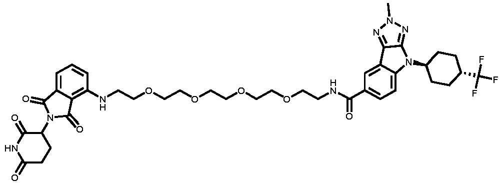

In a 8 ml vial with stir bar were placed 4-(15-amino-4,7,10,13-tetraoxa-1-azapentadecan-1-yl)-2-(2,6-dioxopiperidin-3-yl)-2,3-dihydro-1H-isoindole-1,3-dione (prepared in the same fashion as **intermediate 15,** 6.32 mg; 0.01 mmol; 1.00 eq.), 2-methyl-4-[(1r,4r)-4-(trifluoromethyl)cyclohexyl]-2H,4H-[12,3]triazolo[4,5-b]indole-7-carboxylic acid **intermediate 68** (4.70 mg; 0.01 mmol; 1.00 eq.), 4-methylmorpholine (5.6 µL; 0.05 mmol; 4.00 eq.). The reactants were dissolved in anhydrous DMF (1.00 ml), followed by addition of HATU (5.85 mg; 0.02 mmol; 1.20 eq.) The reaction mixture was stirred at RT for 18 h. The reaction mixture was filtered and purified by RP-HPLC-MS. The target fractions were lyophilized to provide N-(14-{[2-(2,6-dioxopiperidin-3-yl)-1,3-dioxo-2,3-dihydro-1H-isoindol-4-yl]-amino}-3,6,9,12-tetraoxatetradecan-1-yl)-2-methyl-4-[(1r,4r)-4-(trifluoromethyl)cyclohexyl]-2H,4H-[1,2,3]triazolo[4,5-b]indole-7-carboxamide (6.00 mg; 0.01 mmol) as a yellow lyophilizate. LC-MS (ESI+, Method 1): tR = 1.72 min, m/z 841.30 [M+H]+. 1H NMR (700 MHz, DMSO-d6) d 11.09 (s, 1H), 8.52 (t, J = 5.6 Hz, 1H), 8.44 (d, J = 1.7 Hz, 1H), 7.97 (dd, J = 8.7, 1.8 Hz, 1H), 7.74 (d, J = 8.9 Hz, 1H), 7.56 (dd, J = 8.5, 7.0 Hz, 1H), 7.11 (d, J = 8.6 Hz, 1H), 7.03 (d, J = 7.0 Hz, 1H), 6.58 (t, J = 5.9 Hz, 1H), 5.05 (dd, J = 12.9, 5.4 Hz, 1H), 4.58 (tt, J = 11.9, 4.1 Hz, 1H), 4.31 (s, 3H), 3.63 - 3.42 (m, 20H), 3.01 (s, 1H), 2.92 - 2.84 (m, 1H), 2.61 - 2.51 (m, 1H), 2.18 - 2.10 (m, 2H), 2.10 - 1.98 (m, 6H), 1.68 - 1.59 (m, 2H).

### Compound 99

### N-(14-{[2-(2,6-dioxopiperidin-3-yl)-1,3-dioxo-2,3-dihydro-1H-isoindol-4-yl]amino}-3,6,9,12-tetraoxatetradecan-1-yl)-2-methyl-8-{[4-(trifluoromethyl)cyclohexyl]methyl}-2H,8H-pyrazolo[3,4-b]indole-5-carboxamide (Compound 99)

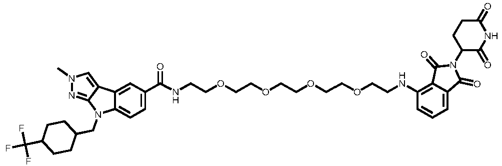

In a 8 ml vial with stir bar were placed 4-(15-amino-4,7,10,13-tetraoxa-1-azapentadecan-1-yl)-2-(2,6-dioxopiperidin-3-yl)-2,3-dihydro-1H-isoindole-1,3-dione (prepared in the same manner as **intermediate 15,** 12.59 mg; 0.03 mmol; 1.00 eq.), 2-methyl-8-{[4-(trifluoromethyl)cyclohexyl]methyl}-2H,8H-pyrazolo[3,4-b]indole-5-carboxylic acid **intermediate 71** (9.70 mg; 0.03 mmol; 1.00 eq.), 4-methylmorpholine (11.2 µL; 0.10 mmol; 4.00 eq.). The reactants were dissolved in anhydrous dimethylformamide (0.50 ml), followed by addition of HATU (11.67 mg; 0.03 mmol; 1.20 eq.). The reaction mixture was stirred at RT for 18 h. The reaction mixture was filtered and directly purified by prep RP-HPLC-MS. The target fractions were lyophilized to provide N-(14-{[2-(2,6-dioxopiperidin-3-yl)-1,3-dioxo-2,3-dihydro-1H-isoindol-4-yl]amino}-3,6,9,12-tetraoxatetradecan-1-yl)-2-methyl-8-{[4-(trifluoromethyl)cyclohexyl]methyl}-2H,8H-pyrazolo[3,4-b]indole-5-carboxamide (14.00 mg; 0.02 mmol) as a yellow lyophilizate. LC-MS (ESI+, Method 1): t_{R} = 1.68 min, m/z 427.80[M+2H]2+. 1H NMR (700 MHz, DMSO-d6) d 11.1 (s, 1H), 8.4 (t, J = 5.6 Hz, 1H), 8.2 (t, J = 2.1 Hz, 1H), 8.0 (s, 1H), 7.8 - 7.8 (m, 1H), 7.6 (dd, J = 8.6, 7.1 Hz, 1H), 7.5 - 7.4 (m, 1H), 7.1 (d, J = 8.6 Hz, 1H), 7.0 (d, J = 7.0 Hz, 1H), 6.6 (t, J = 5.9 Hz, 1H), 5.1 (dd, J = 12.9, 5.5 Hz, 1H), 4.0 - 4.0 (m, 5H), 3.6 (t, J = 5.5 Hz, 2H), 3.6 - 3.5 (m, 14H), 3.5 - 3.4 (m, 4H), 2.9 (ddd, J = 17.1, 13.9, 5.4 Hz, 1H), 2.6 - 2.5 (m, 2H), 2.2 (s, 1H), 2.1 - 2.0 (m, 1H), 1.8 (dq, J = 8.2, 4.9, 4.0 Hz, 2H), 1.7 (d, J = 8.1 Hz, 2H), 1.5 (ddq, J = 18.0, 8.9, 4.5, 4.1 Hz, 1H), 1.2 - 1.1 (m, 4H).

### Compound 110

### N-(14-{[2-(2,6-dioxopiperidin-3-yl)-1,3-dioxo-2,3-dihydro-1H-isoindol-4-yl]amino}-3,6,9,12-tetraoxatetradecan-1-yl)-8-(6-fluoronaphthalen-2-yl)-2-methyl-2H,8H-pyrazolo[3,4-b]indole-5-carboxamide (Compound 110)

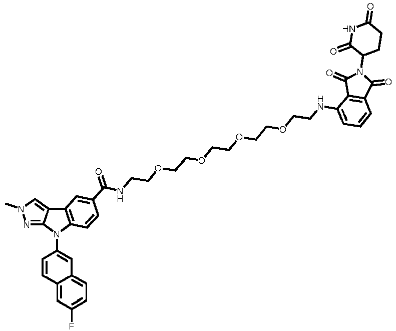

In a 4 ml glass vial 4-(15-amino-4,7,10,13-tetraoxa-1-azapentadecan-1-yl)-2-(2,6-dioxopiperidin-3-yl)-2,3-dihydro-1H-isoindole-1,3-dione (prepared in the same fashion as **intermediate 15,** 34.0 mg; 0.058 mmol; 1.05 eq.) was dissolved in anhydrous N,N-Dimethylformamide (0.6 ml). 4-Methylmorpholine (24.7 µl; 0.223 mmol; 4.00 eq.) and 8-(6-fluoronaphthalen-2-yl)-2-methyl-2H,8H-pyrazolo[3,4-b]indole-5-carboxylic acid (prepared in the same manner as **TEAD ligand 1 free acid,** but the aryl bromide coupling partner during the synthesis of **building block 3** is 2-Bromo-6-fluoronaphthalene instead, 20.0 mg; 0.056 mmol; 1.00 eq.) were added and dissolved. Then HATU (25.3 mg; 0.067 mmol; 1.20 eq.) was added and stirred at RT for 18 h. The yellow reaction solution was purified by prep. HPLC. The target fractions were lyophilized, re-dissolved in DCM and evaporated by vacuum centrifugation to provide N-(14-{[2-(2,6-dioxopiperidin-3-yl)-1,3-dioxo-2,3-dihydro-1H-isoindol-4-yl]amino}-3,6,9,12-tetraoxatetradecan-1-yl)-8-(6-fluoronaphthalen-2-yl)-2-methyl-2H,8H-pyrazolo[3,4-b]indole-5-carboxamide (13.4 mg, 0.016 mmol) as a yellow amorphous solid. LC-MS (ESI+, Method 10): tR = 1.68 min, m/z 834.20 [M+H]+. 1H NMR (500 MHz, DMSO) d 11.08 (s, 1H), 8.48 (t, J = 5.6 Hz, 1H), 8.36 (d, J = 1.8 Hz, 1H), 8.33 (d, J = 2.2 Hz, 1H), 8.20 (s, 1H), 8.15 (dt, J = 9.1, 3.0 Hz, 2H), 8.01 (dd, J = 8.9, 2.2 Hz, 1H), 7.84 (ddd, J = 11.6, 9.4, 2.3 Hz, 2H), 7.69 (d, J = 8.6 Hz, 1H), 7.60 - 7.47 (m, 2H), 7.11 (d, J = 8.6 Hz, 1H), 7.02 (d, J = 7.0 Hz, 1H), 6.63 - 6.53 (m, 1H), 5.05 (dd, J = 12.7, 5.4 Hz, 1H), 4.04 (s, 3H), 3.61 - 3.49 (m, 16H), 3.48 - 3.38 (m, 4H), 2.88 (ddd, J = 16.8, 13.7, 5.4 Hz, 1H), 2.58 (dt, J = 16.5, 2.9 Hz, 1H), 2.55 - 2.46 (m, 1H), 2.02 (ddd, J = 10.6, 5.5, 3.2 Hz, 1H).

### Compound 46

### N-(14-{[2-(2,6-dioxopiperidin-3-yl)-1,3-dioxo-2,3-dihydro-1H-isoindol-4-yl]amino}-3,6,9,12-tetraoxatetradecan-1-yl)-6-methyl-9-[4-(trifluoromethyl)phenyl]-9H-carbazole-3-carboxamide (Compound 46)

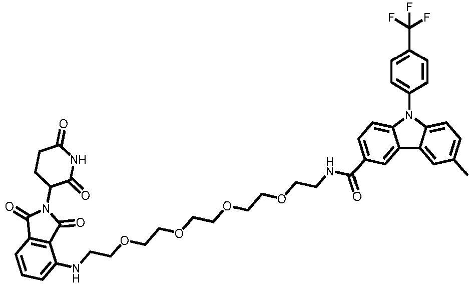

Prepared analogously to compound 50, but using different acid (6-methyl-9-[4-(trifluoromethyl)phenyl]-9H-carbazole-3-carboxylic acid, prepared in the same manner as **TEAD ligand 1,** where building block is ethyl 2'-amino-6-chloro-5'-methyl-[1,1'-biphenyl]-3-carboxylate instead) and amine (4-(15-amino-4,7,10,13-tetraoxa-1-azapentadecan-1-yl)-2-(2,6-dioxo-piperidin-3-yl)-2,3-dihydro-1H-isoindole-1,3-dione; formic acid, prepared in the same manner as **intermediate 15)** coupling partners instead which gave N-(14-{[2-(2,6-dioxopiperidin-3-yl)-1,3-dioxo-2,3-dihydro-1H-isoindol-4-yl]amino}-3,6,9,12-tetraoxatetradecan-1-yl)-6-methyl-9-[4-(trifluoromethyl)phenyl]-9H-carbazole-3-carboxamide (18.0 mg; 0.02 mmol) as a yellow lyophilizate. LC-MS (ESI+, Method 3): tR = 2.72 min, m/z 843.6 [M+H]+. 1H NMR (500 MHz, DMSO-d6) δ 11.1 (s, 1H), 8.8 (d, J = 1.9 Hz, 1H), 8.6 (t, J = 5.6 Hz, 1H), 8.1 - 8.0 (m, 3H), 8.0 (dd, J = 8.7, 1.8 Hz, 1H), 7.9 - 7.9 (m, 2H), 7.6 (dd, J = 8.6, 7.1 Hz, 1H), 7.5 (d, J = 8.6 Hz, 1H), 7.4 (d, J = 8.4 Hz, 1H), 7.3 - 7.3 (m, 1H), 7.1 (d, J = 8.6 Hz, 1H), 7.0 (d, J = 7.0 Hz, 1H), 6.6 (t, J = 5.8 Hz, 1H), 5.1 (dd, J = 12.8, 5.4 Hz, 1H), 3.6 - 3.5 (m, 6H), 3.5 - 3.5 (m, 15H), 3.4 (q, J = 5.6 Hz, 2H), 2.9 (ddd, J = 16.9, 13.8, 5.4 Hz, 1H), 2.6 - 2.5 (m, 2H), 2.0 (dtd, J = 12.8, 5.0, 2.0 Hz, 1H).

### Compound 34

### (2S,4R)-1-[(2S)-3,3-dimethyl-2-(6-{1-[7-({2-methyl-8-[4-(trifluoromethyl)-phenyl]-2H,8H-pyrazolo[3,4-b]indol-5-yl}formamido)heptyl]-1H-1,2,3-triazol-4-yl}hexanamido)butanoyl]-4-hydroxy-N-{[4-(4-methyl-1,3-thiazol-5-yl)phenyl]methyl}pyrrolidine-2-carboxamide (Compound 34)

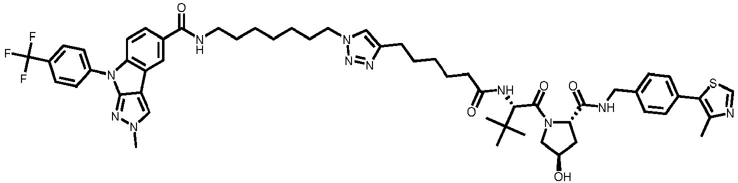

A solution of 1-[7-({2-methyl-8-[4-(trifluoromethyl)phenyl]-2H,8H-pyrazo-lo[3,4-b]indol-5-yl}formamido)heptyl]-2lambda4-triaza-1,2-dien-1-ium-3-ide (prepared analogously to **intermediate** 1 using 7-azido-1-heptanamine as the amine coupling partner instead, 21.40 mg; 0.04 mmol; 1.00 eq.) in DMF (460,00 µl) was added to a solution of (2S,4R)-1-[(2S)-3,3-dimethyl-2-(oct-7-ynamido)butanoyl]-4-hydroxy-N-{[4-(4-methyl-1,3-thiazol-5-yl)phenyl]methyl}pyrrolidine-2-carboxamide **intermediate 22** (23.73 mg; 0.04 mmol; 1.00 eq.) in DMF (400.00 µl). Copper(II) sulfate pentahydrate (1.07 mg; 0.004 mmol; 0.10 eq.) and sodium ascorbate (17.01 mg; 0.09 mmol; 2.00 eq.) were dissolved in water and the resulting orange suspension was added to the reaction mixture. The reaction mixture was stirred for 18 h at rt. It was filtered and the product was purified by HPLC to give (2S,4R)-1-[(2S)-3,3-dimethyl-2-(6-{1-[7-({2-methyl-8-[4-(trifluoromethyl)phenyl]-2H,8H-pyrazolo[3,4-b]indol-5-yl}formamido)heptyl]-1H-1,2,3-triazol-4-yl}hexanamido)butanoyl]-4-hydroxy-N-{[4-(4-methyl-1,3-thiazol-5-yl)phenyl]methyl}pyrrolidine-2-carboxamide (17.4 mg, 0.02 mmol, 39%) after lyophilization.LC-MS (ESI+, Method 2): tR = 2.57 min, m/z 525.4 [M+2H]2+. ¹H NMR (500 MHz, DMSO-d6) δ 9.0 (s, 1H), 8.5 (t, J = 6.1 Hz, 1H), 8.4 (t, J = 5.6 Hz, 1H), 8.3 (d, J = 1.8 Hz, 1H), 8.2 (s, 1H), 8.1 (d, J = 8.3 Hz, 2H), 8.0 (d, J = 8.3 Hz, 2H), 7.9 - 7.7 (m, 4H), 7.4 (q, J = 8.0 Hz, 4H), 5.1 (d, J = 3.6 Hz, 1H), 4.5 (d, J = 9.4 Hz, 1H), 4.5 - 4.4 (m, 2H), 4.3 (s, 1H), 4.3 (t, J = 7.2 Hz, 2H), 4.2 (dd, J = 15.9, 5.5 Hz, 1H), 4.0 (s, 3H), 3.7 - 3.6 (m, 2H), 2.6 (t, J = 7.6 Hz, 2H), 2.4 (s, 3H), 2.3 - 2.2 (m, 1H), 2.2 - 2.1 (m, 1H), 2.1 - 2.0 (m, 1H), 1.9 - 1.9 (m, 1H), 1.8 - 1.7 (m, 2H), 1.6 - 1.4 (m, 7H), 1.4 - 1.2 (m, 9H), 0.9 - 0.9 (m, 9H).

### Compound 35

### (2S,4R)-1-[(2S)-3,3-dimethyl-2-(6-{1-[6-({2-methyl-8-[4-(trifluoromethyl)phenyl]-2H,8H-pyrazolo[3,4-b]indol-5-yl}formamido)hexyl]-1H-1,2,3-triazol-4-yl}hexanamido)butanoyl]-4-hydroxy-N-{[4-(4-methyl-1,3-thiazol-5-yl)phenyl]methyl}pyrrolidine-2-carboxamide (Compound 35)

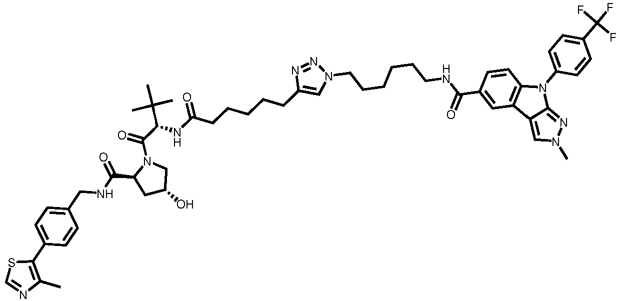

Prepared analogously to **compound 34,** using N-(6-azidohexyl)-2-methyl-8-[4-(trifluoromethyl)phenyl]-2H,8H-pyrazolo[3,4-b]indole-5-carboxamide instead of 1-[7-({2-methyl-8-[4-(trifluoromethyl)phenyl]-2H,8H-pyrazolo[3,4-b]indol-5-yl}formamido)heptyl]-2lambda4-triaza-1,2-dien-1-ium-3-ide as the azide component instead. This azide component is prepared analogously to **intermediate** 1, using 6-azido-1-hexanamine instead as the amine coupling partner. This adapted procedure gave (2S,4R)-1-[(2S)-3,3-dimethyl-2-(6-{1-[6-({2-methyl-8-[4-(trifluoromethyl)-phenyl]-2H,8H-pyrazolo[3,4-b]indol-5-yl}formamido)hexyl]-1H-1,2,3-triazol-4-yl}hexanamido)butanoyl]-4-hydroxy-N-{[4-(4-methyl-1,3-thiazol-5-yl)phenyl]methyl}pyrrolidine-2-carboxamide (4.8 mg, 0.01 mmol, 11%). LC-MS (ESI+, Method 2): tR = 2.52 min, m/z 518.5 [M+2H]2+. 1H NMR (500 MHz, DMSO-d6) δ 9.0 (s, 1H), 8.5 (t, J = 6.1 Hz, 1H), 8.4 (t, J = 5.6 Hz, 1H), 8.3 (d, J = 1.8 Hz, 1H), 8.2 (s, 1H), 8.1 (d, J = 8.4 Hz, 2H), 8.0 (d, J = 8.5 Hz, 2H), 7.9 - 7.8 (m, 3H), 7.8 (d, J = 8.7 Hz, 1H), 7.4 (d, J = 8.3 Hz, 2H), 7.4 (d, J = 8.3 Hz, 2H), 5.1 (d, J = 3.9 Hz, 1H), 4.5 (d, J = 9.3 Hz, 1H), 4.5 - 4.4 (m, 2H), 4.4 - 4.3 (m, 1H), 4.3 (t, J = 7.1 Hz, 2H), 4.2 (dd, J = 15.8, 5.5 Hz, 1H), 4.0 (s, 3H), 3.7 - 3.6 (m, 2H), 3.3 (d, J = 7.0 Hz, 2H), 2.6 (t, J = 7.6 Hz, 2H), 2.4 (s, 3H), 2.3 (dt, J = 14.8, 7.6 Hz, 1H), 2.1 (ddd, J = 14.3, 8.2, 6.3 Hz, 1H), 2.0 (ddd, J = 13.0, 7.7, 2.7 Hz, 1H), 1.9 (ddd, J = 12.9, 8.5, 4.6 Hz, 1H), 1.8 (p, J = 7.3 Hz, 2H), 1.6 - 1.4 (m, 6H), 1.4 (p, J = 7.0 Hz, 2H), 1.3 (p, J = 7.4 Hz, 4H), 0.9 (s, 9H).

### Table 1

Table 1 below shows exemplary compounds of the present invention. They have been synthesized as described in the Examples above or similar thereto.

**Table 1**

| **Compound No.** | **Structure** |
|---|---|
| 1 | 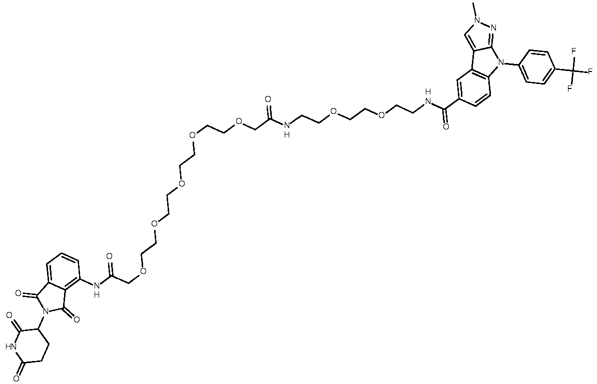 |
| 2 | 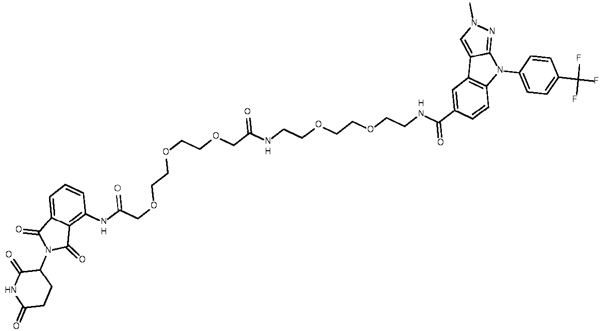 |
| 3 | 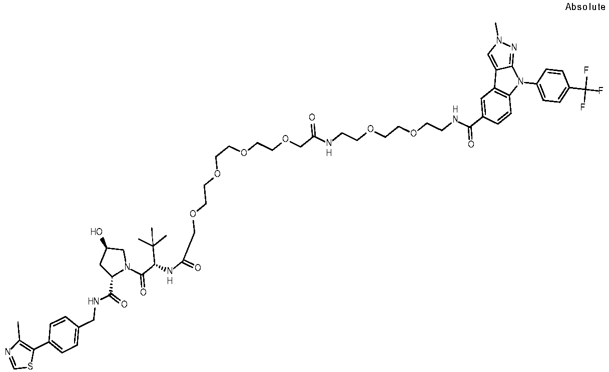 |
| 4 | 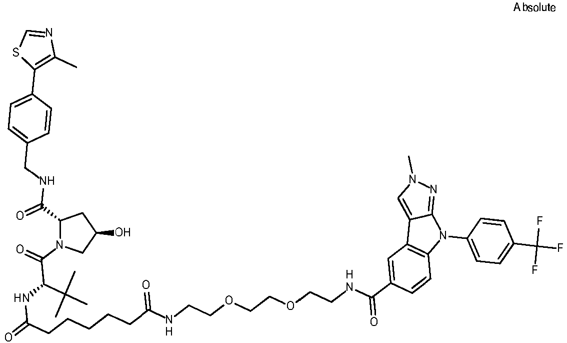 |
| 4-N | 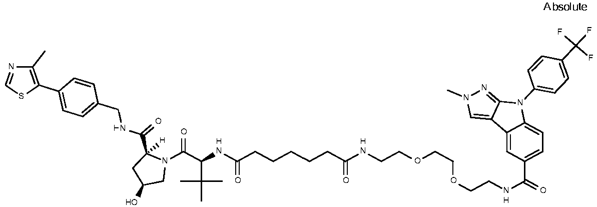 |
| 5 | 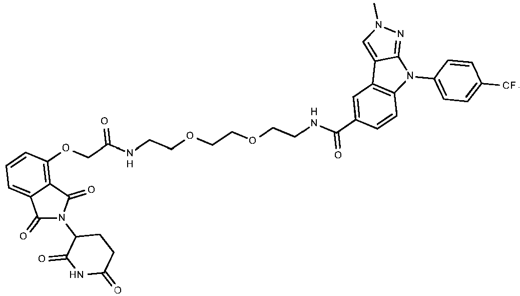 |
| 6 | 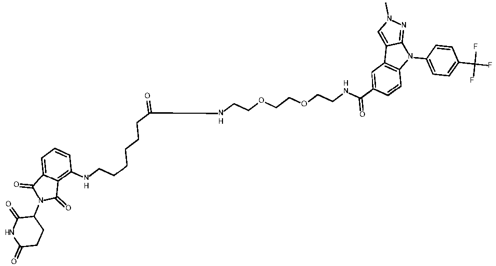 |
| 7 | 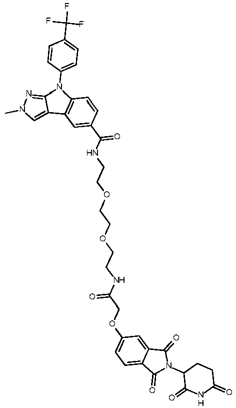 |
| 8 | 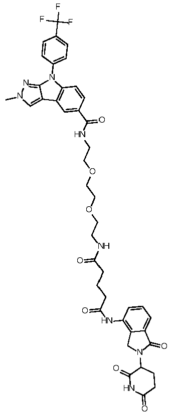 |
| 9 | 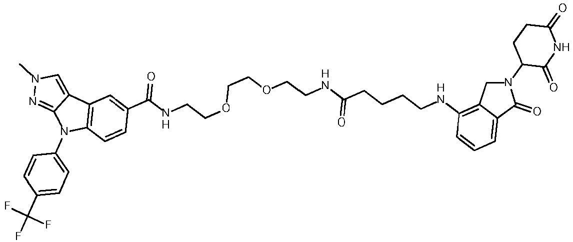 |
| 10 | 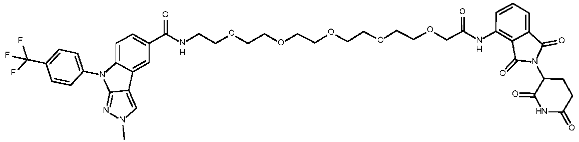 |
| 11 | 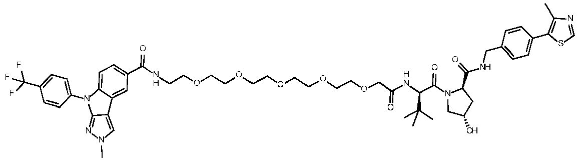 |
| 12 | 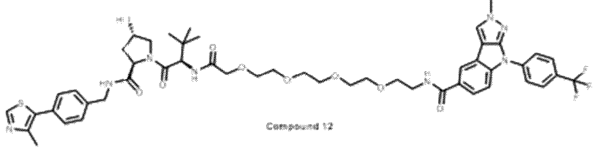 |
| 13 | 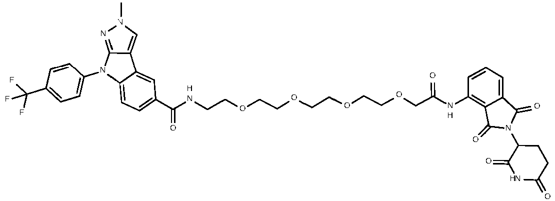 |
| 14 | 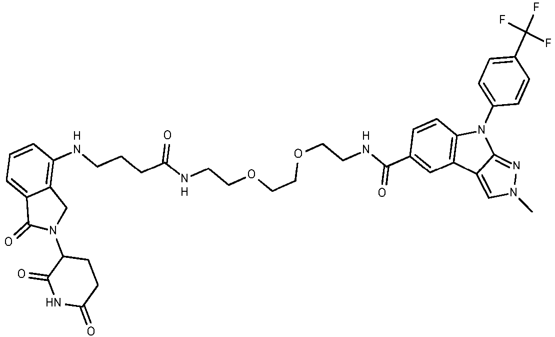 |
| 15 | 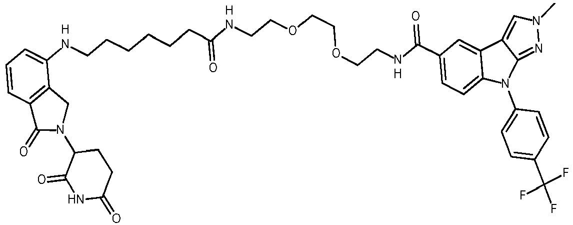 |
| 16 | 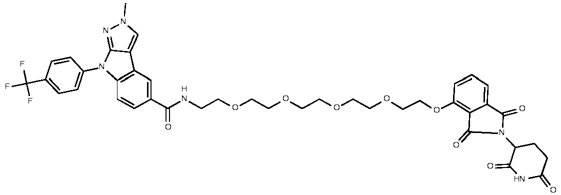 |
| 17 | 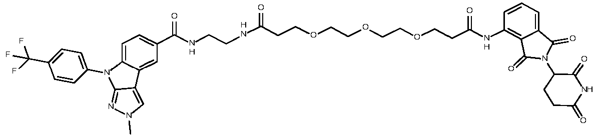 |
| 18 | 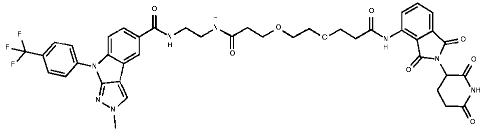 |
| 19 | 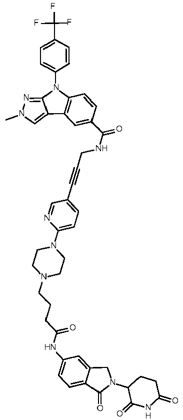 |
| 20 | 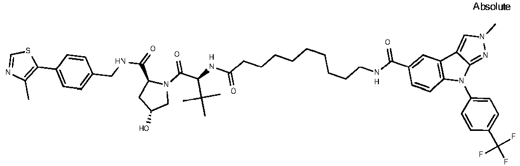 |
| 21 | 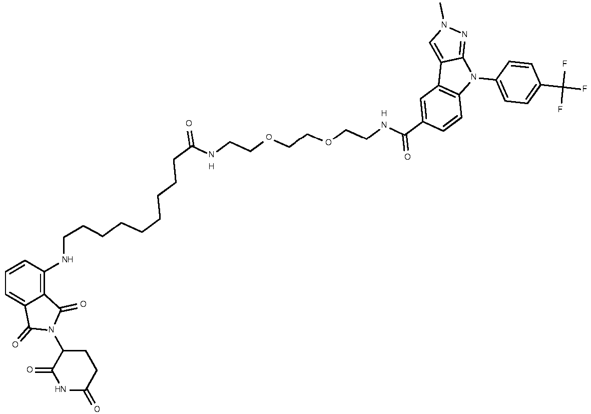 |
| 22 | 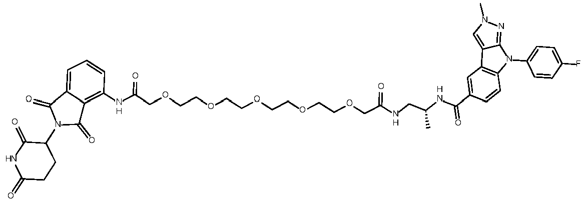 |
| 23 | 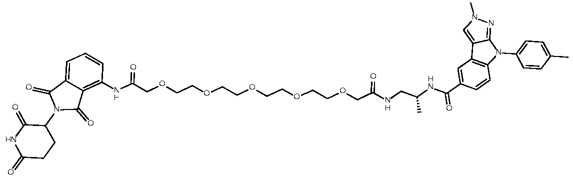 |
| 24 | 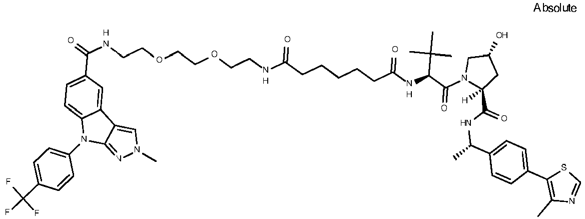 |
| 25 | 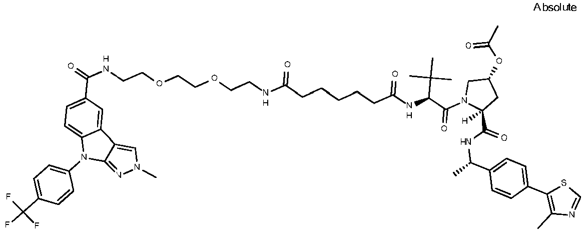 |
| 26 | 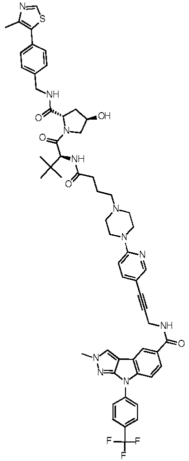 |
| 27 | 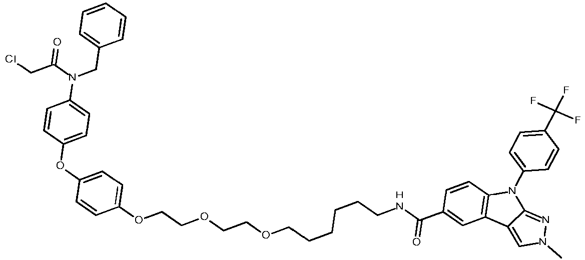 |
| 28 | 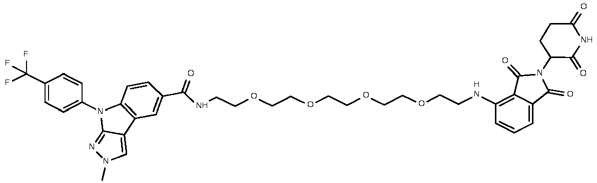 |
| 29 | 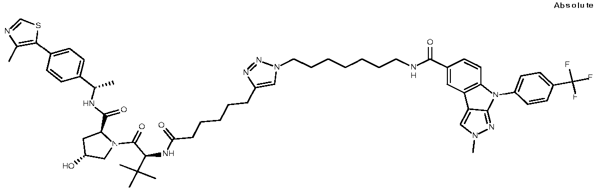 |
| 30 | 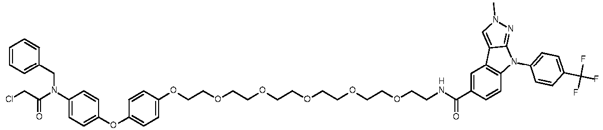 |
| 31 | 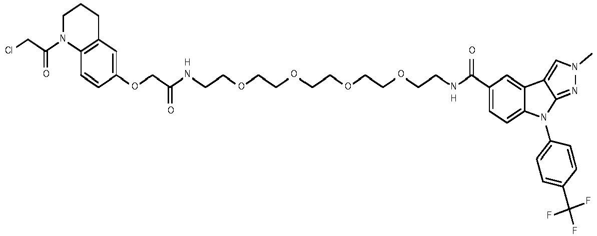 |
| 32 | 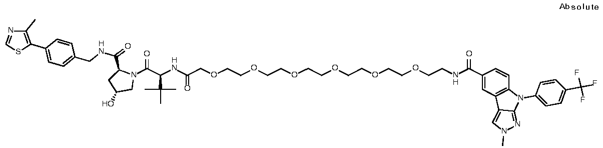 |
| 33 | 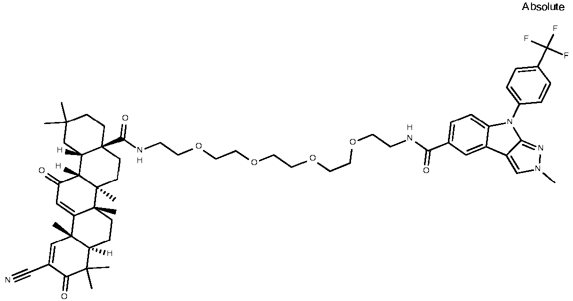 |
| 34 | 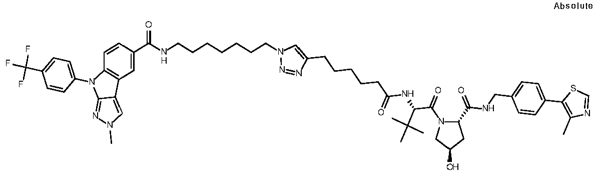 (2S,4R)-1-[(2S)-3,3-dimethyl-2-(6-{1-[7-({2-methyl-8-[4-(trifluoromethyl)phenyl]-2H,8H-pyrazolo[3,4-b]indol-5-yl}formamido)heptyl]-1H-1,2,3-triazol-4-yl}hexanamido)butanoyl]-4-hydroxy-N-{[4-(4-methyl-1,3-thiazol-5-yl)phenyl]methyl}pyrrolidine-2-carboxamide |
| 35 | 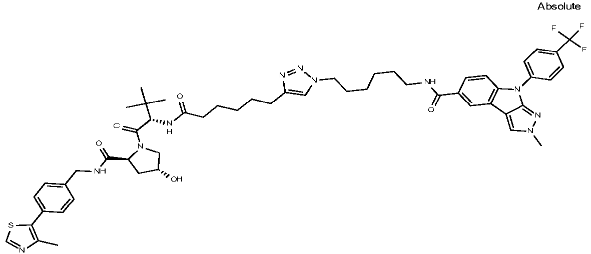 (2S,4R)-1-[(2S)-3,3-dimethyl-2-(6-{1-[6-({2-methyl-8-[4-(trifluoromethyl)phenyl]-2H,8H-pyrazolo[3,4-b]indol-5-yl}formamido)hexyl]-1H-1,2,3-triazol-4-yl}hexanamido)butanoyl]-4-hydroxy-N-{[4-(4-methyl-1,3-thiazol-5-yl)phenyl]methyl}pyrrolidine-2-carboxamide |
| 36 | 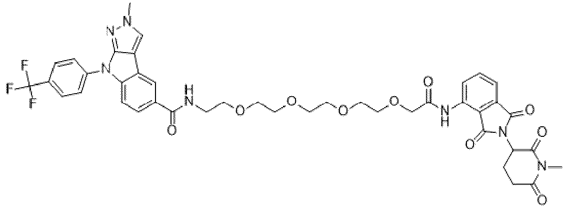 |
| 37 | 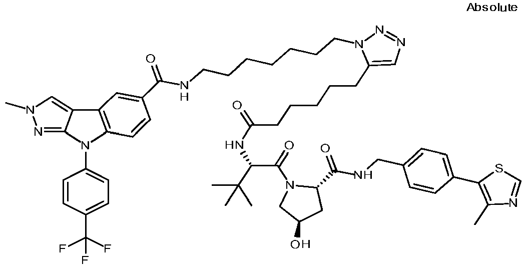 |
| 38 | 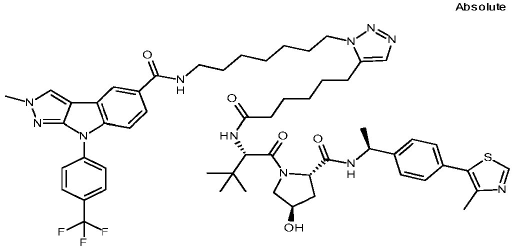 |
| 42 | 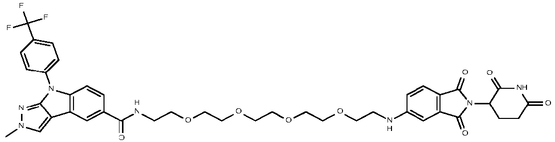 |
| 43 | 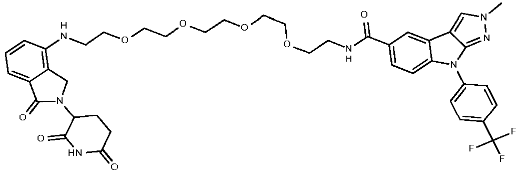 |
| 45 | 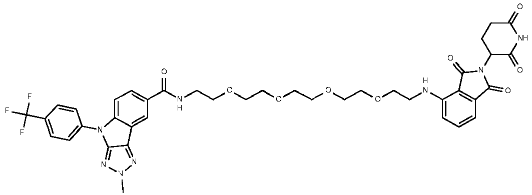 |
| 46 | 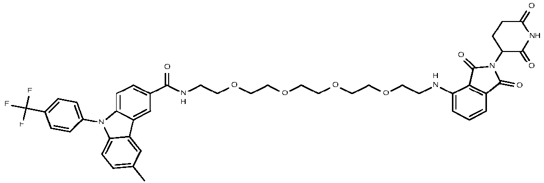 |
| 47 | 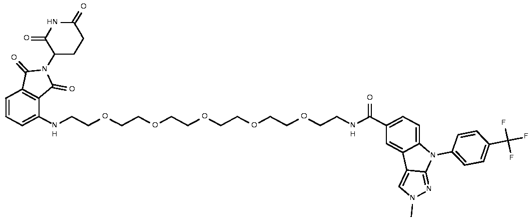 |
| 48 | 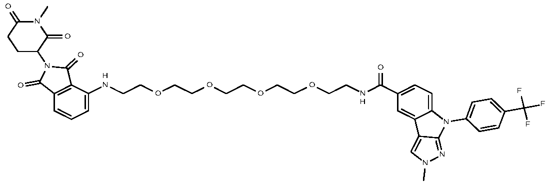 |
| 49 | 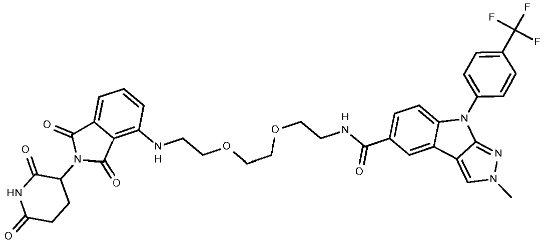 |
| 50 | 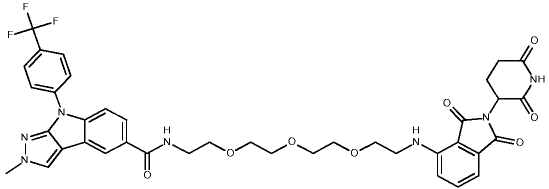 |
| 51 | 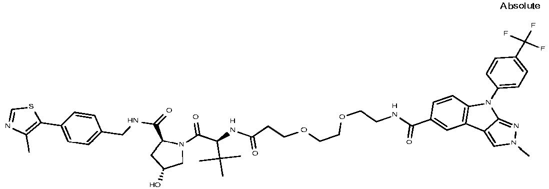 |
| 52 | 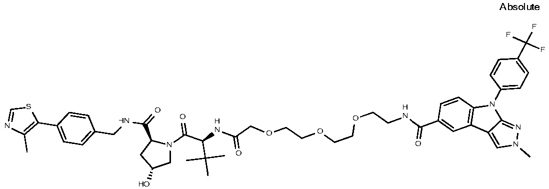 |
| 53 | 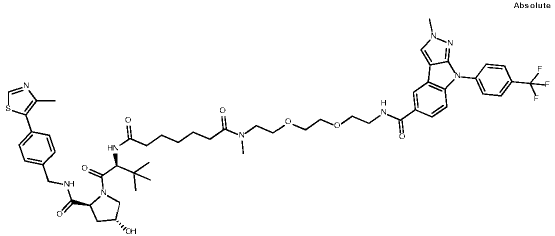 |
| 54 | 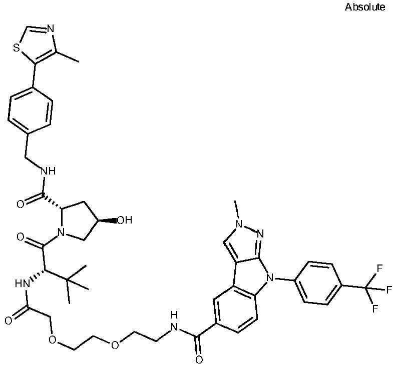 |
| 55 | 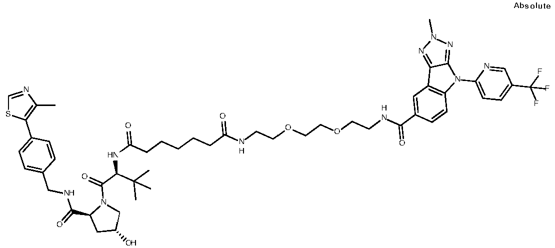 |
| 56 | 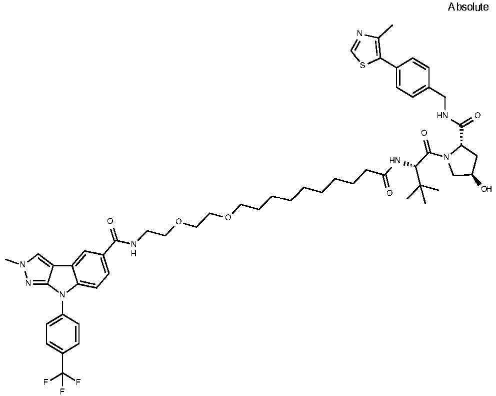 |
| 57 | 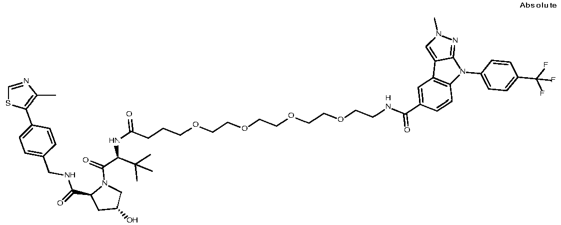 |
| 59 | 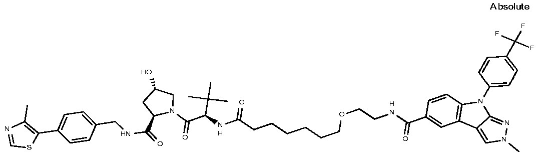 |
| 60 | 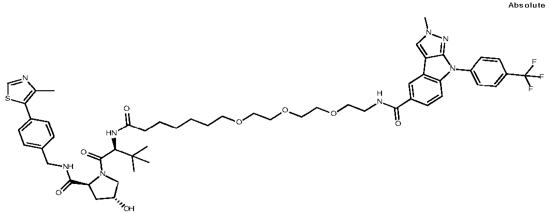 |
| 61 | 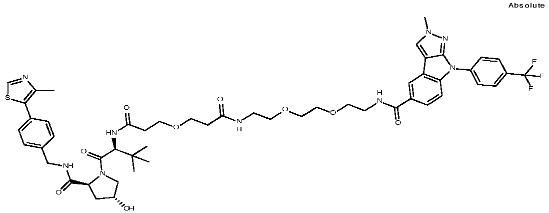 |
| 63 | 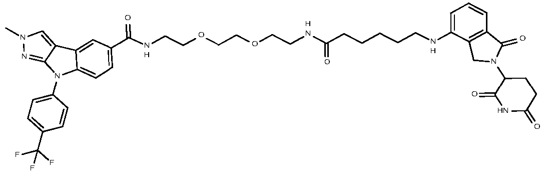 |
| 67 | 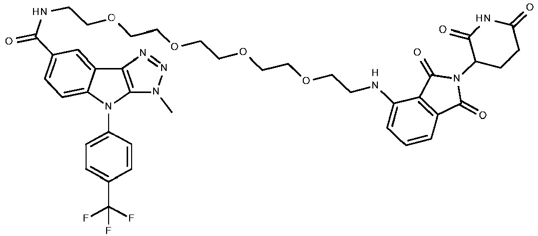 |
| 69 | 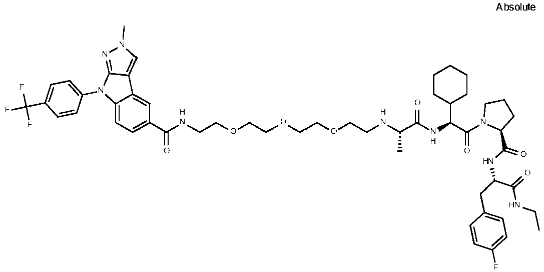 |
| 73 | 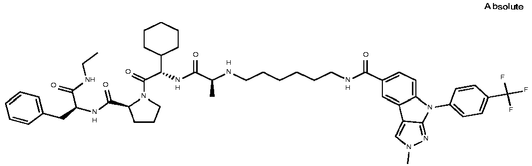 |
| 74 | 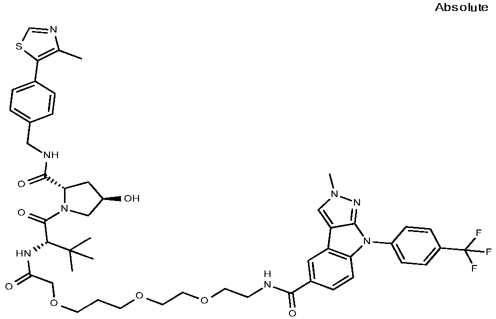 |
| 75 | 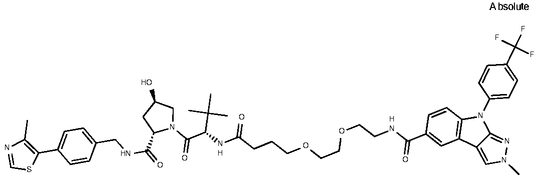 |
| 77 | 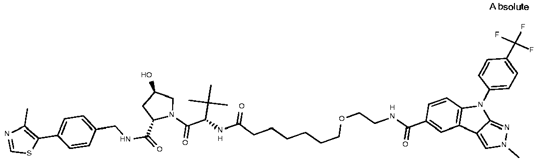 |
| 80 | 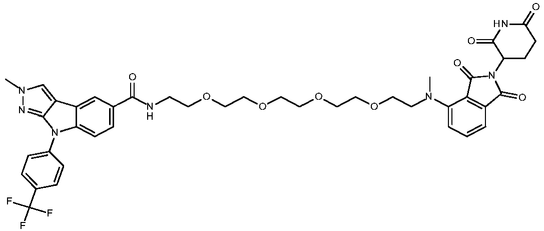 |
| 81 | 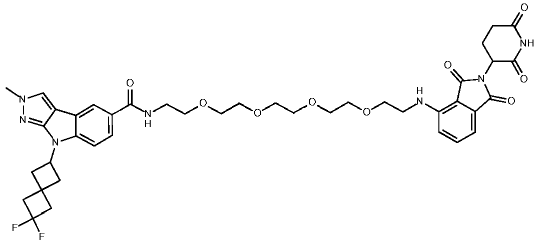 |
| 82 | 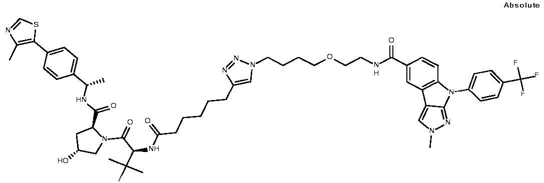 |
| 84 | 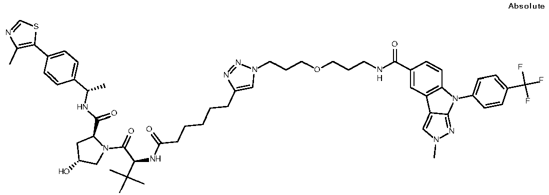 |
| 87 | 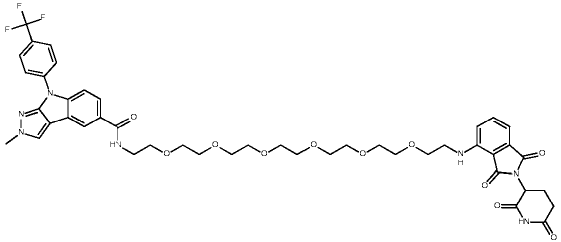 |
| 88 | 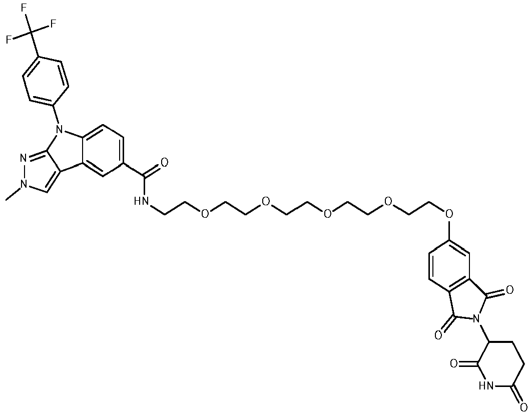 |
| 95 | 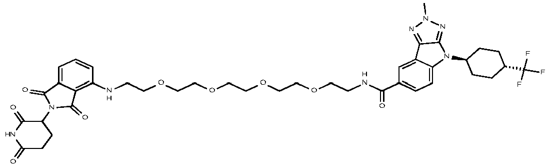 |
| 110 | 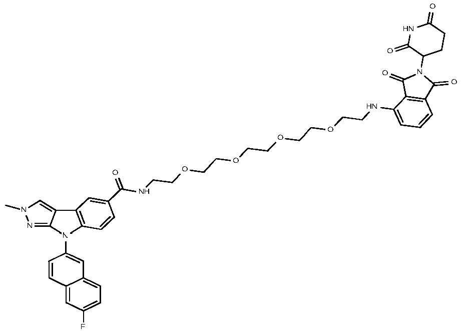 |
| 117 | 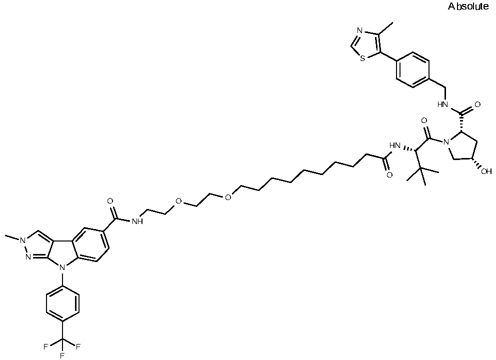 |

### Biological Assays

### 1. Thermal shift measurement / Differential Scanning Fluorimetry (DSF)

Interaction of exemplary compounds with the various TEAD isoforms TEAD1, TEAD2, TEAD3, and TEAD4 was performed in a DSF assay. YAP-binding domains (YBD) of the four isoforms were subcloned and heterogeneously expressed in *E.coli* as a host. Initial purification was perfomed by using affinity tags, which got removed by overnight protease treatment. Further purification of TEAD isoforms was performed by Size-Exclusion Chromatography (SEC). Target protein containing fractions were pooled, concentrated with an Amicon (10 KDa MWCO) and quantified using the Bradford assay. Exemplary compound testing in the DSF assay for determining the shift of the TEAD isoform melting temperatures has been performed as described below:
DSF was performed in MicroAmp^{™} EnduraPlate^{™} Optical 96-Well Clear Reaction Plates (Applied Biosystems, Life Technologies, California, USA) using a QuantStudio 7 Flex Real-Time PCR System (Applied Biosystems). Protein samples were briefly centrifuged before preparation. The final reaction mixture (20 µL of total volume) contained 11.8 µM of hsTEAD1 (209-426), 7.3 µM of hsTEAD2 (217-447), 7.9 µM of hsTEAD3 (216-435) or 11.7 µM of hsTEAD4 (217-424), and 2 or 4-fold of Protein Thermal Shift^{™} Dye (Applied Biosystems) diluted in protein buffer - 20 mM Tris pH 8.0, 150 mM NaCl (+ 0.5 mM TCEP for TEAD1 experiments). Prior to the addition of the dye, proteins were incubated with either 1 µL DMSO 100% (apo) or 100 µM of each exemplary compound (1 µL at 2 mM in 100% DMSO added to the 19 µL reaction mixture) for 2 hours at 4 °C. The melting curve data was recorded from 25 to 90 °C with an increment rate of 0.016 °C.s⁻¹. Excitation and emission filters were applied for Protein Thermal Shift^{™} Dye (470 nm and 520 nm, respectively) and for ROX reference dye (580 nm and 623 nm, respectively). The melting temperatures (Tm) were obtained by calculating the midpoint of each transition, using the Protein Thermal Shift Software^{™} version 1.3. The thermal stability effect of each compound (ΔTm) was calculated by subtracting the calculated Tm in presence and absence (apo) of compound. All samples were tested in triplicates.

Table 2 below summarizes the results of the DSF thermal shift measurements. Values for thermal shift are given as ΔTm in °C.

**Table 2**

| **Compound No.** | **ΔTm [°C]** | | | |
|---|---|---|---|---|
| | TEAD1 (209-426) | TEAD2 (217-447) | TEAD3 216-435 | TEAD4 217-434 |
| 13 | 6.1 | 0.4 | 1.7 | 0 |
| 11 | 4.5 | 0.3 | 1.3 | -0.2 |
| 6 | -1.1 | - | - | 1.4 |
| 5 | 3.9 | -0.1 | 0.9 | 2.8 |
| 4 | 2.9 | 1 | 0.6 | 0 |
| 3 | 3.4 | - | - | 0.7 |
| 2 | 4.1 | 0.7 | 1.3 | 0 |
| 1 | 4.7 | 0 | 0 | - |

### 3. Surface plasmon resonance (SPR) assay

Interaction of exemplary compounds with the TEAD isoform TEAD1 has been tested in a SPR assay described below for determining kinetic rate constant, KD:
The kinetic and affinity parameters of protein-exemplary compound interactions were evaluated by SPR. hsTEAD1 (209-426) was immobilized onto a CM7 (Series S) sensor chip via the standard amine coupling procedure, at 15 °C. Prior to immobilization, the carboxymethylated surface of the chip was activated with 400 mM 1-ethyl-3-(3-dimethylaminopropyl)-carbodiimide and 100 mM N-hydroxysuccinimide for 10 min. hsTEAD1 (209-426) was diluted to 11 µg/mL in 10 mM Bis-Tris at pH 6.5 and immobilized on the activated surface chip for 8 and 10 min, in order to reach 6000 to 9000 response units (RU). The remaining activated carboxymethylated groups were blocked with a 7 min injection of 1 M ethanolamine pH 8.5 after which the surface chip was washed with 0.5% *(w*/*v)* sodium dodecyl sulphate and 50 mM glycine. HBS-N, which consists of 10 mM HEPES pH 7.4 and 150 mM NaCl, was used as the background buffer during immobilization. Exemplary compounds were prediluted in DMSO, diluted 1:50 in running buffer (20 mM Tris pH 7.4, 150 mM NaCl, 1 mM DTT, 5 mM MgCl₂, 0.1 mM EGTA, 0.05% CHAPS) and injected at ten different concentrations using two-fold dilution series, from 100 µM to 0.2 µM. A DMSO solvent correction (1% - 3%) was performed to account for variations in bulk signal and to achieve high-quality data. Interaction analysis cycles consisted of a 240 sec sample injection (30 µL/min, association phase) followed by 600 sec of buffer flow (dissociation phase).

All sensorgrams were processed by first subtracting the binding response recorded from the control surface (reference spot), followed by subtracting of the buffer blank injection from the reaction spot. All datasets were fit to a simple 1:1 Langmuir interaction model to determine the kinetic rate constants, KD. Experiments were performed on a Biacore 4000 (Cytiva, Uppsala, Sweden) at 15 °C and the interactions were evaluated using the provided Biacore 4000 evaluation software.

Table 3 below summarizes the results of the SPR assay. Values for KD are given in µM..

**Table 3**

| **Compound No.** | **KD [µM] TEAD 1 (209-426)** |
|---|---|
| 36 | 0.98 |
| 16 | 3.01 |
| 14 | 0.482 |
| 9 | 0.97 |
| 6 | 1.4 |
| 5 | 1.9 |
| 4 | 0.32 |
| 3 | 0.32 |
| 2 | 1.52 |
| 1 | 1.87 |

### 2. TEAD degradation in SK-HEP1 and NCI-H226 cell lines

Degradation of TEAD isoform TEAD1 by exemplary compounds which has been determined at different time points in two different cell lines, SK-HEP1 and NCl-H226:
For the assay, SK-HEP1 (ATCC HTB-52) and NCI-H226 (ATCC CRL-5826) cells were treated in duplicates with the test compounds in a 10-point dose, 1:3 dilution steps, with the top concentration starting at 30µM (final concentration in assay). After either 16 or 24 hour incubation at 37°C, 95% rH, and 5% CO₂, the cells were fixed with 4% PFA and TEAD1 levels were probed using immunofluorescence. The primary antibody used was Anti-TEF1 (BD, 610293) and the secondary antibody used was Alexa Fluor^{®} 488 Goat Anti-Mouse IgG (H+L) (Molecular Probes, A-11001). Finally, the cell nuclei were co-stained by Hoechst 33258 (Invitrogen, H-3569) and imaged using a high content imager. The amount of TEAD degradation was determined by quantifying the fluorescence intensity levels of Anti-TEF1 in each individual cell per well. DC50 was calculated using curve fitting software Genedata Screener.

**Cell media:** The SK-HEP1 cells were cultured in the following media: MEM, + 10% FCS, +1x GlutaMAX, +100µM NEAA, +10mM HEPES, + 0.5% Pen/Strep.The NCI-H226 cells were cultured in the following media: RPMI 1640, +10% FBS, +1x GlutaMAX, +10mM HEPES, + 0.5% Pen/Strep.

Table 4 and Table 4a below summarize the results of the TEAD degradation assay. Values for the degradation concentration, DC50, at 16 hours and 24 hours, respectively, in Table 4 are given in µM. Ranges of values for the degration concentration, DC50, at 24 hours in Table 4a are as follows:

| | |
|---|---|
| A: | DC50: < 0.1 µM |
| B: | DC50: ≥ 0.1 µM and < 1.0 µM |
| C: | DC50: ≥ 1.0 µM and < 3.0 µM |
| D: | DC50: ≥ 3.0 µM and < 9 µM |
| E: | DC50: ≥ 9 µM and < 30 µM |
| n.d. | Not detectable below threshold given in parantheses |

**Table 4**

| | **DC50 [µM]** | | | |
|---|---|---|---|---|
| **Compound No.** | **SK-HEP1** | | **NCI-H226** | |
| | **16 h** | **24 h** | **16 h** | **24 h** |
| 3 | - | - | 3.0 | 10.0 |
| 4 | 2.0 | 2.0 | 0.7 | 0.9 |
| 5 | - | 30 | - | 20 |
| 7 | - | 2.4 | - | 20 |
| 9 | - | 2.4 | - | 30 |
| 10 | - | - | - | 20 |
| 16 | - | 0.25 | - | 0.6 |
| 19 | - | 0.04 | - | 0.07 |
| 20 | - | 0.19 | - | 0.3 |
| 24 | 0.7 | 0.7 | 1.0 | 1.0 |
| 25 | 1.0 | 1.0 | 1.0 | 1.0 |
| 28 | - | 0.07 | | 0.09 |
| 29 | 0.22 | 0.28 | 0.2 | 0.3 |
| 31 | - | 3.5 | - | 10.0 |
| 33 | - | 0.65 | - | 0.6 |
| 32 | - | 1.1 | - | - |

**Table 4a**

| **Compound No.** | **DC50 NCI-H226** | **DC50 SK-HEP1** |
|---|---|---|
| 1 | n.d. (30) | n.d. (30) |
| 2 | n.d. (30) | n.d. (30) |
| 3 | n.d. (9.5) | n.d. (9.5) |
| 4 | C | B |
| 5 | E | n.d. (30) |
| 6 | B | n.d. (30) |
| 7 | E | C |
| 8 | n.d. (30) | n.d. (30) |
| 9 | n.d. (30) | C |
| 10 | C | n.d. (30) |
| 11 | B | B |
| 12 | D | B |
| 13 | n.d. (30) | n.d. (30) |
| 14 | n.d. (30) | n.d. (30) |
| 15 | C | n.d. (30) |
| 16 | B | B |
| 18 | n.d. (30) | n.d. (30) |
| 17 | n.d. (30) | n.d. (30) |
| 19 | A | A |
| 20 | B | B |
| 36 | n.d. (30) | n.d. (30) |
| 4-N | n.d. (30) | n.d. (30) |
| 21 | n.d. (30) | n.d. (9.5) |
| 22 | n.d. (30) | n.d. (30) |
| 23 | n.d. (30) | n.d. (30) |
| 24 | B | B |
| 26 | n.d. (30) | n.d. (30) |
| 25 | C | B |
| 27 | n.d. (30) | n.d. (30) |
| 28 | A | A |
| 29 | B | B |
| 30 | n.d. (30) | n.d. (30) |
| 31 | E | D |
| 33 | B | B |
| 32 | C | C |
| 34 | B | B |
| 35 | A | A |
| 37 | B | B |
| 38 | B | B |
| 42 | B | A |
| 43 | B | E |
| 45 | A | B |
| 46 | B | B |
| 47 | A | A |
| 48 | n.d. (30) | n.d. (30) |
| 49 | n.d. (30) | n.d. (30) |
| 50 | A | E |
| 51 | n.d. (30) | n.d. (30) |
| 52 | B | B |
| 53 | B | B |
| 54 | n.d. (30) | n.d. (30) |
| 55 | B | B |
| 56 | B | B |
| 57 | C | C |
| 59 | n.d. (30) | n.d. (30) |
| 60 | B | B |
| 61 | D | D |
| 63 | n.d. (30) | n.d. (30) |
| 67 | n.d. (30) | n.d. (30) |
| 69 | E | D |
| 73 | E | D |
| 74 | C | B |
| 75 | n.d. (30) | n.d. (30) |
| 77 | n.d. (10) | B |
| 80 | B | A |
| 81 | n.d. (30) | n.d. (30) |
| 82 | B | B |
| 87 | A | A |
| 88 | B | B |
| 95 | B | B |

In addition. for **compound 29** the degradation concentration, DC50, after 6 h was determined to be 0.28 µM.

### 3. TEAD degradation by compound 4 in NCI-H226 cells - Simple Western

**Compound 4** was analyzed via Simple Western for its TEAD degradation potential in NCI-H226 cells (ATCC CRL-5826). The experiment was performed as follows:
NCI-H226 cells were seeded in 6-well plates and incubated at 37°C and 5% CO₂. After a 24 h or 48 h treatment with 0.1µM, 0.5µM, 1µM, 5µM, 10µM of compound 4 or DMSO (as control) the cells were lysed using HGNT lysis buffer (20mM HEPES pH7.4, 10% (v/v) Glycerin, 150mM NaCl, 1% (v/v) Triton-X-100, 2mM EDTA pH8, 25mM NaF + Protease inhibitors (Roche) + Phosphatase inhibitors (Roche)). Bicinchoninic acid (Thermo Scientific) was used for protein determination. The final protein concentration for Jess loading was 0.5 mg/ml per sample. Simple Western analysis was performed on a Jess system (ProteinSimple) using a 12-230 kDa Separation Module (ProteinSimple, SM-WO04) according to the manufacturer's instructions. Based on primary antibodies γ-Tubulin (4µg/ml; Sigma) and TEAD1 (0.6µg/ml; CST) the anti-mouse NIR and the anti-rabbit detection module were used (ProteinSimple, DM-009 and DM-001). Data analysis was accomplished using Compass Software Version 5.0.0 (ProteinSimple). The amount of TEAD degradation was determined by quantifying the peak area of TEAD1 and normalization to the respective γ-Tubulin peak area. Subsequently, DMSO control was set to 100% TEAD1 protein expression and the TEAD1 expression of each treatment condition was calculated accordingly. Results were blotted in a bar graph against compound concentration using GraphPad Prism Version 8.2.1.

**Cell media:** NCI-H226 cells were cultured in the following media: RPMI 1640, +10% FBS, +1x GlutaMAX, + 1mM Sodium Pyruvate.

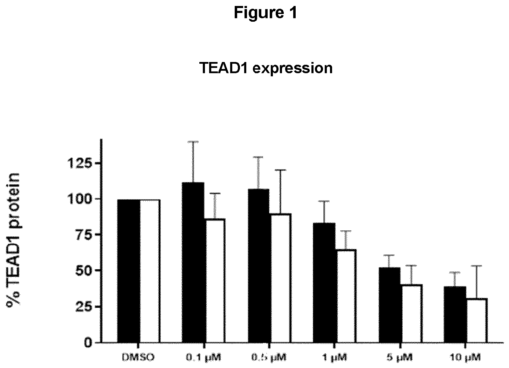

Figure 1 depicts the TEAD1 expression levels (in %, normalized to γ-Tubulin) in NCI-H226 cells at various concentrations of **compound 4** and at time points 24 hours (black bars) and 48 hours (white bars) after treatment. Cell lysates were analyzed via Simple Western and DMSO treated samples were set to 100% TEAD1 protein expression. Mean values of n=5 (24h) or n=2 (48h) experiments are shown. Error bars illustrate the standard deviation

### Formulations

The following examples relate to medicaments:

### Example A: Injection vials

A solution of 100 g of an active ingredient of the formula I and 5 g of disodium hydrogenphosphate in 3 l of bidistilled water is adjusted to pH 6.5 using 2 N hydrochloric acid, sterile filtered, transferred into injection vials, lyophilised under sterile conditions and sealed under sterile conditions. Each injection vial contains 5 mg of active ingredient.

### Example B: Suppositories

A mixture of 20 g of an active ingredient of the formula I with 100 g of soya lecithin and 1400 g of cocoa butter is melted, poured into moulds and allowed to cool. Each suppository contains 20 mg of active ingredient.

### Example C: Solution

A solution is prepared from 1 g of an active ingredient of the formula I, 9.38 g of NaH₂PO₄ · 2 H₂O, 28.48 g of Na₂HPO₄ · 12 H₂O and 0.1 g of benzalkonium chloride in 940 ml of bidistilled water. The pH is adjusted to 6.8, and the solution is made up to 1 l and sterilised by irradiation. This solution can be used in the form of eye drops.

### Example D: Ointment

500 mg of an active ingredient of the formula I are mixed with 99.5 g of Vaseline under aseptic conditions.

### Example E: Tablets

A mixture of 1 kg of active ingredient of the formula I, 4 kg of lactose, 1.2 kg of potato starch, 0.2 kg of talc and 0.1 kg of magnesium stearate is pressed in a conventional manner to give tablets in such a way that each tablet contains 10 mg of active ingredient.

### Example F: Dragees

Tablets are pressed analogously to Example E and subsequently coated in a conventional manner with a coating of sucrose, potato starch, talc, tragacanth and dye.

### Example G: Capsules

2 kg of active ingredient of the formula I are introduced into hard gelatine capsules in a conventional manner in such a way that each capsule contains 20 mg of the active ingredient.

### Example H: Ampoules

A solution of 1 kg of active ingredient of the formula I in 60 l of bidistilled water is sterile filtered, transferred into ampoules, lyophilised under sterile conditions and sealed under sterile conditions. Each ampoule contains 10 mg of active ingredient.

## Claims

1. Compound of the formula I
Q¹-Q²-Q³ I
wherein
Q¹ is a ubiquitin ligase ligand;
Q² is (i) absent; or (ii) a divalent linker formed by an unbranched alkylene chain having 2-25 C atoms, in which 1-8 non-adjacent CH₂ groups may independently from each other be replaced by O, -C(=O)-NH-, -NH-C(=O)-, -C(=O)-N(CH₃)-, -N(CH₃)-C(=O)-, - CH=CH- and/or -C=C-; in which one or two CH₂ groups may optionally bear a methyl substituent; and in which one CH₂ group may be replaced by a moiety selected from the group consisting of 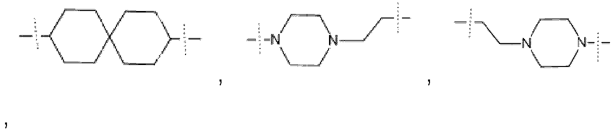 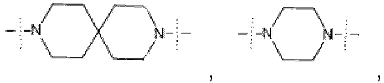 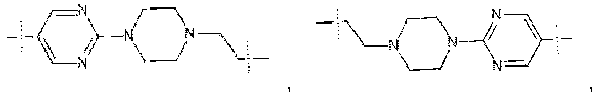 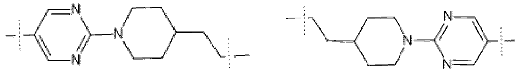 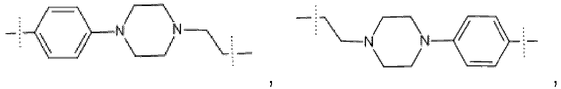 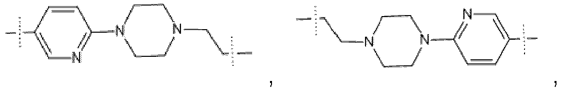 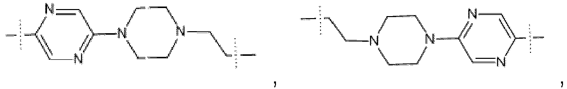 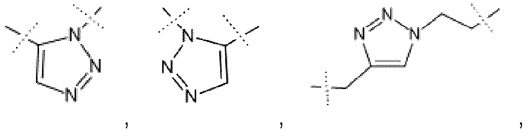 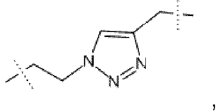 wherein the left-hand end of that moiety is directed towards the Q¹ part of the compound of formula I and the right-hand end of that moiety is directed towards the Q³ part of the compound of formula I
Q³ denotes 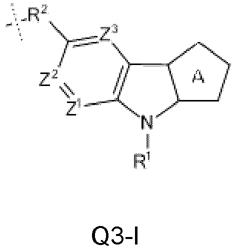 wherein
Ring A represents a five-membered heteroaromatic ring selected from the group consisting of the following ring moieties: 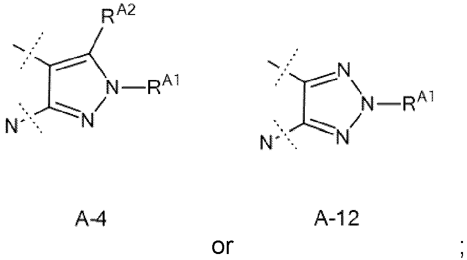 wherein
R^{A1} represents methyl ;
R^{A2} represents H;
Z¹ is CH;
Z² is CH;
Z³ is CH;
R¹ represents Ar¹, Hetar¹, Cyc¹, Hetcyc¹,
R² represents 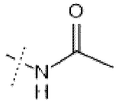 (-NH-C(=O)-);
Ar¹ is a mono- or bicyclic aryl with 6 or 10 ring carbon atoms, wherein that aryl may be unsubstituted or substituted with substituents R^{B1} R^{B2} and/or R^{B3} which may be the same or different;
Hetar¹ is a monocyclic heteroaryl with 5 or 6 ring atoms or a bicyclic heteroaryl with 9 or 10 ring atoms wherein 1, 2, 3 or 4 of said ring atoms is/are a hetero atom(s) selected from N, O and/or S and the remaining are carbon atoms, wherein that heteroaryl may be unsubstituted or substituted with substituents R^{B1}, R^{B2} and/or R^{B3} which may be the same or different;
Cyc¹ is a saturated or partially unsaturated, mono- or bicyclic carbocycle with 3, 4, 5, 6, 7, 8, 9 or 10 ring carbon atoms, wherein that carbocycle may be unsubstituted or substituted with R^{B4}, R^{B5} and/or R^{B6} which may be the same or different;
Hetcyc¹ is a saturated or partially unsaturated, monocyclic heterocycle with 5 or 6 ring atoms wherein 1 or 2 of said ring atoms is/are a hetero atom(s) selected from N, O and/or S and the remaining are carbon atoms, wherein that heterocycle may be unsubstituted or substituted with R^{B4}, R^{B5} and/or R^{B6} which may be the same or different, wherein, if one of the heteroatoms is S, then that heterocycle may also be substituted with R^{B4}, R^{B5}, R^{B6}, R^{B7} and/or R^{B8};
R^{B1}, R^{B2}, R^{B3} represent independently from each other straight-chain or branched C₁₋₆-alkyl, which C₁₋₆-alkyl may be unsubstituted or monosubstituted with -CN or substituted with 1, 2 or 3 halogen, straight-chain or branched C₁₋₄-alkoxy, which C₁₋₄-alkoxy may be unsubstituted or substituted with 1, 2 or 3 halogen, -O-CH₂-C≡CH, straight-chain or branched -S-C₁₋₄-alkyl, which -S-C₁₋₄-alkyl may be unsubstituted or substituted with 1, 2 or 3 halogen, straight-chain or branched C₂₋₆-alkenyl, which C₂₋₆-alkenyl may be unsubstituted or monosubstituted with -CN or substituted with 1, 2 or 3 halogen, F, Cl, Br, -CN, -S(=O)-C₁₋₃-alkyl, S(=O)₂-C₁₋₃-alkyl, -N(C₁₋₃-alkyl)₂, Ar², -CH₂-Ar², Hetar², Cyc², Hetcyc²;
or two adjacent R^{B1}, R^{B2} and/or R^{B3} form together a divalent -C₃₋₄-alkylene radical in which one of the alkylene carbon units may be replaced by a carbonyl unit (-C(=O)-), or a divalent -O-C₂₋₃-alkylene radical;
R^{B4}, R^{B5}, R^{B6} represent independently from each other F, C₁₋₄-alkyl, which C₁₋₄-alkyl may be unsubstituted or substituted with 1, 2 or 3 F, C₁₋₄-alkoxy, phenyl; or
two of R^{B4}, R^{B5} and R^{B6} are attached to the same carbon atom of said carbocycle Cyc¹ or said heterocycle Hetcyc¹ and form a divalent oxo (=O) group; or
R^{B4} and R^{B5} and R^{B7} and R^{B8} are attached to the same sulfur atom of said heterocycle and form two divalent oxo (=O) groups thereby forming an -S(=O)₂- moiety;
Ar² is phenyl which may be unsubstituted or substituted with one or two substituents which are independently from each other selected from OH, F, Cl, Br, C₁₋₄-alkyl and C₁₋₄-alkoxy, wherein that C₁₋₄-alkyl or C₁₋₄-alkoxy group may be substituted with 1, 2 or 3 F atoms;
Hetar² is a monocyclic heteroaryl with 5 or 6 ring atoms wherein 1, 2, 3, 4, 5 of said ring atoms is/are a hetero atom(s) selected from N, O and/or S and the remaining are carbon atoms, wherein that heteroaryl may be unsubstituted or substituted with one or two substituents which are independently from each other selected from F, Cl, Br, C₁₋₄-alkyl and C₁₋₄-alkoxy, wherein that C₁₋₄-alkyl or C₁₋₄-alkoxy group may be substituted with 1, 2 or 3 F atoms;
Cyc² is cyclopropyl, cyclobutyl, cyclopentyl or cyclohexyl, each of which may be unsubstituted or substituted with one or two substituents independently from each other selected from OH, F, Cl, Br, C₁₋₄-alkyl and C₁₋₄-alkoxy, wherein that C₁₋₄-alkyl or C₁₋₄-alkoxy group may be substituted with 1, 2 or 3 F atoms and/or 1 hydroxy group;
Hetcyc² is pyrrolidinyl, piperidinyl, each of which may unsubstituted or substituted with one or two substituents independently from each other selected from OH, F, Cl, Br, C₁₋₄-alkyl and C₁₋₄-alkoxy, wherein that C₁₋₄-alkyl or C₁₋₄-alkoxy group may be substituted with 1, 2 or 3 F atoms and/or 1 hydroxy group;
halogen is F, Cl, Br, I;
or a pharmaceutically acceptable salt, solvate, tautomer and/or stereoisomer thereof.

2. Compound according to claim 1, wherein
Q¹ is a E3 ubiquitin ligase ligand;
optionally Q¹ is a) a CRBN (cereblon) ligand or (b) a VHL (von Hippel-Lindau) ligand or (c) a different type of E3 uibiquitin ligase ligand other than a CRBN or a VHL ligand;
or a pharmaceutically acceptable salt, solvate, tautomer and/or stereoisomer thereof.

3. Compound according to claim 2, wherein (a) the CRBN (cereblon) ligand has a structure of formula Q1-I, Q1-II, Q1-VII or Q1-VIII; (b) the VHL ligand has a structure of formula Q1-III; (c) the different type of E3 uibiquitin ligase ligand has a structure of Q1-IV, Q1-V, Q1-VI, Q1-XII or Q1-XIII: 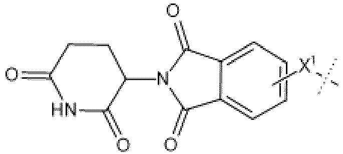 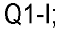 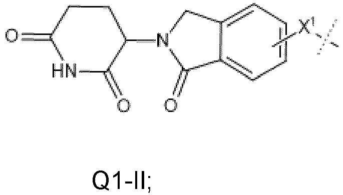 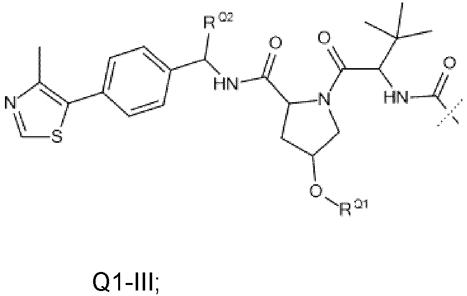 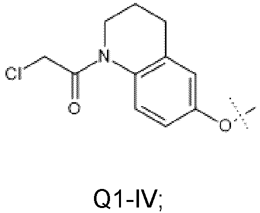 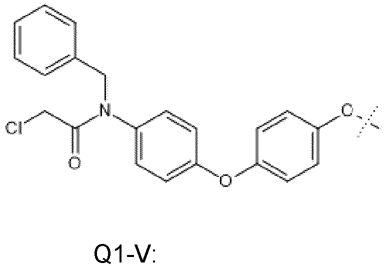 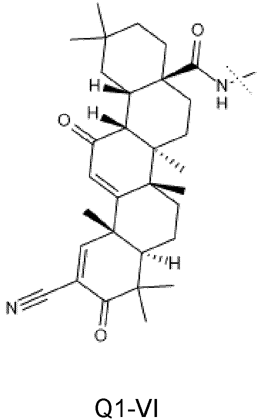 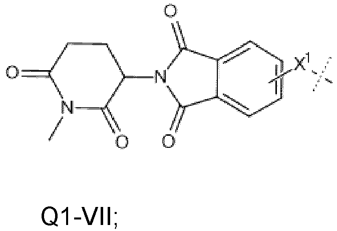 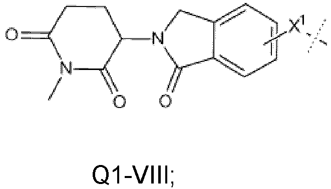 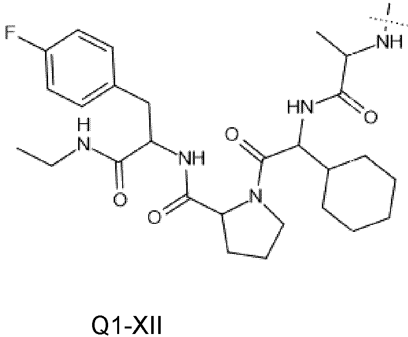 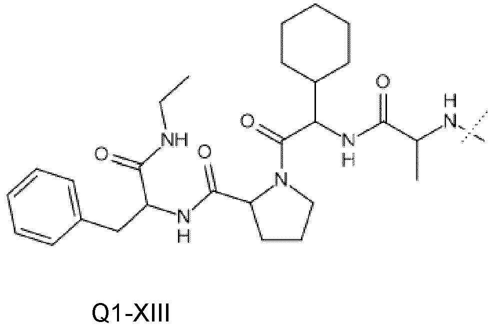 wherein
X¹ denotes a single bond, -O-, -NH-, -NCH₃-, -CH₂-, or -NH-C(=O)-;
R^{Q1} denotes H or -C(=O)-CH₃;
R^{Q2} denotes H or CH₃;
or a pharmaceutically acceptable salt, solvate, tautomer and/or stereoisomer thereof.

4. Compound according to any of the preceding claims, wherein Q¹ is a CRBN (cereblon) ligand; and the CRBN (cereblon) ligand has a structure selected from the structures of formulas Q1-I-1, Q1-I-2, Q1-I-3; Q1-I-4, Q1-I-5, Q1-I-6, Q1-II-1, Q1-II-2, Q1-II-3, Q1-II-4, Q1-II-5, Q1-II-6, Q1-II-7, Q1-VII-1, Q1-VII-2, and Q1-VII-3 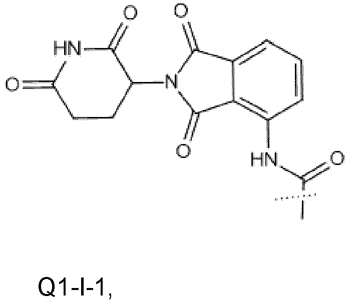 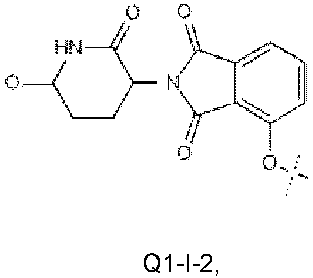 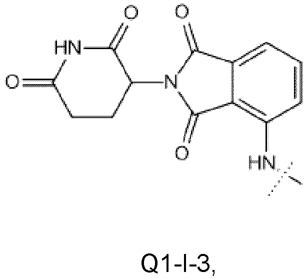 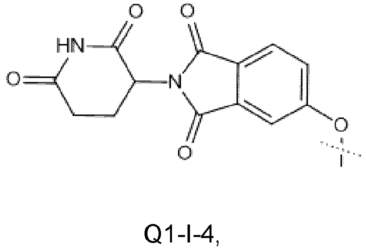 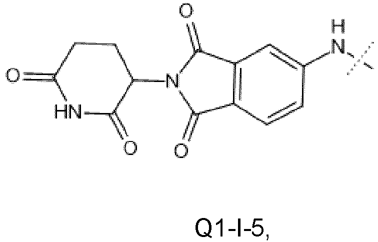 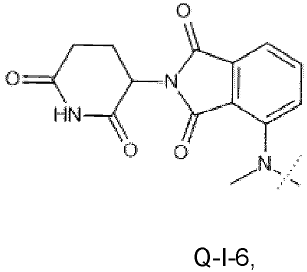 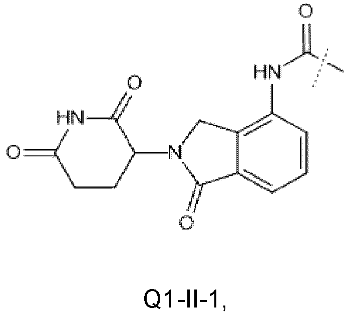 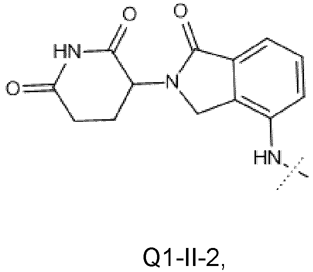 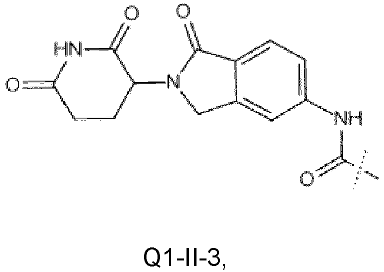 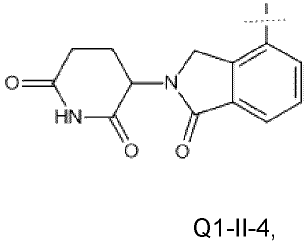 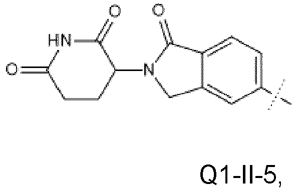 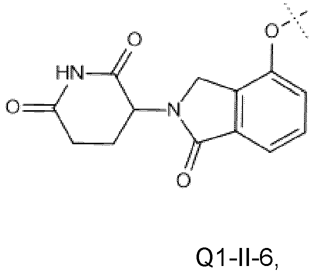 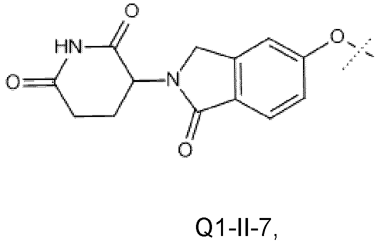 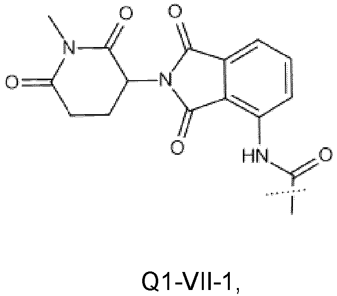 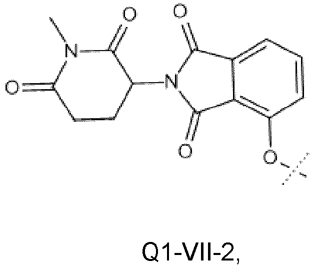 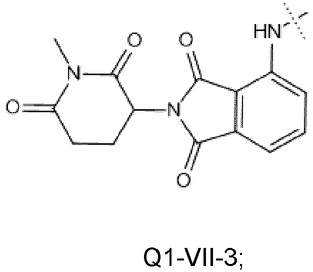 or a pharmaceutically acceptable salt, solvate, tautomer and/or stereoisomer thereof.

5. Compound according to any of claims 1 to 3, wherein Q¹ is a VHL (von-Hippel-Lindau) ligand; and the VHL ligand has a structure selected from formulas Q1-III-1, Q1-III-2, Q1-III-3, Q1-III-4, and Q1-III-5 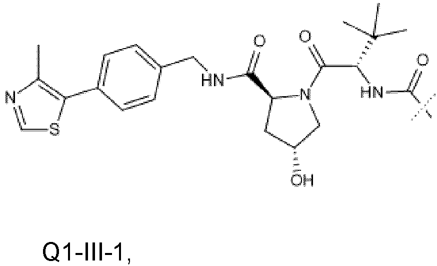 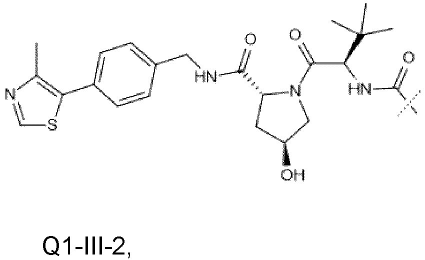 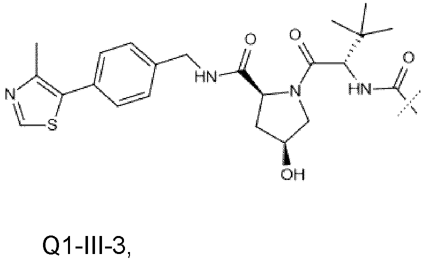 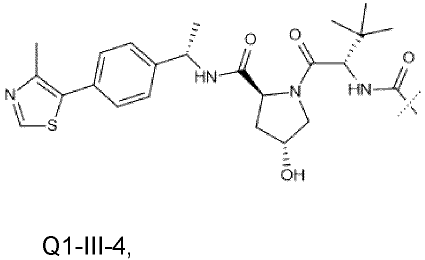 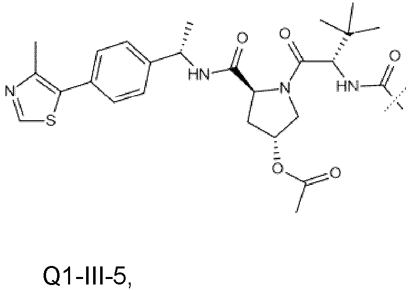 or a pharmaceutically acceptable salt, solvate, tautomer and/or stereoisomer thereof.

6. Compound of any of the preceding claims, wherein
Q² is selected from the group consisting of 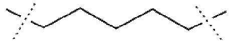 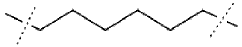 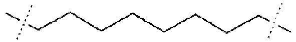 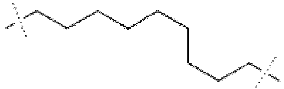 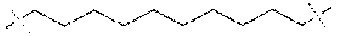 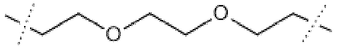 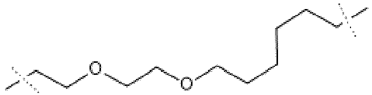 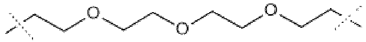 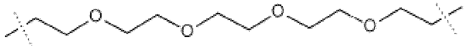 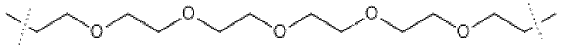 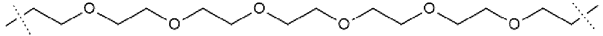 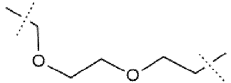 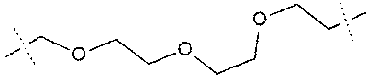 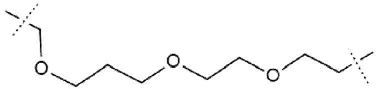 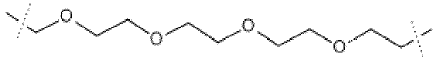 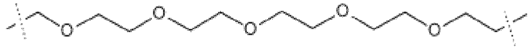 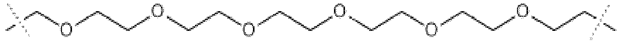 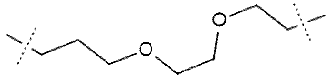 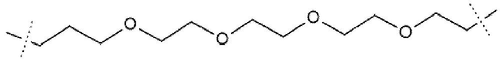 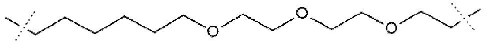 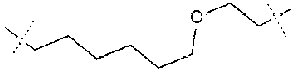 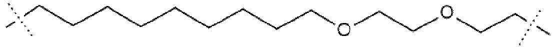 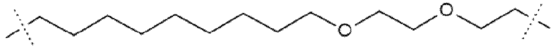 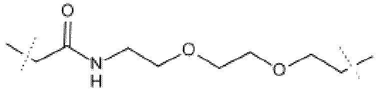 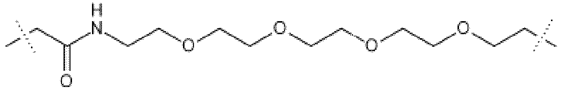 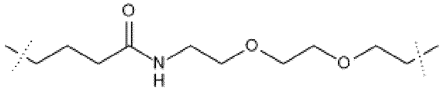 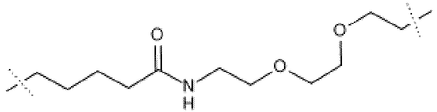 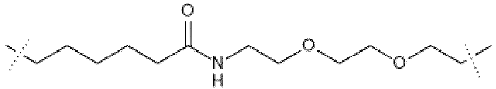 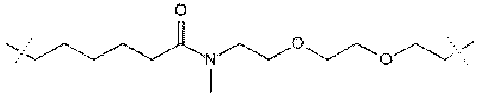 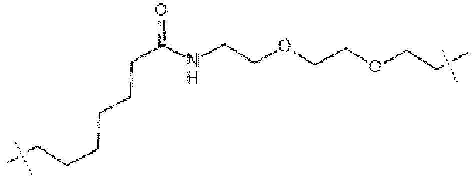 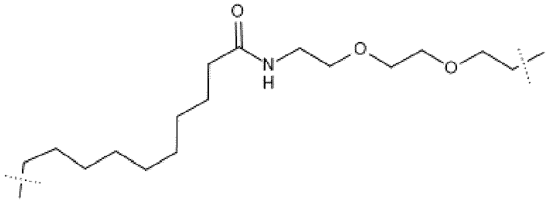 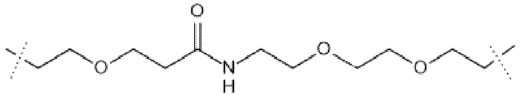 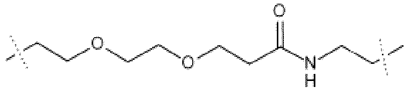 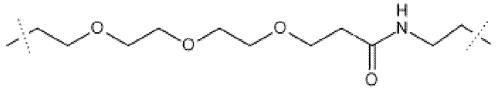 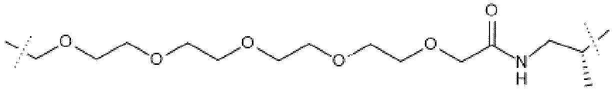 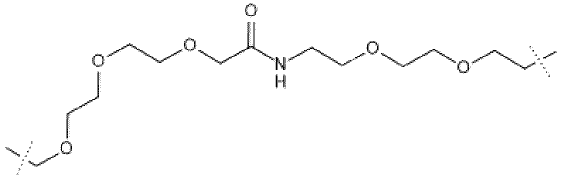 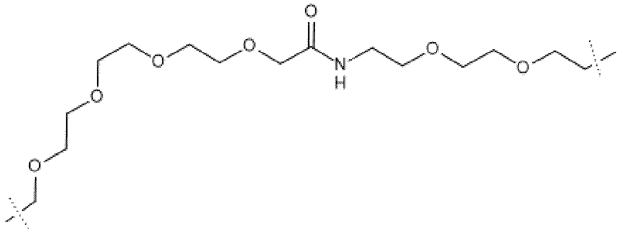 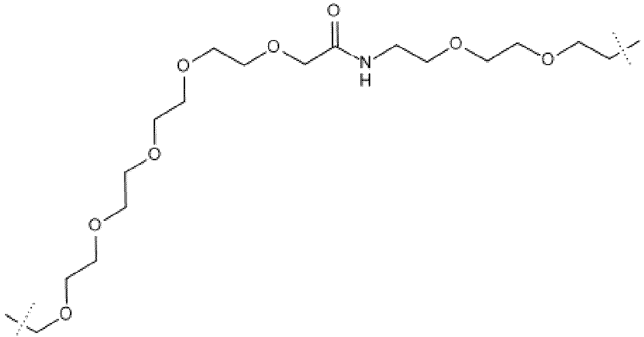 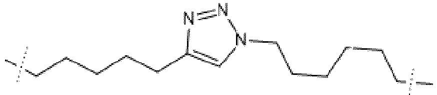 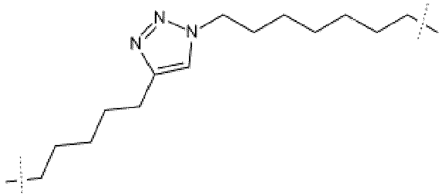 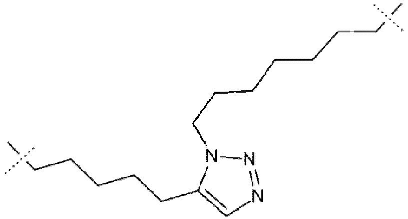 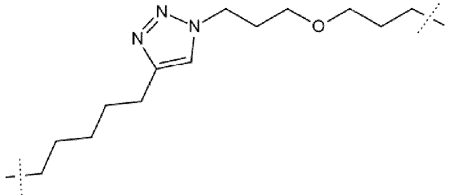 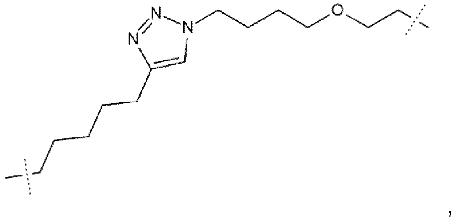
optionally Q2 is selected from the group consisting of 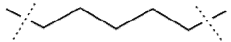 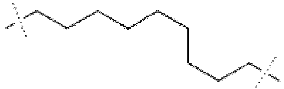 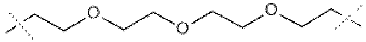 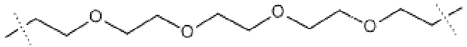 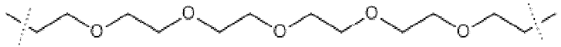 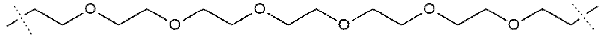 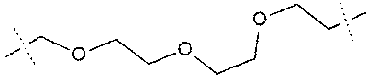 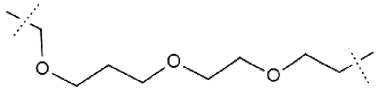 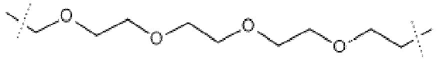 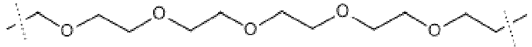 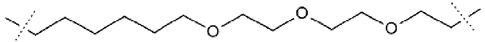 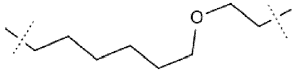 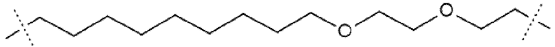 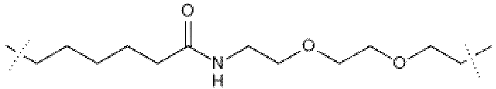 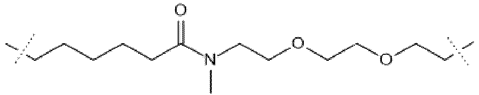 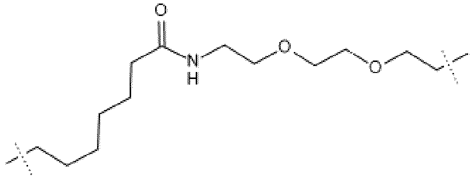 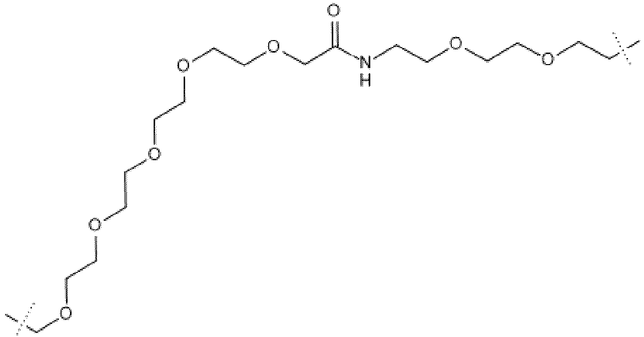 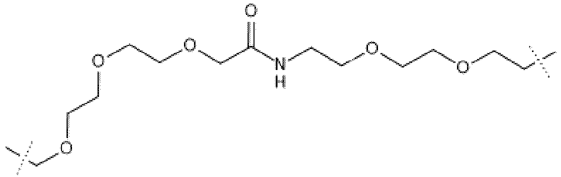 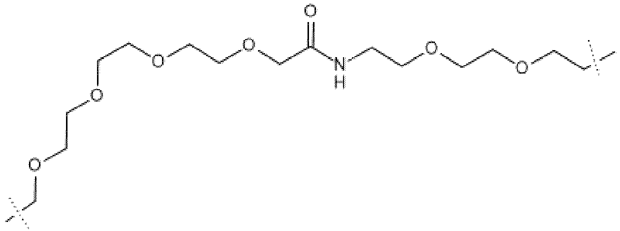 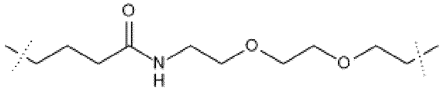 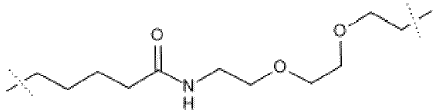 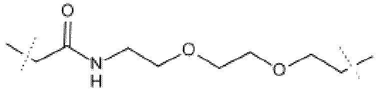 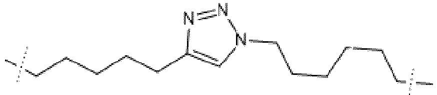 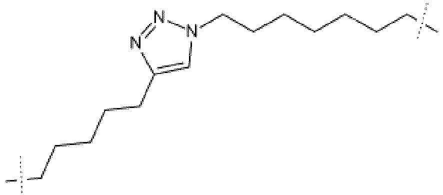 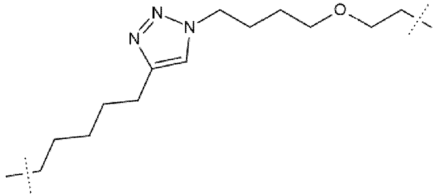 or a pharmaceutically acceptable salt, solvate, tautomer and/or stereoisomer thereof.

7. Compound according to any preceding claim, wherein
R¹ represents phenyl, 3-fluorophenyl, 4-fluorophenyl, 4-chlorophenyl, 4-methylphenyl, 4-ethylphenyl, 4-difluoromethylphenyl, 3-trifluoromethylphenyl, 4-trifluoromethylphenyl, 4-(1,1-difluorethyl)phenyl, 4-(2,2,2-trifluorethyl)phenyl, 4-(1-trifluoromethylcyclopropyl)-phen-1-yl, 4-cyclopentylphenyl, 4-ethoxyphenyl, 4-difluormethoxyphenyl, 4-trifluoromethoxyphenyl, 3-(trifluoromethyl)sulfanylphenyl, 4-(trifluoromethyl)sulfanylphenyl, 3-trifluoromethyl-4-methylphenyl, 2-fluoro-4-trifluoromethylphenyl, 2-fluoro-4-trifluoromethoxyphenyl, 3-fluoro-4-(n-propyl)phenyl, 2,3-dimethyl-4-methoxyphenyl, 6-fluoronaphth-2-yl; 5-trifluoromethylfuran-2-yl; 5-trifluoromethylthiophen-2-yl, 2-trifluoromethyl-1,3-thiazol-4-yl, 3-fluoropyridin-2-yl, 6-methylpyridin-3-yl, 6-methoxypyridin-3-yl, 3-ethylpyridin-2-yl, 6-ethylpyridin-3-yl, 4-difluoromethylpyridin-2-yl, 4-trifluoromethylpyridin-2-yl, 4-trifluoromethoxypyridin-2-yl, 4-cyanopyridin-2-yl, 5-trifluoromethylpyridin-2-yl, 6-trifluoromethylpyridin-2-yl, 6-trifluoromethylpyridin-3-yl (2-trifluoromethylpyridin-5-yl), 6-trifluoromethoxypyridin-3-yl (2-trifluoromethoxypyridin-5-yl), 5-cyanopyridin-2-yl, 5-cyanomethylpyridin-2-yl, 5-methanesulfonylpyridin-2-yl, 6-methoxypyridin-2-yl, 4-methylpyrimidin-2-yl, 4-ethylpyrimidin-2-yl, 4-methylsulfanylpyrimidin-2-yl, 5-cyclopropylpyrimidin-2-yl, 5-ethylpyrimidin-2-yl, 5-difluoromethylpyrimidin-2-yl, 5-trifluoromethylpyrimidin-2-yl, 5-cyanopyrimidin-2-yl, 5-cyano-3-fluoropyridin-2-yl, 5-cyano-6-methylpyridin-2-yl, 3-fluoro-5-(trifluoromethyl)pyridin-2-yl, 5-oxo-5H,6H,7H-cyclopenta[b]pyridin-2-yl, 5,6,7,8-tetrahydroquinolin-2-yl, 5-oxo-5,6,7,8-tetrahydroquinolin-2-yl, 5H,6H,7H-cyclopenta[b]pyridin-2-yl, quinolin-2-yl, isoquinolin-3-yl, 6-methylquinolin-2-yl, 8-methoxyquinolin-4-yl, furo[3,2-b]pyridin-5-yl, quinazolin-2-yl, 6-fluoroquinazolin-2-yl, 1,5-naphthyridin-2-yl; 3-methylcyclobutyl, cyclopentyl, 3-methylcyclopentyl, 3,3-dimethylcyclopentyl, 3-trifluoromethyl-bicyclo[1.1.1]petan-1-yl, cyclohexyl, 4-methylcyclohexyl, 4-(trifluoromethyl)cyclohexyl, 4,4-difluorocyclohexyl, cyclohex-1-enyl, 2-oxocycloheptyl, 6,6-difluorospiro[3.3]heptan-2-yl, 1H-inden-2-yl;
or a pharmaceutically acceptable salt, solvate, tautomer and/or stereoisomer thereof.

8. Compound according to any preceding claim, wherein
R¹ represents 4-methylphenyl, 4-difluoromethylphenyl, 4-trifluormethylphenyl, 4-fluorophenyl;
or a pharmaceutically acceptable salt, solvate, tautomer and/or stereoisomer thereof.

9. Compound according to any preceding claim, wherein
Q³ has a structure of formula Q3-I;
Ring A is ring A-4 or A-12;
R^{A1} is methyl;
R^{A2} is H;
R¹ is 4-trifluormethylphenyl;
R² represents 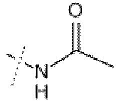 (-NH-C(=O)-);
Z¹, Z² and Z³ each represent CH;
or a pharmaceutically acceptable salt, solvate, tautomer and/or stereoisomer thereof.

10. Compound according to any of the preceding claims, wherein
Q¹ is a
(a) CRBN (cereblon) ligand; and
the CRBN (cereblon) ligand has a structure selected from the structures of formulas Q1-I-1, Q1-I-2, Q1-I-3; Q1-I-4, Q1-I-5, Q1-I-6, Q1-II-1, Q1-II-2, Q1-II-3, Q1-II-4, Q1-II-5 and Q1-VII-1 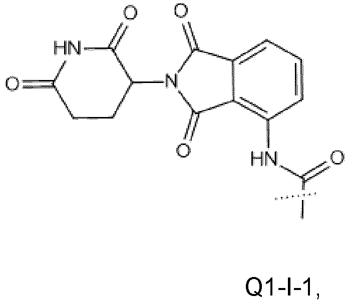 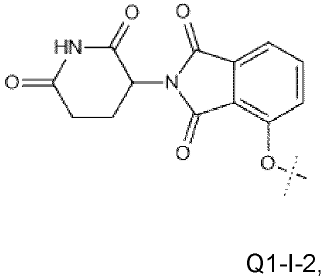 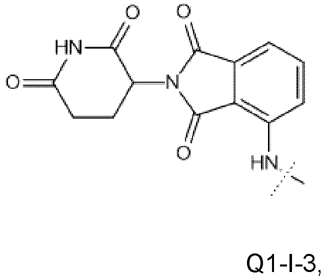 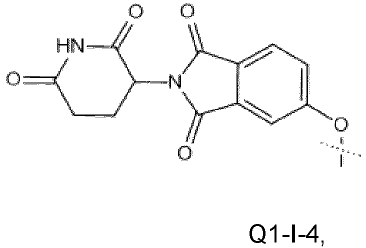 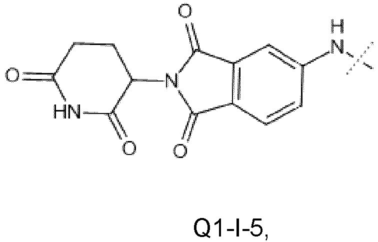 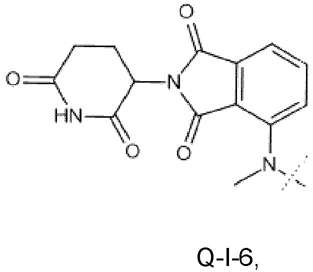 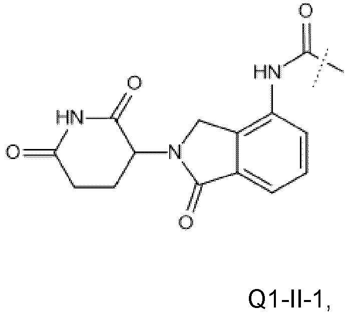 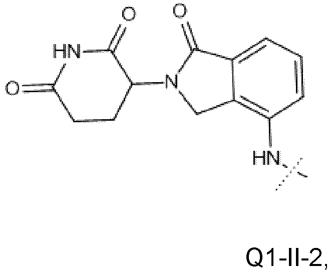 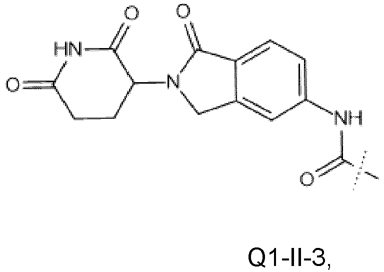 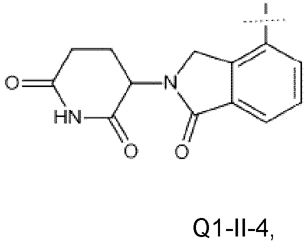 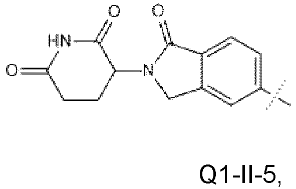 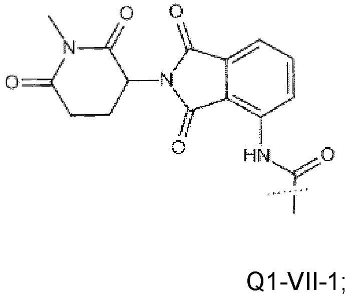 or
(b) a VHL (von Hippel-Lindau) ligand; and the VHL ligand has a structure of selected from the structures of formula Q1-III-1, Q1-III-2, Q1-III-4, and Q1-III-5: 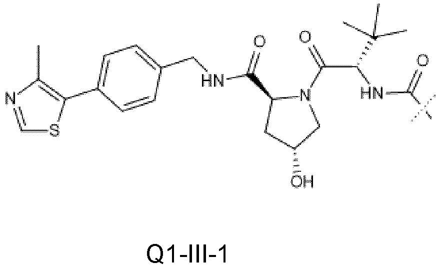 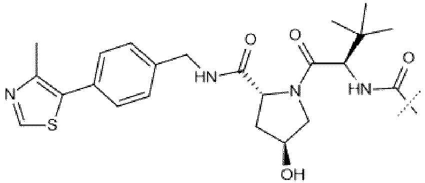 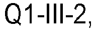 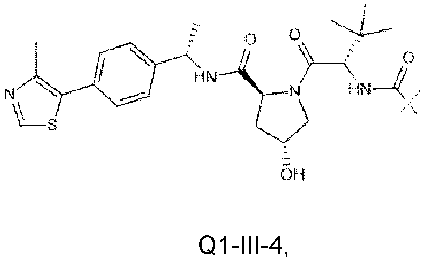 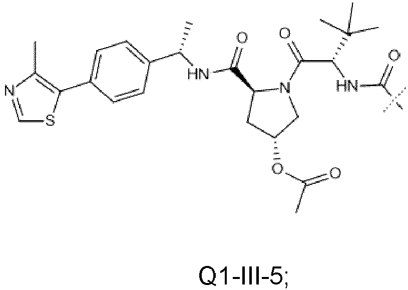
Q² is selected from the group consisting of 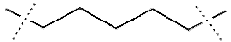 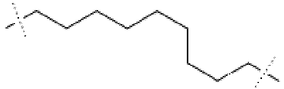 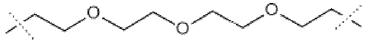 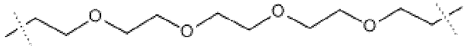 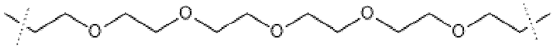 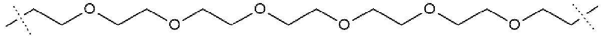 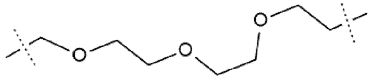 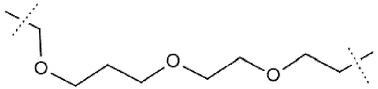 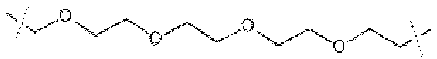 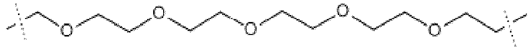 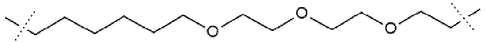 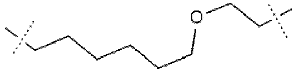 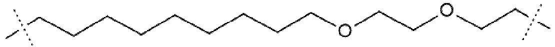 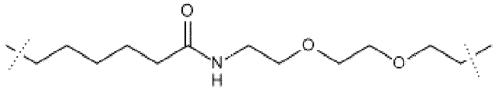 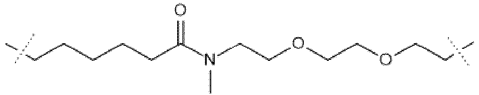 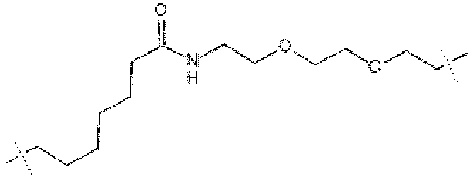 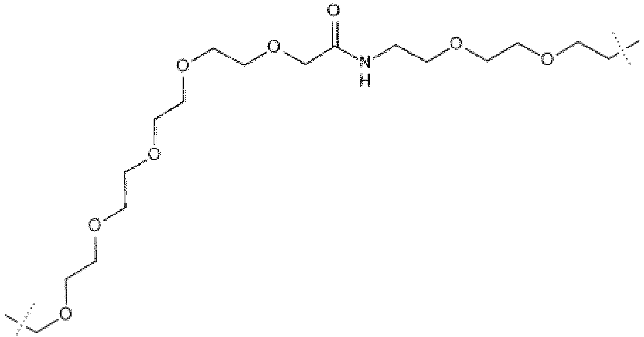 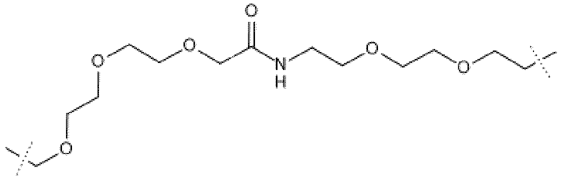 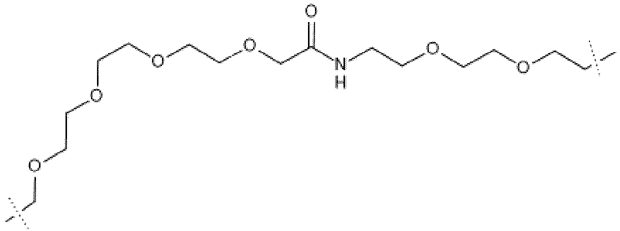 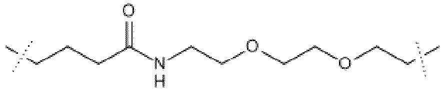 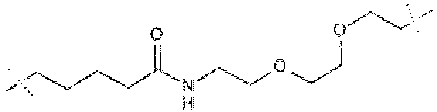 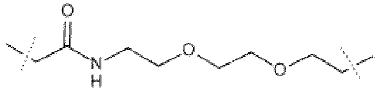 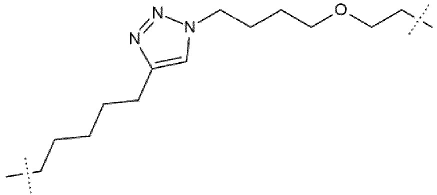 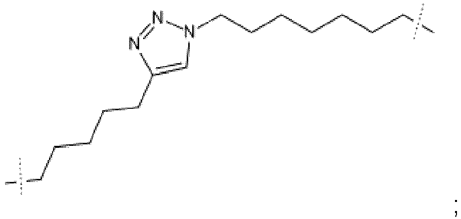
Q³ is Q3-I;
Ring A is ring A-4 or ring A-12;
R^{A1} represents methyl;
R^{A2} represents hydrogen;
R¹ denotes 4-trifluoromethylphenyl;
R² represents 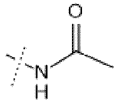 (-NH-C(=O)-);
Z¹, Z² and Z³ each represent CH;
or a pharmaceutically acceptable salt, solvate, tautomer and/or stereoisomer thereof.

11. Compound according to claim 10 wherein
Q¹ is a (a) CRBN ligand and has a structure selected from the structures of formula Q1-I-2, Q1-I-3, Q1-I-5, Q1-I-6, Q1-II-2, Q1-II-3, Q1-II-4, and Q1-II-5; or is a (b) VHL ligand and has structure selected from the structures of formula Q1-III-1 and Q1-III-4;
or a pharmaceutically acceptable salt, solvate, tautomer and/or stereoisomer thereof.

12. Compound according to claim 1, wherein the compound is selected from Table 1
| **Compound No.** | **Structure** |
|---|---|
| 1 | 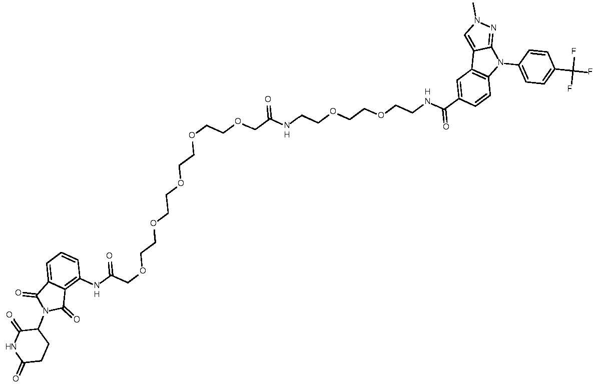 |
| 2 | 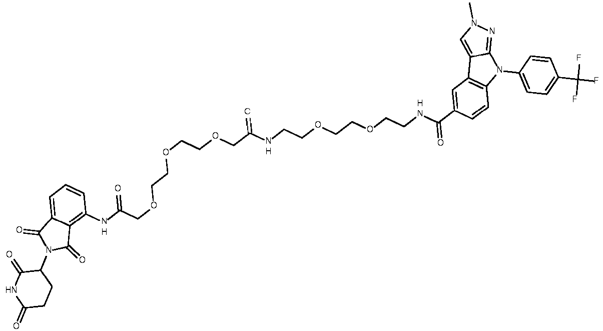 |
| 3 | 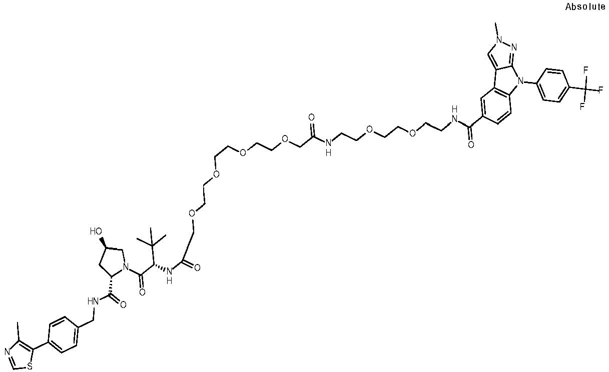 |
| 4 | 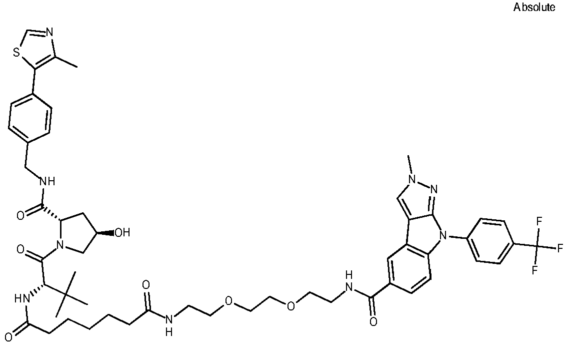 |
| 4-N | 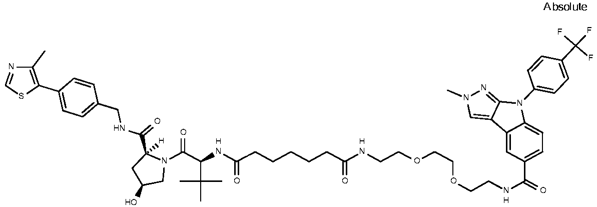 |
| 5 | 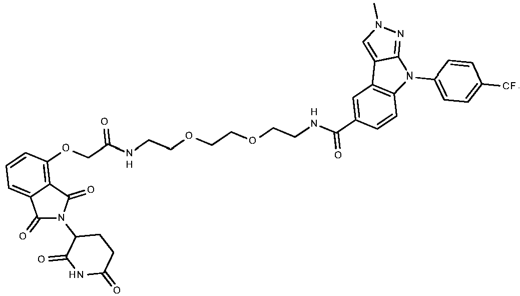 |
| 6 | 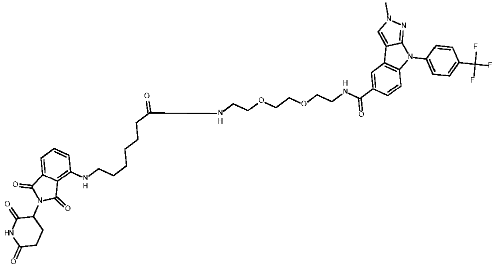 |
| 7 | 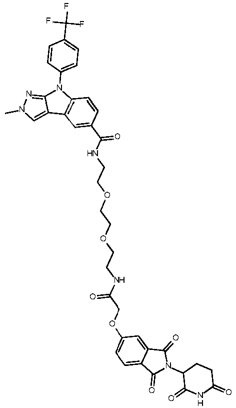 |
| 8 | 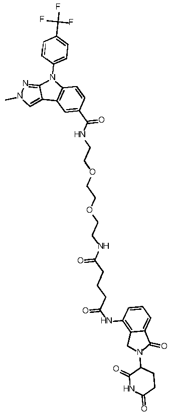 |
| 9 | 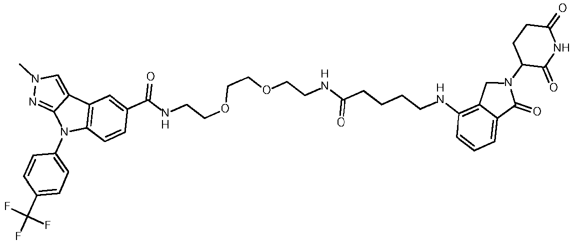 |
| 10 | 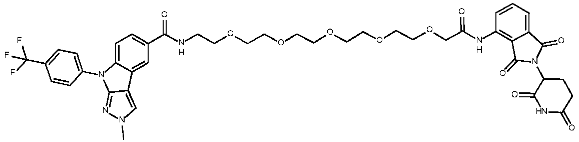 |
| 11 | 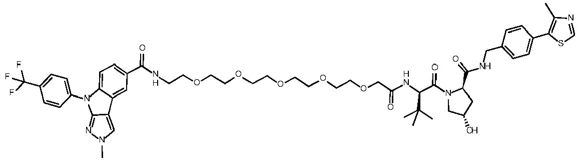 |
| 12 | 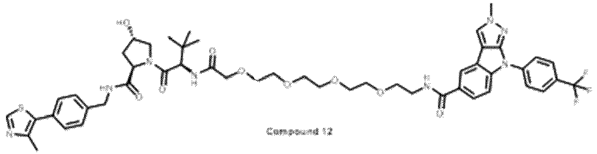 |
| 13 | 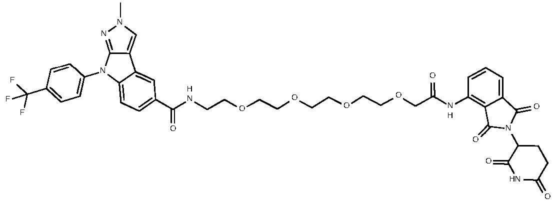 |
| 14 | 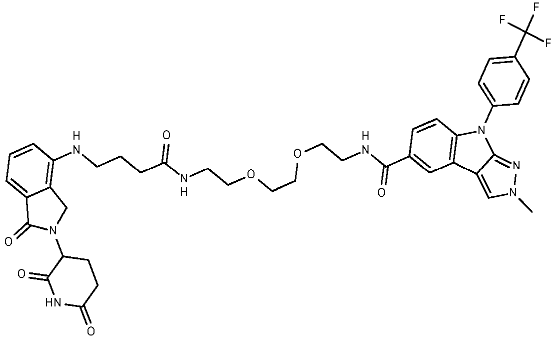 |
| 15 | 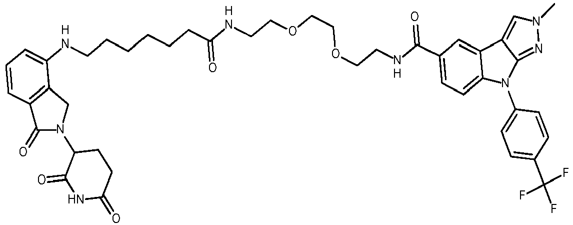 |
| 16 | 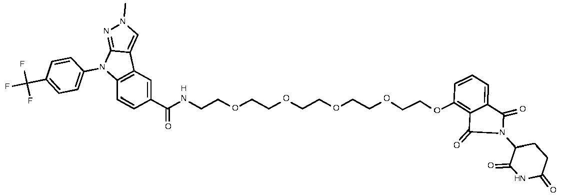 |
| 17 | 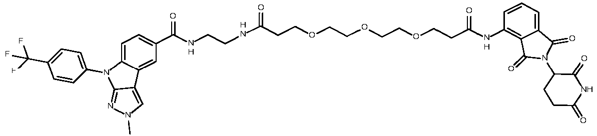 |
| 18 | 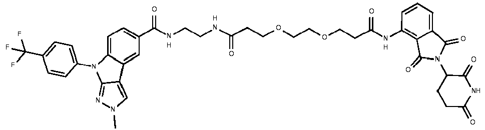 |
| 19 | 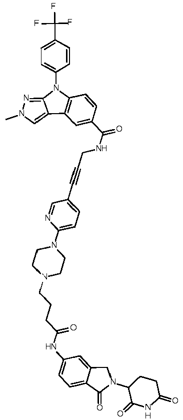 |
| 20 | 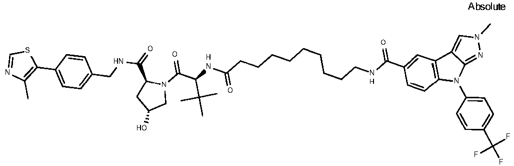 |
| 21 | 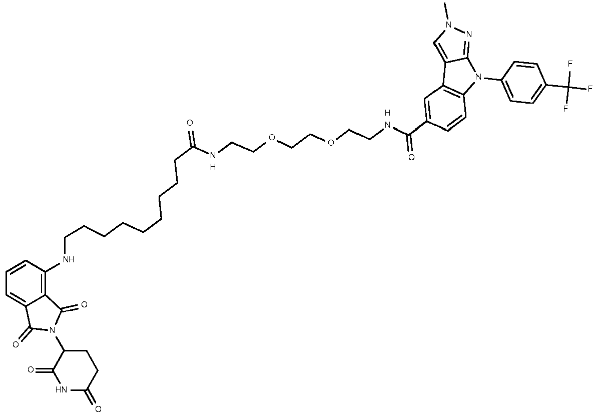 |
| 22 | 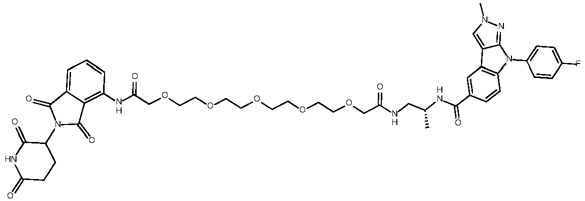 |
| 23 | 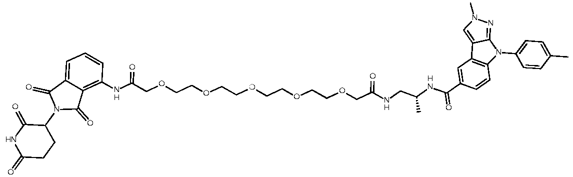 |
| 24 | 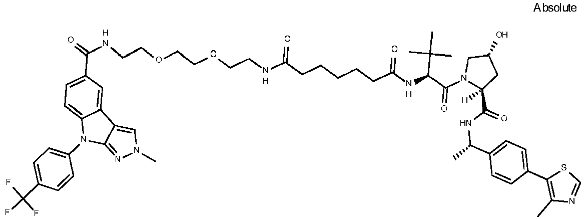 |
| 25 | 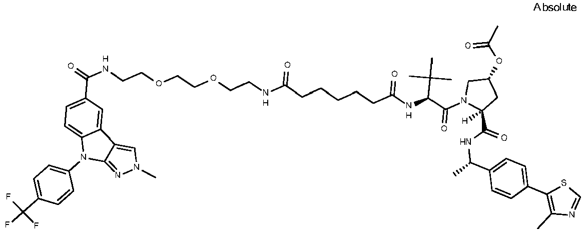 |
| 26 | 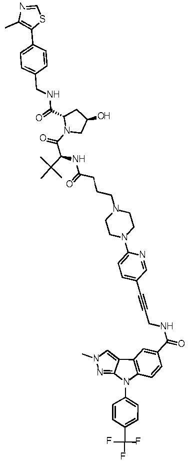 |
| 27 | 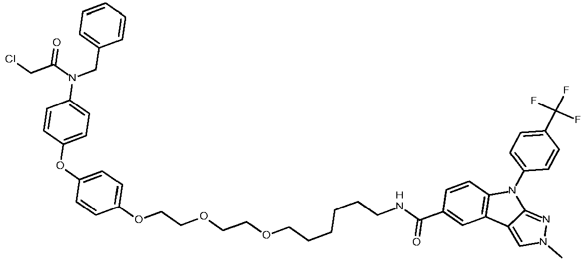 |
| 28 | 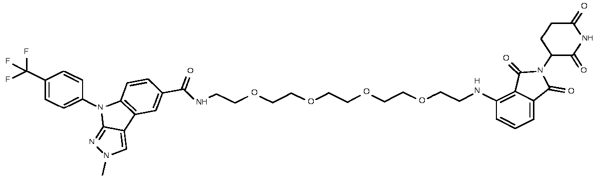 |
| 29 | 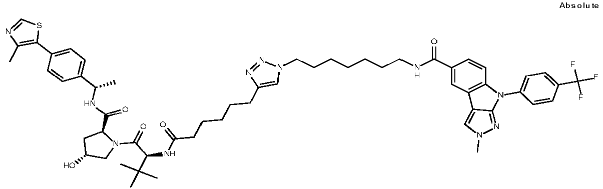 |
| 30 | 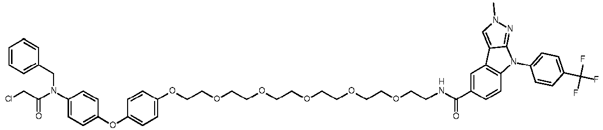 |
| 31 | 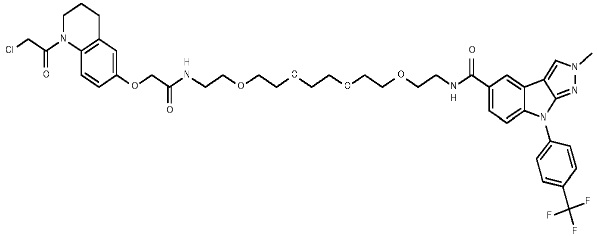 |
| 32 | 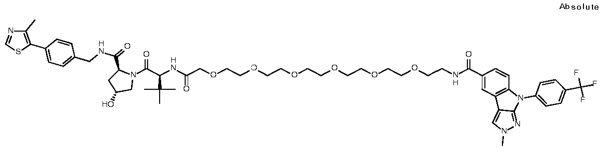 |
| 33 | 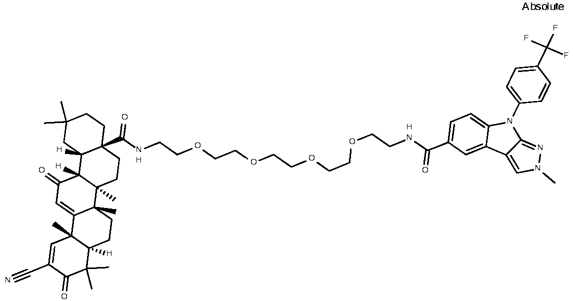 |
| 34 | 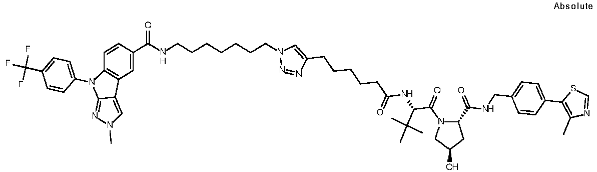 |
| 35 | 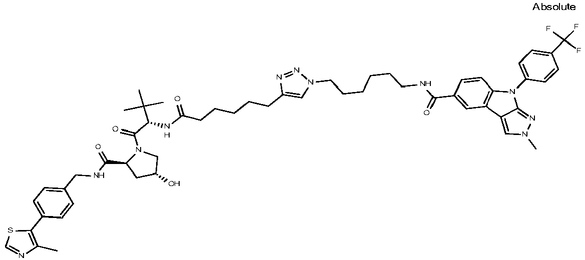 |
| 36 | 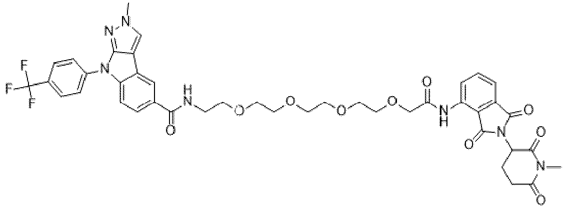 |
| 37 | 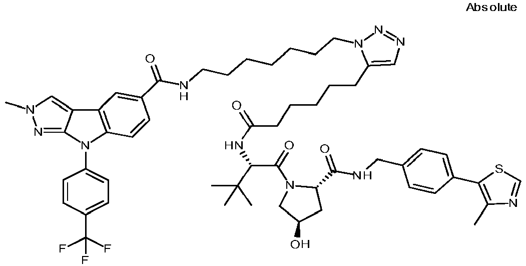 |
| 38 | 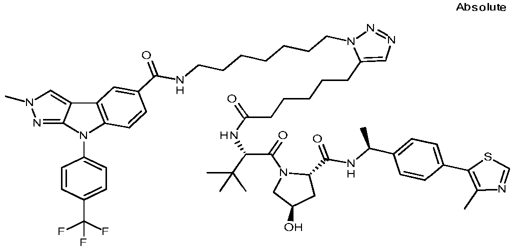 |
| 42 | 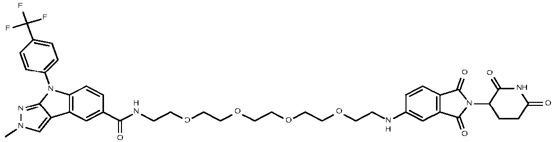 |
| 43 | 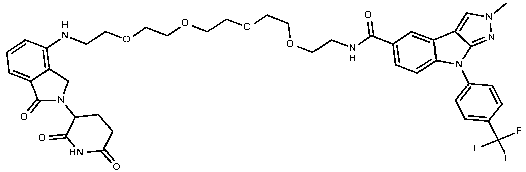 |
| 45 | 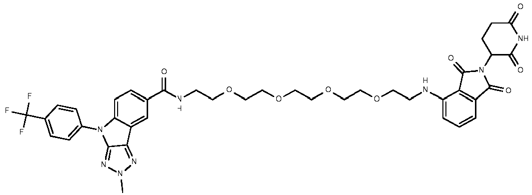 |
| 47 | 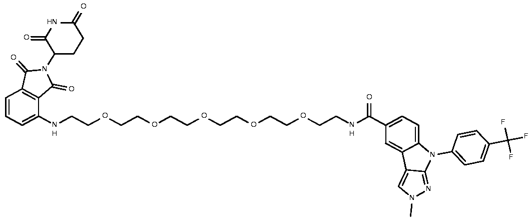 |
| 48 | 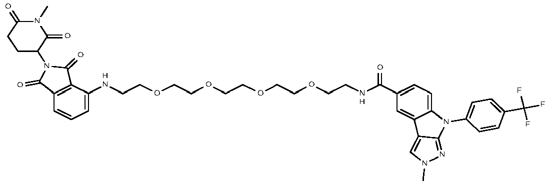 |
| 49 | 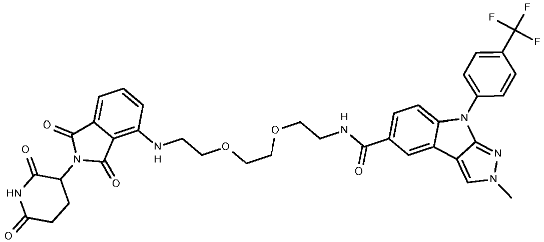 |
| 50 | 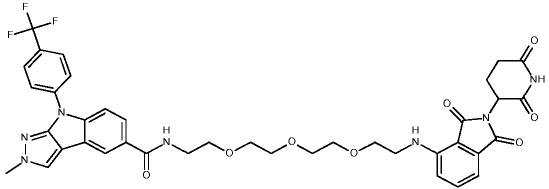 |
| 51 | 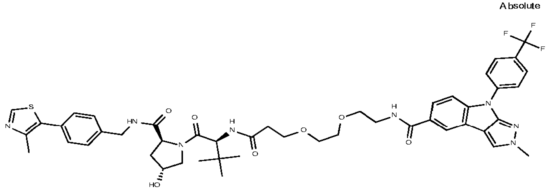 |
| 52 | 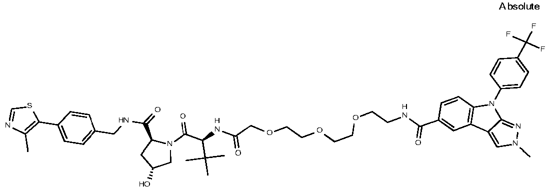 |
| 53 | 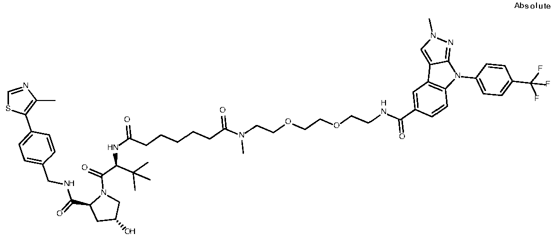 |
| 54 | 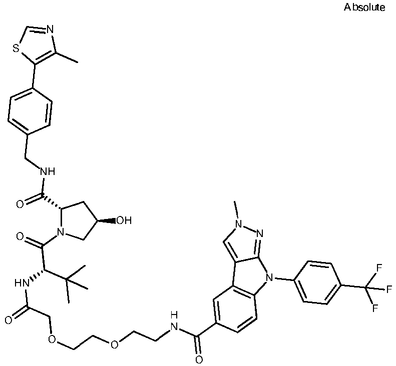 |
| 55 | 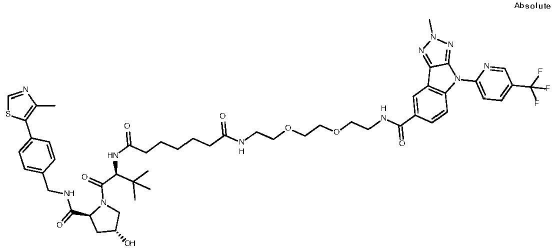 |
| 56 | 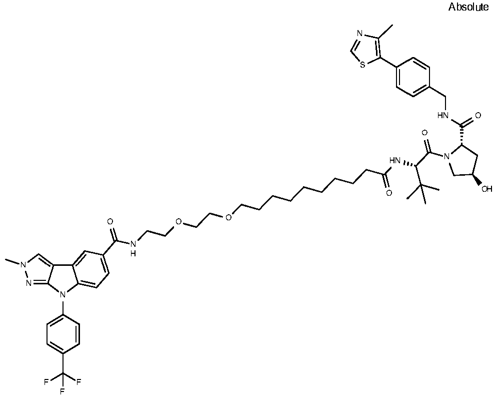 |
| 57 | 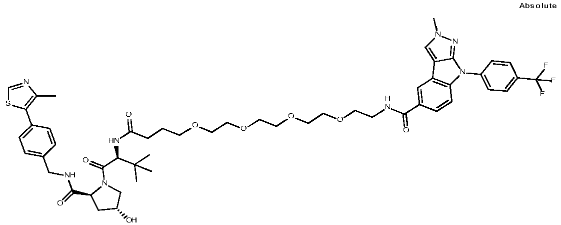 |
| 59 | 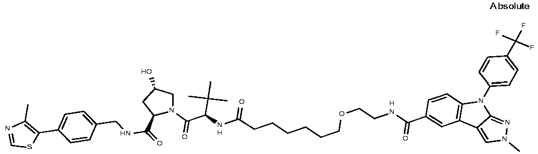 |
| 60 | 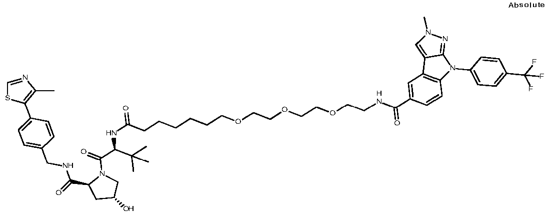 |
| 61 | 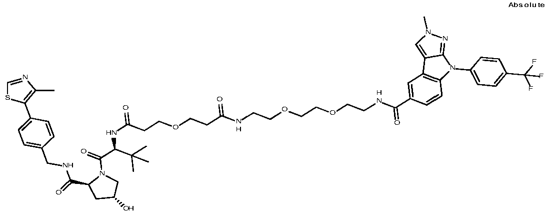 |
| 63 | 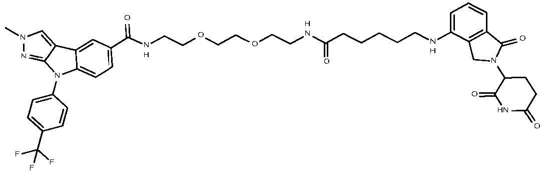 |
| 69 | 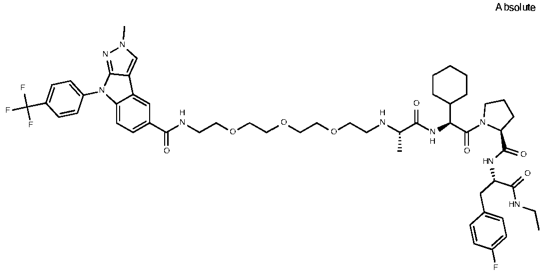 |
| 73 | 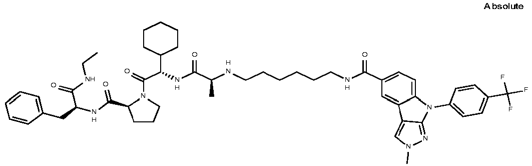 |
| 74 | 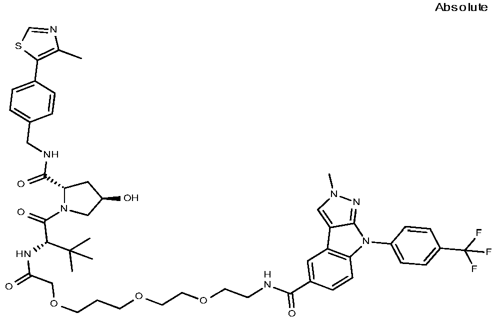 |
| 75 | 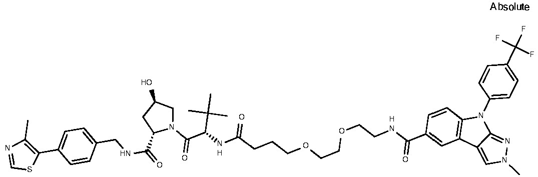 |
| 77 | 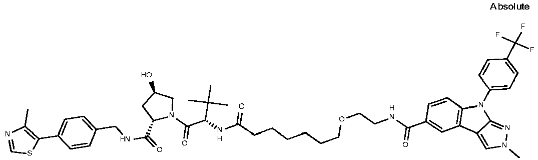 |
| 80 | 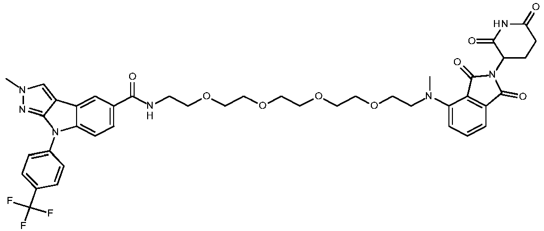 |
| 81 | 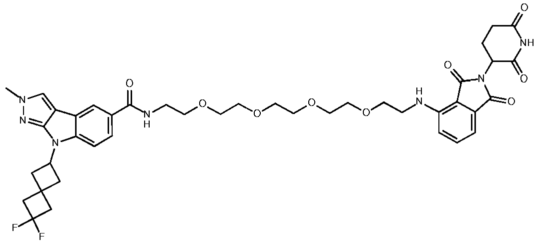 |
| 82 | 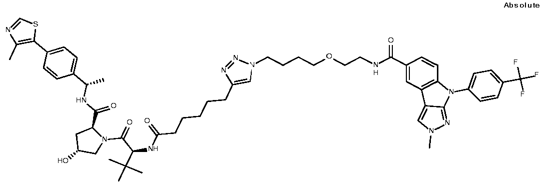 |
| 84 | 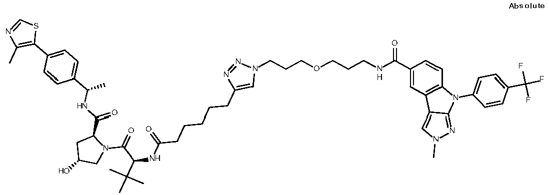 |
| 87 | 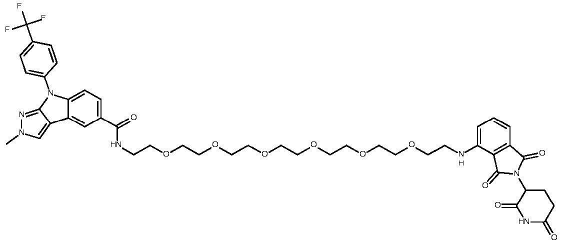 |
| 88 | 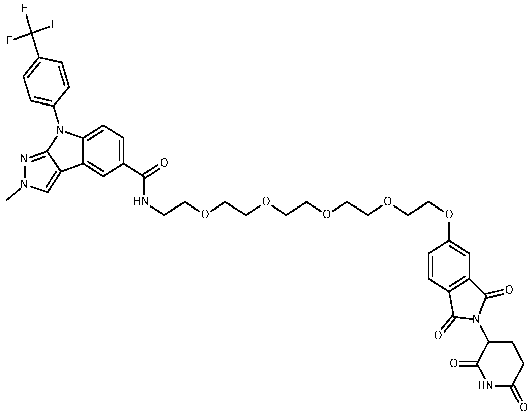 |
| 95 | 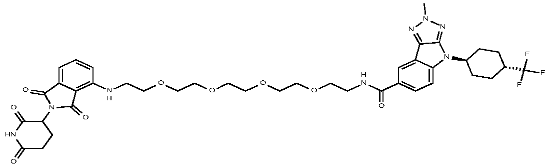 |
| 110 | 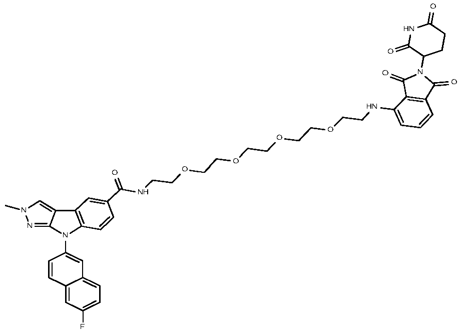 |
| 117 | 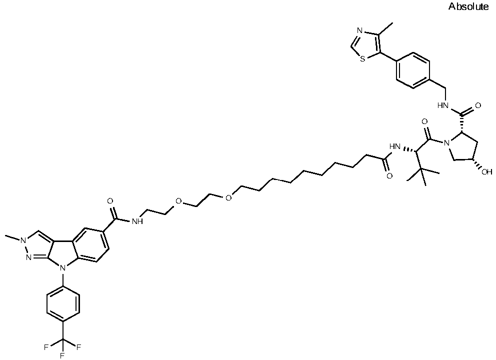 |
, or a pharmaceutically acceptable salt, solvate, tautomer and/or stereoisomer thereof.

13. Compound according to any preceding claim for use as a medicament.

14. Compound according to any one of claims 1 to 12, or a pharmaceutically acceptable salt, solvate, tautomer and/or stereoisomer thereof, for use in the treatment and/or prevention of a disease or condition selected from the group consisting of: cancer, in particular tumors including solid tumors, of breast cancer, lung cancer, liver cancer, ovarian cancer, squamous cancer, renal cancer, gastric cancer, medulloblastoma, colon cancer, pancreatic cancer; cardiovascular diseases and fibrosis, in particular, liver fibrosis.

15. A pharmaceutical composition comprising a compound according to any one of claims 1 to 12, or a pharmaceutically acceptable salt, solvate, tautomer and/or stereoisomer thereof, as an active ingredient together with a pharmaceutically acceptable carrier.

16. The pharmaceutical composition according to claim 15 that further comprises a second active, or a pharmaceutically acceptable salt, solvate, tautomer and/or stereoisomer thereof, wherein that second active ingredient is other than a compound of formula I as defined in any one of claims 1 to 12.

17. Set (kit) comprising separate packs of
a) an effective amount of a compound of formula I according to any one of claims 1 to 12, or a pharmaceutically acceptable salt, solvate, tautomer and/or stereoisomer thereof; and
b) an effective amount of a further active ingredient that further active ingredient not being a compound of formula I as defined in any one of claims 1 to 12.

18. A method of degrading a TEAD protein, comprising contacting the TEAD protein with a compound as defined in any one of claims 1 to 12.

19. A ternary complex, comprising
(i) a compound of any one of claims 1 to 12;
(ii) a TEAD protein; and
(iii) a ubiquitin ligase.

20. Compound selected from the group consisting of the intermediates depicted in Table INT
| **Intermediate** | **Structure** |
|---|---|
| 1 | 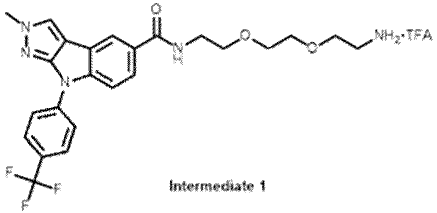 |
| 8 | 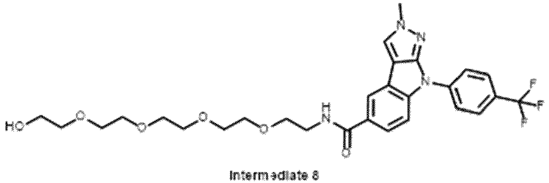 |
| 9 | 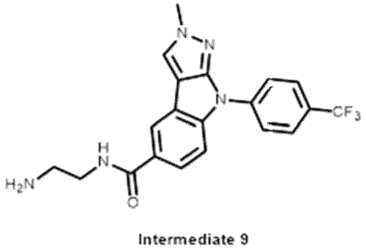 |
| 10 | 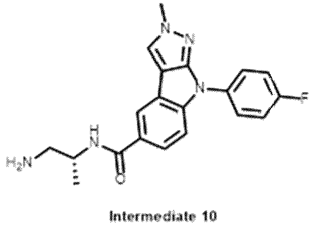 |
| 11 | 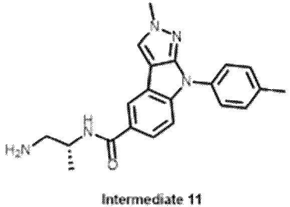 |
| 20 | 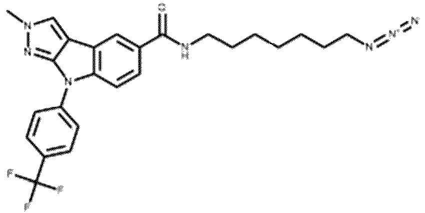 |
| 29 | 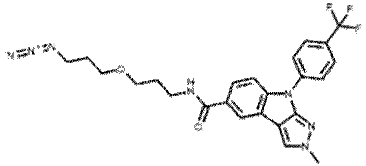 |
| 33 | 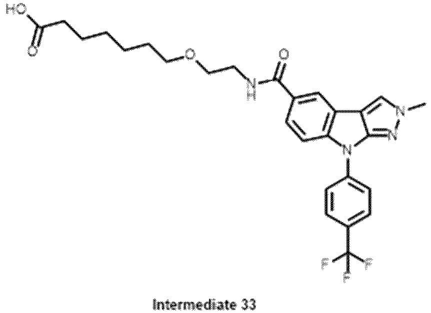 |
| 34 | 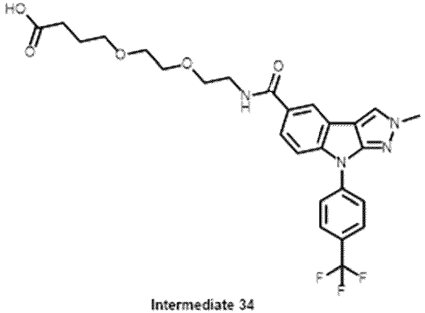 |
| 37 | 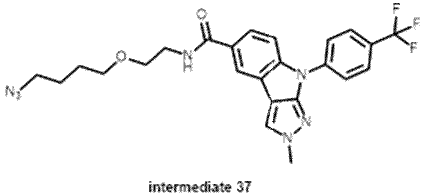 |
| 38 | 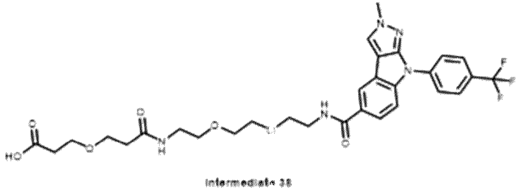 |
| 39 | 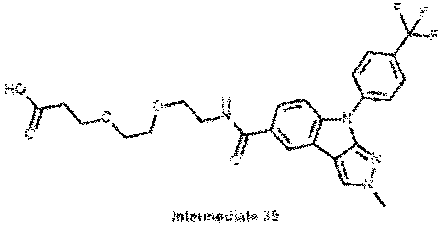 |
| 40 | 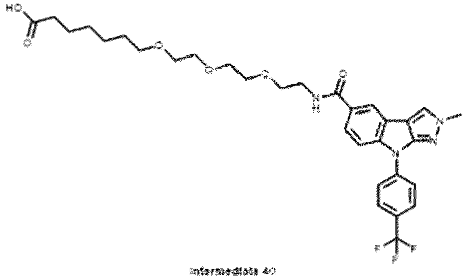 |
| 41 | 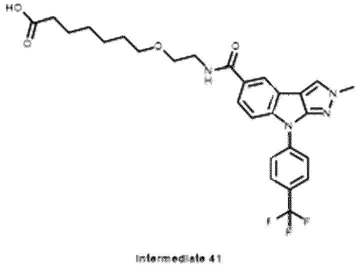 |
| 43 | 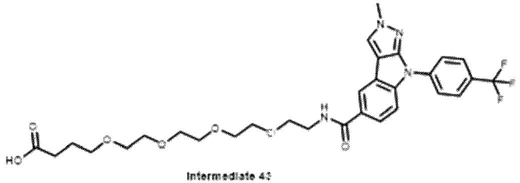 |
| 45 | 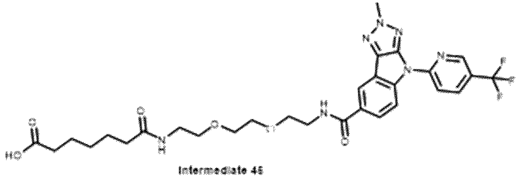 |
| 46 | 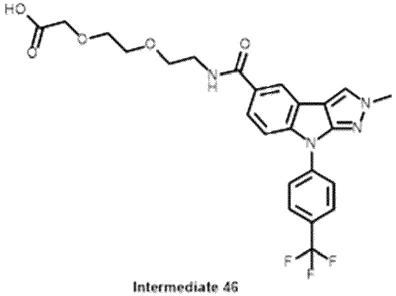 |
| 47 | 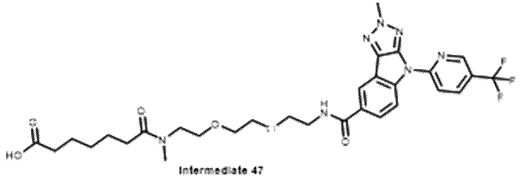 |
| 68 | 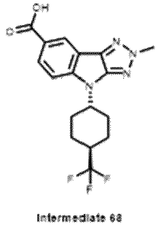 |
| 71 | 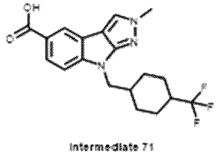 |
, or a pharmaceutically acceptable salt, solvate, tautomer and/or stereoisomer thereof.

## Patentansprüche

1. Verbindung der Formel I
Q¹-Q²-Q³ I
wobei
Q¹ ein Ubiquitin-Ligase-Ligand ist;
Q² (i) fehlt; oder (ii) ein zweiwertiger Linker ist, der durch eine unverzweigte Alkylenkette mit 2-25 C-Atomen gebildet wird, worin 1-8 nicht benachbarte CH₂-Gruppen unabhängig voneinander durch O, -C(=O)-NH-, -NH-C(=O)-, -C(=O)-N(CH₃)-, -N(CH₃)-C(=O)-, -CH=CH- und/oder -C=C- ersetzt sein können; worin eine oder zwei CH₂-Gruppen gegebenenfalls einen Methylsubstituenten tragen können; und worin eine CH₂-Gruppe durch eine Gruppierung ersetzt sein kann, die ausgewählt ist aus der Gruppe bestehend aus 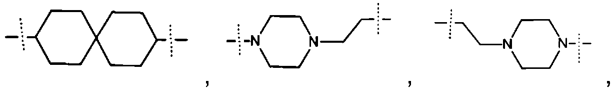 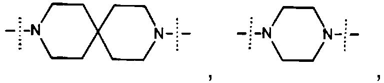 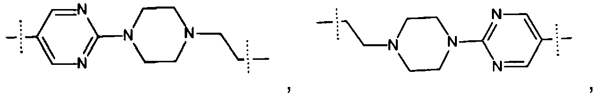 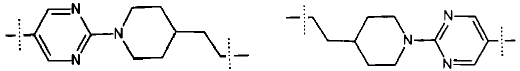 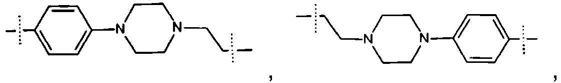 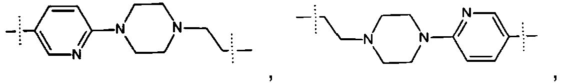 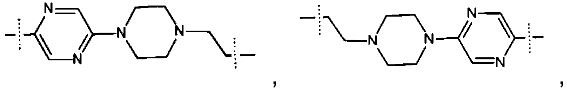 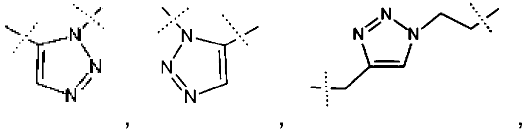 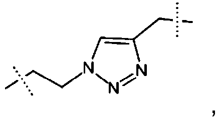 wobei das linke Ende der Gruppierung zum Q¹-Teil der Verbindung der Formel I hin gerichtet ist und das rechte Ende der Gruppierung zum Q³-Teil der Verbindung der Formel I hin gerichtet ist;
Q³ 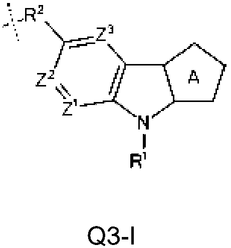 bedeutet, wobei
Ring A einen fünfgliedrigen heteroaromatischen Ring darstellt, der ausgewählt ist aus der Gruppe bestehend aus den folgenden Ringgruppierungen: 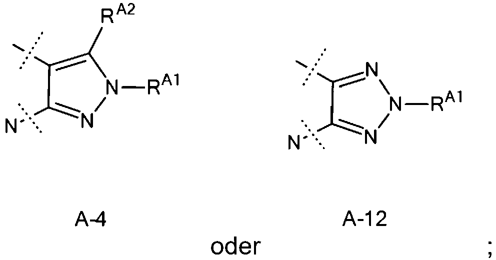 wobei
R^{A1} Methyl darstellt;
R^{A2} H darstellt;
Z¹ ist CH;
Z² ist CH;
Z³ ist CH;
R¹ Ar¹, Hetar¹, Cyc¹, Hetcyc¹ darstellt;
R² 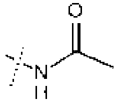 (-NH-C(=O)-) darstellt;
Ar¹ ein mono- oder bicyclisches Aryl mit 6 oder 10 Ringkohlenstoffatomen ist, wobei dieses Aryl unsubstituiert oder mit Substituenten R^{B1}, R^{B2} und/oder R^{B3}, die gleich oder verschieden sein können, substituiert sein kann;
Hetar¹ ein monocyclisches Heteroaryl mit 5 oder 6 Ringatomen oder ein bicyclisches Heteroaryl mit 9 oder 10 Ringatomen ist, wobei 1, 2, 3 oder 4 der Ringatome ein Heteroatom(e) ist/sind, das/die aus N, O und/oder S ausgewählt ist/sind, und die restlichen Kohlenstoffatome sind, wobei dieses Heteroaryl unsubstituiert oder mit Substituenten R^{B1}, R^{B2} und/oder R^{B3}, die gleich oder verschieden sein können, substituiert sein kann;
Cyc¹ ein gesättigter oder teilweise ungesättigter mono- oder bicyclischer Carbocyclus mit 3, 4, 5, 6, 7, 8, 9 oder 10 Ringkohlenstoffatomen ist, wobei dieser Carbocyclus unsubstituiert oder mit R^{B4}, R^{B5} und/oder R^{B6}, die gleich oder verschieden sein können, substituiert sein kann;
Hetcyc¹ ein gesättigter oder teilweise ungesättigter, monocyclischer Heterocyclus mit 5 oder 6 Ringatomen ist, wobei 1 oder 2 der Ringatome ein Heteroatom(e) ist/sind, das/die aus N, O und/oder S ausgewählt ist/sind, und die restlichen Kohlenstoffatome sind, wobei dieser Heterocyclus unsubstituiert oder mit R^{B4}, R^{B5} und/oder R^{B6}, die gleich oder verschieden sein können, substituiert sein kann, wobei, wenn eines der Heteroatome S ist, dieser Heterocyclus auch mit R^{B4}, R^{B5}, R^{B6}, R^{B7} und/oder R^{B8} substituiert sein kann;
R^{B1}, R^{B2}, R^{B3} unabhängig voneinander geradkettiges oder verzweigtes C₁₋₆-Alkyl, wo das C₁₋₆-Alkyl unsubstituiert oder einfach mit -CN substituiert oder mit 1, 2 oder 3 Halogen substituiert sein kann, geradkettiges oder verzweigtes C₁₋₄-Alkoxy, wobei das C₁₋₄-Alkoxy unsubstituiert oder mit 1, 2 oder 3 Halogen substituiert sein kann, -O-CH₂-C≡CH, geradkettiges oder verzweigtes -S-C₁₋₄-Alkyl, wobei das -S-C₁₋₄-Alkyl unsubstituiert oder mit 1, 2 oder 3 Halogen substituiert sein kann, geradkettiges oder verzweigtes C₂₋₆-Alkenyl, wobei das C₂₋₆-Alkenyl unsubstituiert oder einfach mit -CN substituiert oder mit 1, 2 oder 3 Halogen substituiert sein kann, F, Cl, Br, -CN, -S(=O)-C₁₋₃-Alkyl, S(=O)₂-C₁₋₃-Alkyl, -N(C₁₋₃-Alkyl)₂, Ar², -CH₂-Ar², Hetar², Cyc², Hetcyc² darstellen;
oder zwei benachbarte R^{B1}, R^{B2} und/oder R^{B3} zusammen einen zweiwertigen -C₃₋₄-Alkylenrest, worin eine der Alkylenkohlenstoffeinheiten durch eine Carbonyleinheit (-C(=O)-) ersetzt sein kann, oder einen zweiwertigen -O-C₂₋₃-Alkylenrest bilden;
R^{B4}, R^{B5}, R^{B6} unabhängig voneinander F, C₁₋₄-Alkyl, wobei das C₁₋₄-Alkyl unsubstituiert oder mit 1, 2 oder 3 F substituiert sein kann, C₁₋₄-Alkoxy, Phenyl darstellen; oder
zwei von R^{B4}, R^{B5} und R^{B6} an dasselbe Kohlenstoffatom des Carbocyclus Cyc¹ oder des Heterocyclus Hetcyc¹ gebunden sind und eine zweiwertige Oxo(=O)-Gruppe bilden; oder
R^{B4} und R^{B5} und R^{B7} und R^{B8} an dasselbe Schwefelatom des Heterocyclus gebunden sind und zwei zweiwertige Oxo(=O)-Gruppen bilden, wodurch sie eine -S(=O)₂-Gruppierung bilden;
Ar² Phenyl ist, das unsubstituiert oder mit einem oder zwei Substituenten substituiert sein kann, die unabhängig voneinander ausgewählt sind aus OH, F, Cl, Br, C₁₋₄-Alkyl und C₁₋₄-Alkoxy, wobei diese C₁₋₄-Alkyl- oder C₁₋₄-Alkoxygruppe mit 1, 2 oder 3 F-Atomen substituiert sein kann;
Hetar² ein monocyclisches Heteroaryl mit 5 oder 6 Ringatomen ist, wobei 1, 2, 3, 4, 5 der Ringatome ein Heteroatom(e) ist/sind, das/die aus N, O und/oder S ausgewählt ist/sind, und die restlichen Kohlenstoffatome sind, wobei dieses Heteroaryl unsubstituiert oder mit einem oder zwei Substituenten substituiert sein kann, die unabhängig voneinander ausgewählt sind aus F, Cl, Br, C₁₋₄-Alkyl und C₁₋₄-Alkoxy, wobei diese C₁₋₄-Alkyl- oder C₁₋₄-Alkoxygruppe mit 1, 2 oder 3 F-Atomen substituiert sein kann;
Cyc² Cyclopropyl, Cyclobutyl, Cyclopentyl oder Cyclohexyl ist, das jeweils unsubstituiert oder mit einem oder zwei Substituenten substituiert sein kann, die unabhängig voneinander ausgewählt sind aus OH, F, Cl, Br, C₁₋₄-Alkyl und C₁₋₄-Alkoxy, wobei diese C₁₋₄-Alkyl- oder C₁₋₄-Alkoxygruppe mit 1, 2 oder 3 F-Atomen und/oder 1 Hydroxygruppe substituiert sein kann;
Hetcyc² Pyrrolidinyl, Piperidinyl ist, das jeweils unsubstituiert oder mit einem oder zwei Substituenten substituiert sein kann, die unabhängig voneinander ausgewählt sind aus OH, F, Cl, Br, C₁₋₄-Alkyl und C₁₋₄-Alkoxy, wobei diese C₁₋₄-Alkyl- oder C₁₋₄-Alkoxygruppe mit 1, 2 oder 3 F-Atomen und/oder 1 Hydroxygruppe substituiert sein kann;
Halogen F, Cl, Br, I ist;
oder ein pharmazeutisch unbedenkliches Salz, Solvat, Tautomer und/oder Stereoisomer davon.

2. Verbindung nach Anspruch 1, wobei
Q¹ ein E3-Ubiquitin-Ligase-Ligand ist;
gegebenenfalls Q¹ a) ein CRBN(Cereblon)-Ligand oder (b) ein VHL(von Hippel-Lindau)-Ligand oder (c) eine andere Art von E3-Uibiquitin-Ligase-Ligand als ein CRBN- oder ein VHL-Ligand ist;
oder ein pharmazeutisch unbedenkliches Salz, Solvat, Tautomer und/oder Stereoisomer davon.

3. Verbindung nach Anspruch 2, wobei (a) der CRBN(Cereblon)-Ligand eine Struktur der Formel Q1-I, Q1-II, Q1-VII oder Q1-VIII aufweist; (b) der VHL-Ligand eine Struktur der Formel Q1-III aufweist; (c) die andere Art von E3-Uibiquitin-Ligase-Ligand eine Struktur der Formel Q1-IV, Q1-V, Q1-VI, Q1-XII oder Q1-XIII aufweist: 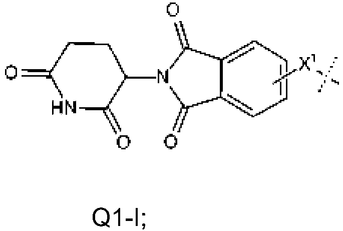 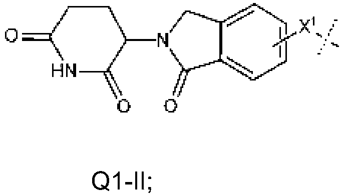 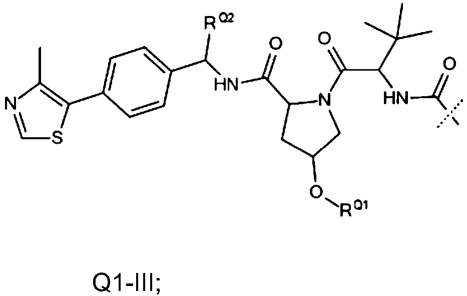 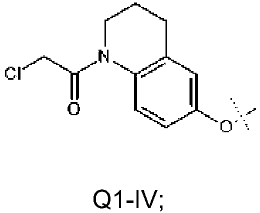 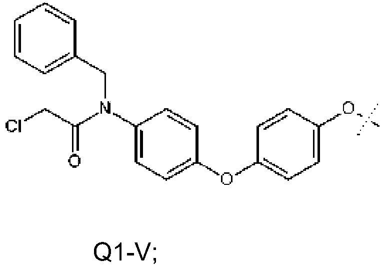 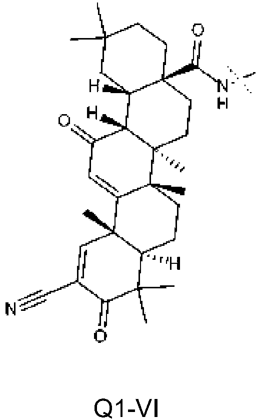 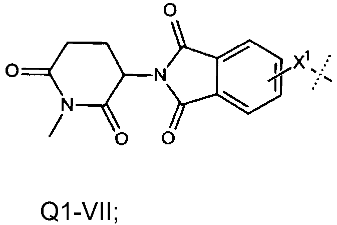 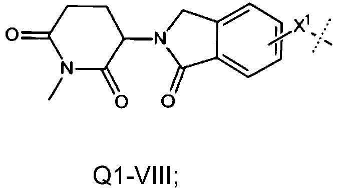 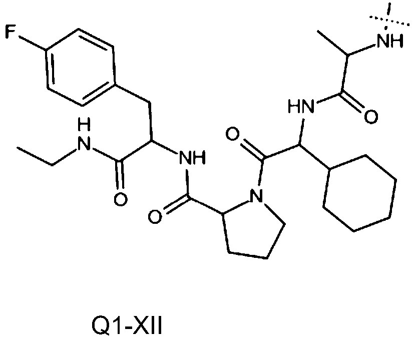 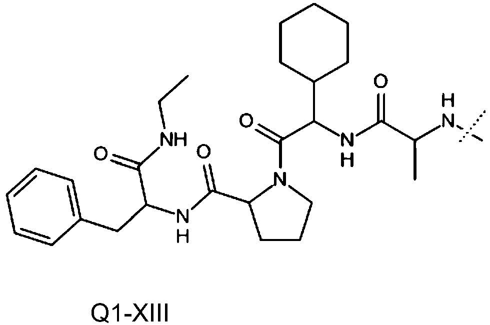 wobei
X¹ eine Einfachbindung, -O-, -NH-, -NCH₃-, -CH₂- oder -NH-C(=O)-bedeutet;
R^{Q1} H oder -C(=O)-CH₃ bedeutet;
R^{Q2} H oder CH₃ bedeutet;
oder ein pharmazeutisch unbedenkliches Salz, Solvat, Tautomer und/oder Stereoisomer davon.

4. Verbindung nach beliebigen der vorhergehenden Ansprüche, wobei Q¹ ein CRBN(Cereblon)-Ligand ist; und der CRBN(Cereblon)-Ligand eine Struktur aufweist, die ausgewählt ist aus den Strukturen der Formeln Q1-I-1, Q1-I-2, Q1-I-3; Q1-I-4, Q1-I-5, Q1-I-6, Q1-II-1, Q1-II-2, Q1-II-3, Q1-II-4, Q1-II-5, Q1-II-6, Q1-II-7, Q1-VII-1, Q1-VII-2 und Q1-VII-3 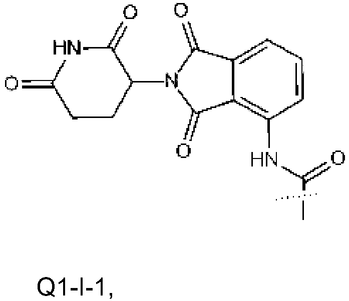 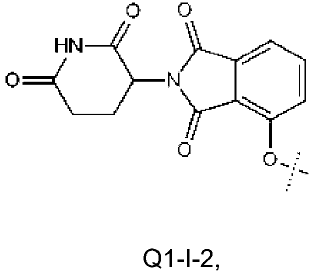 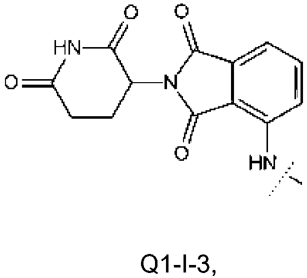 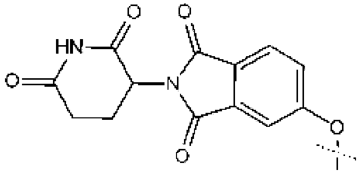 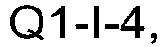 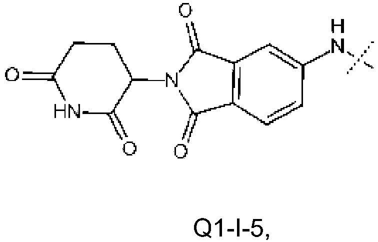 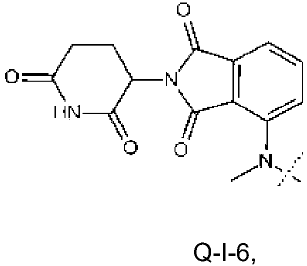 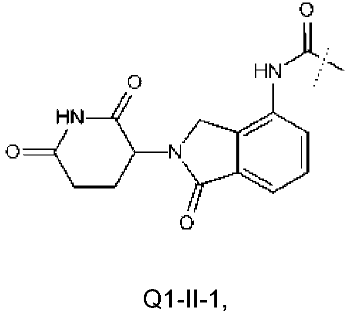 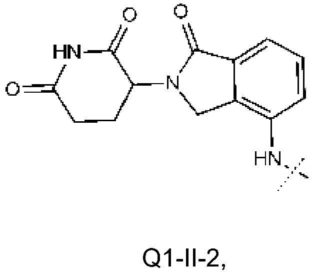 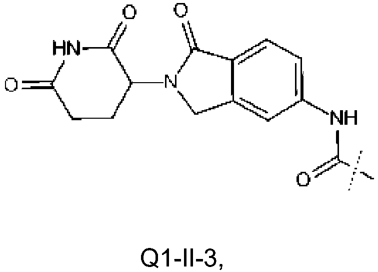 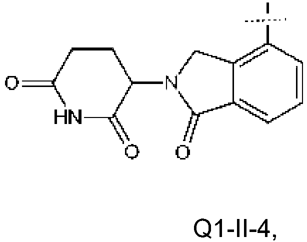 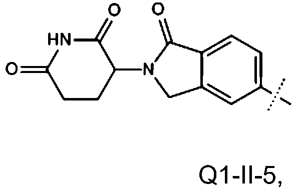 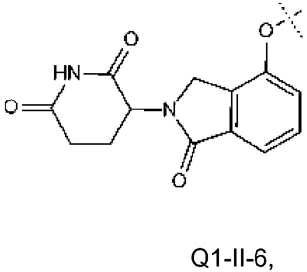 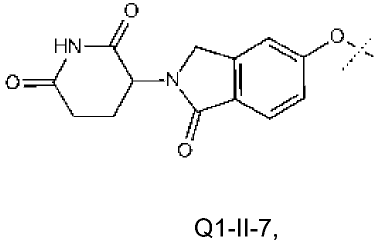 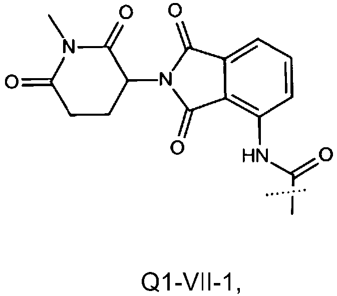 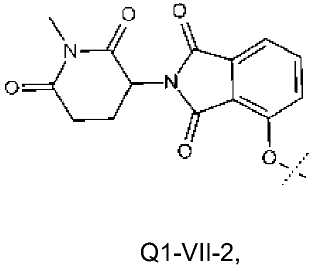 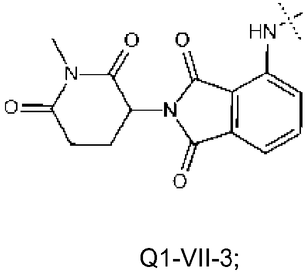 oder ein pharmazeutisch unbedenkliches Salz, Solvat, Tautomer und/oder Stereoisomer davon.

5. Verbindung nach beliebigen der Ansprüche 1 bis 3, wobei Q¹ ein VHL(von-Hippel-Lindau)-Ligand ist; und der VHL-Ligand eine Struktur aufweist, die ausgewählt ist aus den Formeln Q1-III-1, Q1-III-2, Q1-III-3, Q1-III-4 und Q1-III-5 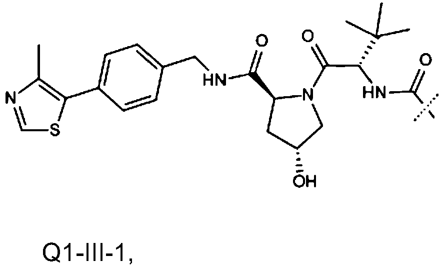 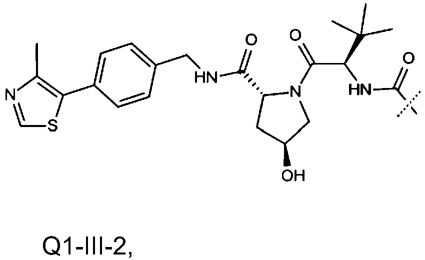 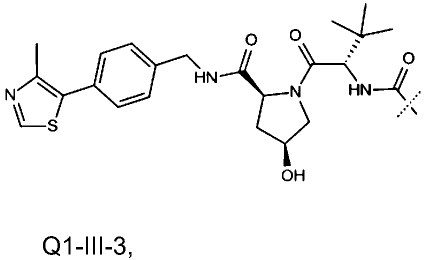 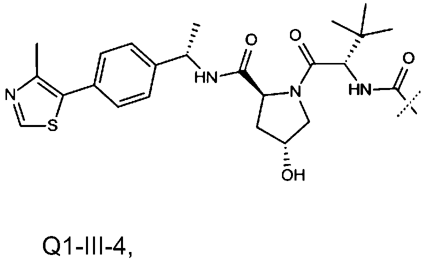 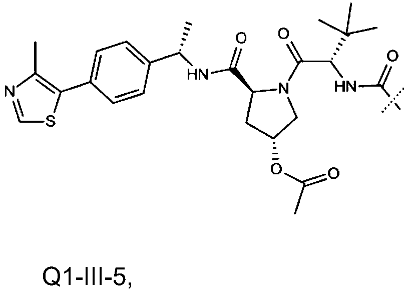 oder ein pharmazeutisch unbedenkliches Salz, Solvat, Tautomer und/oder Stereoisomer davon.

6. Verbindung nach beliebigen der vorhergehenden Ansprüche, wobei
Q² ausgewählt ist aus der Gruppe bestehend aus 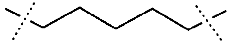 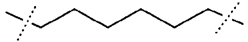 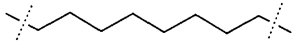 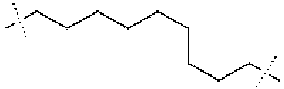 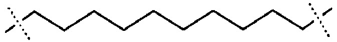 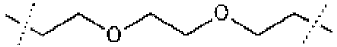 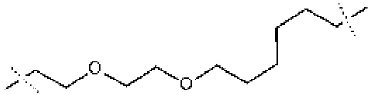 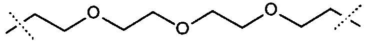 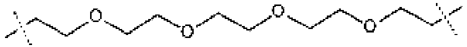 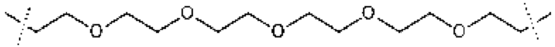 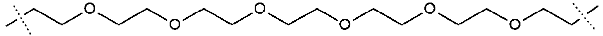 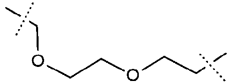 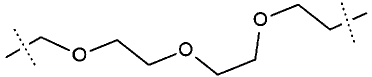 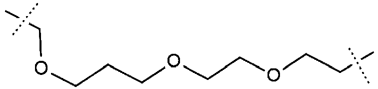 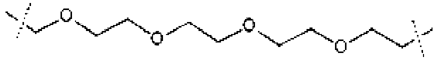 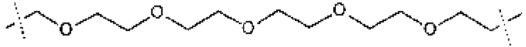 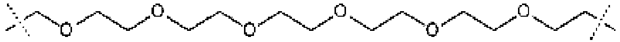 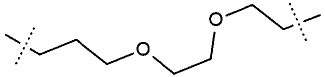 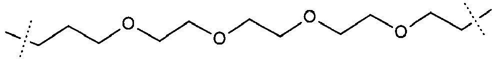 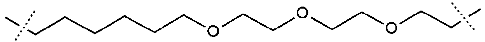 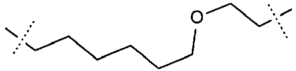 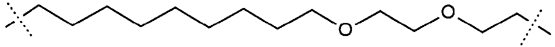 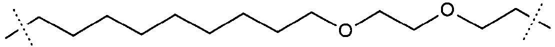 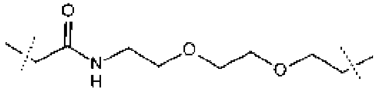 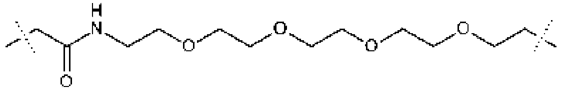 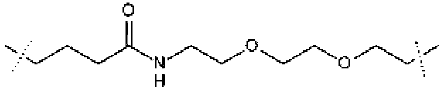 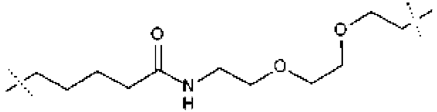 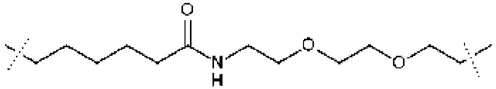 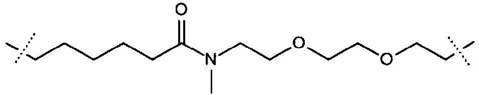 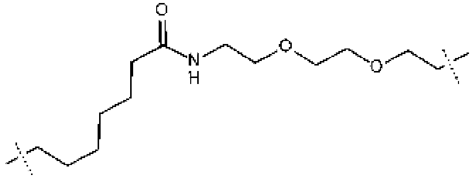 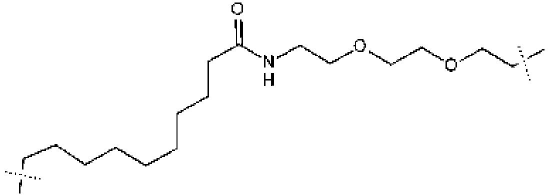 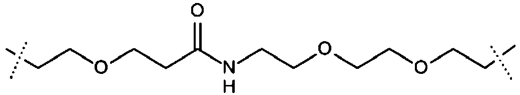 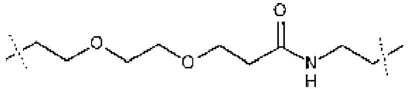 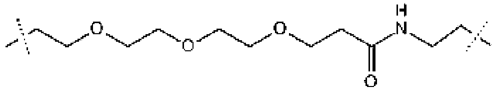 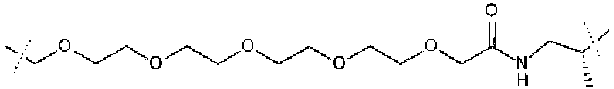 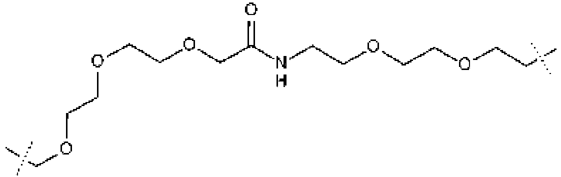 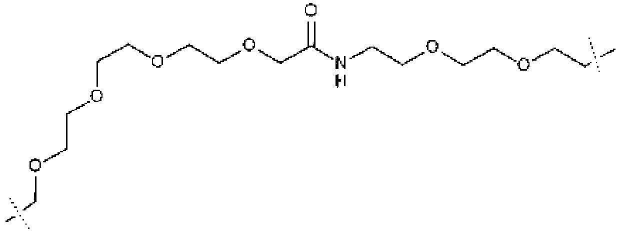 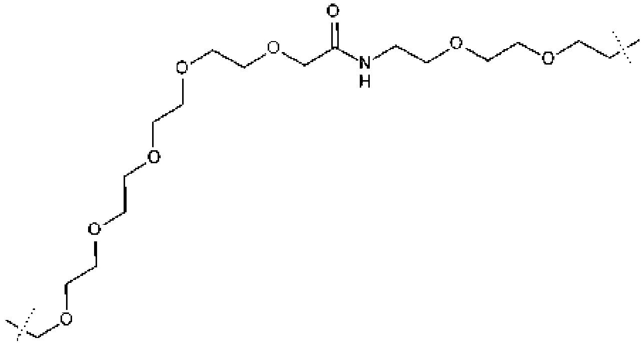 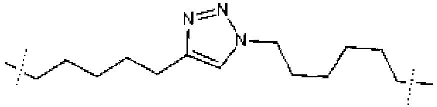 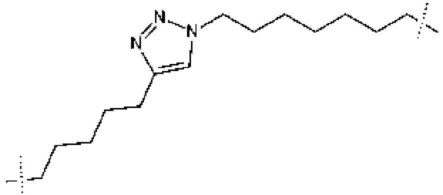 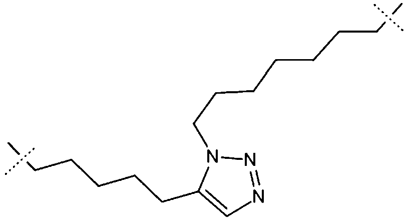 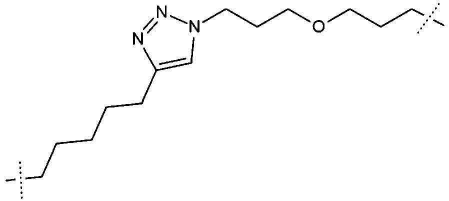 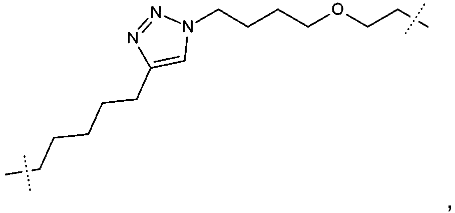
gegebenenfalls Q2 ausgewählt ist aus der Gruppe bestehend aus 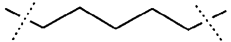 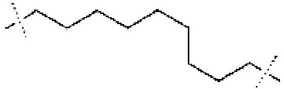 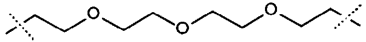 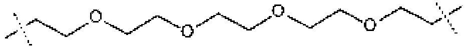 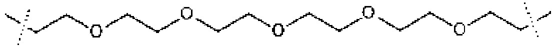 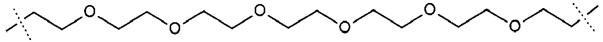 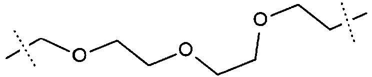 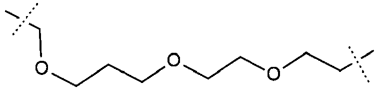 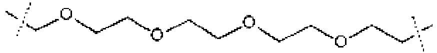 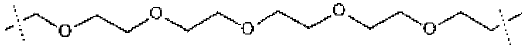 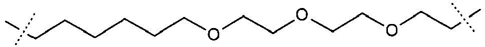 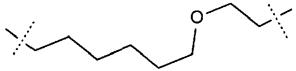 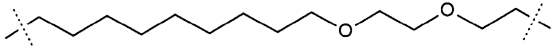 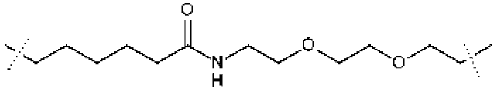 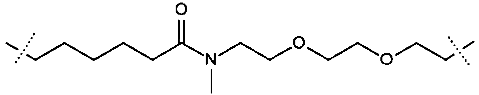 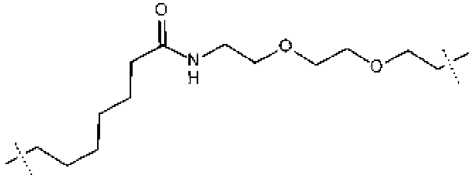 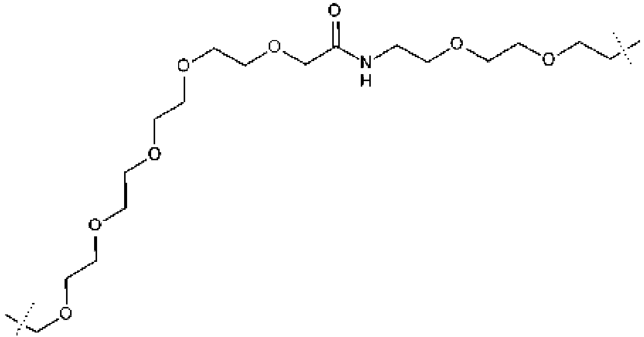 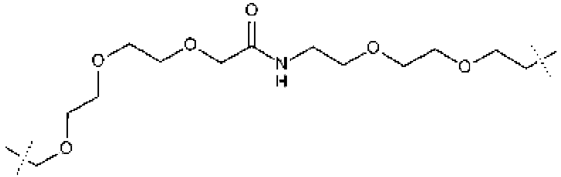 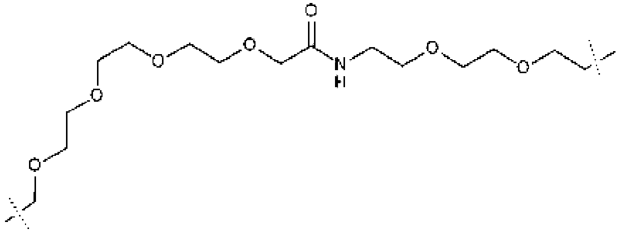 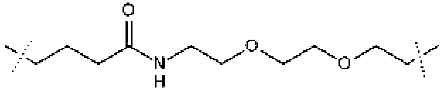 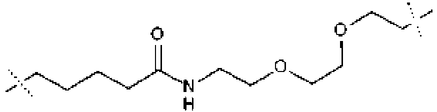 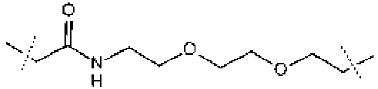 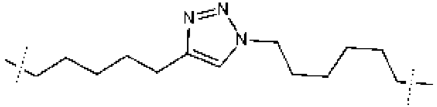 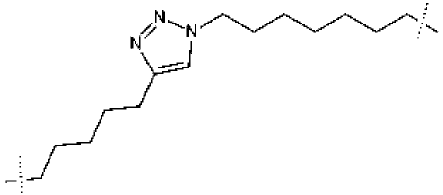 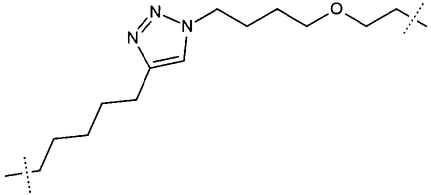 oder ein pharmazeutisch unbedenkliches Salz, Solvat, Tautomer und/oder Stereoisomer davon.

7. Verbindung nach einem beliebigen vorhergehenden Anspruch, wobei
R¹ Phenyl, 3-Fluorphenyl, 4-Fluorphenyl, 4-Chlorphenyl, 4-Methylphenyl, 4-Ethylphenyl, 4-Difluormethylphenyl, 3-Trifluormethylphenyl, 4-Trifluormethylphenyl, 4-(1,1-Difluorethyl)phenyl, 4-(2,2,2-Trifluorethyl)phenyl, 4-(1-Trifluormethylcyclopropyl)-phen-1-yl, 4-Cyclopentylphenyl, 4-Ethoxyphenyl, 4-Difluormethoxyphenyl, 4-Trifluormethoxyphenyl, 3-(Trifluormethyl)-sulfanylphenyl, 4-(Trifluormethyl)sulfanylphenyl, 3-Trifluormethyl-4-methylphenyl, 2-Fluor-4-trifluormethylphenyl, 2-Fluor-4-trifluormethoxyphenyl, 3-Fluor-4-(n-propyl)phenyl, 2,3-Dimethyl-4-methoxyphenyl, 6-Fluornaphth-2-yl; 5-Trifluormethylfuran-2-yl; 5-Trifluormethylthiophen-2-yl, 2-Trifluormethyl-1,3-thiazol-4-yl, 3-Fluorpyridin-2-yl, 6-Methylpyridin-3-yl, 6-Methoxy-pyridin-3-yl, 3-Ethylpyridin-2-yl, 6-Ethylpyridin-3-yl, 4-Difluormethylpyridin-2-yl, 4-Trifluormethylpyridin-2-yl, 4-Trifluormethoxypyridin-2-yl, 4-Cyanopyridin-2-yl, 5-Trifluormethylpyridin-2-yl, 6-Trifluormethylpyridin-2-yl, 6-Trifluormethylpyridin-3-yl (2-Trifluormethylpyridin-5-yl), 6-Trifluormethoxypyridin-3-yl (2-Trifluormethoxypyridin-5-yl), 5-Cyanopyridin-2-yl, 5-Cyanomethylpyridin-2-yl, 5-Methansulfonylpyridin-2-yl, 6-Methoxypyridin-2-yl, 4-Methylpyrimidin-2-yl, 4-Ethylpyrimidin-2-yl, 4-Methylsulfanylpyrimidin-2-yl, 5-Cyclopropylpyrimidin-2-yl, 5-Ethylpyrimidin-2-yl, 5-Difluormethylpyrimidin-2-yl, 5-Trifluormethylpyrimidin-2-yl, 5-Cyanopyrimidin-2-yl, 5-Cyano-3-fluorpyridin-2-yl, 5-Cyano-6-methylpyridin-2-yl, 3-Fluor-5-(trifluormethyl)-pyridin-2-yl, 5-Oxo-5H,6H,7H-cyclopenta[b]pyridin-2-yl, 5,6,7,8-Tetrahydrochinolin-2-yl, 5-Oxo-5,6,7,8-tetrahydrochinolin-2-yl, 5H,6H,7H-Cyclopenta[b]pyridin-2-yl, Chinolin-2-yl, Isochinolin-3-yl, 6-Methylchinolin-2-yl, 8-Methoxychinolin-4-yl, Furo[3,2-b]pyridin-5-yl, Chinazolin-2-yl, 6-Fluorchinazolin-2-yl, 1,5-Naphthyridin-2-yl; 3-Methylcyclobutyl, Cyclopentyl, 3-Methylcyclopentyl, 3,3-Dimethylcyclopentyl, 3-Trifluormethyl-bicyclo-[1.1.1]petan-1-yl, Cyclohexyl, 4-Methylcyclohexyl, 4-(Trifluormethyl)-cyclohexyl, 4,4-Difluorcyclohexyl, Cyclohex-1-enyl, 2-Oxocycloheptyl, 6,6-Difluorspiro[3.3]heptan-2-yl, 1H-Inden-2-yl darstellt;
oder ein pharmazeutisch unbedenkliches Salz, Solvat, Tautomer und/oder Stereoisomer davon.

8. Verbindung nach einem beliebigen vorhergehenden Anspruch, wobei
R¹ 4-Methylphenyl, 4-Difluormethylphenyl, 4-Trifluormethylphenyl, 4-Fluorphenyl darstellt;
oder ein pharmazeutisch unbedenkliches Salz, Solvat, Tautomer und/oder Stereoisomer davon.

9. Verbindung nach einem beliebigen vorhergehenden Anspruch, wobei
Q³ eine Struktur der Formel Q3-I aufweist;
Ring A Ring A-4 oder A-12 ist;
R^{A1} Methyl ist;
R^{A2} H ist;
R¹ 4-Trifluormethylphenyl ist;
R² 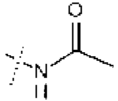 (-NH-C(=O)-) darstellt;
Z¹, Z² und Z³ jeweils CH darstellen; oder ein pharmazeutisch unbedenkliches Salz, Solvat, Tautomer und/oder Stereoisomer davon.

10. Verbindung nach beliebigen der vorhergehenden Ansprüche, wobei
Q¹ ein
(a) CRBN(Cereblon)-Ligand ist; und
der CRBN(Cereblon)-Ligand eine Struktur aufweist, die ausgewählt ist aus den Strukturen der Formeln Q1-I-1, Q1-I-2, Q1-I-3; Q1-I-4, Q1-I-5, Q1-I-6, Q1-II-1, Q1-II-2, Q1-II-3, Q1-II-4, Q1-II-5 und Q1-VII-1 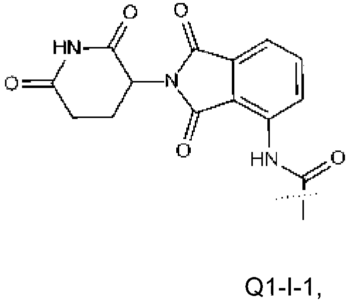 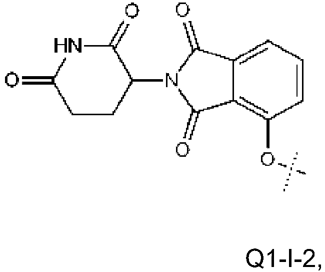 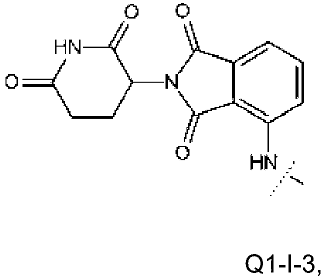 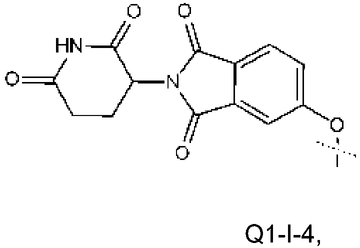 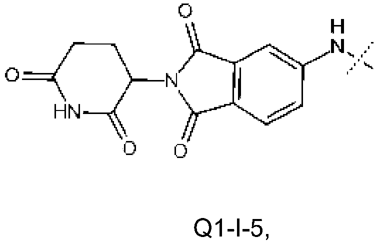 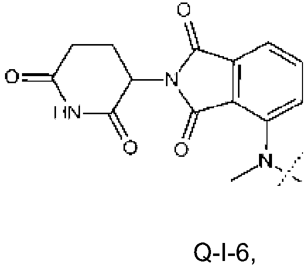 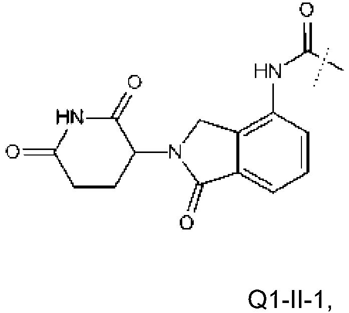 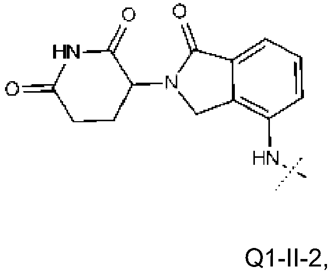 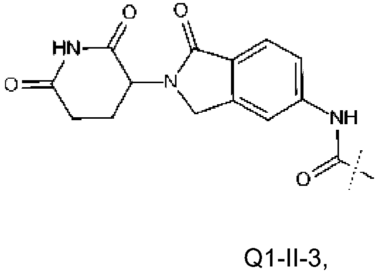 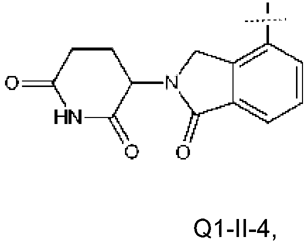 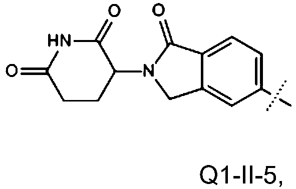 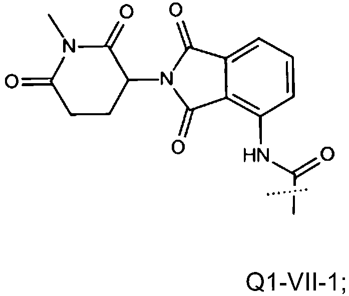 oder
(b) ein VHL(von Hippel-Lindau)-Ligand ist; und der VHL-Ligand eine Struktur aufweist, die ausgewählt ist aus den Strukturen der Formel Q1-III-1, Q1-III-2, Q1-III-4 und Q1-III-5: 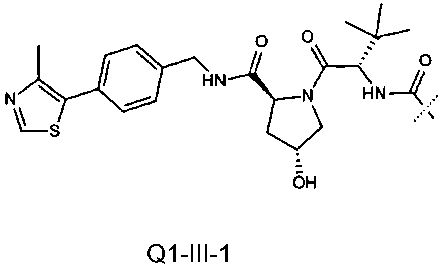 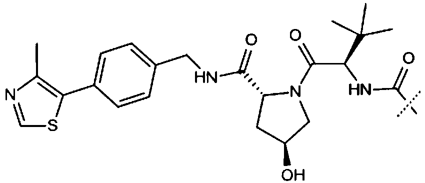 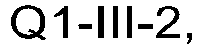 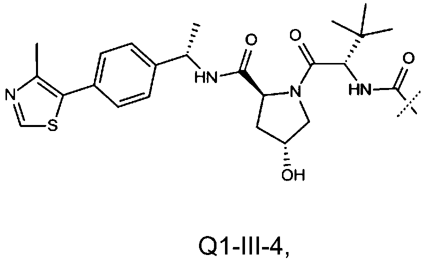 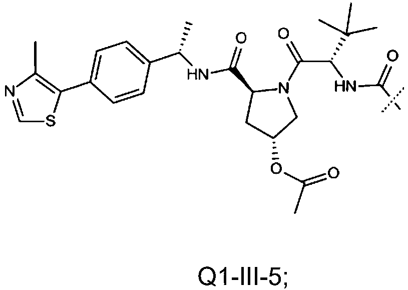
Q² ausgewählt ist aus der Gruppe bestehend aus 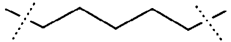 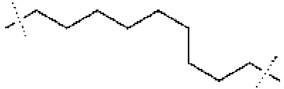 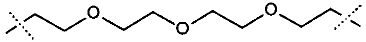 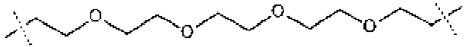 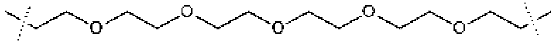 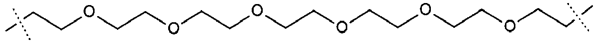 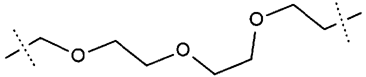 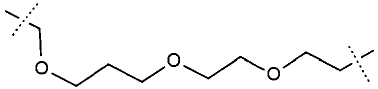 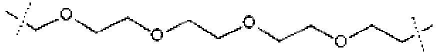 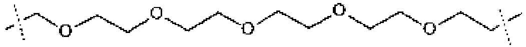 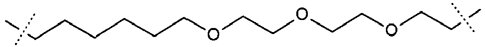 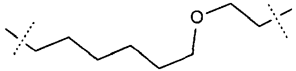 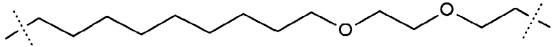 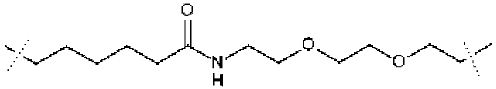 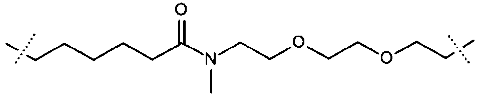 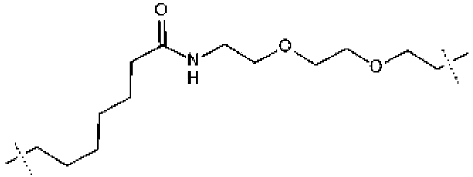 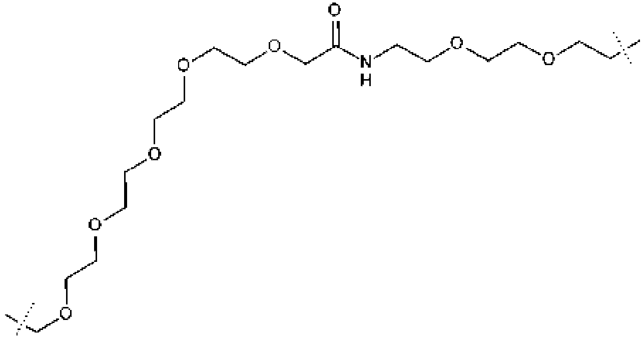 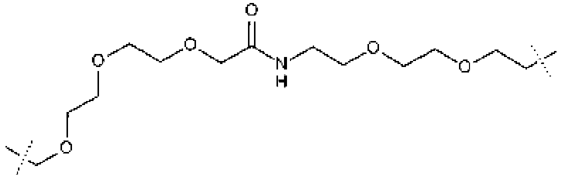 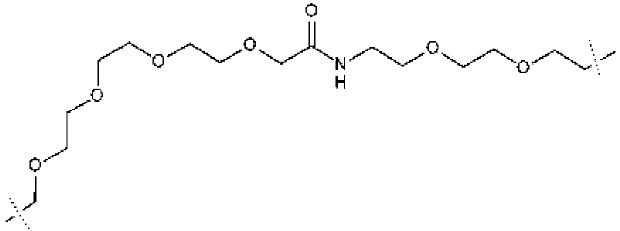 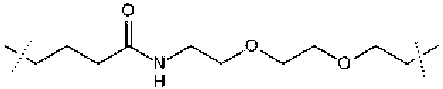 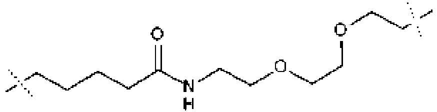 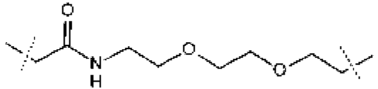 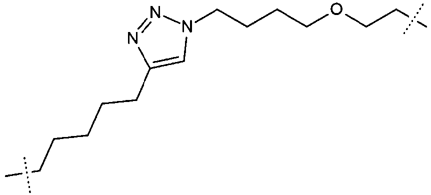 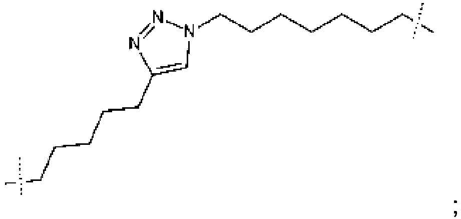
Q³ Q3-I ist;
Ring A Ring A-4 oder Ring A-12 ist;
R^{A1} Methyl darstellt;
R^{A2} Wasserstoff darstellt;
R¹ 4-Trifluormethylphenyl bedeutet;
R² 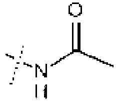 (-NH-C(=O)-) darstellt;
Z¹, Z² und Z³ jeweils CH darstellen;
oder ein pharmazeutisch unbedenkliches Salz, Solvat, Tautomer und/oder Stereoisomer davon.

11. Verbindung nach Anspruch 10, wobei
Q¹ (a) ein CRBN-Ligand ist und eine Struktur aufweist, die ausgewählt ist aus den Strukturen der Formel Q1-I-2, Q1-I-3, Q1-I-5, Q1-I-6, Q1-II-2, Q1-II-3, Q1-I1-4 und Q1-II-5; oder (b) ein VHL-Ligand ist und eine Struktur aufweist, die ausgewählt ist aus den Strukturen der Formeln Q1-III-1 und Q1-III-4;
oder ein pharmazeutisch unbedenkliches Salz, Solvat, Tautomer und/oder Stereoisomer davon.

12. Verbindung nach Anspruch 1, wobei die Verbindung ausgewählt ist aus Tabelle 1
| **Verbindung Nr.** | **Struktur** |
|---|---|
| 1 | 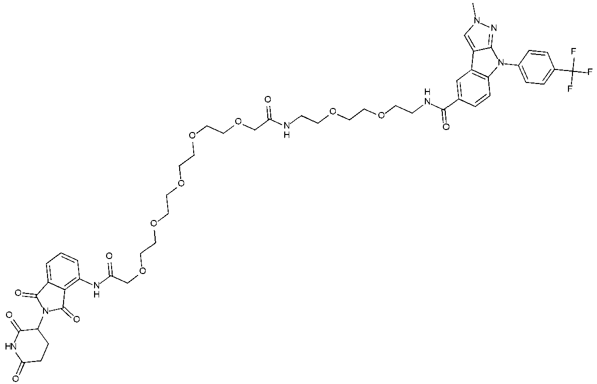 |
| 2 | 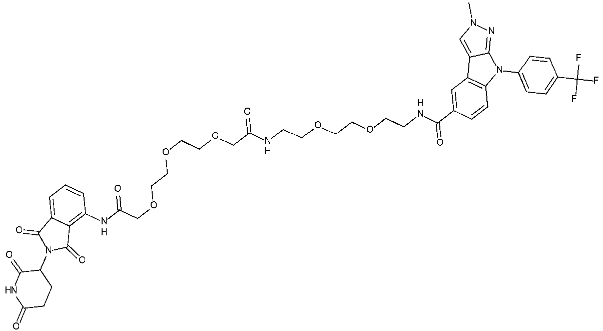 |
| 3 | 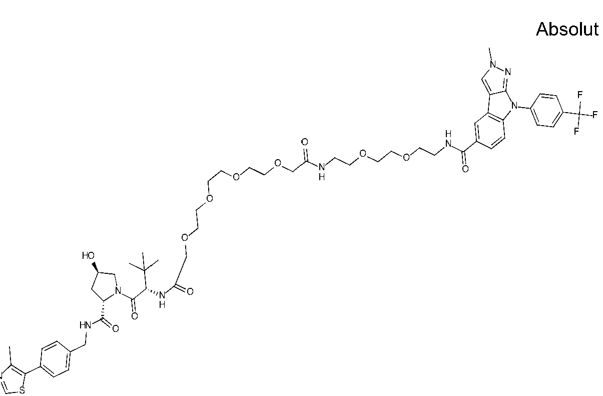 |
| 4 | 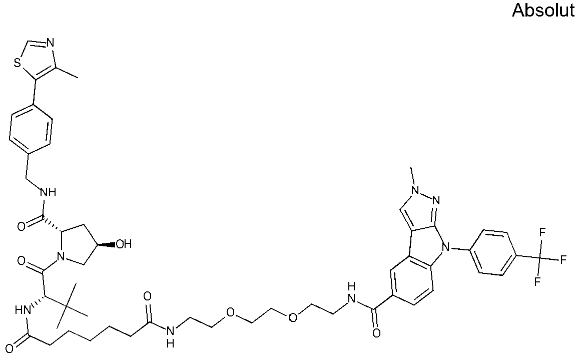 |
| 4-N | 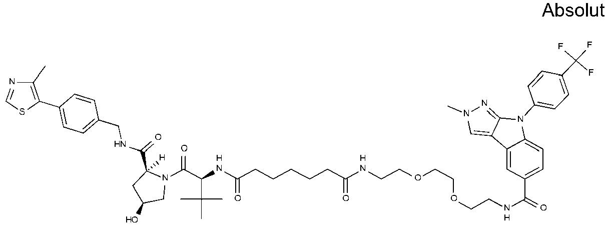 |
| 5 | 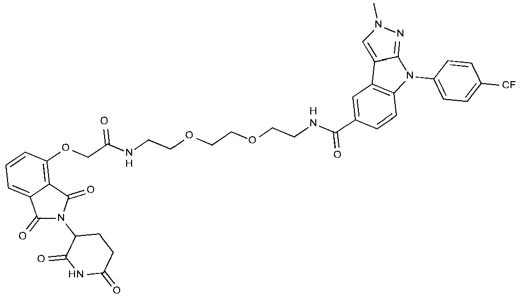 |
| 6 | 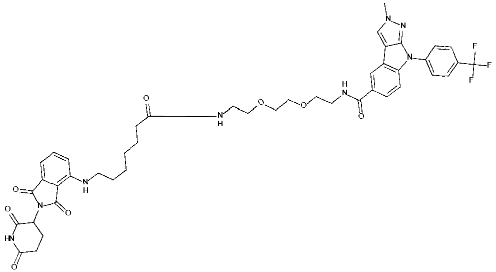 |
| 7 | 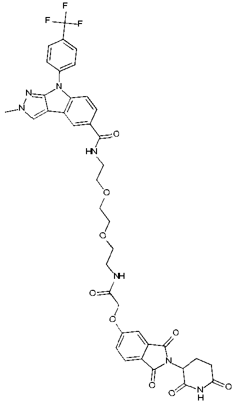 |
| 8 | 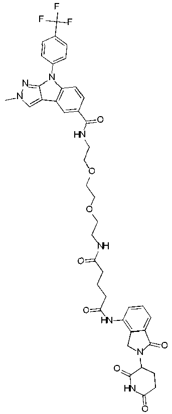 |
| 9 | 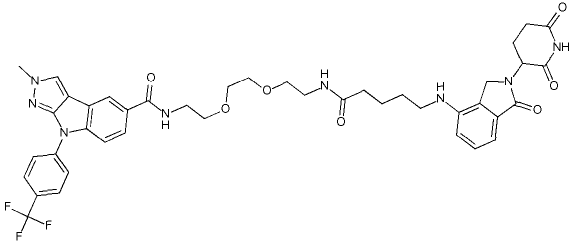 |
| 10 | 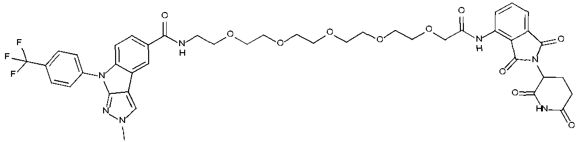 |
| 11 | 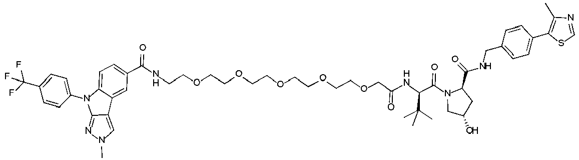 |
| 12 | 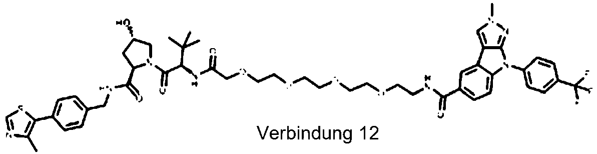 |
| 13 | 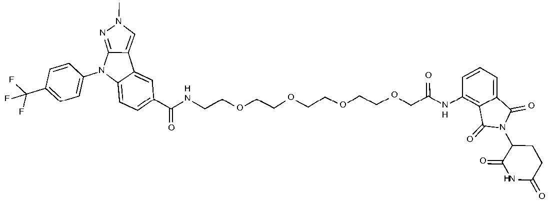 |
| 14 | 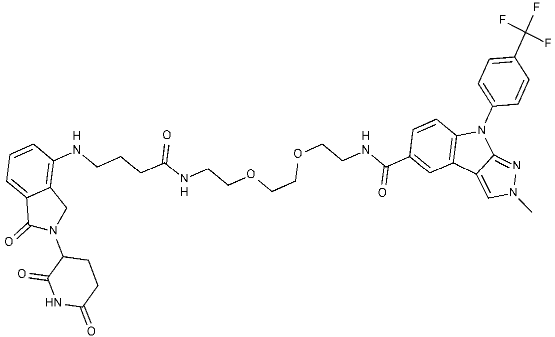 |
| 15 | 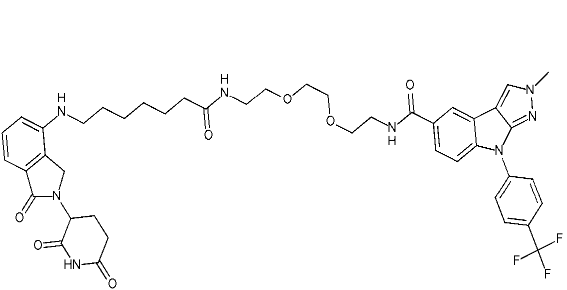 |
| 16 | 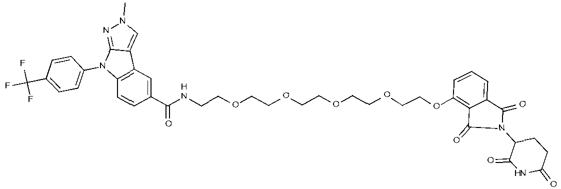 |
| 17 | 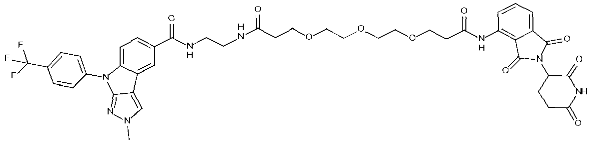 |
| 18 | 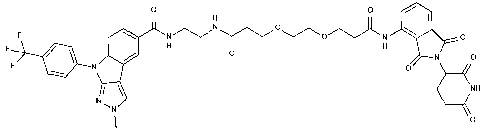 |
| 19 | 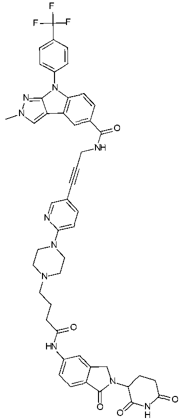 |
| 20 | 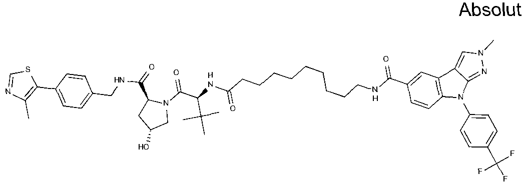 |
| 21 | 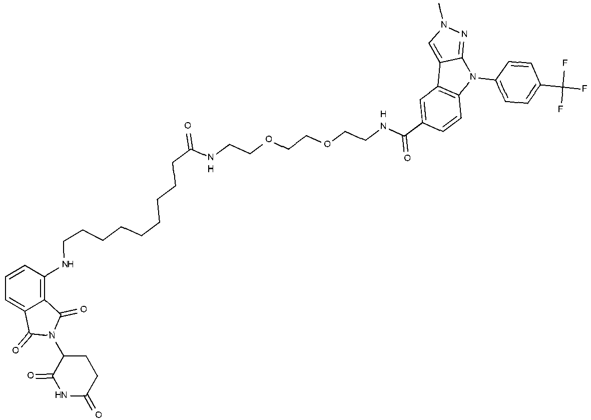 |
| 22 | 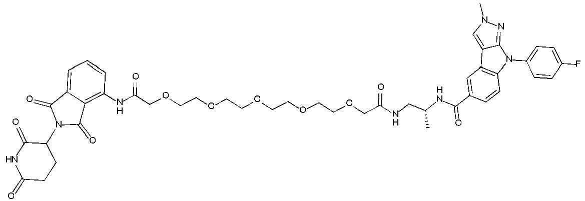 |
| 23 | 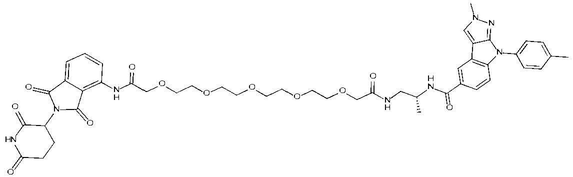 |
| 24 | 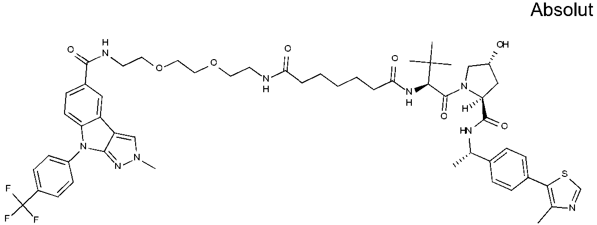 |
| 25 | 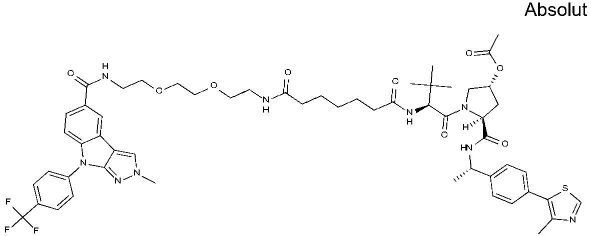 |
| 26 | 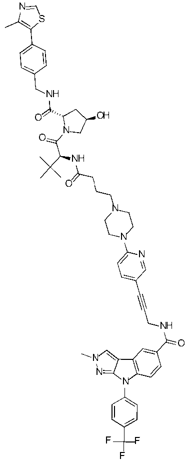 |
| 27 | 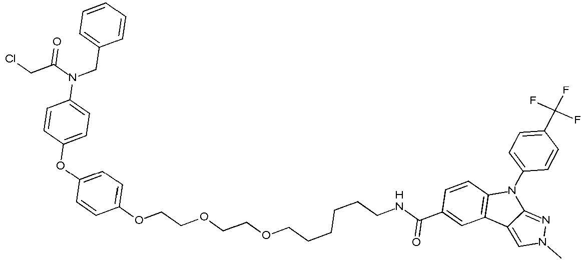 |
| 28 | 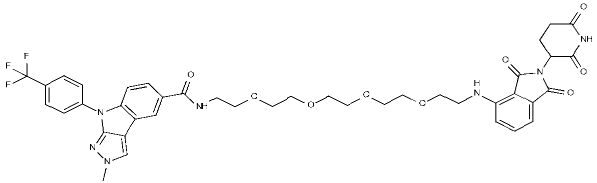 |
| 29 | 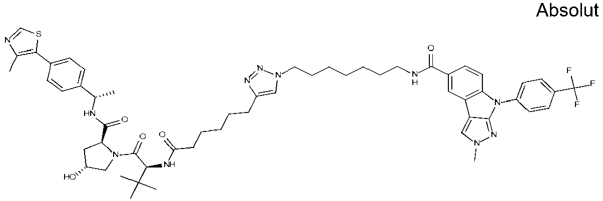 |
| 30 | 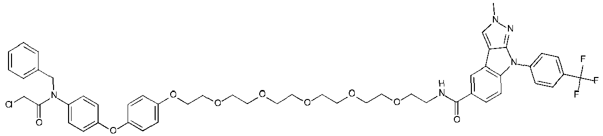 |
| 31 | 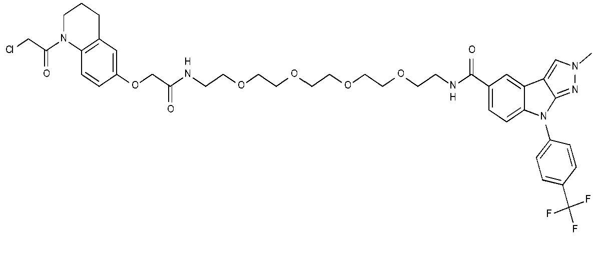 |
| 32 | 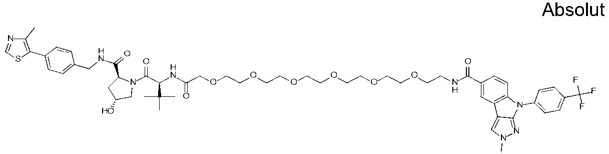 |
| 33 | 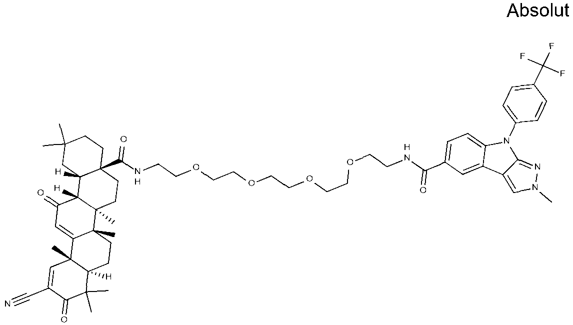 |
| 34 | 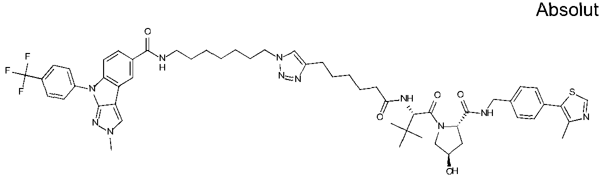 |
| 35 | 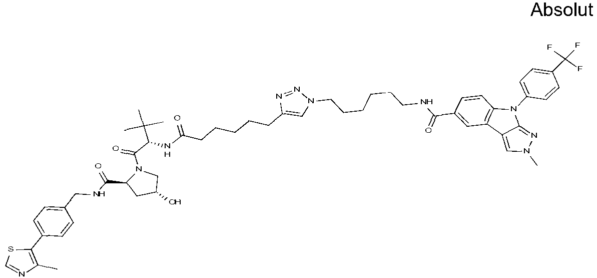 |
| 36 | 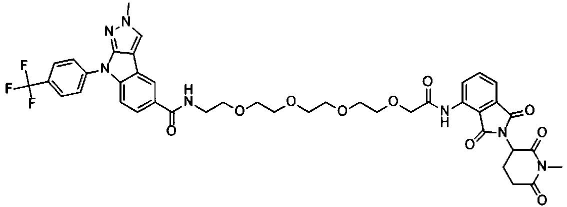 |
| 37 | 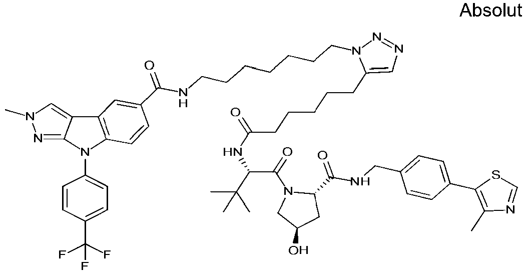 |
| 38 | 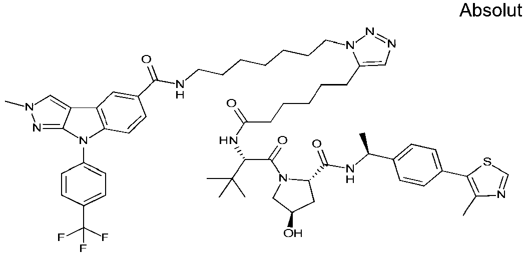 |
| 42 | 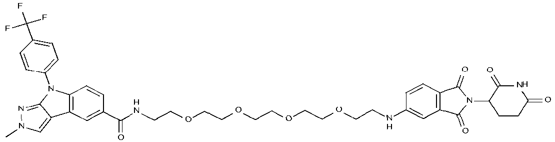 |
| 43 | 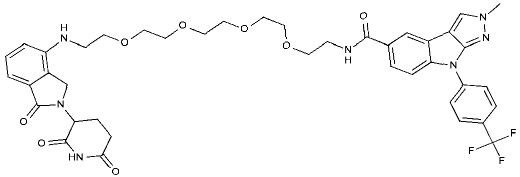 |
| 45 | 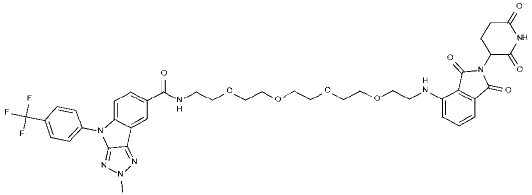 |
| 47 | 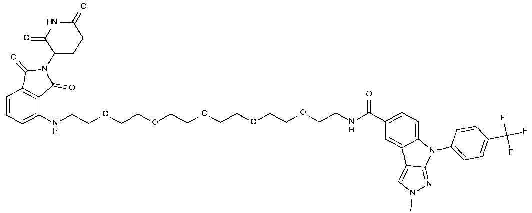 |
| 48 | 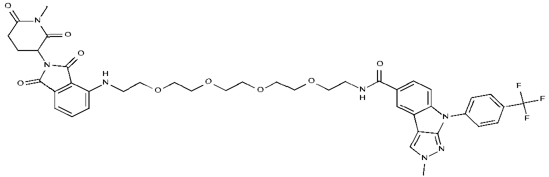 |
| 49 | 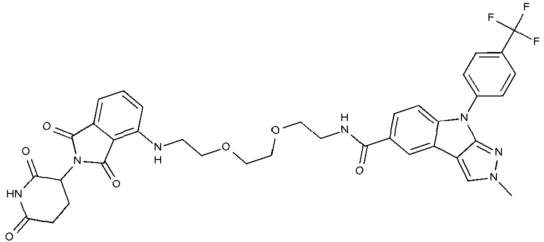 |
| 50 | 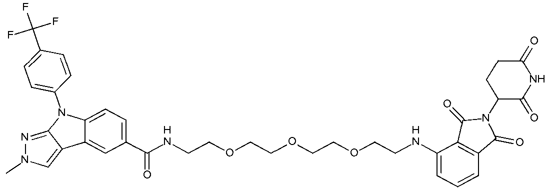 |
| 51 | 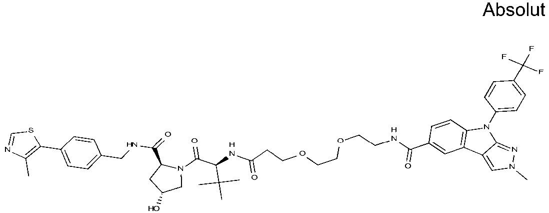 |
| 52 | 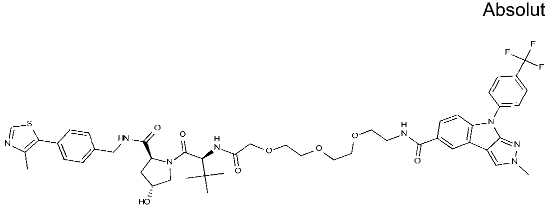 |
| 53 | 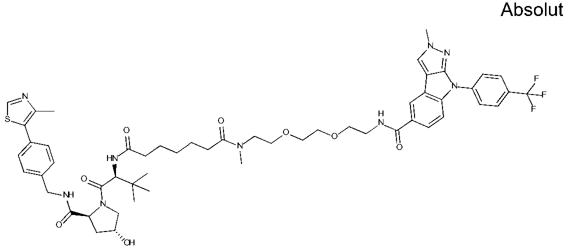 |
| 54 | 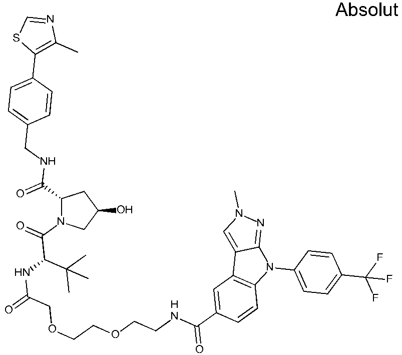 |
| 55 | 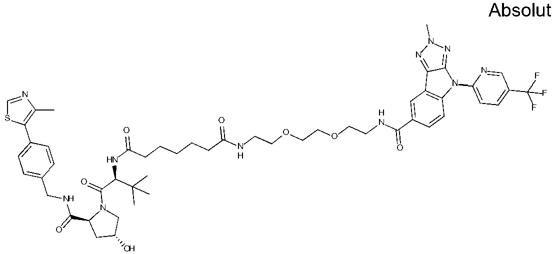 |
| 56 | 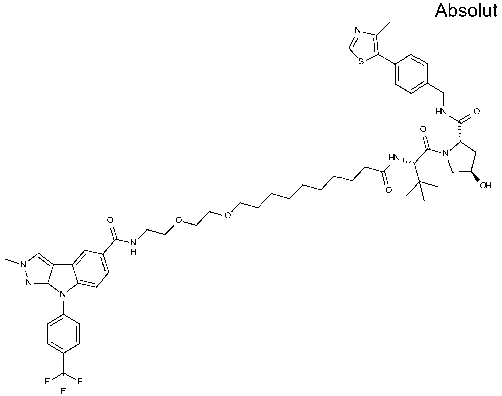 |
| 57 | 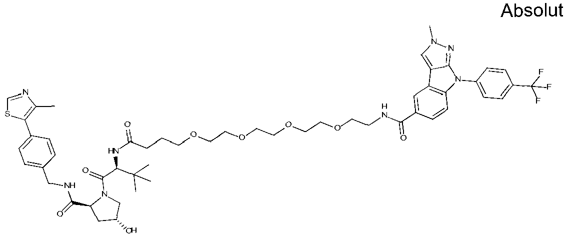 |
| 59 | 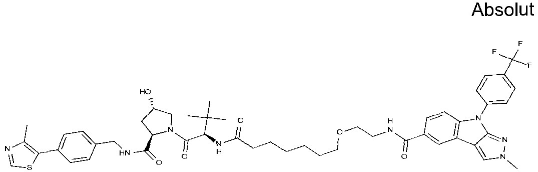 |
| 60 | 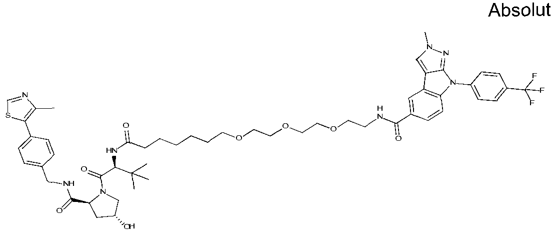 |
| 61 | 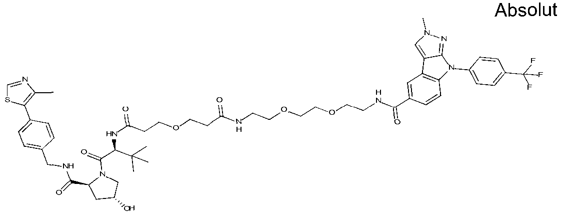 |
| 63 | 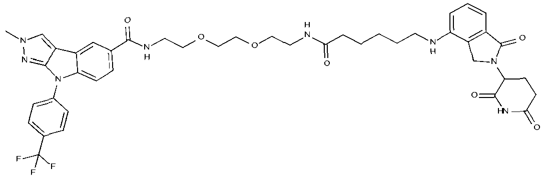 |
| 69 | 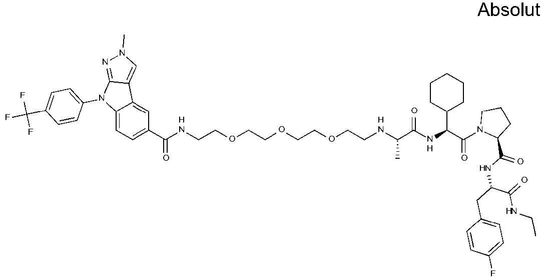 |
| 73 | 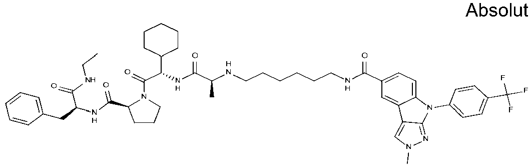 |
| 74 | 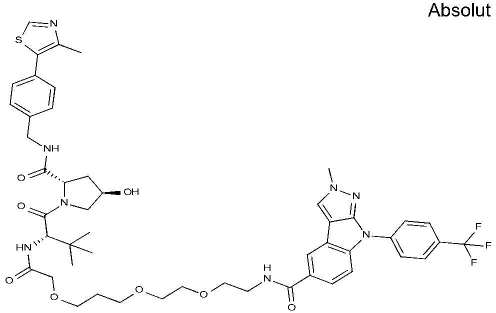 |
| 75 | 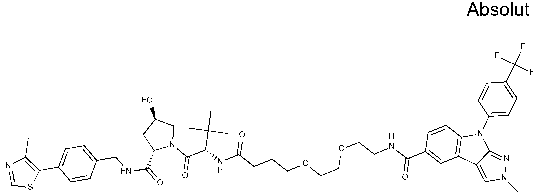 |
| 77 | 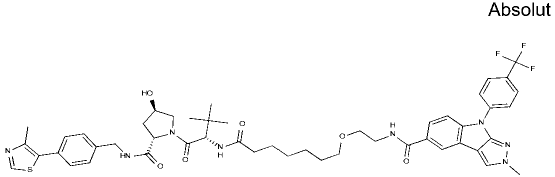 |
| 80 | 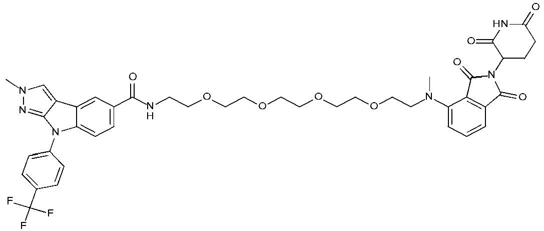 |
| 81 | 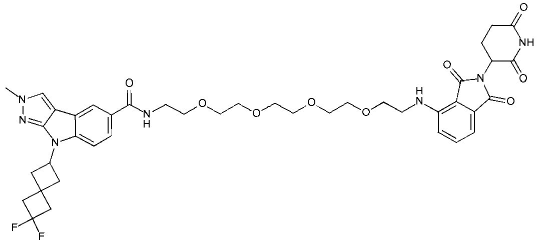 |
| 82 | 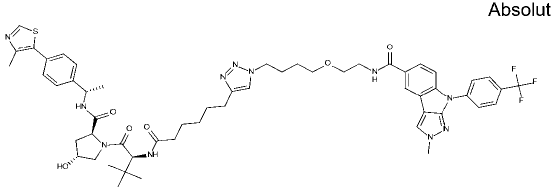 |
| 84 | 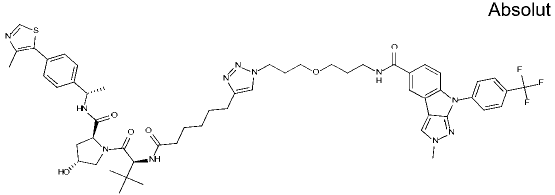 |
| 87 | 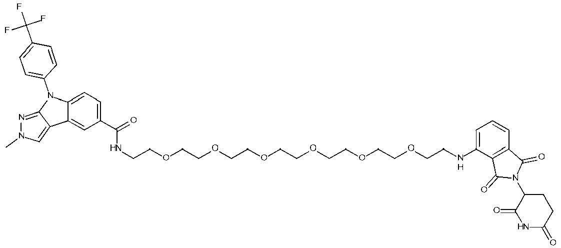 |
| 88 | 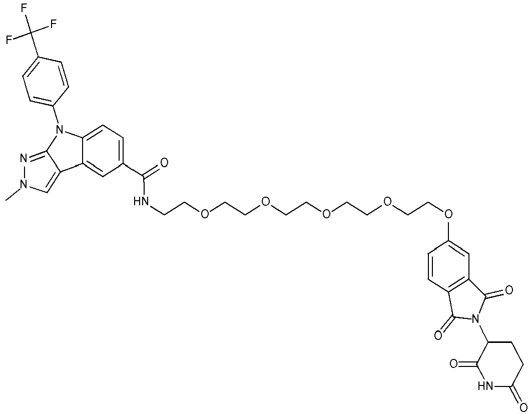 |
| 95 | 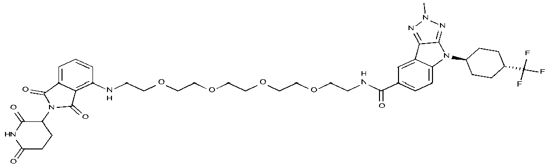 |
| 110 | 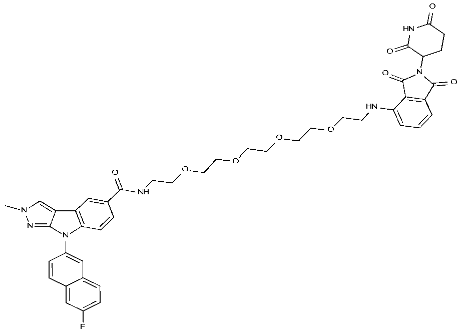 |
| 117 | 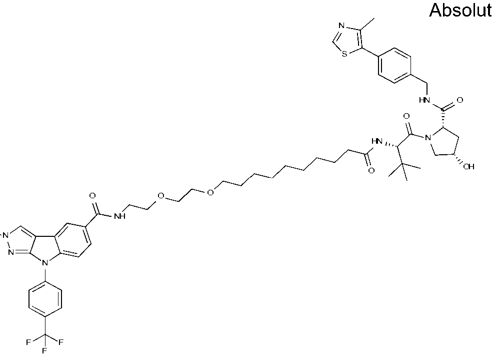 |
oder ein pharmazeutisch unbedenkliches Salz, Solvat, Tautomer und/oder Stereoisomer davon.

13. Verbindung nach einem beliebigen vorhergehenden Anspruch zur Verwendung als Arzneimittel.

14. Verbindung nach einem irgendeinem der Ansprüche 1 bis 12 oder ein pharmazeutisch unbedenkliches Salz, Solvat, Tautomer und/oder Stereoisomer davon, zur Verwendung bei der Behandlung und/oder Prävention einer Krankheit oder eines Leidens, die/das ausgewählt ist aus der Gruppe bestehend aus: Krebs, insbesondere Tumoren einschließlich fester Tumoren, von Brustkrebs, Lungenkrebs, Leberkrebs, Eierstockkrebs, Plattenepithelkrebs, Nierenkrebs, Magenkrebs, Medulloblastom, Kolonkrebs, Bauchspeicheldrüsenkrebs; Herz-Kreislauf-Erkrankungen und Fibrose, insbesondere Leberfibrose.

15. Pharmazeutische Zusammensetzung enthaltend eine Verbindung nach irgendeinem der Ansprüche 1 bis 12 oder ein pharmazeutisch unbedenkliches Salz, Solvat, Tautomer und/oder Stereoisomer davon, als Wirkstoff zusammen mit einem pharmazeutisch unbedenklichen Trägerstoff.

16. Pharmazeutische Zusammensetzung nach Anspruch 15, die ferner einen zweiten Wirkstoff oder ein pharmazeutisch unbedenkliches Salz, Solvat, Tautomer und/oder Stereoisomer davon enthält, wobei dieser zweite Wirkstoff von einer Verbindung der Formel I wie in irgendeinem der Ansprüche 1 bis 12 definiert verschieden ist.

17. Set( Kit ) umfassend getrennte Packungen von
a) einer wirksamen Menge einer Verbindung der Formel I nach irgendeinem der Ansprüche 1 bis 12 oder ein pharmazeutisch unbedenkliches Salz, Solvat, Tautomer und/oder Stereoisomer davon; und
b) einer wirksamen Menge eines weiteren Wirkstoffs, wobei es sich bei dem weiteren Wirkstoff nicht um eine Verbindung der Formel I wie in irgendeinem der Ansprüche 1 bis 12 definiert handelt.

18. Verfahren zum Abbau eines TEAD-Proteins, umfassend das In-Kontakt-Bringen des TEAD-Proteins mit einer Verbindung wie in irgendeinem der Ansprüche 1 bis 12 definiert.

19. Ternärer Komplex, umfassend
(i) eine Verbindung irgendeines der Ansprüche 1 bis 12;
(ii) ein TEAD-Protein; und
(iii) eine Ubiquitinligase.

20. Verbindung ausgewählt aus der Gruppe bestehend aus den in Tabelle INT abgebildeten Zwischenprodukten
| **Zwischenprodukt** | **Struktur** |
|---|---|
| 1 | 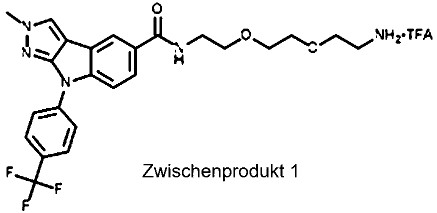 |
| 8 | 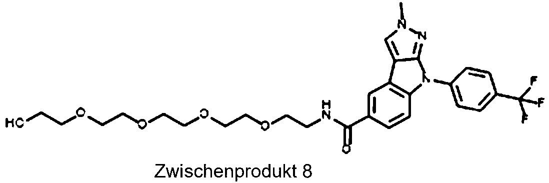 |
| 9 | 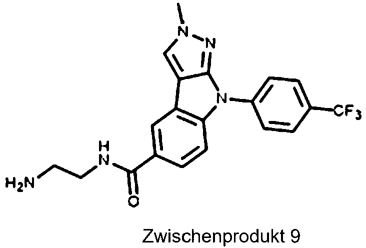 |
| 10 | 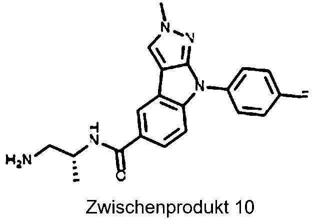 |
| 11 | 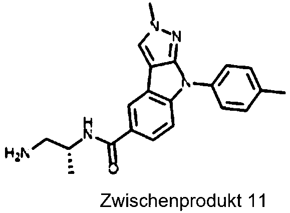 |
| 20 | 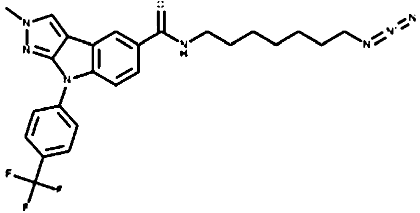 |
| 29 | 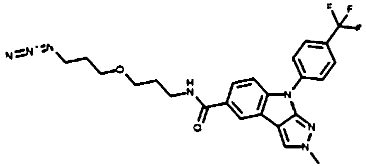 |
| 33 | 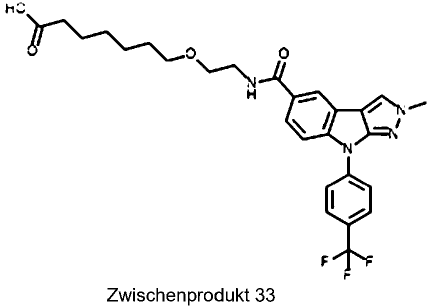 |
| 34 | 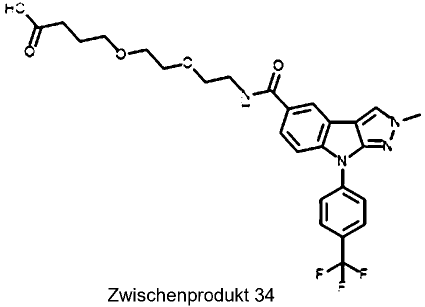 |
| 37 | 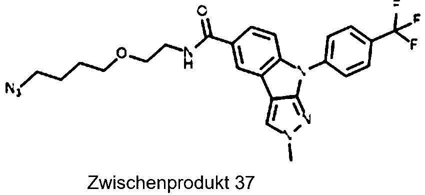 |
| 38 | 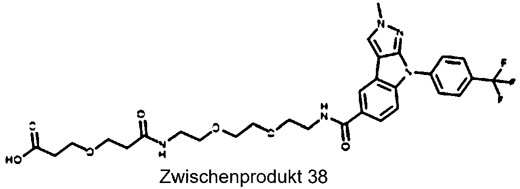 |
| 39 | 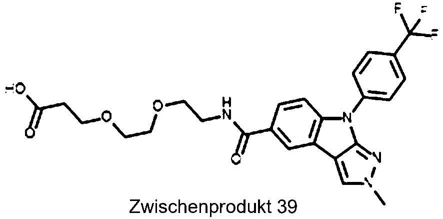 |
| 40 | 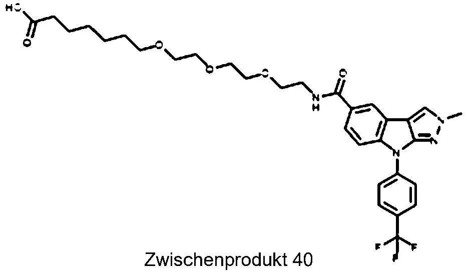 |
| 41 | 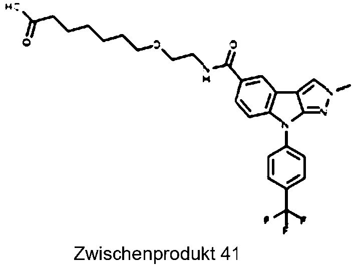 |
| 43 | 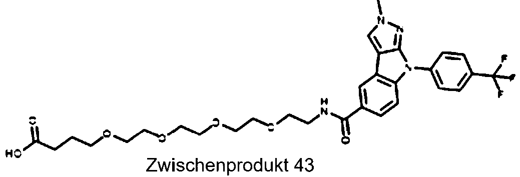 |
| 45 | 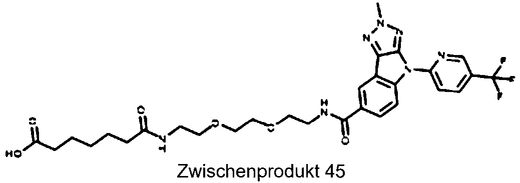 |
| 46 | 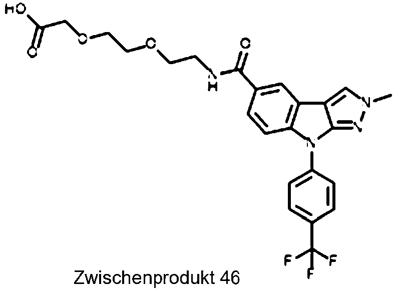 |
| 47 | 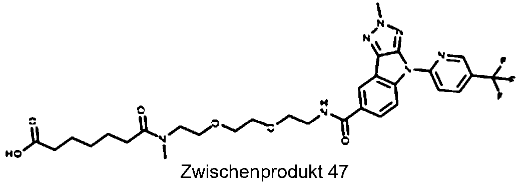 |
| 68 | 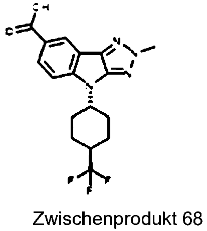 |
| 71 | 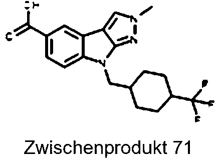 |
oder ein pharmazeutisch unbedenkliches Salz, Solvat, Tautomer und/oder Stereoisomer davon.

## Revendications

1. Composé de formule I
Q¹-Q²-Q³ I
dans laquelle
Q¹ est un ligand d'ubiquitine ligase ;
Q² est (i) absent ; ou (ii) un bras de liaison divalent formé par une chaîne alkylène non ramifiée ayant 2-25 atomes de C, où 1-8 groupements CH₂ non adjacents peuvent indépendamment les uns des autres être remplacés par O, -C(=O)-NH-, -NH-C(=O)-, - C(=O)-N(CH₃)-, -N(CH₃)-C(=O)-, -CH=CH- et/ou -C=C- ; où un ou deux groupements CH₂ peuvent éventuellement porter un substituant méthyle ; et où un groupement CH₂ peut être remplacé par un motif choisi dans le groupe constitué par 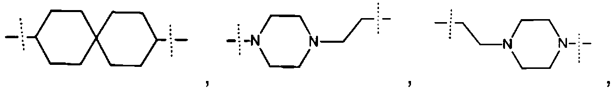 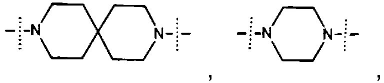 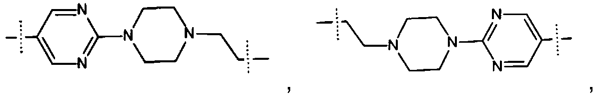 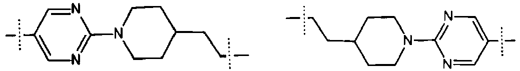 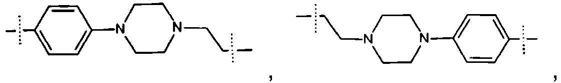 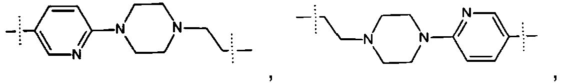 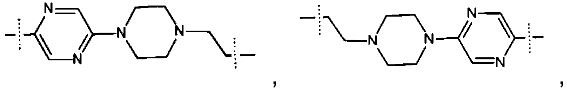 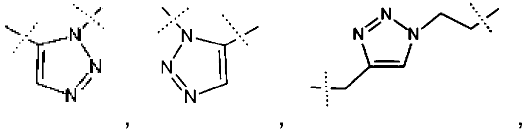 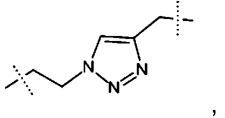 dans lequel l'extrémité gauche de ce motif est orientée vers la partie Q¹ du composé de formule I et l'extrémité droite de ce motif est orientée vers la partie Q³ du composé de formule I ;
Q³ désigne 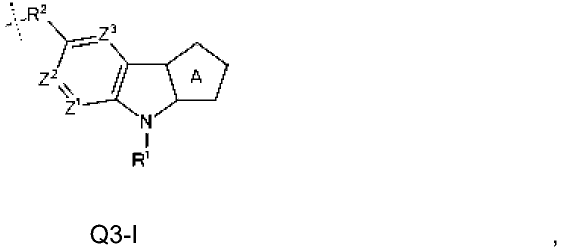 dans lequel
le Cycle A représente un cycle hétéroaromatique de cinq chaînons choisi dans le groupe constitué par les motifs cycliques suivants : 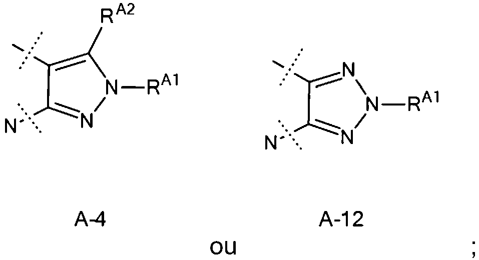 dans lesquels
R^{A1} représente méthyle ;
R^{A2} représente H ;
Z¹ est CH ;
Z² est CH ;
Z³ est CH ;
R¹ représente Ar¹, Hetar¹, Cyc¹, Hetcyc¹ ;
R² représente 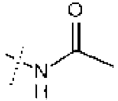 (-NH-C(=O)-) ;
Ar¹ est un groupement aryle mono- ou bicyclique ayant 6 ou 10 atomes de carbone de cycle, où ce groupement aryle peut être non substitué ou substitué par des substituants R^{B1}, R^{B2} et/ou R^{B3} qui peuvent être identiques ou différents ;
Hetar¹ est un groupement hétéroaryle monocyclique ayant 5 ou 6 atomes de cycle ou un groupement hétéroaryle bicyclique ayant 9 ou 10 atomes de cycle où 1, 2, 3 ou 4 parmi lesdits atomes de cycle est/sont un ou des hétéroatome(s) choisi(s) parmi N, O et/ou S et ceux restants sont des atomes de carbone, où ce groupement hétéroaryle peut être non substitué ou substitué par des substituants R^{B1}, R^{B2} et/ou R^{B3} qui peuvent être identiques ou différents ;
Cyc¹ est un carbocycle mono- ou bicyclique, saturé ou partiellement insaturé, ayant 3, 4, 5, 6, 7, 8, 9 ou 10 atomes de carbone de cycle, où ce carbocycle peut être non substitué ou substitué par R^{B4}, R^{B5} et/ou R^{B6} qui peuvent être identiques ou différents ;
Hetcyc¹ est un hétérocycle monocyclique, saturé ou partiellement insaturé, ayant 5 ou 6 atomes de cycle où 1 ou 2 parmi lesdits atomes de cycle est/sont un ou des hétéroatome(s) choisi(s) parmi N, O et/ou S et ceux restants sont des atomes de carbone, où cet hétérocycle peut être non substitué ou substitué par R^{B4}, R^{B5} et/ou R^{B6} qui peuvent être identiques ou différents, où, si l'un des hétéroatomes est S, alors cet hétérocycle peut également être substitué par R^{B4}, R^{B5}, R^{B6}, R^{B7} et/ou R^{B8} ;
R^{B1}, R^{B2}, R^{B3} représentent indépendamment les uns des autres C₁₋₆-alkyle à chaîne linéaire ou ramifiée, lequel C₁₋₆-alkyle pouvant être non substitué ou monosubstitué par -CN ou substitué par 1, 2 ou 3 halogènes, C₁₋₄-alcoxy à chaîne linéaire ou ramifiée, lequel C₁₋₄-alcoxy pouvant être non substitué ou substitué par 1, 2 ou 3 halogènes, -O-CH₂-C=CH, -S-C₁₋₄-alkyle à chaîne linéaire ou ramifiée, lequel -S-C₁₋₄-alkyle pouvant être non substitué ou substitué par 1, 2 ou 3 halogènes, C₂₋₆-alcényle à chaîne linéaire ou ramifiée, lequel C₂₋₆-alcényle pouvant être non substitué ou monosubstitué par -CN ou substitué par 1, 2 ou 3 halogènes, F, CI, Br, -CN, -S(=O)-C₁₋₃-alkyle, S(=O)₂-C₁₋₃-alkyle, -N(C₁₋₃-alkyl)₂, Ar², -CH₂-Ar², Hetar², Cyc², Hetcyc² ;
ou deux R^{B1}, R^{B2} et/ou R^{B3} adjacents forment ensemble un radical -C₃₋₄-alkylène divalent dans lequel l'un des motifs de carbone alkylénique peut être remplacé par un motif carbonyle (-C(=O)-), ou un radical -O-C₂₋₃-alkylène divalent ;
R^{B4}, R^{B5}, R^{B6} représentent indépendamment les uns des autres F, C₁₋₄-alkyle, où C₁₋₄-alkyle peut être non substitué ou substitué par 1, 2 ou 3 F, C₁₋₄-alcoxy, phényle ; ou
deux parmi R^{B4}, R^{B5} et R^{B6} sont fixés au même atome de carbone dudit carbocycle Cyc¹ ou dudit hétérocycle Hetcyc¹ et forment un groupement oxo (=O) divalent ; ou
R^{B4} et R^{B5} et R^{B7} et R^{B8} sont fixés au même atome de soufre dudit hétérocycle et forment deux groupements oxo (=O) divalents formant ainsi un motif -S(=O)₂- ;
Ar² est phényle qui peut être non substitué ou substitué par un ou deux substituants qui sont choisis indépendamment l'un de l'autre parmi OH, F, CI, Br, C₁₋₄-alkyle et C₁₋₄-alcoxy, où ce groupement C₁₋₄-alkyle ou C₁₋₄-alcoxy peut être substitué par 1, 2 ou 3 atomes de F ;
Hetar² est un groupement hétéroaryle monocyclique ayant 5 ou 6 atomes de cycle où 1, 2, 3, 4, 5 parmi lesdits atomes de cycle est/sont un ou des hétéroatome(s) choisi(s) parmi N, O et/ou S et ceux restants sont des atomes de carbone, où ce groupement hétéroaryle peut être non substitué ou substitué par un ou deux substituants qui sont choisis indépendamment l'un de l'autre parmi F, CI, Br, C₁₋₄-alkyle et C₁₋₄-alcoxy, où ce groupement C₁₋₄-alkyle ou C₁₋₄-alcoxy peut être substitué par 1, 2 ou 3 atomes de F ;
Cyc² est cyclopropyle, cyclobutyle, cyclopentyle ou cyclohexyle, chacun d'entre eux pouvant être non substitué ou substitué par un ou deux substituants qui sont choisis indépendamment l'un de l'autre parmi OH, F, CI, Br, C₁₋₄-alkyle et C₁₋₄-alcoxy, où ce groupement C₁₋₄-alkyle ou C₁₋₄-alcoxy peut être substitué par 1, 2 ou 3 atomes de F et/ou 1 groupement hydroxy ;
Hetcyc² est pyrrolidinyle, pipéridinyle, chacun d'entre pouvant être non substitué ou substitué par un ou deux substituants qui sont choisis indépendamment l'un de l'autre parmi OH, F, CI, Br, C₁₋₄-alkyle et C₁₋₄-alcoxy, où ce groupement C₁₋₄-alkyle ou C₁₋₄-alcoxy peut être substitué par 1, 2 ou 3 atomes de F et/ou 1 groupement hydroxy ;
halogène est F, Cl, Br, I ;
ou un sel, solvate, tautomère et/ou stéréoisomère pharmaceutiquement acceptable de celui-ci.

2. Composé selon la revendication 1, dans lequel
Q¹ est un ligand d'ubiquitine ligase E3 ; éventuellement Q¹ est a) un ligand de CRBN (céréblon) ou (b) un ligand de VHL (von Hippel-Lindau) ou (c) un type différent de ligand d'ubiquitine ligase E3 autre qu'un ligand de CRBN ou de VHL ;
ou un sel, solvate, tautomère et/ou stéréoisomère pharmaceutiquement acceptable de celui-ci.

3. Composé selon la revendication 2, dans lequel (a) le ligand de CRBN (céréblon) présente une structure de formule Q1-I, Q1-II, Q1-VII ou Q1-VIII ; (b) le ligand de VHL présente une structure de formule Q1-III ; (c) le type différent de ligand d'ubiquitine ligase E3 présente une structure de formule Q1-IV, Q1-V, Q1-VI, Q1-XII ou Q1-XIII : 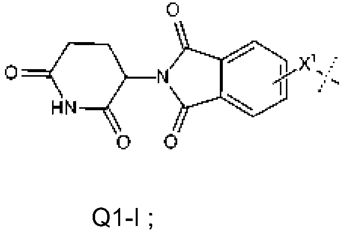 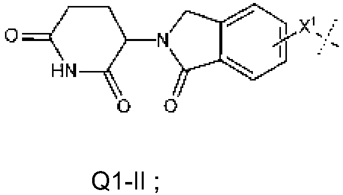 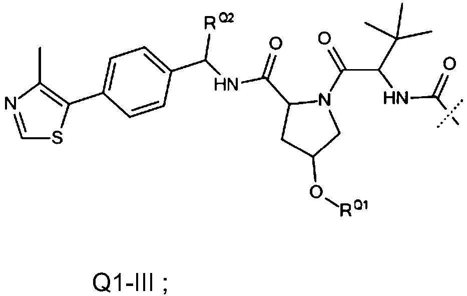 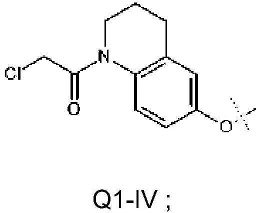 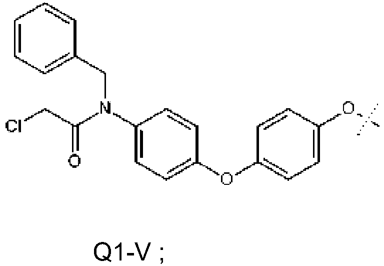 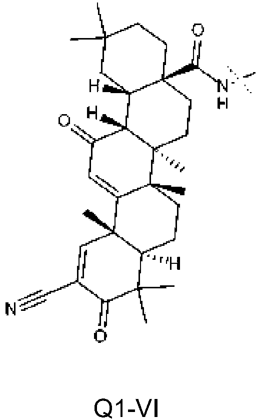 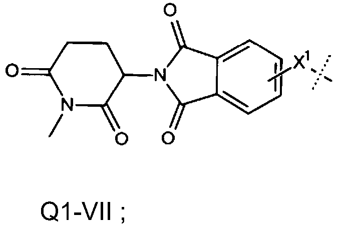 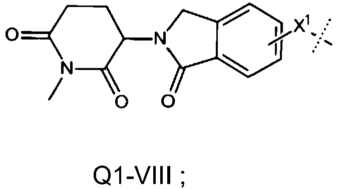 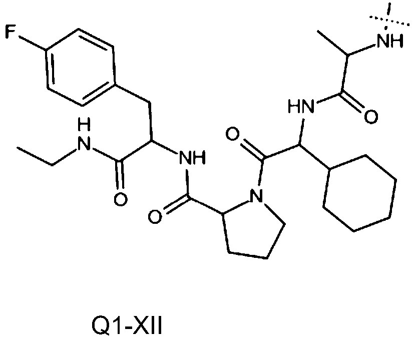 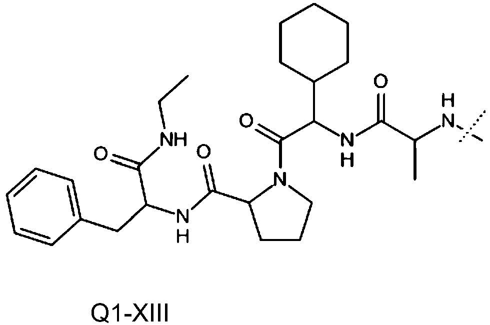 dans laquelle
X¹ désigne une liaison simple, -O-, -NH-, -NCH₃-, -CH₂-, ou -NH-C(=O)- ;
R^{Q1} désigne H ou -C(=O)-CH₃ ;
R^{Q2} désigne H ou CH₃;
ou un sel, solvate, tautomère et/ou stéréoisomère pharmaceutiquement acceptable de celui-ci.

4. Composé selon l'une quelconque des revendications précédentes, dans lequel Q¹ est un ligand de CRBN (céréblon) ; et le ligand de CRBN (céréblon) présente une structure choisie parmi les structures de formules Q1-I-1, Q1-I-2, Q1-I-3 ; Q1-I-4, Q1-I-5, Q1-I-6, Q1-II-1, Q1-II-2, Q1-II-3, Q1-II-4, Q1-II-5, Q1-II-6, Q1-II-7, Q1-VII-1, Q1-VII-2, et Q1-VII-3 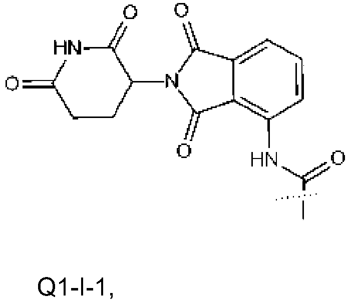 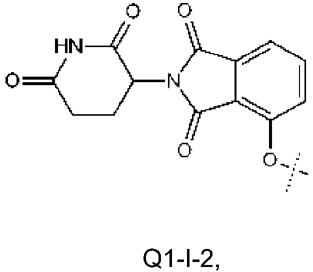 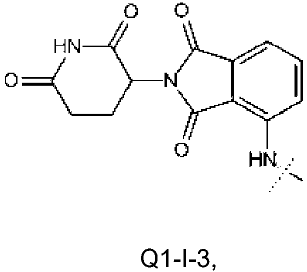 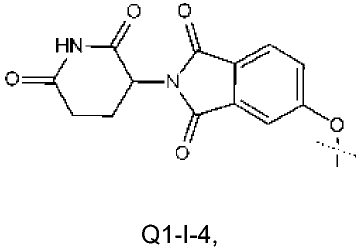 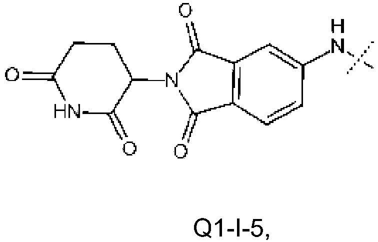 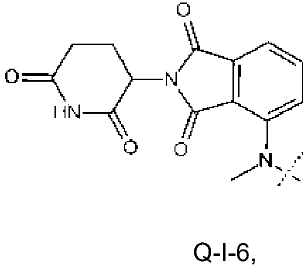 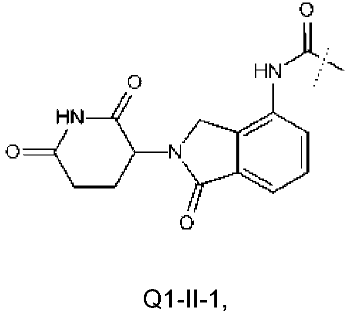 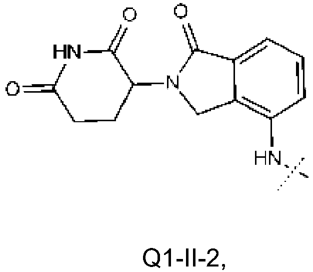 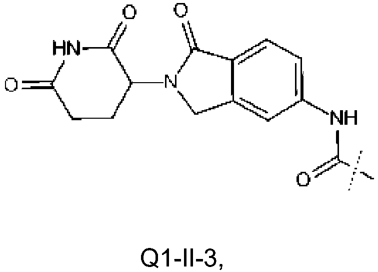 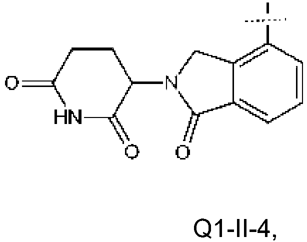 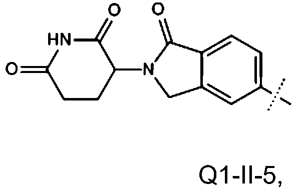 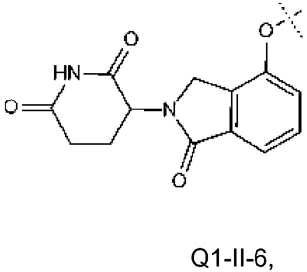 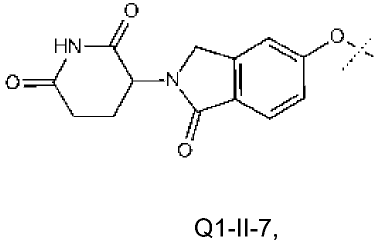 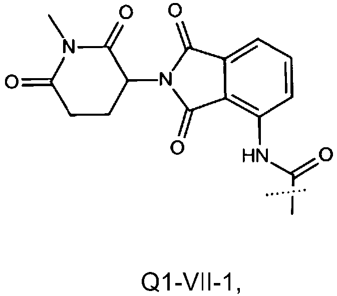 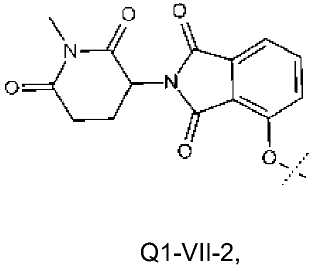 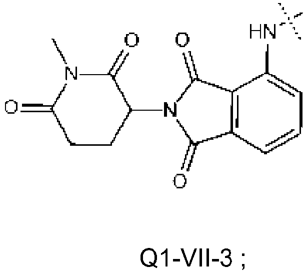 ou un sel, solvate, tautomère et/ou stéréoisomère pharmaceutiquement acceptable de celui-ci.

5. Composé selon l'une quelconque des revendications 1 à 3, dans lequel Q¹ est un ligand de VHL (von-Hippel-Lindau) ; et le ligand de VHL présente une structure choisie parmi les formules Q1-III-1, Q1-III-2, Q1-III-3, Q1-III-4, et Q1-III-5 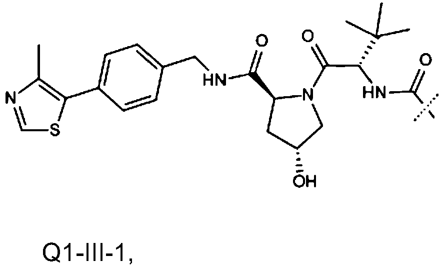 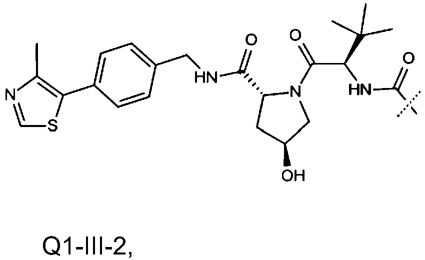 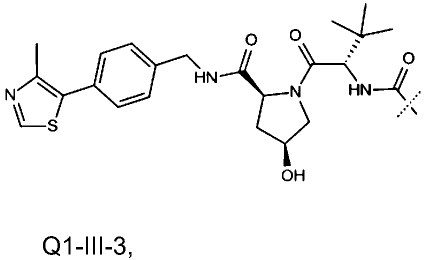 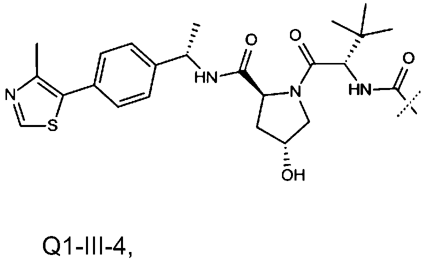 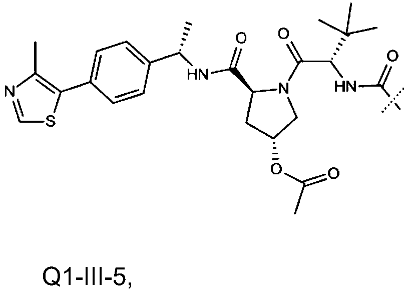 ou un sel, solvate, tautomère et/ou stéréoisomère pharmaceutiquement acceptable de celui-ci.

6. Composé selon l'une quelconque des revendications précédentes, dans lequel
Q² est choisi dans le groupe constitué par 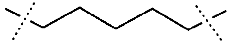 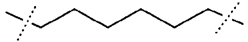 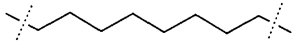 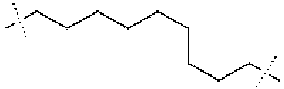 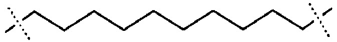 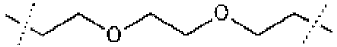 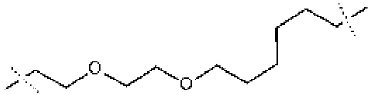 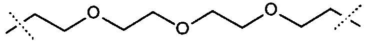 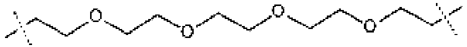 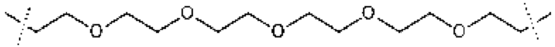 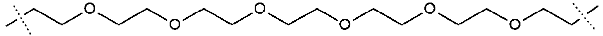 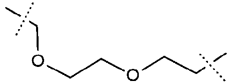 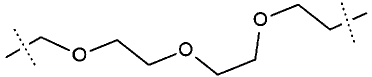 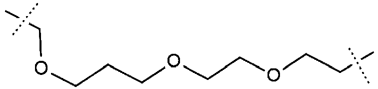 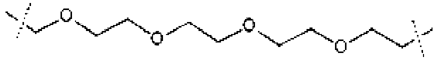 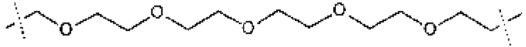 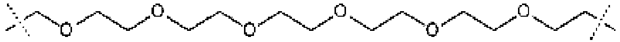 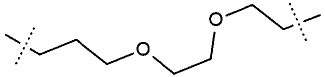 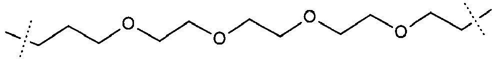 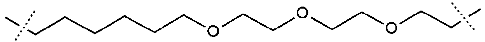 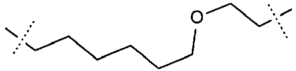 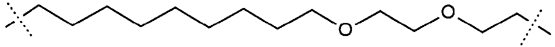 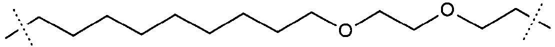 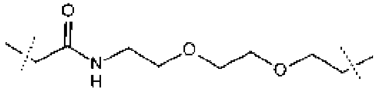 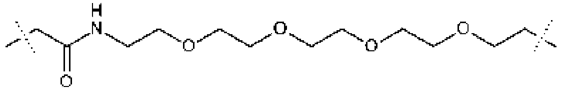 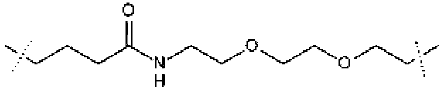 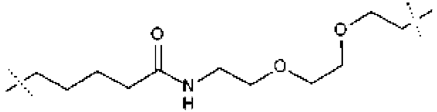 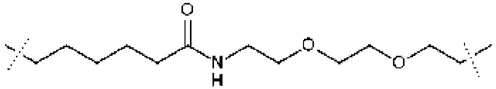 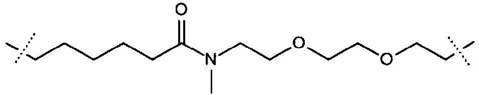 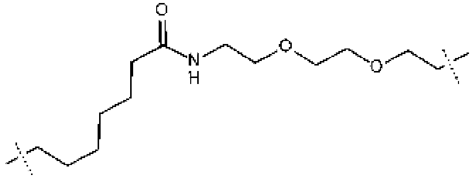 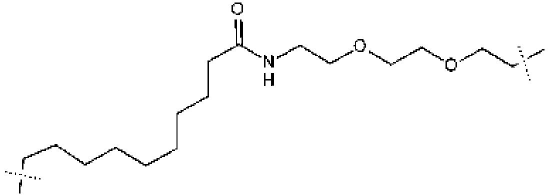 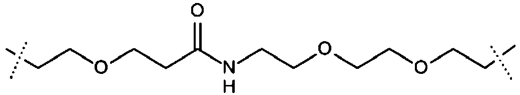 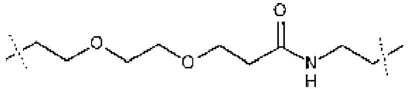 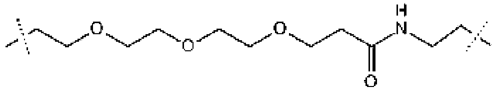 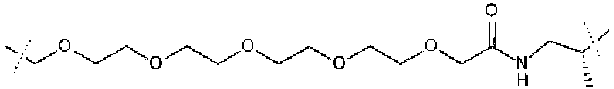 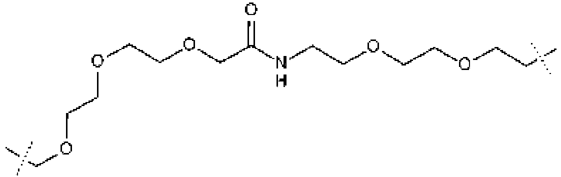 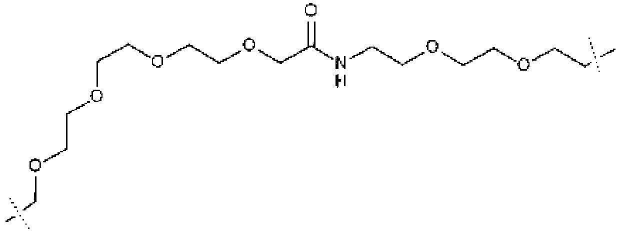 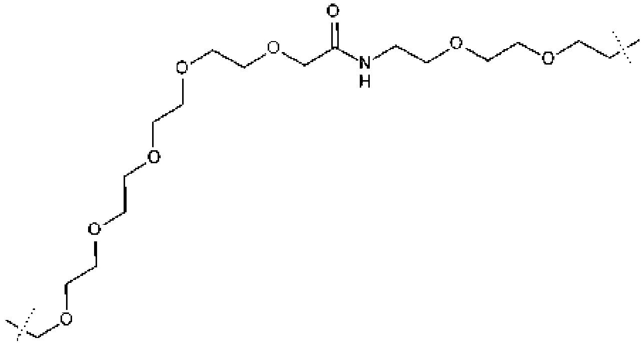 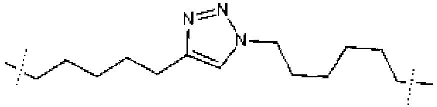 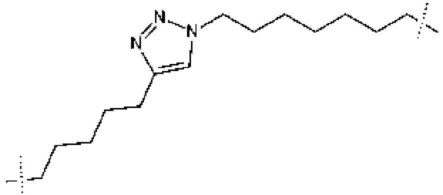 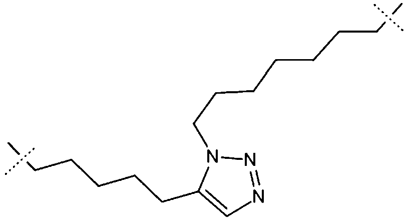 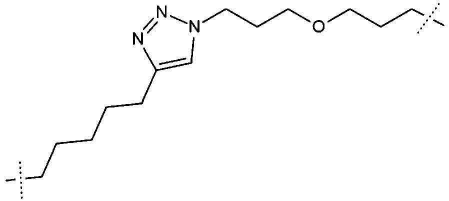 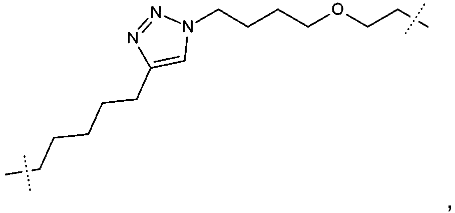
éventuellement Q2 est choisi dans le groupe constitué par 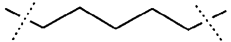 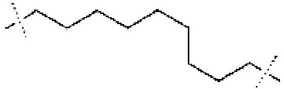 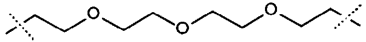 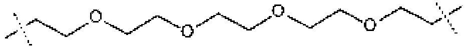 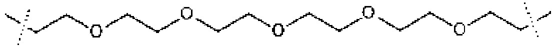 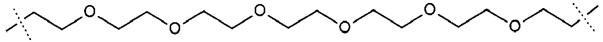 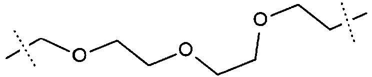 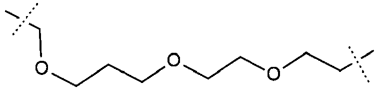 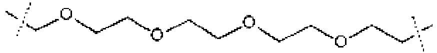 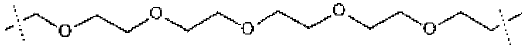 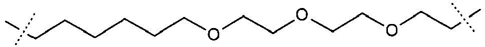 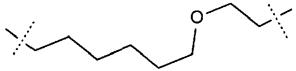 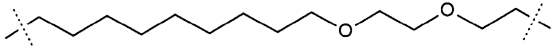 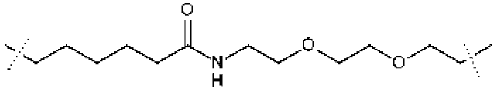 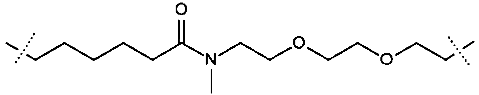 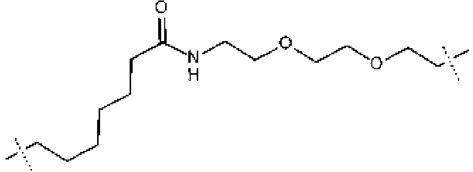 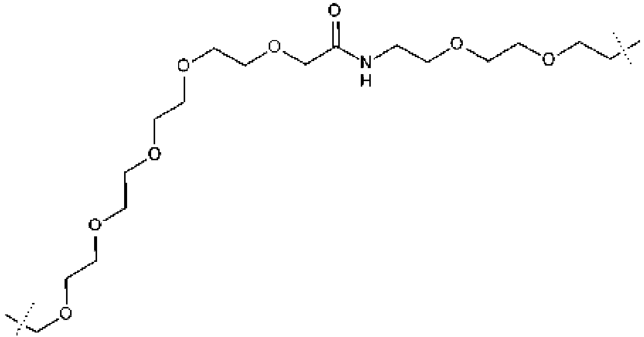 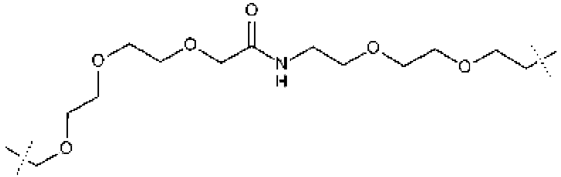 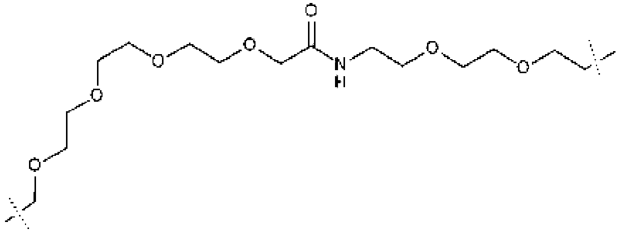 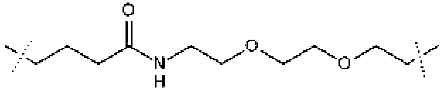 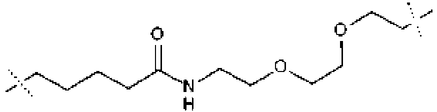 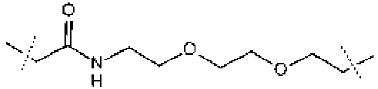 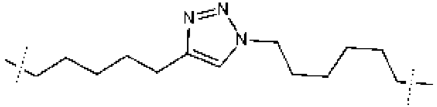 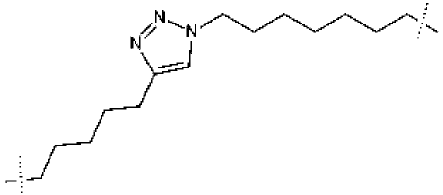 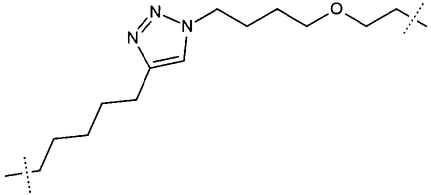 ou un sel, solvate, tautomère et/ou stéréoisomère pharmaceutiquement acceptable de celui-ci.

7. Composé selon l'une quelconque des revendications précédentes, dans lequel
R¹ représente phényle, 3-fluorophényle, 4-fluorophényle, 4-chlorophényle, 4-méthylphényle, 4-éthylphényle, 4-difluorométhylphényle, 3-trifluorométhylphényle, 4-trifluorométhylphényle, 4-(1,1-difluoréthyl)phényle, 4-(2,2,2-trifluoréthyl)phényle, 4-(1-trifluorométhylcyclopropyl)-phén-1-yle, 4-cyclo-pentylphényle, 4-éthoxyphényle, 4-difluorométhoxyphényle, 4-trifluoro-méthoxyphényle, 3-(trifluorométhyl)sulfanylphényle, 4-(trifluorométhyl)-sulfanylphényle, 3-trifluorométhyl-4-méthylphényle, 2-fluoro-4-trifluorométhylphényle, 2-fluoro-4-trifluorométhoxyphényle, 3-fluoro-4-(n-propyl)-phényle, 2,3-diméthyl-4-méthoxyphényle, 6-fluoronapht-2-yle ; 5-trifluoro-méthylfuran-2-yle ; 5-trifluorométhylthiophén-2-yle, 2-trifluorométhyl-1,3-thiazol-4-yle, 3-fluoropyridin-2-yle, 6-méthylpyridin-3-yle, 6-méthoxypyridin-3-yle, 3-éthylpyridin-2-yle, 6-éthylpyridin-3-yle, 4-difluorométhylpyridin-2-yle, 4-trifluorométhylpyridin-2-yle, 4-trifluorométhoxypyridin-2-yle, 4-cyanopyridin-2-yle, 5-trifluorométhylpyridin-2-yle, 6-trifluorométhylpyridin-2-yle, 6-trifluorométhylpyridin-3-yle (2-trifluorométhylpyridin-5-yl), 6-trifluoro-méthoxypyridin-3-yle (2-trifluorométhoxypyridin-5-yl), 5-cyanopyridin-2-yle, 5-cyanométhylpyridin-2-yle, 5-méthanesulfonylpyridin-2-yle, 6-méthoxy-pyridin-2-yle, 4-méthylpyrimidin-2-yle, 4-éthylpyrimidin-2-yle, 4-méthyl-sulfanylpyrimidin-2-yle, 5-cyclopropylpyrimidin-2-yle, 5-éthylpyrimidin-2-yle, 5-difluorométhylpyrimidin-2-yle, 5-trifluorométhylpyrimidin-2-yle, 5-cyano-pyrimidin-2-yle, 5-cyano-3-fluoropyridin-2-yle, 5-cyano-6-méthylpyridin-2-yle, 3-fluoro-5-(trifluorométhyl)pyridin-2-yle, 5-oxo-5H,6H,7H-cyclo-penta[b]pyridin-2-yle, 5,6,7,8-tétrahydroquinoléin-2-yle, 5-oxo-5,6,7,8-tétrahydroquinoléin-2-yle, 5H,6H,7H-cyclopenta[b]pyridin-2-yle, quinoléin-2-yle, isoquinoléin-3-yle, 6-méthylquinoléin-2-yle, 8-méthoxyquinoléin-4-yle, furo[3,2-b]pyridin-5-yle, quinazolin-2-yle, 6-fluoroquinazolin-2-yle, 1,5-naphtyridin-2-yle ; 3-méthylcyclobutyle, cyclopentyle, 3-méthylcyclopentyle, 3,3-diméthylcyclopentyle, 3-trifluorométhyl-bicyclo[1.1.1]pétan-1-yle, cyclohexyle, 4-méthylcyclohexyle, 4-(trifluorométhyl)cyclohexyle, 4,4-difluorocyclohexyle, cyclohex-1-ényle, 2-oxocycloheptyle, 6,6-difluoro-spiro[3.3]heptan-2-yle, 1H-indén-2-yle ;
ou un sel, solvate, tautomère et/ou stéréoisomère pharmaceutiquement acceptable de celui-ci.

8. Composé selon l'une quelconque des revendications précédentes, dans lequel
R¹ représente 4-méthylphényle, 4-difluorométhylphényle, 4-trifluorométhylphényle, 4-fluorophényle ;
ou un sel, solvate, tautomère et/ou stéréoisomère pharmaceutiquement acceptable de celui-ci.

9. Composé selon l'une quelconque des revendications précédentes, dans lequel
Q³ présente une structure de formule Q3-I ;
le Cycle A est le cycle A-4 ou A-12 ;
R^{A1} est méthyle ;
R^{A2} est H ;
R¹ est 4-trifluorométhylphényle ;
R² représente 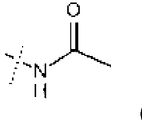 [-NH-C(=O)-) ;
Z¹, Z² et Z³ représentent chacun CH ;
ou un sel, solvate, tautomère et/ou stéréoisomère pharmaceutiquement acceptable de celui-ci.

10. Composé selon l'une quelconque des revendications précédentes, dans lequel
Q¹ est
(a) un ligand de CRBN (céréblon) ; et
le ligand de CRBN (céréblon) présente une structure choisie parmi les structures de formules Q1-I-1, Q1-I-2, Q1-I-3 ; Q1-I-4, Q1-I-5, Q1-I-6, Q1-II-1, Q1-II-2, Q1-II-3, Q1-II-4, Q1-II-5 et Q1-VII-1 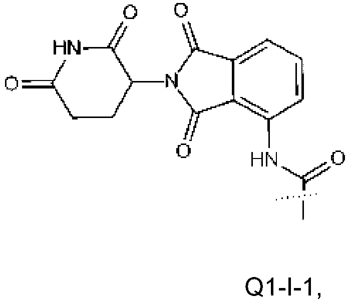 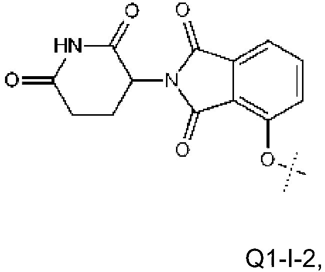 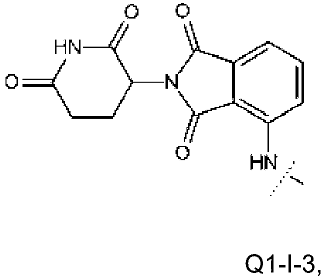 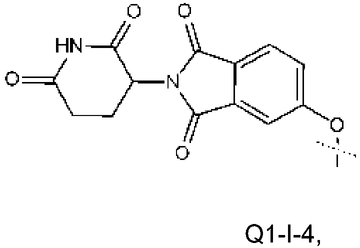 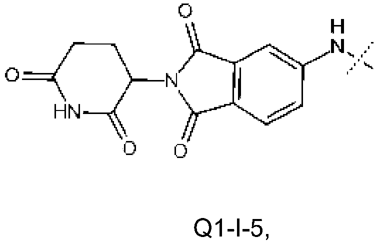 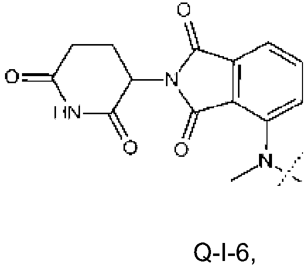 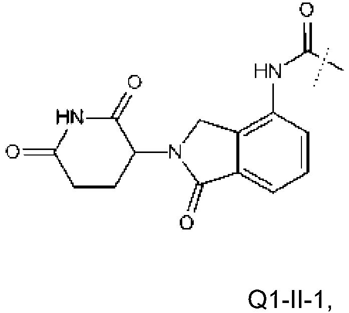 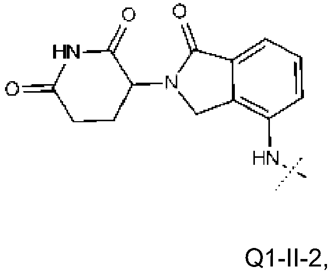 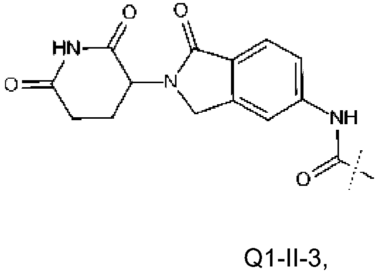 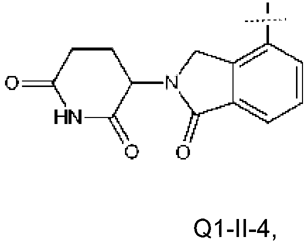 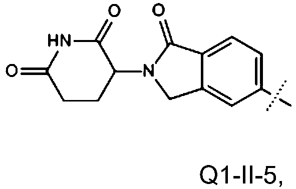 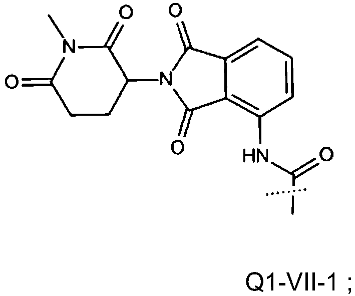 ou
(b) un ligand de VHL (von Hippel-Lindau) ; et le ligand de VHL présente une structure choisie parmi les structures de formule Q1-III-1, Q1-III-2, Q1-III-4, et Q1-III-5 : 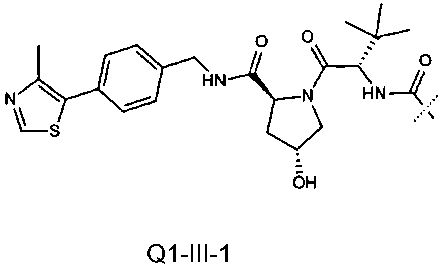 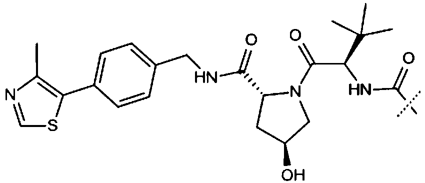 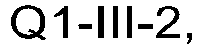 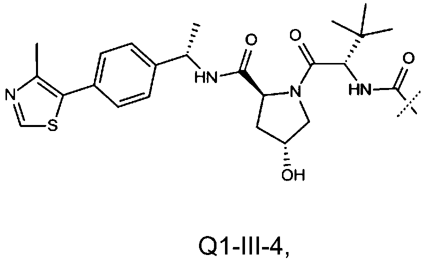 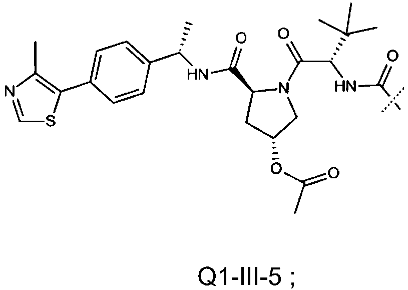
Q² est choisi dans le groupe constitué par 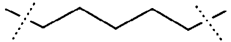 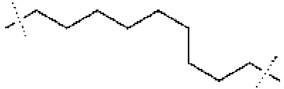 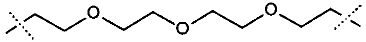 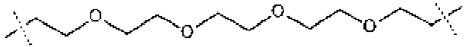 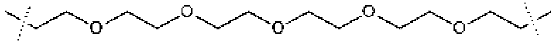 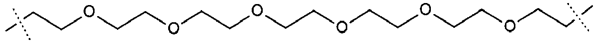 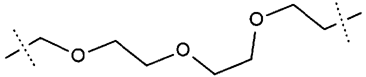 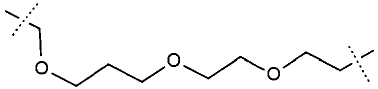 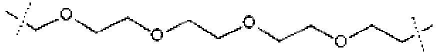 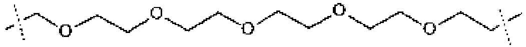 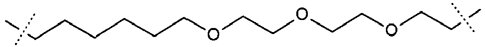 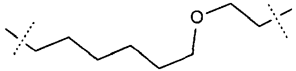 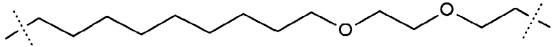 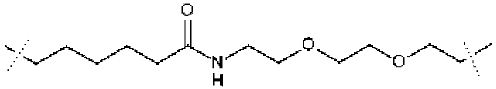 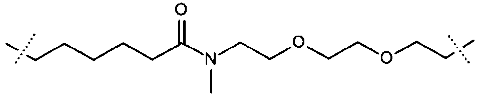 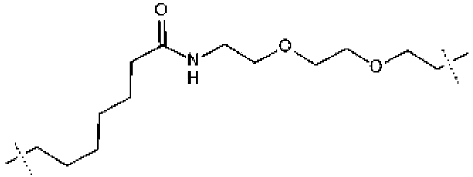 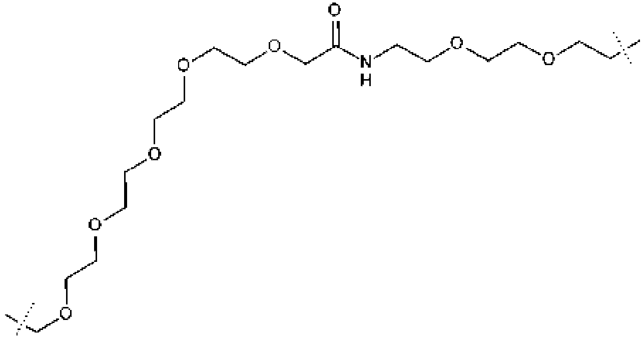 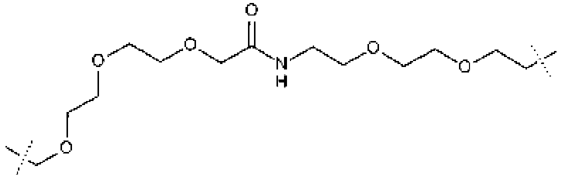 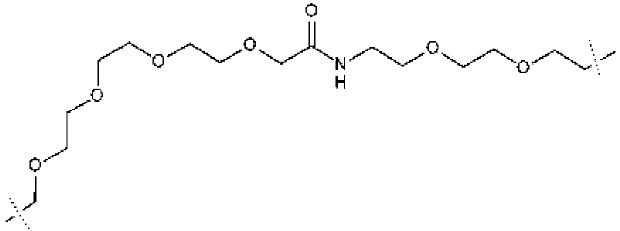 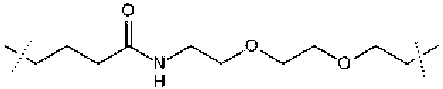 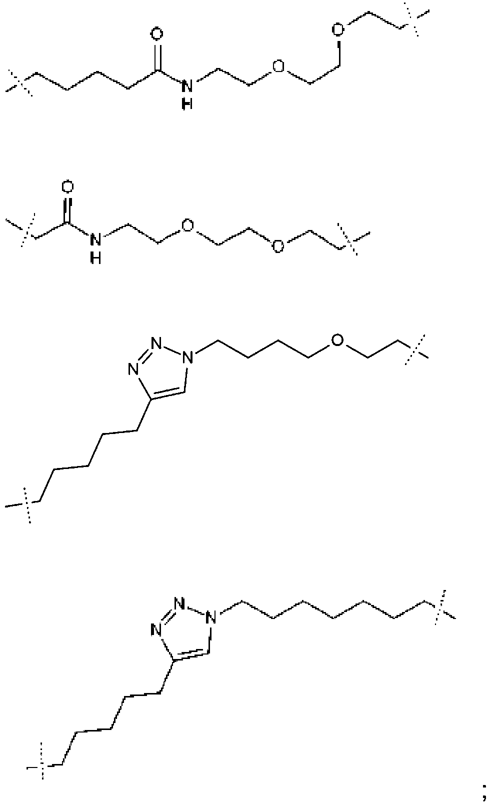
Q³ est Q3-I ;
le Cycle A est le cycle A-4 ou le cycle A-12 ;
R^{A1} représente méthyle ;
R^{A2} représente hydrogène ;
R¹ désigne 4-trifluorométhylphényle ;
R² représente 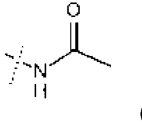 (-NH-C(=O)-) ;
Z¹, Z² et Z³ représentent chacun CH ;
ou un sel, solvate, tautomère et/ou stéréoisomère pharmaceutiquement acceptable de celui-ci.

11. Composé selon la revendication 10, dans lequel
Q¹ est (a) un ligand CRBN et présente une structure choisie parmi les structures de formule Q1-I-2, Q1-I-3, Q1-I-5, Q1-I-6, Q1-II-2, Q1-II-3, Q1-II-4, et Q1-II-5 ; ou est (b) un VHL ligand et présente une structure choisie parmi les structures de formule Q1-III-1 et Q1-III-4 ;
ou un sel, solvate, tautomère et/ou stéréoisomère pharmaceutiquement acceptable de celui-ci.

12. Composé selon la revendication 1, le composé étant choisi dans le Tableau 1
| **Composé n°** | **Structure** |
|---|---|
| 1 | 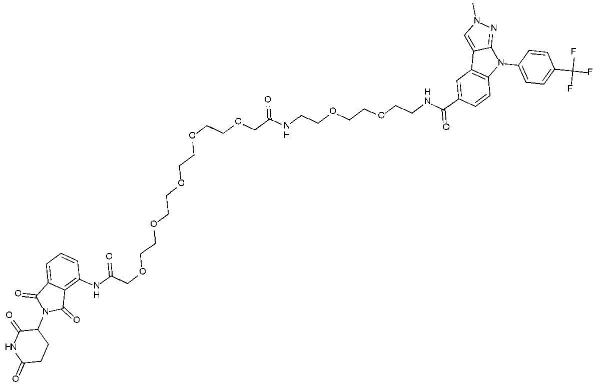 |
| 2 | 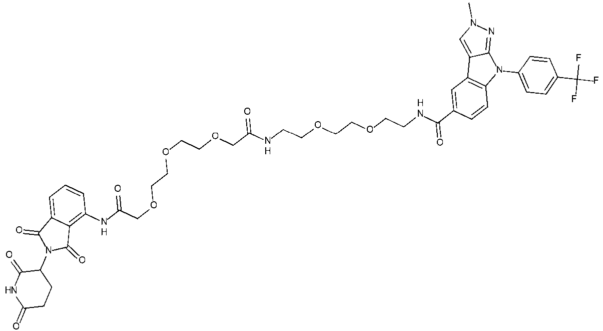 |
| 3 | 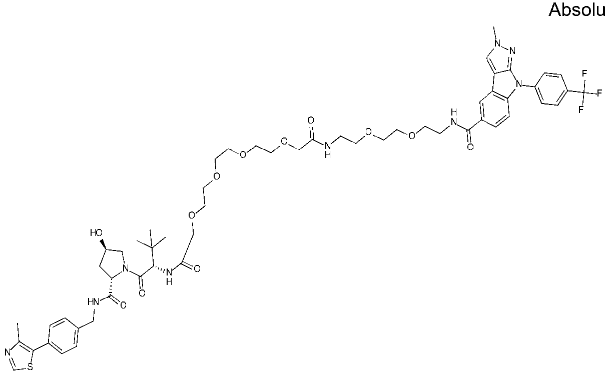 |
| 4 | 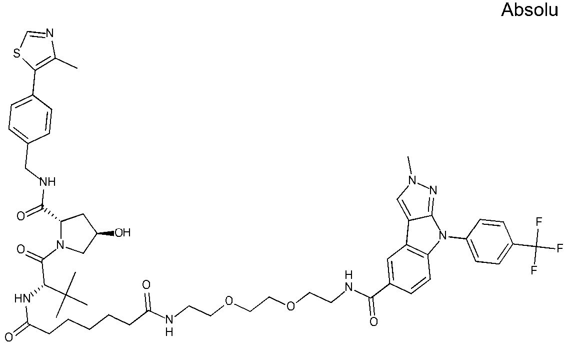 |
| 4-N | 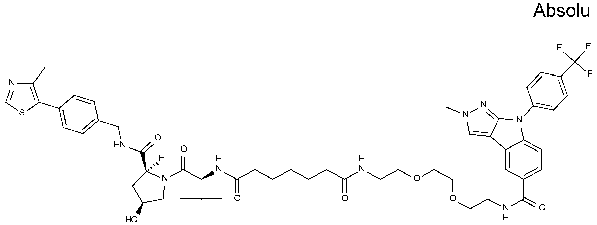 |
| 5 | 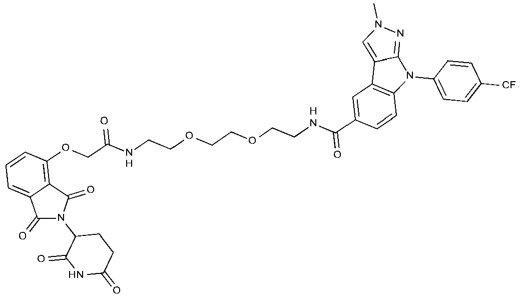 |
| 6 | 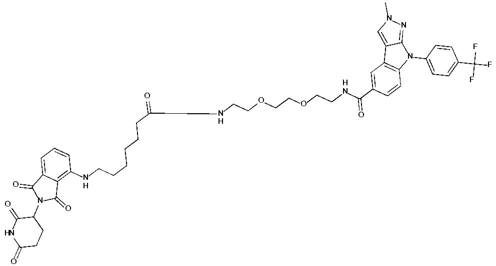 |
| 7 | 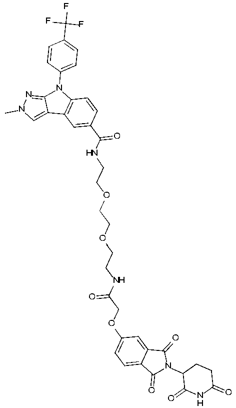 |
| 8 | 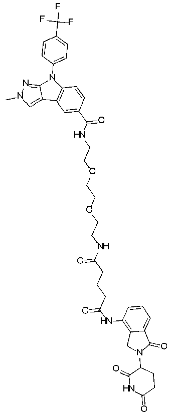 |
| 9 | 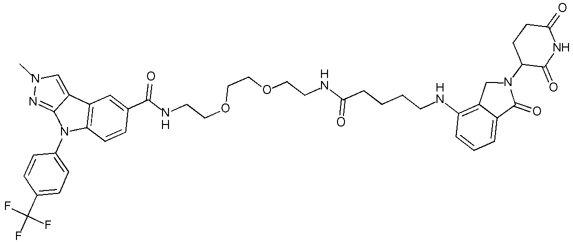 |
| 10 | 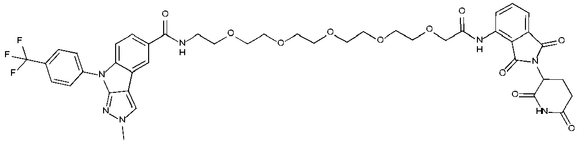 |
| 11 | 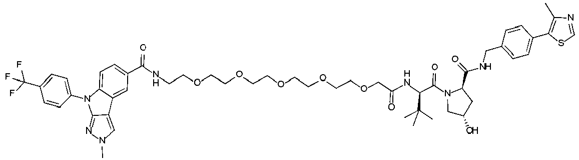 |
| 12 | 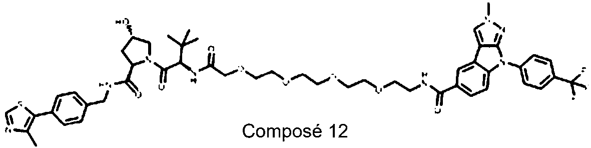 |
| 13 | 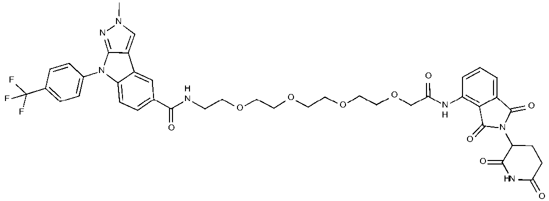 |
| 14 | 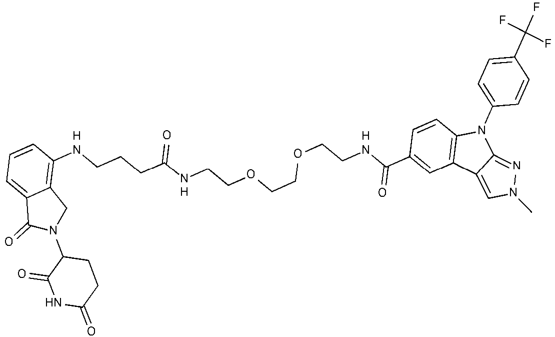 |
| 15 | 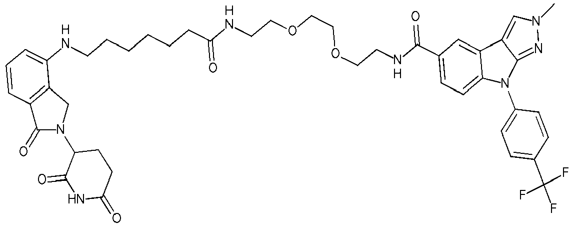 |
| 16 | 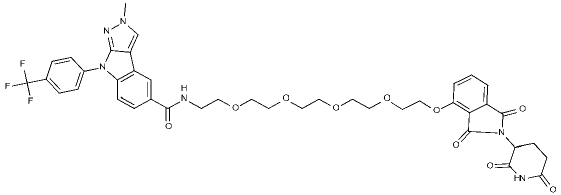 |
| 17 | 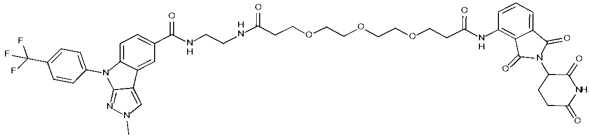 |
| 18 | 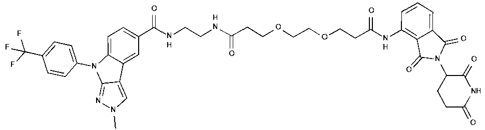 |
| 19 | 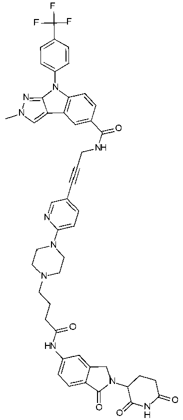 |
| 20 | 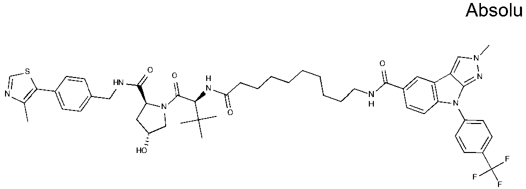 |
| 21 | 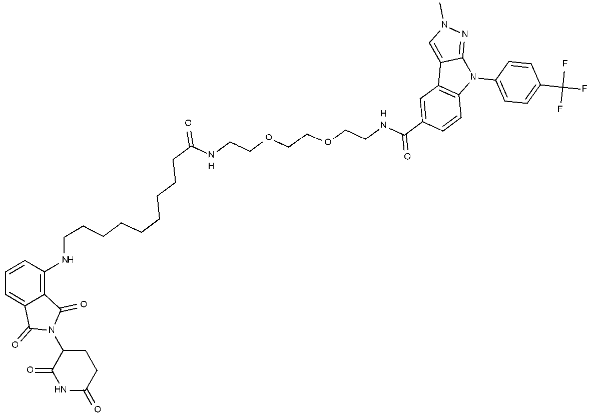 |
| 22 | 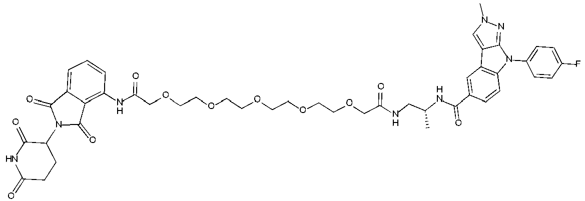 |
| 23 | 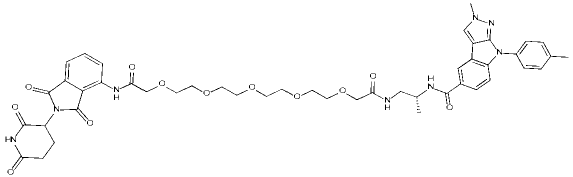 |
| 24 | 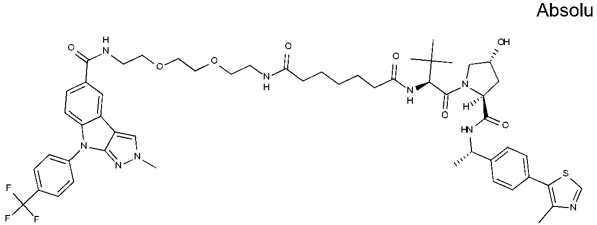 |
| 25 | 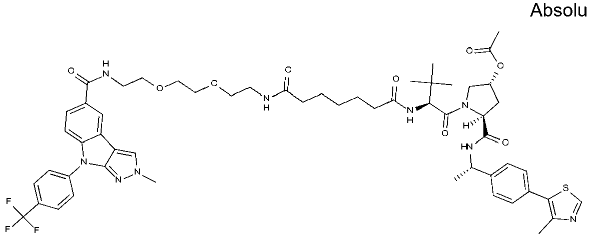 |
| 26 | 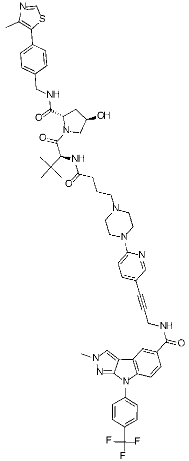 |
| 27 | 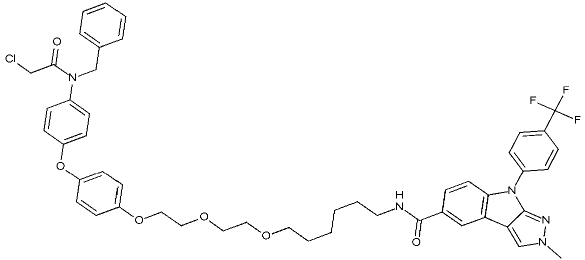 |
| 28 | 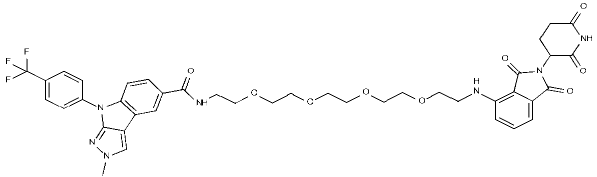 |
| 29 | 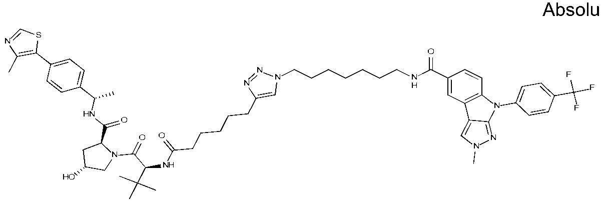 |
| 30 | 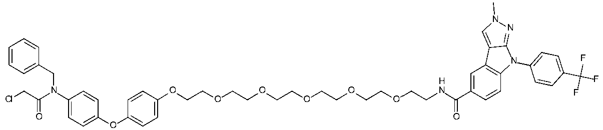 |
| 31 | 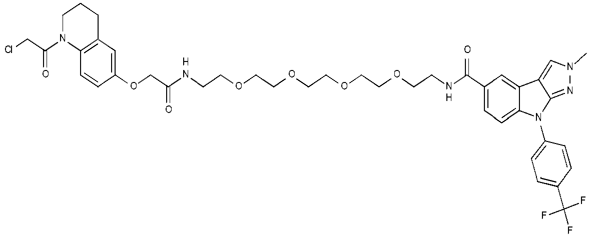 |
| 32 | 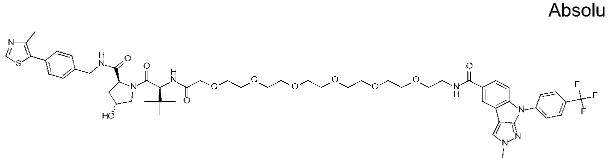 |
| 33 | 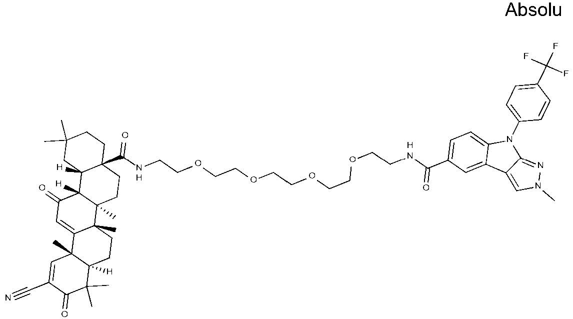 |
| 34 | 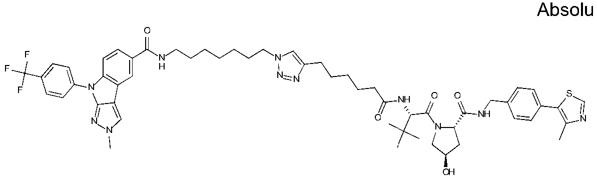 |
| 35 | 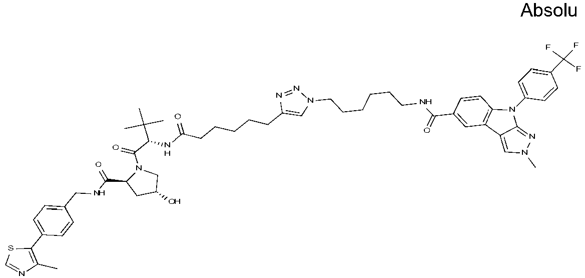 |
| 36 | 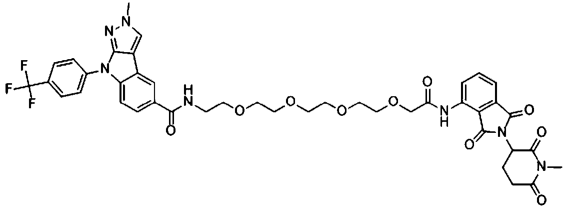 |
| 37 | 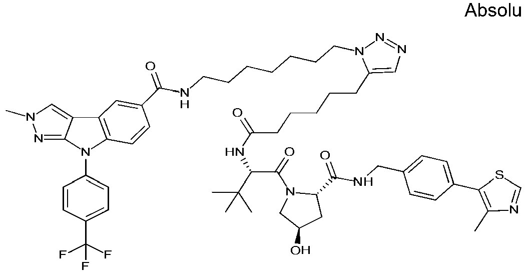 |
| 38 | 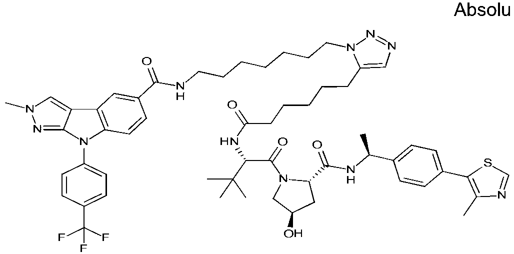 |
| 42 | 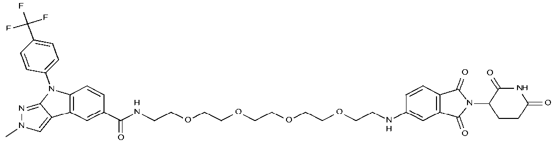 |
| 43 | 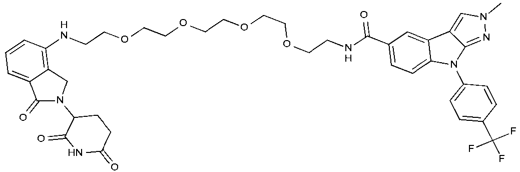 |
| 45 | 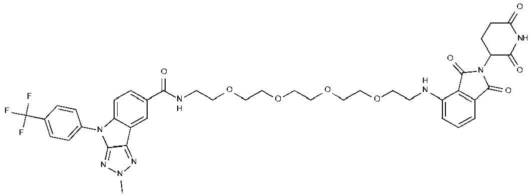 |
| 47 | 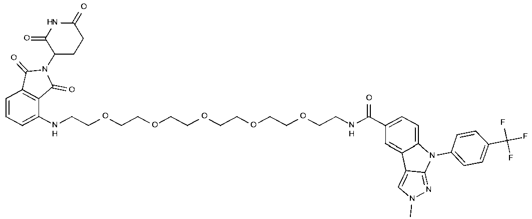 |
| 48 | 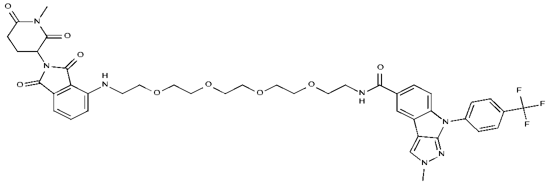 |
| 49 | 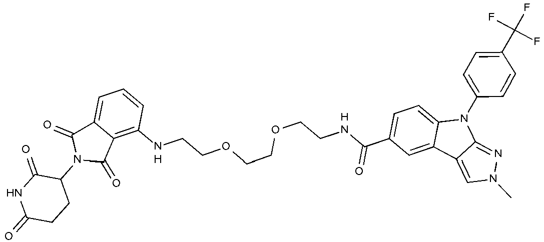 |
| 50 | 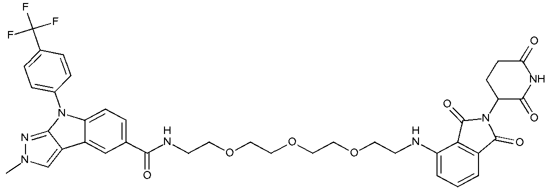 |
| 51 | 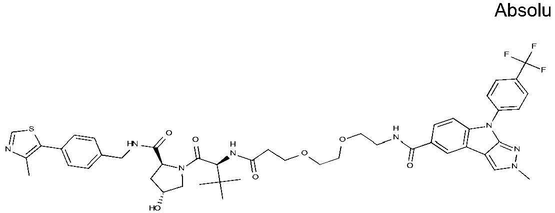 |
| 52 | 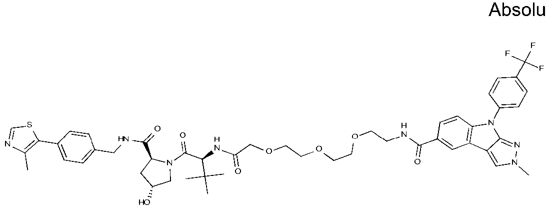 |
| 53 | 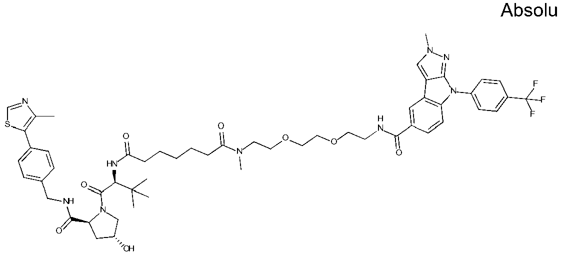 |
| 54 | 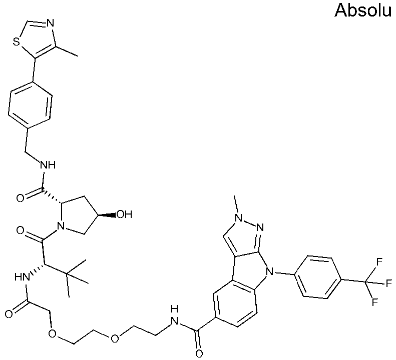 |
| 55 | 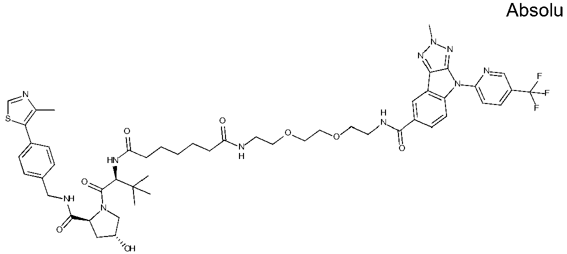 |
| 56 | 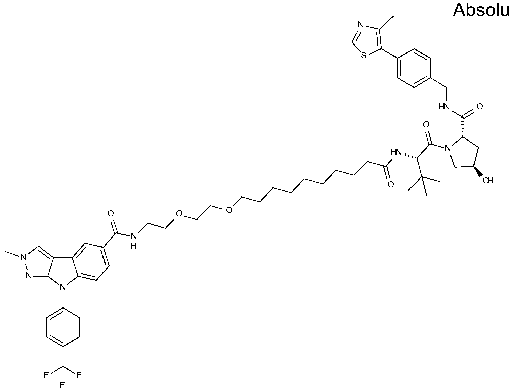 |
| 57 | 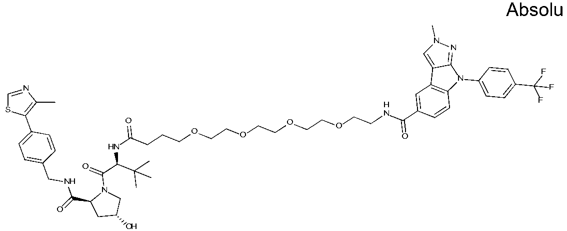 |
| 59 | 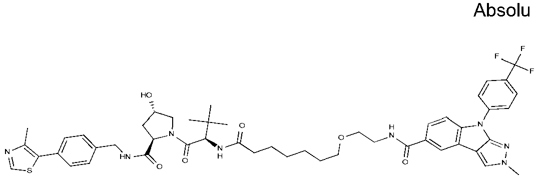 |
| 60 | 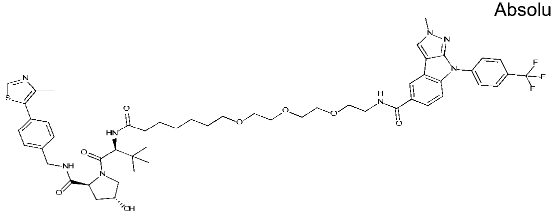 |
| 61 | 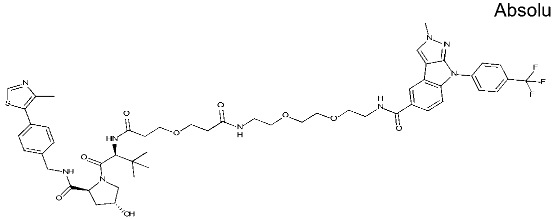 |
| 63 | 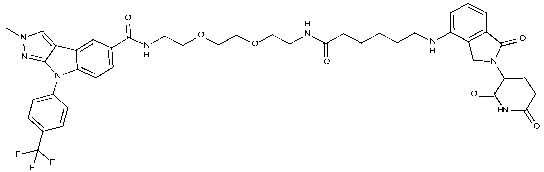 |
| 69 | 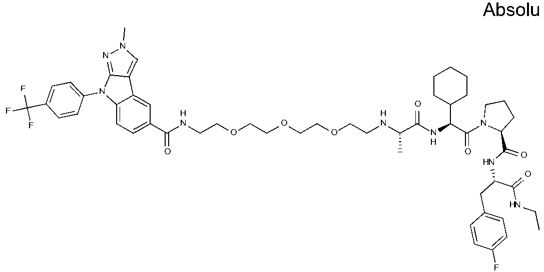 |
| 73 | 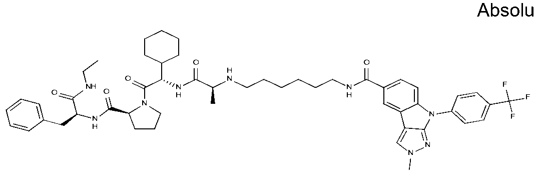 |
| 74 | 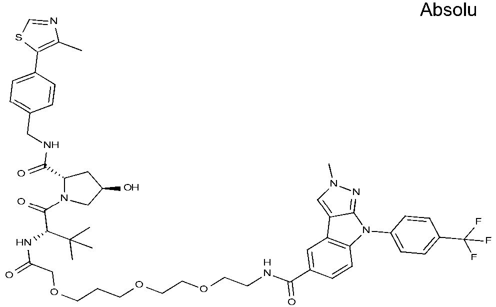 |
| 75 | 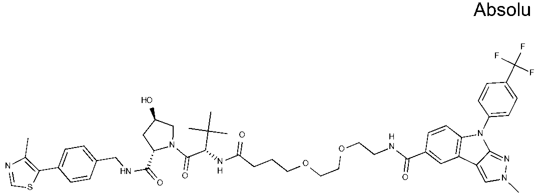 |
| 77 | 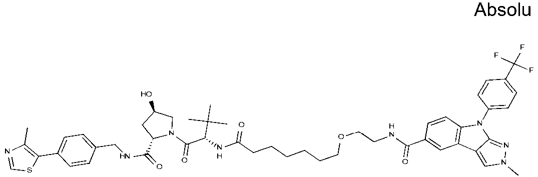 |
| 80 | 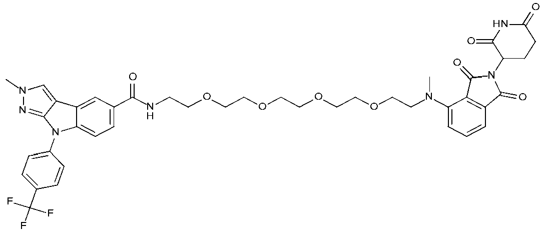 |
| 81 | 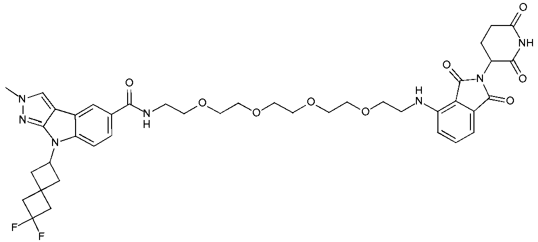 |
| 82 | 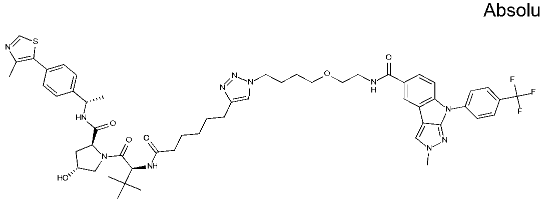 |
| 84 | 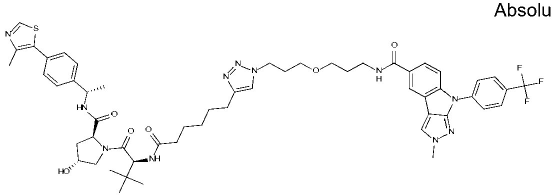 |
| 87 | 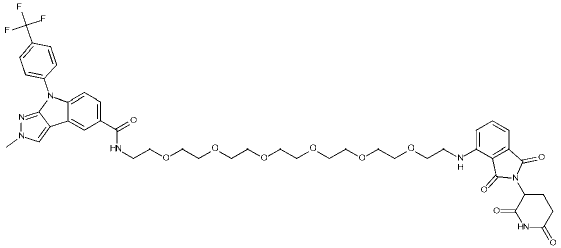 |
| 88 | 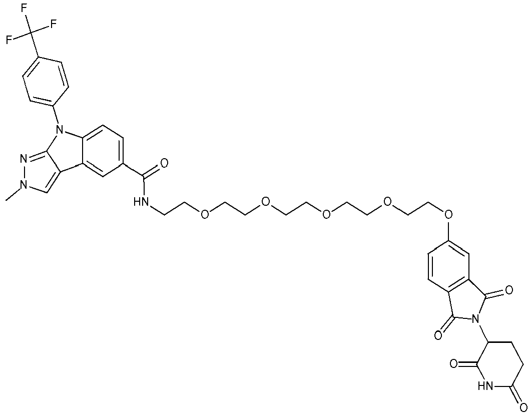 |
| 95 | 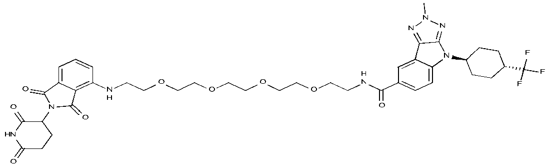 |
| 110 | 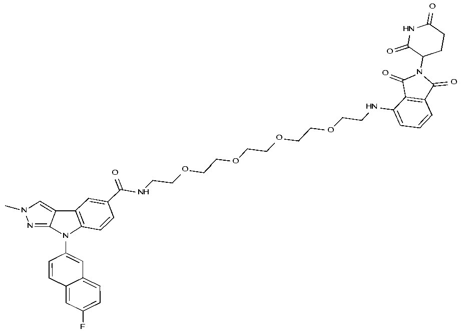 |
| 117 | 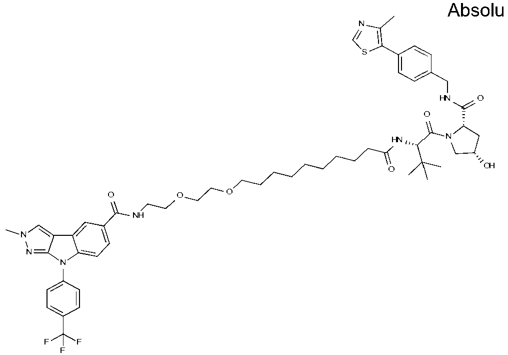 |
, ou un sel, solvate, tautomère et/ou stéréoisomère pharmaceutiquement acceptable de celui-ci .

13. Composé selon l'une quelconque des revendications précédentes, pour une utilisation comme médicament.

14. Composé selon l'une quelconque des revendications 1 à 12, ou un sel, solvate, tautomère et/ou stéréoisomère pharmaceutiquement acceptable de celui-ci, pour une utilisation dans le traitement et/ou la prévention d'une maladie ou d'une condition choisie dans le groupe constitué par : un cancer, en particulier les tumeurs y compris les tumeurs solides, le cancer du sein, le cancer du poumon, le cancer du foie, le cancer de l'ovaire, le cancer épidermoïde, le cancer du rein, le cancer de l'estomac, le médulloblastome, le cancer du côlon, le cancer du pancréas ; les maladies cardiovasculaires et la fibrose, en particulier la fibrose hépatique.

15. Composition pharmaceutique comprenant un composé selon l'une quelconque des revendications 1 à 12, ou un sel, solvate, tautomère et/ou stéréoisomère pharmaceutiquement acceptable de celui-ci, comme ingrédient actif conjointement avec un véhicule pharmaceutiquement acceptable.

16. Composition pharmaceutique selon la revendication 15, comprenant en outre un deuxième ingrédient actif, ou un sel, solvate, tautomère et/ou stéréoisomère pharmaceutiquement acceptable de celui-ci, ce deuxième ingrédient actif étant autre qu'un composé de formule I tel que défini selon l'une quelconque des revendications 1 à 12.

17. Ensemble (kit) comprenant des conditionnements séparés
a) d'une quantité efficace d'un composé de formule I selon l'une quelconque des revendications 1 à 12, ou d'un sel, solvate, tautomère et/ou stéréoisomère pharmaceutiquement acceptable de celui-ci ; et
b) d'une quantité efficace d'un autre ingrédient actif, cet autre ingrédient actif n'étant pas un composé de formule I tel que défini selon l'une quelconque des revendications 1 à 12.

18. Méthode de dégradation d'une protéine TEAD, comprenant la mise en contact de la protéine TEAD avec un composé tel que défini selon l'une quelconque des revendications 1 à 12.

19. Complexe ternaire, comprenant
(i) un composé selon l'une quelconque des revendications 1 à 12 ;
(ii) une protéine TEAD ; et
(iii) une ubiquitine ligase.

20. Composé choisi dans le groupe constitué par les intermédiaires illustrés dans le Tableau INT
| **Intermédiaire** | **Structure** |
|---|---|
| 1 | 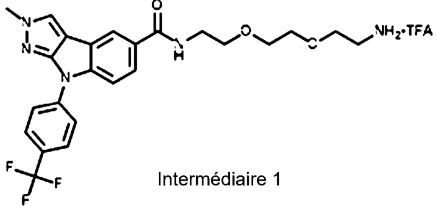 |
| 8 | 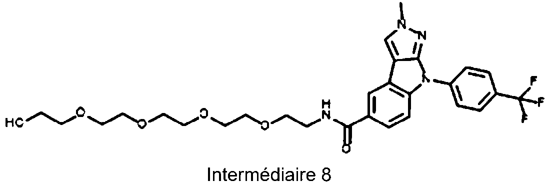 |
| 9 | 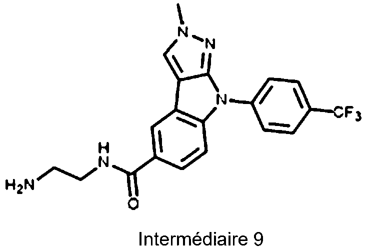 |
| 10 | 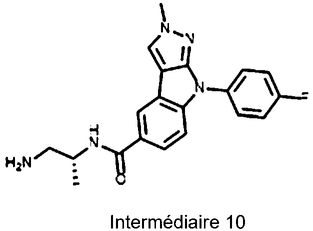 |
| 11 | 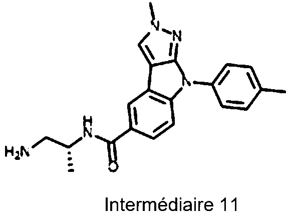 |
| 20 | 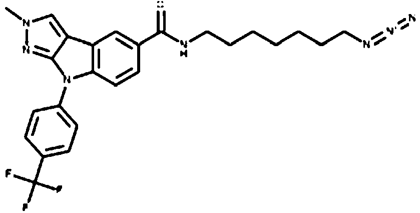 |
| 29 | 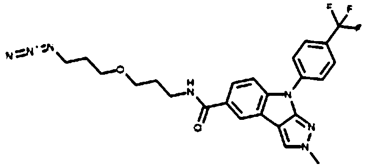 |
| 33 | 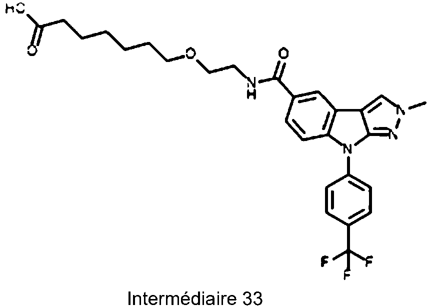 |
| 34 | 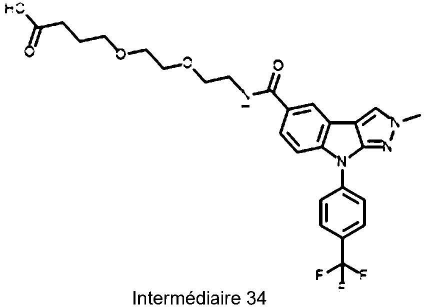 |
| 37 | 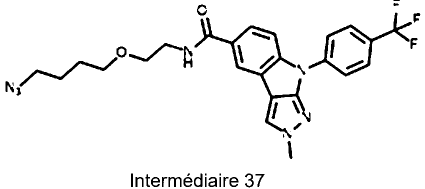 |
| 38 | 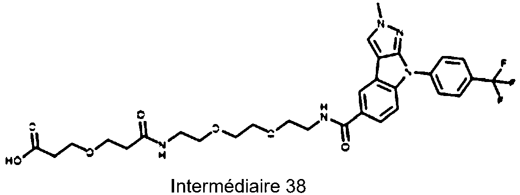 |
| 39 | 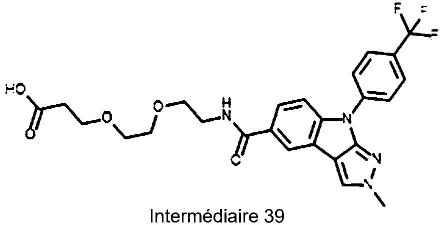 |
| 40 | 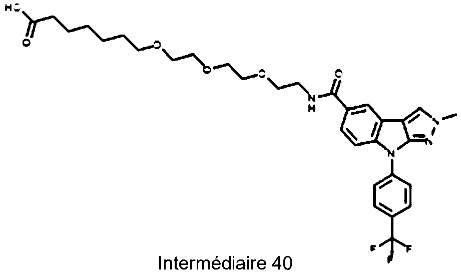 |
| 41 | 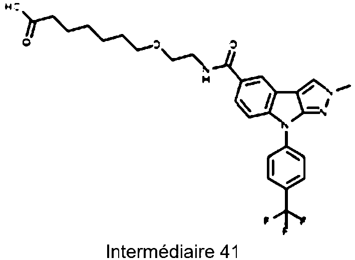 |
| 43 | 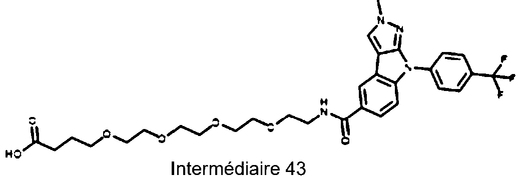 |
| 45 | 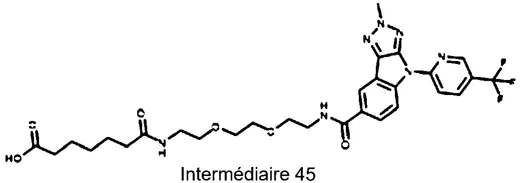 |
| 46 | 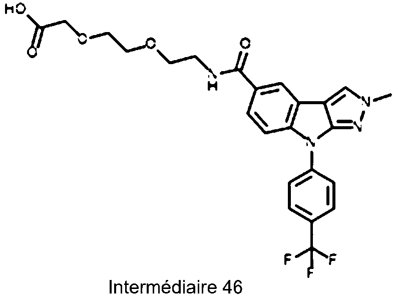 |
| 47 | 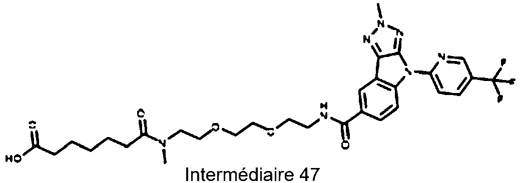 |
| 68 | 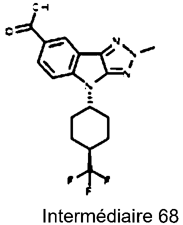 |
| 71 | 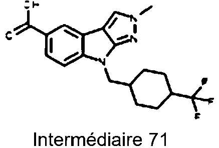 |
, ou un sel, solvate, tautomère et/ou stéréoisomère pharmaceutiquement acceptable de celui-ci.
